(19) Europäisches Patentamt
European Patent Office
Office européen des brevets

(11) EP 4 596 551 A1

(12) **EUROPEAN PATENT APPLICATION**
published in accordance with Art. 153(4) EPC

(43) Date of publication:
**06.08.2025 Bulletin 2025/32**

(21) Application number: **23870941.4**

(22) Date of filing: **27.09.2023**

(51) International Patent Classification (IPC):
***C07D 487/08*** (2006.01) ***C07D 519/00*** (2006.01)
***A61P 35/00*** (2006.01) ***A61K 31/517*** (2006.01)
***A61K 31/4995*** (2006.01)

(52) Cooperative Patent Classification (CPC):
**A61K 31/4995; A61K 31/517; A61K 31/519; A61P 35/00; A61P 35/02; C07D 487/08; C07D 519/00**

(86) International application number:
**PCT/CN2023/122129**

(87) International publication number:
**WO 2024/067714 (04.04.2024 Gazette 2024/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:
**30.09.2022 CN 202211208795**
**09.12.2022 CN 202211583282**
**17.01.2023 CN 202310080287**
**16.03.2023 CN 202310258788**
**16.06.2023 CN 202310721348**
**26.09.2023 CN 202311247776**

(71) Applicant: **Trasveda Ltd.**
**Grand Cayman KY1-1003 (KY)**

(72) Inventors:
- **SHANG, Erchang**
  **Shanghai 201112 (CN)**
- **ZHONG, Boyu**
  **Irving, TX 75038 (US)**
- **ZHANG, Tony Yantao**
  **Fishers, IN 46037 (US)**
- **HOU, Fuliang**
  **Shanghai 201203 (CN)**
- **SONG, Guanglin**
  **Shanghai 200125 (CN)**
- **LIU, Shuai**
  **Shanghai 201203 (CN)**
- **ZHENG, Aijun**
  **Shanghai 201399 (CN)**
- **WANG, Ruixiang**
  **Shanghai 201299 (CN)**
- **WANG, Longfei**
  **Shanghai 201210 (CN)**
- **JIANG, Jun**
  **Shanghai 201203 (CN)**
- **DONG, Chunlan**
  **Shanghai 201203 (CN)**

(74) Representative: **Vossius & Partner**
**Patentanwälte Rechtsanwälte mbB**
**Siebertstraße 3**
**81675 München (DE)**

(54) **COMPOUNDS WITH ANTI-KRAS MUTANT TUMOR ACTIVITY**

(57) The present invention provides compounds with the structure of formula (I) that can be used as KRAS inhibitors, pharmaceutical compositions containing these compounds, methods for preparing these compounds, and uses of these compounds in the treatment of cancer.

EP 4 596 551 A1

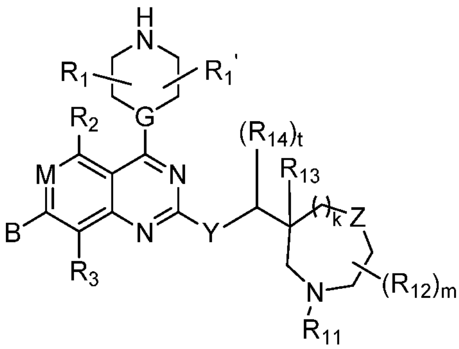

(I)

## Description

### CROSS-REFERENCE TO RELATED APPLICATIONS

This application claims the priority of:

- Chinese Patent Application No. 202211208795.6 filed on September 30, 2022,
- Chinese Patent Application No. 202211583282.3 filed on December 9, 2022,
- Chinese Patent Application No. 2023100802872 filed on January 17, 2023,
- Chinese Patent Application No. 2023102587885 filed on March 16, 2023,
- Chinese Patent Application No. 2023107213489 filed on June 16, 2023, and
- Chinese Patent Application No. 2023112477769 filed on September 26, 2023.

### Field of invention

The present invention relates to the field of pharmaceutical chemistry. More specifically, the present invention relates to a class of compounds with novel structures that can be used as KRAS inhibitors, pharmaceutical compositions containing such compounds, methods for preparing such compounds, and the use of these compounds in the treatment of cancer or tumors.

### Background

Ras, or rat sarcoma oncogene homolog, represents a group of closely related monomeric globular proteins belonging to the GTPase protein family. Specifically, under normal physiological conditions, Ras is activated by growth factors and various other extracellular signals, and is responsible for regulating cell growth, survival, migration, and differentiation. These regulatory functions of Ras are mediated through the transition between the GDP-bound state and GTP-bound state, known as the "molecular switch" (Alamgeer et al., Current Opin Pharmacol. 2013, 13:394-401). The GDP-bound Ras is the inactive form, in a dormant or off state, where the signaling system is turned off. When exposed to certain growth-promoting stimuli, it can be activated, for example, by guanine nucleotide exchange factors (GEFs), which induce the release of GDP and binding of GTP. As a result, Ras is "turned on," converting into its active form, which recruits and activates various downstream effectors to transmit signals. This process allows the transmission of signals from the cell surface to the cytoplasm, thereby controlling numerous critical cellular processes such as differentiation, survival, and proliferation (Zhi Tan et al., Mini-Reviews in Medicinal Chemistry, 2016, 16, 345-357).

Ras possesses GTPase activity, which enables it to hydrolyze the terminal phosphate of GTP, converting it into GDP and thereby transitioning itself into an inactive state. However, the endogenous GTPase activity of Ras is very low, and the conversion of GTP-bound Ras to GDP-bound Ras requires the involvement of exogenous proteins called GAPs (GTPase-Activating Proteins). GAPs interact with Ras and facilitate the conversion of GTP to GDP. Therefore, any mutations in the Ras gene that affect its interaction with GAPs or impair the conversion of GTP to GDP can result in Ras remaining in a persistently activated state. This leads to the continuous transmission of growth and division signals to the cell, stimulating uncontrolled cell proliferation and ultimately contributing to tumor formation and progression.

Among the genes associated with human tumors, there are three ubiquitously expressed Ras genes: H-RAS, K-RAS, and N-RAS, which encode highly homologous proteins of approximately 21 kDa HRas, NRas, and KRas, respectively. In 1982, researchers first discovered that Ras is mutationally activated in cancer cell lines (Chang, E.H. et al., Proceedings of the National Academy of Sciences of the United States of America, 1982, 79(16), 4848-4852). Subsequent large-scale genomic sequencing studies across different cancer types revealed that Ras proteins are mutated in over 30% of cancers, especially with the highest mutation rates in pancreatic cancer (>90%), colorectal cancer (45%), and lung cancer (35%). Transgenic and genetically engineered mouse models have also demonstrated that mutant Ras proteins are sufficient to drive and initiate various types of cancers. Moreover, the Ras oncogene is critical for the maintenance and progression of tumors in multiple cancer types. For example, RNA interference has been shown to slow tumor growth in Ras-mutant cancer cell lines and animal models. These studies have established Ras oncoproteins as a widely accepted and highly attractive target for anticancer drug development in the field of pharmacology.

Studies have shown that Ras mutations most frequently occur in KRas, with approximately 85% of Ras mutation-driven cancers exhibiting KRas mutations. The vast majority of Ras mutations occur at codons G12, G13, and Q61, with

about 80% of KRas mutations specifically occurring at glycine of codon 12, such as in G12C, G12D, G12V, G12A, G12R, G12S, and G13D mutations. KRas mutations are commonly found in pancreatic cancer, lung adenocarcinoma, colorectal cancer, gallbladder cancer, thyroid cancer, and cholangiocarcinoma, and are also observed in 25% of non-small cell lung cancer patients (McCormick, F. et al., Clinical Cancer Research 21(8), 1797-1801, 2015). As a result, KRas mutant proteins have become the most critical focus in Ras drug target research, and the development of inhibitors targeting KRas is considered a highly promising direction in anticancer/tumor drug development.

However, drug development targeting Ras over the past few decades have revealed that due to the smooth surface of the Ras protein, which lacks obvious grooves or pockets for binding small-molecule inhibitors, and its extremely high affinity (picomolar level) for guanine nucleotides, the development of small-molecule inhibitors has been fraught with seemingly insurmountable difficulties. As a result, Ras has long been considered an "undruggable" target in the field. At the same time, there remains a critical need for compounds with diverse structural types or modes of action as KRas inhibitors, to provide more therapeutic options or to offer improved inhibitory activity compared to existing KRas inhibitors, thereby delivering more potent clinical treatments.

The present invention addresses the above and other needs. It provides novel inhibitor compounds with structural innovations that exhibit inhibitory activity against KRas mutant proteins. Compared to existing KRas mutant protein inhibitors in prior art, the compounds of the present invention, due to their improved structural patterns, demonstrate enhanced inhibitory activity against KRas mutant proteins and related tumor suppression. They also possess favorable pharmacokinetic properties, making them highly drug-like. For example, they can be administered in convenient forms, are more readily absorbed *in vivo,* and exhibit reduced toxicity and side effects. Additionally, they offer improved resistance profiles, enhanced safety, and lower risks of drug-drug interactions.

**Summary of invention**

The present invention provides a compound having structural formula (I) as defined herein, a stereoisomer, tautomer, stable isotopic variant, pharmaceutically acceptable salt, or solvate thereof:

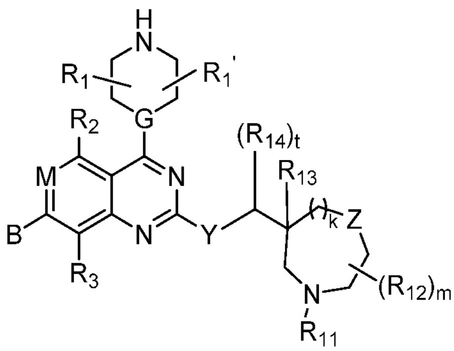

(I)

Wherein the definition of each group is as defined in the detailed description part of the invention.

The present invention further provides a pharmaceutical composition comprising a compound of the present invention, or a stereoisomer, tautomer, stable isotopic variant, pharmaceutically acceptable salt or solvate thereof, and optionally a pharmaceutically acceptable excipient or carrier.

The present invention further provides a compound of the present invention, or a stereoisomer, tautomer, stable isotopic variant, pharmaceutically acceptable salt or solvate thereof, for use as a medicament.

The present invention further provides a compound of the present invention, or a stereoisomer, tautomer, stable isotopic variant, pharmaceutically acceptable salt or solvate thereof, for use as an inhibitor of Ras mutant proteins, especially KRas mutant proteins (such as G12C mutant, G12D mutant, G12V mutant, G12A mutant, G12R mutant, G12S mutant and G13D mutant proteins), preferably KRas G12D.

The present invention further provides a compound of the present invention, or a stereoisomer, tautomer, stable isotopic variant, pharmaceutically acceptable salt or solvate thereof, or a pharmaceutical composition comprising them, for use in the treatment and/or prevention of diseases mediated by Ras mutant proteins, especially KRas mutant proteins (such as G12C, G12D, G12V, G12A, G12R, G12S, and G13D mutant proteins), preferably KRas G12D mutant protein.

The present invention further provides the use of the compound of the present invention, or a stereoisomer, tautomer, stable isotopic variant, pharmaceutically acceptable salt or solvate thereof, or a pharmaceutical composition comprising them, for the treatment and/or prevention of diseases mediated by Ras mutant proteins, especially KRas mutant proteins (such as G12C, G12D, G12V, G12A, G12R, G12S and G13D mutat proteins), preferably KRas G12D mutant protein.

The present invention further provides the use of the compound of the present invention, or a stereoisomer, tautomer, stable isotopic variant, pharmaceutically acceptable salt or solvate thereof, or a pharmaceutical composition comprising them, in the manufacture of a medicament for the treatment and/or prevention of diseases mediated by Ras mutant proteins, especially KRas mutant proteins (such as G12C, G12D, G12V, G12A, G12R, G12S, and G13D mutant proteins), preferably KRas G12D mutant protein.

The present invention further provides a method for the treatment and/or prevention of diseases mediated by Ras mutant proteins, especially KRas mutant proteins (such as G12C, G12D, G12V, G12A, G12R, G12S and G13D mutant proteins), preferably KRas G12D mutant protein. The method comprises administering to a subject in need thereof a therapeutically effective amount of the compound of the present invention, or a stereoisomer, tautomer, stable isotopic variant, pharmaceutically acceptable salt or solvate thereof, or a pharmaceutical composition comprising them.

The present invention further provides a method for treating tumors or cancers, which comprises administering to a patient in need thereof the compound of the present invention, or a stereoisomer, tautomer, stable isotopic variant, pharmaceutically acceptable salt or solvate thereof, or a pharmaceutical composition comprising them.

The present invention further provides the use of the compound of the present invention, or pharmaceutically acceptable salt or solvate thereof, as a KRas inhibitor, particularly as a research tool compound for inhibiting KRas G12D.

The present invention further provides a pharmaceutical combination, which comprises the compound of the present invention, a stereoisomer, tautomer, stable isotopic variant, pharmaceutically acceptable salt or solvate thereof, and one or more other pharmaceutically active agents.

The present invention further provides a method for preparing the compound of the present invention.

## Detailed description of invention

### Definitions

Unless otherwise indicated, each term used in the specification and claims has the meanings set forth below. In the absence of a specific definition for a particular term or phrase, it should be understood in accordance with its ordinary meaning in the art. In case of conflict, the present specification (including the definitions) shalled.

In case of a conflict between the chemical structures and names of the compounds disclosed herein, the chemical structures shall prevail.

The term "Ras mutation" or "Ras mutant protein" as used herein refers to a protein encoded and expressed by Ras gene in which one or more codons are mutated, typically including but not limited to Ras proteins with mutations in glycine at codon 12, glycine at codon 13, or glutamine at codon 61 of Ras, such as mutant HRas, NRas, or KRas. These residues are located at the active site of Ras, and their mutations can impair the intrinsic or GAP-catalyzed GTPase activity of Ras, leading to the persistent presence of GTP-bound Ras.

For the purposes of the present invention, "Ras mutation" or "Ras mutant protein" and "Ras" when describing inhibitory activity are used interchangeably, and generally refer to mutant HRas, NRas, or KRas, for example but not limited to KRas-G12C (mutation of glycine to cysteine at codon G12), KRas-G12D (mutation of glycine to aspartic acid at codon G12), HRas-G12D, NRas-G12D, KRas-G12V (mutation of glycine to valine at codon G12), KRas-G13D (mutation of glycine to aspartic acid at codon G13). Specifically, the terms refer to KRas mutant proteins, more specifically to KRas-G12C, KRas-G12D, KRas-G12V, G12A, G12R, G12S, and KRas-G13D mutant proteins, and most specifically to KRas-G12D mutant protein.

The term "treatment" as used herein refers to the administration of one or more of the compounds of the present invention or their pharmaceutically acceptable salts or solvents to cure, relieve, mitigate or affect the disease or its symptoms to subjects suffering from or having symptoms of the disease described, such as mammals, such as humans. Preferably, treatment is curative or improving.

The term "prevention" used herein is well-known in the art and refers to subjects suspected of developing or predisposing to Ras mutation-mediated disease as defined herein, especially cancer or tumors, such as mammals, or humans administered one or more of the compounds described herein or their pharmaceutically acceptable salts or solvents to reduce the risk of developing the disease as defined, or to prevent the onset of the disease. The term "prevention" includes compounds of this invention used prior to diagnosis or identification of any clinical and/or pathological symptoms.

The terms "inhibit" and "reduce" used herein, or any variant of these terms, refer to the ability of a bioactive agent to reduce the signaling activity of the target by interacting directly or indirectly with the target, and refer to any measurable reduction or complete inhibition of target activity. For example, compared to normal conditions, it can be about, at most or at least about 5%, 10%, 15%, 20%, 25%, 30%, 35%, 40%, 45%, 50% reduction in activity (e.g., KRas activity), 55%, 60%, 65%, 70%, 75%, 80%, 85%, 90%, 95%, 99% or more, or any range derivable therefrom.

The term "selective inhibition" used herein refers to the ability of a biological active agent to preferentially reduce the signaling activity of a target of interest as compared to off-target signaling activity by interacting directly or indirectly with

the target. As far as the compound of this invention is concerned, it has the ability to selectively inhibit G12 or G13 mutations of KRas, HRas or NRas proteins relative to various types of mutations occurring at one or more codons of Ras protein, examples include the G12C mutation, the G12D mutation, the G12V mutation, the G12A mutation, the G12R mutation, the G12S mutation, and the G13D mutation, which are preferred for their ability to selectively inhibit the G12D mutation in the KRas protein. For example, the present invention has an inhibitory activity for a specific Ras mutation that is at least 5%, 10%, 15%, 20%, 25%, 30%, 35%, 40%, 45%, 50%, 55%, 60%, 65%, 70%, 75%, 80%, 85%, 90%, 95%, 99% or more, or any range derivable therefrom, better than that for another specific Ras mutation. Alternatively, it has at least 1-, 2-, 3-, 4-, 5-, 10-, 25-, 50-, 100-, 250-or 500-fold better activity against a specific Ras mutation (e.g., KRas-G12D) compared to its activity against another specific Ras mutation.

The term "Ras mutation-mediated disease" as used herein refers to a disease in which Ras mutation promotes the occurrence and development of the disease, or inhibition of Ras mutation will reduce the incidence of the disease and alleviate or eliminate the symptoms of the disease. For the purposes of the present invention, the "Ras mutation-mediated disease " preferably refers to a disease mediated by KRas mutation, most preferably a disease mediated by KRas-G12D, and even more preferably cancer or a tumor mediated by KRas-G12D.

The term "cancer" or "tumor" used herein refers to abnormal cell growth and proliferation, whether malignant or benign, and all premalignant cells and cancer cells and tissues. For all aspects of this invention, the mentioned cancers or tumors include, but are not limited to, lung adenocarcinoma, lung cancer, bone cancer, pancreatic cancer, skin cancer, head and neck cancer, skin or intraocular melanoma, uterine cancer, ovarian cancer, rectal cancer, anal region cancer, gastric cancer, colon cancer, breast cancer, fallopian tube cancer, endometrial cancer, cervical cancer, vaginal cancer, vulvar cancer, Hodgkin 's disease, esophageal cancer, small bowel cancer, endocrine system cancer, thyroid cancer, parathyroid carcinoma, adrenal carcinoma, soft tissue sarcoma, urethral cancer, penile cancer, prostate cancer, chronic or acute leukemia, lymphocytic lymphoma, bladder cancer, renal or ureteral cancer, renal cell carcinoma, renal pelvis cancer, central nervous system tumor (CNS), primary CNS lymphoma, spinal tumor, brainstem glioma, or pituitary adenoma.

For all aspects of this invention, the cancer or tumor described is preferably associated with Ras mutations, especially KRas mutations, and preferably KRas G12D mutations, including, but not limited to, the tumor types described above and their preferred range. Particularly preferred tumors of the present invention include lung cancer, lung adenocarcinoma, colon cancer, rectal cancer, pancreatic cancer, endometrial cancer, cholangiocarcinoma, leukemia and ovarian cancer.

The terms "Subject", "Individual", or "Patient" used herein refer to a vertebrate. In some embodiments, the vertebrate is a mammal. Mammals include, but are not limited to, farm animals (e.g., cattle), sports animals, pets (e.g., guinea pigs, cats, dogs, rabbits, and horses), primates, mice, and rats. In some embodiments, the mammal is a human.

The term "therapeutically effective amount" as used herein refers to an amount or dose sufficient to elicit a beneficial therapeutic response in a patient afflicted with a "Ras mutation-mediated disease", such as cancer or tumors. A person skilled in the art may determine the effective amount or dose of the active ingredient of the present invention using conventional methodologies, in combination with routine influencing factors.

The term "pharmaceutical combination" as used herein refers to a combination of the compound of the present invention with other active agents to achieve the objectives of the invention. The other active agent(s) may be one or more additional compounds of the present invention, or may be a second or further (e.g., third) compound that is compatible with the compound of the present invention, meaning that they do not adversely affect each other or exhibit complementary activities. For example, such active agents may be known to modulate other biological pathways, regulate different components of the biological pathway involved in the compound of the present invention, or even overlap with the biological target of the compound of the present invention. These active agents are suitably combined in effective amounts to achieve the intended purpose. The other active agent(s) may be co-administered with the compound of the present invention in a single pharmaceutical composition or administered separately in distinct units. When administered separately, the administration may be simultaneous or sequential. Sequential administration may be closely spaced or separated by a longer interval.

The term "pharmaceutically acceptable" as used herein refers to molecular entities and compositions that, when administered in appropriate amounts to an animal, such as a human, do not produce adverse, allergic, or other undesirable reactions.

The term "pharmaceutically acceptable salt" as used herein refers to salts that retain the biological effectiveness and properties of the parent compound and are not biologically or otherwise undesirable. These include acid addition salts and base addition salts. "Pharmaceutically acceptable acid adducts" can be formed from compounds with basic groups with inorganic or organic acids, inorganic acids such as hydrochloric acid, hydrobromic acid, sulfuric acid, nitric acid, carbonic acid, phosphoric acid, etc. organic acids can be selected from aliphatic, aliphatic, aromatic, aromatic, heterocyclic, carboxylic, and sulfonic organic acids, such as formic acid, acetic acid, propionic acid, glycolic acid, gluconic acid, lactic acid, pyruvate, oxalic acid, apple acid, maleic acid, malonic acid, succinic acid, fumaric acid, tartaric acid, citric acid, aspartic acid, glutamic acid, anthranilic acid, benzoic acid, cinnamic acid, mandelic acid, pamoate, phenylacetic acid, methanesulfonic acid, ethanesulfonic acid, benzenesulfonic acid, p-toluenesulfonic acid, salicylic acid, etc. "Pharmaceutically acceptable base addition salts" include those derived from inorganic bases such as salts of sodium, potassium, lithium, ammonium, calcium, magnesium, iron, zinc, copper, manganese, and aluminum, and salts derived from pharmaceutically acceptable organic non-toxic bases, including but not limited to primary, secondary and tertiary amines, substituted ammonium, including naturally occurring substituted amines, cyclic amines and basic ion exchange resins, such as ammonia, isopropylamine, trimethylamine, diethylamine, triethylamine, tripropylamine, ethanolamine, diethanolamine, 2-bis methyl aminoethanol, 2-diethylaminoethanol, tromethamine, dicyclohexylamine, lysine, arginine, histidine, caffeine, procaine, hybamine, choline, betaine, ethylenediamine, glucosamine, methylglucosamine, triethanolamine, theobromine, purine, piperazine, piperidine, N-ethylpiperidine, polyamine resin, etc.

The term "isomer" used herein refers to any stereoisomer, enantiomeric mixture, including racemate, diastereomeric mixture, geometric isomer, atropisomer, and/or tautomer that may exist in the structure of a compound. The methods for determining and separating the stereochemistry of such isomers are well-known to those skilled in the art (S. P. Parker, Ed., McGraw-Hill Dictionary of Chemical Chemistry Terms (1984) McGraw-Hill Book Company, New York; and Eliel, E. and Wilen, S.,"Stereotics of Organic Compounds", John Wiley & Sons, Inc., New York, 1994).

Certain compounds of the present invention contain at least one asymmetric center and thus give rise to stereoisomers. Accordingly, the present invention encompasses all possible isomeric forms of the compounds defined herein, as well as their pharmaceutically acceptable salts or solvates, unless otherwise indicated.

" 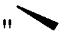 "

or

" 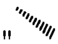 "

used in the structural formulae or structural fragments of the compounds herein represent the absolute configuration of the stereocenter, i.e., the chiral center. Correspondingly, in the naming of the compounds or intermediates provided in the present invention, R or S is used to represent the absolute configuration of the relevant chiral center; " "\" attached to a chiral center indicates the presence of both configurations at the chiral center in a racemic form. For example,

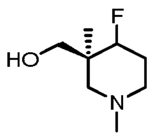

represents a mixture of

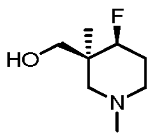

and

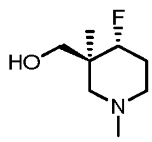
.

In the definition of some compounds of the present invention, axial chirality can also be used to represent the configuration of the compounds. The determination of these configurations follows the Cahn-Ingold-Prelog rules well-known to those skilled in the art. The absolute configurations of axial chirality in the two example structures below are described as follows:

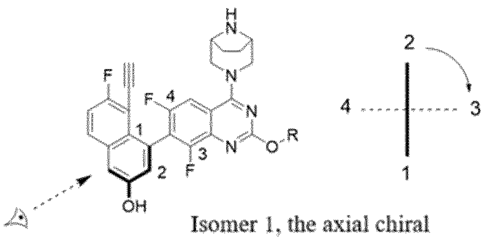

Isomer 1, the axial chiral configuration is labeled Ra

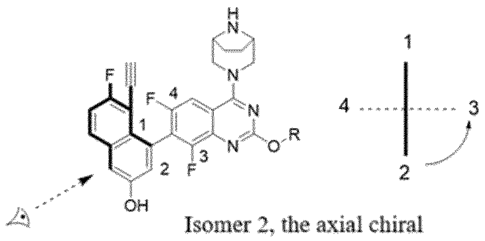

Isomer 2, the axial chiral configuration is labeled Sa

When the axial chiral bond is marked with "*", it indicates that the compound has a single chiral configuration and is obtained through SFC resolution, but the absolute configuration is undetermined, e.g.,

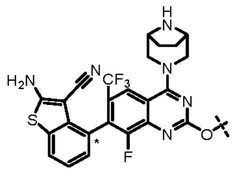

represents

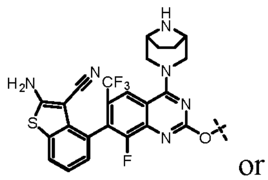

or

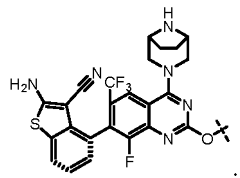

.

**In** the compound definitions, structural formulas, or structural fragments herein, the number of groups attached to each atom is determined by the valence of the atom and does not need to be fully shown. Generally, only non-hydrogen groups are shown in the group definitions, structural formulas, or structural fragments, and the groups not shown generally represent H. Those skilled in the art can easily determine whether the unshown groups exist and the number of them.

It should be understood that when those skilled in the art can determine, based on the compound structure shown herein, that the compound has a pair of chiral isomers and can be easily resolved by conventional methods in the art, then the disclosure of the racemate of the compound herein (whether in the form of a structural formula or a chemical name) shall be regarded as having separately disclosed each isomer of the compound.

In the structural fragments described herein, the symbol " " indicates that the bond intersecting it represents the point of attachment of the structural fragment to the rest of the molecule.

In the cyclic structural fragments described herein, substituents depicted as spanning a chemical bond, such as $-(R_{12})_m$ in

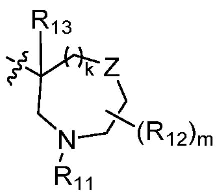

,

indicate that one or more $R_{12}$ substituents may replace one or more chemically feasible substitution sites on the ring, including Z.

The compounds of the present invention include both unlabeled forms and isotopically labeled forms of the compounds. Isotopically labeled forms of the compounds are those in which one or more atoms are replaced by atoms enriched with the corresponding isotope. Examples of isotopes that can be incorporated into the compounds of the present invention include isotopes of hydrogen, carbon, nitrogen, oxygen, fluorine, chlorine, and iodine, such as $^{2}H$, $^{3}H$, $^{11}C$, $^{13}C$, $^{14}C$, $^{15}N$, $^{18}O$, $^{17}O$, $^{35}S$, $^{18}F$, $^{37}Cl$, and $^{125}I$. Such isotopically labeled compounds can be used, for example, as probes in biological assays, analytical tools, or therapeutic agents. In certain embodiments, the compounds of the present invention are provided in unlabeled form. In other embodiments, the compounds are provided in isotopically labeled form, such as

deuterium (D)-labeled form. Specifically, in the general formula (I) and its sub-formulas, one or more hydrogen atoms (H) in the $R_{11}$ and $R_{14}$ groups may be replaced by deuterium (D). For example, $R_{14}$ may independently be H or D, and $R_{11}$ may be substituted by one or more D, particularly a $-C_{1-6}$ alkyl group substituted by one or more D.

The term "solvate" as used herein refers to a solvent addition form of a compound that contains stoichiometric or non-stoichiometric amounts of solvent. This includes any solvated form of the compounds of the present invention, such as solvates with water (e.g., hydrates) or with organic solvents (e.g., methanol, ethanol, or acetonitrile), referred to as methanolates, ethanolates, or acetonitrileates, respectively. It also includes solvates in any polymorphic form. It should be understood that such solvates of the compounds of the present invention also encompass solvates of pharmaceutically acceptable salts of the compounds.

The term "metabolite" used herein refers to a product generated from the metabolism of a compound *in vivo.* Such products can be derived, for example, from oxidation, reduction, hydrolysis, amidation, deamidation, esterification, de-esterification, enzymatic cleavage, etc. of the administered compound. Identification and analysis of metabolites were performed in a manner well-known to those skilled in the art.

The term "pharmaceutically acceptable vehicle" or "pharmaceutically acceptable carrier" used herein refers to one or more compatible solid or liquid fillers or gel substances that are suitable for human use and have sufficient purity and low toxicity, in fact examples include, but are not limited to cellulose and its derivatives (e.g., sodium carboxymethylcellulose, cellulose acetate, etc.), gelatin, talc, solid lubricants (e.g., magnesium stearate), calcium sulfate, vegetable oil, polyols (e.g., propylene glycol, glycerol, mannitol, sorbitol, etc.), emulsifiers (e.g., Tween), humectants (e.g., sodium lauryl sulfate), colorants, flavorants, stabilizers, antioxidants, preservatives, etc.

The term "halogen" or "halogenated" used herein refers to F, Cl, Br, or I. In addition, the term "halogen-substituted" used in the definition of groups herein is intended to include mono- or poly-halogenated groups, in which one or more identical or different halogens replace one or more hydrogens in the corresponding group.

The term alkyl used herein refers to monovalent saturated hydrocarbon groups consisting of straight or branched chains of carbon and hydrogen atoms. Specifically, alkyl groups have 1-10, examples include 1 to 8, 1 to 6, 1 to 5, 1 to 4, 1 to 3, or 1 to 2 carbon atoms. For example, as used herein, the term '$C_1$-$_6$ alkyl' refers to saturated hydrocarbon groups having straight or branched chains of 1 to 6 carbon atoms, examples include methyl, ethyl, propyl (including n-propyl and isopropyl), butyl (including n-butyl, isobutyl, sec-butyl or tert-butyl), pentyl (including n-pentyl, isopentyl, neo-pentyl), n-hexyl, 2-methylpentyl, etc.

The term "-O-alkyl" or "alkoxy" used herein refers to an alkyl group defined herein that is connected by an oxygen atom to the rest of the molecule. Specifically, the -O-alkyl groups have 1-10, examples include 1 to 8, 1 to 6, 1 to 5, 1 to 4, 1 to 3, or 1 to 2 carbon atoms. For example, as used herein, the term "-O-$C_1$-$_6$ alkyl" refers to a saturated hydrocarbon group having a straight or branched chain of 1 to 6 carbon atoms connected to the rest of the molecule by an oxygen atom, examples include-O-methyl, -O-ethyl, -O-propyl (including-O-n-propyl and-O-isopropyl), -O-butyl (including-O-n-butyl, -O-isobutyl, -O-sec-butyl or -O-tert-butyl), -O-pentyl (including-O-n-pentyl, -O-isoamyl, -O-neo-pentyl), -O-n-hexyl, 2-methylpentyl-O-, etc.

The term "-S-alkyl" used herein refers to an alkyl group defined herein that is connected to the rest of the molecule by a sulfur atom. Specifically, the-O-alkyl groups have 1-10, examples include 1 to 8, 1 to 6, 1 to 5, 1 to 4, 1 to 3, or 1 to 2 carbon atoms. For example, as used herein, the term "-S-$C_1$-$_6$ alkyl" refers to a saturated hydrocarbon group of a straight or branched chain having 1 to 6 carbon atoms connected to the rest of the molecule by a sulfur atom, examples include-S-methyl, -S-ethyl, -S-propyl (including-S-n-propyl and-S-isopropyl), -S-butyl (including-S-n-butyl, -S-isobutyl, -S-sec-butyl or -S-tert-butyl), -S-pentyl (including-S-n-pentyl, -S-isoamyl, -S-neo-pentyl), -S-n-hexyl, 2-methylpentyl-S-, etc.

As used herein, the term "optionally halogen-substituted $C_{1-6}$ alkyl" refers to the $C_1$-$_6$ alkyl groups described above, in which one or more (e.g., 1, 2, 3, 4 or 5) hydrogen atoms are optionally replaced by halogens. Those skilled in the art should understand that halogens can be the same or different and can be located on the same or different C atoms when there is more than one halogen substituent. Examples of halogen-substituted $C_1$-$_6$ alkyl groups are e.g.-$CH_2F$, -$CHF_2$, -$CF_3$, -$CCl_3$, - $C_2F_5$, -$C_2Cl_5$, -$CH_2CF_3$, -$CH_2Cl$, -$CH_2CH_2CF_3$ or -$CF(CF_3)_2$, etc.

The term "alkenyl" as used herein refers to an unsaturated hydrocarbon group composed of carbon and hydrogen atoms containing at least one double bond, which can be straight-chain or branched. Specifically, the alkenyl group has 2-8 such as 2 to 6, 2 to 5, 2 to 4, or 2 to 3 carbon atoms. For example, as used herein, the term "$C_2$-$_6$ alkenyl" refers to an alkenyl group with a straight or branched chain of 2 to 6 carbon atoms, such as vinyl, propenyl, allyl, butenyl, pentenyl, etc. The carbon atom in the alkenyl group that is connected to the rest of the molecule can be saturated or alkenyl carbon atoms.

The term "alkynyl" as used herein refers to an unsaturated hydrocarbon group composed of carbon and hydrogen atoms containing at least one double bond, which can be straight-chain or branched. Specifically, the alkynyl group has 2-8 such as 2 to 6, 2 to 5, 2 to 4, or 2 to 3 carbon atoms. For example, as used herein, the term "$C_2$-$_6$ alkynyl" refers to an alkynyl group with a straight or branched chain of 2 to 6 carbon atoms, such as ethynyl, propynyl, butynyl, etc. The carbon atom in the alkynyl group that is connected to the rest of the molecule can be saturated or alkynyl bond carbon atoms.

The term "cycloalkyl" refers to a non-aromatic, fully saturated monocyclic, fused polycyclic, bridged polycyclic, or spirocyclic hydrocarbon group with a specified number of ring carbon atoms. The cycloalkyl groups can contain 3 to 12 carbon atoms (i.e. $C_{3-12}$ cycloalkyl), examples include 3 to 10, 3 to 8, 3 to 7, 3 to 6, 5 to 6 carbon atoms. Examples of suitable cycloalkyl groups include, but are not limited to, monocyclic structures such as cyclopropyl, cyclobutyl, cyclopentyl, cyclohexyl, cycloheptyl, or cycloctyl; or polycyclic (e.g., bicyclic) structures including spiro, fused, or bridged systems, such as bicyclo[1.1.1]pentyl, bicyclo[2.2.1]heptyl, spiro[3.4]octyl, bicyclo[3.1.1]hexyl, bicyclo[3.1.1]heptyl or bicyclo[3.2.1]octyl. For example, the term "$C_{3-6}$ cycloalkyl", as used in this document to define a compound, refers to monocyclopropyl, cyclobutyl, cyclopentyl, or cyclohexyl.

The term "heterocycloalkyl" used herein refers to a monocyclic, fused polycyclic, spirocyclic or bridged polycyclic non-aromatic saturated ring structure that contains one or more (e.g., 1, 2, 3 or 4) heteroatoms independently selected from O, N and S and a specified number of ring atoms, or its N-oxide, or its S-oxide or S-dioxide. The heterocycloalkyl group can contain 3 to 12 ring members (which can be referred to as a 3- to 12-membered heterocycloalkyl), e.g., 3 to 10 ring members, 3 to 8 ring members, 3 to 7 ring members, 4 to 7 ring members, 4 to 6 ring members, 5 to 6 ring members. The heterocycloalkyl group generally contains to 4 (e.g., 1, 2, 3 or 4) heteroatoms. For instance, it can be a 4-7 membered heterocycloalkyl group containing 1 to 3 heteroatoms selected from N, O and S. Examples of suitable heterocycloalkyl groups include, but are not limited to, azetidinyl, oxetanyl, thietanyl, pyrrolidinyl (e.g., 1-pyrrolidinyl, 2-pyrrolidinyl, and 3-pyrrolidinyl), tetrahydrofuryl (e.g., 1-tetrahydrofuryl, 2-tetrahydrofuryl, and 3-tetrahydrofuryl), tetrahydrothienyl (e.g., 1-tetrahydrothienyl, 2-tetrahydrothienyl and 3-tetrahydrothienyl), piperidinyl (e.g., 1-piperidinyl, 2-piperidinyl, 3-piperidinyl and 4-piperidinyl), tetrahydropyranyl (e.g., 4-tetrahydropyranyl), tetrahydrothianyl (e.g., 4-tetrahydrothianyl), morpholinyl (e.g., morpholino), thiomorpholino, dioxanyl, piperazine or azacycloheptyl, diazacycloheptyl e.g., 1,4-diazacycloheptyl, 3,6-diaza-bicyclo[3.1.1]heptyl or 3-aza-bicyclo[3.2.1]octyl. The atom in the heterocycloalkyl group that is connected to the rest of the compound can be either a carbon atom or a heteroatom, as long as it is chemically feasible.

Preferred heterocycloalkyl groups are, for example

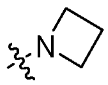 , 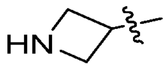 , 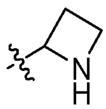 , 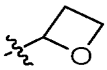 ,

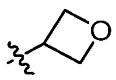 , 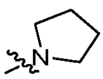 , 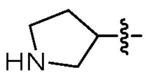 , 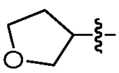 , 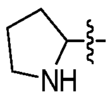 , 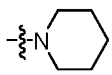 , 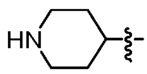 , 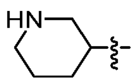 ,

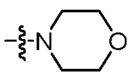 , 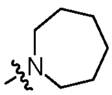 , 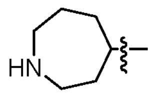 ;

It should be understood that structures with asymmetric centers encompass their racemic and/or single enantiomeric forms, for example,

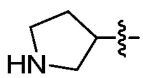

represented

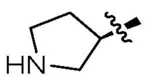 and/or 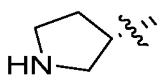 .

The term "heteroaryl" as used herein refers to a monocyclic or fused polycyclic aromatic ring structure containing one or more (e.g., 1, 2, 3, or 4) heteroatoms independently selected from O, N, and S and a specified number of ring atoms, or its N-oxide, or its S-oxide or S-dioxide. Specifically, the aromatic ring structure may have 5 to 12 ring members. The heteroaryl group can be, for example, a 5-6 membered monocyclic ring, or a fused bicyclic structure formed by two fused 6-membered rings, two fused 5-membered rings, a fused 6-membered ring and a 5-membered ring, or a fused 5-membered ring and a 4-membered ring. The heteroaryl ring usually contains up to 4 heteroatoms, more usually up to 3 heteroatoms, and the heteroatoms are independently selected from O, N, and S, where N and S can be in oxidized states such as N-oxide, S=O, or $S(O)_2$. In one embodiment, the heteroaryl group contains at least one ring nitrogen atom, at least one ring sulfur atom, or at least one ring oxygen atom. For example, the heteroaryl group can be a 5-6 membered heteroaryl group containing 1-3 heteroatoms independently selected from N, O or S. For example, a heteroaryl can be a 5-6 membered heteroaryl containing 1-3 heteroatoms independently selected from N, O or S. Examples of suitable 5-membered monocyclic heteroaryl groups include, but are not limited to, pyrrolyl, furyl, thiophenyl, imidazolyl, furazolyl, oxazolyl, oxadiazolyl, oxotriazolyl, isoxazolyl, thiazolyl, isothiazolyl, pyrazolyl, triazolyl, and tetrazolyl groups; examples of suitable 6-membered monocyclic heteroaryl groups include, but are not limited to pyridyl, pyrazinyl, pyrimidine, and triazine groups. For example, heteroaryl groups can also be fused rings containing 1, 2, 3, or 4 heteroatoms independently selected from N, O or S, such as benzofuran, benzothiophene, indole, benzimidazole, indazole, benzotriazole, pyrrolo [2,3-b]pyridine, pyrrolo[2,3-c]pyridine, pyrrolo[3,2-c]pyridine, pyrrolo[3,2-b]pyridine, imidazo[4,5-b]pyridine, imidazo[4,5-c]pyridine, pyrazolo[4,3-d]pyridine, pyrazolo[4,3-c]pyridine, pyrazolo[3,4-c]pyridine, pyrazolo[3,3-c]4-b]pyridine, isoindole, purine, indene, imidazolo[1,2-a]pyridine, imidazolo[1,5-a]pyridine, pyrazolo[1,5-a]pyridazine, pyrrolo[1,2-b]pyrimidine, imidazolo[1,2-c]pyrimidine, 5H-pyrrolo[3,2-b]pyrazine, 1H-pyrazolo[4,3-b]pyrazine, 1H-pyrazolo[3,4-d]pyrimidine, 7H-pyrrolo[2,3-d]pyrimidine, quinoline, isoquinoline, misoprostol, quinazoline, quinoxaline, phthalazine, 1,6-naphthyridine, 1,7-naphthyridine, 1,8-nalidixine, 1,5-nalidixine, 2,6-nalidixine, 2,7-nalidixine, pyrido[3,2-d]pyrimidine, pyrido[4,3-d]pyrimidine, pyrido[3,4-d]pyrimidine, pyrido[2,3-d]pyrimidine, pyrido[2,3-b]pyrazine, pyrido[3,4-b]pyrazine, pyrimidino [5,4-d]pyrimidine, pyrazino[2,3-b]pyrazine, and pyrimido[4,5-d]pyrimidine. The atom in the heterocycloalkyl group that is connected to the rest of the compound can be either a carbon atom or a heteroatom, as long as it is chemically feasible.

The term "hydroxyl" used herein refers to the -OH group.

The term "cyano" used herein refers to the -CN group.

The term "optionally substituted" as used herein, unless otherwise specified, indicates that a group may be either unsubstituted or substituted by one or more substituents (e.g., 1, 2, 3, 4, 5, or more, or any derivable range) listed for that group, where the substituents may be identical or different. In one embodiment, the optionally substituted group has one substituent. In another embodiment, the optionally substituted group has two identical or different substituents. In another embodiment, the optionally substituted group has three identical or different substituents. In another embodiment, the optionally substituted group has four identical or different substituents. In another embodiment, the optionally substituted group has five identical or different substituents.

Many of the groups defined herein are optionally substituted. The list of substituents provided in this definition section is merely exemplary and is not intended to limit the substituents defined in other parts of the specification and the claims.

Unless otherwise specified, $C_{n-n+m}$ or $C_n$-$C_m$ in the compound definition of this invention includes various cases of n to n+m carbons, $C_{1-6}$, for example, includes $C_1$, $C_2$, $C_3$, $C_4$, $C_5$ and $C_6$, Also include any of the ranges n to n+m, $C_{0-6}$ includes $C_1$, $C_2$, $C_3$, $C_4$, $C_5$, $C_6$, $C_{0-1}$, $C_{0-2}$, $C_{0-3}$, $C_{0-4}$, $C_{0-5}$, $C_{1-2}$, $C_{1-3}$, $C_{1-4}$, $C_{2-3}$, etc., $C_{1-6}$ includes $C_{1-2}$, $C_{1-3}$, $C_{1-4}$, $C_{2-6}$, $C_{3-6}$, etc.

In the field of organic synthesis, it is understood by those skilled in the art that all substituents on the compound structure disclosed herein, whether unsubstituted or substituted by defined groups, are designed to ensure chemical feasibility and stability of the molecule. The type and number of substituents are determined by the atomic count and valency of the parent group.

As used in this specification and the appended claims, the term "comprising" and its variants such as "including" and "containing" are intended to mean "including but not limited to," and are not intended to exclude other additives, components, integers, or steps. When an element is described as comprising multiple components, steps, or conditions, it should be understood that the element may also be described as comprising any combination of the multiple components, steps, or conditions, or "consisting of the multiple or combined components, steps, or conditions" or "essentially consisting of the multiple or combined components, steps, or conditions".

It should be understood that when the doses of the compounds of the present invention, pharmaceutical compositions containing them, drug combinations, kits, and related uses and methods are described herein, they are based on the weight of the free form, excluding any salts, hydrates, or solvates thereof, unless specified in the description that the dose is based on the weight of the salt, hydrate, or solvate.

## Problem solved by this invention

As mentioned above, compounds capable of inhibiting Ras mutant proteins, especially KRas mutant proteins (such as G12C, G12D, G12V, G12A, G12R, G12S, and G13D mutant proteins), and more especially KRas-G12D mutant proteins, can be used to treat or prevent mutant protein-mediated disease (e.g., cancer or tumor). Therefore, several structural types of Ras inhibitors have been developed in this field. However, existing KRas inhibitors still face challenges that need to be addressed, including unsatisfactory antitumor activity, toxic side effects leading to poor drug resistance, suboptimal pharmacokinetic properties that hinder convenient administration (i.e., poor "drugability"), or undesirable drug interactions due to inhibition of the cytochrome P450 enzyme system, among others. Furthermore, even for inhibitors with

good antitumor activity, there remains a desire to further enhance their selective inhibitory activity against target proteins in vivo, improve their drug resistance (reduced toxicity or better safety), and optimize their pharmacokinetic properties through structural optimization, thereby providing more and better therapeutic options for clinical use.

## Problem solving method

Through extensive and in-depth research, the inventors have developed a series of compounds that exhibit significant inhibitory activity against Ras mutant proteins, especially KRas mutant proteins (such as G12C, G12D, G12V, G12A, G12R, G12S, and G13D mutant proteins), and more especially, the KRas-G12D mutant protein. By performing structural modifications and activity validation, the inventors discovered that introducing specific types of substituents at certain positions on the benzopyrimidine ring of the KRas inhibitor structure, along with specific combinations of substitution sites and substituent types, resulted in compounds with significantly enhanced inhibitory activity against the KRas-G12D mutant protein compared to existing inhibitors. Moreover, these modified compounds demonstrate excellent safety profiles, reduced risks of drug interactions, and favorable, or even further improved, pharmacokinetic properties, enabling convenient administration methods.

Accordingly, the present invention primarily provides effective Ras inhibitors, specifically KRas inhibitors (e.g., inhibitors targeting G12C, G12D, G12V, G12A, G12R, G12S, and G13D mutations), and more specifically KRas-G12D inhibitor compounds; as well as pharmaceutical compositions containing such compounds as active ingredients; As pharmaceuticals, the compounds are used for treating or preventing tumors or cancers mediated by or benefiting from the inhibition of Ras, specifically KRas (such as G12C, G12D, G12V, G12A, G12R, G12S, and G13D mutations), and more specifically KRas-G12D; methods of using the compounds for treating or preventing diseases such as tumors or cancers mediated by or benefiting from the inhibition of Ras, specifically KRas (such as G12C, G12D, G12V, G12A, G12R, G12S, and G13D mutations), and more specifically KRas-G12D; and the use of the compounds in the manufacture of medicaments for treating or preventing diseases such as tumors or cancers mediated by or benefiting from the inhibition of Ras, specifically KRas (such as G12C, G12D, G12V, G12A, G12R, G12S, and G13D mutations), and more specifically KRas-G12D.

The present invention thereby provides the following technical solutions.

## Compounds of this invention

The terms "compounds of the invention" and "compounds of the present invention," as used throughout this application, unless otherwise defined, encompass the compounds defined in various embodiments and their preferred embodiments herein, or each of their specific embodiments, including their isomers, such as atropisomers, mixtures of enantiomers (particularly racemates), mixtures of diastereomers, geometric isomers, tautomers, solvates, metabolites, prodrugs, isotopic variants, and salts (e.g., pharmaceutically acceptable salts).

Therefore, the above-mentioned various types of isomers and derivatives of the compounds of the present invention are thus all covered within the scope of the present invention. Their respective meanings, preparation methods, and specific examples are as defined in the "Definitions" section above or are well-known in the art. However, preferably, they are the compounds of the present invention and/or pharmaceutically acceptable salts or solvates thereof.

The present invention also encompasses the N-oxides of the compounds of the present invention, as long as these compounds contain basic nitrogen atoms, such as those present in nitrogen-containing heterocycles, and are chemically and biologically feasible. Certain compounds of the present invention may exist in polymorphic or amorphous forms, and thus they also fall within the scope of the present invention.

The present invention provides the following compound embodiments:

**Embodiment 1:** A compound of formula (I), a stereoisomer, tautomer, stable isotopic variant, pharmaceutically acceptable salt or solvate thereof,

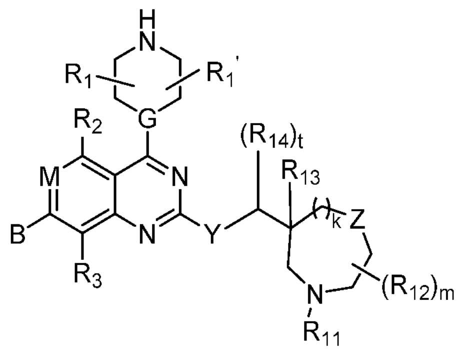

(I)

Wherein:

$R_1$ and $R_1$' together form a introcyclic bridging $-(CH_2)_t-$ or $-CH_2=CH_2-$;

$R_2$ and $R_3$ are each independently selected from H, halogen, $-C_{1-6}$ alkyl optionally substituted by halogen, $C_{2-6}$ alkynyl optionally substituted by halogen, and $-O-C_{1-6}$ alkyl optionally substituted by halogen;

G is selected from CH and N;

Y is selected from O, S and Se;

M is selected from N or C-R $_4$;

Z is selected from N, C, O, S and Se;

B is selected from

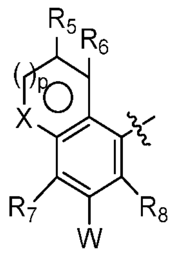 and 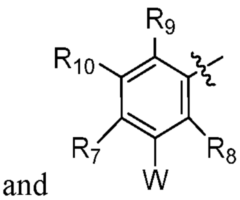 ;

X is selected from C and S, p is selected from 0 and 1, provided that X is S when p is 0, X is C when p is 1;

W is selected from H, halogen, $-C_{1-6}$ alkyl, OH, and $NH_2$;

$R_4$ is selected from H, halogen, CN, $-C_{1-6}$ alkyl, and $-(CH_2)_n-C_{3-6}$ cycloalkyl, wherein the $-C_{1-6}$ alkyl and-$C_{3-6}$ cycloalkyl are each independently and optionally substituted by halogen or CN;

$R_5$ is selected from H, halogen, and $NH_2$;

$R_6$ is selected from H, halogen, CN, $-C_{1-6}$ alkyl, $-O-C_{1-6}$ alkyl, $-S-C_{1-6}$ alkyl, $-Se-C_{1-6}$ alkyl, and $-C_{2-6}$ alkynyl, wherein the $-C_{1-6}$ alkyl and $-C_{2-6}$ alkynyl are each independently and optionally substituted by halogen;

$R_7$ and $R_8$ are each independently selected from H, halogen, $-NO_2$, CN, $-C_{1-6}$ alkyl, $-N(R^a)_2$, - $C(O)N(R^a)_2$ and $-C(O)OR^a$, wherein the $-C_{1-6}$ alkyl is optionally substituted by halogen or $-N(R^a)_2$;

$R_9$ is selected from $-Si(R^b)_3$, CN, $NO_2$, $-C_{1-6}$ alkyl, $-O-C_{1-6}$ alkyl, $-S-C_{1-6}$ alkyl, $-C_{2-6}$ alkenyl, - $C_{2-6}$ alkynyl, $-(CH_2)_n-C_{3-6}$ cycloalkyl, $-(CH_2)_n$-5-6 membered heteroaryl and $-(CH_2)_n$-phenyl, wherein the $-C_{1-6}$ alkyl, $-C_{2-6}$ alkenyl, $-C_{3-6}$ cycloalkyl, 5-6 membered heteroaryl and phenyl are each independently and optionally substituted by a group selected from halogen, $-Se-C_{1-6}$ alkyl, and $C_{1-6}$ alkyl optionally substituted by halogen;

$R^a$ is selected from H and -$C_{1-6}$ alkyl optionally substituted by halogen;

$R^b$ is selected from -$C_{1-6}$ alkyl and -$C_{2-6}$ alkenyl, each optionally substituted by halogen;

$R_{10}$ is selected from H, halogen, CN, -$C_{1-6}$ alkyl, and -$(CH_2)_n$-$C_{3-6}$ cycloalkyl, wherein the -$C_{1-6}$ alkyl and -$C_{3-6}$ cycloalkyl are each independently and optionally substituted by halogen or CN;

$R_{11}$ is selected from H, -$C_{1-6}$ alkyl, -$C_{2-6}$ alkenyl, -$C_{2-6}$ alkynyl, and-$(CH_2)_n$-$C_{3-6}$ cycloalkyl, wherein the $C_{1-6}$ alkyl, -$C_{2-6}$ alkenyl, -$C_{2-6}$ alkynyl or $C_{3-6}$ cycloalkyl is each independently and optionally substituted by halogen, CN,-O-$C_{1-6}$ alkyl or -O-CON$(R^a)_2$;

$R_{12}$ is selected from H, halogen, -CN, -OH, N$(R^a)_2$, -O-$C_{1-6}$ alkyl, -O-$C_{3-6}$ cycloalkyl, -$C_{1-6}$ alkyl, -$C_{2-6}$ alkenyl, -$C_{2-6}$ alkynyl and -$(CH_2)_n$-$C_{3-6}$ cycloalkyl, wherein the $C_{1-6}$ alkyl, -$C_{2-6}$ alkenyl, -$C_{2-6}$ alkynyl or $C_{3-6}$ cycloalkyl at each occurrence is each independently and optionally substituted by halogen, CN or -O$C_{1-6}$ alkyl,

or two $R_{12}$ attached to the same carbon atom form =C$(R^c)_2$, spiro $C_{3-6}$ cycloalkyl, or spiro 4-7 membered heterocycloalkyl, wherein $R^c$ is each independently selected from H, halogen, and -$C_{1-6}$ alkyl optionally substituted halogen, and the spiro $C_{3-6}$ cycloalkyl or spiro 4-7 membered heterocycloalkyl is optionally substituted by halogen and -$C_{1-6}$ alkyl optionally substituted by halogen,

or two $R_{12}$ attached to adjacent ring carbon atoms form $C_{3-4}$ cycloalkyl together with the carbon atoms to which they are attached,

or two $R_{12}$ attached to non-adjacent ring carbon atoms form a bridging methylene or ethylene;

$R_{13}$ is selected from H,-$C_{1-6}$ alkyl, and-$(CH_2)_n$-$C_{3-6}$ cycloalkyl, wherein the -$C_{1-6}$ alkyl and -$C_{3-6}$ cycloalkyl are each independently and optionally substituted by halogen or -O-$C_{1-6}$ alkyl;

or when $R_{12}$ and $R_{13}$ are attached to adjacent ring carbon atoms, they together with the carbon atoms to which they are attached form $C_{3-4}$ cycloalkyl;

$R_{14}$ is selected from H, -$C_{1-6}$ alkyl, and-$(CH_2)_n$-$C_{3-6}$ cycloalkyl, wherein the $C_{1-6}$ alkyl and $C_{3-6}$ cycloalkyl are each independently and optionally substituted by halogen or -O-$C_{1-6}$ alkyl, or two $R_{14}$ attached to the same carbon atom together with the carbon atom to which they are attached form $C_{3-4}$ cycloalkyl;

k is selected from 0 or 1;

m and n are each independently an integer selected from 0 to 2; and

t is an integer selected from 1 to 2.

**Embodiment 1.1:** the compound of formula (I) according to Embodiment 1, or a stereoisomer, tautomer, stable isotopic variant, pharmaceutically acceptable salt or solvate thereof, wherein W is selected from OH and $NH_2$; $R_2$ and $R_3$ are each independently selected from H, halogen, and -O-$C_{1-6}$ alkyl optionally halogen-substituted; $R_5$ is selected from H and halogen; $R_6$ is selected from H, halogen, -$C_{1-6}$ alkyl, -O-$C_{1-6}$ alkyl, -S-$C_{1-6}$ alkyl, -Se-$C_{1-6}$ alkyl, and -$C_{2-6}$ alkynyl, wherein the -$C_{1-6}$ alkyl and -$C_{2-6}$ alkynyl are each independently and optionally substituted by halogen.

**Embodiment 1.2:** the compound of formula (I) according to Embodiment 1, or a stereoisomer, tautomer, stable isotopic variant, pharmaceutically acceptable salt or solvate thereof, wherein $R_2$ and $R_3$ are independently selected from H, halogen, -$C_{1-6}$ alkyl optionally substituted by halogen, and -O-$C_{1-6}$ alkyl optionally substituted by halogen, and when two $R_{12}$ attached to the same carbon atom form =C$(R^c)_2$, spiro $C_{3-6}$ cycloalkyl, or spiro 4-7 membered heterocycloalkyl, wherein $R^c$ is each independently selected from H, halogen and -$C_{1-6}$ alkyl optionally substituted by halogen.

**Embodiment 1.3:** the compound of formula (I) according to Embodiment 1, a stereoisomer, tautomer, stable isotopic variant, pharmaceutically acceptable salt or solvate thereof, wherein W is selected from H, halogen, OH, and $NH_2$.

**Embodiment 1.4:**

**Embodiment 2.1:** the compound of formula (I) according to any one of Embodiments 1 to 1.3, a stereoisomer, tautomer, stable isotopic variant, pharmaceutically acceptable salt or solvate thereof, wherein B is

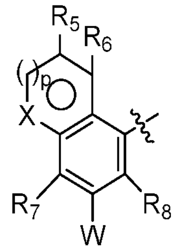

,

wherein p is 1 and X is C, i.e. the fused bicyclic moiety where X is located is

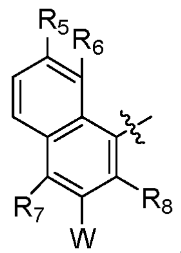

.

**Embodiment 2.1.1:** the compound of formula (I) according to Embodiments 2.1, a stereoisomer, tautomer, stable isotopic variant, pharmaceutically acceptable salt or solvate thereof, wherein $R_5$ is H; or $R_5$ is halogen selected from F, Cl, Br, I; preferably $R_5$ is halogen, and most preferably $R_5$ is F.

**Embodiment 2.1.2:** the compound of formula (I) according to Embodiment 2.1 or 2.1.1, a stereoisomer, tautomer, stable isotopic variant, pharmaceutically acceptable salt or solvate thereof, wherein $R_6$ is H; or $R_6$ is halogen selected from F, Cl, Br, I.

**Embodiment 2.1.3:** the compound of formula (I) according to Embodiment 2.1 or 2.1.1, a stereoisomer, tautomer, stable isotopic variant, pharmaceutically acceptable salt or solvate thereof, wherein $R_6$ is $-C_{1-6}$ alkyl, $-O-C_{1-6}$ alkyl, $-S-C_{1-6}$ alkyl, $-Se-C_{1-6}$ alkyl, optionally substituted by halogen, for example but not limited to $-CH_3$, $-CH_2CH_3$, $-CH_2CH_2CH_3$, $-CH(CH_3)(CH_3)$, $-CH_2CH_2CH_2CH_3$, - $CH_2CH(CH_3)CH_3$, $-C(CH_3)_3$, $-CH_2Cl$, $-CH_2F$, $-CHF_2$, $-CF_3$, $-CCl_3$, $-CH_2CH_2F$, $-CH_2CHF_2$, $-CH_2CF_3$, $-CH_2CH_2CH_2F$, $-CH_2CH_2CHF_2$, $-CH_2CH_2CF_3$, $-C_2F_5$, $-C_2Cl_5$, $-O-CH_3$, $-O-CH_2CH_3$, $-SCH_3$, $-S-CH_2CH_3$, $-SeCH_3$.

**Embodiment 2.1.4:** the compound of formula (I) according to Embodiment 2.1 or 2.1.1, a stereoisomer, tautomer, stable isotopic variant, pharmaceutically acceptable salt or solvate thereof, wherein $R_6$ is $-C_{2-6}$ alkynyl, optionally substituted by halogen, for example but not limited to

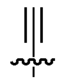

,

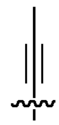

,

preferably

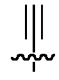

.

**Embodiment 2.1.5:** the compound of formula (I) according to any one of Embodiments 2.1 to 2.1.4, a stereoisomer, tautomer, stable isotopic variant, pharmaceutically acceptable salt or solvate thereof, wherein $R_7$ and $R_8$ are each H; or $R_7$ and $R_8$ are each halogen, preferably F.

**Embodiment 2.1.6:** the compound of formula (I) according to any one of Embodiments 2.1 to 2.1.4, a stereoisomer, tautomer, stable isotopic variant, pharmaceutically acceptable salt or solvate thereof, wherein one of $R_7$ and $R_8$ is H, and the other is selected from halogen, CN and $NO_2$, wherein the halogen is preferably F.

**Embodiment 2.1.7:** the compound of formula (I) according to any one of Embodiments 2.1 to 2.1.4, a stereoisomer, tautomer, stable isotopic variant, pharmaceutically acceptable salt or solvate thereof, wherein one of $R_7$ and $R_8$ is H, and the other is selected from $-C_{1-6}$ alkyl, $-N(R^a)_2$, $-C(O)N(R^a)_2$ and $-C(O)OR^a$, wherein the $-C_{1-6}$ alkyl is optionally substituted by halogen or $-N(R^a)_2$, wherein $R^a$ is optionally selected from H and $-C_{1-6}$ alkyl optionally substituted by halogen, and non-hydrogen $R_7$ or $R_8$ is, for example but not limited to $-CH_3$, $-CH_2CH_3$, $-CH_2CH_2CH_3$, $-CH(CH_3)(CH_3)$, - $CH_2CH_2CH_2CH_3$, $-CH_2CH(CH_3)CH_3$, $-C(CH_3)_3$, $-CH_2Cl$, $-CH_2F$, $-CHF_2$, $-CF_3$, $-CCl_3$, $-CH_2CH_2F$, - $CH_2CHF_2$, $-CH_2CF_3$, $-CH_2CH_2CH_2F$, $-CH_2CH_2CHF_2$, $-CH_2CH_2CF_3$, $-C_2F_5$, $-C_2Cl_5$, $-CH_2NH_2$, - $CH_2NHCH_3$, $-CH_2N(CH_3)_2$, $-NH_2$, $-NHCH_3$, $-N(CH_3)_2$, $-C(O)NH_2$, $-C(O)NHCH_3$, $-C(O)N(CH_3)_2$, - $C(O)OH$, $-C(O)OCH_3$.

**Embodiment 2.1.8:** the compound of formula (I) according to any one of Embodiments 2.1 to 2.1.4, a stereoisomer, tautomer, stable isotopic variant, pharmaceutically acceptable salt or solvate thereof, wherein one of $R_7$ and $R_8$ is selected from H, halogen, $-NO_2$, CN and $-C_{1-6}$ alkyl, and the other is selected from $-C_{1-6}$ alkyl, $-N(R^a)_2$, $-C(O)N(R^a)_2$ and $-C(O)OR^a$, wherein the $-C_{1-6}$ alkyl is optionally substituted by halogen or $-N(R^a)_2$, and $R^a$ is selected from H and $-C_{1-6}$ alkyl optionally substituted by halogen, as specifically exemplified in Embodiment 2.1.7.

**Embodiment 2.1.9:** the compound of formula (I) according to any one of Embodiments 2.1 to 2.1.8, a stereoisomer, tautomer, stable isotopic variant, pharmaceutically acceptable salt or solvate thereof, wherein W is -OH.

**Embodiment 2.1.10:** the compound of formula (I) according to any one of Embodiments 2.1 to 2.1.8, a stereoisomer, tautomer, stable isotopic variant, pharmaceutically acceptable salt or solvate thereof, wherein W is $-NH_2$.

**Embodiment 2.1.11:** the compound of formula (I) according to any one of Embodiment 2.1, a stereoisomer, tautomer, stable isotopic variant, pharmaceutically acceptable salt or solvate thereof, wherein B is

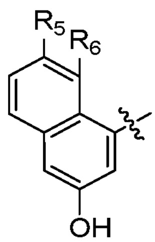

,

wherein $R_5$ is H or halogen, preferably halogen, $R_6$ is selected from halogen, $-C_{2-6}$ alkynyl and $-C_{1-6}$ alkyl, e.g.,

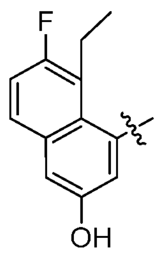 , 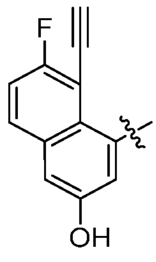 , 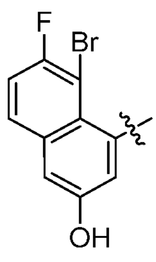 , 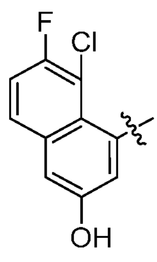 , 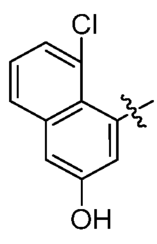 ,

and

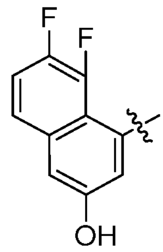

.

**Embodiment 2.2:** the compound of formula (I) according to any one of Embodiments 1 to 1.3, a stereoisomer, tautomer, stable isotopic variant, pharmaceutically acceptable salt or solvate thereof, wherein B is

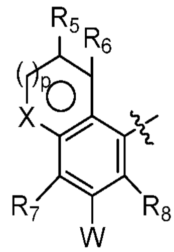

,

wherein p is 0 and X is S, i.e. the fused bicyclic moiety where X is located is

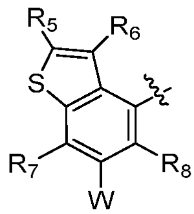

.

**Embodiment 2.2.1:** the compound of formula (I) according to Embodiment 2.2, a stereoisomer, tautomer, stable isotopic variant, pharmaceutically acceptable salt or solvate thereof, wherein $R_5$ is H; or $R_5$ is halogen selected from F, Cl, Br, I; preferably $R_5$ is halogen, and most preferably, $R_5$ is F.

**Embodiment 2.2.1.1:** the compound of formula (I) according to Embodiment 2.2, a stereoisomer, tautomer, stable isotopic variant, pharmaceutically acceptable salt or solvate thereof, wherein $R_5$ is $NH_2$.

**Embodiment 2.2.2:** the compound of formula (I) according to any one of Embodiments 2.2 to 2.2.1.1, a stereoisomer, tautomer, stable isotopic variant, pharmaceutically acceptable salt or solvate thereof, wherein $R_6$ is H; or $R_6$ is halogen selected from F, Cl, Br, I.

**Embodiment 2.2.2.1:** the compound of formula (I) according to any one of Embodiments 2.2 to 2.2.1.1, a stereoisomer, tautomer, stable isotopic variant, pharmaceutically acceptable salt or solvate thereof, wherein $R_6$ is CN.

**Embodiment 2.2.3:** the compound of formula (I) according to any one of Embodiments 2.2 to 2.2.1.1, a stereoisomer, tautomer, stable isotopic variant, pharmaceutically acceptable salt or solvate thereof, wherein $R_6$ is $-C_{1-6}$ alkyl, $-O-C_{1-6}$ alkyl, $-S-C_{1-6}$ alkyl, $-Se-C_{1-6}$ alkyl, optionally substituted by halogen, for example but not limited to $-CH_3$, $-CH_2CH_3$, $-CH_2CH_2CH_3$, $-CH(CH_3)(CH_3)$, $-CH_2CH_2CH_2CH_3$, $-CH_2CH(CH_3)CH_3$, $-C(CH_3)_3$, $-CH_2Cl$, $-CH_2F$, $-CHF_2$, $-CF_3$, $-CCl_3$, $-CH_2CH_2F$, $-CH_2CHF_2$, $-CH_2CF_3$, $-CH_2CH_2CH_2F$, $-CH_2CH_2CHF_2$, $-CH_2CH_2CF_3$, $-C_2F_5$, $-C_2Cl_5$, $-O-CH_3$, $-O-CH_2CH_3$, $-SCH_3$, $-S-CH_2CH_3$, $-SeCH_3$.

**Embodiment 2.2.4:** the compound of formula (I) according to any one of Embodiments 2.2 to 2.2.1.1, a stereoisomer, tautomer, stable isotopic variant, pharmaceutically acceptable salt or solvate thereof, wherein $R_6$ is $-C_{2-6}$ alkynyl, optionally substituted by halogen, for example but not limited to

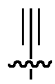, 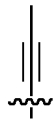,

preferably

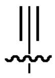.

**Embodiment 2.2.5:** the compound of formula (I) according to any one of Embodiments 2.2 to 2.2.4, a stereoisomer, tautomer, stable isotopic variant, pharmaceutically acceptable salt or solvate thereof, wherein $R_7$ and $R_8$ are each H; or $R_7$ and $R_8$ are each halogen, preferably F.

**Embodiment 2.2.6:** the compound of formula (I) according to any one of Embodiments 2.2 to 2.2.4, a stereoisomer, tautomer, stable isotopic variant, pharmaceutically acceptable salt or solvate thereof, wherein one of $R_7$ and $R_8$ is H, the other is selected from halogen, CN and $NO_2$, wherein the halogen is preferably F; for example, $R_7$ is H and $R_8$ is halogen, preferably F, or $R_8$ is H and $R_7$ is halogen, preferably F.

**Embodiment 2.2.7:** the compound of formula (I) according to any one of Embodiments 2.2 to 2.2.4, a stereoisomer, tautomer, stable isotopic variant, pharmaceutically acceptable salt or solvate thereof, wherein one of $R_7$ and $R_8$ is H, the other is selected from -$C_{1-6}$ alkyl, -$N(R^a)_2$, -$C(O)N(R^a)_2$ and-$C(O)OR^a$, wherein the -$C_{1-6}$ alkyl is optionally substituted by halogen or -$N(R^a)_2$, and $R^a$ is optionally selected from H and -$C_{1-6}$ alkyl optionally substituted by halogen, and non-hydrogen $R_7$ or $R_8$ is, for example but not limited to -$CH_3$, -$CH_2CH_3$, -$CH_2CH_2CH_3$, -$CH(CH_3)(CH_3)$, - $CH_2CH_2CH_2CH_3$, -$CH_2CH(CH_3)CH_3$, -$C(CH_3)_3$, -$CH_2Cl$, -$CH_2F$, -$CHF_2$, -$CF_3$, -$CCl_3$, -$CH_2CH_2F$, - $CH_2CHF_2$, -$CH_2CF_3$, -$CH_2CH_2CH_2F$, -$CH_2CH_2CHF_2$, -$CH_2CH_2CF_3$, -$C_2F_5$, -$C_2Cl_5$, -$CH_2NH_2$, - $CH_2NHCH_3$, -$CH_2N(CH_3)_2$, -$NH_2$, -$NHCH_3$, -$N(CH_3)_2$, -$C(O)NH_2$, -$C(O)NHCH_3$, -$C(O)N(CH_3)_2$, -$C(O)OH$, -$C(O)OCH_3$.

**Embodiment 2.2.8:** the compound of formula (I) according to any one of Embodiments 2.2 to 2.2.4, a stereoisomer, tautomer, stable isotopic variant, pharmaceutically acceptable salt or solvate thereof, wherein one of $R_7$ and $R_8$ is selected from H, halogen, -$NO_2$, CN and -$C_{1-6}$ alkyl, the other is selected from -$C_{1-6}$ alkyl, -$N(R^a)_2$, -$C(O)N(R^a)_2$ and-$C(O)OR^a$, wherein the -$C_{1-6}$ alkyl is optionally substituted by halogen or -$N(R^a)_2$, and $R^a$ is selected from H and -$C_{1-6}$ alkyl optionally substituted by halogen, as specifically exemplified in Embodiment 2.2.7.

**Embodiment 2.2.9:** the compound of formula (I) according to any one of Embodiments 2.2 to 2.2.8, a stereoisomer, tautomer, stable isotopic variant, pharmaceutically acceptable salt or solvate thereof, wherein W is -OH.

**Embodiment 2.2.10:** the compound of formula (I) according to any one of Embodiments 2.2 to 2.2.8, a stereoisomer, tautomer, stable isotopic variant, pharmaceutically acceptable salt or solvate thereof, wherein W is -$NH_2$.

**Embodiment 2.2.11:** the compound of formula (I) according to Embodiment 2.2, a stereoisomer, tautomer, stable isotopic variant, pharmaceutically acceptable salt or solvate thereof, wherein B is

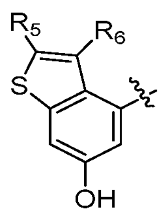,

wherein $R_5$ is H or halogen, $R_6$ is selected from halogen, -$C_{2-6}$ alkynyl and -$C_{1-6}$ alkyl, e.g.

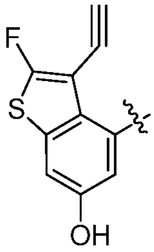 , 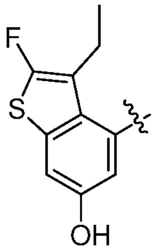 , 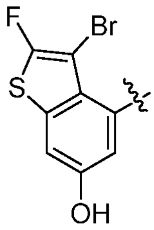 , 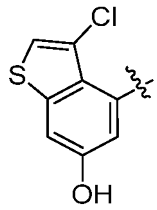 , 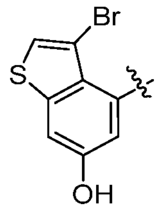 , and 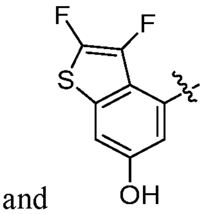 .

**Embodiment 2.2.12:** the compound of formula (I) according to any one of Embodiments 2.2 to 2.2.8, a stereoisomer, tautomer, stable isotopic variant, pharmaceutically acceptable salt or solvate thereof, wherein W is -H.

**Embodiment 2.2.13:** the compound of formula (I) according to any one of Embodiments 2.2 to 2.2.8, a stereoisomer, tautomer, stable isotopic variant, pharmaceutically acceptable salt or solvate thereof, wherein W is halogen and preferably F; or W is -$C_{1-6}$ alkyl, e.g., -$CH_3$ or -$CH_2CH_3$.

**Embodiment 2.2.14:** the compound of formula (I) according to Embodiment 2.2, a stereoisomer, tautomer, stable isotopic variant, pharmaceutically acceptable salt or solvate thereof, wherein B is

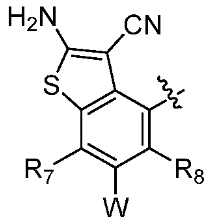 ,

wherein W is selected H, -$C_{1-6}$ alkyl and halogen, $R_7$ and $R_8$ are each H, or each halogen, or one is H and the other is halogen or halogen-substituted $C_{1-6}$ alkyl, or one is halogen and the other is halogen-substituted C $_{1-6}$ alkyl, wherein the halogen is preferably F; preferably, W is selected from H, $R_7$ and $R_8$ are each H, or one is H and the other is halogen, wherein the halogen is preferably F.

**Embodiment 2.2.15:** the compound of formula (I) according to Embodiment 2.2.14,a stereoisomer, tautomer, stable isotopic variant, pharmaceutically acceptable salt or solvate thereof, wherein examples of B include, but are not limited to:

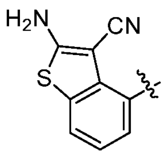 , 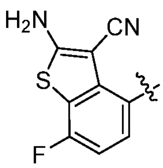 , 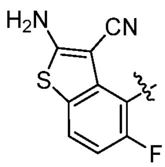 , 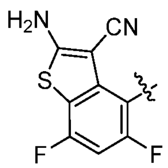 , 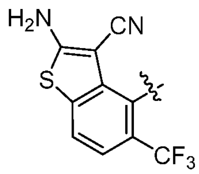 , 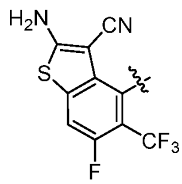 ,

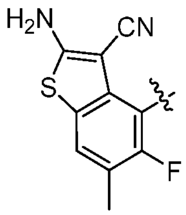 .

**Embodiment 2.2.16:** the compound of formula (I) according to Embodiments 2.2 to 2.2.15, a stereoisomer, tautomer, stable isotopic variant, pharmaceutically acceptable salt or solvate thereof, wherein the position marked with an asterisk in

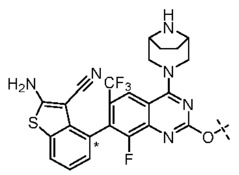

exhibits axial chirality, including

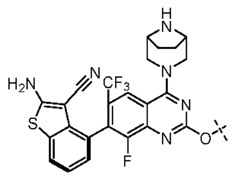 or 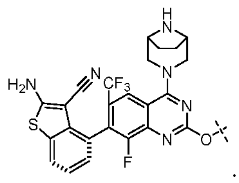 .

**Embodiment 2.3:** the compound of formula (I) according to any one of Embodiments 1 to 1.3, a stereoisomer, tautomer, stable isotopic variant, pharmaceutically acceptable salt or solvate thereof, wherein B is

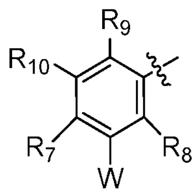 .

**Embodiment 2.3.1:** the compound of formula (I) according to Embodiment 2.3, a stereoisomer, tautomer, stable isotopic variant, pharmaceutically acceptable salt or solvate thereof, wherein $R_7$ and $R_8$ are each H; or $R_7$ and $R_8$ are each halogen, preferably F.

**Embodiment 2.3.2:** the compound of formula (I) according to Embodiment 2.3, a stereoisomer, tautomer, stable isotopic variant, pharmaceutically acceptable salt or solvate thereof, wherein one of $R_7$ and $R_8$ is H, the other is selected from halogen, CN and $NO_2$, wherein halogen is preferably F, e.g., $R_7$ is H, and $R_8$ is selected from halogen, CN and $NO_2$, wherein the halogen is preferably F.

**Embodiment 2.3.3:** the compound of formula (I) according to Embodiment 2.3, a stereoisomer, tautomer, stable isotopic variant, pharmaceutically acceptable salt or solvate thereof, wherein one of $R_7$ and $R_8$ is H, the other is selected from $-C_{1-6}$ alkyl, $-N(R^a)_2$, $-C(O)N(^a)_2$, and $-C(O)OR^a$, wherein the $-C_{1-6}$ alkyl is optionally substituted by halogen or $-N(R^a)_2$, and $R^a$ is selected from H and $-C_{1-6}$ alkyl optionally substituted by halogen, e.g., $R_7$ is H and $R_8$ is selected from groups above; the non-hydrogen $R_7$ or $R_8$ is, for example but not limited to, $-CH_3$, $-CH_2CH_3$, $-CH_2CH_2CH_3$, $-CH(CH_3)(CH_3)$, $-CH_2CH_2CH_2CH_3$, $-CH_2CH(CH_3)CH_3$, $-C(CH_3)_3$, $-CH_2Cl$, $-CH_2F$, $-CHF_2$, $-CF_3$, $-CCl_3$, $-CH_2CH_2F$, - $CH_2CHF_2$, $-CH_2CF_3$, $-CH_2CH_2CH_2F$, $-CH_2CH_2CHF_2$, $-CH_2CH_2CF_3$, $-C_2F_5$, $-C_2Cl_5$, $-CH_2NH_2$, - $CH_2NHCH_3$, $-CH_2N(CH_3)_2$, $-NH_2$, $-NHCH_3$, $-N(CH_3)_2$, $-C(O)NH_2$, $-C(O)NHCH_3$, $-C(O)N(CH_3)_2$, - $C(O)OH$, $-C(O)OCH_3$.

**Embodiment 2.3.4:** the compound of formula (I) according to Embodiment 2.3, a stereoisomer, tautomer, stable isotopic variant, pharmaceutically acceptable salt or solvate thereof, wherein one of $R_7$ and $R_8$ is selected from H, halogen, $-NO_2$, CN and $-C_{1-6}$ alkyl, and the other is selected from $-C_{1-6}$ alkyl, $-N(R^a)_2$, $-C(O)N(R^a)_2$ and $-C(O)OR^a$, wherein the $-C_{1-6}$ alkyl is optionally substituted by halogen or $-N(R^a)_2$, and $R^a$ is selected from H and $-C_{1-6}$ alkyl optionally substituted by halogen, wherein the non-hydrogen groups are specifically exemplified as in Embodiment 2.3.3.

**Embodiment 2.3.4.1:** the compound of formula (I) according to Embodiment 2.3.4, a stereoisomer, tautomer, stable isotopic variant, pharmaceutically acceptable salt or solvate thereof, wherein $R_7$ is selected from halogen, preferably F; $R_8$ is CN.

**Embodiment 2.3.5:** the compound of formula (I) according to any one of Embodiments 2.3 to 2.3.4, a stereoisomer, tautomer, stable isotopic variant, pharmaceutically acceptable salt or solvate thereof, wherein

$R_{10}$ is H.

**Embodiment 2.3.6:** the compound of formula (I) according to any one of Embodiments 2.3 to 2.3.4, a stereoisomer, tautomer, stable isotopic variant, pharmaceutically acceptable salt or solvate thereof, wherein $R_{10}$ is halogen or CN, e.g., F, Cl, Br, I, CN, preferably F or Cl.

**Embodiment 2.3.7:** the compound of formula (I) according to any one of Embodiments 2.3 to 2.3.4, a stereoisomer, tautomer, stable isotopic variant, pharmaceutically acceptable salt or solvate thereof, wherein $R_{10}$ is $-C_{1-6}$ alkyl, preferably $-C_{1-3}$ alkyl, optionally substituted by halogen or CN, for example but not limited to $-CH_3$, $-CH_2CH_3$, $-CH_2CH_2CH_3$, $-CH(CH_3)(CH_3)$, $-CH_2CH_2CH_2CH_3$, - $CH_2CH(CH_3)CH_3$, $-C(CH_3)_3$, $-CH_2Cl$, $-CH_2F$, $-CHF_2$, $-CF_3$, $-CCl_3$, $-CH_2CH_2F$, $-CH_2CHF_2$, $-CH_2CF_3$, $-CH_2CH_2CH_2F$, $-CH_2CH_2CHF_2$, $-CH_2CH_2CF_3$, $-C_2F_5$, $-C_2Cl_5$, $-CH_2CN$, $-CH_2CH_2CN$, - $CH_2CH_2CH_2CN$.

**Embodiment 2.3.8:** the compound of formula (I) according to any one of Embodiments 2.3 to 2.3.4, a stereoisomer, tautomer, stable isotopic variant, pharmaceutically acceptable salt or solvate thereof, wherein $R_{10}$ is $-(CH_2)_n-C_{3-6}$ cycloalkyl, preferably $-C_{3-6}$ cycloalkyl, optionally substituted by halogen or CN; for example but not limited to

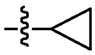, 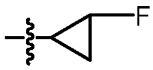, 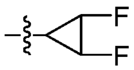, 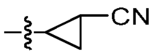,

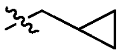, 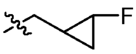, 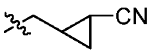, 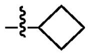, 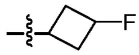, 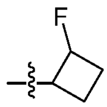, 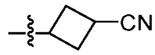, 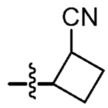,

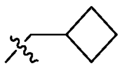, 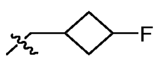, 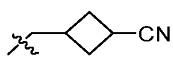, 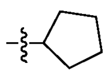, 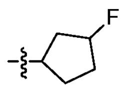, 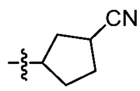, 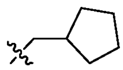,

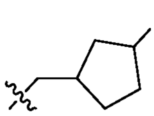, 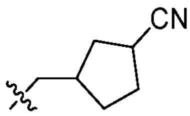, 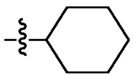, 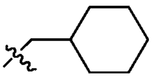.

**Embodiment 2.3.9:** the compound of formula (I) according to Embodiment 2.3, a stereoisomer, tautomer, stable isotopic variant, pharmaceutically acceptable salt or solvate thereof, wherein $R_7$ is H, $R_8$ is selected from H, CN, halogen, $NO_2$, $-C_{1-6}$ alkyl, $-N(R^a)_2$, $-C(O)N(R^a)_2$ and $-C(O)OR^a$, wherein the $-C_{1-6}$ alkyl is optionally substituted by halogen or $-N(R^a)_2$, $R^a$ is selected from H and $-C_{1-6}$ alkyl optionally substituted by halogen, and $R_{10}$ is selected from halogen and $-C_{1-6}$ alkyl; each as specifically exemplified in Embodiment 2.3.3, 2.3.6 and 2.3.7.

**Embodiment 2.3.10:** the compound of formula (I) according to any one of Embodiments 2.3 to 2.3.9, a stereoisomer, tautomer, stable isotopic variant, pharmaceutically acceptable salt or solvate thereof, wherein $R_9$ is $-Si(R^b)_3$, wherein $R^b$ is selected from $-C_{1-6}$ alkyl and $-C_{2-6}$ alkenyl, each optionally substituted by halogen; for example but not limited to $-Si(CH_3)_3$, $-Si(CH_3)_2(CH_2CH_3)$, - $Si(CH_3)_2(CH=CH_2)$.

**Embodiment 2.3.11:** the compound of formula (I) according to any one of Embodiments 2.3 to 2.3.9, a stereoisomer, tautomer, stable isotopic variant, pharmaceutically acceptable salt or solvate thereof, wherein $R_9$ is $-C_{1-6}$ alkyl, optional substituted by halogen, $-Se-C_{1-6}$ and $C_{1-6}$ alkyl optionally substituted by halogen; for example but not limited to $-CH_3$, $-CH_2CH_3$, $-CH_2CH_2CH_3$, - $CH(CH_3)(CH_3)$, $-CH_2CH_2CH_2CH_3$, $-CH_2CH(CH_3)CH_3$, $-C(CH_3)_3$, $-CH_2Cl$, $-CH_2F$, $-CHF_2$, $-CF_3$, - $CCl_3$, $-CH_2CH_2F$, $-CH_2CHF_2$, $-CH_2CF_3$, $-CH_2CH_2CH_2F$, $-CH_2CH_2CHF_2$, $-CH_2CH_2CF_3$, $-C(CH_3)_2CF_3$, $-CH_2(Se-CH_3)$, $-CH(CH_3)(Se-CH_3)$, $-C(CH_3)_2(Se-CH_3)$, $-C_2F_5$, $-C_2Cl_5$.

**Embodiment 2.3.12:** the compound of formula (I) according to any one of Embodiments 2.3 to 2.3.9, a stereoisomer, tautomer, stable isotopic variant, pharmaceutically acceptable salt or solvate thereof, wherein

$R_9$ is $-C_{2-6}$ alkenyl, optionally substituted by halogen, $-Se-C_{1-6}$ alkyl and $C_{1-6}$ alkyl optionally substituted by halogen; for example but not limited to $-CH=CH_2$, $-CH_2CH=CH_2$, $-CH=CF_2$, $-CF=CF_2$, $-C(CH_3)=CH_2$, $-C(CF_3)=CH_2$, $-C(CH_3)=CF_2$, $-CH=CHCF_3$, $-C(CH_3)=CHCF_3$, $-CH_2CH=CF_2$, $-CH_2CF=CF_2$, $-CH_2C(CF_3)=CH_2$, $-CH_2C(CH_3)=CF_2$, $-CH_2CH=CHCF_3$, $-CH_2C(CH_3)=CHCF_3$, $-CH=CH(Se-CH_3)$, $-C(Se-CH_3)=CH_2$, $-CF=CH(Se-CH_3)$.

**Embodiment 2.3.13:** the compound of formula (I) according to any one of Embodiments 2.3 to 2.3.9, a stereoisomer, tautomer, stable isotopic variant, pharmaceutically acceptable salt or solvate thereof, wherein $R_9$ is $-C_{2-6}$ alkynyl, optionally substituted by halogen, $-Se-C_{1-6}$ alkyl and $C_{1-6}$ alkyl optionally substituted by halogen; for example but not limited to $-C\equiv CH$, $-CH_2C\equiv CH$, $-C\equiv CF$, $-C\equiv CF$, $-C\equiv C(CH_3)$, $-C\equiv C(CF_3)$, $-CH_2C\equiv CF$, $-CH_2C\equiv C(CF_3)$, $-C\equiv C-Se-CH_3$, $-C\equiv C-Se-CF_3$.

**Embodiment 2.3.14:** the compound of formula (I) according to any one of Embodiment 2.3 to 2.3.9, a stereoisomer, tautomer, stable isotopic variant, pharmaceutically acceptable salt or solvate thereof, wherein $R_9$ is $-O-C_{1-6}$ alkyl or $-S-C_{1-6}$ alkyl, optionally substituted by halogen, $-Se-C_{1-6}$ alkyl and $C_{1-6}$ alkyl optionally substituted by halogen; for example but not limited to $-O-CH_3$, $-O-CH_2CH_3$, $-O-CH_2CH_2CH_3$, $-O-CH(CH_3)(CH_3)$, $-O-CH_2CH_2CH_2CH_3$, $-O-CH_2CH(CH_3)CH_3$, $-O-C(CH_3)_3$, $-O-CH_2Cl$, $-O-CH_2F$, $-O-CHF_2$, $-O-CF_3$, $-O-CCl_3$, $-O-CH_2CH_2F$, $-O-CH_2CHF_2$, $-O-CH_2CF_3$, $-O-CH_2CH_2CH_2F$, $-O-CH_2CH_2CHF_2$, $-O-CH_2CH_2CF_3$, $-O-C(CH_3)_2CF_3$, $-O-CH_2(Se-CH_3)$, $-O-CH(CH_3)(Se-CH_3)$, $-O-C(CH_3)_2(Se-CH_3)$, $-O-C_2F_5$, $-O-C_2Cl_5$, $-S-CH_3$, $-S-CH_2CH_3$, $-S-CH_2CH_2CH_3$, $-S-CH(CH_3)(CH_3)$, $-S-CH_2CH_2CH_2CH_3$, $-S-CH_2CH(CH_3)CH_3$, $-S-C(CH_3)_3$, $-S-CH_2Cl$, $-S-CH_2F$, $-S-CHF_2$, $-S-CF_3$, $-S-CCl_3$, $-S-CH_2CH_2F$, $-S-CH_2CHF_2$, $-S-CH_2CF_3$, $-S-CH_2CH_2CH_2F$, $-S-CH_2CH_2CHF_2$, $-S-CH_2CH_2CF_3$, $-S-C(CH_3)_2CF_3$, $-S-CH_2(Se-CH_3)$, $-S-CH(CH_3)(Se-CH_3)$, $-S-C(CH_3)_2(Se-CH_3)$, $-S-C_2F_5$, $-S-C_2Cl_5$.

**Embodiment 2.3.15:** the compound of formula (I) according to any one of Embodiments 2.3 to 2.3.9, a stereoisomer, tautomer, stable isotopic variant, pharmaceutically acceptable salt or solvate thereof, wherein $R_9$ is $-(CH_2)_n-C_{3-6}$ cycloalkyl, preferably $-C_{3-6}$ cycloalkyl, most preferably cyclopropyl, optionally substituted by halogen, $-Se-C_{1-6}$ alkyl and $C_{1-6}$ alkyl optionally substituted by halogen; for example but not limited to

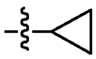, 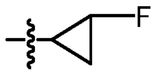, 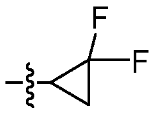, 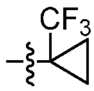, 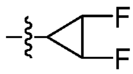,

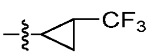, 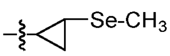, 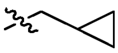, 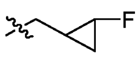, 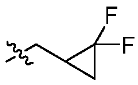, 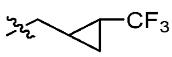,

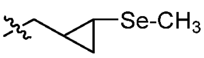, 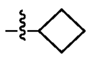, 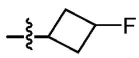, 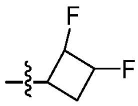, 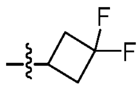, 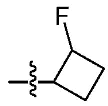, 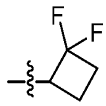,

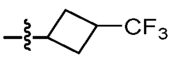, 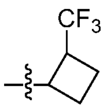, 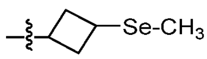, 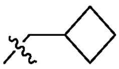, 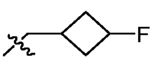, 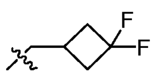,

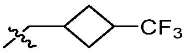, 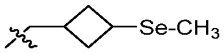, 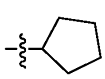, 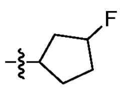, 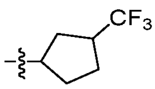, 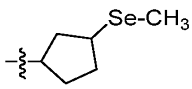,

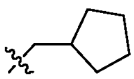, 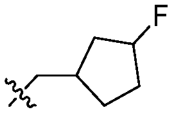, 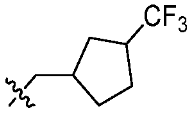, 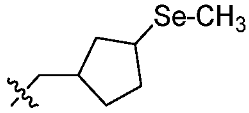, 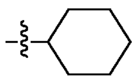, 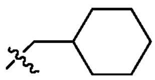.

**Embodiment 2.3.16:** the compound of formula (I) according to any one of Embodiments 2.3 to 2.3.9, a stereoisomer, tautomer, stable isotopic variant, pharmaceutically acceptable salt or solvate thereof, wherein $R_9$ is a $-(CH_2)_n$-5-6 membered heteroaryl group, preferably a 5-6 membered heteroaryl group, optionally substituted by halogen, -Se-$C_{1-6}$ alkyl and $C_{1-6}$ alkyl optionally substituted by halogen; for example, a 5-6 membered heteroaryl group containing 1 to 3 heteroatoms independently selected from N, O and S, a 5-6 membered heteroaryl group containing 1 to 3 N atoms, a 5-6 membered heteroaryl group containing 1 to 3 heteroatoms selected from N and O, a 5-6 membered heteroaryl group containing 1 to 3 heteroatoms selected from N and S; specific examples include, but are not limited to:

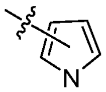, 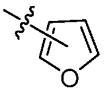, 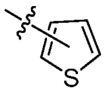, 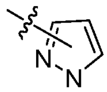, 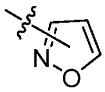, 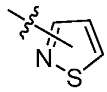, 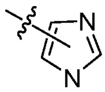, 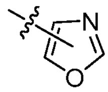, 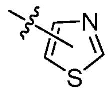,

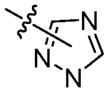, 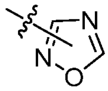, 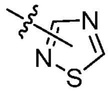, 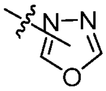, 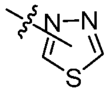, 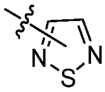, 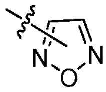, 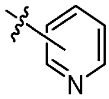, 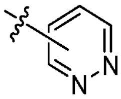,

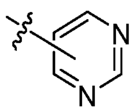, 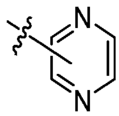,

each is optionally substituted by halogen, -Se-$C_{1-6}$ alkyl and $C_{1-6}$ alkyl optionally substituted by halogen, for example, substituted by F, Cl, Br, I, $-CH_3$, $-CH_2CH_3$, - $CH_2CH_2CH_3$, $-CH(CH_3)(CH_3)$, $-CH_2CH_2CH_2CH_3$, $-CH_2CH(CH_3)CH_3$, $-C(CH_3)_3$, $-CH_2Cl$, $-CH_2F$, $-CHF_2$, $-CF_3$, $-CCl_3$, $-CH_2CH_2F$, $-CH_2CHF_2$, $-CH_2CF_3$, $-CH_2CH_2CH_2F$, $-CH_2CH_2CHF_2$, - $CH_2CH_2CF_3$, $-C(CH_3)_2CF_3$, $-Se-CH_3$, $-Se-CH_2-CH_3$, $-C_2F_5$ and/or $-C_2Cl_5$.

**Embodiment 2.3.17:** the compound of formula (I) according to any one of Embodiments 2.3 to 2.3.9, a stereoisomer, tautomer, stable isotopic variant, pharmaceutically acceptable salt or solvate thereof, wherein $R_9$ is phenyl, optionally substituted by halogen, -Se-$C_{1-6}$ alkyl and $C_{1-6}$ alkyl optionally substituted by halogen; for example, substituted by F, Cl, Br, I, $-CH_3$, $-CH_2CH_3$, - $CH_2CH_2CH_3$, $-CH(CH_3)(CH_3)$, $-CH_2CH_2CH_2CH_3$, $-CH_2CH(CH_3)CH_3$, $-C(CH_3)_3$, $-CH_2Cl$, $-CH_2F$, - $CHF_2$, $-CF_3$, $-CCl_3$, $-CH_2CH_2F$, $-CH_2CHF_2$, $-CH_2CF_3$, $-CH_2CH_2CH_2F$, $-CH_2CH_2CHF_2$, $-CH_2CH_2CF_3$, $-C(CH_3)_2CF_3$, $-Se-CH_3$, $-Se-CH_2-CH_3$, $-C_2F_5$ and/or $-C_2Cl_5$.

**Embodiment 2.3.18:** the compound of formula (I) according to any one of Embodiments 2.3 to 2.3.9, a stereoisomer, tautomer, stable isotopic variant, pharmaceutically acceptable salt or solvate thereof, wherein $R_9$ is CN or $NO_2$.

**Embodiment 2.3.19:** the compound of formula (I) according to any one of Embodiments 2.3 to 2.3.9, a stereoisomer, tautomer, stable isotopic variant, pharmaceutically acceptable salt or solvate thereof, wherein $R_9$ is selected from $-Si(R^b)_3$, $-C_{1-6}$ alkyl and $-(CH_2)_n-C_{3-6}$ cycloalkyl, wherein the - $C_{1-6}$ alkyl and $-C_{3-6}$ cycloalkyl are each independently and optionally substituted by halogen, -Se-$C_{1-6}$ alkyl and $C_{1-6}$ alkyl optionally substituted by halogen, and $R_b$ is selected from $-C_{1-6}$ alkyl and $-C_{2-6}$ alkenyl, each optionally substituted by halogen.

**Embodiment 2.3.20:** the compound of formula (I) according to Embodiment 2.3, a stereoisomer, tautomer, stable isotopic variant, pharmaceutically acceptable salt or solvate thereof, wherein $R_7$ is H, $R_8$ is selected from H, CN, halogen, $NO_2$, $-C_{1-6}$ alkyl, $-N(R^a)_2$, $-C(O)N(R^a)_2$ and $-C(O)OR^a$, wherein the $-C_{1-6}$ alkyl is optionally substituted by halogen or $-N(R^a)_2$, wherein $R^a$ is selected from H and $-C_{1-6}$ alkyl optionally substituted by halogen, $R_9$ is selected from $-Si(R^b)_3$, $NO_2$, CN, $-C_{1-6}$ alkyl, $-(CH_2)_n-C_{3-6}$ cycloalkyl, wherein the $-C_{1-6}$ alkyl and $-C_{3-6}$ cycloalkyl are each independently and optionally substituted by groups selected from halogen, -Se-$C_{1-6}$ alkyl and $C_{1-6}$ alkyl optionally substituted by halogen, and $R_{10}$ is selected from halogen and $-C_{1-6}$ alkyl.

**Embodiment 2.3.20.1:** the compound of formula (I) according to Embodiment 2.3, a stereoisomer, tautomer, stable isotopic variant, pharmaceutically acceptable salt or solvate thereof, wherein $R_7$ is halogen, preferably F, $R_8$ is selected from CN, $R_9$ is selected from halogen, $-C_{3-6}$ cycloalkyl optionally substituted by halogen, and $-C_{1-6}$ alkyl optionally substituted by halogen, and $R_{10}$ is selected from halogen and $-C_{1-6}$ alkyl optionally substituted by

halogen.

**Embodiment 2.3.21:** the compound of formula (I) according to any one of Embodiments 2.3 to 2.3.20.1, a stereoisomer, tautomer, stable isotopic variant, pharmaceutically acceptable salt or solvate thereof, wherein W is -OH.

**Embodiment 2.3.22:** the compound of formula (I) according to any one of Embodiments 2.3 to 2.3.20.1, a stereoisomer, tautomer, stable isotopic variant, pharmaceutically acceptable salt or solvate thereof, wherein W is $-NH_2$.

**Embodiment 2.3.23:** the compound of formula (I) according to Embodiment 2.3, a stereoisomer, tautomer, stable isotopic variant, pharmaceutically acceptable salt or solvate thereof, wherein B is

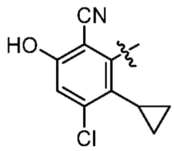 , 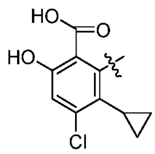 , 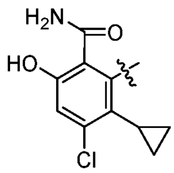 , 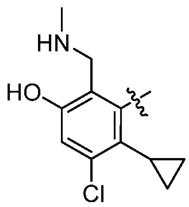 , 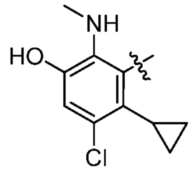 , 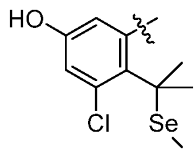 ,

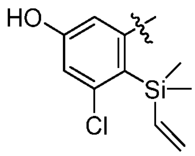 , 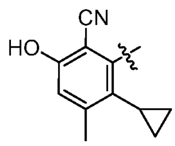 , 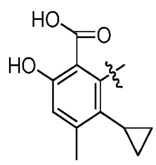 , 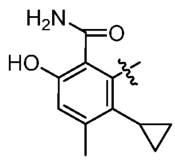 , 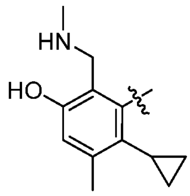 , 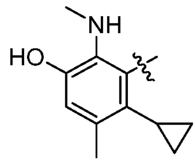 ,

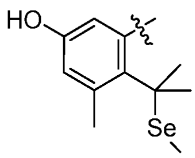 , 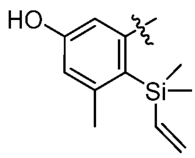 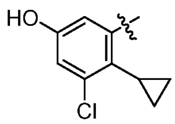 , 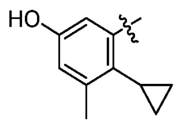 , 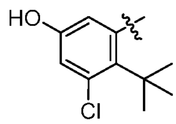 , 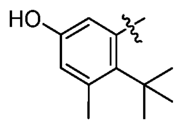 ,

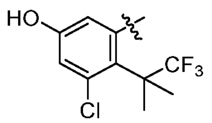 , 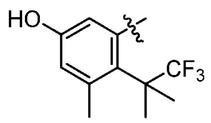 , 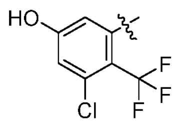 , 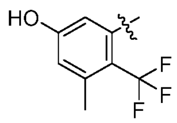 , 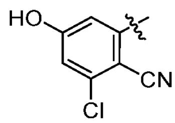 ,

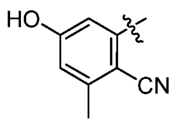 , 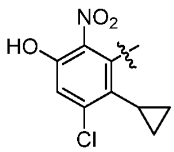 , 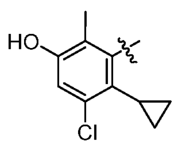 , 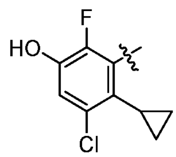 , 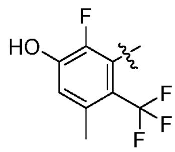 , 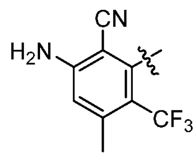 ,

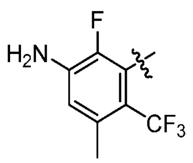 , 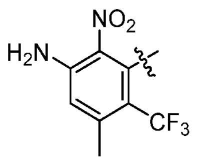 , 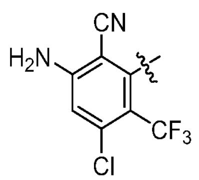 , 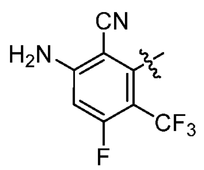 , 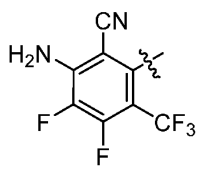 ,

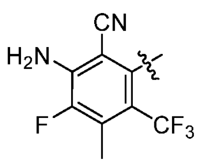, 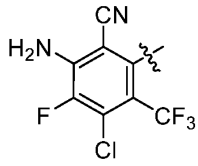, 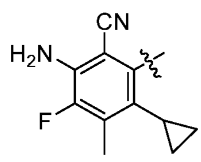, 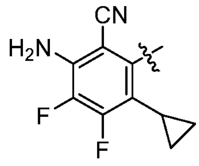, 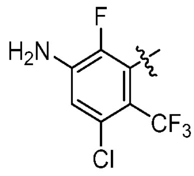,

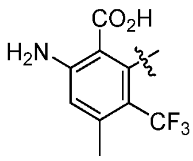, 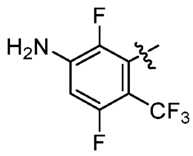, 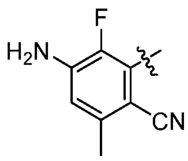, 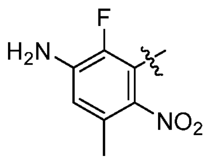, 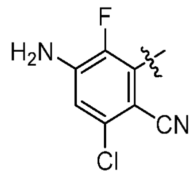,

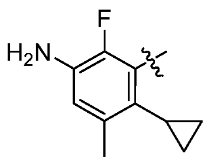, 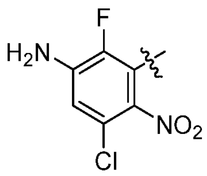, 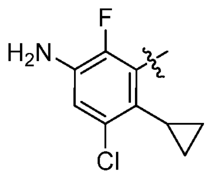, 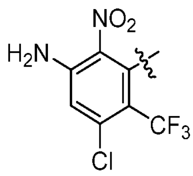, 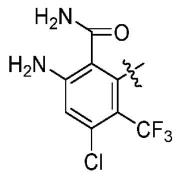,

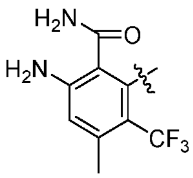.

**Embodiment 3.1:** the compound of formula (I) according to any one of Embodiments 1 to 2.3.23, a stereoisomer, tautomer, stable isotopic variant, pharmaceutically acceptable salt or solvate thereof, wherein G is N.

**Embodiment 3.2:** the compound of formula (I) according to Embodiment 1 to 2.3.23, a stereoisomer, tautomer, stable isotopic variant, pharmaceutically acceptable salt or solvate thereof, wherein G is C.

**Embodiment 3.3:** the compound of formula (I) according to any one of Embodiments 1 to 2.3.23, a stereoisomer, tautomer, stable isotopic variant, pharmaceutically acceptable salt or solvate thereof, wherein $R_1$ and $R_1$' together form $-CH_2-$, $-CH_2CH_2-$ or $-CH_2=CH_2-$.

**Embodiment 3.4:** the compound of formula (I) according to any one of Embodiments 1 to 2.3.23, a stereoisomer, tautomer, stable isotopic variant, pharmaceutically acceptable salt or solvate thereof, wherein examples of the heterocycle carrying $R_1$ and $R_1$' and containing G are

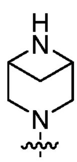, 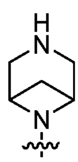, 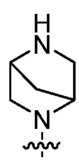,

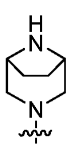, 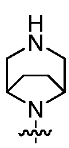, 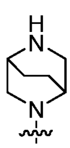, 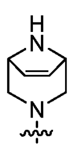, 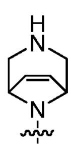, 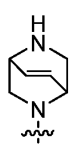, 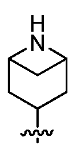, 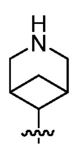, 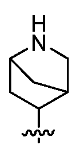, 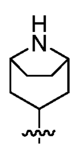, 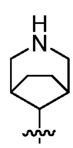, 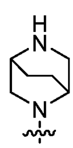, 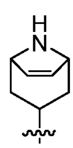, 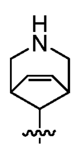,

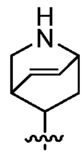

preferably

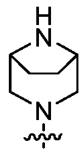

**Embodiment 4.1:** the compound of formula (I) according to any one of Embodiments 1 to 3.4, a stereoisomer, tautomer, stable isotopic variant, pharmaceutically acceptable salt or solvate thereof, wherein $R_2$ is H; or $R_2$ is CN.

**Embodiment 4.2:** the compound of formula (I) according to any one of Embodiments 1 to 3.4, a stereoisomer, tautomer, stable isotopic variant, pharmaceutically acceptable salt or solvate thereof, wherein $R_2$ is selected from halogen and -O-$C_{1-6}$ alkyl optionally substituted by halogen, for example but not limited to F, Cl, Br, I, -O-$CH_3$, -O-$CH_2CH_3$, -O-$CH_2CH_2CH_3$, -O-$CH(CH_3)(CH_3)$, -O-$CH_2CH_2CH_2CH_3$, -O-$CH_2CH(CH_3)CH_3$, -O-$C(CH_3)_3$, -O-$CH_2Cl$, -O-$CH_2F$, -O-$CHF_2$, -O-$CF_3$, -O-$CCl_3$, -O-$CH_2CH_2F$, -O-$CH_2CHF_2$, -O-$CH_2CF_3$, -O-$CH_2CH_2CH_2F$, -O-$CH_2CH_2CHF_2$, -O-$CH_2CH_2CF_3$, -O-$C(CH_3)_2CF_3$, -O-$C_2F_5$, -O-$C_2Cl_5$; preferably -O-$CH_3$; $R_2$ is selected from -O-$C_{1-6}$ alkyl wherein the alkyl is optionally substituted by one or more isotopes such as deuterium (D), e.g., -O-$CD_3$.

**Embodiment 4.2.1:** the compound of formula (I) according to any one of Embodiments 1 to 3.4, a stereoisomer, tautomer, stable isotopic variant, pharmaceutically acceptable salt or solvate thereof, wherein $R_2$ is selected from -$C_{1-6}$ alkyl optionally substituted by halogen, for example but not limited to -$CH_3$, -$CH_2CH_3$, -$CH_2CH_2CH_3$, -$CH(CH_3)(CH_3)$, -$CH_2CH_2CH_2CH_3$, -$CH_2CH(CH_3)CH_3$, -$C(CH_3)_3$, -$CH_2Cl$, -$CH_2F$, -$CHF_2$, -$CF_3$, -$CCl_3$, -$CH_2CH_2F$, -$CH_2CHF_2$, -$CH_2CF_3$, -$CH_2CH_2CH_2F$, - $CH_2CH_2CHF_2$, -$CH_2CH_2CF_3$, -$C(CH_3)_2CF_3$, -$C_2F_5$, -$C_2Cl_5$; preferably -$CH_3$.

**Embodiment 4.2.2:** the compound of formula (I) according to any one of Embodiments 1 to 3.4, a stereoisomer, tautomer, stable isotopic variant, pharmaceutically acceptable salt or solvate thereof, wherein $R_2$ is selected from -$C_{2-6}$ alkynyl optionally substituted by halogen, for example but not limited to -C≡CH, -C≡CF, -C≡C-$CH_3$, -$CH_2$-C≡CH.

**Embodiment 4.3:** the compound of formula (I) according to any one of Embodiments 1 to 4.2.2, a stereoisomer, tautomer, stable isotopic variant, pharmaceutically acceptable salt or solvate thereof, wherein $R_3$ is selected from halogen and -O-$C_{1-6}$ alkyl optionally substituted by halogen, for example but not limited to F, Cl, Br, I, -O-$CH_3$, -O-$CH_2CH_3$, -O-$CH_2CH_2CH_3$, -O-$CH(CH_3)(CH_3)$, -O-$CH_2CH_2CH_2CH_3$, -O-$CH_2CH(CH_3)CH_3$, -O-$C(CH_3)_3$, -O-$CH_2Cl$, -O-$CH_2F$, -O-$CHF_2$, -O-$CF_3$, -O-$CCl_3$, -O-$CH_2CH_2F$, -O-$CH_2CHF_2$, -O-$CH_2CF_3$, -O-$CH_2CH_2CH_2F$, -O-$CH_2CH_2CHF_2$, -O-$CH_2CH_2CF_3$, -O-$C(CH_3)_2CF_3$, -O-$C_2F_5$, -O-$C_2Cl_5$; preferably $R_3$ is selected from halogen, most preferably F.

**Embodiment 4.3.1:** the compound of formula (I) according to any one of Embodiment 1 to 4.2.2, a stereoisomer, tautomer, stable isotopic variant, pharmaceutically acceptable salt or solvate thereof, wherein $R_3$ is selected from -$C_{1-6}$ alkyl optionally substituted by halogen, for example but not limited to -$CH_3$, -$CH_2CH_3$, -$CH_2CH_2CH_3$, -$CH(CH_3)(CH_3)$, -$CH_2CH_2CH_2CH_3$, -$CH_2CH(CH_3)CH_3$, -$C(CH_3)_3$, -$CH_2Cl$, -$CH_2F$, -$CHF_2$, -$CF_3$, -$CCl_3$, -$CH_2CH_2F$, -$CH_2CHF_2$, -$CH_2CF_3$, -$CH_2CH_2CH_2F$, - $CH_2CH_2CHF_2$, -$CH_2CH_2CF_3$, -$C(CH_3)_2CF_3$, -$C_2F_5$, -$C_2Cl_5$; preferably -$CH_3$.

**Embodiment 4.4:** the compound of formula (I) according to any one of Embodiments 1 to 3.4, a stereoisomer, tautomer, stable isotopic variant, pharmaceutically acceptable salt or solvate thereof, wherein $R_2$ is H and $R_3$ is halogen, preferably F; or $R_2$ is -C $_{2-6}$ alkynyl, preferably -C≡CH and $R_3$ is halogen, preferably F; or $R_2$ is -O$C_{1-6}$ alkyl, preferably -$OCH_3$, -$OCD_3$, -$OCH_2CH_3$ and $R_3$ is halogen, preferably F.

**Embodiment 5.1:** the compound of formula (I) according to any one of Embodiments 1 to 4.4, a stereoisomer, tautomer, stable isotopic variant, pharmaceutically acceptable salt or solvate thereof, wherein M is N.

**Embodiment 5.2:** the compound of formula (I) according to any one of Embodiments 1 to 4.4, a stereoisomer, tautomer, stable isotopic variant, pharmaceutically acceptable salt or solvate thereof, wherein M is C-$R_4$.

**Embodiment 5.2.1:** the compound of formula (I) according to Embodiment 5.2, a stereoisomer, tautomer, stable isotopic variant, pharmaceutically acceptable salt or solvate thereof, wherein $R_4$ is H.

**Embodiment 5.2.2:** the compound of formula (I) according to Embodiment 5.2, a stereoisomer, tautomer, stable isotopic variant, pharmaceutically acceptable salt or solvate thereof, wherein $R_4$ is halogen, preferably Cl, F.

**Embodiment 5.2.3:** the compound of formula (I) according to Embodiment 5.2, a stereoisomer, tautomer, stable isotopic variant, pharmaceutically acceptable salt or solvate thereof, wherein $R_4$ is CN.

**Embodiment 5.2.4:** the compound of formula (I) according to Embodiment 5.2, a stereoisomer, tautomer, stable isotopic variant, pharmaceutically acceptable salt or solvate thereof, wherein $R_4$ is - $C_{1-6}$ alkyl, optionally substituted by halogen or CN; for example but not limited to $-CH_3$, $-CH_2CH_3$, - $CH_2CH_2CH_3$, $-CH(CH_3)(CH_3)$, $-CH_2CH_2CH_2CH_3$, $-CH_2CH(CH_3)CH_3$, $-C(CH_3)_3$, $-CH_2Cl$, $-CH_2F$, - $CHF_2$, $-CF_3$, $-CCl_3$, $-CH_2CH_2F$, $-CH_2CHF_2$, $-CH_2CF_3$, $-CH_2CH_2CH_2F$, $-CH_2CH_2CHF_2$, $-CH_2CH_2CF_3$, $-C(CH_3)_2CF_3$, $-CH_2CN$, $-CH_2CH_2CN$; preferably $-CF_3$.

**Embodiment 5.2.5:** the compound of formula (I) according to Embodiment 5.2, a stereoisomer, tautomer, stable isotopic variant, pharmaceutically acceptable salt or solvate thereof, wherein $R_4$ is - $(CH_2)_n$-$C_{3-6}$ cycloalkyl, optionally substituted by halogen or CN;

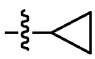 , 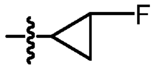 , 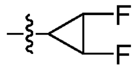 ,

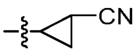 , 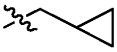 , 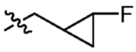 , 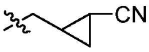 , 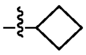 , 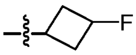 , 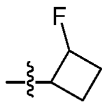 , 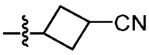 ,

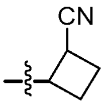 , 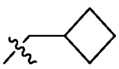 , 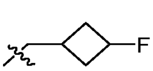 , 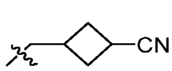 , 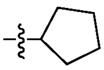 , 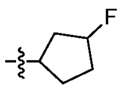 , 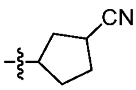 , 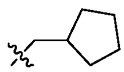 ,

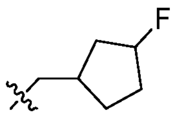 , 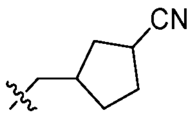 , 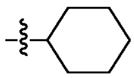 , 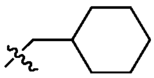 .

**Embodiment 5.3:** the compound of formula (I) according to any one of Embodiments 1 to 4.4, a stereoisomer, tautomer, stable isotopic variant, pharmaceutically acceptable salt or solvate thereof, wherein M is selected from N, C-F, C-Cl, C-CN and C-$CF_3$.

**Embodiment 5.4:** the compound of formula (I) according to any one of Embodiments 1 to 3.4, a stereoisomer, tautomer, stable isotopic variant, pharmaceutically acceptable salt or solvate thereof, wherein M is C-$R_4$ in structural fragment

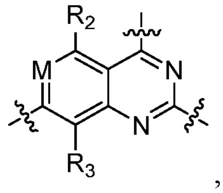,

wherein $R_4$ is selected from halogen (preferably F or Cl) or -$C_{1-6}$ alkyl optionally substituted by halogen (preferably -$CF_3$), $R_2$ is H and $R_3$ is halogen, preferably F; or M is N, $R_2$ is selected from H, -$C_{2-6}$ alkynyl (preferably -C≡CH) and - $OC_{1-6}$ alkyl (preferably -$OCH_3$, -$OCD_3$, -$OCH_2CH_3$), and $R_3$ is halogen, preferably F; specific examples include, but are not limited to:

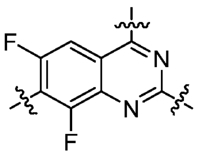, 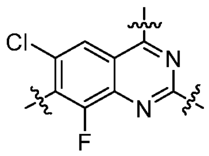, 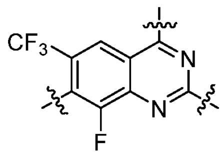, 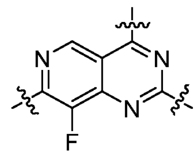, 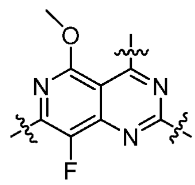,

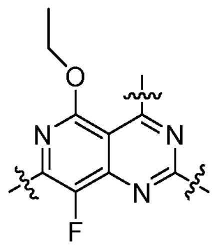, 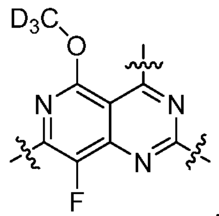, 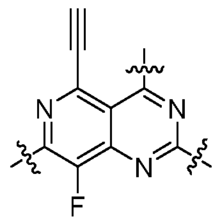.

**Embodiment 6.1:** the compound of formula (I) according to Embodiment 1 to 5.4, a stereoisomer, tautomer, stable isotopic variant, pharmaceutically acceptable salt or solvate thereof, wherein Y is O.

**Embodiment 6.2:** the compound of formula (I) according to any one of Embodiments 1 to 5.4, a stereoisomer, tautomer, stable isotopic variant, pharmaceutically acceptable salt or solvate thereof, wherein Y is S.

**Embodiment 6.3:** the compound of formula (I) according to any one of Embodiments 1 to 5.4, a stereoisomer, tautomer, stable isotopic variant, pharmaceutically acceptable salt or solvate thereof, wherein Y is Se.

**Embodiment 7.1:** the compound of formula (I) according to any one of Embodiments 1 to 6.3, a stereoisomer, tautomer, stable isotopic variant, pharmaceutically acceptable salt or solvate thereof, wherein $R_{14}$ is H; or one or both $R_{14}$ is D.

**Embodiment 7.2:** the compound of formula (I) according to Embodiment 1 to 6.3, a stereoisomer, tautomer, stable isotopic variant, pharmaceutically acceptable salt or solvate thereof, wherein $R_{14}$ is -$C_{1-6}$ alkyl, optionally substituted by halogen or $C_{1-6}$ alkoxy; for example but not limited to -$CH_3$, -$CH_2CH_3$, -$CH_2CH_2CH_3$, -$CH(CH_3)(CH_3)$, -$CH_2CH_2CH_2CH_3$, -$CH_2CH(CH_3)CH_3$, - $C(CH_3)_3$, -$CH_2$-$OCH_3$, -$CH_2$-O-$CH_2CH_3$, -$CH_2CH_2$-O-$CH_3$, -$CH_2CH_2$-O-$CH_2CH_3$, -$CH_2F$, -$CH_2Cl$, - $CHF_2$, -$CF_3$, -$CCl_3$, -$CH_2CH_2F$, -$CH_2CHF_2$, -$CH_2CF_3$, -$CH_2CH_2CH_2F$, -$CH_2CH_2CHF_2$, -$CH_2CH_2CF_3$, -$C_2F_5$, -$C_2Cl_5$, -$CF(CF_3)_2$.

**Embodiment 7.3:** the compound of formula (I) according to any one of Embodiments 1 to 6.3, a stereoisomer, tautomer, stable isotopic variant, pharmaceutically acceptable salt or solvate thereof, wherein $R_{14}$ is -$(CH_2)_n$-$C_{3-6}$ cycloalkyl, and the $C_{3-6}$ cycloalkyl is optionally substituted by halogen or $C_{1-6}$ alkoxy; for example but not limited to

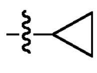, 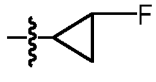, 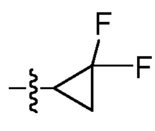, 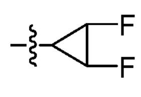,

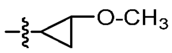, 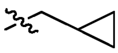, 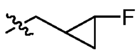, 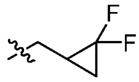, 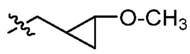, 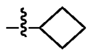, 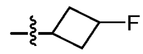,

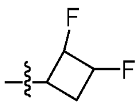, 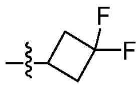, 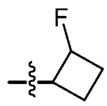, 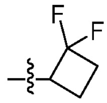, 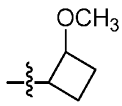, 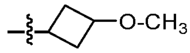, 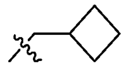,

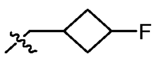, 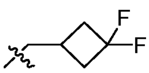, 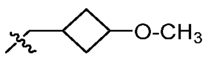, 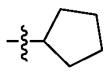, 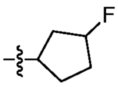, 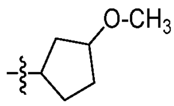, 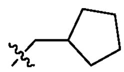,

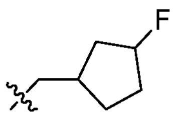, 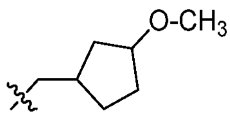, 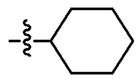, 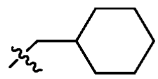.

**Embodiment 7.4:** the compound of formula (I) according to any one of Embodiments 1 to 6.3, a stereoisomer, tautomer, stable isotopic variant, pharmaceutically acceptable salt or solvate thereof, wherein two $R_{14}$ attached to the same carbon atom together with the carbon atom to which they are attached form a $C_{3-4}$ cycloalkyl group, such as cyclopropyl, cyclobutyl.

**Embodiment 8.1:** the compound of formula (I) according to any one of Embodiments 1 to 7.4, a stereoisomer, tautomer, stable isotopic variant, pharmaceutically acceptable salt or solvate thereof, wherein the structural fragment

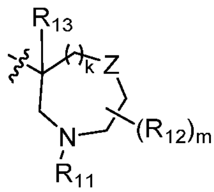,

when k is 0, the structural fragment is

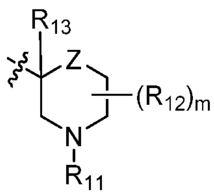,

wherein Z is selected from N, C, O, S and Se, preferably selected from C, O and Se, for example but not limited to

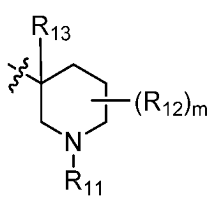, 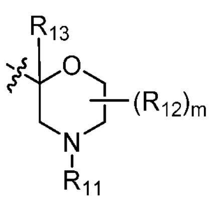, 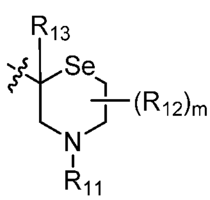.

**Embodiment 8.2:** the compound of formula (I) according to any one of Embodiments 1 to 7.4, a stereoisomer, tautomer, stable isotopic variant, pharmaceutically acceptable salt or solvate thereof, wherein the structural fragment

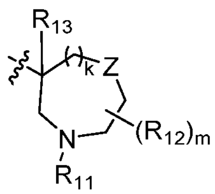

,

when k is 1, the structural fragment is

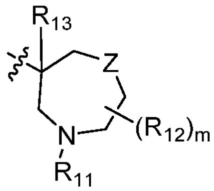

,

wherein Z is selected from N, C, O, S and Se, preferably from C, O and Se, for example but not limited to

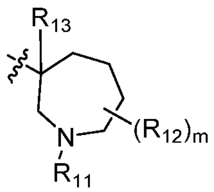 , 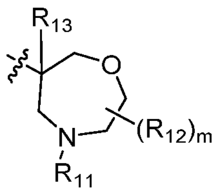 , 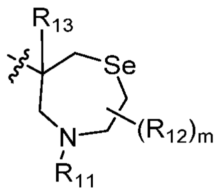 .

**Embodiment 8.3:** the compound of formula (I) according to any one of Embodiments 1 to 8.2, a stereoisomer, tautomer, stable isotopic variant, pharmaceutically acceptable salt or solvate thereof, wherein $R_{13}$ is H.

**Embodiment 8.3.1:** the compound of formula (I) according to any one of Embodiments 1 to 8.2, a stereoisomer, tautomer, stable isotopic variant, pharmaceutically acceptable salt or solvate thereof, wherein $R_{13}$ is halogen, preferably F.

**Embodiment 8.3.2:** the compound of formula (I) according to any one of Embodiments 1 to 8.2, a stereoisomer, tautomer, stable isotopic variant, pharmaceutically acceptable salt or solvate thereof, wherein $R_{13}$ is $-C_{1-6}$ alkyl, optionally substituted by halogen or $C_{1-6}$ alkoxy, for example but not limited to $-CH_3$, $-CH_2CH_3$, $-CH_2CH_2CH_3$, $-CH(CH_3)(CH_3)$, $-CH_2CH_2CH_2CH_3$, $-CH_2CH(CH_3)CH_3$, $-C(CH_3)_3$, $-CH_2-OCH_3$, $-CH_2-O-CH_2CH_3$, $-CH_2CH_2-O-CH_3$, $-CH_2CH_2-O-CH_2CH_3$, $-CH_2F$, $-CH_2Cl$, $-CHF_2$, $- CF_3$, $-CCl_3$, $-CH_2CH_2F$, $-CH_2CHF_2$, $-CH_2CF_3$, $-CH_2CH_2CH_2F$, $-CH_2CH_2CHF_2$, $-CH_2CH_2CF_3$, $-C_2F_5$, $-C_2Cl_5$, $-CF(CF_3)_2$.

**Embodiment 8.3.3:** the compound of formula (I) according to any one of Embodiments 1 to 8.2, a stereoisomer, tautomer, stable isotopic variant, pharmaceutically acceptable salt or solvate thereof, wherein $R_{13}$ is $-(CH_2)_n-C_{3-6}$ cycloalkyl, wherein $C_{3-6}$ cycloalkyl is optionally substituted by halogen or $C_{1-6}$ alkoxy; for example but not limited to

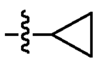 , 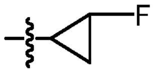 , 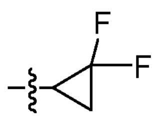 , 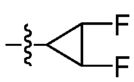 ,

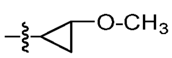 , 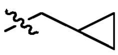 , 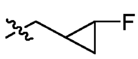 , 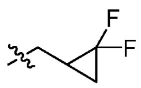 , 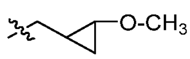 , 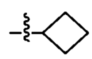 , 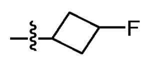 ,

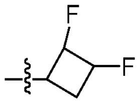, 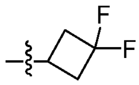, 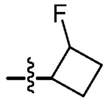, 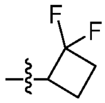, 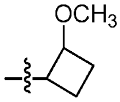, 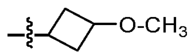, 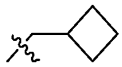,

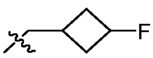, 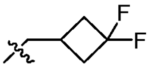, 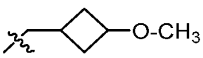, 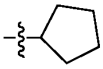, 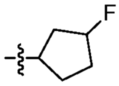, 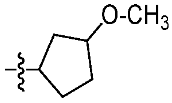, 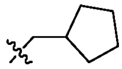,

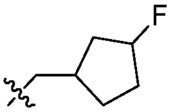, 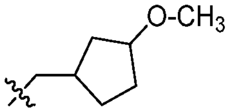, 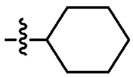, 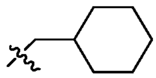.

**Embodiment 8.3.4:** the compound of formula (I) according to any one of Embodiments 1 to 8.2, a stereoisomer, tautomer, stable isotopic variant, pharmaceutically acceptable salt or solvate thereof, wherein $R_{12}$ and $R_{13}$ attached to the adjacent ring carbon atoms together with the carbon atoms to which they are attached form $C_{3-4}$ cycloalkyl, preferably cyclopropyl; for example but not limited to

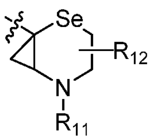, 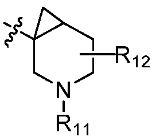, 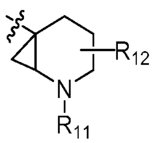, 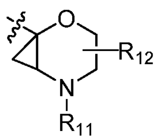, 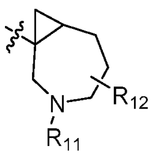, 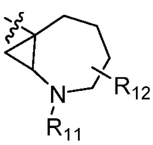,

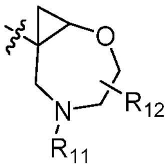, 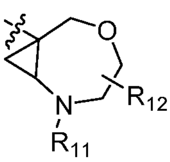, 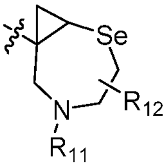, 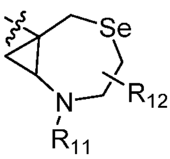.

**Embodiment 8.3.4:** the compound of formula (I) according to any one of Embodiments 1 to 8.2, a stereoisomer, tautomer, stable isotopic variant, pharmaceutically acceptable salt or solvate thereof, wherein $R_{13}$ is selected from H, halogen, and $-C_{1-6}$ alkyl optionally substituted by halogen.

**Embodiment 8.4:** the compound of formula (I) according to any one of Embodiments 1 to 8.3.4, a stereoisomer, tautomer, stable isotopic variant, pharmaceutically acceptable salt or solvate thereof, wherein $R_{11}$ is H.

**Embodiment 8.4.1:** the compound of formula (I) according to any one of Embodiments 1 to 8.3.4, a stereoisomer, tautomer, stable isotopic variant, pharmaceutically acceptable salt or solvate thereof, wherein $R_{11}$ is $-C_{1-6}$ alkyl, optionally substituted by halogen, CN, $-C_{1-6}$ alkoxy or $-O-CON(R^a)_2$, preferably optionally substituted by halogen or $-C_{1-6}$ alkoxy; for example but not limited to $-CH_3$, $-CH_2CH_3$, $-CH_2CH_2CH_3$, $-CH(CH_3)(CH_3)$, $-CH_2CH_2CH_2CH_3$, $-CH_2CH(CH_3)CH_3$, $-C(CH_3)_3$, $-CH_2-OCH_3$, $-CH_2-O-CH_2CH_3$, $-CH_2CH_2-O-CH_3$, $-CH_2CH_2-O-CH_2CH_3$, $-CH(CH_3)CH_2-OCH_3$, $-CH_2CH(CH_3)-OCH_3$, $-CH_2F$, $-CH_2Cl$, $-CHF_2$, $-CF_3$, $-CCl_3$, $-CH_2CH_2F$, $-CH_2CHF_2$, $-CH(CH_3)F$, $-CH(CH_3)CH_2F$, $-CH_2CH(CH_3)F$, $-CH_2CF_3$, $-CH_2CH_2CH_2F$, $-CH_2CH_2CHF_2$, $-CH_2CH_2CF_3$, $-C_2F_5$, $-C_2Cl_5$, $-CF(CF_3)_2$, $-CH_2CN$, $-CH_2CH_2CN$,

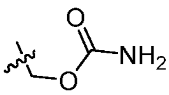, 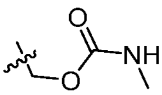, 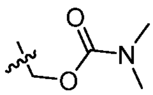.

**Embodiment 8.4.1.1:** the compound of formula (I) according to any one of Embodiments 1 to 8.3.4, a stereoisomer, tautomer, stable isotopic variant, pharmaceutically acceptable salt or solvate thereof, wherein $R_{11}$ is $-C_{1-6}$ alkyl, wherein the hydrogen atoms are replaced by one or more isotopes D, e.g., $-CD_3$.

**Embodiment 8.4.2:** the compound of formula (I) according to any one of Embodiments 1 to 8.3.4, a stereoisomer, tautomer, stable isotopic variant, pharmaceutically acceptable salt or solvate thereof, wherein $R_{11}$ is-$C_{2-6}$ alkenyl or -$C_{2-6}$ alkynyl, optionally substituted by halogen, CN, -$C_{1-6}$ alkoxy or -O-CON($R^a$)$_2$; for example but not limited to vinyl, propyl, ethynyl, each optionally substituted by halogen or -$C_{1-6}$ alkoxy.

**Embodiment 8.4.3:** the compound of formula (I) according to any one of Embodiments 1 to 8.3.4, a stereoisomer, tautomer, stable isotopic variant, pharmaceutically acceptable salt or solvate thereof, wherein $R_{11}$ is -$(CH_2)_n$-$C_{3-6}$ cycloalkyl, and the -$C_{3-6}$ cycloalkyl is optionally substituted by halogen, CN, -$C_{1-6}$ alkoxy or -O-CON($R^a$)$_2$; for example but not limited to

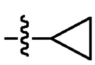, 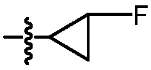,

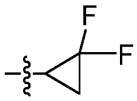, 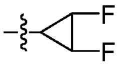, 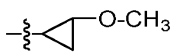, 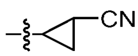, 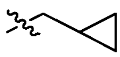, 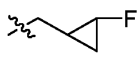, 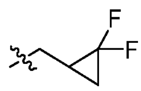,

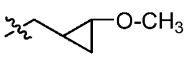, 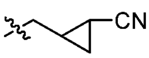, 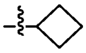, 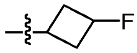, 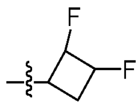, 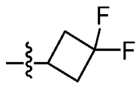, 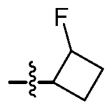, 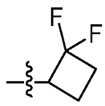,

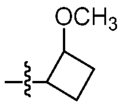, 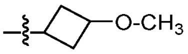, 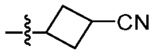, 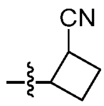, 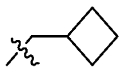, 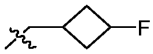, 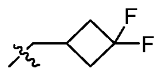,

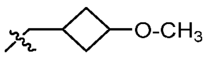, 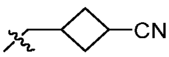, 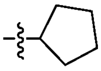, 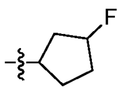, 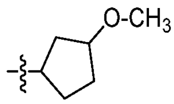, 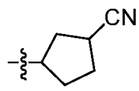, 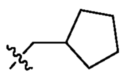,

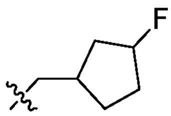, 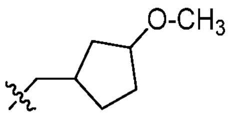, 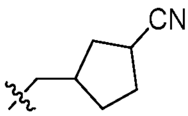, 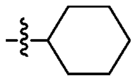, 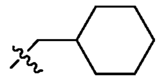.

**Embodiment 8.4.4:** the compound of formula (I) according to any one of Embodiments 1 to 8.3.4, a stereoisomer, tautomer, stable isotopic variant, pharmaceutically acceptable salt or solvate thereof, wherein $R_{11}$ is -$C_{1-6}$ alkyl, optionally substituted by halogen or -$C_{1-6}$ alkoxy; e.g., -$CH_3$, - $CH_2CH_2$-O-$CH_3$, -$CH_2CH_2F$; or $R_{11}$ is -$C_{1-6}$ alkyl, wherein one or more hydrogen atoms are replaced by isotope D, preferably -$CD_3$.

**Embodiment 8.5:** the compound of formula (I) according to any one of Embodiments 1 to 8.4.4, a stereoisomer, tautomer, stable isotopic variant, pharmaceutically acceptable salt or solvate thereof, wherein $R_{12}$ is H.

**Embodiment 8.5.1:** the compound of formula (I) according to any one of Embodiments 1 to 8.4.4, a stereoisomer, tautomer, stable isotopic variant, pharmaceutically acceptable salt or solvate thereof, wherein $R_{12}$ is halogen, e.g., F, Cl, Br, I, preferably F.

**Embodiment 8.5.2:** the compound of formula (I) according to any one of Embodiments 1 to 8.4.4, a stereoisomer, tautomer, stable isotopic variant, pharmaceutically acceptable salt or solvate thereof, wherein $R_{12}$ is -N($R^a$)$_2$, for example -$NH_2$, -$NHCH_3$, -N($CH_3$)$_2$, N($CH_3$)($CH_2CH_3$).

**Embodiment 8.5.3:** the compound of formula (I) according to any one of Embodiments 1 to 8.4.4, a stereoisomer, tautomer, stable isotopic variant, pharmaceutically acceptable salt or solvate thereof, wherein $R_{12}$ is -OH.

**Embodiment 8.5.4:** the compound of formula (I) according to any one of Embodiments 1 to 8.4.4, a stereoisomer, tautomer, stable isotopic variant, pharmaceutically acceptable salt or solvate thereof, wherein $R_{12}$ is $-O-C_{1-6}$ alkyl, and the $C_{1-6}$ alkyl is optionally substituted by halogen, CN or $C_{1-6}$ alkoxy; for example but not limited to $-O-CH_3$, $-O-CH_2CH_3$, $-O-CH_2CH_2CH_3$, $-O-CH(CH_3)(CH_3)$, $-O-CH_2CH_2CH_2CH_3$, $-O-CH_2CH(CH_3)CH_3$, $-O-C(CH_3)_3$, $-O-CH_2Cl$, $-O-CH_2CN$, $-O-CH_2F$, $-O-CHF_2$, $-O-CF_3$, $-O-CCl_3$, $-O-CH_2CH_2F$, $-O-CH_2CH_2CN$, $-O-CH_2CHF_2$, $-O-CH_2CF_3$, $-O-CH_2CH_2CH_2F$, $-O-CH_2CH_2CHF_2$, $-O-CH_2CH_2CF_3$, $-O-C(CH_3)_2CF_3$, $-O-C_2F_5$, $-O-C_2Cl_5$, $-O-CH_2-OCH_3$, $-O-CH_2-O-CH_2CH_3$, $-O-CH_2CH_2-O-CH_3$, $-O-CH_2CH_2-O-CH_2CH_3$.

**Embodiment 8.5.5:** the compound of formula (I) according to any one of Embodiments 1 to 8.4.4, a stereoisomer, tautomer, stable isotopic variant, pharmaceutically acceptable salt or solvate thereof, wherein $R_{12}$ is $-C_{1-6}$ alkyl, optionally substituted by halogen, CN or $C_{1-6}$ alkoxy, for example but not limited to $-CH_3$, $-CH_2CH_3$, $-CH_2CH_2CH_3$, $-CH(CH_3)(CH_3)$, $-CH_2CH_2CH_2CH_3$, $-CH_2CH(CH_3)CH_3$, $-C(CH_3)_3$, $-CH_2-OCH_3$, $-CH_2-O-CH_2CH_3$, $-CH_2CH_2-O-CH_3$, $-CH_2CH_2-O-CH_2CH_3$, $-CH_2F$, $-CH_2Cl$, $-CH_2CN$, $-CHF_2$, $-CF_3$, $-CCl_3$, $-CH_2CH_2F$, $-CH_2CH_2CN$, $-CH_2CHF_2$, $-CH_2CF_3$, $-CH_2CH_2CH_2F$, $-CH_2CH_2CHF_2$, $-CH_2CH_2CF_3$, $-C_2F_5$, $-C_2Cl_5$, $-CF(CF_3)_2$.

**Embodiment 8.5.6:** the compound of formula (I) according to any one of Embodiments 1 to 8.4.4, a stereoisomer, tautomer, stable isotopic variant, pharmaceutically acceptable salt or solvate thereof, wherein $R_{12}$ is $-O-C_{3-6}$ cycloalkyl, wherein $C_{3-6}$ cycloalkyl is optionally substituted by halogen, CN or $C_{1-6}$ alkoxy; for example but not limited to

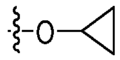, 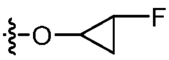, 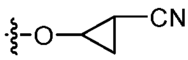,

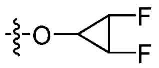, 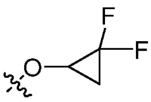, 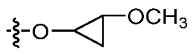, 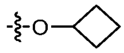, 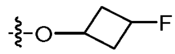, 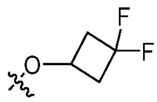,

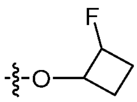, 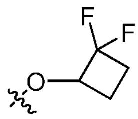, 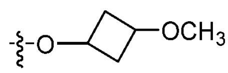, 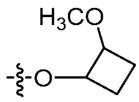, 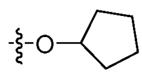, 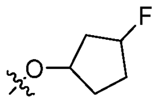,

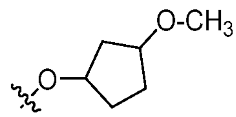, 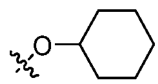.

**Embodiment 8.5.7:** the compound of formula (I) according to any one of Embodiments 1 to 8.4.4, a stereoisomer, tautomer, stable isotopic variant, pharmaceutically acceptable salt or solvate thereof, wherein $R_{12}$ is $-(CH_2)_n-C_{3-6}$ cycloalkyl optionally substituted by halogen, CN or $C_{1-6}$ alkoxy, for example but not limited to

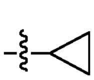, 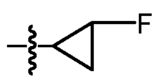, 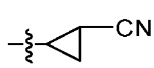, 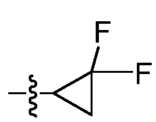, 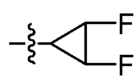,

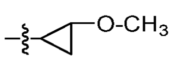, 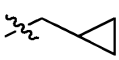, 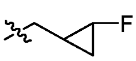, 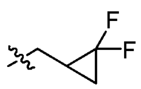, 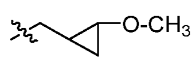, 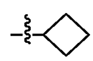, 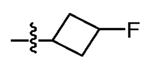,

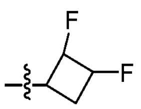, 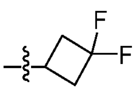, 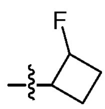, 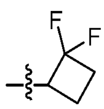, 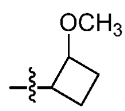, 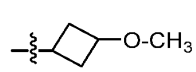, 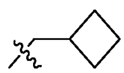,

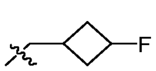, 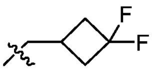, 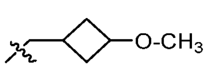, 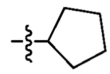, 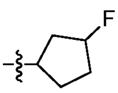, 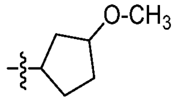, 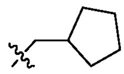,

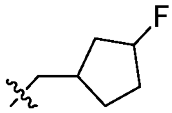, 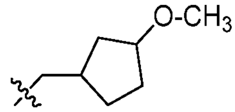, 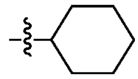, 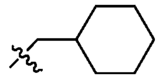.

**Embodiment 8.5.8:** the compound of formula (I) according to any one of Embodiments 1 to 8.4.4, a stereoisomer, tautomer, stable isotopic variant, pharmaceutically acceptable salt or solvate thereof, wherein $R_{12}$ attached to adjacent ring carbon atoms together with the carbon atoms to which they are attached form $C_{3-4}$ cycloalkyl, preferably cyclopropyl; for example but not limited to

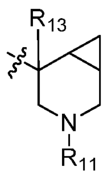, 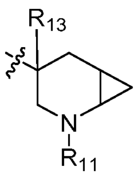, 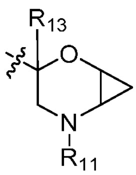, 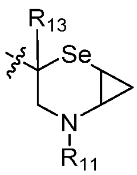, 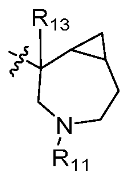, 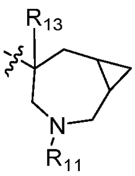, 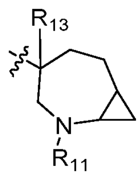, 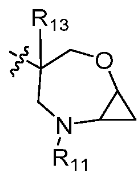,

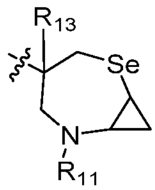.

**Embodiment 8.5.9:** the compound of formula (I) according to any one of Embodiments 1 to 8.4.4, a stereoisomer, tautomer, stable isotopic variant, pharmaceutically acceptable salt or solvate thereof, wherein two $R_{12}$ attached to non-adjacent ring carbon atoms together form bridging methylene or ethylene.

**Embodiment 8.5.10:** the compound of formula (I) according to any one of Embodiments 1 to 8.4.4, a stereoisomer, tautomer, stable isotopic variant, pharmaceutically acceptable salt or solvate thereof, wherein $R_{12}$ is CN.

**Embodiment 8.5.11:** the compound of formula (I) according to any one of Embodiments 1 to 8.4.4, a stereoisomer, tautomer, stable isotopic variant, pharmaceutically acceptable salt or solvate thereof, wherein $R_{12}$ is $-C_{2-6}$ alkenyl or $-C_{2-6}$ alkynyl, optionally substituted by halogen, CN or $-C_{1-6}$ alkoxy; for example but are not limited to vinyl, propenyl, ethynyl, each optionally substituted by halogen, CN or $-C_{1-6}$ alkoxyl.

**Embodiment 8.5.12:** the compound of formula (I) according to any one of Embodiments 1 to 8.4.4, a stereoisomer, tautomer, stable isotopic variant, pharmaceutically acceptable salt or solvate thereof, wherein two $R_{12}$ attached to the same carbon atom form $=C(R^c)_2$, wherein $R^c$ each independently selected from H, F, Cl, Br, I, $-C_{1-6}$ alkyl optionally substituted by halogen; for example but not limited to $=CH_2$, $=CF_2$, $=CCl_2$, $=C(CH_3)_2$, $=C(CF_3)_2$.

**Embodiment 8.5.13:** the compound of formula (I) according to any one of Embodiments 1 to 8.4.4, a stereoisomer, tautomer, stable isotopic variant, pharmaceutically acceptable salt or solvate thereof, wherein two $R_{12}$ attached to the same carbon atom form spiro $C_{3-6}$ cycloalkyl or spiro 4-7 membered heterocycloalkyl, for example but not limited to spirocyclopropyl, spirocyclobutyl, spirocyclopentyl, spiroazetidine, spiroazolidine, optionally substituted by halogen (preferably F) or - $C_{1-6}$ alkyl optionally substituted by halogen (preferably-$CF_3$).

**Embodiment 8.5.14:** the compound of formula (I) according to any one of Embodiments 1 to 8.4.4, a stereoisomer, tautomer, stable isotopic variant, pharmaceutically acceptable salt or solvate thereof, wherein $R_{12}$ is selected from H, halogen, -OH, $N(R^a)_2$, $-O-C_{1-6}$ alkyl, $-O-C_{3-6}$ cycloalkyl, $-C_{1-6}$ alkyl and-$(CH_2)_n-C_{3-6}$ cycloalkyl, wherein the $C_{1-6}$ alkyl or $C_{3-6}$ cycloalkyl at each occurrence is each independently and optionally substituted by halogen or $-OC_{1-6}$ alkyl,

or two $R_{12}$ attached to adjacent ring carbon atoms together with the carbon atoms to which they are attached form $C_{3-4}$ cycloalkyl,
or two $R_{12}$ attached to non-adjacent ring carbon atoms together form bridging methylene or ethylene.

**Embodiment 8.5.15:** the compound of formula (I) according to any one of Embodiments 1 to 8.4.4, a stereoisomer, tautomer, stable isotopic variant, pharmaceutically acceptable salt or solvate thereof, wherein $R_{12}$ is selected from H, halogen, CN, $-C_{1-6}$ alkyl optionally substituted by halogen, $-C_{2-6}$ alkynyl optionally substituted by halogen, and $-O-C_{1-6}$ alkyl optionally substituted by halogen, for example but not limited to H, F, CN, $-C\equiv CH$, $-CH_2F$, $-CHF_2$, $-O-CH_3$; or two $R_{12}$ attached to the same carbon atom form $=C(R^c)_2$, wherein $R^c$ each independently selected from H, halogen and $-C_{1-6}$ alkyl optionally substituted by halogen, for example but not limited to $=CH_2$, $=CF_2$, $=CCl_2$, $=C(CH_3)_2$, $=C(CF_3)_2$.

**Embodiment 8.5.16:** the compound of formula (I) according to any one of Embodiments 1 to 8.5.15, a stereoisomer, tautomer, stable isotopic variant, pharmaceutically acceptable salt or solvate thereof, wherein the ring carbon atom to which $R_{12}$ is attached is adjacent to the ring carbon atom to which $R_{13}$ is attached and not adjacent to the ring $N-R_{11}$, i.e.

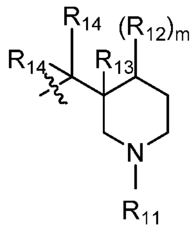

,

preferably

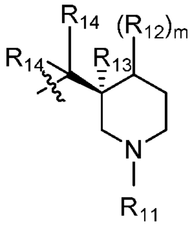

.

**Embodiment 9.1:** the compound of formula (I) according to any one of Embodiments 1 to 6.3, a stereoisomer, tautomer, stable isotopic variant, pharmaceutically acceptable salt or solvate thereof, wherein Z is selected from C, O, and Se in structural fragment

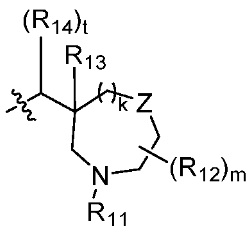

;

k is 0 or 1; $R_{11}$ is $-C_{1-6}$ alkyl optionally substituted by $-O-C_{1-6}$ alkyl or halogen; $R_{12}$ is selected from H, halogen, CN, $-C_{1-6}$ alkyl optionally substituted by halogen, $-C_{2-6}$ alkynyl optionally substituted by halogen, and $-O-C_{1-6}$ alkyl optionally substituted by halogen, or two $R_{12}$ attached to the same carbon atom form $=C(R^c)_2$, wherein $R^c$ is independently selected from H, halogen and $-C_{1-6}$ alkyl optionally substituted by halogen; $R_{13}$ is selected from H, halogen and $-C_{1-6}$ alkyl optionally substituted by halogen; and $R_{14}$ is H; or
Z is selected from C, O and Se; k is 0 or 1; $R_{11}$ is $-C_{1-6}$ alkyl substituted by one or more hydrogen isotopes such as D; $R_{12}$ selected from H, halogen, CN, $-C_{1-6}$ alkyl optionally substituted by halogen, $-C_{2-6}$ alkyl optionally substituted by halogen, and $-O-C_{1-6}$ alkyl optionally substituted by halogen, or two $R_{12}$ attached to the same carbon atom form $=C(R^c)_2$, wherein $R^c$ is independently selected from H, halogen and $-C_{1-6}$ alkyl optionally

substituted by halogen; $R_{13}$ is selected from H, halogen and - $C_{1-6}$ alkyl optionally substituted by halogen; and $R_{14}$ is H or hydrogen isotope such as D.

**Embodiment 9.2:** the compound of formula (I) according to any one of Embodiments 1 to 6.3, a stereoisomer, tautomer, stable isotopic variant, pharmaceutically acceptable salt or solvate thereof, wherein examples of structural fragment

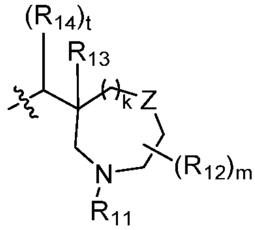

include, but are not limited to

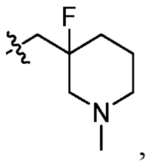,

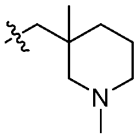, 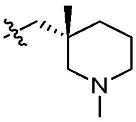, 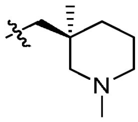, 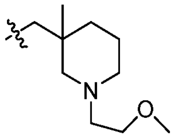, 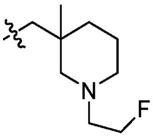, 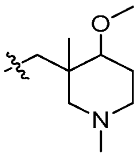, 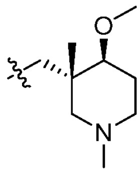,

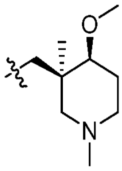, 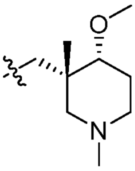, 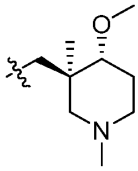, 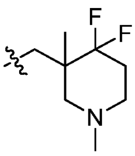, 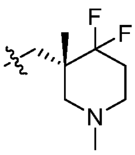, 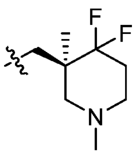, 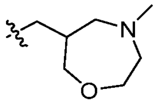,

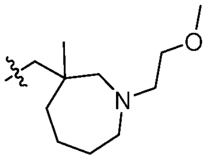, 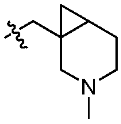, 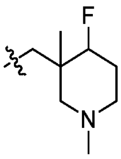, 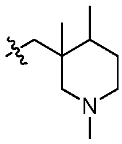, 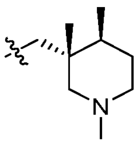, 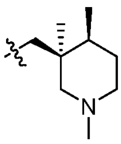, 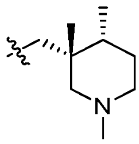,

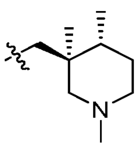, 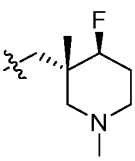, 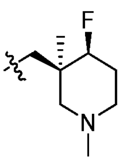, 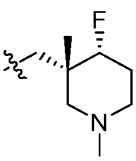, 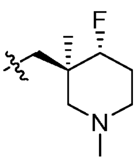, 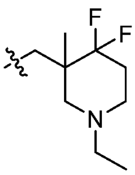, 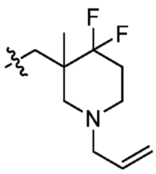,

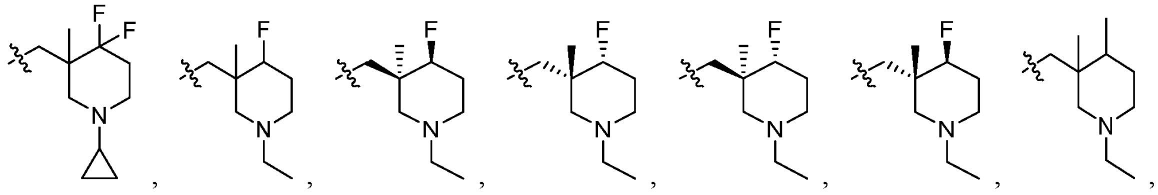
, , , , , , ,

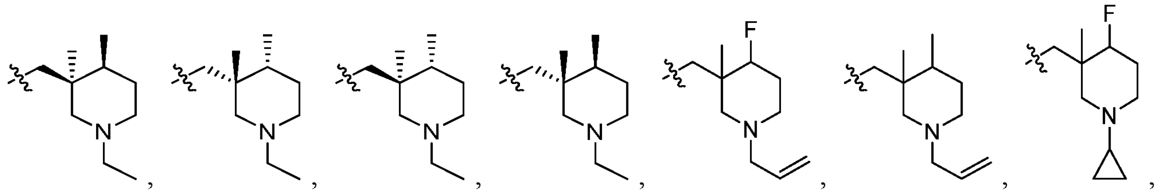
, , , , , , ,

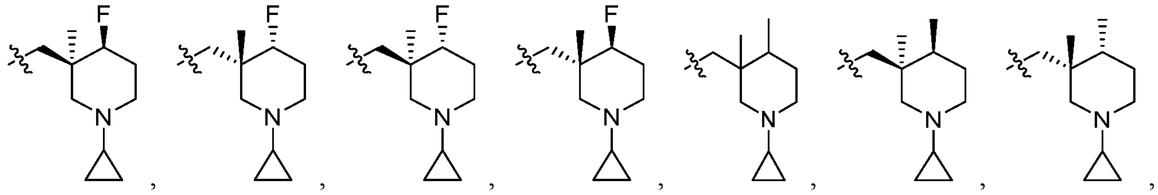
, , , , , , ,

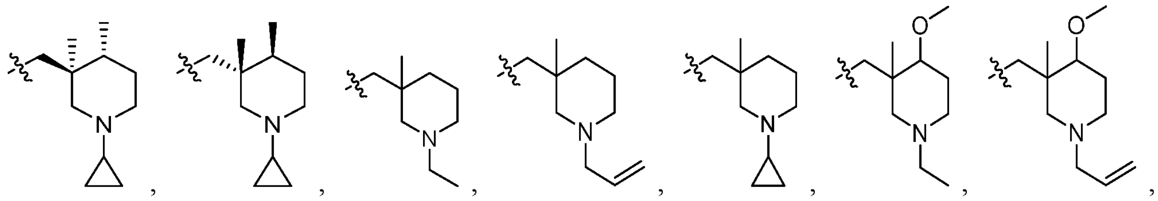
, , , , , , ,

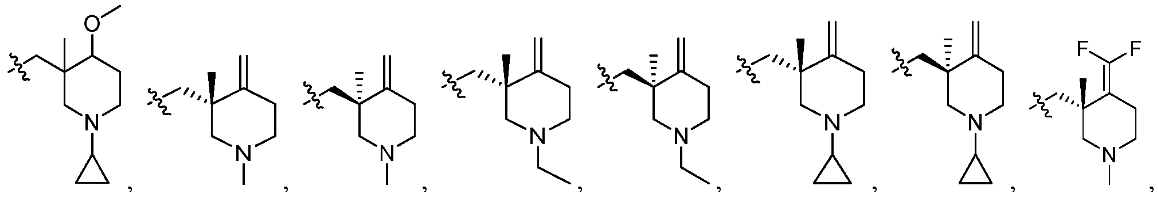
, , , , , , ,

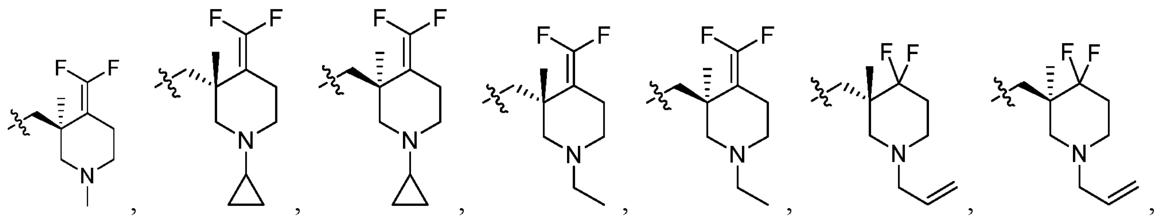
, , , , , , ,

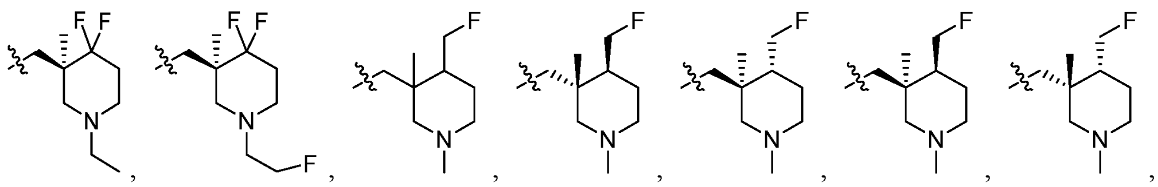
, , , , , , ,

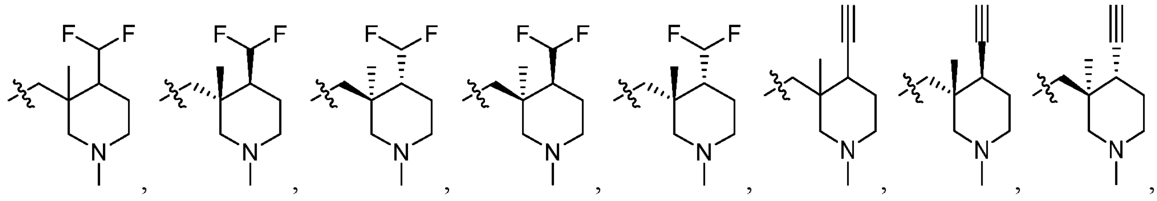
, , , , , , , ,

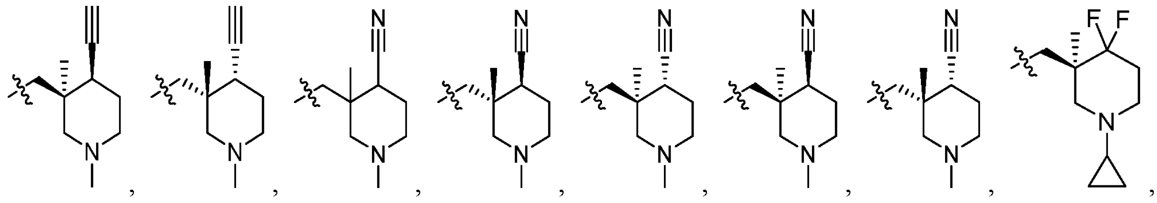
, , , , , , , ,

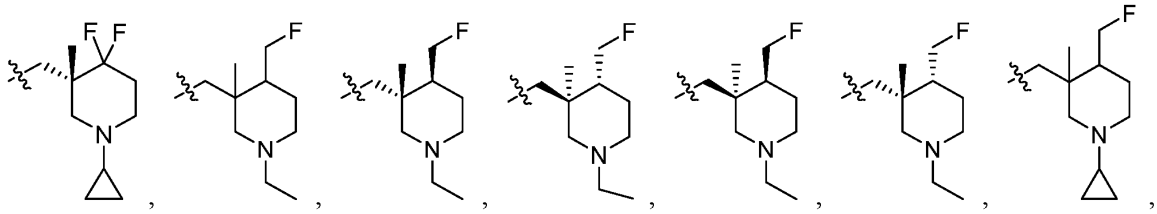
, , , , , , ,

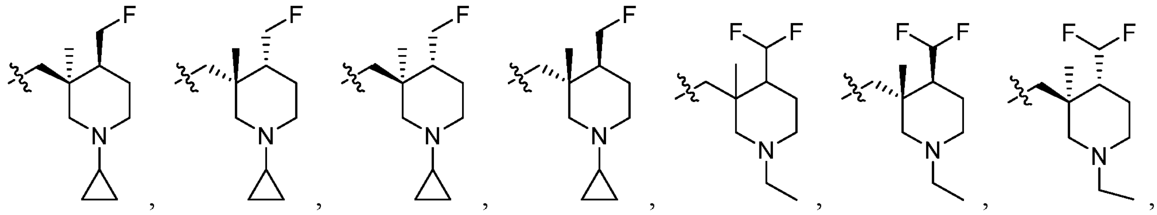
, , , , , , ,

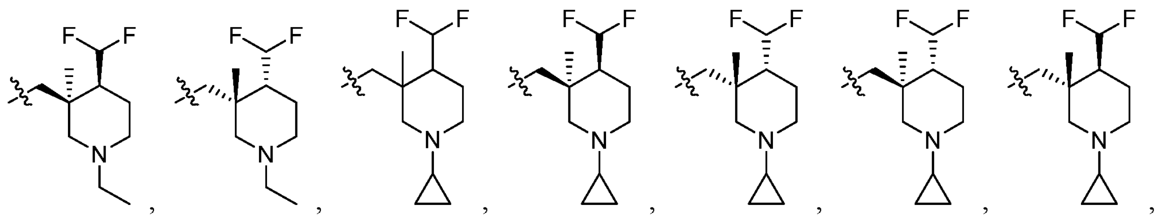
, , , , , , ,

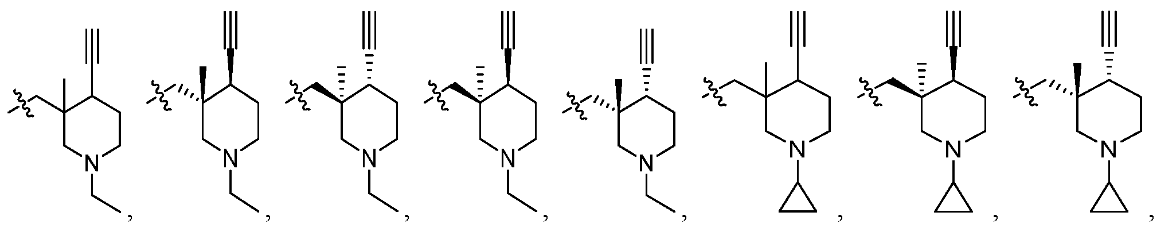
, , , , , , , ,

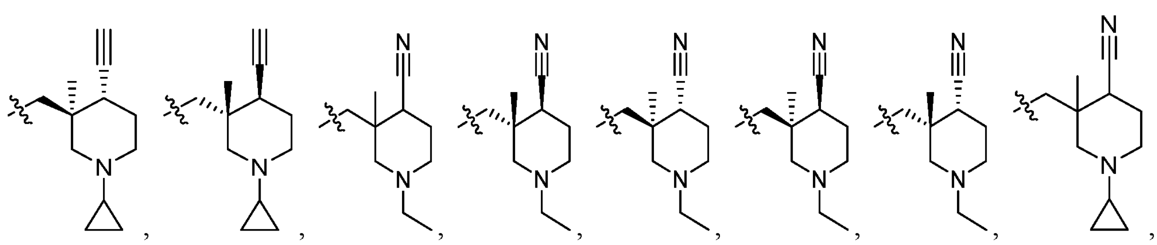
, , , , , , , ,

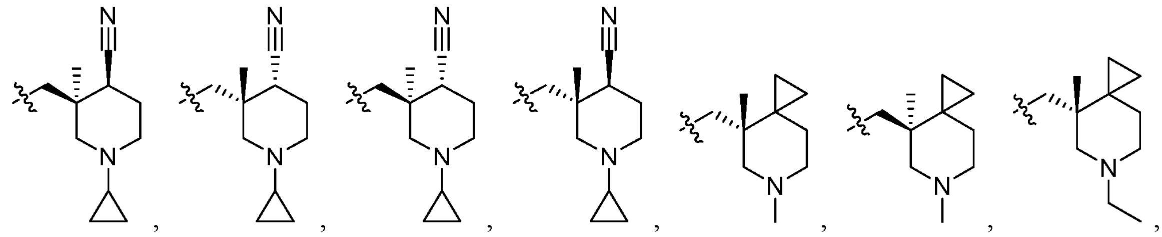

, , , , , , ,

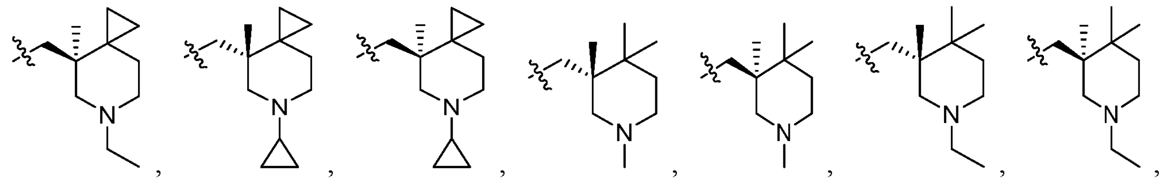

, , , , , , ,

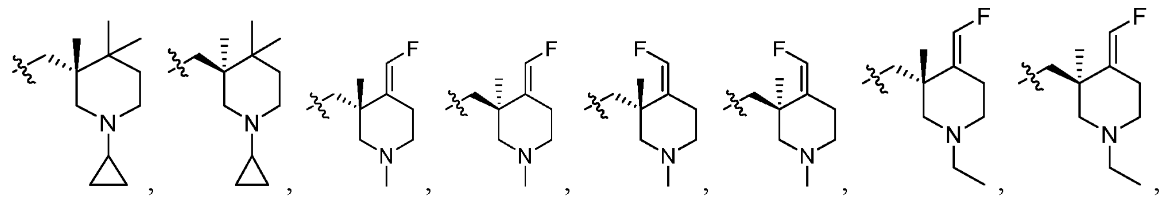

, , , , , , , ,

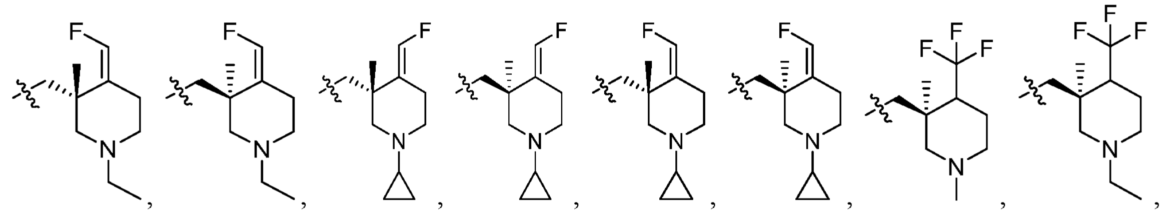

, , , , , , , ,

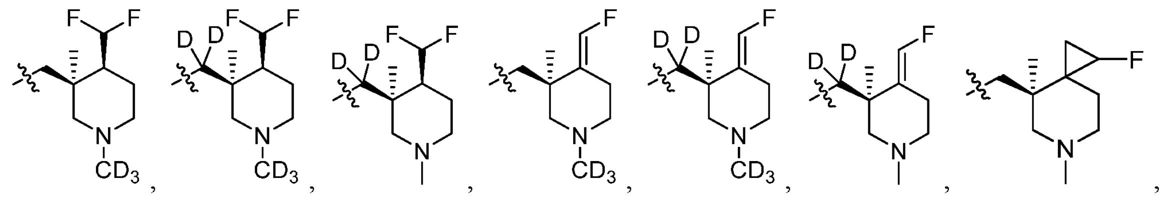

, , , , , , ,

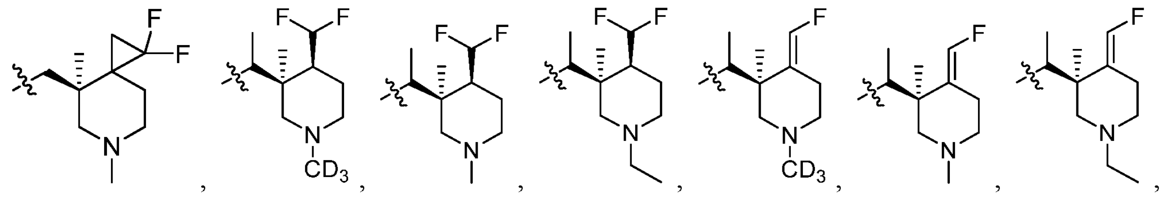

, , , , , , ,

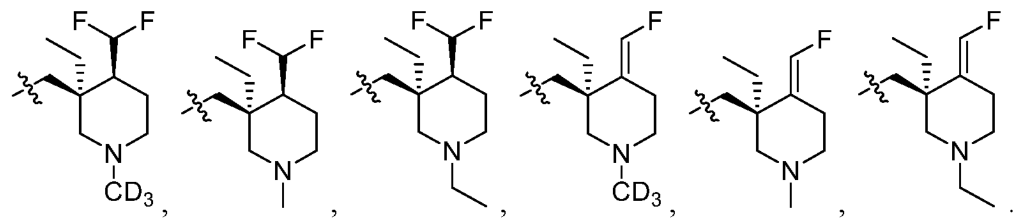

, , , , , .

**Embodiment 9.3:** the compound of formula (I) according to any one of Embodiments 1 to 6.3, a stereoisomer, tautomer, stable isotopic variant, pharmaceutically acceptable salt or solvate thereof, wherein the structural fragment

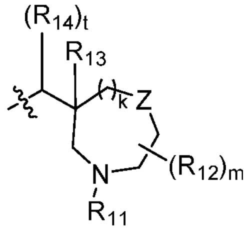

is

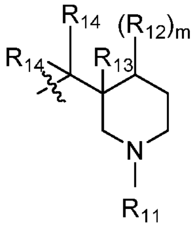

e.g.

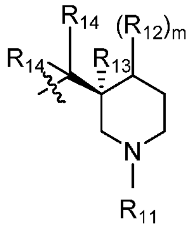

wherein $R_{11}$ is -$C_{1-6}$ alkyl optionally substituted by -O-$C_{1-6}$ alkyl or halogen or D, preferably -$C_{1-3}$ alkyl optionally substituted by one or more D; and/or $R_{12}$ is selected from halogen, CN, -$C_{1-6}$ alkyl optionally substituted by halogen, -$C_{2-6}$ alkynyl optionally substituted by halogen, and -O-$C_{1-6}$ optionally substituted by halogen, or two $R_{12}$ attached to the same carbon atom form =$C(R^c)_2$ or spiro $C_{3-6}$ cycloalkyl, wherein $R^c$ is each independently selected from H, halogen and -$C_{1-6}$ alkyl optionally substituted by halogen, and m is selected from 1 or 2; and/or $R_{14}$ is independently selected from H and D; and/or $R_{13}$ is -$C_{1-6}$ alkyl, preferably -$C_{1-3}$ alkyl; wherein $R_{12}$ is preferably selected from -$C_{1-6}$ alkyl optionally substituted by halogen, or two $R_{12}$ attached to the same carbon atom form =$C(R^c)_2$, where $R^c$ is each independently selected from H and halogen; specific examples of $R_{12}$ include, but are not limited to, fluorine, fluoromethyl, difluoromethyl, methyl, methoxy, ethynyl, cyano, fluoromethylene, difluoromethylene, methylene, difluoro, spirocyclopropyl, dimethyl;

In one Embodiment, the structural fragment is

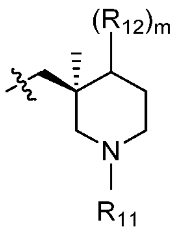

wherein $R_{11}$ is -$C_{1-6}$ alkyl optionally substituted by -O-$C_{1-6}$ alkyl or halogen, preferably -$C_{1-3}$ alkyl; and/or $R_{12}$ is selected from halogen, CN, -$C_{1-6}$ alkyl optionally substituted by halogen, -$C_{2-6}$ alkynyl optionally substituted by halogen, and -O-$C_{1-6}$ alkyl optionally substituted by halogen, or two $R_{12}$ attached to the same carbon atom form =$C(R^c)_2$ or spiro $C_{3-6}$ cycloalkyl, $R^c$ is each independently selected from H, halogen and -$C_{1-6}$ alkyl optionally substituted by halogen, and m is selected from 1 or 2; wherein $R_{12}$ is preferably selected from -$C_{1-6}$ alkyl substituted by halogen, or two $R_{12}$ attached to the same carbon atom form =$C(R^c)_2$, wherein $R^c$ is each independently selected from H and halogen; specific examples of $R_{12}$ include, but are not limited to fluorine,

fluoromethyl, difluoromethyl, methyl, methoxy, ethynyl, cyano, fluoromethylene, difluoromethylene, methylene, difluoro, spirocyclopropyl, dimethyl.

**Embodiment 9.4:** the compound of formula (I) according to any one of Embodiments 1 to 6.3, a stereoisomer, tautomer, stable isotopic variant, pharmaceutically acceptable salt or solvate thereof, wherein structural fragment

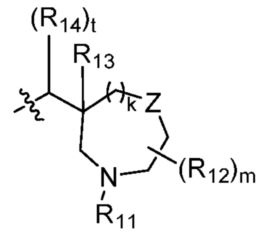

is

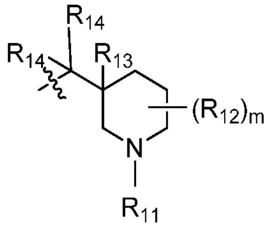

,

e.g.,

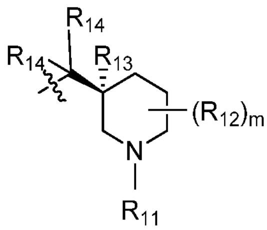

,

wherein $R_{11}$ is -$C_{1-6}$ alkyl optionally substituted by -O-$C_{1-6}$ alkyl, halogen or D, preferably -$C_{1-3}$ alkyl optionally substituted by one or more D; and/or $R_{12}$ is selected from halogen, CN, -$C_{1-6}$ alkyl optionally substituted by halogen, -$C_{2-6}$ alkynyl optionally substituted by halogen and -O-$C_{1-6}$ alkyl optionally substituted by halogen, or two $R_{12}$ attached to the same carbon form =$C(R^c)_2$ or spiro $C_{3-6}$ cycloalkyl, where $R^c$ is each independently selected from H, halogen and -$C_{1-6}$ alkyl optionally substituted by halogen, and m is selected from 1 or 2; and/or $R_{14}$ is each independently selected from H and D; and/or $R_{13}$ is -$C_{1-6}$ alkyl, preferred-$C_{1-3}$ alkyl; wherein $R_{12}$ is preferably selected from -$C_{1-6}$ alkyl optionally substituted by halogen, or two $R_{12}$ attached to the same carbon form =$C(R^c)_2$, wherein $R^c$ is each independently selected from H and halogen; specific examples of $R_{12}$ include, but are not limited to, fluorine, fluoromethyl, difluoromethyl, methyl, methoxy, acetylenyl, cyano, fluoromethylene, difluoromethylene, methylene, difluoro, spirocyclopropyl, dimethyl; In one Embodiment, the structural fragment is

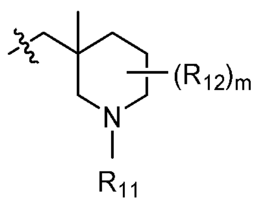

,

where $R_{11}$ is -$C_{1-6}$ alkyl, -$C_{2-6}$ alkenyl, or -$C_{3-6}$ cycloalkyl optionally substituted by -O-$C_{1-6}$ alkyl or halogen, examples include methyl, ethyl, cyclopropyl, propenyl, isopropyl, fluoroethyl; and/or $R_{12}$ is selected from halogen, CN, -$C_{1-6}$ alkyl optionally substituted by halogen, -$C_{2-6}$ alkyl optionally substituted by halogen, and -O-$C_{1-6}$ alkyl

optionally substituted by halogen, or two $R_{12}$ attached to the same carbon atom form $=C(R^c)_2$ or spiro $C_{3-6}$ cycloalkyl, wherein $R^c$ is each independently selected from H, halogen and $-C_{1-6}$ alkyl optionally substituted by halogen, and m is selected from 1 or 2; wherein $R_{12}$ is preferably selected from $-C_{1-6}$ alkyl optionally substituted by halogen, or two $R_{12}$ attached to the same carbon atom form $=C(R^c)_2$, wherein $R^c$ is each independently selected from H and halogen; specific examples of $R_{12}$ include fluorine, fluoromethyl, difluoromethyl, methyl, methoxy, ethynyl, cyano, fluoromethylene, difluoromethylene, methylene, difluoro, spirocyclopropyl, dimethyl.

**Embodiment 10.1:** the compound of formula (I) according to Embodiment 1, a stereoisomer, tautomer, stable isotopic variant, pharmaceutically acceptable salt or solvate thereof, which has the following sub-general formula:

<table>
<tr><td colspan="2">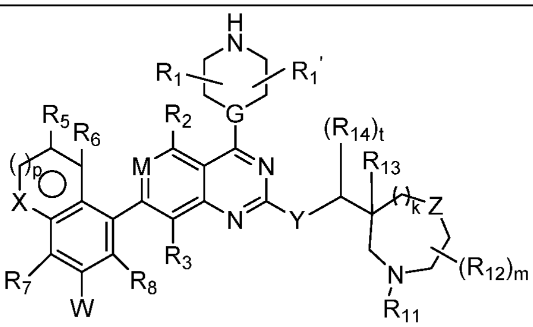<br>(I-A)</td></tr>
<tr><td>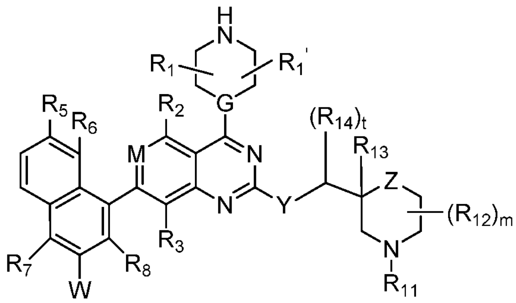<br>(I-A-1)</td><td>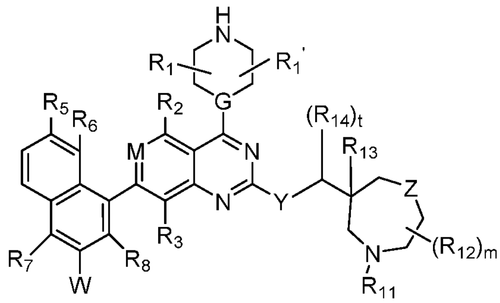<br>(I-A-2)</td></tr>
<tr><td>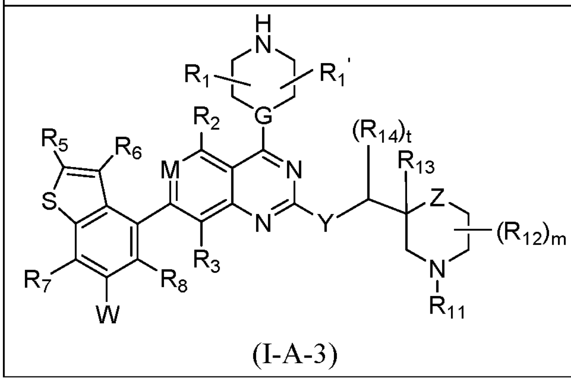<br>(I-A-3)</td><td>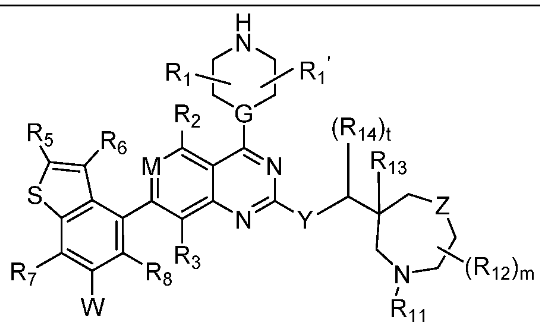<br>(I-A-4)</td></tr>
</table>

Wherein each substituent has the meaning defined in the respective preceding embodiments.

**Embodiment 10.1.1:** the compound of formula (I) according to Embodiment 10.1, a stereoisomer, tautomer, stable isotopic variant, pharmaceutically acceptable salt or solvate thereof, wherein:

Yis O;

G is CH or N;

M is $C-R_4$;

Z is selected from C, Se and O;

W is -OH or $-NH_2$;

$R_1$ and $R_1$' together form $-(CH_2)_t-$;

$R_2$ is H;

$R_3$ is halogen;

$R_4$ is halogen;

$R_5$ is selected from H and halogen;

$R_6$ is selected from halogen, $-C_{1-6}$ alkyl, and $-C_{2-6}$ alkynyl;

$R_7$ and $R_8$ are each independently selected from H, halogen, CN and $NO_2$;

$R_{11}$ is $-C_{1-6}$ alkyl optionally substituted by $-O-C_{1-6}$ alkyl or halogen;

$R_{12}$ is selected from H, halogen, CN, $-C_{1-6}$ alkyl optionally substituted by halogen, $-C_{2-6}$ alkynyl optionally substituted by halogen, and $-O-C_{1-6}$ alkyl optionally substituted by halogen, or two $R_{12}$ attached to the same carbon atom form $=C(R^c)_2$, wherein $R^c$ is each independently selected from H, halogen and $-C_{1-6}$ alkyl optionally substituted by halogen;

$R_{13}$ is selected from H, halogen and $-C_{1-6}$ alkyl optionally substituted by halogen;

$R_{14}$ is H;

k is selected from 0 or 1;

m and n are each independently selected from integers 0 to 2; and

t is selected from 1 or 2.

**Embodiment 10.1.2:** the compound of formula (I) according to Embodiments 10.1 to 10.1.1, a stereoisomer, tautomer, stable isotopic variant, pharmaceutically acceptable salt or solvate thereof, wherein:

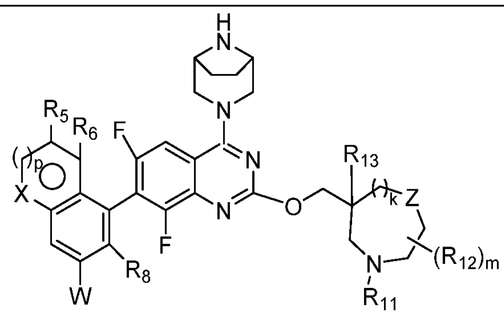

(I-A')

| | |
|---|---|
| 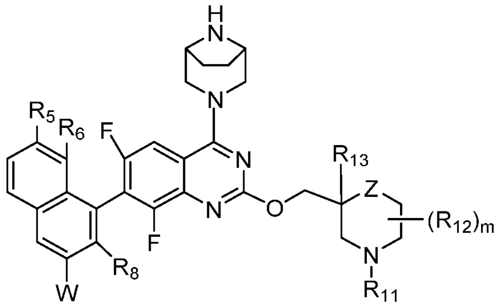 (I-A-1') | 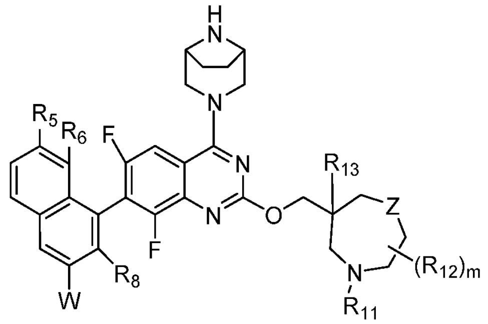 (I-A-2') |
| 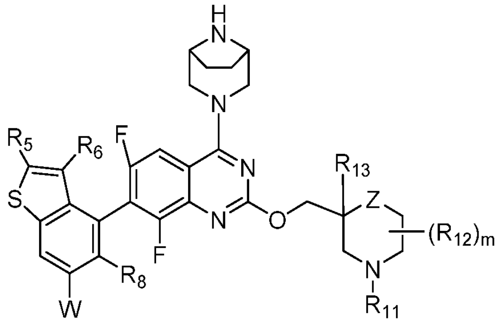 (I-A-3') | 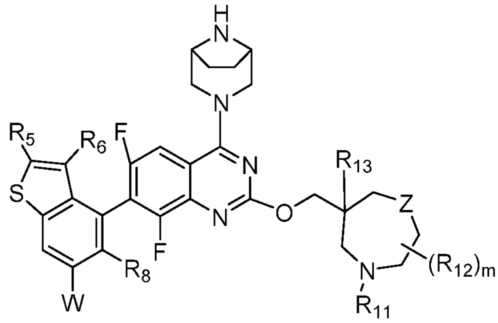 (I-A-4') |

Wherein:

Z is selected from C and O;

W is -OH or $-NH_2$;

$R_5$ is selected from H and halogen, preferably halogen, most preferably F;

$R_6$ is selected from halogen, $-C_{1-6}$ alkyl and $-C_{2-6}$ alkynyl, preferably $-C_{2-6}$ alkynyl, and most preferably ethynyl;

$R_8$ is selected from H and halogen, preferably H or F;

$R_{11}$ is $-C_{1-6}$ alkyl optionally substituted by $-O-C_{1-6}$ alkyl or halogen, preferably $-CH_3$, $-CH_2CH_3$, $- CH_2CH_2-O-CH_3$, $-CH_2CH_2-O-F$;

$R_{12}$ is selected from H, halogen, CN, $-C_{1-6}$ alkyl optionally substituted by halogen, $-C_{2-6}$ alkynyl optionally substituted by halogen, $-O-C_{1-6}$ alkyl optionally substituted by halogen, preferably H, F, CN, -C≡CH, $-CH_2F$, $-CHF_2$, $CH_3$, and $-O-CH_3$, or two $R_{12}$ attached to the same carbon atom form $=C(R^c)_2$, wherein $R^c$ is each independently selected from H, halogen and $-C_{1-6}$ alkyl optionally substituted halogen, preferably $=CH_2$, $=CF_2$, $=CCl_2$, $=C(CH_3)_2$, $=C(CF_3)_2$;

$R_{13}$ is selected from H, halogen and $-C_{1-6}$ alkyl optionally substituted halogen, preferably H, F and $- CH_3$;

k is selected from 0 or 1; and

m is selected from integers from 0 to 2.

**Embodiment 10.1.3:** the compound of formula (I) according to Embodiment 10.1, a stereoisomer, tautomer, stable isotopic variant, pharmaceutically acceptable salt or solvate thereof, which has the following formula:

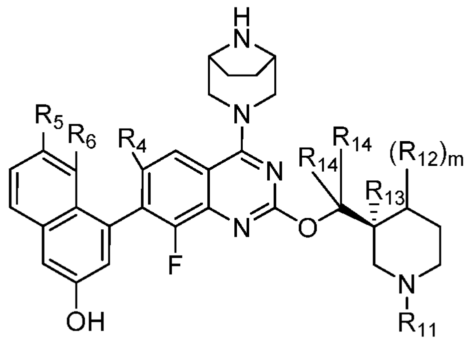

(I-A-1"),

e.g.,

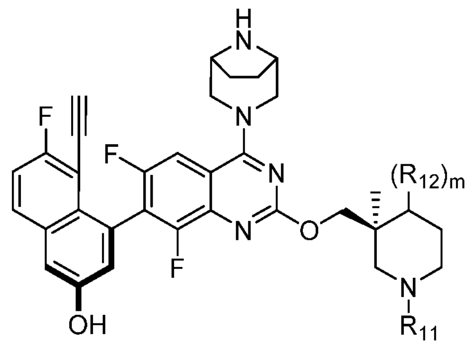

,

wherein $R_5$ is halogen, preferably F; and/or $R_6$ is -$C_{2-6}$ alkynyl, preferably ethynyl; and/or $R_4$ is halogen, preferably F; and/or $R_{14}$ is each independently selected from H and D; and/or $R_{13}$ is -$C_{1-6}$ alkyl, preferably -$C_{1-3}$ alkyl; and/or $R_{11}$ is -$C_{1-6}$ alkyl optionally substituted by -O-$C_{1-6}$ alkyl or halogen or D, preferably -$C_{1-3}$ alkyl optionally substituted by one or more D, preferably methyl, ethyl; and/or $R_{12}$ is selected from halogen, CN, -$C_{1-6}$ alkyl optionally substituted by halogen, -$C_{2-6}$ alkynyl optionally substituted by halogen, and -O-$C_{1-6}$ alkyl optionally substituted by halogen, or two $R_{12}$ attached to the same carbon atom form =C($R^c$)$_2$ or spiro $C_{3-6}$ cycloalkyl, wherein $R^c$ is each independently selected from H, halogen and -$C_{1-6}$ alkyl optionally substituted by halogen, and m is selected from 1 or 2; wherein $R_{12}$ is preferably selected from $C_{1-6}$ alkyl optionally substituted by halogen (preferably F), or two $R_{12}$ attached to the same carbon atom form =C($R^c$)$_2$, wherein $R^c$ is each independently selected from H and halogen (preferably F); specific examples of $R_{12}$ include, but are not limited to fluorine, fluoromethyl, difluoromethyl, methyl, methoxy, ethynyl, cyano, fluoromethylene, difluoromethylene, methylene, difluoro, spirocyclopropyl, dimethyl; specifically, wherein

Wherein $R_{11}$ is -$C_{1-6}$ alkyl labeled by one or more hydrogen isotopes such as D, preferably -$CD_3$ alkyl; and/or $R_{12}$ is selected from halogen, CN, -$C_{1-6}$ alkyl optionally substituted by halogen, -$C_{2-6}$ alkynyl optionally substituted by halogen, and -O-$C_{1-6}$ alkyl optionally substituted by halogen, or two $R_{12}$ attached to the same carbon atom form =C($R^c$)$_2$ or spiro $C_{3-6}$ cycloalkyl, wherein $R^c$ is each independently selected from H, halogen and -$C_{1-6}$ alkyl optionally substituted by halogen, and m is selected from 1 or 2; wherein $R_{12}$ is preferably selected from -$C_{1-6}$ alkyl substituted by halogen (preferably F) or two $R_{12}$ attached to the same carbon atom form =C($R^c$)$_2$, wherein $R^c$ is each independently selected from H and halogen (preferably F); specific examples of $R_{12}$ include, but are not limited to fluoromethyl, difluoromethyl, methyl, methoxy, ethynyl, cyano, fluoromethylene, difluoromethylene, methylene, difluoro, spirocyclopropyl, dimethyl.

**Embodiment 10.1.4:** the compound of formula (I) according to Embodiment 10.1, a stereoisomer, tautomer, stable isotopic variant, pharmaceutically acceptable salt or solvate thereof, which has the following formula:

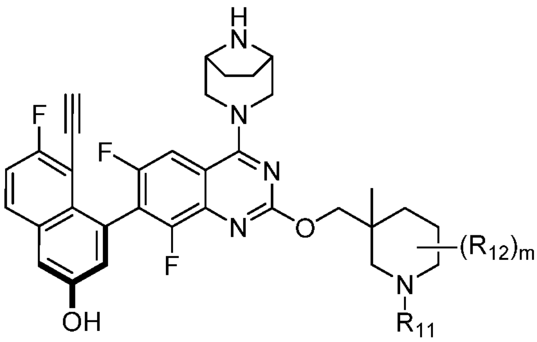

(I-A-1'''),

where $R_{11}$ is $-C_{1-6}$ alkyl, $-C_{2-6}$ alkenyl or $-C_{3-6}$ cycloalkyl optionally substituted by $-O-C_{1-6}$ alkyl or halogen, examples include methyl, ethyl, cyclopropyl, propenyl, isopropyl, fluoroethyl; $R_{12}$ is selected from halogen, CN, $-C_{1-6}$ alkyl optionally substituted by halogen, $-C_{2-6}$ alkynyl optionally substituted by halogen and $-O-C_{1-6}$ alkyl optionally substituted by halogen, or two $R_{12}$ attached to the same carbon atom form $=C(R^c)_2$ or spiro $C_{3-6}$ cycloalkyl, wherein $R^c$ is each independently selected from H, halogen and $-C_{1-6}$ alkyl optionally substituted by halogen, and m is selected from 1 or 2; specific examples of $R_{12}$ include fluorine, fluoromethyl, difluoromethyl, methyl, methoxy, ethynyl, cyano, fluoromethylene, difluoromethyl, methylene, difluoro, spirocyclopropyl, dimethyl; or

Wherein $R_{11}$ is $-C_{1-6}$ alkyl labeled by one or more hydrogen isotopes such as D, and preferably $-CD_3$ alkyl; $R_{12}$ is selected from halogen, CN,$-C_{1-6}$ alkyl optionally substituted by halogen, $-C_{2-6}$ alkynyl optionally substituted by halogen, and $-O-C_{1-6}$ alkyl optionally substituted by halogen, or two $R_{12}$ attached to the same carbon atom form $=C(R^c)_2$ or spiro $C_{3-6}$ cycloalkyl, wherein $R^c$ is each independently selected from H, halogen and $-C_{1-6}$ alkyl optionally substituted by halogen, and m is selected from 1 or 2; specific examples of $R_{12}$ include fluorine, fluoromethyl, difluoromethyl, methyl, methoxy, ethynyl, cyano, fluoromethylene, difluoromethylene, methylene, difluoro, spirocyclopropyl, dimethyl; and/or unshown H of two $R_{14}$ are replaced by D.

**Embodiment 10.1.5:** the compound of formula (I) according to Embodiment 10.1, a stereoisomer, tautomer, stable isotopic variant, pharmaceutically acceptable salt or solvate thereof, which has the following formula:

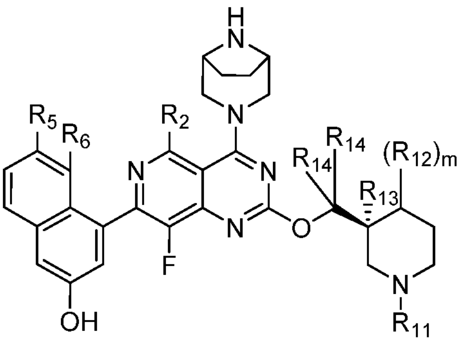

(I-A-1''''),

e.g.,

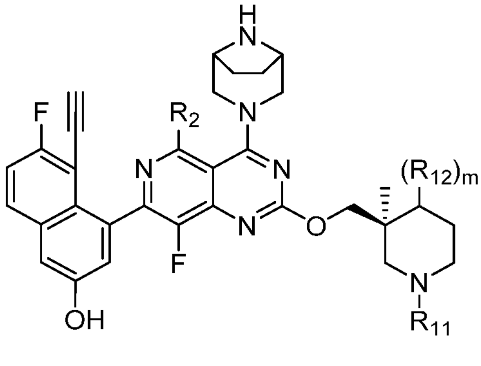

,

wherein $R_2$, $R_5$, $R_6$, $R_{13}$, $R_{14}$, $R_{11}$ and $R_{12}$ are each as defined generally or specifically in the above respective Embodiments; preferably, $R_5$ is halogen, preferably F; and/or $R_6$ is -$C_{2-6}$ alkynyl, preferably ethynyl; and/or $R_{14}$ is each independently selected from H and D; $R_{13}$ is -$C_{1-6}$ alkyl, preferably -$C_{1-3}$ alkyl; and/or $R_2$ is selected from H, -$C_{2-6}$ alkynyl, -$C_{1-6}$ alkyl optionally substituted by halogen or D, and - O-$C_{1-6}$ alkyl optionally substituted by halogen or D; and/or $R_{11}$ is -$C_{1-6}$ alkyl optionally substituted by -O-C $_{1-6}$ alkyl, halogen or D, preferably -$C_{1-3}$ alkyl optionally substituted by one or more D, preferably -$CD_3$, methyl, ethyl; and/or $R_{12}$ is selected from halogen, CN, -$C_{1-6}$ alkyl optionally substituted by halogen, -$C_{2-6}$ alkynyl optionally substituted by halogen, and -O-$C_{1-6}$ alkyl optionally substituted by halogen, or two $R_{12}$ attached to the same carbon atom form =$C(R^c)_2$ or spiro $C_{3-6}$ cycloalkyl, wherein $R^c$ is each independently selected from H, halogen and -$C_{1-6}$ alkyl optionally substituted by halogen, and m is selected from 1 or 2; wherein $R_{12}$ is preferably selected from -$C_{1-6}$ alkyl optioanlly substituted by halogen (preferably F), or two $R_{12}$ attached to the same carbon atom form =$C(R^c)_2$, wherein $R^c$ is each independently selected from H and halogen (preferably F); specific examples of $R_{12}$ include, but are not limited to fluorine, fluoromethyl, difluoromethyl, methyl, methoxy, ethynyl, cyano, fluoromethylene, difluoromethylene, methylene, difluoro, spirocyclopropyl, dimethyl; specifically, wherein

$R_2$ is selected from H, -$C_{1-6}$ alkyl optionally substituted by halogen or D, -$C_{2-6}$ alkynyl and -O-$C_{1-6}$ alkyl optionally substituted by halogen or D, wherein $R_2$ is preferably selected from H,-$C_{2-6}$ alkynyl and -O-$C_{1-6}$ alkyl optionally substituted by halogen or D; and/or $R_{11}$ is -$C_{1-6}$ alkyl optionally labeled by one or more hydrogen isotopes such as D, preferably -$CD_3$; and/or $R_{12}$ is selected from halogen, CN, -$C_{1-6}$ alkyl optionally substituted by halogen, -$C_{2-6}$ alkynyl optionally substituted by halogen, and -O-$C_{1-6}$ alkyl optionally substituted by halogen, or two $R_{12}$ attached to the same carbon atom form =$C(R^c)_2$ or spiro $C_{3-6}$ cycloalkyl, $R^c$ is independently selected from H, halogen and -$C_{1-6}$ alkyl optionally substituted by halogen, and m is selected from 1 or 2; wherein $R_{12}$ is preferably selected from -$C_{1-6}$ alkyl optionally substituted by halogen (preferably F), or two $R_{12}$ attached to the same carbon atom form =$C(R^c)_2$, wherein $R^c$ is each independently selected from H and halogen (preferably F); specific examples of $R_{12}$ include, but are not limited to fluorine, fluoromethyl, difluoromethyl, methyl, methoxy, ethynyl, cyano, fluoromethylene, difluoromethylene, methylene, difluoro, spirocyclopropyl, and dimethyl.

**Embodiment 10.1.6:** the compound of formula (I) according to Embodiment 10.1, a stereoisomer, tautomer, stable isotopic variant, pharmaceutically acceptable salt or solvate thereof, which has the following formula:

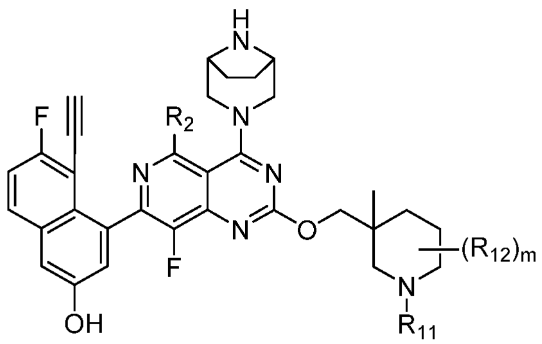

(I-A-1'''''),

wherein $R_2$, $R_{11}$ and $R_{12}$ are each as defined generally or specifically in the above respective Embodiments; $R_2$ is preferably selected from H, -$C_{1-6}$ alkyl optionally substituted by halogen and -O-$C_{1-6}$ alkyl optionally substituted by halogen; $R_{11}$ is - $C_{1-6}$ alkyl optionally substituted by -O-$C_{1-6}$ alkyl or halogen, -$C_{2-6}$ alkenyl or -$C_{3-6}$ cycloalkyl, examples include methyl, ethyl, cyclopropyl, propenyl, isopropyl, fluoroethyl; $R_{12}$ is selected from halogen, CN, -$C_{1-6}$ alkyl optionally substituted by halogen, -$C_{2-6}$ alkynyl optionally substituted by halogen, and -O-$C_{1-6}$ alkyl optionally substituted by halogen, or two $R_{12}$ attached to the same carbon atom form =$C(R^c)_2$ or spiro $C_{3-6}$ cycloalkyl, wherein $R^c$ is each independently selected from H, halogen and-$C_{1-6}$ alkyl optionally substituted by halogen, and m is selected from 1 or 2; specific examples of $R_{12}$ include fluorine, fluoromethyl, difluoromethyl, methyl, methoxy, acetylenyl, cyano, fluoromethylene, difluoromethylene, methylene, difluoro, spirocyclopropyl, dimethyl; or

$R_2$ is preferably selected from H, -$C_{1-6}$ alkyl optionally substituted by halogen, -$C_{2-6}$ alkynyl, and -O-$C_{1-6}$ alkyl optionally substituted by halogen; $R_{11}$ is -$C_{1-6}$ alkyl labeled by one or more hydrogen isotopes such as D, preferably -$CD_3$; $R_{12}$ is selected from halogen, CN, -$C_{1-6}$ alkyl optionally substituted by halogen, -$C_{2-6}$ alkynyl optionally substituted by halogen, -O-$C_{1-6}$ alkyl optionally substituted by halogen, or two $R_{12}$ attached to the same carbon atom form =$C(R^c)_2$ or spiro $C_{3-6}$ cycloalkyl, wherein $R^c$ is each independently selected from H, halogen and -$C_{1-6}$ alkyl optionally substituted by halogen, and m is selected from 1 or 2; specific examples of $R_{12}$ include fluorine, fluoromethyl, difluoromethyl, methyl, methoxy, ethynyl, cyano, fluoromethylene, difluoromethylene,

methylene, difluoro, spirocyclopropyl, dimethyl; and/or unshown H of two $R_{14}$ are replaced by D.

**Embodiment 10.1.7:** the compound of formula (I) according to Embodiment 10.1, a stereoisomer, tautomer, stable isotopic variant, pharmaceutically acceptable salt or solvate thereof, which has the following formula:

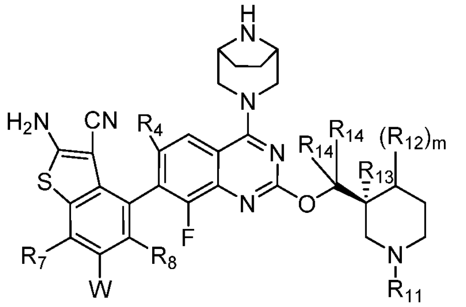

(I-A-3"), e.g.,

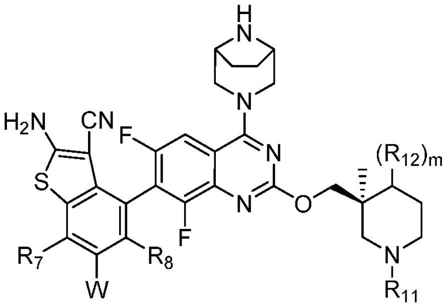

,

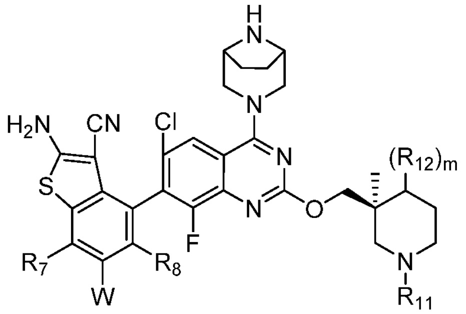

,

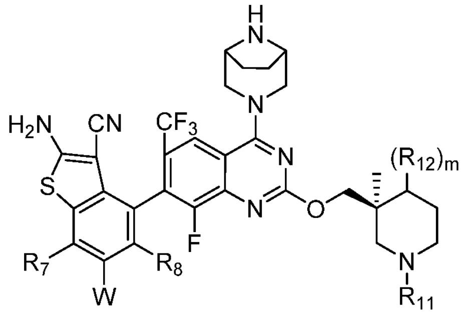

,

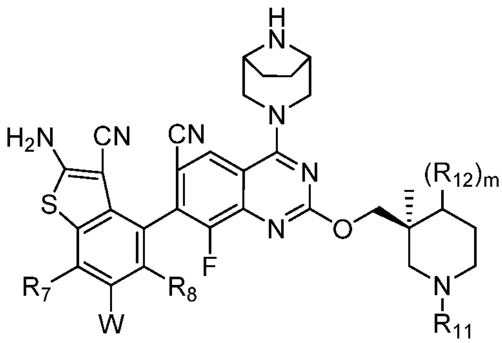

,

wherein W, $R_4$, $R_7$, $R_8$, $R_{13}$, $R_{14}$, $R_{11}$ and $R_{12}$ are each as defined generally or specifically in the above respective Embodiments; preferably, $R_{13}$ is -$C_{1-6}$ alkyl, preferably -$C_{1-3}$ alkyl; and/or $R_{14}$ is each independently H or D; and/or W is selected from H, -$C_{1-6}$ alkyl and halogen, preferably H; and/or $R_7$ and $R_8$ are both H, or both are halogen (preferably F), or one of them is H and the other is halogen (preferably F), or one of them is H and the other is $C_{1-6}$ alkyl substituted by halogen (preferably F); and/or $R_4$ is selected from halogen (preferably F or Cl), CN and -$C_{1-6}$ alkyl optionally substituted by halogen (preferably F); and/or $R_{11}$ is -$C_{1-6}$ alkyl optionally substituted by -O-$C_{1-6}$ alkyl or halogen or D, preferably -$C_{1-3}$ alkyl optionally substituted by one or more D, preferably-$CD_3$, methyl, ethyl; and/or $R_{12}$ is selected from halogen, CN, -$C_{1-6}$ alkyl optionally substituted by halogen, -$C_{2-6}$ alkynyl optionally substituted by halogen, and -O-$C_{1-6}$ alkyl optionally substituted by halogen, or two $R_{12}$ attached to the same carbon atom form =$C(R^c)_2$ or spiro $C_{3-6}$ cycloalkyl, wherein $R^c$ is each independently selected from H, halogen and -$C_{1-6}$ alkyl optionally substituted by halogen, and m is selected from 1 or 2; wherein $R_{12}$ is preferably selected from -$C_{1-6}$ alkyl substituted by halogen (preferably F), or two $R_{12}$ attached to the same carbon atom form =$C(R^c)_2$, wherein $R^c$ is each independently selected from H and halogen (preferably F); specific examples of $R_{12}$ include, but are not limited to, fluorine, fluoromethyl, difluoromethyl, methyl, methoxy, ethynyl, cyano, fluoromethylene, difluoromethylene, methylene, difluoro, spirocyclopropyl, dimethyl; specifically, wherein
W is selected from H and halogen; and/or $R_7$ and $R_8$ are both H, or both halogen (preferred F), or one of them is H and the other is halogen (preferred F), or one of them is H and the other is $C_{1-6}$ alkyl substituted by halogen

(preferred F); and/or $R_{11}$ is -$C_{1-6}$ alkyl optionally labeled by one or more hydrogen isotopes such as D, preferably -$CD_3$ ; and/or $R_{12}$ is selected from halogen, CN, -$C_{1-6}$ alkyl optionally substituted by halogen, -$C_{2-6}$ alkynyl optionally substituted by halogen, and -O-$C_{1-6}$ alkyl optionally substituted by halogen, or two $R_{12}$ attached to the same carbon atom form =$C(R^c)_2$ or spiro $C_{3-6}$ cycloalkyl, wherein $R^c$ is each independently selected from H, halogen and -$C_{1-6}$ alkyl optionally substituted by halogen, and m is selected from 1 or 2; wherein the halogen is preferably F; wherein $R_{12}$ is preferably selected from -$C_{1-6}$ alkyl substituted by halogen (preferably F), or two $R_{12}$ attached to the same carbon atom form =$C(R^c)_2$, wherein $R^c$ is each independently selected from H and halogen (preferably F); specific examples of $R_{12}$ include, but are not limited to fluorine, fluoromethyl, difluoromethyl, methyl, methoxy, ethynyl, cyano, fluoromethylene, difluoromethylene, methylene, difluoro, spirocyclopropyl, and dimethyl.

**Embodiment 10.1.8:** the compound of formula (I) according to Embodiment 10.1, a stereoisomer, tautomer, stable isotopic variant, pharmaceutically acceptable salt or solvate thereof, which has the following formula:

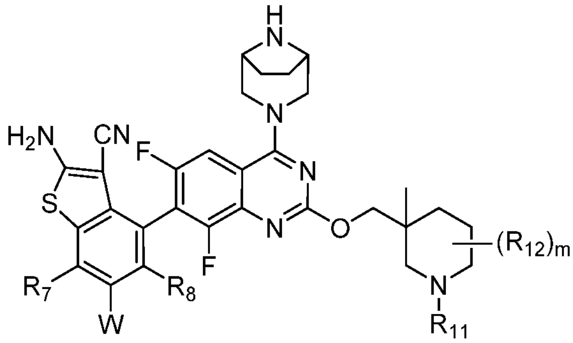
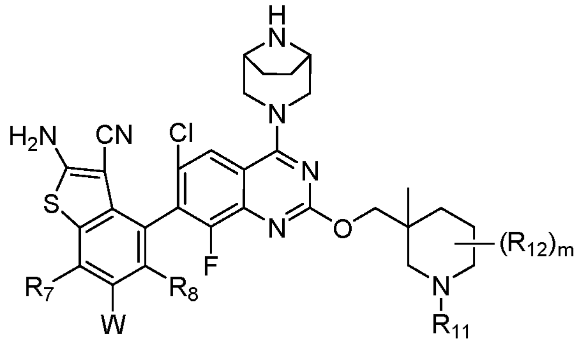
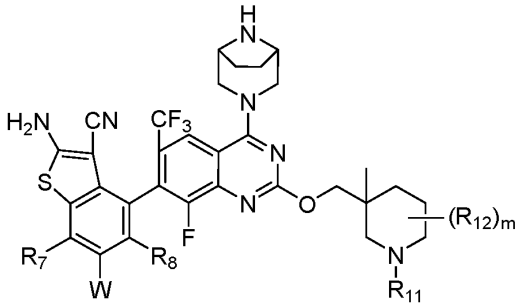
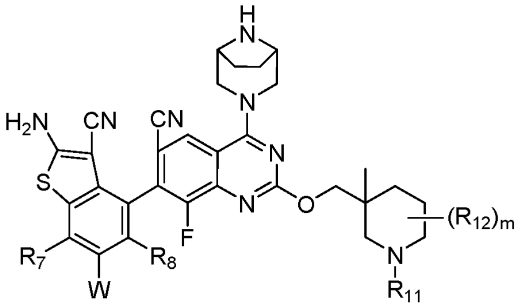
(I-A-3'''),

where W, $R_7$, $R_8$, $R_{11}$ and $R_{12}$ are each as defined generally or specifically in the above respective Embodiments; preferably, W is selected from H and halogen; $R_7$ and $R_8$ are both H, or both halogen, or one of them is H and the other is halogen, or one of them is H and the other is $C_{1-6}$ alkyl optionally substituted by halogen; $R_{11}$ is -$C_{1-6}$ alkyl optionally substituted by -O-$C_{1-6}$ alkyl or halogen, preferably -$C_{1-3}$ alkyl, preferably methyl, ethyl; $R_{12}$ is selected from halogen, CN, -$C_{1-6}$ alkyl optionally substituted by halogen, -$C_{2-6}$ alkynyl optionally substituted by halogen and -O-$C_{1-6}$ alkyl optionally substituted by halogen, or two $R_{12}$ attached to the same carbon atom form =$C(R^c)_2$ or spiro $C_{3-6}$ cycloalkyl, wherein $R^c$ is each independently selected from H, halogen, and -$C_{1-6}$ alkyl optionally substituted by halogen, and m is selected from 1 or 2, wherein halogen is preferably F; specific examples of $R_{12}$ include, but are not limited to fluorine, fluoromethyl, difluoromethyl, methyl, methoxy, acetylenyl, cyano, fluoromethylene, difluoromethylene, methylene, difluoro, spirocyclopropyl, dimethyl; or preferably

W is selected from H and halogen; $R_7$ and $R_8$ are both H, or both halogen, or one of them is H and the other is halogen, or one of them is H and the other is $C_{1-6}$ alkyl optionally substituted by halogen; $R_{11}$ is -$C_{1-6}$ alkyl labeled by one or more hydrogen isotopes such as D, and preferably -$CD_3$; $R_{12}$ is selected from halogen, CN, -$C_{1-6}$ alkyl optionally substituted by halogen, -$C_{2-6}$ alkynyl optionally substituted by halogen, and -O-$C_{1-6}$ alkyl optionally substituted by halogen, or two $R_{12}$ attached to the same carbon atom form =$C(R^c)_2$ or spiro $C_{3-6}$ cycloalkyl, wherein $R^c$ is independently selected from H, halogen, and -$C_{1-6}$ alkyl optionally substituted by halogen, and m is selected from 1 or 2, wherein the halogen is preferably F; specific examples of $R_{12}$ include, but are not limited to fluorine, fluoromethyl, difluoromethyl, methyl, methoxy, ethynyl, cyano, fluoromethylene, difluoromethylene, methylene, difluoro, spirocyclopropyl, dimethyl; and/or unshown H of two $R_{14}$ are replaced by D.

**Embodiment 10.1.9:** the compound of formula (I) according to Embodiment 10.1, a stereoisomer, tautomer, stable isotopic variant, pharmaceutically acceptable salt or solvate thereof, which has the following formula:

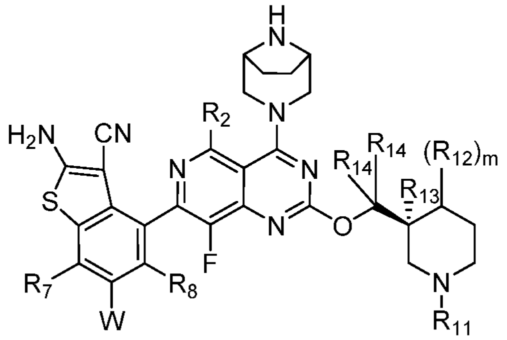

(I-A-3''''),

e.g.

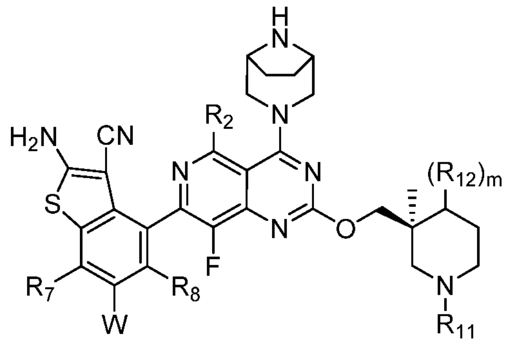

,

wherein W, $R_2$, $R_7$, $R_8$, $R_{13}$, $R_{14}$, $R_{11}$ and $R_{12}$ are each as defined generally or specifically in the above respective Embodiments; preferably, $R_{13}$ is $-C_{1-6}$ alkyl, preferably $-C_{1-3}$ alkyl; and/or $R_{14}$ is each independently H or D; and/or W is selected from H, $-C_{1-6}$ alkyl and halogen, preferably H; and/or $R_7$ and $R_8$ are both H, or both halogen (preferably F), or one of them is H and the other is halogen (preferably F), or one of them is H and the other is $C_{1-6}$ alkyl substituted by halogen (preferably F); and/or $R_2$ is selected from H, $-C_{2-6}$ alkynyl, $-C_{1-6}$ alkyl optionally substituted halogen or D, or $-O-C_{1-6}$ alkyl optionally substituted halogen or D, and/or $R_{11}$ is $-C_{1-6}$ alkyl optionally substituted by $-O-C_{1-6}$ alkyl or halogen or D, preferably $-C_{1-3}$ alkyl substituted by one or more D, preferably $-CD_3$, methyl, ethyl; and/or $R_{12}$ is selected from halogen, CN, $-C_{1-6}$ alkyl optionally substituted halogen, $-C_{2-6}$ alkynyl optionally substituted halogen, and $-O-C_{1-6}$ alkyl optionally substituted halogen, or two $R_{12}$ attached to the same carbon atom form $=C(R^c)_2$ or spiro $C_{3-6}$ cycloalkyl, wherein $R^c$ is each independently selected from H, halogen and $-C_{1-6}$ alkyl optionally substituted halogen, and m is selected from 1 or 2; wherein $R_{12}$ is preferably selected from $-C_{1-6}$ alkyl substituted by halogen (preferably F), or two $R_{12}$ attached to the same carbon atom form $=C(R^c)_2$, wherein $R^c$ is each independently selected from H and halogen (preferably F); specific examples of $R_{12}$ include, but are not limited to fluorine, fluoromethyl, difluoromethyl, methyl, methoxy, ethynyl, cyano, fluoromethylene, difluoromethylene, methylene, difluoro, spirocyclopropyl, dimethyl; specifically, wherein

W is selected from H and halogen; and/or $R_7$ and $R_8$ are both H, or both halogen (preferred F), or one of them is H and the other is halogen (preferred F), or one of them is H and the other is $C_{1-6}$ alkyl substituted by halogen (preferred F); and/or $R_2$ is selected from H, $-C_{1-6}$ alkyl optionally substituted by halogen or D, $-C_{2-6}$ alkynyl, and $-O-C_{1-6}$ alkyl optionally substituted by halogen or D, wherein $R_2$ is preferably selected from H, $-C_{2-6}$ alkynyl and $-O-C_{1-6}$ alkyl optionally substituted by halogen or D; and/or $R_{11}$ is $-C_{1-6}$ alkyl optionally labeled by one or more hydrogen isotopes such as D, preferably - $CD_3$; and/or $R_{12}$ is selected from halogen, CN, $-C_{1-6}$ alkyl optionally substituted by halogen, $-C_{2-6}$ alkynyl optionally substituted by halogen, and $-O-C_{1-6}$ alkyl optionally substituted by halogen, or two $R_{12}$ attached to the same carbon atom form $=C(R^c)_2$ or spiro $C_{3-6}$ cycloalkyl, wherein $R^c$ is each independently selected from H, halogen and $-C_{1-6}$ alkyl optionally substituted by halogen, and m is selected from 1 or 2; wherein $R_{12}$ is preferably selected from $-C_{1-6}$ alkyl substituted by halogen (preferably F), or two $R_{12}$ attached to the same carbon atom form $=C(R^c)_2$, wherein $R^c$ is each independently selected from H and halogen (preferably F); specific examples of $R_{12}$ include, but are not limited to fluorine, fluoromethyl, difluoromethyl, methyl, methoxy, ethynyl, cyano, fluoromethylene, difluoromethylene, methylene, difluoro, spirocyclopropyl, and

dimethyl.

**Embodiment 10.1.10:** the compound of formula (I) according to Embodiment 10.1, a stereoisomer, tautomer, stable isotopic variant, pharmaceutically acceptable salt or solvate thereof, which has the following formula:

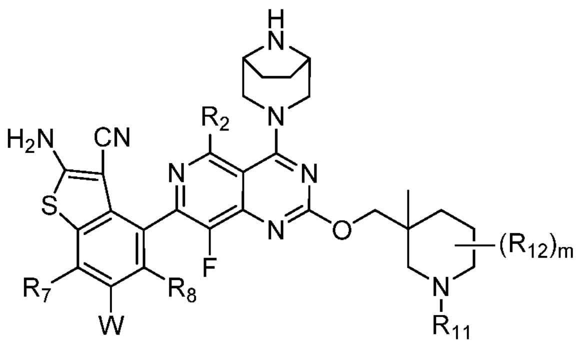

(I-A-3'''''),

wherein W, $R_2$, $R_7$, $R_8$, $R_{11}$ and $R_{12}$ are each as defined generally or specifically in the above respective Embodiments; preferably, W is selected from H and halogen; $R_7$ and $R_8$ are both H, or both halogen, or one of them is H and the other is halogen, or one of them is H and the other is $C_{1-6}$ alkyl substituted by halogen; $R_2$ is selected from H, $-C_{1-6}$ alkyl optionally substituted by halogen and $-O-C_{1-6}$ alkyl optionally substituted by halogen; $R_{11}$ is $-C_{1-6}$ alkyl optionally substituted by $-O-C_{1-6}$ alkyl or halogen, preferably $-C_{1-3}$ alkyl, preferably methyl, ethyl; $R_{12}$ is selected from halogen, CN, $-C_{1-6}$ alkyl optionally substituted by halogen, $-C_{2-6}$ alkynyl optionally substituted by halogen, and $-O-C_{1-6}$ alkyl optionally substituted by halogen, or two $R_{12}$ attached to the same carbon atom form $=C(R^c)_2$ or spiro $C_{3-6}$ cycloalkyl, wherein $R^c$ is each independently selected from H, halogen, and $-C_{1-6}$ alkyl optionally substituted by halogen, and m is selected from 1 or 2, wherein halogen is preferably F; specific examples of $R_{12}$ include, but are not limited to fluorine, fluoromethyl, difluoromethyl, methyl, methoxy, acetylenyl, cyanyl, fluoromethylene, difluoromethylene, methylene, difluoro, spirocyclopropyl, dimethyl; or preferably W is selected from H and halogen; $R_7$ and $R_8$ are both H, or both halogen, or one of them is H and the other is halogen, or one of them is H and the other is $C_{1-6}$ alkyl optionally substituted by halogen; $R_2$ is selected from H, $-C_{1-6}$ alkyl optionally substituted by halogen, $-C_{2-6}$ alkynyl, and $-O-C_{1-6}$ alkyl optionally substituted by halogen; $R_{11}$ is $-C_{1-6}$ alkyl labeled by one or more hydrogen isotopes such as D, preferably $-CD_3$; $R_{12}$ is selected from halogen, CN, $-C_{1-6}$ alkyl optionally substituted by halogen, $-C_{2-6}$ alkynyl optionally substituted by halogen, and $-O-C_{1-6}$ alkyl optionally substituted by halogen, or two $R_{12}$ attached to the same carbon atom form $=C(R^c)_2$ or spiro $C_{3-6}$ cycloalkyl, wherein $R^c$ is each independently selected from H, halogen, and $-C_{1-6}$ alkyl optionally substituted by halogen, and m is selected from 1 or 2, wherein halogen is preferably F; specific examples of $R_{12}$ include, but are not limited to fluorine, fluoromethyl, difluoromethyl, methyl, methoxy, ethynyl, cyano, fluoromethylene, difluoromethylene, methylene, difluoro, spirocyclopropyl, dimethyl; and/or unshown H of two $R_{14}$ are replaced by D.

**Embodiment 10.2:** the compound of formula (I) according to Embodiment 1, a stereoisomer, tautomer, stable isotopic variant, pharmaceutically acceptable salt or solvate thereof, which has the following formula:

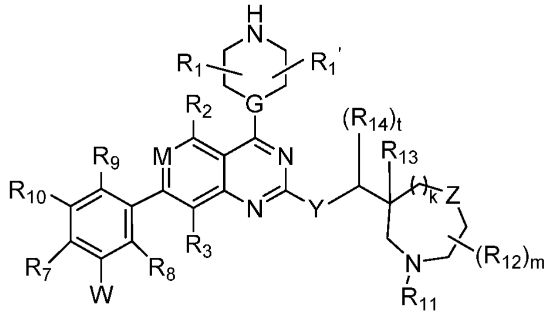

(I-B)

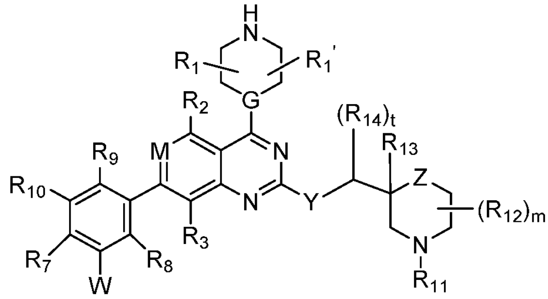

(I-B-1)

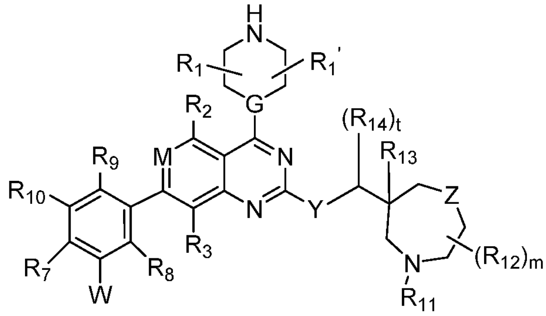

(I-B-2)

Wherein each substituent has the meanings defined in the corresponding embodiments described above.

**Embodiment 10.2.1:** the compound of formula (I) according to Embodiment 10.2, a stereoisomer, tautomer, stable isotopic variant, pharmaceutically acceptable salt or solvate thereof, wherein:

Yis O;

G is CH or N;

M is C-$R_4$;

Z is selected from C, Se and O;

W is -OH or -$NH_2$;

$R_1$ and $R_1$' together form -$(CH_2)_t$-;

$R_2$ is H;

$R_3$ is halogen;

$R_4$ is halogen;

$R_7$ is H;

$R_8$ is selected from H, CN, halogen, $NO_2$, -$C_{1-6}$ alkyl, -$N(R^a)_2$, -C(O)$N(R^a)_2$ and-C(O)$OR^a$, wherein - $C_{1-6}$ alkyl is optionally substituted by halogen or -$N(R^a)_2$, wherein $R^a$ is selected from H and -$C_{1-6}$ alkyl optionally substituted by halogen;

$R_9$ is selected from -$Si(R^b)_3$, -$C_{1-6}$ alkyl, and -$(CH_2)_n$-$C_{3-6}$ cycloalkyl, wherein -$C_{1-6}$ alkyl and -$C_{3-6}$ cycloalkyl are each independently and optionally substituted by halogen, -Se-$C_{1-6}$ alkyl and -$C_{1-6}$ alkyl optionally substituted by halogen, and $R_b$ is selected from -$C_{1-6}$ alkyl and -$C_{2-6}$ alkenyl, each optionally substituted by halogen;

$R_{10}$ is selected from halogen and -$C_{1-6}$ alkyl;

$R_{11}$ is -$C_{1-6}$ alkyl optionally substituted by -O-$C_{1-6}$ alkyl or halogen;

$R_{12}$ is selected from H, halogen, CN, -$C_{1-6}$ alkyl optionally substituted by halogen, -$C_{2-6}$ alkynyl optionally substituted by halogen and -O-$C_{1-6}$ alkyl optionally substituted by halogen, or two $R_{12}$ attached to the same carbon atom form =$C(R^c)_2$, wherein $R^c$ is each independently selected from H, halogen and -$C_{1-6}$ alkyl optionally substituted by halogen;

$R_{13}$ is selected from H, halogen and -$C_{1-6}$ alkyl optionally substituted by halogen;

$R_{14}$ is H;

k is selected from 0 or 1;

m and n are each independently selected from integers 0 to 2; and

t is selected from 1 or 2.

**Embodiment 10.2.2:** the compound of formula (I) according to Embodiment 10.2 or 10.2.1, a stereoisomer, tautomer, stable isotopic variant, pharmaceutically acceptable salt or solvate thereof, which has the following sub-general formula:

| | |
|---|---|
| 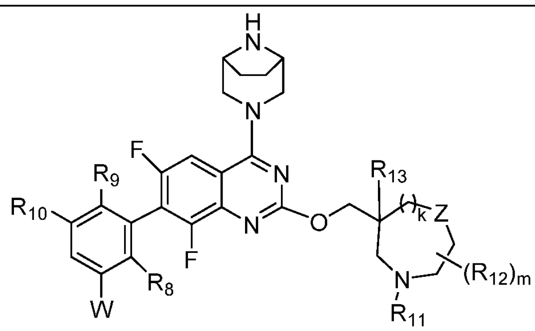 (I-B') | |
| 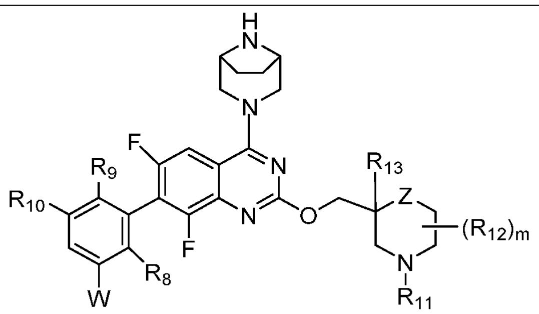 (I-B-1') | 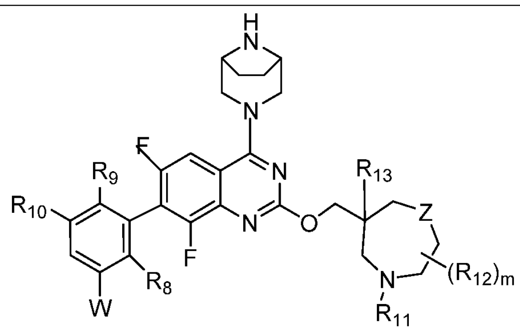 (I-B-2') |

wherein:

Z is selected from C and O;

W is -OH or -$NH_2$;

$R_8$ is selected from H, CN, halogen, $NO_2$, -$C_{1-6}$ alkyl, -$N(R^a)_2$, -$C(O)N(R^a)_2$ and -$C(O)OR^a$, wherein -$C_{1-6}$ alkyl is optionally substituted by halogen or -$N(R^a)_2$, wherein $R^a$ is selected from H and -$C_{1-6}$ alkyl optionally substituted by halogen;

$R_9$ is selected from $-Si(R^b)_3$, $-C_{1-6}$ alkyl, and $-C_{3-6}$ cycloalkyl, wherein $-C_{1-6}$ alkyl and $-C_{3-6}$ cycloalkyl are each independently and optionally substituted by halogen or $-Se-C_{1-6}$ alkyl, and $R_b$ is selected from $-C_{1-6}$ alkyl and $-C_{2-6}$ alkenyl;

$R_{10}$ is selected from halogen and $-C_{1-6}$ alkyl, preferably Cl and $-CH_3$;

$R_{11}$ is $-C_{1-6}$ alkyl optionally substituted by $-O-C_{1-6}$ alkyl or halogen, preferably $-C_{1-6}$ alkyl, more preferably $-CH_3$;

$R_{12}$ is selected from H, halogen, CN, $-C_{1-6}$ alkyl optionally substituted by halogen, $-C_{2-6}$ alkynyl optionally substituted by halogen and $-O-C_{1-6}$ alkyl, preferably H, F, CN, $-C{\equiv}CH$, $-CH_2F$, $-CHF_2$, $CH_3$ and $-O-CH_3$, or two $R_{12}$ attached to the same carbon atom form $=C(R^c)_2$ , wherein $R^c$ is each independently selected from H, halogen and $-C_{1-6}$ alkyl optionally substituted by halogen, preferably $=CH_2$, $=CF_2$, $= CCl_2$, $=C(CH_3)_2$, $=C(CF_3)_2$;

$R_{13}$ is selected from H, halogen and $-C_{1-6}$ alkyl optionally substituted by halogen, preferably H, F and $-CH_3$;

$R_{14}$ is H;

k is selected from 0 or 1; and

m is selected from integers from 0 to 2.

**Embodiment 10.2.3:** the compound of formula (I) according to Embodiment 10.2, a stereoisomer, tautomer, stable isotopic variant, pharmaceutically acceptable salt or solvate thereof, which has the following formula:

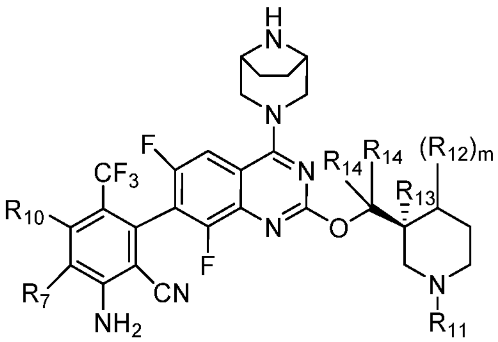

(I-B-1"),

e.g.,

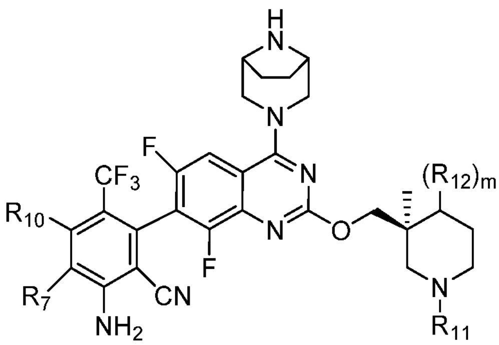

,

wherein $R_7$, $R_{10}$, $R_{13}$, $R_{14}$, $R_{11}$ and $R_{12}$ are each as defined generally or specifically in the above respective Embodiments; preferably, $R_{13}$ is $-C_{1-6}$ alkyl, preferably $-C_{1-3}$ alkyl; and/or $R_{14}$ is each independently H or D; and/or $R_7$ is selected from H and halogen; and/or $R_{10}$ is selected from $-C_{1-6}$ alkyl and halogen; and/or $R_{11}$ is $-C_{1-6}$ alkyl optionally substituted by $-O-C_{1-6}$ alkyl or halogen or D, preferably $-C_{1-3}$ alkyl optionally substituted by one or more D, preferably $-CD_3$, methyl, ethyl; and/or $R_{12}$ is selected from halogen, CN, $-C_{1-6}$ alkyl optionally substituted by

halogen, -$C_{2-6}$ alkynyl optionally substituted by halogen and -O-$C_{1-6}$ alkyl optionally substituted by halogen, or two $R_{12}$ attached to the same carbon form =$C(R^c)_2$ or spiro $C_{3-6}$ cycloalkyl, wherein $R^c$ is each independently selected from H, halogen and -$C_{1-6}$ alkyl optionally substituted by halogen, and m is selected from 1 or 2; wherein $R_{12}$ is preferably selected from -$C_{1-6}$ alkyl optionally substituted by halogen, or two $R_{12}$ attached to the same carbon form =$C(R^c)_2$, wherein $R^c$ is each independently selected from H and halogen; wherein the halogen is preferably F or Cl; specific examples of $R_{12}$ include, but are not limited to fluorine, fluoromethyl, difluoromethyl, methyl, methoxy, ethynyl, cyano, fluoromethylene, difluoromethylene, methylene, difluoro, spirocyclopropyl, dimethyl.

**Embodiment 10.2.4:** the compound of formula (I) according to Embodiment 10.2, a stereoisomer, tautomer, stable isotopic variant, pharmaceutically acceptable salt or solvate thereof, which has the following formula:

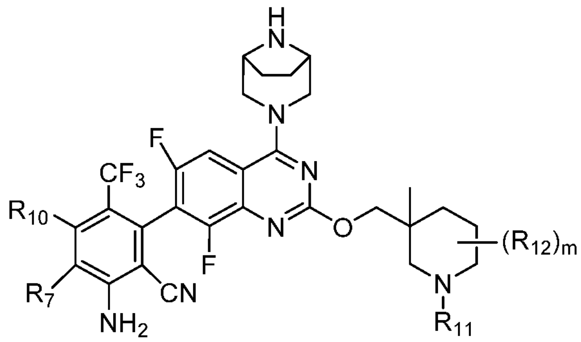

(I-B-1'''),

where $R_7$, $R_{10}$, $R_{11}$ and $R_{12}$ are each as defined generally or specifically in the above respective Embodiments; preferably, $R_7$ is selected from H and halogen; $R_{10}$ is selected from -$C_{1-6}$ alkyl and halogen; $R_{11}$ is selected from -$C_{1-6}$ alkyl optionally substituted by -O-$C_{1-6}$ alkyl or halogen, preferably -$C_{1-3}$ alkyl, preferably methyl, ethyl; $R_{12}$ is selected from halogen, CN, -$C_{1-6}$ alkyl optionally substituted by halogen, -$C_{2-6}$ alkynyl optionally substituted by halogen and -O-$C_{1-6}$ alkyl optionally substituted by halogen, or two $R_{12}$ attached to the same carbon form =$C(R^c)_2$ or spiro $C_{3-6}$ cycloalkyl, wherein $R^c$ is each independently selected from H, halogen and -$C_{1-6}$ alkyl optionally substituted by halogen, and m is selected from 1 or 2; wherein the halogen is preferably F or Cl; specific examples of $R_{12}$ include, but are not limited to fluorine, fluoromethyl, difluoromethyl, methyl, methoxy, ethynyl, cyano, fluoromethylene, difluoromethylene, methylene, difluoro, spirocyclopropyl, dimethyl.

**Embodiment 10.2.5:** the compound of formula (I) according to Embodiment 10.2, a stereoisomer, tautomer, stable isotopic variant, pharmaceutically acceptable salt or solvate thereof, which has the following formula:

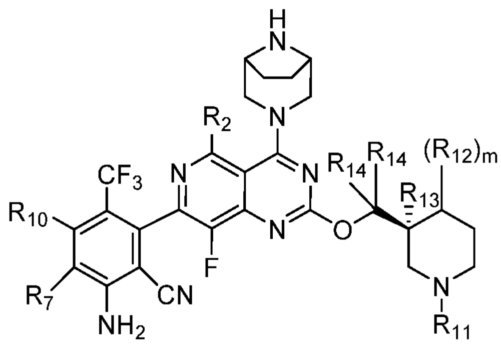

(I-B-1''''),

e.g.

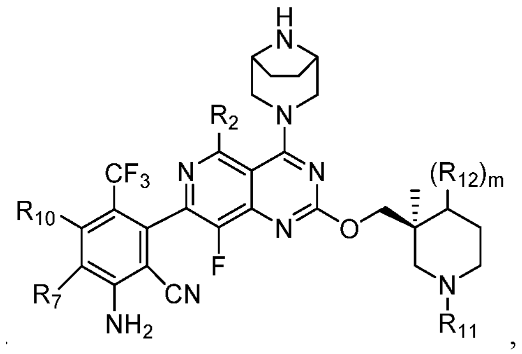

where $R_2$, $R_7$, $R_{10}$, $R_{13}$, $R_{14}$, $R_{11}$ and $R_{12}$ are each as defined generally or specifically in the above respective Embodiments; preferably, $R_{13}$ is -$C_{1-6}$ alkyl, preferably -$C_{1-3}$ alkyl; and/or $R_{14}$ is independently H or D; and/or $R_2$ is selected from H, -$C_{2-6}$ alkynyl, -$C_{1-6}$ alkyl optionally substituted by halogen or D, and -O-$C_{1-6}$ alkyl optionally substituted by halogen or D; and/or $R_7$ is selected from H and halogen; and/or $R_{10}$ is selected from -$C_{1-6}$ alkyl and halogen; and/or $R_{11}$ is -$C_{1-3}$ alkyl optionally substituted by one or more D, preferably -$CD_3$, methyl, ethyl; and/or $R_{12}$ is selected from halogen, CN, -$C_{1-6}$ alkyl optionally substituted by halogen, -$C_{2-6}$ alkynyl optionally substituted by halogen, and -O-$C_{1-6}$ alkyl optionally substituted by halogen, or two $R_{12}$ attached to the same carbon atom form =$C(R^c)_2$ or spiro $C_{3-6}$ cycloalkyl, wherein $R^c$ is each independently selected from H, halogen and -$C_{1-6}$ alkyl optionally substituted by halogen, and m is selected from 1 or 2; wherein $R_{12}$ is preferably selected from -$C_{1-6}$ alkyl optionally substituted by halogen, or two $R_{12}$ attached to the same carbon atom form =$C(R^c)_2$, wherein $R^c$ is each independently selected from H and halogen; wherein the halogen is preferably F or Cl; specific examples of $R_{12}$ include, but are not limited to fluorine, fluoromethyl, difluoromethyl, methyl, methoxy, acetylenyl, cyano, fluoromethylene, difluoromethylene, methylene, difluoro, spirocyclopropyl, dimethyl.

**Embodiment 10.2.6:** the compound of formula (I) according to Embodiment 10.2, a stereoisomer, tautomer, stable isotopic variant, pharmaceutically acceptable salt or solvate thereof, which has the following formula:

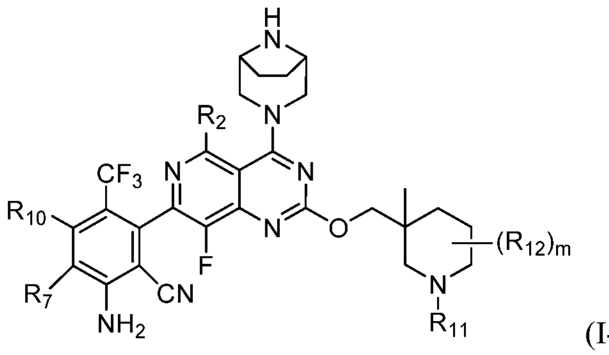

(I-B-1""") ,

wherein $R_2$, $R_7$, $R_{10}$, $R_{11}$ and $R_{12}$ are each as defined generally or specifically in the above respective Embodiments; preferably, $R_2$ is selected from H, -$C_{1-6}$ alkyl optionally substituted by halogen, and -O-$C_{1-6}$ alkyl optionally substituted by halogen; $R_7$ is selected from H and halogen; $R_{10}$ is selected from -$C_{1-6}$ alkyl and halogen; $R_{11}$ is -$C_{1-6}$ alkyl optionally substituted by -O-$C_{1-6}$ alkyl or halogen, preferably -$C_{1-3}$ alkyl, preferably methyl, ethyl; $R_{12}$ is selected from halogen, CN, -$C_{1-6}$ alkyl optionally substituted by halogen, -$C_{2-6}$ alkynyl optionally substituted by halogen, and -O-$C_{1-6}$ alkyl optionally substituted by halogen, or two $R_{12}$ attached to the same carbon atom form =$C(R^c)_2$ or spiro $C_{3-6}$ cycloalkyl, wherein $R^c$ is each independently selected from H, halogen and -$C_{1-6}$ alkyl optionally substituted by halogen, and m is selected from 1 or 2; wherein the halogen is preferably F or Cl; specific examples of $R_{12}$ include but are not limited to fluorine, fluoromethyl, difluoromethyl, methyl, methoxy, ethynyl, cyano, fluoromethylene, difluoromethylene, methylene, difluoro, spirocyclopropyl, dimethyl.

**Embodiment 10.2.7:** the compound of formula (I) according to Embodiment 10.2 or 10.2.6, a stereoisomer, tautomer, stable isotopic variant, pharmaceutically acceptable salt or solvate thereof, wherein $R_{11}$ is -$C_{1-6}$ alky labeled by one or more hydrogen isotopes such as D, preferably -$CD_3$; and/or unshown H of two $R_{14}$ are replaced by D.

**Embodiment 11:** the compound, selected from the following compounds of examples or a pharmaceutically acceptable salt or solvate thereof.

It should be noted that the compounds of the present invention encompass each of the above independent embodiments or each of the specific embodiments, and also encompass any combination or sub-combination of the above embodiments or specific embodiments to form an embodiment, and also encompass the embodiments formed by any combination of the above preferred or exemplified embodiments.

## Beneficial effects of the invention

As described previously, it is known that Ras mutant proteins, especially KRas mutant proteins, play a role in tumorigenesis and a variety of other diseases. We have surprisingly found that the compounds of the present invention with the above structural features can potently inhibit cell proliferation in cell lines carrying KRas mutant proteins (such as G12C, G12D, G12V, G12A, G12R, G12S and G13D mutant proteins), especially the KRas-G12D mutant protein, and thus have potential value as anti-proliferative, pro-apoptotic and/or anti-invasive drugs in the prevention, containment and/or treatment of related tumor diseases. In particular, the compounds of the present invention are expected to be used for preventing or treating those diseases or conditions mediated by Ras mutant proteins (such as G12C, G12D, G12V, G12A, G12R, G12S and G13D mutant proteins), especially the KRas-G12D mutant protein, or those diseases or conditions that benefit from the inhibition of Ras mutations (such as G12C, G12D, G12V, G12A, G12R, G12S and G13D mutation), especially the KRas-G12D mutant protein, such as the cancers or tumors as defined herein.

Specifically, through research, it has been found that the compounds of the present invention can achieve one or more of the following technical effects:

- High mutant protein inhibitory activity: The compounds of the present invention, especially the compounds specifically exemplified in the context herein, exhibit proliferation inhibitory activity against KRas G12D mutant cells in the KRAS G12D mutant cell AGS cell proliferation inhibition assay, with $IC_{50}$ value ranging from 10 pM ~ 10 μM, such as, 10 pM ~ 5 μM, 100 pM ~ 5 μM, 500 pM ~ 1 μM, 0.001 ~ 10 μM, 100 pM ~ 1 μM, 100 pM ~ 0.5 μM, 0.001 ~ 5 μM, 0.01 ~ 1 μM, preferably 100 pM ~ 1 μM, 0.001 ~ 0.5 μM, more preferably 100 pM ~ 0.5 μM, 0.001 ~ 0.1 μM, and most preferably 1 ~ 50 nM, 100 pM ~ 0.1 μM, as shown in the Activity Example 1.
- The compounds of the present invention, especially the compounds specifically exemplified in the context herein, also exhibit potent proliferation inhibitory activity against KRas G12D mutant cells in the KRAS G12D mutant cell AGS (3D) cell proliferation inhibition assay, with $IC_{50}$ value ranging from 0.001 ~ 5 μM, such as 0.001 ~ 1 μM, preferably 0.001 ~ 0.5 μM, more preferably 0.001 ~ 0.1 μM, and most preferably less than 1 ~ 50 nM, as shown in Activity Example 5.
- Favorable pharmacokinetic properties, such as a longer $t_{1/2}$, which, for example, allows for an extended dosing interval. Longer half-life, allowing patients to have better compliance; having the best comprehensive safety/activity effect in terms of $AUC_{0-t}$ data, having better drug-likeness, and higher bioavailability, as shown in Activity Example 2; and
- Remarkably satisfactory safety, a reduced risk of drug interactions, and no significant inhibitory effect on key CYP subtypes involved in drug metabolism, as shown in Activity Example 3; and
- Excellent in vivo pharmacodynamic properties, significantly inhibiting tumor volume while having good safety. For example, it demonstrates excellent target-related tumor inhibitory activity in the human pancreatic cancer AsPC-1 xenograft mouse model and the human pancreatic cancer cell HPAC subcutaneous xenograft tumor NOD/SCID mouse model, and there is no significant change in the body weight of the mice, as shown in Activity Examples 4 and 7.

Based on the beneficial effects of the compounds of the present invention described above, the present invention also provides the following technical solutions in various aspects.

## Compounds of the present invention for treatment or as medicine

In one aspect, the present invention provides the compounds of the present invention, preferably their pharmaceutically acceptable salts or solvates, for use as medicine.

In another aspect, the present invention provides the compounds of the present invention, preferably their pharmaceutically acceptable salts or solvates, for use as inhibitors of KRas mutant proteins (such as G12C, G12D, G12V, G12A, G12R, G12S, and G13D mutation proteins), more specifically as RAS G12D inhibitors.

In yet another aspect, the present invention provides the compounds of the present invention, preferably their pharmaceutically acceptable salts or solvates, for the treatment and/or prevention of diseases or disorders mediated by Ras mutant proteins, specifically KRas mutant proteins (such as G12C, G12D, G12V, G12A, G12R, G12S, and G13D mutation proteins), more specifically KRAS G12D mutant protein, or diseases or disorders that benefit from the inhibition of

Ras mutations, specifically KRas mutant proteins (such as G12C, G12D, G12V, G12A, G12R, G12S, and G13D mutation proteins), more specifically KRAS G12D mutant protein.

In a specific embodiment, the present invention provides the compounds of the present invention for the treatment and/or prevention of diseases in which Ras mutant proteins, specifically KRas mutant proteins (such as G12C, G12D, G12V, G12A, G12R, G12S and G13D mutation proteins), more specifically KRAS G12D mutant protein, promote the occurrence and development of the diseases, or in which the inhibition of Ras mutant proteins, specifically KRas mutant proteins (such as G12C, G12D, G12V, G12A, G12R, G12S and G13D mutation proteins), more specifically KRAS G12D mutant protein, will reduce the incidence of the diseases and decrease or eliminate the symptoms of the diseases. Such diseases include, for example, tumors or cancers, including but not limited to: lung cancer, lung adenocarcinoma, bone cancer, pancreatic cancer, skin cancer, head and neck cancer, cutaneous or intraocular melanoma, uterine cancer, ovarian cancer, rectal cancer, cancer of the anal region, gastric cancer, colon cancer, breast cancer, fallopian tube cancer, endometrial cancer, cervical cancer, vaginal cancer, vulvar cancer, Hodgkin's disease, esophageal cancer, small intestine cancer, cancer of the endocrine system, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or ureteral cancer, renal cell carcinoma, renal pelvic cancer, central nervous system (CNS) tumors, primary CNS lymphoma, spinal tumors, brainstem glioma or pituitary adenoma.

The present invention especially provides compounds of formula (I) or their isomers, and their pharmaceutically acceptable salts or solvates, which can be used for the treatment of patients suffering from pancreatic cancer, colon cancer, rectal cancer, lung adenocarcinoma, lung cancer, cholangiocarcinoma, endometrial cancer, ovarian cancer, leukemia; most preferably for the treatment of patients selected from those with pancreatic cancer, colon cancer, rectal cancer, lung adenocarcinoma, cholangiocarcinoma.

## Pharmaceutical compositions and their administration

In another aspect, the present invention provides a pharmaceutical composition comprising a compound of formula (I) as defined above, preferably a pharmaceutically acceptable salt or solvate thereof, and a pharmaceutically acceptable carrier or excipient. The pharmaceutical composition of the present invention can be used for treating or preventing diseases mediated by Ras mutations, especially KRas mutations, such as diseases mediated by KRas G12C, KRas G12D, KRas G12V, G12A, G12R, G12S or KRas G13D mutations, especially KRas G12D mutations, such as tumors or cancers.

The pharmaceutical composition of the present invention described above can be formulated by techniques known to those skilled in the art, such as the techniques disclosed in the 20th edition of Remington's Pharmaceutical Sciences. For example, it can be formulated into tablets, powders, capsules, lozenges, granules, solutions, dispersions, suspensions, syrups, sprays, suppositories, gels, emulsions, patches, etc. The composition may contain conventional components in pharmaceutical formulations, such as diluents (e.g., glucose, lactose or mannitol), carriers, pH regulators, buffers, sweeteners, fillers, stabilizers, surfactants, wetting agents, lubricants, emulsifiers, suspending agents, preservatives, antioxidants, opacifying agents, glidants, processing aids, colorants, perfumes, flavoring agents, other known additives, and other active agents. Suitable carriers and excipients are well known to those skilled in the art and are described in detail, for example, in Ansel, Howard C., et al., Ansel's Pharmaceutical Dosage Forms and Drug Delivery Systems. Philadelphia: Lippincott, Williams & Wilkins, 2004.

The administration and application of the pharmaceutical composition of the present invention are in accordance with good medical practice. Factors to be considered in this context include the specific disorder being treated, the specific mammal being treated, the clinical condition of the individual patient, the cause of the disorder, the site of agent delivery, the method of administration, the administration schedule, and other factors well known to physician practitioners. The optimal dosage level and administration frequency of the compound or pharmaceutical composition of the present invention can be determined by those skilled in the art through standard tests in the field of pharmaceutical research.

The composition of the present invention can be administered in any suitable manner, including orally, topically (including buccal and sublingual), rectally, vaginally, transdermally, parenterally, subcutaneously, intraperitoneally, intratracheally, intradermally, intrathecally, by inhalation, epidurally, and intranasally, and for local treatment if desired, it can also be administered intralesionally. Parenteral infusion includes intramuscular, intravenous, intraarterial, intraperitoneal or subcutaneous administration. In some embodiments, the pharmaceutical composition of the present invention is administered orally.

For a human subject weighing 70 kg, the suitable dosage range of the compound of the present invention can be routinely determined by those skilled in the art, for example, it can be 1-1000 mg per day.

When the dosage of a drug or its pharmaceutically acceptable salt is described herein, it should be understood that the dosage is based on the weight of the free base and does not include any hydrates or solvates thereof, unless it is indicated in the specification that the dosage is based on the weight of the salt, hydrate or solvate.

## Methods of treatment and uses

As described above, the compounds of the present invention and the compounds of various specific embodiments thereof, especially the compounds specifically prepared and characterized in the examples, exhibit inhibitory effects on Ras mutations, especially KRas mutations, such as KRas G12C, KRas G12D, KRas G12V, G12A, G12R, G12S or KRas G13D mutations, especially KRas G12D mutations.

Therefore, in another aspect, the present invention provides a method for inhibiting Ras mutations, especially KRas mutations, preferably KRas G12D mutations in cells, which comprises contacting the cells with a compound of the present invention, preferably a pharmaceutically acceptable salt or solvate thereof, to inhibit the activity of Ras mutations, especially KRas mutations (such as G12C mutation, G12D mutation, G12V mutation, G12A mutation, G12R mutation, G12S mutation and G13D mutation), preferably KRas G12D mutation in the cells.

Based on the same properties, the present invention also correspondingly provides a method for inhibiting abnormal cell growth in a mammal, which comprises administering to the mammal a therapeutically effective amount of a compound of the present invention, preferably a pharmaceutically acceptable salt or solvate thereof, or a pharmaceutical composition comprising a compound of the present invention, preferably a pharmaceutically acceptable salt or solvate thereof.

In another aspect, the present invention provides a method for treating and/or preventing a disease mediated by a Ras mutation, especially a KRas mutation (such as G12C mutation, G12D mutation, G12V mutation, G12A mutation, G12R mutation, G12S mutation and G13D mutation), preferably a KRas G12D mutation, which comprises administering to a subject in need thereof a therapeutically effective amount of a compound of the present invention, preferably a pharmaceutically acceptable salt or solvate thereof, or a pharmaceutical composition comprising a compound of the present invention, preferably a pharmaceutically acceptable salt or solvate thereof.

In another aspect, the present invention provides the use of a compound of the present invention, preferably a pharmaceutically acceptable salt or solvate thereof, or a pharmaceutical composition comprising a compound of the present invention, preferably a pharmaceutically acceptable salt or solvate thereof, for inhibiting Ras mutations, especially KRas mutations, preferably KRas G12D mutations in cells, or for inhibiting abnormal cell growth in a mammal, or for treating and/or preventing a disease mediated by a Ras mutation, especially a KRas mutation, preferably KRas G12C, KRas G12D, KRas G12V, KRasG12A, KRasG12R, KRasG12S or KRas G13D, most preferably KRas G12D mutation.

In another aspect, the present invention provides the use of a compound of the present invention, preferably a pharmaceutically acceptable salt or solvate thereof, or a pharmaceutical composition comprising a compound of the present invention, preferably a pharmaceutically acceptable salt or solvate thereof, in the preparation of a medicament for treating and/or preventing a disease mediated by a Ras mutation, especially a KRas mutation (such as G12C mutation, G12D mutation, G12V mutation, G12A mutation, G12R mutation, G12S mutation and G13D mutation), preferably a KRas G12D mutation.

For each of the technical solutions of the methods and uses provided by the present invention described above, the abnormal cell growth or the disease mediated by a Ras mutation, especially a KRas mutation, preferably KRas G12C, KRas G12D, KRas G12V, KRasG12A, KRasG12R, KRasG12S or KRas G13D, most preferably KRas G12D mutation especially refers to cancer or tumor. Exemplary cancers or tumors include but are not limited to lung cancer, lung adenocarcinoma, bone cancer, pancreatic cancer, skin cancer, head and neck cancer, cutaneous or intraocular melanoma, uterine cancer, ovarian cancer, rectal cancer, cancer of the anal region, gastric cancer, colon cancer, breast cancer, fallopian tube cancer, endometrial cancer, cervical cancer, vaginal cancer, vulvar cancer, Hodgkin's disease, esophageal cancer, small intestine cancer, cancer of the endocrine system, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or ureteral cancer, renal cell carcinoma, renal pelvic cancer, central nervous system (CNS) tumors, primary CNS lymphoma, spinal tumors, brainstem glioma or pituitary adenoma.

For each of the technical solutions of the methods and uses provided by the present invention described above, the abnormal cell growth or the disease mediated by a Ras mutation, especially a KRas mutation (such as G12C mutation, G12D mutation, G12V mutation, G12A mutation, G12R mutation, G12S mutation and G13D mutation), preferably KRas G12D is preferably selected from pancreatic cancer, colon cancer, rectal cancer, lung adenocarcinoma, lung cancer, cholangiocarcinoma, endometrial cancer, ovarian cancer, leukemia; most preferably selected from pancreatic cancer, colon cancer, rectal cancer, lung adenocarcinoma, cholangiocarcinoma.

Therefore, in a preferred embodiment of this aspect, the present invention provides each of the above technical solutions of the methods and uses for treating or preventing cancer or tumor by inhibiting KRas G12V and/or KRas-G12D mutations. In a further preferred embodiment, the present invention provides each of the above technical solutions of the methods and uses for treating or preventing pancreatic cancer, colon cancer, rectal cancer, lung adenocarcinoma and cholangiocarcinoma by inhibiting KRas-G12D mutations.

The present invention also provides the use of a compound of the present invention, preferably a pharmaceutically acceptable salt or solvate thereof, as a KRas inhibitor (such as an inhibitor of G12C mutation, G12D mutation, G12V

mutation, G12A mutation, G12R mutation, G12S mutation and G13D mutation) in research, especially as a research tool compound for inhibiting KRas G12D. Therefore, the present invention relates to the in vitro use of a compound of the present invention, preferably a pharmaceutically acceptable salt or solvate thereof, as a KRas inhibitor, especially a KRas G12D inhibitor, and particularly relates to the in vitro use of a compound of the present invention, preferably a pharmaceutically acceptable salt or solvate thereof, as a research tool compound that acts as a KRas inhibitor, especially a KRas G12D inhibitor. The present invention also relates to a method for inhibiting KRas (such as G12C mutation, G12D mutation, G12V mutation, G12A mutation, G12R mutation, G12S mutation and G13D mutation), especially KRas G12D, especially an in vitro method, which comprises administering a compound of the present invention, preferably a pharmaceutically acceptable salt or solvate thereof, to a sample (such as a biological sample). It should be understood that the term "in vitro" is used in this specific context with the meaning of "outside a living human body or animal body", which specifically includes experiments performed with cells, cell or subcellular extracts and/or biomolecules in an artificial environment, for example, in an aqueous solution or culture medium that can be provided in a flask, test tube, culture dish, microtiter plate, etc.

## Pharmaceutical combinations

The compounds of the present invention can be administered as the sole active ingredient or in combination with other drugs or therapies.

Therefore, in another aspect, the present invention provides a pharmaceutical combination, which comprises or consists of a compound of the present invention, preferably a pharmaceutically acceptable salt or solvate thereof, and other active agents. This pharmaceutical combination is used for inhibiting abnormal cell growth in mammals, or for treating and/or preventing diseases mediated by Ras mutations, preferably KRas mutations (such as G12C, G12D, G12V, G12A, G12R, G12S, and G13D mutations), and most preferably KRas-G12D mutation.

The other active agents can be one or more additional compounds of the present invention, or can be a second or additional (e.g., third) compound that is compatible with the compounds of the present invention, i.e., does not adversely affect each other, or has complementary activities. For example, these active agents can be compounds known to regulate other biological activity pathways or can be compounds that regulate different components in the biological activity pathway involved in the compounds of the present invention, or even compounds that overlap with the biological targets of the compounds of the present invention.

In a specific embodiment, other active agents that can be used in combination with the compounds of the present invention include, but are not limited to, chemotherapeutic agents, therapeutic antibodies, and radiotherapy. For example, alkylating agents, antimetabolites, cell-cycle inhibitors, mitotic inhibitors, topoisomerase inhibitors, anti-hormonal drugs, angiogenesis inhibitors, and cytotoxic agents.

The other active agents used in combination with the present invention can be administered simultaneously, separately, or sequentially with the compounds of the present invention via the same or different administration routes. The other active agents can be co-administered with the compounds of the present invention in a single pharmaceutical composition or administered separately from the compounds of the present invention in different discrete units, such as a combination product, preferably in the form of a kit. When administered separately, they can be administered simultaneously or successively, and the successive administration can be close or far apart in time. They can be prepared and/or formulated by the same or different manufacturers. Moreover, the compounds of the present invention and other active agents can be added to the combination therapy (i) before the combination product is sent to the physician (e.g., in the case of a kit containing the compound of the present invention and another drug); (ii) by the physician himself/herself (or under the physician's guidance) immediately before administration; (iii) by the patient himself/herself, for example, during the sequential administration of the compound of the present invention and other active agents.

The compounds of the present invention can also be combined with anti-tumor therapies, which include but are not limited to surgery, radiation therapy, transplantation (e.g., stem cell transplantation, bone marrow transplantation), tumor immunotherapy, and chemotherapy.

Therefore, in another aspect, the present invention also provides a kit, which comprises two or more separate pharmaceutical compositions, at least one of which contains a compound of the present invention or a pharmaceutically acceptable salt or solvate thereof, and a device for separately containing the compositions, such as containers, dispensing bottles, or discrete foil packages, for example, blister packs for packaging tablets, capsules, etc., and also includes instructions for use. The kits of the present invention are particularly suitable for administering different dosage forms, such as oral and parenteral dosage forms, or for administering different compositions at different dosage intervals.

For the technical solutions of the pharmaceutical compositions, pharmaceutical combinations, or kits of the present invention described above, the abnormal cell growth or the diseases mediated by Ras mutations, especially KRas mutations, preferably KRas G12C, KRas G12D, KRas G12V, KRas G12A, KRas G12R, KRas G12S, or KRas G13D, and most preferably KRas G12D mutation are as defined above for the methods and uses of the present invention.

For the compounds, pharmaceutical compositions, methods, uses, pharmaceutical combinations, and kits of the

present invention described above, the compounds in the examples herein are preferred.

**Preparation methods of the compounds of the present invention**

In another aspect, the present invention also provides a preparation method for the compounds defined in the present invention.

The compounds of the present invention can be prepared by a variety of methods, including the general methods given below, the methods disclosed in the examples, or methods similar thereto.

Standard synthetic methods and operations for preparing organic compounds and for the transformation and manipulation of functional groups are known in the art and can be found in standard textbooks, for example, Smith M.B., "March's Advanced Organic Chemistry: Reactions, Mechanisms, and Structure", 7th edition, Wiley, 2013). For each reaction step of each general synthetic scheme, appropriate reaction conditions are known to those skilled in the art or can be routinely determined. The method steps for synthesizing the compounds of the present invention can be carried out under reaction conditions known per se (including those specifically mentioned), in the absence or usually in the presence of a solvent or diluent (including, for example, a solvent or diluent that is inert to the reagents used and can dissolve the reagents used), in the absence or presence of a catalyst, a condensing agent or a neutralizing agent (such as an ion exchanger, such as a cation exchanger, for example, in the H+ form), according to the nature of the reaction and/or the reactants, at a reduced, normal or elevated temperature (for example, about -100°C to about 190°C, including, for example, about -78 °C to about 150°C, such as about 0 °C to about 125 °C, room temperature, - 20 to 40 °C or the reflux temperature), under atmospheric pressure or in a closed container, when appropriate, under pressure, and/or in an inert atmosphere such as an argon or nitrogen atmosphere.

Unless otherwise specified, the starting materials and reagents used in the preparation of the compounds are commercially available, or are compounds known in the literature, or can be prepared by those skilled in the art by the methods described below, by methods similar to those given below, or by standard methods known in the art. Unless otherwise stated in the method description, the applicable solvents are those conventional solvents well known to those skilled in the art that are suitable for the specific type of reaction involved, such as water, esters, ethers, liquid aromatic hydrocarbons, alcohols, nitriles, halogenated hydrocarbons, amides, bases, carboxylic anhydrides, cyclic, straight-chain or branched hydrocarbons, or mixtures of these solvents. Such solvent mixtures can also be used for work-up, for example, work-up by chromatography or partition.

If necessary, the starting materials and intermediates in the synthetic reaction process can be separated and purified by conventional techniques, which include but are not limited to filtration, distillation, crystallization, chromatography, etc. If the intermediates and the final products are obtained in solid form, purification can also be carried out by recrystallization or aging. The materials can be characterized by conventional methods including physical constants and spectroscopic data. The reaction mixture is worked up in a conventional manner, for example, by mixing with water, separating the phases, and purifying the crude product by chromatography when appropriate.

Those skilled in the art can recognize whether there are stereocenters in the compounds of the present invention. At all stages of the reaction, a mixture of the formed isomers can be separated into individual isomers, such as diastereoisomers or enantiomers, or into any desired mixture of isomers, such as a racemate or a mixture of diastereoisomers, see, for example, E. L. Eliel, S. H. Wilen and L. N. Mander's "Stereochemistry of Organic Compounds" (Wiley-Interscience, 1994).

In the case where a mixture of stereoisomers is generated during the preparation of the compounds of the present invention, the individual stereoisomers of the compounds of the present invention can be obtained by resolution. For example, starting from the compounds of the present invention obtained as a mixture of stereoisomers, using well-known methods, such as forming diastereomeric pairs, forming salts with optically active acids, followed by fractional crystallization and regeneration of the free base, or by chiral preparative chromatography; alternatively, starting materials or intermediates with established stereochemistry can be used, or any known chiral resolution method can be used to obtain an optically pure or enantiomerically enriched synthetic intermediate, which can then be used as such in the subsequent steps at various stages of the above synthetic process.

In certain specific cases, it may be necessary to protect specific reactive groups using appropriate protecting groups to avoid interference with the reactions of other reactive groups. Suitable protecting groups and methods for protection and deprotection using such suitable protecting groups are well known to those skilled in the art; examples thereof can be found in T. Greene and P. Wuts, Protective Groups in Organic Synthesis (3rd edition), John Wiley & Sons, NY (1999).

Only the general synthetic schemes for synthesizing the compounds of the present invention are illustrated below. Other routes known to those of ordinary skill in the art, as well as other reactants and intermediates, can also be used to obtain the compounds of the present invention.

For the sake of clarity, in the exemplary synthetic schemes described below, unless otherwise specified, $R_1$~$R_{14}$, X, Y, Z, M, G, W, k, n, m and t appearing in the structural formulas of each intermediate compound are defined as above for

the compounds of the present invention, where PG represents a suitable protecting group that can be determined by those skilled in the art based on their knowledge of organic chemistry.

**Synthetic scheme A**

The synthesis of some compounds of general formulas I-A and I-B of the present invention can be prepared according to the following scheme or its appropriate variants, wherein, unless otherwise specified, the variables are defined as above.

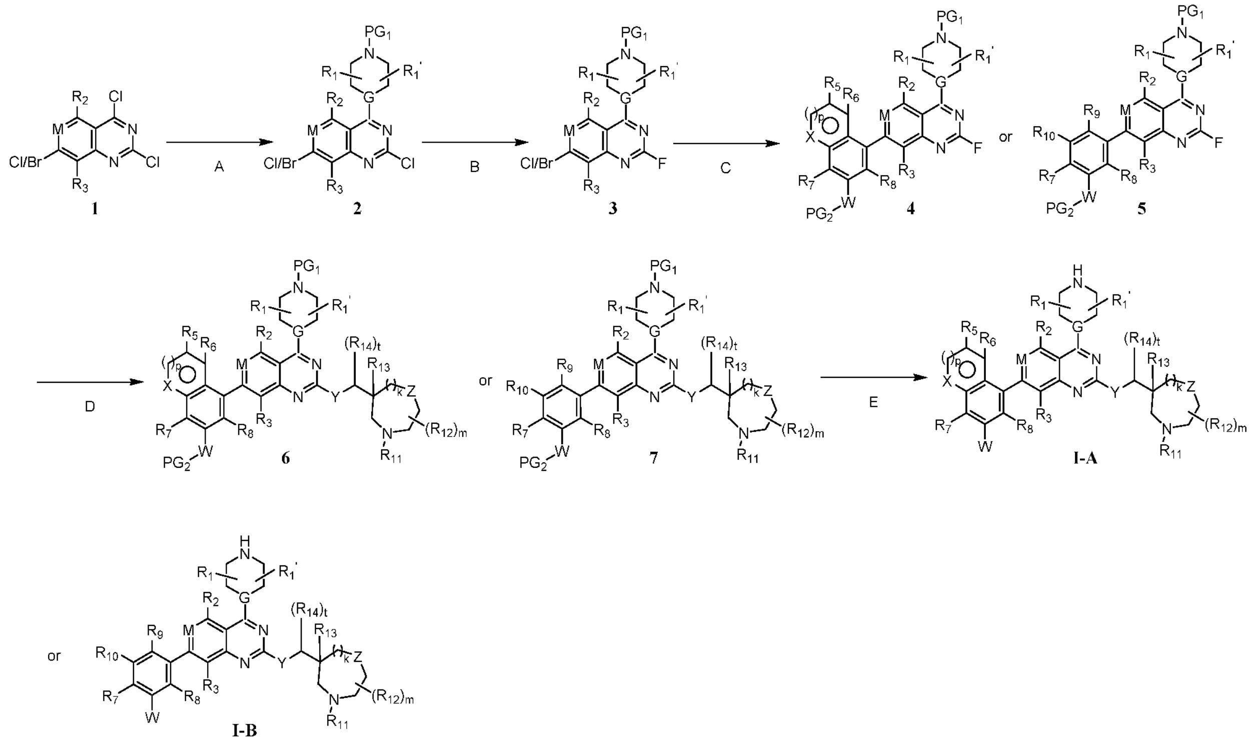


Some representative compounds of this invention can be synthesized according to the scheme. Compound 1 is commercially available or can be obtained in accordance with or similar to the methods used in the embodiments herein. In Step A, compound 1 undergoes an aromatic nucleophilic substitution reaction (when G is N) or a metal-catalyzed coupling reaction (when G is C) to give compound 2. Typical conditions for aromatic nucleophilic substitution are, for example, DIEA/THF, NaH/THF, etc.; typical metal-catalyzed coupling reactions include Suzuki coupling reaction, Negishi coupling reaction, etc. In step B, compound 2 undergoes a halogen exchange reaction and is fluorinated under conditions such as KF/DMSO to give compound 3. In step C, a phenol or an aromatic amine compound is introduced into compound 3 through a metal-catalyzed coupling reaction to give compound 4 or 5. In step D, compound 4 or 5 reacts with an alcohol, a thiol or a selenium compound through an aromatic nucleophilic substitution reaction under conditions such as DIEA/dioxane, NaH/THF, DABCO/$Cs_2CO_3$/ACN to give compound 6 or 7. In step E, the possible protecting groups on compound 6 or 7 are removed to give compounds of general formulas I-A or I-B.

It should be noted that the removal of the protecting groups in step E can be adjusted according to the protecting groups carried by the molecule, and it can be a one-step reaction or a multi-step reaction. Conventional $PG_1$ protecting groups such as Boc can be removed under conditions such as trifluoroacetic acid or hydrochloric acid; conventional $PG_2$ protecting groups such as MOM can also be removed under conditions such as trifluoroacetic acid or hydrochloric acid; conventional $PG_2$ protecting groups such as TIPS can be removed under conditions such as CsF/DMF; conventional $PG_2$ protecting groups such as PMB can be removed under conditions such as trifluoroacetic acid; conventional $PG_2$ protecting agents such as Me can be removed under conditions such as boron tribromide.

**Synthetic scheme B**

Some compounds of general formulas I-A and I-B of the present invention can also be prepared according to the following scheme or its appropriate variants.

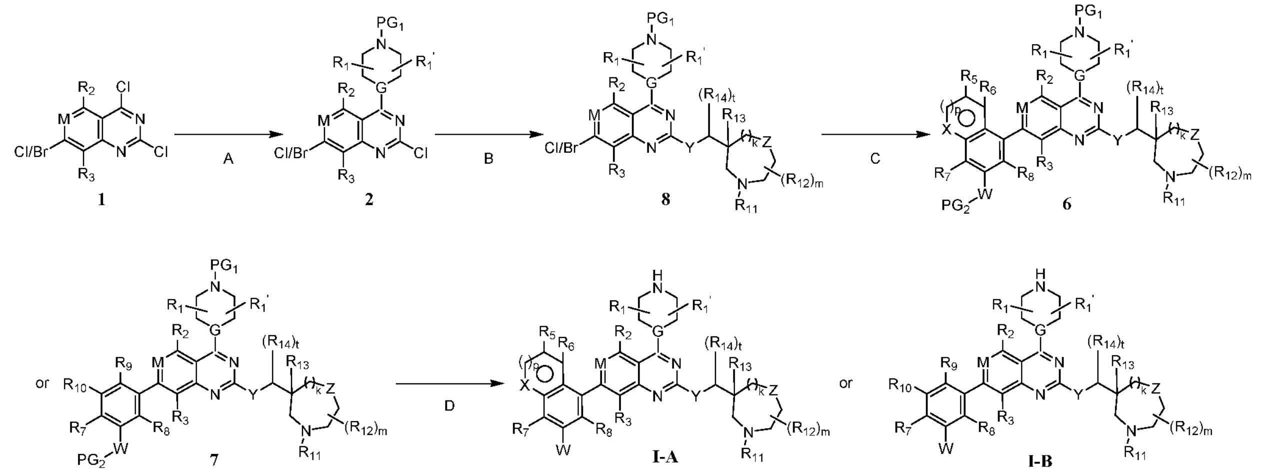


1, 2, 8, 6

or 7, I-A or I-B

In step A, the synthesis of compound 2 can be carried out by referring to Synthetic Scheme A. In step B, compound 2 undergoes an aromatic nucleophilic substitution reaction to give compound 8. In step C, a phenol or an aromatic amine fragment is introduced into the latter through a metal-catalyzed coupling reaction to give compound 6 or 7. In step D, the protecting groups on compound 6 or 7 are removed to give compounds of general formulas I-A or I-B. The typical conditions for the coupling reactions, nucleophilic substitution reactions, and protecting-group removal reactions involved in this synthetic scheme are similar to the relevant reaction conditions described in Synthetic Scheme A and can be implemented by reference.

## Synthetic scheme C

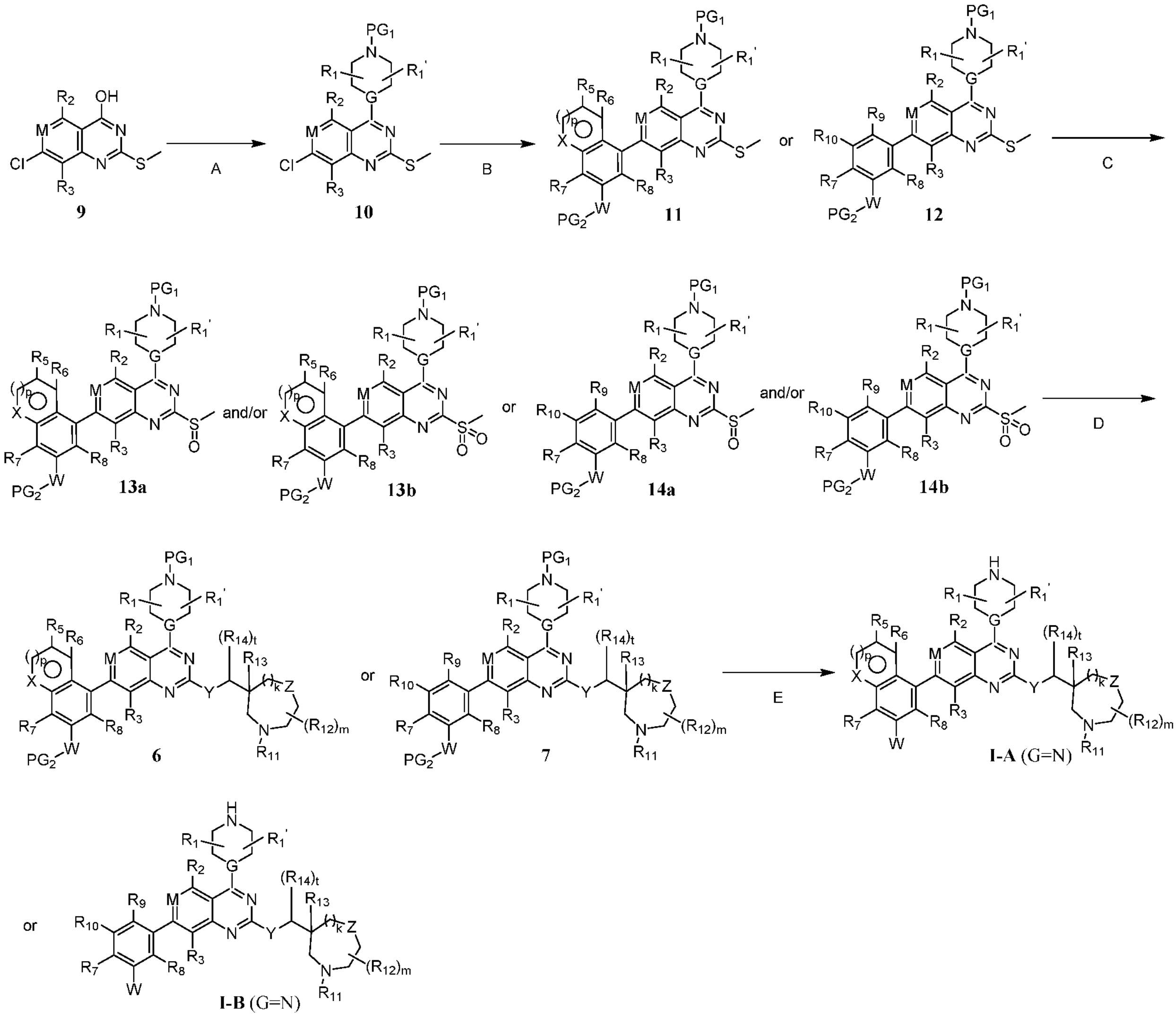


Some representative compounds (G = N) of the present invention can be synthesized according to the above-mentioned scheme. Compound 9 is commercially available or can be obtained by the methods used in the examples herein or methods similar thereto. In step A, compound 9 undergoes an amination reaction in the presence of a condensing agent (such as BOP, PyAOP, etc.) to give compound 10. In step B, compound 10 undergoes a metal-catalyzed coupling reaction to introduce the corresponding B fragment, giving compound 11 or 12. In step C, compound 11 or 12 is oxidized by an oxidant (such as mCPBA) to give compound 13a/13b (or a mixture of 13a and 13b) or 14a/14b (or a mixture of 14a and 14b), respectively. The obtained compound 13a/b or 14a/b undergoes an aromatic nucleophilic substitution reaction to give compound 6 or 7, and the latter removes the possible protecting agent in step E to give compound I-A' or I-B'.

It should be noted that the typical reaction conditions and reagents used in the metal-catalyzed coupling reactions, aromatic nucleophilic substitution reactions, and protecting-agent removal reactions involved in the above synthetic scheme are well known in the art, fall within the scope of the routine experience of those skilled in the art, or can be determined by those skilled in the art by making appropriate changes based on the typical conditions of such reactions in the art and the characteristics of the starting materials and target products used.

## Synthetic examples

The present invention will be further described below in conjunction with examples. It should be noted that the following examples are exemplary and should not be regarded as limiting the protection scope of the present invention.

In the description of the embodiments and the subsequent specific examples herein, the following abbreviations are used:

ACN (Acetonitrile); Boc (tert-butoxycarbonyl); BAST (bis(2-methoxyethyl)sulfuraminotrifluoride); $CDCl_3$ (deuterated chloroform); DAST (diethylaminosulfur trifluoride); DCM (dichloromethane); DIEA or DIPEA (*N*,*N*-diisopropylethylamine); DMF (*N*,*N*-dimethylformamide); DMSO (dimethylsulfoxide); DMSO-$d_6$ (hexadeuterated dimethylsulfoxide); EA (ethyl

acetate); EDTA-K2 (ethylenediaminetetraacetic acid dipotassium salt); EtOH (ethanol); FCC (flash column chromatography); g (grams); h (hours); HCl (hydrogen chloride); HCl-MeOH or HCl/MeOH (hydrogen chloride in methanol); HLM (human liver microsomes); $H_2O$ (water); $H_2SO_4$ (sulfuric acid); IV (intravenous); $K_2CO_3$ (potassium carbonate); LCMS (liquid chromatography mass spectrometry); LC-MS/MS (liquid chromatography-mass spectrum-mass spectrum online); MeOH (methanol); Methanol-*d₄* (tetradeuterated methanol); mg (milligrams); MHz (megahertz); min (minutes); mL (milliliters); mmol (millimolar); MOM (methoxymethyl ether); MTBE (methyl tert-butyl ether); m/z (mass-to-charge ratio); $N_2$ (nitrogen); NaCl (sodium chloride); NaH (sodium hydride); $NaHCO_3$ (sodium bicarbonate); $Na_2SO_3$ (sodium sulfite); $Na_2SO_4$ (sodium sulfate); NCS (chlorobutaneimide); $NH_4Cl$ (ammonium chloride); NMR (nuclear magnetic resonance); $PdCl_2$(dtbpf) or Pd(dtbpf)$Cl_2$ (1,1'-bis(di-tert-butylphosphino) ferrocene palladium chloride); $PdCl_2$(dppf) or Pd(dppf)$Cl_2$ (1,1'-bisdiphenylphosphino ferrocene palladium dichloride); $Pd(OAc)_2$ (palladium acetate); $Pd(PPh_3)_4$ (tetraphylphosphonium palladium); PE (petroleum ether); PO (oral administration); $POCl_3$ (phosphorus oxychloride); r.t. (room temperature); SFC (supercritical fluid chromatography); $SiO_2$ (silica gel); TBAF (tetrabutylammonium fluoride); TEA (triethylamine); TFA (trifluoroacetic acid); THF (tetrahydrofuran); TIPS (triisopropylsilyl); TLC (thin-layer chromatography); TsOH (p-toluenesulfonic acid); TsOH·$H_2O$ (p-toluenesulfonic acid monohydrate); μL (microliter); μM (micromolar concentration); μmol (micromolar).

In the following examples, the names and structures of the synthesized target compounds are given. Any deviation between the name and the structure is not intentional, and in such a case, the structure shall prevail.

The experimental methods for which specific conditions are not specified in the following examples are generally carried out according to the conventional conditions of such reactions or according to the conditions recommended by the manufacturer. Unless otherwise stated, percentages and parts are weight percentages and weight parts. Unless otherwise stated, the ratios of liquids are volume ratios.

The experimental materials and reagents used in the following examples can be obtained from commercial sources, prepared according to the methods of the prior art, or prepared according to methods similar to those disclosed in this application, unless otherwise specified.

In the following examples, the $^1$H-NMR spectra were recorded using a Bruker (400 MHz), and the chemical shifts are expressed as δ (ppm) relative to the peaks of the deuterated solvents ($CDCl_3$: δ = 7.26 ppm; $CD_3OD$: δ = 3.31 ppm; DMSO-$d_6$: δ = 2.50 ppm); the mass spectra were recorded using an Aglient 1100 liquid chromatograph + Aglient G6100 mass spectrometer LCMS liquid chromatography-mass spectrometry instrument.

**Chiral analytical method:**

**SFC-1:** Waters UPCC, analytical column: Daicel Chiralpak® IC, 100 * 3 mm 3 μm; mobile phase A: $CO_2$, mobile phase B: MeOH (0.1% DEA); flow rate: 1.5 mL/min; column temperature: 35°C; backpressure: 1800 psi; gradient: 0-0.5 min A/B = 95/5, 0.5-5.0 min A/B = 95/5-60/40, 5.0-8.0 min A/B = 60/40.

**Chiral analysis 2:** Agilent 1260 Infinity, separation column: Daicel Chiralpak® IG, 4.6 * 100 mm 5 μm; mobile phase A: *n*-hexane, mobile phase B: ETOH/DCM = 9/1 (0.1% DEA); flow rate: 1.0 mL/min; column temperature: 35 °C; gradient: 0-20 min A/B = 60/40.

**SFC-3:** Waters UPCC, analytical column: Daicel Chiralpak® IG, 100 * 3 mm 3 μm; mobile phase A: $CO_2$, mobile phase B: MeOH; flow rate: 1.5 mL/min; column temperature: 35 °C; backpressure: 1800 psi; gradient: 0-8.0 min A/B = 90/10.

**SFC-4:** Waters UPCC, analytical column: Daicel Chiralpak® IG, 100 * 4.6 mm 5 μm; mobile phase A: $CO_2$, mobile phase B: MeOH (0.1% DEA); flow rate: 2.0 mL/min; column temperature: 40 °C; backpressure: 1800 psi; gradient: 0-8.0 min A/B = 60/40.

**Chiral analysis 5:** SHIMADZU LC-20AD, separation column: Daicel Chiralpak® IA, 150 * 4.6 mm 5 μm; mobile phase A: *n*-hexane, mobile phase B: ETOH (+ 0.1% DEA); flow rate: 1.0 mL/min; column temperature: 35 °C; gradient: 0-10 min A/B = 90/10.

**SFC-6:** Waters UPCC, analytical column: Daicel Chiralpak® OZ, 100 * 3 mm 3 μm; mobile phase A: $CO_2$, mobile phase B: MeOH (0.1% DEA); flow rate: 1.5 mL/min; column temperature: 35 °C; back pressure: 1800 psi; gradient: 0-8.0 min A/B = 70/30.

**SFC-7:** Waters UPCC, analytical column: Daicel ChiralCEL® IC, 100 * 3 mm 3 μm; mobile phase A: $CO_2$, mobile phase B: MeOH; flow rate: 2.0 mL/min; column temperature: 35°C; back pressure: 1800 psi; gradient: 0-8.0 min A/B =

60/40.

**SFC-8:** Waters UPCC, analytical column: Daicel ChiralCEL ® IC, 100 * 3 mm 3 μm; mobile phase A: $CO_2$, mobile phase B: IPA (+ 0.1% 7.0 mol/l Ammonia); flow rate: 1.5 mL/min; column temperature: 35 °C; back pressure: 1800 psi; gradient: 0-8.0 min A/B = 55/45.

**SFC-9:** Waters UPCC, analytical column: Daicel Chiralpak ® IG, 100 * 3 mm 3 μm; mobile phase A: $CO_2$, mobile phase B: IPA (0.1% DEA); flow rate: 1.5 mL/min; column temperature: 35 °C; backpressure: 1800 psi; gradient: 0-8.0 min A/B = 70/30.

**SFC-10:** Waters UPCC, analytical column: Daicel ChiralCEL ® OZ, 100 * 3 mm 3 μm; mobile phase A: $CO_2$, mobile phase B: MeOH (0.1% DEA); flow rate: 1.5 mL/min; column temperature: 35 °C; back pressure: 1800 psi; gradient: 0-8.0 min A/B = 70/30.

**SFC-11:** Waters UPCC, analytical column: Daicel ChiralCEL ® OZ, 100 * 3 mm 3 μm; mobile phase A: $CO_2$, mobile phase B: MeOH (+0.1% 7.0 mol/l Ammonia); flow rate: 1.5 mL/min; column temperature: 35 °C; backpressure: 1800 psi; gradient: 0-8.0 min A/B = 60/40.

**SFC-12:** Waters UPCC, analytical column: REGIS(S,S)WHELK-O1, 100 * 3 mm 3 μm; mobile phase A: $CO_2$, mobile phase B: MeOH (+0.1% 7.0 mol/l Ammonia); flow rate: 1.5 mL/min; column temperature: 35 °C; backpressure: 1800 psi; gradient: 0-5.0 min A/B = 80/20.

**SFC-13:** Waters UPCC, analytical column: Daicel ChiralCEL ® ID, 100 * 3 mm 3 μm; mobile phase A: $CO_2$, mobile phase B: IPA (+0.1% DEA); flow rate: 1.5 mL/min; column temperature: 35 °C; backpressure: 1800 psi; gradient: 0-5.0 min A/B = 70/30.

**SFC-14:** Waters UPCC, analytical column: Daicel ChiralCEL ® IC, 100 * 3 mm 3 μm; mobile phase A: $CO_2$, mobile phase B: IPA (+0.1% DEA); flow rate: 1.5 mL/min; column temperature: 35 °C; back pressure: 1800 psi; gradient: 0-8.0 min A/B = 70/30.

**SFC-15:** Waters UPCC, analytical column: Daicel ChiralCEL ® IC, 100 * 3 mm 3 μm; mobile phase A: $CO_2$, mobile phase B: MeOH (+0.1% DEA); flow rate: 1.5 mL/min; column temperature: 35 °C; backpressure: 1800 psi; gradient: 0-8.0 min A/B = 85/15.

**SFC-16:** Waters UPCC, analytical column: (S,S)WHELK-O1, 100 * 3 mm 3 μm; mobile phase A: $CO_2$, mobile phase B: MeOH (+0.1% DEA); Flow rate: 1.5 mL/min; column temperature: 35 °C ; backpressure: 1800 psi; gradient: 0-5.0 min A/B = 70/30.

## Intermediate A-1

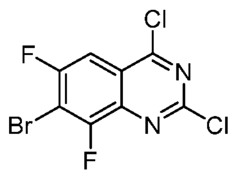

7-bromo-2,4-dichloro-6,8-difluoroquinazoline

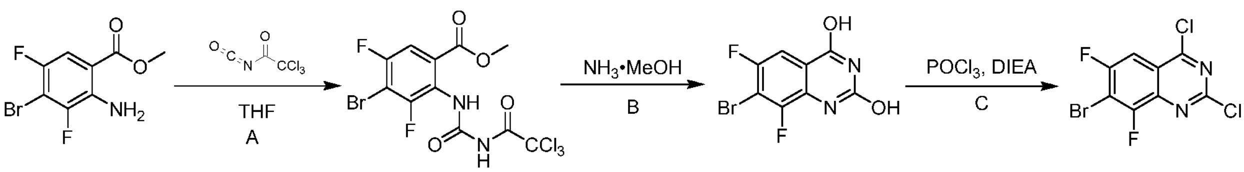

**Step A:** methyl 4-bromo-3,5-difluoro-2-(3-(2,2,2-trichloroacetyl)ureido)benzoate

At room temperature, methyl 2-amino-4-bromo-3,5-difluorobenzoate (34 g, 128 mmol) and THF (500 mL) were added to a round-bottom flask equipped with a magnetic stir bar. Under stirring at rt, 2,2,2-trichloroacetyl isocyanate (28.9 g, 153 mmol) was added dropwise to the system. The resulting mixture was stirred at room temperature for 2 hours and then concentrated under reduced pressure to obtain methyl 4-bromo-3,5-difluoro-2-(3-(2,2,2-trichloroacetyl)ureido) benzoate (crude product) as a brown solid, which was directly used in the next step. LCMS (m/z): 452.8 (M + H).

**Step B:** 7-bromo-6,8-difluoroquinazoline-2,4-diol

At room temperature, methyl 4-bromo-3,5-difluoro-2-(3-(2,2,2,2-trichloroacetyl)ureido)benzoate obtained in the previous step was added to a round-bottom flask equipped with a magnetic stir bar, and then $NH_3$ (400 mL, 7M MeOH solution) was added. The resulting mixture was stirred at room temperature for 2 hours, and the reaction was monitored by LCMS until it was complete. After concentration under reduced pressure, the obtained solid was slurried with methyl *tert*-butyl ether and filtered to afford 7-bromo-6,8-difluoroquinazoline-2,4-diol (32 g, with a total yield of 90% over the two steps) as a pale-yellow solid. LCMS (m/z): 277.0 (M + H).

**Step C:** 7-bromo-2,4-dichloro-6,8-difluoroquinazoline

7-bromo-6,8-difluoroquinazoline-2,4-diol (6 g, 22 mmol), $POCl_3$ (50 mL) were added to a round bottom flask equipped with a magnetic stir bar. DIEA (12 mL) was added dropwise to the system under stirring at room temperature. After the dropwise addition was completed, the system was heated to 110 °C and stirred overnight. The reaction solution was concentrated under reduced pressure to about 30 mL and then poured into water (500 mL), and a precipitate was formed. The solid was collected by filtration and dried to afford 7-bromo-2,4-dichloro-6,8-difluoroquinazoline (5 g, yield 74%) as a yellow solid. LCMS (m/z): 312.9 (M + H).

## Intermediate B-1

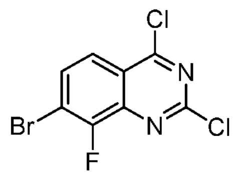

7-bromo-2,4-dichloro-8-fluoroquinazoline

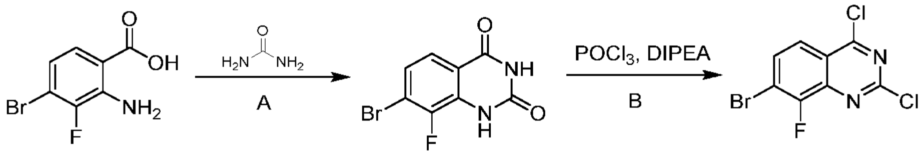


**Step A:** 7-bromo-8-fluoroquinazoline-2,4(1*H*,3*H*)-dione

At room temperature, 2-amino-4-bromo-3-fluorobenzoic acid (10.0 g, 42.7 mmol) and urea (25.7 g, 427.3 mmol) were mixed together. The mixture was heated to 200 °C and stirred for 2 h. The reaction mixture gradually changed from a solid state to a liquid state and then back to a solid state. After the reaction was completed monitored by LCMS, it was washed with hot water (250 mL). The solid was collected by filtration to obtain 7-bromo-8-fluoroquinazoline-2,4(1*H*,3*H*)-dione (12 g, crude product). LCMS (m/z): 258.9 (M + H).

**Step B:** 7-bromo-2,4-dichloro-8-fluoroquinazoline

At room temperature, DIPEA (13.5 mL, 77.2 mmol) was added to a mixture of 7-bromo-8-fluoroquinazoline-2,4(1*H*,3*H*)-dione (4.0 g) and $POCl_3$ (35.9 mL, 386.0 mmol). The mixture was heated to 100 °C and stirred for 5 h. After the reaction was completed, most of the solvent and base were removed by concentration. ACN (10 mL) was added for

dilution. Under stirring at room temperature, the obtained diluted solution was slowly added dropwise to water, and a solid precipitated. The precipitate was filtered and dried to afford 7-bromo-2,4-dichloro-8-fluoroquinazoline (3.8 g, yield 83%) as a yellow solid. LCMS (m/z): 296.8 (M + H).

## Intermediate C-1 and intermediate C-1a

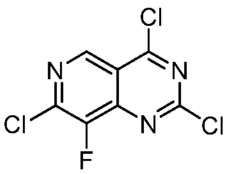

and

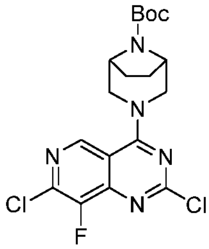

7-Chloro-2,4-dichloro-8-fluoropyrido[4,3-*d*]pyrimidine and *tert*-butyl (1*R*,5*S*)-3-(7-chloro-2-chloro-8-fluoropyrido[4,3-*d*]pyrimidin-4-yl)-3,8-diazabicyclo[3.2.1]octane-8-carboxylate

Intermediate C-1 and intermediate C-1a were prepared and characterized by referring to the literature WO2021041671A1.

## Intermediate A-1a

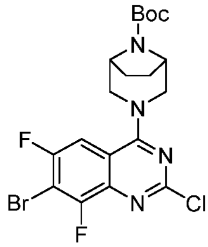

*tert*-Butyl (1R,5S)-3-(7-bromo-2-chloro-6,8-difluoroquinazolin-4-yl)-3,8-diazabicyclo[3.2.1]octane-8-carboxylate

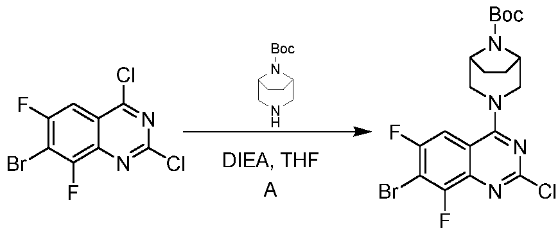


**Step** A: *tert*-butyl (1*R*,5*S*)-3-(7-bromo-2-chloro-6,8-difluoroquinazolin-4-yl)-3,8-diazabicyclo[3.2.1]octane-8-carboxylate

7-bromo-2,4-dichloro-6,8-difluoroquinazoline (8.0 g, 26 mmol), DIEA (8.0 mL) and THF (80 mL) were added to a round-bottom flask equipped with a magnetic stir bar. Under stirring at room temperature, *tert*-butyl (1*R*,5*S*)-3,8-diazabicyclo[3.2.1]octane-8-carboxylate (6.0 g, 28 mmol) was slowly added to the reaction system. The resulting mixture was stirred for 1 hour. The reaction solution was concentrated, poured into water, and filtered. The filter cake was collected and dried to obtain *tert*-butyl (1*R*,5*S*)-3-(7-bromo-2-chloro-6,8-difluoroquinazolin-4-yl)-3,8-diazabicyclo[3.2.1]octane-8-carboxylate (9.0 g, yield 72%) as a yellow solid. LCMS (ESI, m/z): 499.0 (M + H).

## Intermediate A-1b

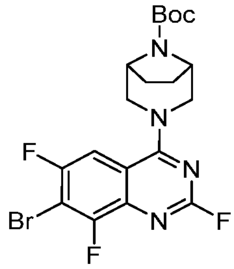

*tert*-Butyl (1*R*,5*S*)-3-(7-bromo-2,6,8-trifluoroquinazolin-4-yl)-3,8-diazabicyclo[3.2.1]octane-8-carboxylate

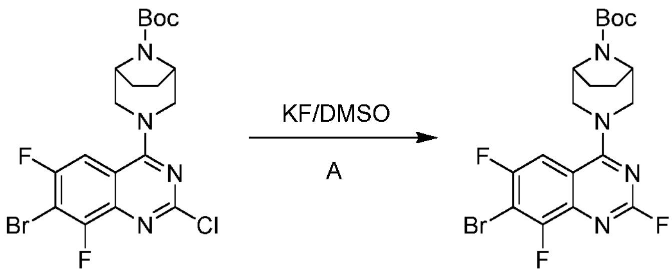


**Step** A: *tert*-butyl (1*R*,5*S*)-3-(7-bromo-2,6,8-trifluoroquinazolin-4-yl)-3,8-diazabicyclo[3.2.1]octane-8-carboxylate

A mixture of *tert*-butyl (1*R*,5*S*)-3-(7-bromo-2-chloro-6,8-difluoroquinazolin-4-yl)-3,8-diazabicyclo[3.2.1]octane-8-carboxylate (9.0 g, 8.4 mmol), KF (21.4 g, 368 mmol), and DMSO (100 mL) was stirred overnight at 100°C. After the reaction was completed monitored by LCMS, the reaction solution was cooled to room temperature. Under stirring, the reaction solution was slowly poured into water (1 L), and a yellow solid precipitated. The solid was filtered and the filter cake was washed with water (200 mL). The filter cake was collected and dried to obtain *tert*-butyl (1R,5S)-3-(7-bromo-2,6,8-trifluoroquinazolin-4-yl)-3,8-diazabicyclo[3.2.1]octane-8-carboxylate (7.7 g, yield 88%) as a yellow solid. LCMS (m/z): 473.4 (M + H).

**Referring to the above synthetic scheme, the following intermediates were also synthesized in the present invention:**

| Intermediate No. | A-2 | A-2a | A-2b |
|---|---|---|---|
| Structure | 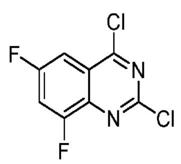 | 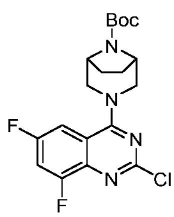 | 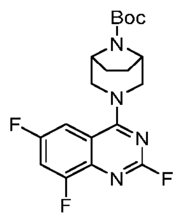 |
| Characterization data | LCMS (m/z): 235.0 (M + H) | LCMS (m/z): 411.1 (M + H) | LCMS (m/z): 395.2 (M + H) |

## Intermediate A-1b-I

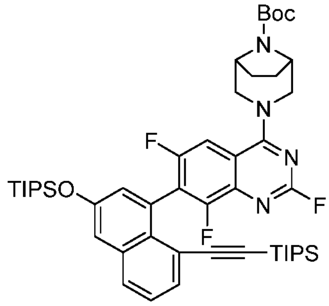

*tert*-Butyl (1*R*,5*S*)-3-(2,6,8-trifluoro-7-(7-fluoro-8-(triisopropylsilyl)ethynyl)-3-(triisopropylsilyl)oxy)naphthaen-1-yl)quinazolin-4-yl)-3,8-diazabicyclo[3.2.1]octane-8-carboxylate

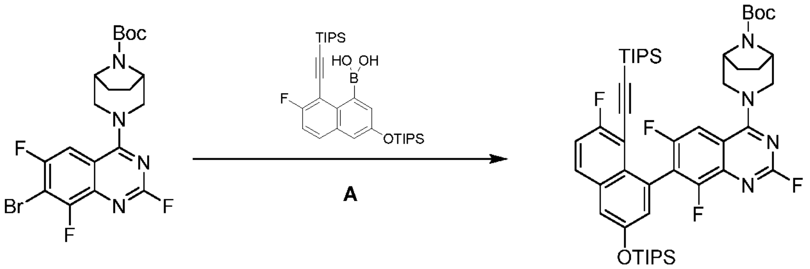


**Step A:** *tert*-butyl (1*R*,5*S*)-3-(2,6,8-trifluoro-7-(7-fluoro-8-(triisopropylsilyl)ethynyl)-3-(triisopropylsilyl)oxy)naphthaen-1-yl)quinazolin-4-yl)-3,8-diazabicyclo[3.2.1]octane-8-carboxylate

At room temperature, *tert*-butyl (1*R*,5*S*)-3-(7-bromo-2,6,8-trifluoroquinazolin-4-yl)-3,8-diazabicyclo[3.2.1]octane-8-carboxylate (3.0 g, 6.34 mmol), (7-fluoro-8-(triisopropylsilyl)ethynyl)-3-(triisopropylsilyl)oxy)naphthalen-1-yl) boronic acid (4.13 g, 7.61 mmol), $Pd(OAc)_2$ (71 mg, 0.32 mmol), BIDIME (209 mg, 0.63 mmol), $K_3PO_4$ (4.04 g, 19.02 mmol) were dissolved in tert-amyl alcohol (30 mL), The system was purged with $N_2$ three times, then heated to 110 °C and stirred overnight. When the reaction was finished (detected by LCMS), the mixture was filtered through celite and washed with EA (50 mL). The resulting organic phase was concentrated and purified by FCC ($SiO_2$, EA/PE = 0 ~ 50%) affording a yellow solid, *tert*-butyl (1*R*,5*S*)-3-(2,6,8-trifluoro-7-(7-fluoro-8-(triisopropylsilyl)ethynyl)-3-(triisopropylsilyl)oxy) naphthaen-1-yl)quinazolin-4-yl)-3,8-diazabicyclo[3.2.1]octane-8-carboxylate (2.3g, yield 41%). $^1H$ NMR (400 MHz, Chloroform-d) δ 7.74 (dd, *J* = 9.1, 5.6 Hz, 1H), 7.39 (dd, *J* = 9.8, 1.7 Hz, 1H), 7.34 (d, *J* = 2.6 Hz, 1H), 7.31 - 7.28 (m, 1H), 7.10 (d, *J* = 2.6 Hz, 1H), 4.86 - 4.61 (m, 1H), 4.54 - 4.26 (m, 2H), 4.20 - 4.01 (m, 1H), 3.92 - 3.69 (m, 1H), 3.58 - 3.36 (m, 1H), 2.11 - 1.97 (m, 3H), 1.53 (s, 9H), 1.36 - 1.23 (m, 4H), 1.17 - 1.08 (m, 18H), 0.95 - 0.79 (m, 18H), 0.61 - 0.49 (m, 3H). LCMS (m/z): 891.4 (M + H).

## Intermediate A-1b-I1 and A-1b-I2

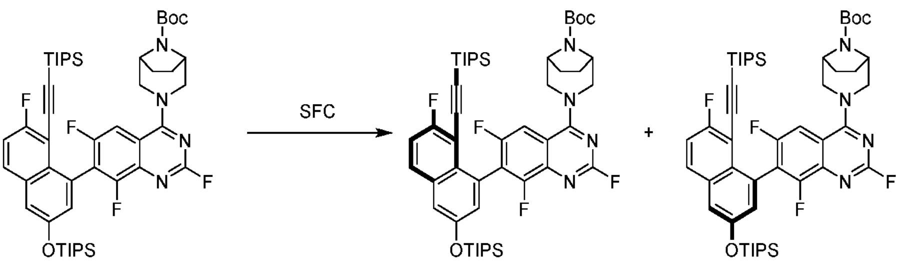


isomer 1 isomer 2

**A-1b-I1** **A-1b-I2**

The compound *tert*-butyl (1*R*,5*S*)-3-(2,6,8-trifluoro-7-(7-fluoro-8-(triisopropylsilyl)ethynyl)-3-(triisopropylsilyl)oxy)naphthaen-1-yl)quinazolin-4-yl)-3,8-diazabicyclo[3.2.1]octane-8-carboxylate (intermediate A-1b-I, 10g) was resolved by SFC (SFC150, Waters) (separation column: DAICEL CHIRALPAK ® IC, 250 * 25 mm, 10μm; mobile phase: $CO_2$/MeOH = 75/25; Flow rate: 70 mL/min), and the isomer 1 eluted first was obtained as intermediate A-1b-I1 (4.5 g, with a relatively shorter retention time). Chiral analytical method SFC-1, Rt = 3.855 min. LCMS (m/z): 891.4 (M + H). The isomer 2 subsequently eluted was intermediate A-1b-I2 (4.9 g, r with a relatively longer retention time). Chiral analytical method SFC-1, Rt = 4.198 min. $^1$H NMR (400 MHz, Chloroform-d) δ 7.74 (dd, *J* = 9.1, 5.7 Hz, 1H), 7.40 (dd, *J* = 9.7, 1.7 Hz, 1H), 7.34 (d, *J* = 2.5 Hz, 1H), 7.29 (d, *J* = 8.7 Hz, 1H), 7.10 (d, *J* = 2.6 Hz, 1H), 4.81 - 4.64 (m, 1H), 4.51 - 4.32 (m, 2H), 4.18 - 4.03 (m, 1H), 3.91 - 3.69 (m, 1H), 3.52 - 3.32 (m, 1H), 2.30 - 1.93 (m, 3H), 1.53 (s, 9H), 1.37 - 1.21 (m, 4H), 1.16 - 1.05 (m, 18H), 0.94 - 0.80 (m, 18H), 0.62 - 0.47 (m, 3H). $^{19}$F NMR (376 MHz, Chloroform-*d*) δ-47.14,-105.65,-112.24,-120.80. LCMS (m/z): 891.4 (M + H).

## Intermediate C-2 and Intermediate C-2a

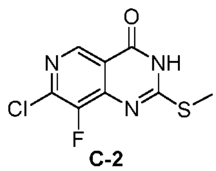

C-2

and

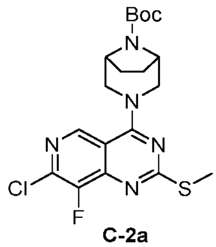

C-2a

7-chloro-8-fluoro-2-(methylthio)pyrido[4,3-*d*]pyrimidin-4(3H)-one *and tert*-butyl (1*R*,5*S*)-3-(7-chloro-8-fluoro-2-(methylthio)pyrido[4,3-*d*]pyrimidin-4-yl)-3,8-diazabicyclo[3.2.1]octane-8-carboxylate

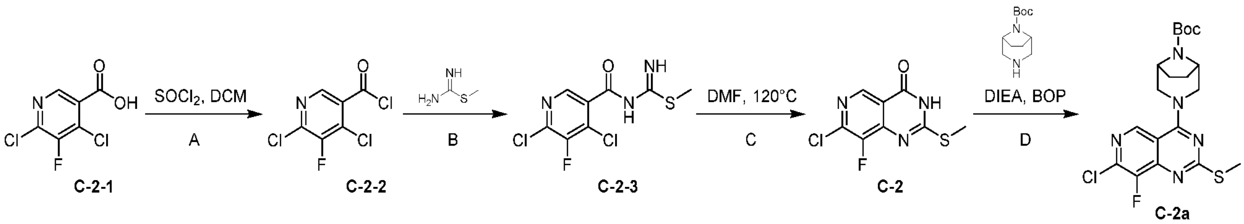


**Step A:** 4,6-dichloro-5-fluoronicotinoyl chloride

Under stirring at room temperature, thionyl chloride (2.25 mL, 31 mmol) was slowly added to a solution of 4,6-dichloro-5-fluoronicotinic acid (5.0 g, 23.4 mmol) in DCM (100 mL), and then DMF (175 mg, 2.4 mmol) was added. The resulting reaction solution was stirred at 50 °C for 2 h. After the reaction was completed (monitored by TLC), it was concentrated, and a small amount of toluene was added for azeotropic distillation. Then, 4,6-dichloro-5-fluoronicotinoyl chloride (4.5 g, yield 83%) as a yellow solid was obtained, which was directly used in the subsequent reaction.

**Step B:** (4,6-dichloro-5-fluoronicotinoyl)carbamimidothioate

Under stirring at 0 °C, a mixed solution of 4,6-dichloro-5-fluoronicotinoyl chloride (4.5 g, 19.8 mmol) and 1,2-dimethoxyethane (20 mL) was slowly added dropwise to a mixed solution of 2-methylisothiourea sulfate (15 g, 49.5 mmol) and 1 M aqueous NaOH solution (70 mL), and the mixture was stirred at the same temperature for 1 h. The precipitated solid was filtered and dried to obtain methyl (4,6-dichloro-5-fluoronicotinoyl)carbamimidothioate (5.0 g, yield 90%). LCMS (m/z): 282.1 (M + H).

**Step C:** 7-chloro-8-fluoro-2-(methylthio)pyrido[4,3-d]pyrimidin-4(3*H*)-one

(4,6-dichloro-5-fluoronicotinoyl)carbamimidothioate (5.0 g, 17.8 mmol) was dissolved in DMF (40 mL), and the solution was heated to 120 °C and stirred for 3 h. After the reaction was completed (monitored by LCMS), it was cooled to room temperature, and water (200 mL) was added. The precipitated solid was filtered and dried to obtain the product, (4,6-dichloro-5-fluoronicotinoyl)carbamimidothioate (3.6 g, yield 82%). LCMS (m/z): 245.6 (M + H).

**Step D:** *tert*-butyl (1*R*,5*S*)-3-(7-chloro-8-fluoro-2-(methylthio)pyrido[4,3-d]pyrimidin-4-yl)-3,8-diazabicyclo[3.2.1]octane-8-carboxylate

At room temperature, *tert*-butyl 3,8-diazabicyclo[3.2.1]octane-8-carboxylate (1.0 g, 4.9 mmol), BOP (2.3 g, 5.3 mmol) and DIEA (1.0 g, 8.1 mmol) were added to a solution of 7-chloro-8-fluoro-2-(methylthio)pyrido[4,3-d]pyrimidin-4(3*H*)-one (1.0 g, 4.0 mmol) in DMF (15 mL). The reaction solution was stirred at 50 °C for 2 h. After the reaction was completed (monitored by LCMS), the reaction solution was poured into 100 mL of ice water to quench the reaction. The mixture was extracted with ethyl acetate. The combined organic phases were washed with saturated brine, dried over anhydrous sodium sulfate, filtered and concentrated. The obtained crude product was purified by FCC ($SiO_2$, EtOAc/PE = 0-20%) to afford the yellow solid product, *tert*-butyl (1*R*,5*S*)-3-(7-chloro-8-fluoro-2-(methylthio)pyrido[4,3-*d*]pyrimidin-4-yl)-3,8-diazabicyclo[3.2.1]octane-8-carboxylate (980 mg, yield 55%). LCMS (m/z): 440.1 (M + H).

## Intermediates D-1, D-2 and D-2a

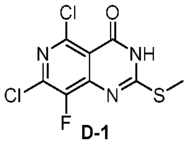
D-1 ,
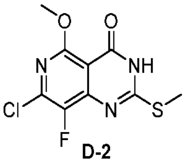
D-2 and
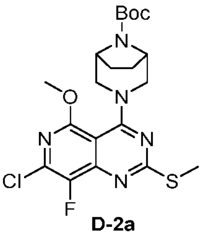
D-2a

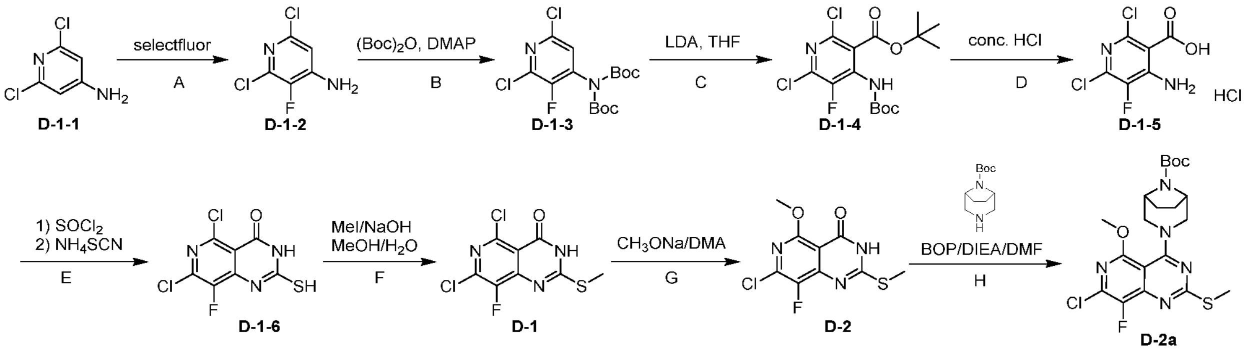


**Step A:** 2,6-dichloro-3-fluoropyridin-4-amine

Selectfluor (68 g, 180 mmol) was added to a solution of 2,6-dichloropyridin-4-amine (25 g, 154 mmol) in methanol/water (V/V = 5:1, 300 mL) at room temperature. The resulting mixture was stirred at 50 °C for 48 h, concentrated under reduced pressure, diluted with ethyl acetate, washed sequentially with water and brine, and dried over anhydrous sodium sulfate. The crude product was concentrated by filtration and purified by FCC ($SiO_2$, EA/PE = 0-10%) to obtain 2,6-dichloro-3-fluoropyridin-4-amine (10g) as a white solid. LCMS (m/z): 180.9 (M + H).

**Step B:** *tert*-butyl (*tert*-butoxycarbonyl)(2,6-dichloro-3-fluoropyridin-4-yl)carbamate

Under stirring at room temperature, 4-dimethylaminopyridine (307 mg, 2.75 mmol) and di-*tert*-butyl dicarbonate (30 g, 138 mmol) were added to a solution of 2,6-dichloro-3-fluoropyridin-4-amine (10 g, 55 mmol) in tetrahydrofuran (100 mL). The resulting mixture was heated to 60 °C and stirred for 16 h. The reaction was completed (monitored by TLC), then concentrated to obtain a crude product. After slurry with methanol, *tert*-butyl (*tert*-butoxycarbonyl)(2,6-dichloro-3-fluoropyridin-4-yl)carbamate (16 g) was obtained as a white solid. LCMS (m/z): 381.2 (M + H).

**Step C:** *tert*-butyl 4-((*tert*-butoxycarbonyl)amino)-2,6-dichloro-5-fluoronicotinate

Under a dry ice-ethanol bath, LDA (2.0 M, 63 mL, 126 mmol) was slowly added to a solution of *tert*-butyl (*tert*-butoxycarbonyl) (2,6-dichloro-3-fluoropyridin-4-yl) carbamate (16g, 42 mmol) in THF (200 mL). The resulting mixture is stirred for 1h. The reaction was completed (monitored by TLC), quenched by adding an appropriate amount of acetic acid, diluted with EA, washed with water, and dried over anhydrous sodium sulfate. After filtration and concentration, the obtained crude product was purified by FCC ($SiO_2$, EA/PE = 0-20%) to obtain *tert*-butyl 4-((*tert*-butoxycarbonyl)amino)-2,6-dichloro-5-fluoronicotinate (13g).

**Step D:** 4-amino-2,6-dichloro-5-fluoronicotinic acid hydrochloride

Concentrated hydrochloric acid (30 mL) was added to a solution of *tert*-butyl 4-((*tert*-butoxycarbonyl)amino)-2,6-dichloro-5-fluoronicotinate (13 g, 34 mmol) in dioxane (90 mL) at room temperature. The resulting mixture was stirred for 3h at room temperature. After the reaction was completed (monitored by LCMS), 4-amino-2,6-dichloro-5-fluoronicotinic acid hydrochloride (8 g) was obtained through concentration. LCMS (m/z): 224.9 (M + H).

**Step E:** 5,7-dichloro-8-fluoro-2-mercaptopyrido[4,3-*d*]pyrimidin-4(3*H*)-one

A mixed solution of 4-amino-2,6-dichloro-5-fluoronicotinic acid (8 g, 30.8 mmol) and thionyl chloride (200 mL) was stirred at 50 °C for 3 h. Then it was concentrated, and the residue was dissolved in acetone (50 mL) to obtain Solution 1. At room temperature, a mixed solution of ammonium thiocyanate (7 g, 92 mmol) in acetone solution (160 mL) was added dropwise to Solution 1, and the resulting reaction solution was continuously stirred at room temperature for 1 h. After the reaction was completed (monitored by LCMS), the reaction solution was poured into water, filtered, and the filter cake was dried to give 5,7-dichloro-8-fluoro-2-mercaptopyrido[4,3-*d*]pyrimidin-4(3*H*)-one (5 g). LCMS (m/z): 265.9 (M + H).

**Step F:** 5,7-dichloro-8-fluoro-2-(methylthio)pyrido[4,3-d]pyrimidin-4(3*H*)-one (Intermediate **D-1**)

At room temperature, a mixed solution of 5,7-dichloro-8-fluoro-2-mercaptopyrido[4,3-*d*]pyrimidin-4(3*H*)-one (5 g, 18.8 mmol), methanol (380 mL), aqueous sodium hydroxide solution (0.1 M, 380 mL, 380 mmol), and methyl iodide (5.3 g, 380 mmol) was stirred for 2 h. After the reaction was completed (monitored by LCMS), the reaction solution was poured into 1000 mL of water and acidified to pH ~ 6 with concentrated hydrochloric acid. The solution was filtered, and the filter cake was dried to obtain the product 5,7-dichloro-8-fluoro-2-(methylthio)pyrido[4,3-*d*]pyrimidin-4(3*H*)-one (4 g). LCMS (m/z): 279.9 (M + H).

**Step G:** 7-chloro-8-fluoro-5-methoxy-2-(methylthio)pyrido[4,3-d]pyrimidin-4(3*H*)-one (Intermediate **D-2**)

A mixture of 5,7-dichloro-8-fluoro-2-(methylthio)pyrido[4,3-*d*]pyrimidin-4(3*H*)-one (400 mg, 1.4 mmol), sodium methoxide (0.38 g, 7.5 mmol), DMA (10 mL), and methanol (2 mL) was stirred at 50 °C for 16 h. After the reaction was completed (monitored by LCMS), it was diluted with water, adjusted to pH ~ 3 with concentrated hydrochloric acid, filtered, and the filter cake was collected and dried to obtain the product 7-chloro-8-fluoro-5-methoxy-2-(methylthio)pyrido[4,3-*d*]pyrimidin-4(3*H*)-one (250 mg). LCMS (m/z): 276.0 (M + H).

**Step H:** *tert*-butyl (1*R*,5*S*)-3-(7-chloro-8-fluoro-5-methoxy-2-(methylthio)pyrido[4,3-d]pyrimidin-4-yl)-3,8-diazabicyclo[3.2.1]octane-8-carboxylate (Intermediate **D-2a**)

A mixed solution of 7-chloro-8-fluoro-5-methoxy-2-(methylthio)pyrido[4,3-*d*]pyrimidin-4(3*H*)-one (250 mg, 0.91 mmol), *tert*-butyl (1*R*,5*S*)-3,8-diazabicyclo[3.2.1]octane-8-carboxylate (290 mg, 1.4 mmol), BOP (620 mg, 1.4 mmol), and *N,N*-diisopropylethylamine (361 mg, 2.8 mmol) in DMF (5 mL) was stirred at 45 °C for 3 h. After the reaction was completed monitored by LCMS, the reaction solution was poured into water, filtered, and the filter cake was collected and dried to obtain *tert*-butyl (1*R*,5*S*)-3-(7-chloro-8-fluoro-5-methoxy-2-(methylthio)pyrido[4,3-d]pyrimidin-4-yl)-3,8-diazabicyclo[3.2.1]octane-8-carboxylate (300 mg) . LCMS (m/z): 470.3 (M + H).

## Intermediates D-3, D-3a and D-4, D-4a

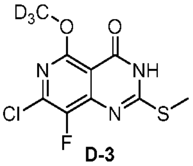
D-3 ,

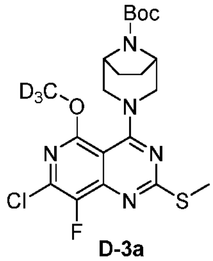
D-3a and

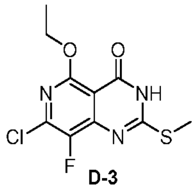
D-3 ,

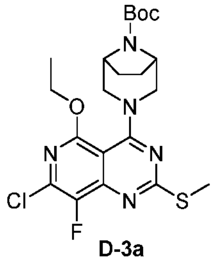
D-3a

The synthesis of intermediates D-3, D-3a and D-4, D-4a was carried out according to the synthesis of intermediates D-2, D-2a mentioned above.

## Intermediates E-1, E-1a and E-1b

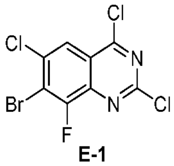
E-1 ,
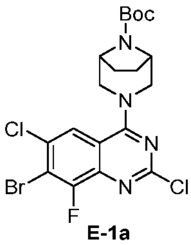
E-1a and
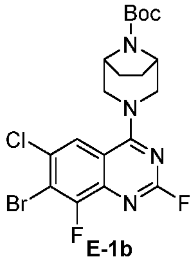
E-1b

7-bromo-2,4,6-trichloro-8-fluoroquinazoline (E-1)

*tert*-butyl ((1*R*,5*S*)-3-(7-bromo-6-chloro-2,8-difluoroquinazolin-4-yl)-3,8-diazabicyclo[3.2.1]octane-8-carboxylate (E-1a)

*tert*-butyl ((1*R*,5*S*)-3-(7-bromo-6-chloro-2,8-difluoroquinazolin-4-yl)-3,8-diazabicyclo[3.2.1]octane-8-carboxylate (E-1b)

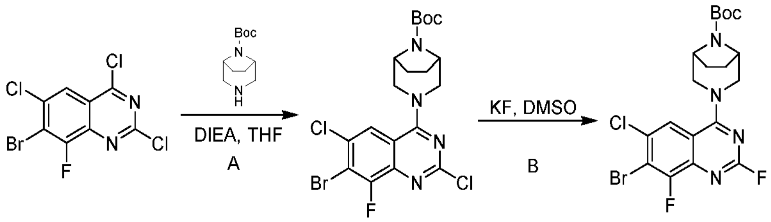


E-1 E-1a E-1b

**Step** A: *tert*-butyl ((1*R*,5*S*)-3 -(7-bromo-2,6-dichloro-8-fluoroquinazolin-4-yl)-3,8-diazabicyclo[3.2.1]octane-8-carboxylate

At room temperature, *tert*-butyl (1*R*,5*S*)-3,8-diazabicyclo[3.2.1]octane-8-carboxylate (1.77 g, 8.32 mmol) was added to a mixture of 7-bromo-2,4,6-trichloro-8-fluoroquinazoline (CAS: 1698028-11-3, 2.50 g, 7.57 mmol), DIEA (1.96 g, 15.1 mmol) and THF (50 mL). The resulting mixture was stirred for 1 h at room temperature. After the reaction was completed (monitored by LCMS), it was concentrated under reduced pressure and further purified by FCC ($SiO_2$, EA/PE=0-30%) to afford *tert*-butyl ((1*R*,5*S*)-3-(7-bromo-2,6-dichloro-8-fluoroquinazolin-4-yl)-3,8-diazabicyclo[3.2.1]octane-8-carboxylate (2.0 g, yield 52%) as a yellow solid. LCMS (m/z): 506.9.

**Step B:** *tert*-butyl ((1*R*,5*S*)-3-(7-bromo-6-chloro-2,8-difluoroquinazolin-4-yl)-3,8-diazabicyclo[3.2.1]octane-8-carboxylate

KF (2.29 g, 39.5 mmol) was added to a mixture of *tert*-butyl ((1*R*,5*S*)-3-(7-bromo-2,6-dichloro-8-fluoroquinazolin-4-yl)-3,8-diazabicyclo[3.2.1]octane-8-carb oxyl ate (2.00 g, 3.95 mmol) and DMSO (50 mL) at room temperature. The resulting mixture was heated to 110°C and stirred for 16 h. After the reaction was completed (monitored by LCMS), the reaction solution was cooled to room temperature, water (200 mL) was added, and the mixture was extracted with EA (50 mL × 3). The combined organic phases were washed with brine (200 mL), dried over anhydrous sodium sulfate, filtered, and concentrated. The crude product obtained was purified by FCC ($SiO_2$, EA/PE = 0-25%) to afford yellow solid *tert*-butyl ((1*R*,5*S*)-3-(7-bromo-6-chloro-2,8-difluoroquinazolin-4-yl)-3,8-diazabicyclo[3.2.1]octane-8-carboxylate (1.6 g, yield 83%). LC-MS (m/z): 489.0 (M + H) and 491.0 (M + H).

## Intermediate E-2b

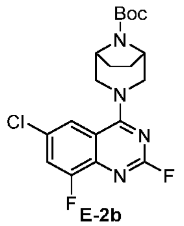

E-2b

*tert*-butyl (1*R*,5*S*)-3-(6-chloro-2,8-difluoroquinazolin-4-yl)-3,8-diazabicyclo[3.2.1]octane-8-carboxylate

Synthesis of intermediate E-2b was carried out according to the protocol described for intermediate E-1b, using 2,4,6-trichloro-8-fluoroquinazoline (CAS: 2205387-69-3) instead of 7-bromo-2,4,6-trichloro-8-fluoroquinazoline in Step A.

## Intermediates F-1, F-1a and F-1b

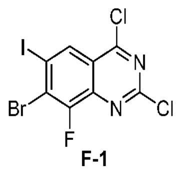

F-1 ,

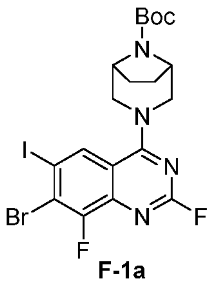

F-1a and

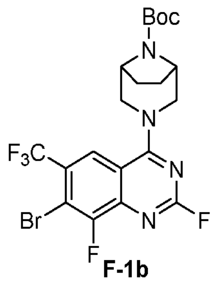

F-1b

7-bromo-2,4-dichloro-8-fluoro-6-iodoquinazoline (**F-1**)

*tert*-butyl (1*R*,5*S*)-3-(7-bromo-2,8-difluoro-6-iodoquinazolin-4-yl)-3,8-diazabicyclo[3.2.1]octane-8-carboxylate (**F-1a**)

*tert*-butyl (1*R*,5*S*)-3-(7-bromo-2,8-difluoro-6-(trifluoromethyl)quinazolin-4-yl)-3,8-diazabicyclo[3.2.1]octane-8-carboxylate (**F-1b**)

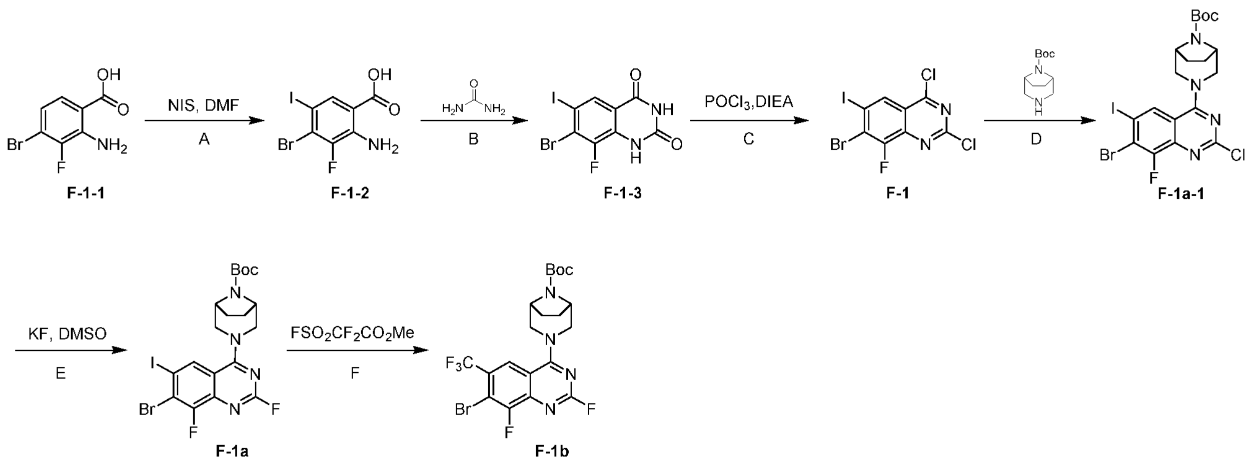


F-1-1 F-1-2 F-1-3 F-1 F-1a-1 F-1a F-1b

**Step A:** 2-amino-4-bromo-3-fluoro-5-iodobenzoic acid

NIS (50.6 g, 225 mmol) was added to a mixture of 2-amino-4-bromo-3-fluorobenzoic acid (50 g, 215 mmol) and DMF (500 m L) at room temperature, and the resulting mixture was stirred at 80°C for 3 h. After the reaction was completed (monitored by LCMS), the reaction solution was cooled, poured into water (2 L), and extracted with ethyl acetate (4 L). The

organic layer was dried over anhydrous sodium sulfate and concentrated by filtration to obtain 2-amino-4-bromo-3-fluoro-5-iodobenzoic acid (50 g, yield 65%) as a yellow solid. LCMS (m/z): 360.2 (M + H).

**Step B:** 7-bromo-8-fluoro-6-iodoquinazolin-2,4-(1*H*,3*H*)-dione

A mixture of compound 2-amino-4-bromo-3-fluoro-5-iodobenzoic acid (50 g, 139 mmol) and urea (168 g) was stirred for 2 h at 200 °C. After the reaction was completed, the system was cooled to room temperature, slurried with water, and the solid was collected by filtration. After drying, 7-bromo-8-fluoro-6-iodoquinazolin-2,4-(1*H*,3*H*)-dione (48 g, yield 90%) was obtained as a yellow solid. LCMS (m/z): 384.8 (M + H).

**Step C:** 7-bromo-2,4-dichloro-8-fluoro-6-iodoquinazoline

DIEA (40 mL) was slowly added to a mixture of 7-bromo-8-fluoro-6-iodoquinazolin-2,4-(1*H*,3*H*)-dione (20 g, 52 mmol) and phosphorus oxychloride (160 mL) at room temperature. The resulting mixture was stirred at 100°C for 16 h. After the reaction was completed (monitored by TLC), the reaction solution was concentrated, slowly poured into water, filtered to collect the filter cake. After drying, 7-bromo-2,4-dichloro-8-fluoro-6-iodoquinazoline (18.5 g, yield 85%) was obtained as a yellow solid.

**Step D:** *tert*-butyl (1*R*,5*S*)-3-(7-bromo-2-chloro-8-fluoro-6-iodoquinazolin-4-yl)-3,8-diazabicyclo[3.2.1]octane-8-carboxylate

A mixture of 7-bromo-2,4-dichloro-8-fluoro-6-iodoquinazoline (18.5 g, 44 mmol), *tert*-butyl (1*R*,5*S*)-3,8-diazabicyclo[3.2.1]octane-8-carboxylate (9.3 g, 44 mmol), DIEA (17 g, 132 mmol) and tetrahydrofuran (200 mL) was stirred for 1 h at room temperature. After the reaction was completed (monitored by TLC), the reaction solution was poured into water (2 L), filtered, and the solid was collected and dried to afford *tert*-butyl (1*R*,5*S*)-3-(7-bromo-2-chloro-8-fluoro-6-iodoquinazolin-4-yl)-3,8-diazabicyclo[3.2.1]octane-8-carboxylate (17.6 g, yield 67%) as a yellow solid. LCMS (m/z): 596.6 (M + H).

**Step E:** *tert*-butyl (1*R*,5*S*)-3-(7-bromo-2,8-difluoro-6-iodoquinazolin-4-yl)-3,8-diazabicyclo[3.2.1]octane-8-carboxylate

A mixture of *tert*-butyl (1*R*,5*S*)-3-(7-bromo-2-chloro-8-fluoro-6-iodoquinazolin-4-yl)-3,8-diazabicyclo[3.2.1]octane-8-carboxylate (10g, 16.8 mmol), potassium fluoride (8g, 138 mmol), and DMSO (100m L) were stirred at 110°C for 16h. After the reaction was completed (monitored by LCMS), the reaction solution was poured into water (2 L), filtered, the solid was collected. After drying, and *tert*-butyl (1*R*,5*S*)-3-(7-bromo-2,8-difluoro-6-iodoquinazolin-4-yl)-3,8-diazabicyclo [3.2.1]octane-8-carboxylate (9 g, yield 92%) was obtained as a yellow solid. LCMS (m/z): 583.3 (M + H).

**Step F:** *tert*-butyl (1*R*,5*S*)-3-(7-bromo-2,8-difluoro-6-(trifluoromethyl)quinazolin-4-yl)-3,8-diazabicyclo[3.2.1]octane-8-carboxylate

A mixture of *tert*-butyl (1*R*,5*S*)-3-(7-bromo-2,8-difluoro-6-iodoquinazolin-4-yl)-3,8-diazabicyclo[3.2.1]octane-8-carboxylate (1g, 1.7 mmol), methyl fluorosulfonyldifluoroacetate (660 mg, 0.44 mL, 3.4 mmol), cuprous iodide (1g, 5.2 mmol), HMPA (2 mL) and DMF (10 mL) was heated to 110 °C by microwave irradiation for 1 h. After the reaction was completed, the reaction solution was filtered, water was added and the mixture was extracted with ethyl acetate. The organic phase was dried over anhydrous sodium sulfate, filtered, and concentrated. The crude product obtained was purified with FCC ($SiO_2$, EA/PE = 0-20%) to afford *tert*-butyl (1*R*,5*S*)-3-(7-bromo-2,8-difluoro-6-(trifluoromethyl)quinazolin-4-yl)-3,8-diazabicyclo[3.2.1]octane-8-carboxylate (400 mg, yield 45%) as a yellow solid. LCMS (m/z): 523.6 (M + H).

## Intermediate F-1c

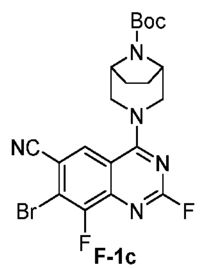

**F-1c**

*tert*-Butyl (1*R*,5*S*)-3-(7-bromo-6-cyano-2,8-difluoroquinazolin-4-yl)-3,8-diazabicyclo[3.2.1]octane-8-carboxylate

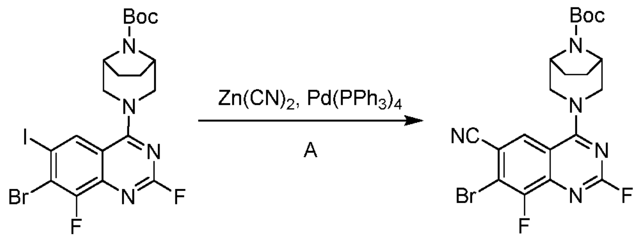


F-1a F-1c

**Step A:** *tert*-butyl (1*R*,5*S*)-3-(7-bromo-6-cyano-2,8-difluoroquinazolin-4-yl)-3,8-diazabicyclo[3.2.1]octane-8-carboxylate

Under nitrogen protection, a mixture of *tert*-butyl (1*R*,5*S*)-3-(7-bromo-2,8-difluoro-6-iodoquinazolin-4-yl)-3,8-diazabicyclo[3.2.1]octane-8-carboxylate (600 mg, 1.0 mmol), zinc cyanide (160 mg, 0.5 mmol), $Pd(PPh_3)_4$ (120 mg, 0.1 mmol) and DMF (10 m L) was stirred for 16 h at 110 °C. After the reaction was completed (monitored by LCMS), the reaction solution was filtered, water was added to the filtrate and extracted with ethyl acetate. The organic phase was dried over anhydrous sodium sulfate, filtered and concentrated. The crude product obtained was purified with FCC ($SiO_2$, EA/PE = 0-30%) to afford *tert*-butyl (1*R*,5*S*)-3-(7-bromo-6-cyano-2,8-difluoroquinazolin-4-yl)-3,8-diazabicyclo[3.2.1]octane-8-carboxylate (280 mg, yield 58%) as a yellow solid. LCMS (m/z): 480.0 (M + H).

## Intermediate G-1 and G-1a

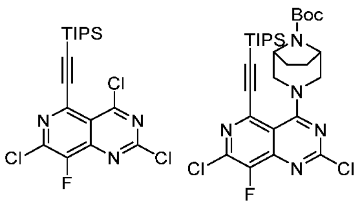

G-1 G-1a

2,4,7-trichloro-8-fluoro-5-(triisopropylsilyl)ethynyl)pyrido[4,3-d]pyrimidine (**G-1**)

*tert*-butyl (1*R*,5*S*)-3-(2,7-dichloro-8-fluoro-5-(triisopropylsilyl)ethynyl)pyrido[4,3-d]pyrimidin-4-yl)-3,8-diazabicyclo[3.2.1]octane-8-carboxylate (**G-1a**)

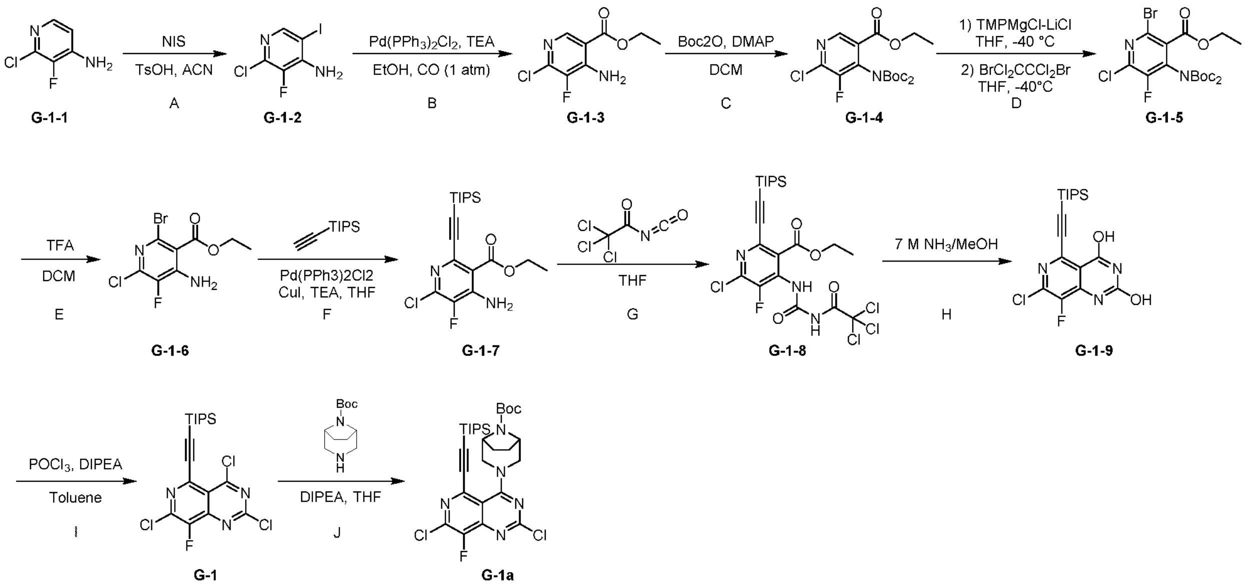


**Step** A: 2-chloro-3-fluoro-5-iodopyridin-4-amine

At room temperature, 2-chloro-3-fluoro-4-aminopyridine (25.0 g, 171 mmol) was dissolved in 250 mL of acetonitrile. Then NIS (35.4 g, 205 mmol) and *p*-toluenesulfonic acid monohydrate (3.24 g, 17.1 mmol) were added to the above system respectively. The resulting mixture was heated to 70 °C and stirred overnight. After the reaction was completed (monitored by LCMS), it was cooled to room temperature. The reaction solution was poured into water (500 mL) to quench the reaction and then extracted with ethyl acetate (800 mL × 3). The combined organic phases were washed successively with saturated $NaHCO_3$ solution, saturated $Na_2S_2O_3$ solution and brine, dried over anhydrous sodium sulfate, filtered and concentrated. The obtained crude product was purified by FCC ($SiO_2$, EA/PE = 0-50%) to obtain 2-chloro-3-fluoro-5-iodopyridin-4-amine (42 g, yield 90%) as a white solid. LCMS (m/z): 272.9 (M + H). $^1H$ NMR (400 MHz, DMSO-$d_6$) δ 8.10 (s, 1H), 6.69 (s, 2H). $^{19}F$ NMR (376 MHz, DMSO-$d_6$) δ-138.90.

**Step B:** ethyl 4-amino-6-chloro-5-fluoronicotinate

At room temperature, 2-chloro-3-fluoro-5-iodopyridin-4-amine (40 g, 147 mmol) was dissolved in 400 mL of absolute ethanol. The system was purged with CO three times. Then $Pd(PPh_3)_2Cl_2$ (10.3 g, 14.7 mmol) and TEA (95.0 g, 735 mmol) were added to the reaction flask, and the CO gas was purged three times. The resulting mixture was heated to 80 °C and reacted overnight under a CO gas atmosphere. After the reaction was completed (monitored by LCMS), it was cooled to room temperature. The reaction solution was filtered through celite. The filtrate was concentrated, and the obtained crude product was purified by FCC ($SiO_2$, EA/PE = 0-50%) to obtain ethyl 4-amino-6-chloro-5-fluoronicotinate (30 g, yield 93%) as a white solid. LCMS (m/z): 219.0 (M + H).

**Step C:** ethyl 4-(bis(tert-butoxycarbonyl)amino)-6-chloro-5-fluoronicotinate

Under stirring at room temperature, $Boc_2O$ (65.9 g, 302 mmol) was added dropwise to a solution of ethyl 4-amino-6-chloro-5-fluoronicotinate (30.0 g, 137 mmol) and DMAP (3.4 g, 27.5 mmol) in anhydrous dichloromethane (600 mL). After the addition was completed, the mixture was heated to 45 °C and reacted overnight. Imidazole (9.33 g, 137 mmol) was added to the system. After stirring for half an hour, the mixture was washed with saturated ammonium chloride solution (300 mL × 3), and the organic phase was separated. The organic phase was further washed with saturated brine (300 mL × 2), dried over anhydrous sodium sulfate, filtered and concentrated to dryness to obtain ethyl 4-(bis(tert-butoxycarbonyl)amino)-6-chloro-5-fluoronicotinate (45.9 g, yield 80%) as a yellow solid. LCMS (m/z): 419.1 (M + H).

**Step D:** ethyl 4-(bis(*tert*-butoxycarbonyl)amino)-2-bromo-6-chloro-5-fluoronicotinate

A solution of ethyl 4-(bis(*tert*-butoxycarbonyl)amino)-6-chloro-5-fluoronicotinate (45.0 g, 107 mmol) in anhydrous THF (450 mL) was cooled to -40 °C with a dry ice-acetonitrile bath. At this temperature, a THF solution of TMPMgCl-LiCl (161 mL, 161 mmol, 1 M) was added dropwise with stirring. After the addition was completed, the mixture was continuously stirred at this temperature for 4 h. Then a solution of dibromotetrachloroethane (42.0 g, 129 mmol) in THF

(100 mL) was added dropwise. The mixture was continuously stirred at -40 °C for 4 h. The reaction was quenched by adding 500 mL of saturated ammonium chloride solution, and then extracted with EtOAc (500 mL × 3). The combined organic phases were washed with brine, dried over anhydrous sodium sulfate, filtered and concentrated. The obtained crude product was purified by FCC ($SiO_2$, EA/DCM = 0-50%) to obtain ethyl 4-(bis(*tert*-butoxycarbonyl)amino)-2-bromo-6-chloro-5-fluoronicotinate (17.0 g, yield 32%) as a white solid. LCMS (m/z): 497.0 (M + H).

**Step E:** ethyl 4-amino-2-bromo-6-chloro-5-fluoronicotinate

Under stirring at room temperature, TFA (50 mL) was added at once to a solution of ethyl 4-(bis(*tert*-butoxycarbonyl)amino)-2-bromo-6-chloro-5-fluoronicotinate (9.5 g, 19.1 mmol) in dichloromethane (50 mL). After stirring at room temperature for 2 h, the mixture was concentrated to dryness. The residue was neutralized with 200 mL of saturated sodium bicarbonate solution, and then extracted with EtOAc (200 mL × 3). The combined organic phases were washed with brine, dried over anhydrous sodium sulfate, filtered and concentrated to obtain ethyl 4-amino-2-bromo-6-chloro-5-fluoronicotinate (5.6 g, yield 99%) as a white solid. LCMS (m/z): 296.9 (M + H).

**Step F:** ethyl 4-amino-6-chloro-5-fluoro-2-((triisopropylsilyl)ethynyl)nicotinate

At room temperature, ethyl 4-amino-2-bromo-6-chloro-5-fluoronicotinate (5.60 g, 18.8 mmol), $Pd(PPh_3)_2Cl_2$ (1.32 g, 1.88 mmol) and CuI (717 mg, 3.76 mmol) were dissolved in anhydrous THF (150 mL). The reaction flask was evacuated and purged with nitrogen three times. Triisopropylsilylacetylene (4.46 g, 24.5 mmol) and TEA (5.71 g, 56.5 mmol) were added, and the nitrogen was purged three times. The resulting mixture was heated to 45 °C and reacted for 2 h. After the reaction was completed (monitored by LCMS), it was cooled to room temperature. The reaction solution was filtered through celite. The filtrate was concentrated to dryness. The obtained crude product was purified by FCC ($SiO_2$, EA/PE = 0-10%) to obtain ethyl 4-amino-6-chloro-5-fluoro-2-((triisopropylsilyl)ethynyl)nicotinate (7 g, yield 93%) as a white solid. LCMS (m/z): 399.1 (M + H).

**Step G:** ethyl 6-chloro-5-fluoro-4-(3-(2,2,2-trichloroacetyl)ureido)-2-((triisopropylsilyl)ethynyl)nicotinate

Under stirring at room temperature, 2,2,2-trichloroacetyl isocyanate (3.97 g, 21.1 mmol) was added to a solution of ethyl 4-amino-6-chloro-5-fluoro-2-((triisopropylsilyl)ethynyl)nicotinate (7.00 g, 17.5 mmol) in anhydrous THF (150 mL). The resulting mixture was stirred at room temperature for 1 h. Then it was concentrated to dryness to obtain ethyl 6-chloro-5-fluoro-4-(3-(2,2,2-trichloroacetyl)ureido)-2-((triisopropylsilyl)ethynyl)nicotinate (11 g, crude product) as a white solid. It was used directly in the next step without purification. LCMS (m/z): 586.0 (M + H).

**Step H:** 7-chloro-8-fluoro-5-((triisopropylsilyl)ethynyl)pyrido[4,3-d]pyrimidine-2,4-diol

At room temperature, ethyl 6-chloro-5-fluoro-4-(3-(2,2,2-trichloroacetyl)ureido)-2-((triisopropylsilyl)ethynyl)nicotinate (11 g, crude product) was dissolved in an ammonia-methanol solution (50 mL, 7 M, 350 mmol). The resulting mixture was stirred at room temperature for 2 h. Then it was concentrated to dryness. The obtained crude product was slurried with PE/EA (100 mL/10 mL), filtered to obtain 7-chloro-8-fluoro-5-((triisopropylsilyl)ethynyl)pyrido[4,3-d]pyrimidin-2,4-diol (5.8 g, yield 84%) as a white solid. LCMS (m/z): 396.1 (M + H).

**Step I:** 2,4,7-trichloro-8-fluoro-5-((triisopropylsilyl)ethynyl)pyrido[4,3-*d*]pyrimidine

At room temperature, phosphorus oxychloride (1.55 g, 10.1 mmol) and TEA(1.31 g, 10.1 mmol) were added to a solution of 7-chloro-8-fluoro-5-((triisopropylsilyl)ethynyl)pyrido[4,3-*d*]pyrimidin-2,4-diol (1.00 g, 2.53 mmol) in anhydrous toluene (20 mL). The resulting mixture was heated to 100 °C and stirred overnight, then concentrated. The obtained crude product, 2,4,7-trichloro-8-fluoro-5-((triisopropylsilyl)ethynyl)pyrido[4,3-*d*]pyrimidine (crude product), was used directly in the next step.

**Step J:** *tert*-butyl (1*R*,5*S*)-3-(2,7-dichloro-8-fluoro-5-((triisopropylsilyl)ethynyl)pyrido[4,3-*d*]pyrimidin-4-yl)-3,8-diazabicyclo[3.2.1]octane-8-carboxylate

Under nitrogen atmosphere, a solution of 2,4,7-trichloro-8-fluoro-5-((triisopropylsilyl)ethynyl)pyrido[4,3-*d*]pyrimidine (the crude product obtained in Step I) in anhydrous dichloromethane (20 mL) was cooled to -40 °C. DIPEA (976 mg, 7.56 mmol) was added, followed by a solution of *tert*-butyl 3,8-diazabicyclo[3.2.1]octane-8-carboxylate (534 mg, 2.52 mmol) in DCM (2 mL). The resulting mixture was stirred at -40 °C for 1 h. The reaction was quenched by adding saturated ammonium chloride solution (30 mL) and extracted with DCM (30 mL × 3). The combined organic phases were dried and

concentrated. The obtained crude product was purified by FCC ($SiO_2$, EA/PE = 0-10%) to obtain *tert*-butyl (1*R*,5*S*)-3-(2,7-dichloro-8-fluoro-5-((triisopropylsilyl)ethynyl)pyrido[4,3-*d*]pyrimidin-4-yl)-3,8-diazabicyclo[3.2.1]octane-8-carboxylate (960 mg, two-step yield 63%) as a white solid. LCMS (m/z): 608.3 (M + H).

**Intermediate k1**

(7-fluoro-8-((triisopropylsilyl)ethynyl)-3-((triisopropylsilyl)oxy)naphthalen-1-yl) boric acid

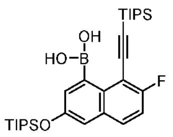

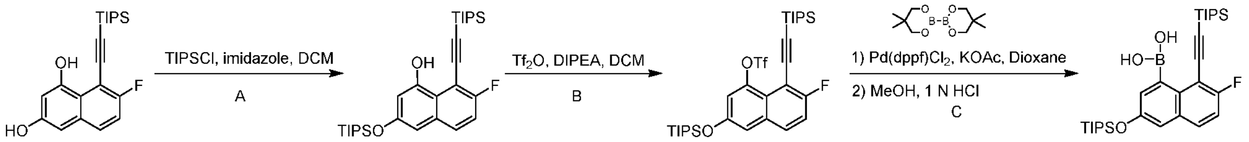


k1-1 k1-2 k1-3 k1

**Step A:** 7-fluoro-8-((triisopropylsilyl)ethynyl)-3-((triisopropylsilyl)oxy)naphthalen-1-ol

TIPSCl (177 g, 920 mmol) was added dropwise to a solution of 7-fluoro-8-((triisopropylsilyl)ethynyl)naphthalene-1,3-diol (300 g, 837 mmol) and imidazole (119 g, 1.76 mol) in DCM (3 L) under stirring in an ice bath. After the addition was completed, the system was slowly warmed to room temperature and stirred for 6 h. The reaction was completed (monitored by TLC). Water (900 mL) was added, and the mixture was stirred for 30 min, then the layers were separated. The aqueous phase was extracted with DCM (900 mL). The combined organic phases were dried over anhydrous sodium sulfate. After filtration and concentration to dryness, the product was purified by silica gel plug (PE/EA = 50:1) to obtain 7-fluoro-8-((triisopropylsilyl)ethynyl)-3-((triisopropylsilyl)oxy)naphthalen-1-ol (397 g, yield 92%).

**Step B:** 7-fluoro-8-(triisopropylsilyl)ethynyl)-3-((triisopropylsilyl)oxy)naphthalen-1-trifluoromethanesulfonate

At -45 ~-35 °C, trifluoromethanesulfonic anhydride (326 g, 1.16 mol) was added dropwise to a solution of 7-fluoro-8-((triisopropylsilyl)ethynyl)-3-((triisopropylsilyl)oxy)naphthalen-1-ol (397 g, 0.77 mol) and DIPEA (298 g, 2.31 mol) in DCM (4 L). After the addition was completed, the mixture was stirred for 0.5 h at the same temperature. The reaction was completed (monitored by TLC). The system was added to water (800 mL), the layers were separated, and the aqueous phase was extracted with DCM (1.2 L). The combined organic phases were dried over anhydrous sodium sulfate, filtered, and concentrated to dryness. After purification by silica gel plug (PE/EA = 50:1), 7-fluoro-8-(triisopropylsilyl)ethynyl)-3-((triisopropylsilyl)oxy)naphthalen-1-trifluoromethanesulfonate (469 g, yield 94%) was obtained.

**Step C:** (7-fluoro-8-((triisopropylsilyl)ethynyl)-3-((triisopropylsilyl)oxy)naphthalen-1-yl)boronic acid

Under nitrogen protection, Pd(dppf)$Cl_2$ (13.2 g, 18.2 mmol) was added to a solution of 7-fluoro-8-(triisopropylsilyl)ethynyl)-3-((triisopropylsilyl)oxy)naphthalen-1-trifluoromethanesulfonate (235 g, 0.36 mol), 5,5,5',5'-tetramethyl-2,2'-bis(1,3,2-dioxaborinane) (164 g, 0.73 mol) and potassium acetate (107 g, 1.1 mol) in dioxane (2.4 L). The system was heated to 85 °C and stirred for 20 h. The reaction was completed (monitored by TLC), then cooled to room temperature, filtered through celite, and rinsed with EA. After concentration, the product was purified by silica gel column (EA/PE = 0-5%) to obtain a crude compound.

The above-obtained crude compound was dissolved in methanol (1.2 L), and 1N HCl (2.4 L) was added. The resulting mixture was stirred at room temperature for 30 min. EA (2.4 L) was added, and the mixture was stirred for another 2 h. After standing for layer separation, the organic phase was washed successively with water (2.4 L) and saturated brine (2.4 L×2). After concentration, (7-fluoro-8-((triisopropylsilyl)ethynyl)-3-((triisopropylsilyl)oxy)naphthalen-1-yl)boronic acid (183 g, yield 92%) was obtained. $^1H$ NMR (400 MHz, Chloroform-d) δ 7.66 - 7.60 (m, 1H), 7.34 (d, *J* = 2.5 Hz, 1H), 7.24 - 7.19 (m, 1H), 7.18 (d, *J* = 2.5 Hz, 1H), 4.52 (s, 2H), 1.35 - 1.29 (m, 3H), 1.24 - 1.21 (m, 3H), 1.20 - 1.17 (m, 18H), 1.12 (d, *J* = 7.3 Hz, 18H). LCMS (m/z): 543.3 (M + H).

# Intermediate k2 and k3

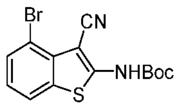

**k2**

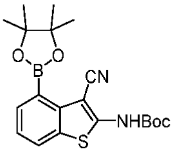

**k3**

*tert*-Butyl (3-cyano-4-(4,4,5,5-tetramethyl-1,3,2-dioxaborolan-2-yl)benzo[b]thiophen-2-yl)carbamate (**k3**)

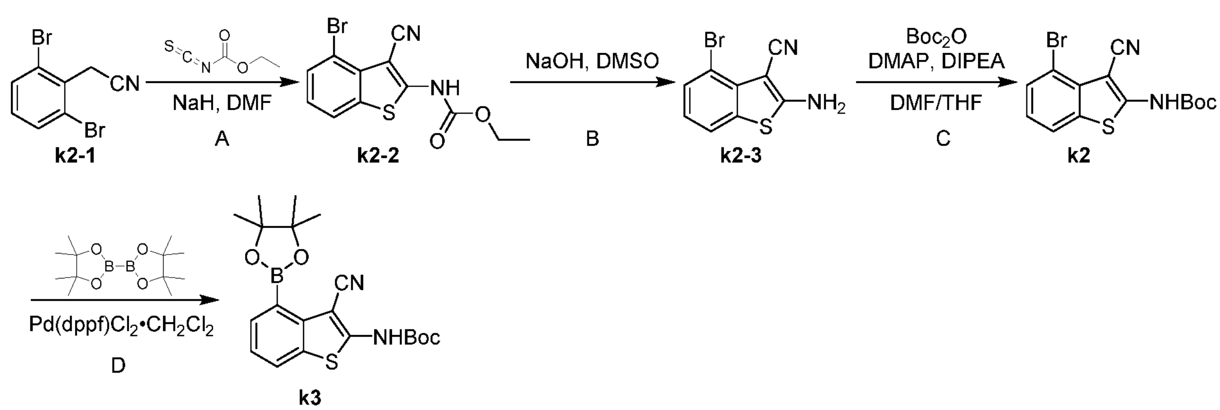


**Step A:** ethyl (4-bromo-3-cyanobenzo[b]thiophen-2-yl)carbamate

Sodium hydride (60%, 0.73 g, 22 mmol) was added to a solution of 2-(2,6-dibromophenyl)acetonitrile (5.0 g, 18 mmol) in DMF (50 mL) in an ice bath under nitrogen atmosphere. The reaction was stirred for 10 min at 0 °C. Ethyl isothiocyanatoformate (2.14 mL, 18 mmol) was slowly added to the reaction. After addition, the reaction was allowed to reach room temperature and stirred for 1 h. Then the reaction system was heated to 100 °C and stirred for 1 h. DMF was removed by concentration under reduced pressure. Water (100 mL) and ethyl acetate (10 mL) were added, and the resulting mixture was stirred at room temperature for 15 min. A large amount of yellow solid precipitated after stirring. The solid was filtered, washed with water and dried to obtain ethyl (4-bromo-3-cyanobenzo[b]thiophen-2-yl)carbamate (3.9 g) as a yellow solid. $^1H$ NMR (400 MHz, DMSO-$d_6$) δ 11.69 (s, 1H), 7.93 (dd, *J* = 8.0, 1.0 Hz, 1H), 7.60 (dd, *J* = 7.8, 1.0 Hz, 1H), 7.28 - 7.14 (m, 1H), 4.24 (q, *J* = 7.1 Hz, 2H), 1.29 (t, *J* = 7.1 Hz, 3H).

**Step B:** 2-amino-4-bromobenzo[*b*]thiophene-3-carbonitrile

Sodium hydroxide (3.7 g, 93 mmol) was added to a mixed solution of ethyl (4-bromo-3-cyanobenzo[*b*]thiophen-2-yl)carbamate (3.9 g, 12 mmol) in DMSO (12 mL) and water (18 mL). The reaction system was stirred at 125 °C for 16 h. The reaction system was cooled to room temperature, poured into 100 mL of ice-water, filtered, washed with water and dried to obtain 2-amino-4-bromobenzo[b]thiophene-3-carbonitrile (2.2 g) as a pale-yellow solid. $^1H$ NMR (400 MHz, DMSO-$d_6$) δ 7.96 (s, 2 H), 7.70 (dd, *J* = 7.9, 1.0 Hz, 1H), 7.46 (dd, *J* = 7.9, 1.0 Hz, 1H), 7.14 - 6.96 (m, 1H).

**Step C:** *tert*-butyl (4-bromo-3-cyanobenzo[b]thiophen-2-yl)carbamate

4-dimethylaminopyridine (104 mg, 0.85 mmol) and *N,N*-diisopropylethylamine (2.3 mL, 26 mmol) were successively added to a mixed solution of 2-amino-4-bromobenzo[b]thiophene-3-carbonitrile (2.2 g, 8.7 mmol) in DMF (29 mL) and THF (4.5 mL). Then, di-*tert*-butyl dicarbonate (2.2 mL, 9.5 mmol) was slowly added dropwise. After the addition was completed, the reaction system was stirred at room temperature for 24 h. After the reaction was completed, water (100 mL) was added to the reaction system. A yellow solid precipitated. The solid was collected by filtration, washed with water and dried to obtain *tert*-butyl (4-bromo-3-cyanobenzo[*b*]thiophen-2-yl)carbamate (1.4 g) as a pale-yellow solid.

**Step D:** *tert*-butyl (3-cyano-4-(4,4,5,5-tetramethyl-1,3,2-dioxaborolan-2-yl)benzo[*b*]thiophen-2-yl)carbamate

*tert-butyl* (4-bromo-3-cyanobenzo[b]thiophen-2-yl)carbamate (1.4 g, 3.9 mmol), bis(pinacolato)diboron (1.5 g, 5.9 mmol), potassium acetate (0.77 g, 7.8 mmol) and [1,1'-bis(diphenylphosphino)ferrocene]dichloropalladium(II) dichloromethane complex (0.3 g, 0.35 mmol) were successively added to a 50 mL round-bottom flask. Then, 1,4-dioxane (5 mL) was added to the reaction flask. The reaction was then purged with nitrogen. The reaction was heated to 105 °C for 3 h. After the reaction was completed, the filtrate was collected by filtration and concentrated. The crude product was purified by FCC ($SiO_2$, petroleum ether/tetrahydrofuran ~ 0-30%) to obtain *tert*-butyl (3-cyano-4-(4,4,5,5-tetramethyl-1,3,2-dioxaborolan-2-yl)benzo[*b*]thiophen-2-yl)carbamate (1.6 g) as a pale-yellow solid. LCMS (m/z): 423.0 (M + Na).

## Intermediates k4, k5, and k5-A

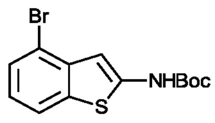

**k4** ,

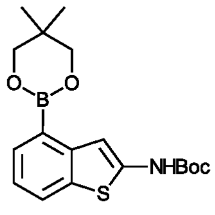

**k5** and

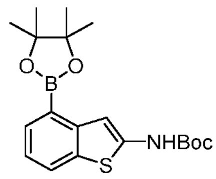

**k5-A**

*tert*-butyl (4-bromobenzo[*b*]thiophen-2-yl)carbamate (**k4**) and tert-butyl (4-(5,5-dimethyl-1,3,2-dioxaborolan-2-yl)benzo[*b*]thiophen-2-yl)carbamate (**k5**)

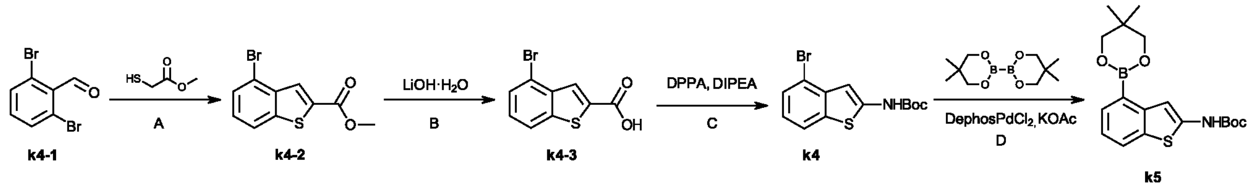


**Step A:** methyl 4-bromobenzo[*b*]thiophene-2-carboxylate

At room temperature, 2,6-dibromobenzaldehyde (15.7 g, 59.5 mmol), methyl mercaptoacetate (5.6 mL, 65.4 mmol), $K_2CO_3$ (15.7 g, 119.0 mmol) and DMF (200 mL) were successively added to a flask equipped with a magnetic stir bar, and the resulting mixture was heated to 110°C and stirred for 10 h. After the reaction was completed (monitored by TLC), the reaction solution was poured into 300 mL of water. A large amount of yellow solid was precipitated, filtered, and the filter cake was washed with water and dried at 50 °C to obtain the crude product methyl 4-bromobenzo[b]thiophene-2-carboxylate (18 g), LCMS (m/z): 270.9 (M + H).

**Step B:** 4-bromobenzo[*b*]thiophene-2-carboxylic acid

$LiOH \cdot H_2O$ (5.4 mL, 129.7 mmol) and methyl 4-bromobenzo[*b*]thiophene-2-carboxylate (7.0 g, 25.9 mmol) were successively added to a mixed solution of THF (10 mL) and $H_2O$ (2 mL). The resulting mixture was stirred at room temperature for 16 h. After the reaction was completed (monitored by LCMS), the pH of the system was adjusted to 3 with 1 M hydrochloric acid. The mixture was extracted with EA (60 mL × 3), dried over anhydrous $Na_2SO_4$, filtered and concentrated under reduced pressure to obtain crude 1-benzothiophene-2-carboxylic acid (8.0 g). LCMS (m/z): 256.9 (M + H).

**Step C:** *tert*-butyl (4-bromobenzo[*b*]thiophen-2-yl)carbamate

4-bromobenzo[*b*]thiophene-2-carboxylic acid (2.5 g), DPPA (3.2 mL, 14.7 mmol), DIEA (2.0 mL, 19.5 mmol), toluene (25 mL) and *tert*-butanol (1.4 mL, 14.7 mmol) were successively added to a reaction flask. The resulting mixture was stirred at 100 °C for 16 h. After the reaction was completed (monitored by LCMS), it was concentrated under reduced pressure. The crude product was purified by FCC ($SiO_2$, EA/PE = 0-30%) to obtain *tert*-butyl (4-bromo-1-benzothiophene-2-yl)carbamate (3.0 g). LCMS (m/z): 271.9 (M-56).

**Step D:** *tert*-butyl (4-(5,5-dimethyl-1,3,2-dioxaborinan-2-yl)benzo[*b*]thiophen-2-yl)carbamate

*tert*-butyl (4-bromobenzo[*b*]thiophen-2-yl)carbamate (2.9 g, 8.9 mmol), 5,5,5',5'-tetramethyl-2,2'-bis(1,3,2-dioxaborinane) (5.0 g, 22.3 mmol), DephosPd$Cl_2$ (0.64 g, 0.9 mmol), KOAc (2.6 g, 26.7 mmol) and 1,4-dioxane (30 mL) were successively added to a reaction flask. The system was purged with nitrogen three times. The reaction solution was stirred at 95 °C for 1 h. After the reaction was completed (monitored by LCMS), it was concentrated under reduced pressure. The crude product was purified by FCC ($SiO_2$, EA/PE = 0-30%) to obtain *tert*-butyl (4-(5,5-dimethyl-1,3,2-dioxaborinan-2-yl) benzo[*b*]thiophen-2-yl)carbamate (3.1 g, yield 98%).

The synthesis of intermediate k5-A was carried out following the protocol for Intermediate k5 using bis(pinacolato)diboron instead of 5,5,5',5'-tetramethyl-2,2'-bis(1,3,2-dioxaborinane) in Step D.

## Intermediate k6 and k7

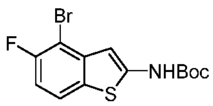

k6

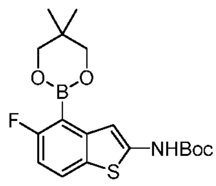

k7

*tert-butyl* (4-bromo-5-fluorobenzo[b]thiophen-2-yl)carbamate (**k6**) and *tert*-butyl (4-(5,5-dimethyl-1,3,2-dioxaborinan-2-yl)-5-fluorobenzo[*b*]thiophen-2-yl)carbamate (**k7**)

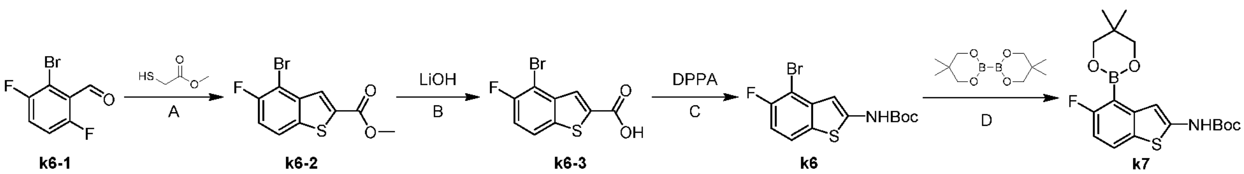


k6-1 k6-2 k6-3 k6 k7

**Step A:** methyl 4-bromo-5-fluoro-1-benzothiophene-2-carboxylate

Under nitrogen atmosphere at room temperature, potassium carbonate (37.5 g, 271.4 mmol) and methyl mercaptoacetate (17.3 g, 169.2 mmol) were added to a solution of 2-bromo-3,6-difluorobenzaldehyde (30 g, 135.7 mmol) in THF (500 mL). The resulting reaction solution was heated to 45 °C and stirred for 4 h, and then heated to 90 °C and stirred for 16 h. After the reaction was complete (monitored by LCMS), the reaction solution was concentrated to dryness. Water was added to the concentrated solution, and then extracted with ethyl acetate. The combined organic phases were washed with brine, dried over anhydrous sodium sulfate, filtered and concentrated to dryness. The obtained crude product was purified by FCC ($SiO_2$, DCM/PE = 0-8%) to obtain methyl 4-bromo-5-fluoro-1-benzothiophene-2-carboxylate (24.3 g, yield 62%) as an off-white solid. LCMS (m/z): 288.9 (M + H).

**Step B:** 4-bromo-5-fluoro-1-benzothiophene-2-carboxylic acid

Under nitrogen atmosphere, lithium hydroxide monohydrate (10.6 g, 252.2 mmol) was added to a solution of methyl 4-bromo-5-fluoro-1-benzothiophene-2-carboxylate (24.3 g, 84.1 mmol) in THF (170 mL) and water (57 mL). The resulting mixture was continuously stirred at room temperature for 3 h. After the reaction was completed (monitored by LCMS), the reaction solution was concentrated to dryness. Dilute hydrochloric acid aqueous solution (1N) was added to the concentrated residue until pH ≈ 5. A solid precipitated, and the mixture was continuously stirred at room temperature for 0.5 h, then filtered. The filter cake was rinsed with water and dried in vacuum to obtain 4-bromo-5-fluoro-1-benzothiophene-2-carboxylic acid (22.5 g, yield 98%) as an off-white solid. LCMS (m/z): 274.9 (M + H).

**Step C:** *tert*-butyl (4-bromo-5-fluorobenzo[*b*]thiophen-2-yl)carbamate

Under nitrogen atmosphere, triethylamine (11.6 g, 115.4 mmol) and diphenylphosphoryl azide (27.2 g, 98.9 mmol) were added to a solution of 4-bromo-5-fluoro-1-benzothiophene-2-carboxylic acid (22.5 g, 82.4 mmol) in anhydrous toluene (135 mL) and anhydrous *tert*-butanol (45 mL). The resulting mixture was stirred at 100 °C for 16 h. After the reaction was completed (monitored by LCMS), the reaction mixture was poured into 100 mL saturated aqueous sodium bicarbonate solution and then extracted with ethyl acetate. The combined organic phases were washed with saturated brine, dried over anhydrous sodium sulfate, filtered and concentrated to dryness. The obtained crude product was purified by slurrying with EA/PE = 1/10 (80 mL) to obtain *tert*-butyl (4-bromo-5-fluorobenzo[*b*]thiophen-2-yl)carbamate (28 g, yield 98%) as a yellow solid.. LCMS (m/z): 289.9 (M + H).

**Step D:** *tert*-butyl (4-(5,5-dimethyl-1,3,2-dioxaborinan-2-yl)-5-fluorobenzo[*b*]thiophen-2-yl)carbamate

Under nitrogen atmosphere at room temperature, neopentyl glycol diborate (4.88 g, 21.7 mmol), potassium acetate (2.83 g, 28.9 mmol) and DPEphosPd$Cl_2$ (1.04 g, 1.45 mmol) were added to a solution of *tert*-butyl (4-bromo-5-fluorobenzo[b]thiophen-2-yl)carbamate (5 g, 14.5 mmol) in 1,4-dioxane (80 mL). The resulting reaction solution was heated to 90 °C and stirred for 16 h. After the reaction was completed (monitored by LCMS), the reaction solution was filtered through celite. The filter cake was rinsed with ethyl acetate, and the filtrate was concentrated to dryness. The obtained crude product was purified by FCC ($SiO_2$, EA/PE = 0-10%) to obtain *tert*-butyl (4-(5,5-dimethyl-1,3,2-dioxaborinan-2-yl)-5-fluorobenzo[*b*]thiophen-2-yl)carbamate (3.2 g, yield 58%) as a white solid. LCMS (m/z): 256.0 (M + H-68-56). $^1$H NMR (400 MHz, DMSO-$d_6$) δ 10.73 (s, 1H), 7.86 - 7.77 (m, 1H), 7.14 (d, *J* = 0.7 Hz, 1H), 6.90 (dd, *J* = 9.8, 8.6 Hz, 1H), 3.81 (s, 4H), 1.49 (s, 9H), 1.02 (s, 6H).

## Intermediate k8 and k9

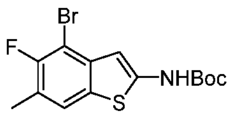

**k8**

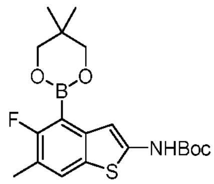

**k9**

*tert*-butyl (4-bromo-5-fluoro-6-methylbenzo[*b*]thiophen-2-yl)carbamate (**k8**) and *tert*-butyl (4-(5,5-dimethyl-1,3,2-dioxaborinan-2-yl)-5-fluoro-6-methylbenzo[b]thiophen-2-yl)carbamate (k9)

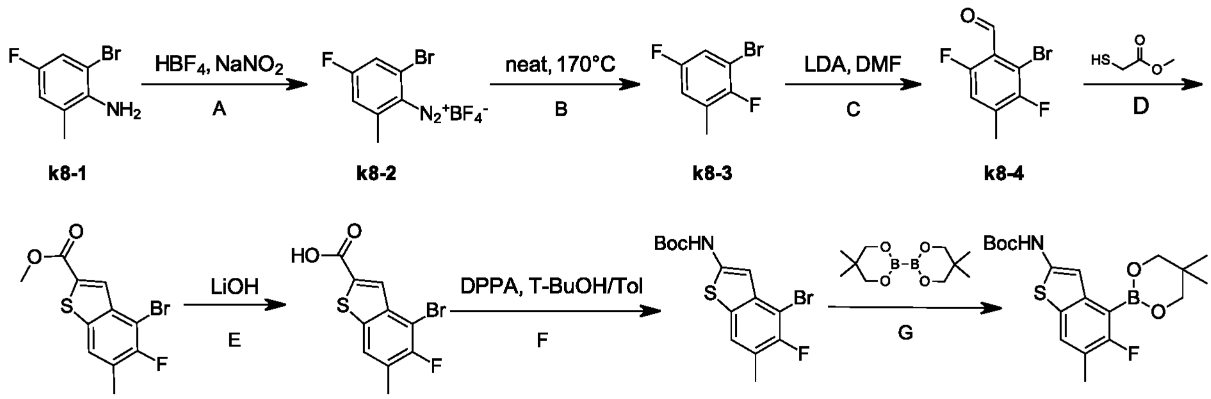


k8-1 k8-2 k8-3 k8-4 k8-5 k8-6 k8 k9

**Step A:** 1-(2-bromo-4-fluoro-6-methylphenyl)diazonium tetrafluoroborate

A solution of sodium nitrite (9.5g, 138.2 mmol) in water (20 mL) was slowly added to 2-bromo-4-fluoro-6-methylaniline (23.5g, 115.2 mmol) in aqueous fluoroboric acid (140 mL, 50 wt%) under nitrogen atmosphere at 0-5°C. The resulting mixture was stirred at 0 °C for 1 h. After the reaction was completed (monitored by LCMS), the reaction was

filtered and the filter cake was washed with aqueous fluoroboric acid (50 mL, 50 wt%) and ethyl acetate (50 mL). The filter cake was dried under vacuum to give the off-white solid product 1-(2-bromo-4-fluoro-6-methylphenyl)diazonium tetrafluoroborate (31 g, yield 89%). LCMS (m/z): 216.9 (M + H).

**Step B:** 1-bromo-2,5-difluoro-3-methylbenzene

Under nitrogen atmosphere, 1-(2-bromo-4-fluoro-6-methylphenyl)diazonium tetrafluoroborate was added to a single-neck bottle with a stir bar. The resulting mixture was heated to 170 °C for stirring 7 h. After the reaction was completed (monitored by LCMS), the crude product obtained was purified by FCC ($SiO_2$, PE) 1-bromo-2,5-difluoro-3-methylbenzene (16 g, yield 76%) as a colorless liquid. GCMS (m/z): 206.0 (M). $^1H$ NMR (400 MHz, DMSO-$d_6$) δ 7.55 - 7.48 (m, 1H), 7.29 - 7.22 (m, 1H), 2.28 (s, 3H).

**Step C:** 2-bromo-3,6-difluoro-4-methylbenzaldehyde

To a solution of 1-bromo-2,5-difluoro-3-methylbenzene (16 g, 77.3 mmol) in anhydrous THF (150 mL) under nitrogen atmosphere at -70 °C, LDA (46 mL, 2M THF solution, 92.7 mmol) was added slowly dropwise. The reaction was stirred at the same temperature for 40 min, after which anhydrous DMF (17 g, 231.9 mmol) was added to the reaction. The resulting mixture was stirred at - 70 °C for 1h. After the reaction was completed (monitored by LCMS), the reaction was poured into 100 mL of saturated aqueous ammonium chloride solution and extracted with ethyl acetate. The combined organic phase was washed with brine, dried over anhydrous sodium sulfate, filtered and concentrated to dryness, and the resulting crude product was purified by FCC ($SiO_2$, EA/PE = 0 - 4%) to obtain 2-bromo-3,6-difluoro-4-methylbenzaldehyde (15.5 g, yield 85%) as a pale-yellow solid. LCMS (m/z): 236.9 (M + H).

**Step D:** methyl 4-bromo-5-fluoro-6-methylbenzo[b]thiophene-2-carboxylate

Potassium carbonate (18.2 g, 131.9 mmol) and methyl mercaptoacetate (8.4 g, 79.1 mmol) were added to a solution of 2-bromo-3,6-difluoro-4-methylbenzaldehyde (15.5 g, 66.0 mmol) in tetrahydrofuran (200 mL) under nitrogen atmosphere at room temperature. The resulting mixture was heated to 45 °C and stirred for 4 h and then heated to 90 °C and stirred for 16 h. After the reaction was completed (monitored by LCMS), the reaction was concentrated to dryness, water was added to the residue and then extracted with ethyl acetate. The combined organic phase was washed with brine, dried over anhydrous sodium sulfate, filtered and concentrated to dryness, and the resulting crude product was purified by slurrying in EA (100 mL) to afford methyl 4-bromo-5-fluoro-6-methylbenzo[b]thiophene-2-carboxylate (7.5 g, yield 37%) as a pale-yellow solid. LCMS (m/z): 302.9 (M + H).

**Step E:** 4-bromo-5-fluoro-6-methylbenzo[*b*]thiophene-2-carboxylic acid

To a solution of methyl 4-bromo-5-fluoro-6-methylbenzo[*b*]thiophene-2-carboxylate (7.5 g, 24.7 mmol) in tetrahydrofuran (60 mL) and water (20 mL), lithium hydroxide monohydrate (3.1 g, 74.3 mmol) was added. The resulting mixture was stirred at room temperature for 3 h. After the reaction was completed (monitored by LCMS), the reaction was concentrated. Dilute hydrochloric acid aqueous solution (1 N) was added to the concentrate until pH ≈ 5, and a solid precipitated. The mixture was further stirred at room temperature for 0.5 h and then filtered. The filter cake was washed with water and dried under vacuum to obtain 4-bromo-5-fluoro-6-methylbenzo[*b*]thiophene-2-carboxylic acid (6.7 g, yield 94%) as an off-white solid. LCMS (m/z): 288.9 (M + H).

**Step F:** *tert*-butyl (4-bromo-5-fluoro-6-methylbenzo[*b*]thiophen-2-yl)carbamate

Under nitrogen atmosphere, to a solution of 4-bromo-5-fluoro-6-methylbenzo[*b*]thiophene-2-carboxylic acid (6.7 g, 23.3 mmol) in anhydrous toluene (40 mL) and anhydrous *tert*-butanol (13 mL), triethylamine (3.3g, 32.6 mmol) and diphenyl azide phosphate (7.7g, 27.9 mmol) were added. The resulting mixture was stirred at 100 °C for 16 h. After the reaction was completed (monitored by LCMS), the reaction was poured into 100 mL saturated aqueous sodium bicarbonate solution and then extracted with ethyl acetate. The combined organic phase was washed with brine, dried over anhydrous sodium sulfate, filtered, concentrated to dryness. The crude product was purified by slurrying in EA/PE = 1/10 (30 mL) to afford *tert*-butyl (4-bromo-5-fluoro-6-methylbenzo[*b*]thiophen-2-yl)carbamate (7.1 g, yield 85%) as a yellow solid. LCMS (m/z): 303.9 (M + H-56).

**Step G:** *tert*-butyl (4-(5,5-dimethyl-1,3,2-dioxaborinan-2-yl)-5-fluoro-6-methylbenzo[b]thiophen-2-yl)carbamate

To a solution of *tert*-butyl (4-bromo-5-fluoro-6-methylbenzo[*b*]thiophen-2-yl)carbamate (3.0 g, 8.3 mmol) in 1,4-dioxane (48 mL) at room temperature, neopentyl glycol diborate (2.8g, 12.5 mmol), potassium acetate (1.63g, 16.6 mmol) and DPEphosPd$Cl_2$ (594 mg, 0.83 mmol) were added. The resulting mixture was heated to 90 °C and stirred for 16 h. After the reaction was completed (monitored by LCMS), the reaction was filtered through celite, the filter cake was eluted by ethyl acetate, and the filtrate was concentrated and dried, and the resulting crude product was purified by FCC ($SiO_2$, EA/PE = 0-10%) to obtain *tert*-butyl (4-(5,5-dimethyl-1,3,2-dioxaborinan-2-yl)-5-fluoro-6-methylbenzo[b]thiophen-2-yl) carbamate (1.5g, yield 46%) as a white solid product. LCMS (m/z): 270.0 (M + H-68-56). $^1$H NMR (400 MHz, DMSO-$d_6$) δ 10.65 (s, 1H), 7.68 (d, *J* = 7.1 Hz, 1H), 7.06 (s, 1H), 3.80 (s, 4H), 2.24 (d, 3H), 1.48 (s, 9H), 1.02 (s, 6H).

## Intermediate k10 and k11

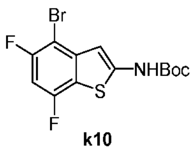
k10
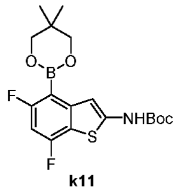
k11

*tert*-butyl (4-bromo-5,7-difluorobenzo[b]thiophen-2-yl)carbamate (k10) and tert-butyl (4-(5,5-dimethyl-1,3,2-dioxaborinan-2-yl)-5,7-difluorobenzo[b]thiophen-2-yl)carbamate (k11)

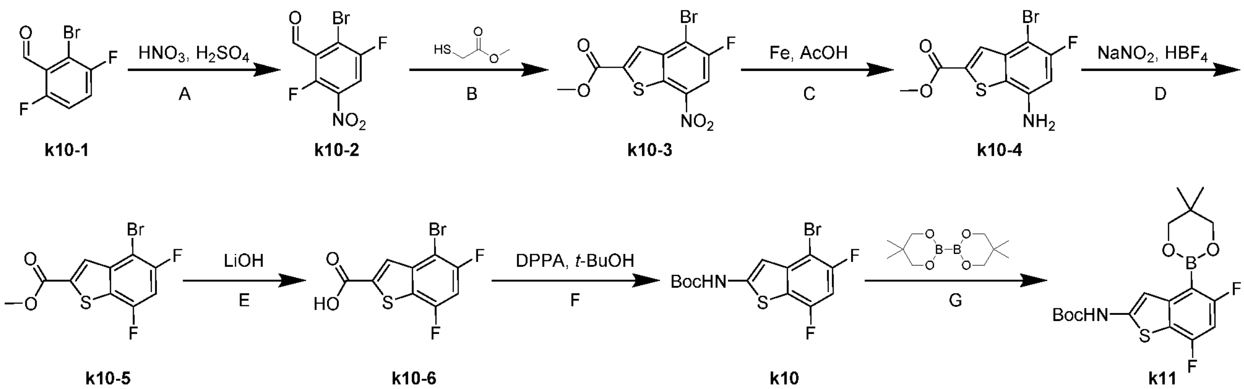

k10-1 k10-2 k10-3 k10-4 k10-5 k10-6 k10 k11

**Step A:** 2-bromo-3,6-difluoro-5-nitrobenzaldehyde

Concentrated sulfuric acid (90 mL) was slowly added dropwise to fuming nitric acid (42.7 g, 678.7 mmol) in a three-neck bottle at 0 °C with stirring, the resulting mixture was stirred at the same temperature for 10 min. 2-bromo-3,6-difluorobenzaldehyde (30 g, 135.7 mmol) was added to the reaction in batches and the reaction was stirred at 0 °C for 10 min. The resulting reaction solution was stirred at room temperature for 1 h. After the reaction was completed (monitored by LCMS), the reaction solution was slowly poured into ice-cold water and extracted with ethyl acetate. The combined organic phase was washed with brine, dried over anhydrous sodium sulfate, filtered and concentrated to dryness, and the resulting crude product was purified by FCC ($SiO_2$, EA/PE = 0 - 10%) to obtain 2-bromo-3,6-difluoro-5-nitrobenzaldehyde (31.3 g, yield 87%) as a pale-yellow solid. $^1$H NMR (400 MHz, DMSO-$d_6$) δ 10.18 (s, 1H), 8.61 (dd, *J* = 8.0, 6.5 Hz, 1H)

**Step B:** methyl 4-bromo-5-fluoro-7-nitrobenzo[b]thiophene-2-carboxylate

Potassium carbonate (23.4g, 169 mmol) and methyl mercaptoacetate (14.4g, 135 mmol) were added to a solution of 2-bromo-3,6-difluoro-5-nitrobenzaldehyde (30.0g, 113 mmol) in DMF (300 mL) at 0°C. The resulting mixture was stirred at room temperature for 12 h. After the reaction was completed (monitored by LCMS), the reaction was slowly added to ice-cold water, filtered, and the filter cake was purified by slurring (EA/PE = 2/1) to afford methyl 4-bromo-5-fluoro-7-nitrobenzo[*b*]thiophene-2-carboxylate (15 g, yield 40%) as a yellow solid. LCMS (m/z): 333.9 (M + H).

**Step C:** methyl 7-amino-4-bromo-5-fluorobenzo[*b*]thiophene-2-carboxylate

To a solution of methyl 4-bromo-5-fluoro-7-nitrobenzo[b]thiophene-2-carboxylate (15.0 g, 44.9 mmol) in ethanol

(130 mL) and acetic acid (20 mL), iron powder (12.0 g, 224 mmol) was added at 0°C, and the reaction was stirred at room temperature for 2 h. After the reaction was completed (monitored by LCMS), the reaction was filtered through celite. The filtrate was washed with water, saturated sodium bicarbonate aqueous solution and brine. The organic phase was dried over anhydrous sodium sulfate, filtered and concentrated to dryness, and the target product methyl 7-amino-4-bromo-5-fluorobenzo[*b*]thiophene-2-carboxylate (10.0 g, yield 74%) was obtained. LCMS (m/z): 303.9 (M + H).

**Step D:** methyl 4-bromo-5,7-difluorobenzo*[*b]thiophene-2-carboxylate

To a solution of methyl 7-amino-4-bromo-5-fluorobenzo[*b*]thiophene-2-carboxylate (9.5g, 31.2 mmol) in tetrafluoroboric acid (80 mL, 40 wt% aqueous solution), sodium nitrite (4.1 g, 46.9 mmol) was added, and the reaction was stirred at room temperature for 1 h. After LCMS monitoring showed that the starting material was completely converted to the diazonium salt, the reaction solution was filtered, and the filter cake was collected as a yellow solid. The resulting yellow solid was stirred at 200°C for 20 min. After the reaction was completed (monitored by LCMS), the crude product was purified by FCC ($SiO_2$, EA/PE = 0 - 45%) to afford methyl 4-bromo-5,7-difluorobenzo[b]thiophene-2-carboxylate (1.6 g, yield 17%) as a yellow solid. $^1$H NMR (400 MHz, DMSO-$d_6$) δ 8.03 (d, *J* = 3.3 Hz, 1H), 7.81 (dd, *J* = 9.8, 9.2 Hz, 1H), 3.93 (s, 3H). $^{19}$F NMR (376 MHz, DMSO-$d_6$) δ-106.95 ~-106.97,-112.37 ~-112.40 and 4-bromo-5,7-difluorobenzo[*b*]thiophene-2-carboxylic acid (1.4 g, yield 15%). $^1$H NMR (400 MHz, DMSO-$d_6$) δ 14.11 (s, 1H), 8.01 (d, *J* = 3.3 Hz, 1H), 7.83 - 7.75 (m, 1H). $^{19}$F NMR (376 MHz, DMSO-$d_6$) δ-107.30-107.32,-112.50-112.52.

**Step E:** 4-bromo-5,7-difluorobenzo[*b*]thiophene-2-carboxylic acid

To a solution of methyl 4-bromo-5,7-difluorobenzo[*b*]thiophene-2-carboxylate (1.6 g, 5.2 mmol) in tetrahydrofuran (10 mL), methanol (3 mL), and water (3 mL), lithium hydroxide monohydrate (656 mg, 15.6 mmol) was added and the reaction was stirred at room temperature for 2 h. After the reaction was completed (monitored by LCMS), water (30 mL) and 1N hydrochloric acid solution (5 mL) were added to the reaction solution and then extracted with ethyl acetate. The combined organic phase was washed with brine, dried over anhydrous sodium sulfate, filtered, concentrated and dried to obtain the crude product 4-bromo-5,7-difluorobenzo[b]thiophene-2-carboxylic acid (1.4 g, yield 92%).

**Step F:** *tert*-butyl (4-bromo-5,7-difluorobenzo[*b*]thiophen-2-yl)carbamate

To a solution of 4-bromo-5,7-difluorobenzo[*b*]thiophene-2-carboxylic acid (2.8 g, 9.5 mmol) in toluene (40 mL) and *tert*-butanol (10 mL), triethylamine (2.9 g, 28.4 mmol) and diphenyl phosphoryl azide (3.7 g, 14.3 mmol) were added at room temperature. The resulting mixture was heated to 100 °C and stirred for 12 h. After the reaction was completed (monitored by LCMS), the reaction was concentrated to dryness, and the resulting crude product was purified by FCC ($SiO_2$, EA/PE = 0-15%) to afford *tert*-butyl (4-bromo-5,7-difluorobenzo[*b*]thiophen-2-yl)carbamate (3.0 g, yield 86%) as a yellow solid. LCMS (m/z): 307.8 (M-56).

**Step G:** *tert*-butyl (4-(5,5-dimethyl-1,3,2-dioxaborinan-2-yl)-5,7-difluorobenzo[*b*]thiophen-2-yl)carbamate

Under nitrogen atmosphere, to a solution of *tert*-butyl (4-bromo-5,7-difluorobenzo[*b*]thiophen-2-yl)carbamate (2.5 g, 6.8 mmol) in 1,4-dioxane (40 mL), neopentyl glycol diborate (2.0 g, 8.9 mmol), potassium acetate (2.0 g, 20.6 mmol) and DPEphosPd$Cl_2$ (501 mg, 0.7 mmol) were added at room temperature. The resulting mixture was heated to 90°C and stirred for 5 h. After the reaction was completed (monitored by GCMS), the reaction was concentrated to dryness, and the resulting crude product was purified by FCC ($SiO_2$, EA/PE = 0-10%) to obtain *tert-butyl* (4-(5,5-dimethyl-1,3,2-dioxaborinan-2-yl)-5,7-difluorobenzo[*b*]thiophen-2-yl)carbamate (1.4g, yield 51%) as a yellow solid product. $^1$H NMR (400 MHz, DMSO-$d_6$) δ 10.98 (s, 1H), 7.24 (d, *J* = 3.8 Hz, 1H), 7.00 - 6.91 (m, 1H), 3.80 (s, 4H), 1.01 (s, 6H). $^{19}$F NMR (376 MHz, DMSO-$d_6$) δ-103.69 --103.85,-111.30 --111.46.

## Intermediate k12 and k13

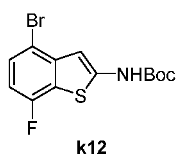
k12

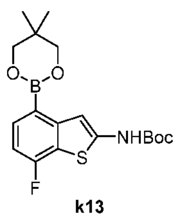
k13

*tert*-butyl (4-bromo-7-fluorobenzo[b]thiophen-2-yl)carbamate (**k12**) and *tert*-butyl (4-(5,5-dimethyl-1,3,2-dioxaborinan-2-yl)-7-fluorobenzo[b]thiophen-2-yl)carbamate (**k13**)

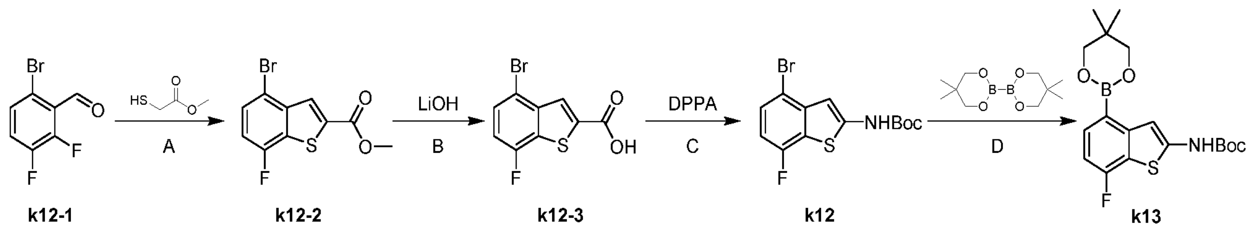


**k12-1** **k12-2** **k12-3** **k12** **k13**

**Step A:** methyl 4-bromo-7-fluorobenzo[*b*]thiophene-2-carboxylate

At room temperature, 6-bromo-2,3-difluorobenzaldehyde (15 g, 68.2 mmol), methyl mercaptoacetate (6.7 mL, 75 mmol), $K_2CO_3$ (18.8 g, 13.6 mmol), DMF (150 mL) were successively added to a reaction flask with a stir bar, and the resulting mixture was heated to 110 °C and stirred for 10 h. After the reaction was completed (monitored by TLC), the reaction was poured into 300 mL of water. The large amount of yellow solid precipitated was filtered, and the filter cake was washed with water, dried at 50 °C to obtain methyl 4-bromo-7-fluorobenzo[b]thiophene-2-carboxylate (13 g, yield 66%), GCMS (m/z): 288/290 ($M^{\bullet+}$). $^1H$ NMR (400 MHz, Chloroform-d) δ 8.16 - 8.13 (m, 1H), 7.55 - 7.47 (m, 1H), 7.08 - 6.98 (m, 1H), 3.98 (s, 3H). $^{19}F$ NMR (376 MHz, Chloroform-d) δ-116.23.

**Step B:** 4-bromo-7-fluorobenzo[*b*]thiophene-2-carboxylic acid

LiOH·$H_2O$ (7.3 g, 173.6 mmol) and methyl 4-bromo-7-fluorobenzo[*b*]thiophene-2-carboxylate (10 g, 34.7 mmol) was added successively to a mixture of THF (100 mL) and $H_2O$ (20 mL), and the resulting mixture was stirred at room temperature for 16 h. After the reaction was completed (monitored by LCMS), the pH of the system was adjusted to 3 with 1 M hydrochloric acid, and the mixture was extracted with EA (80 mL × 3). The combined organic phase was dried over anhydrous $Na_2SO_4$, filtered, and concentrated under reduced pressure to give 4-bromo-7-fluorobenzo[*b*]thiophene-2-carboxylic acid (9.3 g, yield 98%).

**Step C:** *tert*-butyl (4-bromo-7-fluorobenzo[*b*]thiophen-2-yl)carbamate

The 4-bromo-7-fluorobenzo[*b*]thiophene-2-carboxylic acid (9.3 g), DPPA (11 mL, 51 mmol), DIPEA (8.8 mL, 51 mmol), toluene (100 mL), *tert*-butanol (4.8 mL, 51 mmol) were successively added to a reaction flask and the resulting mixture was stirred 16 h at 100°C. After the reaction was completed (monitored by LCMS), the crude product was concentrated under reduced pressure and purified by FCC ($SiO_2$, EA/PE = 0-30%) to obtain tert-butyl (4-bromo-7-fluorobenzo[*b*]thiophen-2-yl)carbamate (10 g, yield 85%). $^1H$ NMR (400 MHz, Chloroform-*d*) δ 7.40 - 7.34 (m, 2H), 6.84 - 6.73 (m, 2H), 1.56 (s, 9H). $^{19}F$ NMR (376 MHz, Chloroform-*d*) δ-118.33.

**Step D:** *tert*-butyl (4-(5,5-dimethyl-1,3,2-dioxaborinan-2-yl)-7-fluorobenzo[*b*]thiophen-2-yl) carbamate

*tert*-butyl (4-bromo-7-fluorobenzo[*b*]thiophen-2-yl)carbamate (10 g, 29 mmol), 5,5,5',5'-tetramethyl-2,2'-bis(1,3,2-dioxaborinane) (24.9 g, 110 mmol), DPEPhosPd$Cl_2$ (2.1 g, 2.9 mmol), KOAc (8.5 g, 87 mmol), 1,4-dioxane (150 mL) were successively added to a reaction flask, and degassed with nitrogen for three times. The resulting mixture was stirred for 1 h at 95 °C. After the reaction was completed (monitored by LCMS), the crude product was concentrated under reduced pressure and subjected to FCC ($SiO_2$, EA/PE = 0-30%) purification to obtain *tert*-butyl (4-(5,5-dimethyl-1,3,2-dioxaborinan-2-yl)-7-fluorobenzo[*b*]thiophen-2-yl) carbamate (12 g). LCMS (m/z): 255.9 (M + H-68-56).

## Intermediate k14

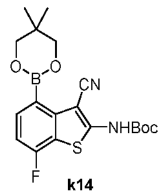

k14

*tert-butyl* (3-cyano-4-(5,5-dimethyl-1,3,2-dioxaborinan-2-yl)-7-fluorobenzo[*b*]thiophen-2-yl) carbamate

Intermediate **k14** was synthesized and characterized according to the method described in the literature WO2021118877A1.

## Intermediate a1

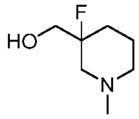

(3-fluoro-1-methylpiperidin-3-yl)methanol

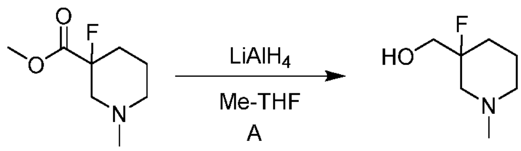


**Step A:** (3-fluoro-1-methylpiperidin-3-yl)methanol

Under a nitrogen ($N_2$) atmosphere, 2-methyltetrahydrofuran (5 mL) was added to methyl 3-fluoro-1-methylpiperidine-3-carboxylate (200 mg, 0.76 mmol). Then lithium aluminum hydride (1.5 mL, 1.5 mmol, 1.0 mol/L in tetrahydrofuran solution) was slowly added dropwise at 0 °C. The resulting mixture was stirred at 70 °C for 5 hours. After the reaction was completed (monitored by LCMS), 1 mL of water and 30 mL of ethyl acetate were successively added to quench the reaction. The resulting mixture was filtered through celite and the filtrate was concentrated and dried to obtain a colorless oily crude product (3-fluoro-1-methylpiperidin-3-yl)methanol (130 mg, yield 77%). $^{1}H$ NMR (400 MHz, DMSO-$d_6$) $\delta$ 4.89 (s, 1H), 3.53 - 3.27 (m, 3H), 2.48 - 2.22 (m, 2H), 2.21 - 2.04 (m, 4H), 1.65 - 1.42 (m, 4H). $^{19}F$ NMR (376 MHz, DMSO-$d_6$) $\delta$-165.75.

## Intermediate a2

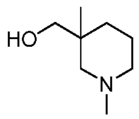

(1,3-dimethylpiperidin-3-yl) methanol

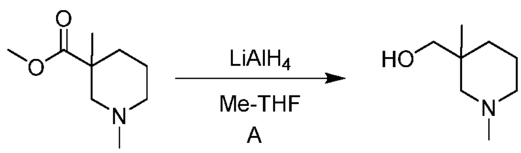

**Step A:** (1,3-dimethylpiperidin-3-yl) methanol

Under a nitrogen ($N_2$) atmosphere, 2-methyltetrahydrofuran (2 mL) was added to methyl 3-fluoro-1-methylpiperidin-3-carboxylate (300 mg, 1.23 mmol). Then, lithium aluminum hydride (2.5 mL, 2.5 mmol, 1.0 mol/L in tetrahydrofuran solution) was then slowly added dropwise at 0 °C. The resulting mixture was stirred at 70 °C for 4 hours. After the reaction was completed (monitored by LCMS), 1 mL water and 30 mL ethyl acetate were successively added to quench the reaction. The resulting mixture was filtered through celite and the filtrate was concentrated to dryness to obtain a colorless oily crude product (3-fluoro-1-methylpiperidin-3-yl)methanol (150 mg, yield 85%). LCMS (m/z): 144.0 (M + H).

## Intermediate a3

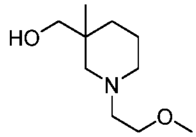

(1-(2-methoxyethyl)-3-methylpiperidin-3-yl)methanol

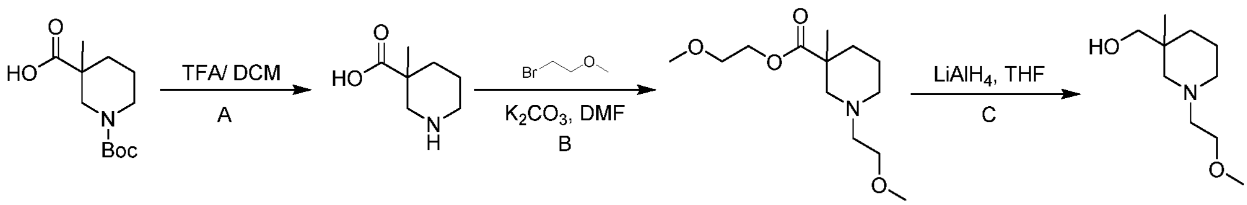


**Step A:** 3-methylpiperidine-3-carboxylic acid trifluoroacetate

Dichloromethane (2 mL) and trifluoroacetic acid (2 mL) were added to 1-(tert-butoxycarbonyl)-3-methylpiperidine-3-carboxylic acid (700 mg, 2.88 mmol) at room temperature. The resulting mixture was stirred at room temperature for 1 hour. The reaction was concentrated to dryness to give the yellow solid crude product 3-methylpiperidine-3-carboxylic acid trifluoroacetate (500 mg, crude), which was directly used in the subsequent reaction.

**Step B:** 2-methoxyethyl 1-(2-methoxyethyl)-3-methylpiperidine-3-carboxylate

At room temperature, DMF (5 mL) was added to 3-methylpiperidine-3-carboxylic acid (300 mg). Potassium carbonate (1.45 g, 10.5 mmol) and 1-bromo-2-methoxyethane (728 mg, 21.1 mmol) were successively added. The resulting mixture was stirred at room temperature for 12 hours. After the reaction was completed as monitored by TLC (100% EtOAc), the reaction was filtered, concentrated and dried, and the resulting crude product was purified by FCC ($SiO_2$, EtOAc/PE = 50-100%) to obtain the colorless oily crude product 2-methoxyethyl 1-(2-methoxyethyl)-3-methylpiperidine-3-carboxylate (290 mg). $^1$H NMR (400 MHz, Chloroform-d) δ 4.28 - 4.18 (m, 2H), 3.59 (t, $J$ = 4.8 Hz, 2H), 3.49 (t, $J$ = 6.1 Hz, 2H), 3.38 (s, 3H), 3.33 (s, 3H), 3.14 - 3.02 (m, 1H), 2.66 - 2.43 (m, 3H), 2.26 - 1.87 (m, 4H), 1.72 - 1.55 (m, 2H), 1.15 (s, 3H).

**Step C:** (1-(2-methoxyethyl)-3-methylpiperidin-3-yl)methanol

Under $N_2$ atmosphere, anhydrous tetrahydrofuran (2 mL) was added to 2-methoxyethyl 1-(2-methoxyethyl)-3-methylpiperidine-3-carboxylate (230 mg, 0.09 mmol). Lithium aluminum hydride (2.2 mL, 2.2 mmol, 1.0 mol/L in tetrahydrofuran solution) was then slowly added dropwise at 0°C. The resulting mixture was stirred for half an hour at 0 °C. After the reaction was completed (monitored by LCMS), 1 mL water and 30 mL ethyl acetate were successively added to quench the reaction. The resulting mixture was filtered through celite and the filtrate was concentrated and dried to yield yellow oily crude product (1-(2-methoxyethyl)-3-methylpiperidin-3-yl) methanol (110 mg, yield 66%). LCMS (m/z): 188.1 (M + H).

## Intermediate a4

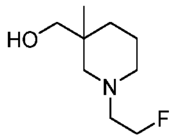

(1-(2-fluoroethyl)-3-methylpiperidin-3-yl) methanol

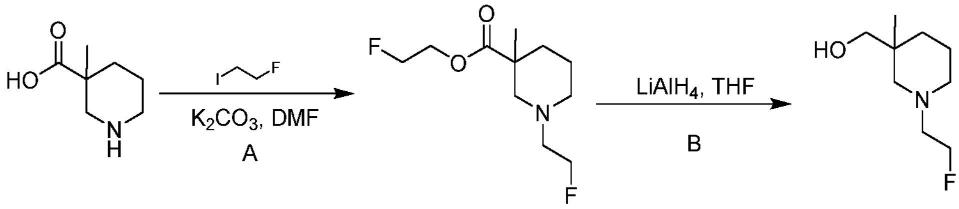


**Step A:** 2-fluoroethyl 1-(2-fluoroethyl)-3-methylpiperidine-3-carboxylate

Potassium carbonate (965 mg, 6.9 mmol) and 1-fluoro-2-iodoethane (607 mg, 3.5 mmol) were added successively to a solution of 3-methylpiperidine-3-carboxylic acid (200 mg, 1.4 mmol) in DMF (5 mL) at room temperature. The resulting solution was stirred at room temperature for 12 hours. After the reaction was completed (monitored by TLC, 100% EtOAc), the reaction was filtered and concentrated to dryness. The resulting crude product was purified by FCC ($SiO_2$, EtOAc/PE = 50-100%) to obtain 2-fluoroethyl 1-(2-fluoroethyl)-3-methylpiperidine-3-carboxylate (180 mg, yield 55%). $^1H$ NMR (400 MHz, Chloroform-*d*) δ 4.69 - 4.63 (m, 1H), 4.62 - 4.57 (m, 1H), 4.56 - 4.51 (m, 1H), 4.49 - 4.44 (m, 1H), 4.41 - 4.28 (m, 2H), 2.74 - 2.59 (m, 2H), 2.22 - 1.97 (m, 4H), 1.75 - 1.53 (m, 4H), 1.17 (s, 3H). LCMS (m/z): 236.0 (M + H).

**Step B:** (1-(2-fluoroethyl)-3-methylpiperidin-3-yl)methanol

Under a nitrogen ($N_2$) atmosphere, anhydrous tetrahydrofuran (2 mL) was added to 2-fluoroethyl 1-(2-fluoroethyl)-3-methylpiperidine-3-carboxylate (180 mg, 0.76 mmol). Lithium aluminum hydride (2.3 mL, 2.3 mmol, 1.0 mol/L in tetrahydrofuran solution) was then slowly added dropwise at 0 °C. The resulting solution was stirred for half an hour at 0 °C. After the reaction was completed (monitored by LCMS), 1 mL water and 30 mL ethyl acetate were successively added to quench the reaction. The resulting mixture was filtered through celite and the filtrate was concentrated and dried to give yellow oily crude product (1-(2-fluoroethyl)-3-methylpiperidin-3-yl)methanol (100 mg, yield 74%). LCMS (m/z): 176.1 (M + H).

## Intermediate a5

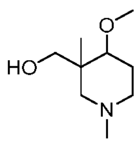

(4-methoxy-1,3-dimethylpiperidin-3-yl)methanol

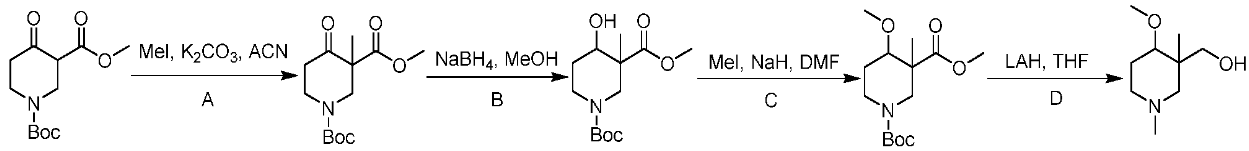

**Step A:** 1-(*tert*-butyl) 3-methyl 3-methyl-4-oxopiperidine-1,3-dicarboxylate

At room temperature, $CH_3I$ (13.79 g, 97.17 mmol) was added dropwise to a stirred mixture of 1-(*tert*-butyl) 3-methyl 4-oxopiperidine-1,3-dicarboxylate (5.0 g, 19.43 mmol), $K_2CO_3$ (8.06 g, 58.30 mmol) and anhydrous acetonitrile (50 mL). After the addition was completed, the reaction was carried out at room temperature overnight. After the reaction was completed as detected by TLC, the reaction solution was poured into $NH_4Cl$ (100 mL) and extracted with EA (100 mL × 3). The combined organic phase was washed with saturated NaCl (50 mL), concentrated and purified by FCC ($SiO_2$, EA/PE = 0-20%) to obtain 1-(*tert*-butyl) 3-methyl 3-methyl-4-oxopiperidine-1,3-dicarboxylate (4.85 g, yield 92%) as a colorless oily liquid. LCMS (m/z): 216.0 (M + H-56). $^1H$ NMR (400 MHz, Methanol-*d*4) δ 4.51 (d, *J* = 13.7 Hz, 1H), 4.23 - 4.05 (m, 1H), 3.73 (s, 3H), 3.47 - 3.27 (m, 1H), 3.17 (d, *J* = 13.7 Hz, 1H), 2.75 (s, 1H), 2.57 - 2.41 (m, 1H), 1.50 (s, 9H), 1.28 (s, 3H).

**Step B:** 1-(*tert*-butyl) 3-methyl 4-hydroxy-3-methylpiperidine-1,3-dicarboxylate

$NaBH_4$ (230 mg, 6.08 mmol) was added to a mixture of 1-(*tert*-butyl) 3-methyl 3-methyl-4-oxopiperidine-1,3-dicarboxylate (1.5 g, 5.53 mmol) and MeOH (15 mL) in an ice bath, and the reaction was stirred at room temperature for 15 minutes till TLC shows that the raw material has disappeared. The reaction solution was poured into $NH_4Cl$ (50 mL), extracted with EA (50 mL × 3), the collected organic phase was washed with saturated NaCl (30 mL). The crude product obtained after concentrating the organic solution was purified by FCC ($SiO_2$, EA/PE = 0-60%) to obtain 1-(*tert*-butyl) 3-methyl 4-hydroxy-3-methylpiperidine-1,3-dicarboxylate (800 mg, yield 53%) as a colorless oily liquid. LCMS (m/z): 218.0 (M + H-56). $^1H$ NMR (400 MHz, Methanol-$d_4$) δ 3.91 - 3.83 (m, 1H), 3.71 - 3.66 (m, 3H), 3.64 - 3.47 (m, 3H), 1.90 - 1.78 (m, 1H), 1.73 - 1.56 (m, 1H), 1.45 (s, 9H), 1.14 (s, 3H).

**Step C:** 1-(*tert*-butyl) 3-methyl 4-methoxy-3-methylpiperidine-1,3-dicarboxylate

NaH (219 mg, 5.49 mmol, 60%) was added to a mixture of 1-(*tert*-butyl) 3-methyl 4-hydroxy-3-methylpiperidine-1,3-dicarboxylate (500 mg, 1.83 mmol) and DMF (12 mL) in an ice bath and stirred for 20 minutes. $CH_3I$ (1.3 g, 9.15 mmol) was then added to the above solution and stirred for 3 h under the ice-bath condition. After the reaction was completed (monitored by TLC), the reaction was poured into aqueous $NH_4Cl$ (80 mL), extracted by EA (50 mL × 3). The combined organic phase was washed with saturated NaCl (20 mL), and the crude product obtained after concentration and FCC ($SiO_2$, EA/PE = 0-20%) to give 1-(*tert*-butyl) 3-methyl 4-methoxy-3-methylpiperidine-1,3-dicarboxylate (350 mg, yield 67%) as a colorless oily liquid. LCMS (m/z): 232.0 (M + H-56).

**Step D:** (4-methoxy-1,3-dimethylpiperidin-3-yl)methanol

$LiAH_4$ (5.39 mL, 5.39 mmol, 1M in THF) was added to 1-(*tert*-butyl) 3-methyl 4-methoxy-3-methylpiperidine-1,3-dicarboxylate (310 mg, 1.08 mmol), and the reaction was heated to 70 °C and stirred for 2 hours. After the reaction was completed as detected by LCMS, under an ice-bath condition, $Na_2SO_4 \cdot 10H_2O$ was slowly added to the reaction solution to quench $LiAlH_4$ until no more gas was generated. Then anhydrous sodium sulfate was added to dry the solution. The solution was filtered, and the mother liquor was collected and concentrated to obtain (4-methoxy-1,3-dimethylpiperidin-3-yl)methanol (170 mg, yield 91%) as a colorless liquid. LCMS (m/z): 174.0 (M + H). $^1H$ NMR (400 MHz, Methanol-$d_4$) δ 3.69 - 3.52 (m, 2H), 3.34 (s, 3H), 3.22 - 3.03 (m, 1H), 2.80 - 2.42 (m, 2H), 2.33 - 2.17 (m, 4H), 2.13 - 1.50 (m, 4H), 1.08 - 0.88 (m, 3H).

## Intermediate a5A

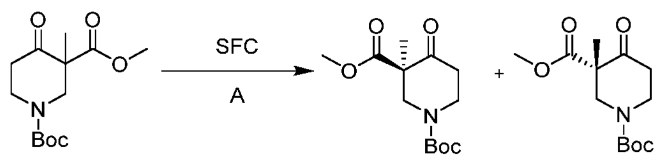


a5A a5B

The compound 1-(*tert*-butyl) 3-methyl 3-methyl-4-oxopiperidine-1,3-dicarboxylate (120g) was resolved by SFC (SFC150, Waters) (separation column: DAICEL CHIRALPAK®IG, 250*50 mm, 10μm; Mobile phase: $CO_2$/MeOH=90/10; flow rate: 120 mL/min). The first-eluted isomer, Isomer 1, was obtained as compound a5A (52.8 g, with a relatively shorter retention time). Chiral analytical method SFC-3, Rt = 0.682 min. $^1H$ NMR (400 MHz, Chloroform-*d*) δ 4.59 - 4.42 (m, 1H), 4.26 - 3.98 (m, 1H), 3.73 (s, 3H), 3.42 - 3.24 (m, 1H), 3.16 - 3.01 (m, 1H), 2.93 - 2.63 (m, 1H), 2.58 - 2.40 (m, 1H), 1.49 (s, 9H), 1.31 (s, 3H). LCMS (m/z): 216.1 (M-56 + H). The subsequently eluted isomer, Isomer 2, was obtained as compound a5B

(52.4 g, with a relatively longer retention time). Chiral analytical method SFC-3, Rt = 1.035 min. $^1$H NMR (400 MHz, Chloroform-d) δ 4.60 - 4.41 (m, 1H), 4.24 - 3.94 (m, 1H), 3.73 (s, 3H), 3.42 - 3.24 (m, 1H), 3.17 - 3.00 (m, 1H), 2.93 - 2.64 (m, 1H), 2.56 - 2.40 (m, 1H), 1.49 (s, 9H), 1.31 (s, 3H).

## Intermediate a5A-1

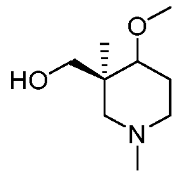

(3*S*)-(4-methoxy-1,3-dimethylpiperidin-3-yl)methanol

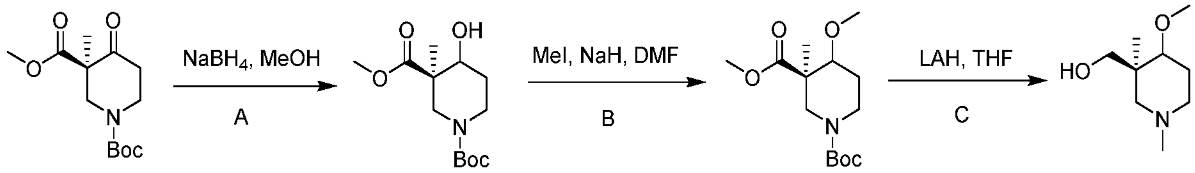


a5A a5A-1-1 a5A-1-2 a5A-1

**Step A:** 1-(*tert*-butyl) 3-methyl (3*R*)-4-hydroxy-3-methylpiperidine-1,3-dicarboxylate

Under an ice bath, $NaBH_4$ (279 mg, 7.37 mmol) was added in batches to a solution of 1-(*tert*-butyl) 3-methyl (*R*)-3-methyl-4-oxopiperidine-1,3-dicarboxylate (intermediate **a5A,** 2.0 g, 7.37 mmol) in MeOH (50 mL). The mixture was stirred under the ice-bath condition for 10 min, and TLC showed that the starting material had disappeared. Then the reaction solution was poured into $NH_4Cl$ (100 mL) and extracted with EA (100 mL × 3). The collected organic phase was washed with saturated aqueous NaCl solution (30 mL). The crude product obtained after concentrating the organic phase was purified by FCC ($SiO_2$, EA/PE = 0%-40%) to obtain 1-(*tert*-butyl) 3-methyl (3*R*)-4-hydroxy-3-methylpiperidine-1,3-dicarboxylate (1.7 g, yield 85%) as a colorless oily liquid. LCMS (m/z): 218.1 (M + H-56). $^1$H NMR (400 MHz, Chloroform-*d*) δ 4.05 - 3.93 (m, 1H), 3.78 - 3.70 (m, 3H), 3.69 - 3.59 (m, 2H), 3.35 - 3.22 (m, 1H), 3.20 - 3.10 (m, 1H), 1.97 - 1.82 (m, 1H), 1.77 - 1.63 (m, 1H), 1.54 - 1.40 (m, 9H), 1.29 - 1.18 (m, 3H).

**Step B:** 1-(*tert*-butyl) 3-methyl (3*R*)-4-methoxy-3-methylpiperidine-1,3-dicarboxylate

NaH (439 mg, 10.94 mmol, 60%) was added to a mixture of 1-(*tert*-butyl) 3-methyl (3*R*)-4-hydroxy-3-methylpiperidine-1,3-dicarboxylate (1.0 g, 3.66 mmol) and DMF (10 mL) in an ice bath, and the reaction was stirred for 20 minutes. $CH_3I$ (1.56 g, 10.94 mmol) was then added to the above mixture, and the mixture was stirred for additional 3 h in an ice bath. After the reaction was completed (monitored by TLC), the reaction was poured into saturated aqueous $NH_4Cl$ solution (80 mL) and extracted with EA (50 mL × 3). The combined organic phase was washed with saturated aqueous NaCl solution (20 mL), and crude product obtained after concentration was subjected to FCC ($SiO_2$, EA/PE = 0 ~ 40%) purification to give 1-(*tert*-butyl) 3-methyl (3*R*)-4-methoxy-3-methylpiperidine-1,3-dicarboxylate (1.0 g, yield 95%) as a white solid. LCMS (m/z): 232.1 (M + H-56). $^1$H NMR (400 MHz, Chloroform-$d_3$) δ 3.98 - 3.78 (m, 1H), 3.75 - 3.61 (m, 4H), 3.56 - 3.47 (m, 1H), 3.46 - 3.36 (m, 1H), 3.34 (s, 1H), 3.30 (s, 2H), 3.19 - 2.86 (m, 1H), 1.89 - 1.56 (m, 2H), 1.45 (s, 9H), 1.16 (s, 3H).

**Step C:** (3*S*)-(4-methoxy-1,3-dimethylpiperidin-3-yl)methanol

At room temperature, $LiAH_4$ (2.04 mL, 2.04 mmol, 1 M in THF) was added to 1-(*tert*-butyl) 3-methyl (3*R*)-4-methoxy-3-methylpiperidine-1,3-dicarboxylate (200 mg, 0.696 mmol)and the reaction was heated to 70 °C and stirred for 2h. After the reaction was completed (monitored by LCMS), $Na_2SO_4$•$10H_2O$ was slowly added to quench $LiAH_4$ in an ice bath until no more gas was generated. The mixture was diluted with a small amount of EA, dried over anhydrous sodium sulfate, and filtered. The filtrate was collected and concentrated to obtain (3*S*)-(4-methoxy-1,3-dimethylpiperidin-3-yl) methanol (120 mg, yield 99%) as a colorless liquid. LCMS (m/z): 174.1 (M + H). $^1$H NMR (400 MHz, Chloroform-$d_3$) δ 3.95 - 3.53 (m, 4H), 3.30 (s, 2H), 3.27 (s, 1H), 3.13 - 2.55 (m, 2H), 2.16 (s, 1H), 2.13 (s, 2H), 2.04 - 1.81 (m, 3H), 0.89 (s, 1H), 0.83

(s, 2H).

## Intermediate a5A-2

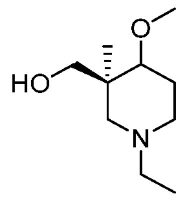

((3S)-1-ethyl-4-methoxy-3-methylpiperidin-3-yl)methanol

The synthesis of intermediate a5A-2 was carried out according to the method described for intermediate a6A-2.

## Intermediate a6

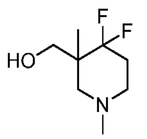

(4,4-difluoro-1,3-dimethylpiperidin-3-yl)methanol

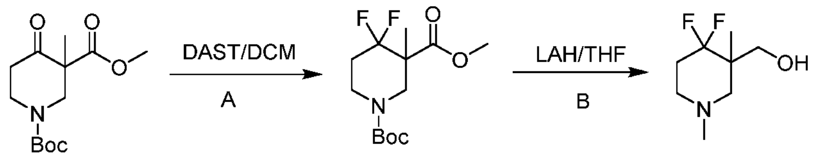


**Step** A: 1-(*tert*-butyl) 3-methyl 4,4-difluoro-3-methylpiperidine-1,3-dicarboxylate

DAST (4.87 mL, 5.94 g, 36.86 mmol) was added to a mixture of 1-(*tert*-butyl) 3-methyl-4-oxopiperidine-1,3-dicarboxylate (1.0 g, 3.69 mmol) and DCM (10 mL) at room temperature, and the reaction was heated to 50 °C for overnight reaction. After the reaction was completed (monitored by LCMS), the reaction was cooled to room temperature, slowly poured into a saturated aqueous $NaHCO_3$ solution, extracted with EA (100 mL × 3). The combined organic phase was washed with saturated NaCl (50 mL). The crude product obtained after concentrating the organic solution was purified by FCC ($SiO_2$, EA/PE = 0-10%) to obtain 1-(*tert*-butyl) 3-methyl 4,4-difluoro-3-methylpiperidine-1,3-dicarboxylate (290 mg, yield 27%) as a colorless oily liquid. LCMS (m/z): 238.0 (M + H-56).

**Step B:** (4,4-difluoro-1,3-dimethylpiperidin-3-yl)methanol

At room temperature, $LiAH_4$ (4.94 mL, 4.94 mmol, 1 M in THF) was added to 1-(*tert*-butyl) 3-methyl 4,4-difluoro-3-methylpiperidine-1,3-dicarboxylate (290 mg, 989 umol). The reaction was heated to 70 °C and stirred for 2.5 hours. After the reaction was completed (monitored by LCMS), $Na_2SO_4$•$10H_2O$ was slowly added to quench $LiAH_4$ in an ice bath until no more gas was generated. The mixture was diluted with EA, dried over anhydrous sodium sulfate, filtered. The filtrate was collected and concentrated to obtain (4-methoxy-1,3-dimethylpiperidin-3-yl)methanol (170 mg, yield 96%) as a colorless liquid. LCMS (m/z): 180.0 (M + H).

## Intermediate a6A-1

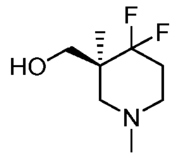

(*S*)-(4,4-difluoro-1,3-dimethylpiperidin-3-yl)methanol

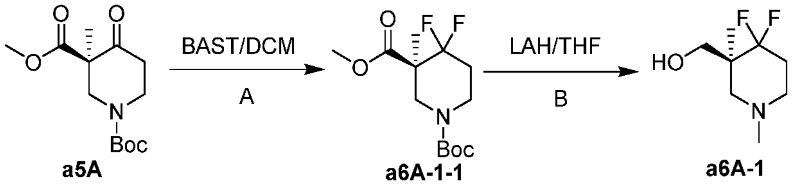


**a5A** **a6A-1-1** **a6A-1**

**Step A:** 1-(*tert*-butyl) 3-methyl (*S*)-4,4-difluoro-3-methylpiperidine-1,3-dicarboxylate

BAST (19.57 g, 16.3 mL, 88.46 mmol) was added to a solution of 1-(*tert*-butyl) 3-methyl (*R*)-3-methyl-4-oxopiperidine-1,3-dicarboxylate (intermediate **a5A,** 8.0 g, 29.49 mmol) in DCM (40 mL), and the reaction was stirred overnight at 50 °C. After the reaction was completed (monitored by LCMS), the reaction was slowly poured into a saturated aqueous $NaHCO_3$ (100 mL) solution and extracted with DCM (100 mL × 3). The combined organic phases were concentrated and then purified by ($SiO_2$, EA/PE = 0-10%) to give 1-(*tert*-butyl) 3-methyl (*S*)-4,4-difluoro-3-methylpiperidine-1,3-dicarboxylate (3.2 g, yield 37%) as a colorless oil. LCMS (m/z): 238.1 (M-56 + H).

**Step B:** (*S*)-(4,4-difluoro-1,3-dimethylpiperidin-3-yl)methanol

At room temperature, $LiAH_4$ (18.62 mL, 18.62 mmol, 1 M in THF) was added tol-(*tert*-butyl) 3-methyl (*S*)-4,4-difluoro-3-methylpiperidine-1,3-dicarboxylate (1.82 g, 6.21 mmol), and the temperature was raised to 70 °C and stirred for 2 h. After the reaction was completed (monitored by LCMS), under ice-bath conditions, $Na_2SO_4$•$10H_2O$ was slowly added to quench $LiAH_4$ until no more gas was generated. The solution was dried over anhydrous sodium sulfate, filtered. The filtrate was collected and concentrated to give (*S*)-(4,4-difluoro-1,3-dimethylpiperidin-3-yl)methanol (1 g, yield 91%) as colorless oily liquid. LCMS (m/z): 180.1 (M + H).

## Intermediate a6A-2

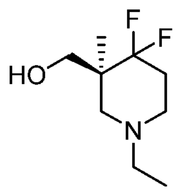

(*S*)-(4,4-difluoro-1,3-dimethylpiperidin-3-yl)methanol

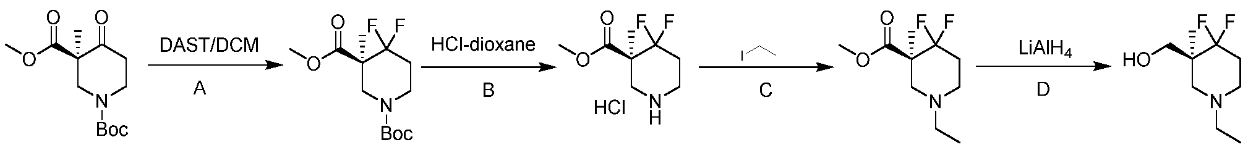


**a5A** **a6A-1-1** **a6A-2-1** **a6A-2-2** **a6A-2**

**Step A:** 1-(*tert*-butyl) 3-methyl (*S*)-4,4-difluoro-3-methylpiperidine-1,3-dicarboxylate

1-(*tert*-butyl) 3-methyl (*R*)-3-methyl-4-oxopiperidine-1,3-dicarboxylate (**a5A,** 10g, 36.86 mmol) was dissolved into 100 mL of anhydrous tetrahydrofuran, and degassed with nitrogen for three times. In an ice bath, the temperature of the reaction was cooled to 0 °C and [bis(2-methoxyethyl)amine]sulfur trifluoride (24.46 g, 20.39 mL, 110.57 mmol) was added dropwise to the reaction. The reaction was warmed to room temperature and stirred overnight. After the reaction was completed as monitored by TLC, the reaction solution was poured into 500 mL of a semi-saturated aqueous sodium bicarbonate solution and stirred for 10 min until no more bubbles were generated. The mixture was extracted with dichloromethane three times. The combined organic phases were washed with brine, dried over anhydrous sodium sulfate, filtered, and concentrated to obtain the crude product. The crude product was subjected to FCC ($SiO_2$, EA/PE = 0-17%) purification to give colorless oily product 1-(*tert*-butyl) 3-methyl (*S*)-4,4-difluoro-3-methylpiperidine-1,3-dicarboxylate (4.88 g, yield 43%). LCMS (m/z): 187.9 (M-56 + H). 1H NMR (400 MHz, Chloroform-d) δ 4.09 - 3.79 (m, 1H), 3.74 (s, 3H), 3.70 - 3.15 (m, 3H), 2.60 - 2.18 (m, 1H), 2.07 - 1.91 (m, 1H), 1.45 (s, 9H), 1.33 (s, 3H).

**Step B:** methyl (*S*)-4,4-difluoro-3-methylpiperidine-3-carboxylate hydrochloride

At room temperature, 4 M HCl-dioxane (30 mL) was added to 1-(*tert*-butyl) 3-methyl (*S*)-4,4-difluoro-3-methylpiperidine-1,3-dicarboxylate (3.1 g, 10.57 mmol), and the resulted mixture was stirred for 1 h. The solution was concentrated under reduced pressure, and then co-evaporated with EA (20 mL) twice to obtain methyl (*S*)-4,4-difluoro-3-methylpiperidine-3-carboxylate hydrochloride (2.3 g, yield 95%) as a white solid.

**Step C:** methyl (*S*)-1-ethyl-4,4-difluoro-3-methylpiperidine-3-carboxylate

Potassium carbonate (429.22 mg, 3.11 mmol) and iodoethane (484.38 mg, 3.11 mmol) were successively added to a solution of methyl (*S*)-4,4-difluoro-3-methylpiperidine-3-carboxylate hydrochloride (200 mg, 1.04 mmol) in acetonitrile (2 mL) at room temperature and the resulting mixture stirred overnight at 90 °C. After the reaction was completed (monitored by TLC), the reaction was filtered with celite and the filter cake was washed twice with acetonitrile. The filtrate was collected, concentrated to dryness to afford an oily product methyl (*S*)-1-ethyl-4,4-difluoro-3-methylpiperidine-3-carboxylate (110 mg, yield 48%), which was directly used in the next step without further purification. LCMS (m/z): 222.1 (M + H).

**Step D:** (*S*)-(4,4-difluoro-1-(ethyl)-3-methylpiperidin-3-yl)methanol

A 1M $LiAlH_4$-THF solution (0.95 mL, 0.95 mmol) was added dropwise to a solution of methyl (*S*)-1-ethyl-4,4-difluoro-3-methylpiperidine-3-carboxylate (110 mg, 0.474 mmol) in anhydrous tetrahydrofuran (5 mL) in an ice bath. The resulting mixture was stirred at room temperature for 20 minutes. After the reaction was completed (monitored by TLC), the reaction was quenched by addition of $Na_2SO_4{\cdot}10H_2O$ until no bubbles were generated and dried by addition of approximately 5 grams of anhydrous sodium sulfate. The resulting mixture was filtered with celite, and the filter cake was washed with anhydrous tetrahydrofuran for three times. The filtrate was collected and concentrated to dryness to give colorless oily product (*S*)-(4,4-difluoro-1-(ethyl)-3-methylpiperidin-3-yl)methanol (90 mg, yield 98%), which was directly used in the next step without further purification. LCMS (m/z): 194.1 (M + H).

## Intermediate a6A-3

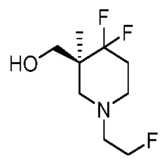

(*S*)-(4,4-difluoro-1-(2-fluoroethyl)-3-methylpiperidin-3-yl)methanol

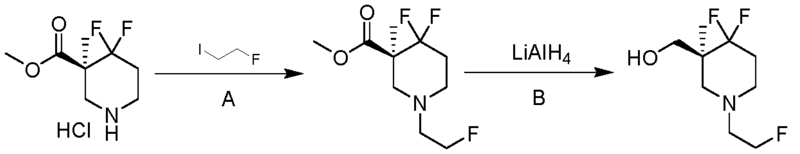


**a6A-2-1** **a6A-3-1** **a6A-3**

**Step A:** methyl (*S*)-4,4-difluoro-1-(2-fluoroethyl)-3-methylpiperidine-3-carboxylate

Potassium carbonate (429 mg, 3.11 mmol) and 1-fluoro-2-iodoethane (540 mg, 3.11 mmol) were successively added to a solution of methyl (*S*)-4,4-difluoro-3-methylpiperidine-3-carboxylate hydrochloride (200 mg, 1.04 mmol) in acetonitrile (2 mL), and the resulting mixture was stirred overnight at 90 °C. After the reaction was completed (monitored by LCMS), the reaction was filtered with celite and the filter cake was washed twice with acetonitrile. The filtrate was collected and concentrated to dryness to give the colorless oily product methyl (S)-4,4-difluoro-1-(2-fluoroethyl)-3-methylpiperidine-3-carboxylate (180 mg, yield 73%), which was used directly in the next step without purification. LCMS (m/z): 240.1 (M + H).

**Step B:** (*S*)-(4,4-difluoro-1-(2-fluoroethyl)-3-methylpiperidin-3-yl)methanol

Under ice-bath conditions, a 1M $LiAlH_4$-THF solution (1.5 mL, 1.50 mmol) was added dropwise to a solution of methyl (*S*)-4,4-difluoro-1-(2-fluoroethyl)-3-methylpiperidine-3-carboxylate (180 mg, 0.752 mmol) in anhydrous tetrahydrofuran (5 mL). The reaction was stirred at room temperature for 20 minutes. After the reaction was completed (monitored by LCMS), $Na_2SO_4$•$10H_2O$ was added to quench the reaction until no bubbles were generated, and the resulting mixture was dried by adding approximately 5 grams of anhydrous sodium sulfate. The resulting mixture was filtered with celite, and the filter cake was washed with anhydrous tetrahydrofuran three times. The filtrate was collected and concentrated to dryness to afford a colorless oily product (S)-(4,4-difluoro-1-(2-fluoroethyl)-3-methylpiperidin-3-yl)methanol (155 mg, yield 98%), which was used directly in the next step without purification. LCMS (m/z): 212.1 (M + H).

## Intermediate a6A-4

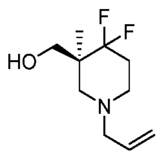

(*S*)-(4,4-difluoro-1-allyl-3-methylpiperidin-3-yl)methanol

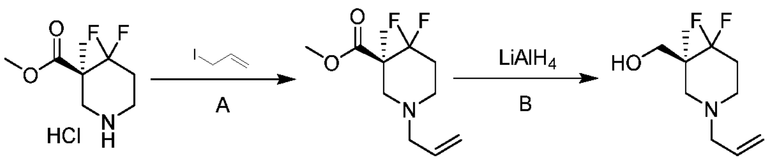


**a6A-2-1** **a6A-4-1** **a6A-4**

**Step A:** methyl (*S*)-1-allyl-4,4-difluoro-3-methylpiperidine-3-carboxylate

At room temperature, 3-bromopropyl-1-ene (316 mg, 2.61 mmol) was added to a mixture of methyl (*S*)-4,4-difluoro-3-methylpiperidine-3-carboxylate hydrochloride (200 mg, 0.871 mmol), $K_2CO_3$ (463 mg, 2.61 mmol) in ACN (5 mL). The reaction was heated to 90 °C and stirred overnight. After the reaction was completed (monitored by TLC), the reaction was filtered through celite. The collected filtrate was concentrated and purified by FCC ($SiO_2$, EA/PE = 0-20%) to obtain colorless oily product methyl (*S*)-1-allyl-4,4-difluoro-3-methylpiperidine-3-carboxylate (160 mg, yield 79%). LCMS (m/z): 234.1 (M + H).

**Step B:** (*S*)-(1-allyl-4,4-difluoro-3-methylpiperidin-3-yl)methanol

At room temperature, $LiAH_4$ (1.37 mL, 1.37 mmol, 1 M THF solution) was added to methyl (*S*)-1-allyl-4,4-difluoro-3-methylpiperidine-3-carboxylate (160 mg, 0.686 mmol), and the reaction was stirred 10 min at room temperature. After the reaction was completed (monitored by TLC), under ice-bath conditions, $Na_2SO_4$•$10H_2O$ was slowly added to quench $LiAH_4$ in until no more gas was generated. The resulting mixture was dried over the anhydrous sodium sulfate and filtered. The filtrate was collected and concentrated to obtain a colorless oily liquid (*S*)-(1-allyl-4,4-difluoro-3-methylpiperidin-3-yl)methanol (140 mg, yield 99%). LCMS (m/z): 206.1 (M + H).

## Intermediate a6A-5

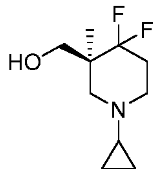

(*S*)-(4,4-difluoro-1-cyclopropyl-3-methylpiperidin-3-yl)methanol

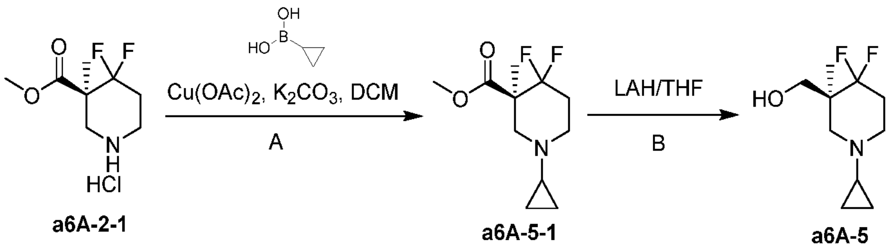


**a6A-2-1** **a6A-5-1** **a6A-5**

**Step A:** methyl (*S*)-1-cyclopropyl-4,4-difluoro-3-methylpiperidine-3-carboxylate

Under an air atmosphere, a mixture of methyl (*S*)-4,4-difluoro-3-methylpiperidine-3-carboxylate hydrochloride (300 mg, 1.31 mmol), cyclopropyl boric acid (449 mg, 5.23 mmol), $K_2CO_3$ (542 mg, 3.92 mmol, $Cu(OAc)_2$ (23.73 mg, 0.131 mmol) in DCM (20 mL) was stirred at 40 °C for 3 days. After the reaction was completed (monitored by LCMS), the reaction was poured into $H_2O$ (20 mL) and extracted with DCM (30 mL × 3). The organic phase was combined, concentrated and purified by FCC ($SiO_2$, EA/PE = 0-15%) to obtain the colorless oily liquid methyl (S)-1-cyclopropyl-4,4-difluoro-3-methylpiperidine-3-carboxylate (67 mg, yield 22%). LCMS (m/z): 234.1 (M + H). $^1$H NMR (400 MHz, $CDCl_3$) δ 3.63 (s, 3H), 3.04 (d, *J* = 11.7 Hz, 1H), 2.82 - 2.63 (m, 1H), 2.61 - 2.37 (m, 2H), 2.35 - 2.12 (m, 1H), 1.96 - 1.79 (m, 1H), 1.64 - 1.53 (m, 1H), 1.23 (s, 3H), 0.44 - 0.32 (m, 2H), 0.30 - 0.13 (m, 2H). $^{19}$F NMR (376 MHz, C $DCl_3$) δ-106.32,-106.96.

Step B: (*S*)-(4,4-difluoro-1-cyclopropyl-3-methylpiperidin-3-yl)methanol

At room temperature, $LiAlH_4$ (0.18 mL, 0.18 mmol, 1 M THF solution) was added to a solution of methyl (*S*)-1-cyclopropyl-4,4-difluoro-3-methylpiperidine-3-carboxylate (42 mg, 0.18 mmol) in anhydrous THF (3 mL), and the reaction was stirred for 10 min at room temperature. After the reaction was completed (monitored by TLC), under ice-bath conditions, $Na_2SO_4$•$10H_2O$ was slowly added to quench $LiAlH_4$ until no more gas was generated. The resulting mixture was diluted with a small amount of EA, dried over anhydrous sodium sulfate solution, filtered. The filtrate was collected and concentrated to obtain colorless oily liquid (*S*)-(4,4-difluoro-1-cyclopropyl-3-methylpiperidin-3-yl)methanol (32 mg, yield 87%). LCMS (m/z): 206.1 (M + H).

## Intermediate a7

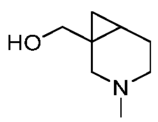

(3-methyl-3-azabicyclo[4.1.0]heptan-1-yl)methanol

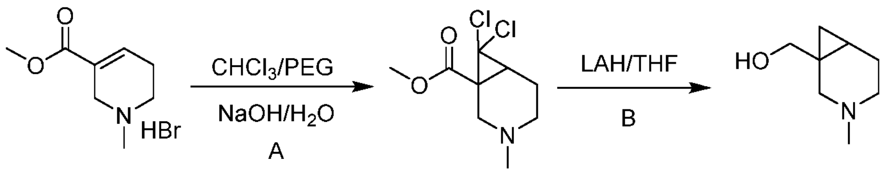


**Step A:** methyl 7,7-dichloro-3-methyl-3-azabicyclo[4.1.0]heptane-1-carboxylate

At room temperature, polyethylene glycol (1.0 g) was added to a mixture of methyl 1-methyl-1,2,5,6-tetrahydropyridine-3-carboxylate hydrobromide (1.3 g, 5.5 mmol), sodium hydroxide (45 mL, 50% wt) and $CHCl_3$ (150 mL). After the addition, the mixture was heated to 80 °C and stirred overnight. After the reaction was completed (monitored by LCMS), water (50 mL) was added and the resulting mixture was extracted with DCM (50 mL × 3). The organic phase was washed with brine. The organic phase was collected, concentrated and further purified by FCC ($SiO_2$, EA/PE = 0 - 25%) to obtain colorless liquid methyl 7,7-dichloro-3-methyl-3-azabicyclo[4.1.0]heptane-1-carboxylate (150 mg, yield 11%). LC-MS (m/z): 238.0 (M + H).

**Step B:** (3-methyl-3-azabicyclo[4.1.0]heptan-1-yl)methanol

At room temperature, $LiAlH_4$ (3.16 mL, 1 M THF solution, 3.16 mmol) was added dropwise to a solution of methyl 7,7-dichloro-3-methyl-3-azabicyclo[4.1.0]heptane-1-carboxylate (150 mg, 0.63 mmol) in THF (2 mL), and the reaction was heated to 70 °C and stirred overnight. After the reaction was completed (monitored by LCMS), the reaction was quenched with $Na_2SO_4$•$10H_2O$ until no gas was generated. The reaction solution was filtered through celite, and the obtained filtrate was concentrated at a low temperature (35 °C) to obtain (3-methyl-3-azabicyclo[4.1.0]heptan-1-yl) methanol (40 mg, yield 45%) as a colorless liquid, which was used directly in the subsequent reaction. LC-MS (m/z): 142.0 (M + H).

## Intermediate a8

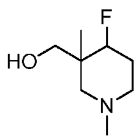

(4-fluoro-1,3-dimethylpiperidin-3-yl)methanol

## Intermediate a8A-1

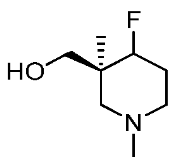

(3*S*)-(4-fluoro-1,3-dimethylpiperidin-3-yl)methanol

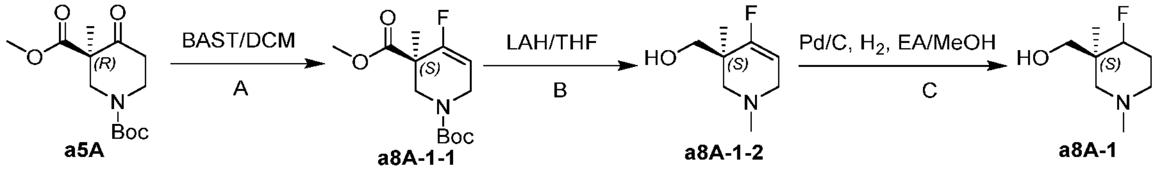


a5A a8A-1-1 a8A-1-2 a8A-1

**Step A:** 1-(*tert*-butyl) 3-methyl (*S*)-4-fluoro-3-methyl-3,6-dihydropyridine-1,3(2*H*)-dicarboxylate

Under ice-bath conditions, BAST (19.57 g, 16.3 mL, 88.46 mmol) was added dropwise into a solution of 1-(tert-butyl) 3-methyl (R)-3-methyl-4-oxopiperidine-1,3-dicarboxylate (**intermediate a5A,** 8.0 g, 29.49 mmol) in DCM (40 mL), and the reaction was stirred overnight at room temperature. After the reaction was completed (monitored by TLC), the reaction mixture was slowly poured into a semi-saturated $NaHCO_3$ (100 mL) solution and the resulting mixture was extracted with DCM (100 mL × 3). The combined organic phase was concentrated and purified by FCC ($SiO_2$, EA/PE = 0-10%) to obtain colorless oily product 1-(tert-butyl) 3-methyl (*S*)-4-fluoro-3-methyl-3,6-dihydropyridine-1,3(2*H*)-dicarboxylate (1.0 g, yield 12%). LCMS (m/z): 218.1 (M-56 + H).

**Step B:** (*S*)-(4-fluoro-1,3-dimethyl-1,2,3,6-tetrahydropyridin-3-yl)methanol

At room temperature, $LiAH_4$ (4.02 mL, 4.02 mmol, 1M THF solution) was added to 1-(tert-butyl) 3-methyl (*S*)-4-fluoro-3-methyl-3,6-dihydropyridine-1,3(2*H*)-dicarboxylate (500 mg, 1.83 mmol). The reaction was heated to 70°C and stirred for 1 h. After the reaction was completed (monitored by LCMS), under ice-bath conditions, $Na_2SO_4$•$10H_2O$ was slowly added to quench $LiAH_4$ until no more gas was generated. The resulting mixture was dried over anhydrous sodium sulfate and filtered. The filtrate was collected and concentrated to obtain colorless oily liquid (*S*)-(4-fluoro-1,3-dimethyl-1,2,3,6-tetrahydropyridin-3-yl)methanol (284mg, yield 98%). LCMS (m/z): 160.1 (M + H).

**Step C:** (3*S*)-(4-fluoro-1,3-dimethylpiperidin-3-yl)methanol

Under nitrogen atmosphere at room temperature, Pd/C (5% wt, 374 mg, 0.176 mmol) was added to a mixture of (*S*)-(4-fluoro-1,3-dimethyl-1,2,3,6-tetrahydropyridin-3-yl)methanol (280 mg, 1.76 mmol) in EA:MeOH=1:1 (20 mL). The resulting mixture was purged with hydrogen and then reacted at room temperature under a pressure of 60 psi $H_2$ for 2 h. After the reaction was completed (monitored by TLC), the reaction was filtered through celite, washed with EA (50 ml), and the organic phase was concentrated to give colorless oily liquid (3*S*)-(4-fluoro-1,3-dimethylpiperidin-3-yl)methanol (220 mg, yield 78%). LCMS (m/z): 162 (M + H).

## Intermediate a8A-2

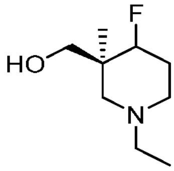

((3*S*)-1-ethyl-4-fluoro-3-methylpiperidin-3-yl)methanol

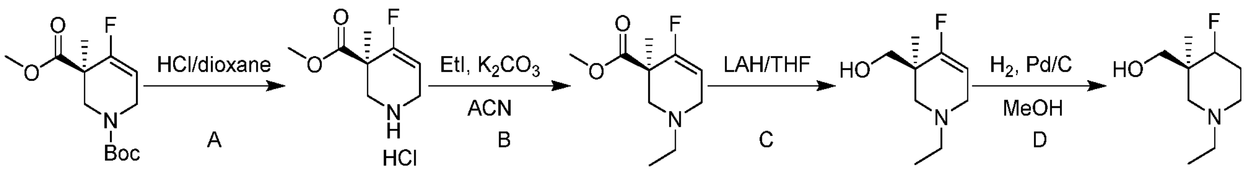


a8A-1-1 a8A-2-1 a8A-2-2 a8A-2-3 a8A-2

**Step A:** methyl (*S*)-4-fluoro-3-methyl-1,2,3,6-tetrahydropyridine-3-carboxylate hydrochloride

At room temperature, 4M HCl-dioxane (15 mL) was added to 1-(*tert*-butyl) 3-methyl (*S*)-4-fluoro-3-methyl-3,6-dihydropyridine-1,3(2*H*)-dicarboxylate (1 g, 3.66 mml), and the resulting mixture was stirred for 1 h. After the reaction was completed (monitored by LCMS), the reaction was concentrated under reduced pressure to remove the acid solution to obtain methyl (*S*)-4-fluoro-3-methyl-1,2,3,6-tetrahydropyridine-3-carboxylate hydrochloride (760 mg, yield 99%) as a yellowish solid. LCMS (m/z): 174.1 (M + H).

**Step B:** methyl (*S*)-1-ethyl-4-fluoro-3-methyl-1,2,3,6-tetrahydropyridine-3-carboxylate

At room temperature, iodoethane (1.7 g, 10.88 mmol) was added to a mixture of methyl (*S*)-4-fluoro-3-methyl-1,2,3,6-tetrahydropyridine-3-carboxylate hydrochloride (760 mg, 3.63 mmol), $K_2CO_3$ (1.5 g, 10.88 mmol) in ACN (10 mL), and the resulting mixture was stirred for 5 h at 90 °C. After the reaction was completed (monitored by TLC), the reaction was filtered through celite. Filtrate was collected, concentrated, and then purified by FCC ($SiO_2$, EA/PE = 0-20%) to obtain colorless oily product methyl (*S*)-1-ethyl-4-fluoro-3-methyl-1,2,3,6-tetrahydropyridine-3-carboxylate (317 mg, yield 43%). LCMS (m/z): 202.1 (M + H).

**Step C:** (*S*)-(1-ethyl-4-fluoro-3-methyl-1,2,3,6-tetrahydropyridin-3-yl)methanol

At room temperature, $LiAlH_4$ (1.58 mL, 1.58 mmol, 1 M THF solution) was added to a solution of methyl (*S*)-1-ethyl-4-fluoro-3-methyl-1,2,3,6-tetrahydropyridine-3-carboxylate (317 mg, 1.58 mmol) in THF (3 mL) , and the mixture was stirred at room temperature for 10 min. After the reaction was completed (monitored by TLC), under ice-bath conditions, $LiAH_4$ was quenched by slowly adding $Na_2SO_4$•$10H_2O$ until no more gas was generated. The resulting mixture was diluted with an appropriate amount of EA and dried by adding anhydrous sodium sulfate. The resulting mixture was filtered and concentrated to give a colorless oily liquid (S)-(1-ethyl-4-fluoro-3-methyl-1,2,3,6-tetrahydropyridin-3-yl) methanol (250 mg, yield 92%). LCMS (m/z): 174.1 (M + H).

**Step D:** ((3*S*)-1-ethyl-4-fluoro-3-methylpiperidin-3-yl)methanol

Under nitrogen atmosphere at room temperature, Pd/C (10 wt%, 154 mg, 144 umol) was added to a solution of (*S*)-(1-ethyl-4-fluoro-3-methyl-1,2,3,6-tetrahydropyridin-3-yl)methanol (250 mg, 1.44 mmol) in MeOH (50 mL). The resulting mixture was degassed with hydrogen twice and stirred 2 h at 25°C under a hydrogen balloon atmosphere. After the reaction was completed (monitored by LCMS), the reaction was filtered with celite, washed with EA (50 ml), and the filtrate was combined and concentrated to give colorless oily liquid ((3*S*)-1-ethyl-4-fluoro-3-methylpiperidin-3-yl) methanol (200 mg, yield 79%). LCMS (m/z): 176.1 (M + H).

## Intermediate a9A-1

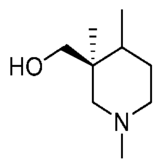

(3*S*)-(1,3,4-trimethylpiperidin-3-yl)methanol

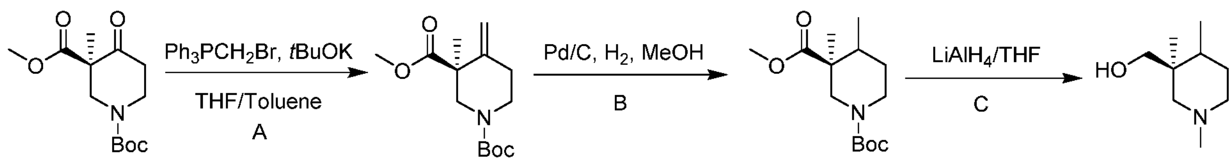


a5A a9A-1-1 a9A-1-2 a9A-1

**Step A:** 1-(*tert*-butyl) 3-methyl (*S*)-3-methyl-4-methylenepiperidine-1,3-dicarboxylate

Under ice-bath conditions, potassium *tert*-butoxide THF solution (5.25 mL, 1 M, 5.25 mmol) was added dropwise to a solution of methyltriphenylphosphonium bromide (1.89 g, 5.25 mmol) in toluene (10 mL). The mixture was stirred 0.5 h in an ice bath. A solution of 1-(*tert*-butyl) 3-methyl (*R*)-3-methyl-4-oxopiperidine-1,3-dicarboxylate (intermediate **a5A,** 1.00 g, 3.50 mmol) in toluene (5 mL) was added dropwise into the above mixture, and the resulting mixture was stirred at the

same temperature for 1h. Then the reaction was slowly heated to 110 °C and stirred overnight. After the reaction was completed (monitored by TLC), the reaction was cooled to room temperature, concentrated to dryness under reduced pressure. The resulting crude product was purified by FCC ($SiO_2$, EA/PE = 0-100%) to give colorless oily product 1-(*tert*-butyl) 3-methyl (S)-3-methyl-4-methylenepiperidine-1,3-dicarboxylate (750 mg, yield 75%). LCMS (m/z): 214.1 (M-56 + H), 292.1 (M + Na).

**Step B:** 1-(*tert*-butyl) 3-methyl (3*S*)-3,4-dimethylpiperidine-1,3-dicarboxylate

Under nitrogen atmosphere at room temperature, Pd/C (500 mg, 10% w/w, 0.47 mmol) was added to a solution of 1-(*tert*-butyl) 3-methyl (*S*)-3-methyl-4-methylenepiperidine-1,3-dicarboxylate (750 mg, 2.78 mmol) in methanol (20 mL). The reaction was degassed with a hydrogen balloon and stirred overnight in a hydrogen atmosphere. After the reaction was completed (monitored by TLC), it was filtered through celite. The filtrate was concentrated to obtain a crude product, which was purified by FCC ($SiO_2$, EA/PE = 0-50%) to give 1-(*tert*-butyl) 3-methyl (3*S*)-3,4-dimethylpiperidine-1,3-dicarboxylate (600 mg, yield 79%) as a colorless oil. LCMS (m/z): 216.1 (M-56 + H), 294.1 (M + Na). $^1H$ NMR (400 MHz, Methanol-$d_4$) δ 4.08 - 4.02 (m, 0.23 H), 3.98 (dd, *J* = 13.7, 1.5 Hz, 1H), 3.91 - 3.78 (m, 1.23 H), 3.72 (s, 0.69 H), 3.67 (s, 3H), 3.21 - 2.76 (m, 2.46 H), 2.20 - 2.06 (m, 0.23 H), 1.81 - 1.29 (m, 14.9 H), 1.20 (s, 3H), 1.07 (s, 0.69H), 1.02 (d, *J* = 6.6 Hz, 3H), 0.86 (d, *J* = 6.8 Hz, 0.69H).

**Step C:** (3*S*)-(1,3,4-trimethylpiperidin-3-yl)methanol

$LiAlH_4$-THF (1 M, 5.26 mmol, 5.26 mL) was added dropwise to a solution of 1-(tert-butyl) 3-methyl (3*S*)-3,4-dimethylpiperidine-1,3-dicarboxylate (500 mg, 1.75 mmol) in THF (5 mL) at room temperature, and the resulting mixture was heated to 70 °C and stirred for 3 h. After the reaction was completed (monitored by LCMS), the reaction was quenched by adding $Na_2SO_4$•$10H_2O$ until no more gas was generated, and the reaction solution was filtered through celite to obtain colorless liquid (3*S*)-(1,3,4-trimethylpiperidin-3-yl)methanol (250 mg, yield 91%). LC-MS (m/z): 158.2 (M + H).

## Intermediate a9A-2

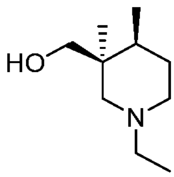

((3*S*,4*S*)-1-ethyl-3,4-dimethylpiperidin-3-yl)methanol

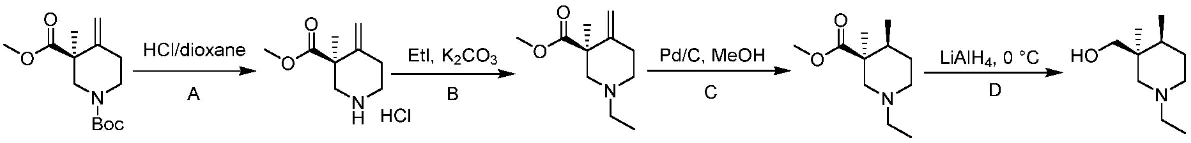


a9A-1-1 a9A-2-1 a9A-2-2 a9A-2-3 a9A-2

**Step A:** methyl (*S*)-3-methyl-4-methylenepiperidine-3-carboxylate hydrochloride

At room temperature, 1-(*tert*-butyl) 3-methyl (*S*)-3-methyl-4-methylenepiperidine-1,3-dicarboxylate (400 mg, 1.48 mmol) was added to HCl/dioxane (4M) (5 mL) and stirred 1 hour at room temperature. After the reaction was completed as monitored by LCMS, the mixture was concentrated to give methyl (*S*)-3-methyl-4-methylenepiperidine-3-carboxylate hydrochloride (304 mg, yield 100%) as a white solid. LC-MS (m/z): 170.1 (M + H).

**Step B:** methyl (*S*)-1-ethyl-3-methyl-4-methylenepiperidine-3-carboxylate

At room temperature, potassium carbonate (1.02 g, 7.43 mmol) was added to a mixture of methyl (*S*)-3-methyl-4-methylenepiperidine-3-carboxylate hydrochloride (304 mg, 1.49 mmol), iodoethane (695 mg, 4.45 mmol) in anhydrous acetonitrile (10 mL). The resulting mixture was heated to 90 °C and stirred overnight. After the reaction was completed (monitored by LCMS), the reaction was filtered and washed with EA (20 mL). The filtrate was collected, concentrated, and

further purified by FCC (EA/PE = 0-80%) to give methyl (*S*)-1-ethyl-3-methyl-4-methylenepiperidine-3-carboxylate (120 mg, yield 48%) as a colorless liquid. LC-MS (m/z): 198.1 (M + H).

**Step C:** methyl (3*S*,4*S*)-1-ethyl-3,4-dimethylpiperidine-3-carboxylate

At room temperature, Pd/C (18 mg, 10% wt) was added to a mixture of methyl (*S*)-1-ethyl-3-methyl-4-methylenepiperidine-3-carboxylate (180 mg, 0.910 mmol) in MeOH (5 mL). After the addition, the mixture was stirred at room temperature under $H_2$ (60 Psi) overnight. The reaction was completed as monitored by LCMS, it was filtered and washed with MeOH (20 mL). The filtrate was collected and concentrated to give methyl (3*S*)-1-ethyl-3,4-dimethylpiperidine-3-carboxylate (150 mg, yield 82%) as a colorless liquid. LC-MS (m/z): 200.1 (M + H).

**Step D:** ((3*S*,4*S*)-1-ethyl-3,4-dimethylpiperidin-3-yl)methanol

Under ice-bath conditions, $LiAlH_4$ (1 M, 0.75 mmol, 0.75 mL) was added dropwise into a solution of methyl (3*S*)-1-ethyl-3,4-dimethylpiperidine-3-carboxylate (150 mg, 0.75 mmol) in THF (5 mL). The reaction was stirred for 0.5 hours at the same temperature. After the reaction was completed (monitored by LCMS), the reaction was quenched by adding $Na_2SO_4$•$10H_2O$ until no more gas was generated. The reaction solution was filtered through celite, and the resulting filtrate was concentrated at 35 °C to obtain ((3S)-1-ethyl-3,4-dimethylpiperidin-3-yl)methanol (100 mg, yield 78%) as a colorless liquid. LC-MS (m/z): 172.1 (M + H).

## Intermediate a10A-1

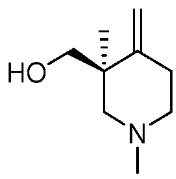

(*S*)-(1,3-dimethyl-4-methylenepiperidin-3-yl)methanol

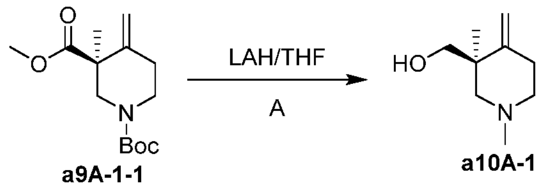


a9A-1-1 a10A-1

**Step A:** (*S*)-(1,3-dimethyl-4-methylenepiperidin-3-yl)methanol

At room temperature, $LiAlH_4$ (0.7 mL, 2.5 M THF solution, 1.75 mmol) was added dropwise into a solution of 1-(*tert*-butyl) 3-methyl (*S*)-3-methyl-4-methylenepiperidine-1,3-dicarboxylate (150 mg, 0.56 mmol) in THF (2 mL). After the addition was completed, the resulting mixture was heated to 70 °C and stirred for 3 h. After the reaction was completed (monitored by TLC), the reaction was quenched by adding $Na_2SO_4$•$10H_2O$, then dried by adding anhydrous sodium sulfate, diluted by ethyl acetate. The resulting mixture was filtered with celite to remove solid, and the filtrate was concentrated to obtain (*S*)-(1,3-dimethyl-4-methylenepiperidin-3-yl)methanol (120 mg, yield 80%) as a colorless oil liquid, which was used directly in the subsequent reaction.

## Intermediate a10A-2

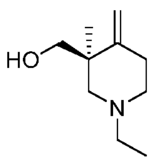

((*S*)-1-ethyl-3-methyl-4-methylenepiperidin-3-yl)methanol

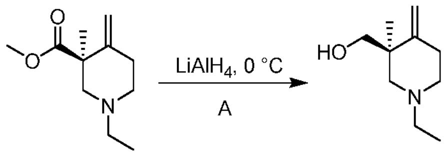


**a9A-2-2** **a10A-2**

**Step A:** ((*S*)-1-ethyl-3-methyl-4-methylenepiperidin-3-yl)methanol

Under an ice-bath, $LiAlH_4$ (1 M, 0.66 mmol, 0.66 mL) was added dropwise into a solution of methyl (*S*)-1-ethyl-3-methyl-4-methylenepiperidine-3-carboxylate (130 mg, 0.66 mmol) in THF (5 mL). After the addition, the mixture was stirred in the ice-bath for 0.5 h. After the reaction was completed monitoring by LCMS, $Na_2SO_4 \cdot 10H_2O$ was added to quench the reaction until no more gas was generated. The reaction was filtered through celite, and the resulting filtrate was concentrated at 35 °C to give ((*S*)-1-ethyl-3-methyl-4-methylenepiperidin-3-yl)methanol (90 mg, 81% yield) as a colorless liquid. LC-MS (m/z): 170.1 (M + H).

## Intermediate a11A-1

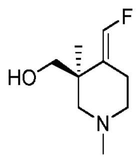

(*S,E*)-(4-(fluoromethylene)-1,3-dimethylpiperidin-3-yl) methanol

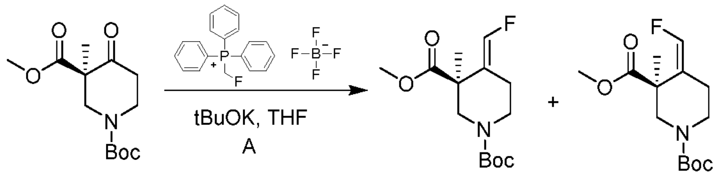


**a5A** **a11A-1-1** **a11A-2-1**

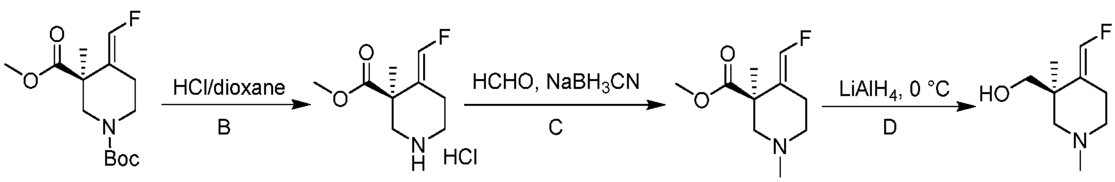


**a11A-1-1** **a11A-1-2** **a11A-1-3** **a11A-1**

**Step A:** 1-(tert-butyl) 3-methyl (*S,E*)-4-(fluoromethylene)-3-methylpiperidine-1,3-dicarboxylate and 1-(tert-butyl) 3-methyl (*S,Z*)-4-(fluoromethylene)-3-methylpiperidine-1,3-dicarboxylate

(Fluoromethylene)triphenylphosphonium tetrafluoroborate (10.56 g, 27.64 mmol) as dissolved in anhydrous THF (50 mL), and the system was degassed with nitrogen for three times. Under a dry-ice/ethanol bath, the temperature of the reaction solution was lowered to -70°C, and potassium *tert*-butoxide tetrahydrofuran (27.64 mL, 1 M, 27.64 mmol) solution was added dropwise to the reaction. The mixture was stirred at the same temperature for 1 hour. Then, a solution of *1-(tert-butyl)* 3-methyl (*R*)-3-methyl-4-oxopiperidine-1,3-dicarboxylate (intermediate **a5A,** 5.0 g, 18.43 mmol) in anhydrous THF (15 mL) was added dropwise into the reaction. After the addition was completed, the resulting mixture

was slowly warmed to room temperature and stirred overnight. After the reaction was completed (monitored by LCMS), the reaction was slowly poured into water (100 mL) and extracted three times with ethyl acetate. The combined organic phases were washed with brine, dried over anhydrous sodium sulfate, filtered, and concentrated to obtain a crude product. The crude product was purified by FCC ($SiO_2$, E A/PE = 0-15%) to afford 1-(*tert*-Butyl) 3-methyl (*S,E*)-4-(fluoromethylene)-3-methylpiperidine-1,3-dicarboxylate (1.98 g, Yield 37%) as a colorless oil. LCMS (m/z): 232.1 (M-56 + H). $^1$H NMR (400 MHz, Chloroform-*d*) $\delta$ 6.55 (d, *J* = 84.7, 1H), 4.35 (d, *J* = 13.2 Hz, 1H), 4.10 - 3.82 (m, 1H), 3.69 (s, 3H), 3.00 - 2.85 (m, 1H), 2.76 (d, *J* = 13.1 Hz, 1H), 2.71 - 2.60 (m , 1H), 2.31 - 2.08 (m, 1H), 1.46 (s, 9H), 1.29 (s, 3H); The double-bond configuration of compound a11A-1-1 was determined by the NOE signal between H1 and H2; And 1-(tert-butyl) 3-methyl (*S,Z*)-4-(fluoromethylene)-3-methylpiperidine-1,3-dicarboxylate (600 mg, yield 11%) as a colorless oil. LCMS (m/z): 232.1 (M-56 + H). $^1$H NMR (400 MHz, Chloroform-*d*) $\delta$ 6.43 (d, *J* = 83.7, 1H), 3.86 - 3.75 (m, 1H), 3.71 (s, 3H), 3.62 - 3.47 (m, 1H), 3.42 - 3.29 (m, 2H), 2.24 - 2.06 (m, 2H), 1.46 (s, 9H), 1.43 - 1.39 (m, 3H); The double-bond configuration of compound a11A-2-1 was determined by the NOE signal between H1 and H3.

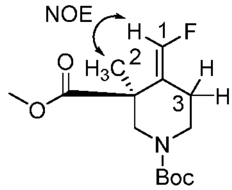

**a11A-1-1**

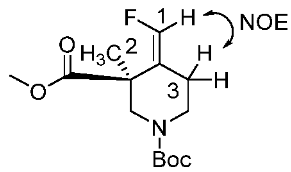

**a11A-2-1**

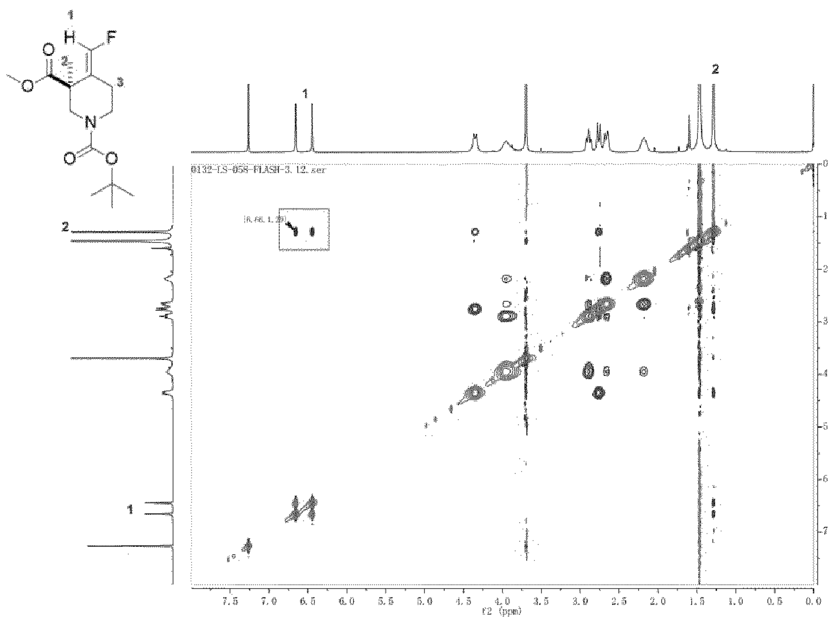

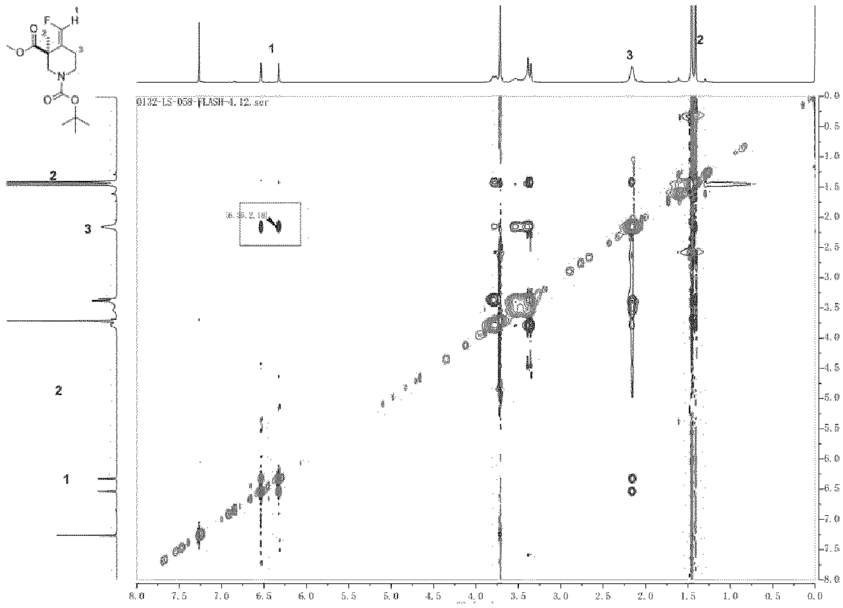

**Step B:** methyl (*S*,*E*)-4-(fluoromethylene)-3-methylpiperidine-3-carboxylate hydrochloride

At room temperature, 4M HCl-dioxane (10 mL) was added to 1-(tert-butyl) 3-methyl *(S,E)-4-*(fluoromethylene)-3-methylpiperidine-1,3-dicarboxylate (600mg, 2.09 mmol), and the mixture was stirred at room temperature for 1 h. The acid solution was concentrated to give methyl *(S,E)-4-*(fluoromethylene)-3-methylpiperidine-3-carboxylate hydrochloride (572 mg, yield 100%) as a white solid. LCMS (m/z): 188.1 (M + H).

**Step C:** methyl (*S*,*E*)-4-(fluoromethylene)-1,3-dimethylpiperidine-3-carboxylate

(Methyl (*S*,*E*)-4-(fluoromethylene)-3-methylpiperidine-3-carboxylate hydrochloride (370 mg, 1.98 mmol) was dissolved in methanol (5 mL) at room temperature, and triethylamine was added dropwise to the reaction until pH ~ 10. The reaction was stirred for 10 minutes, then glacial acetic acid was added dropwise until the pH of the reaction was ~ 4. Formaldehyde aqueous solution (481.15 mg, 5.93 mmol) was added to the reaction and stirred at room temperature for 30 min. Sodium cyanoborohydride (136.62 mg, 2.17 mmol) was added to the reaction and stirred for 2 h at room temperature. After the reaction was completed as monitored by LCMS, the solvent was removed under vacuum. After co-evaporation with anhydrous tetrahydrofuran twice, methyl *(S,E)-4-*(fluoromethylene)-1,3-dimethylpiperidine-3-carboxylate (380 mg, yield 96%) was obtained as a white solid. LCMS (m/z): 202.1 (M + H).

**Step D:** (*S,E*)-(4-(fluoromethylene)-1,3-dimethylpiperidin-3-yl)methanol

Under ice-bath conditions, a 1M solution of $LiAlH_4$-THF (2.83 mL, 107.5 mg, 2.83 mmol) was added dropwise to a solution of methyl (*E*)-4-(fluoromethylene)-1,3-dimethylpiperidine-3-carboxylate (380 mg, 1.89 mmol) in anhydrous THF (5 mL). The resulting mixture was stirred at room temperature for 20 min. After the reaction was completed (monitored by LCMS), the reaction was quenched with sodium sulfate decahydrate until no more bubbles were generated. About 5 g of anhydrous sodium sulfate was added to remove water. The resulting mixture was filtered with celite and the filter cake was washed three times with anhydrous tetrahydrofuran. The filtrate was collected and concentrated to dryness to afford (*S,E*)-(4-(fluoromethylene)-1,3-dimethylpiperidin-3-yl)methanol (300 mg, yield 92%) as a colorless oil. LCMS (m/z): 174.1 (M + H).

## Intermediate a11A-2

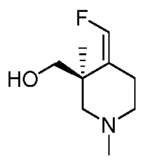

(*S,Z*)-(4-(fluoromethylene)-1,3-dimethylpiperidin-3-yl)methanol

The synthesis of intermediate a11A-2 was carried out according to the protocol described for intermediate a11A-1 using 1-(*tert*-butyl) 3-methyl (*S,Z*)-4-(fluoromethylene)-3-methylpiperidine-1,3-dicarboxylate (compound a11A-2-1) instead of 1-(*tert*-butyl) 3-methyl (*S,E*)-4-(fluoromethylene)-3-methylpiperidine-1,3-dicarboxylate (compound a11A-1-1).

## Intermediate a11A-3

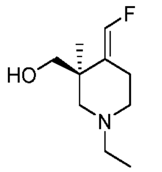

(*S,E*)-(1-ethyl-4-(fluoromethylene)-3-methylpiperidin-3-yl)methanol

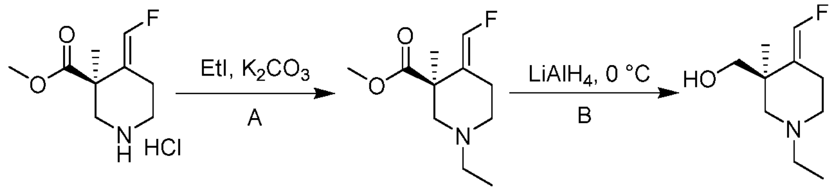


**a11A-1-2** **a11A-3-1** **a11A-3**

**Step A:** methyl (*S*,*E*)-1-ethyl-4-(fluoromethylene)-3-methylpiperidine-3-carboxylate

At room temperature, potassium carbonate (370.73 mg, 2.68 mmol) and iodoethane (278.92 mg, 1.79 mmol) were successively added to a solution of methyl (*S,E*)-4-(fluoromethylene)-3-methylpiperidine-3-carboxylate hydrochloride (200 mg, 0.894 mmol) in acetonitrile (6 mL), the resulting mixture was heated to 90 °C and stirred overnight. After the reaction was completed (monitored by LCMS), the reaction was filtered with celite and the filter cake was washed twice with acetonitrile. The filtrate was collected, concentrated to dryness, and the resulting crude product was purified by FCC ($SiO_2$, EA/PE = 0-90%) to afford methyl (*S,E*)-1-ethyl-4-(fluoromethylene)-3-methylpiperidine-3-carboxylate (100 mg, yield 69%) as a colorless oil. LCMS (m/z): 216.1 (M + H).

**Step B:** (*S,E*)-(1-ethyl-4-(fluoromethylene)-3-methylpiperidin-3-yl)methanol

Under ice-bath conditions, a 1M solution of $LiAlH_4$-THF (0.7 mL, 0.7 mmol) was added dropwise to a solution of methyl (*S,E*)-1-ethyl-4-(fluoromethylene)-3-methylpiperidine-3-carboxylate (100 mg, 0.46 mmol) in anhydrous tetrahydrofuran (5 mL). The resulting mixture was stirred at room temperature for 20 min. After the reaction was completed (monitored by LCMS), sodium sulfate decahydrate was added to quench the reaction until no bubbles were generated. Then about 5 g of anhydrous sodium sulfate was added for drying. The reaction solution was filtered with celite and the filter cake was washed three times with anhydrous tetrahydrofuran. The filtrate was collected and concentrated to dryness to obtain (*S,E*)-(1-ethyl-4-(fluoromethylene)-3-methylpiperidin-3-yl)methanol (80 mg, yield 92%) as a colorless oil. LCMS (m/z): 188.1 (M + H).

## Intermediate a12A-1

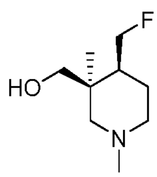

((3*S*,4*S*)-4-(fluoromethyl)-1,3-dimethylpiperidin-3-yl) methanol

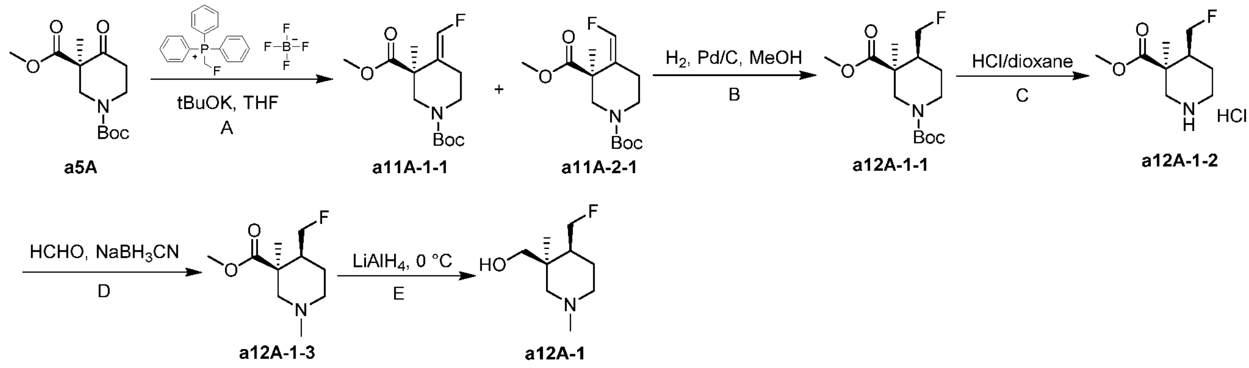


**Step A:** 1-(*tert*-butyl) 3-methyl (*S*,*E*)-4-(fluoromethylene)-3-methylpiperidine-1,3-dicarboxylate and 1-(tert-butyl) 3-methyl (*S*,*Z*)-4-(fluoromethylene)-3-methylpiperidine-1,3-dicarboxylateand

The synthesis of Step A was carried out according to the synthesis described in Step A of **Intermediate a11A-1.**

**Step B:** 1-(*tert*-butyl) 3-methyl (3*S*,4*S*)-4-(fluoromethyl)-3-methylpiperidine-1,3-dicarboxylate

Under nitrogen atmosphere at room temperature, Wet Pd/C (400 mg, 10% w/w) was added to a solution of the mixture of 1-(*tert*-butyl) 3-methyl (*S,E)*-4-(fluoromethylene)-3-methylpiperidine-1,3-dicarboxylate and 1-(*tert*-butyl) 3-methyl (*S,Z*)-4-(fluoromethylene)-3-methylpiperidine-1,3-dicarboxylate (850 mg, 2.96 mmol) in methanol (10 mL). The reaction was degassed with a hydrogen balloon and stirred overnight in a hydrogen atmosphere. After the reaction was completed (monitored by TLC), it was filtered with celite and the filtrate was concentrated to dryness to give the crude product which was purified by FCC ($SiO_2$, EA/PE = 0-10%) to give 1-(tert-butyl) 3-methyl (3S,4S)-4-(fluoromethyl)-3-methylpiperidine-1,3-dicarboxylate (600 mg, yield 70%) as a colorless oil. LCMS (m/z): 234.1 (M-56 + H), 312.1 (M + Na). $^1$H NMR (400 MHz, Methanol-d4) δ 4.76 - 4.70 (m, 0.5 H), 4.64 - 4.52 (m, 1H), 4.46 - 4.41 (m, 0.5 H), 4.23 (d, *J* = 13.7 Hz, 1H), 4.08 - 3.99 (m, 1H), 3.67 (s, 3H), 3.06 - 2.86 (m, 1H), 2.86 - 2.70 (m, 1H), 1.95 - 1.81 (m, 1H), 1.80 - 1.68 (m, 2H), 1.46 (s, 9H), 1.27 (s, 3H). $^{19}$F NMR (376 MHz, Methanol-*d*4) δ 221.80.

**Step C:** methyl (3*S*,4*S*)-4-(fluoromethyl)-3-methylpiperidine-3-carboxylate hydrochloride

At room temperature, 3M hydrochloric acid-dioxane (10 mL, 30 mmol) was added dropwise to a solution of 1-(*tert*-butyl) 3-methyl (3*S*,4*S*)-4-(fluoromethyl)-3-methylpiperidine-1,3-dicarboxylate (600 mg, 2.07 mmol) in ethyl acetate (10 mL), the resulting mixture was stirred at room temperature for 1 h. After the reaction was completed (monitored by LCMS), solvent was concentrated under reduced pressure to give methyl (3*S*,4*S*)-4-(fluoromethyl)-3-methylpiperidine-3-carboxylate hydrochloride (500 mg, crude) as a white solid. LCMS (m/z): 190.1 (M + H).

**Step D:** methyl (3*S*,4*S*)-4-(fluoromethyl)-1,3-dimethylpiperidine-3-carboxylate

At room temperature, methyl (3*S*,4*S*)-4-(fluoromethyl)-3-methylpiperidine-3-carboxylate hydrochloride (500 mg, crude from previous step) was dissolved in methanol (10 mL), and an aqueous formaldehyde solution (2 mL, 35 to 40% w/w, ~24 mmol) was added then. The resulting mixture was stirred for 2 h at room temperature. Sodium cyanoborohydride (696 mg, 11.1 mmol) was added in batches, and the resulting mixture was stirred at room temperature for 2 h. After the reaction was completed (monitored by LCMS), saturated aqueous $NH_4Cl$ was added to the reaction, and the mixture was extracted three times with ethyl acetate. The combined organic phase was concentrated to give the crude product which was purified by FCC ($SiO_2$, EA/PE = 0 - 100%) to afford methyl (3S,4S)-4-(fluoromethyl)-1,3-dimethylpiperidine-3-carboxylate (200 mg, yield 44%) as a colorless oil. LCMS (m/z): 204.1 (M + H).

**Step E:** ((3S,4S)-4-(fluoromethyl)-1 ,3-dimethylpiperidin-3-yl)methanol

At room temperature, $LiAlH_4$-THF (1.7 mL, 1M, 1.7 mmol) was slowly added dropwise to a solution of methyl (3*S*,4*S*)-4-(fluoromethyl)-1,3-dimethylpiperidine-3-carboxylate (200 mg, 0.869 mmol) in anhydrous THF (2 mL). The

resulting mixture was stirred at room temperature for 0.5 hour. After the reaction was completed (monitored by TLC), the reaction was cooled in an ice bath, and quenched by adding sodium sulfate decahydrate until no bubbles were generated. An appropriate amount of ethyl acetate was added. The resulting mixture was dried over anhydrous sodium sulfate, filtered, and concentrated to dryness to obtain ((3*S*,4*S*)-4-(fluoromethyl)-1,3-dimethylpiperidin-3-yl)methanol (100 mg, yield 58%) as a colorless oil crude product. LCMS (m/z): 176.1 (M + H).

## Intermediate a12A-2

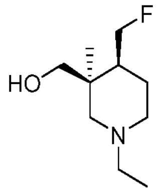

((3*S*,4*S*)-1-ethyl-4-(fluoromethyl)-3-methylpiperidin-3-yl)methanol

The synthesis of intermediate a12A-2 was carried out according to the protocol for intermediate **a11A-3** using methyl (3*S*,4*S*)-4-(fluoromethyl)-3-methylpiperidine-3-carboxylate hydrochloride **(a12A-1-2)** in place of methyl (*S*,*E*)-4-(fluoromethylene)-3-methylpiperidine-3-carboxylate hydrochloride **(a11A-1-2)** in step A.

## Intermediate a13A-1

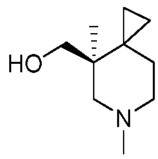

(*S*)-(4,6-dimethyl-6-azaspiro[2.5]octan-4-yl)methanol

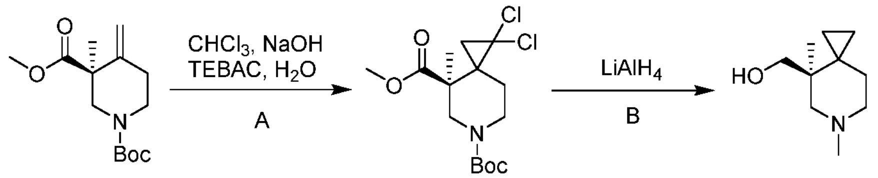


**a9A-1-1** **a13A-1-1** **a13A-1**

**Step A:** 6-(*tert*-butyl) 4-methyl (4*S*)-1,1-dichloro-4-methyl-6-azaspiro[2.5]octane-4,6-dicarboxylate

At room temperature, TEBAC (25.4 mg, 0.11 mmol) was added to a mixture of 1-(*tert*-butyl) 3-methyl (*S*)-3-methyl-4-methylenepiperidine-1,3-dicarboxylate (250 mg, 0.93 mmol) and sodium hydroxide (7.5 mL, 50% wt) in $CHCl_3$ (25 mL). The reaction was heated to 80 °C and stirred overnight. After the reaction was completed (monitored by LCMS), water (50 mL) was added, and the mixture was extracted with DCM (50 mL × 3). The organic phase was washed with brine, collected, concentrated, and further purified by FCC ($SiO_2$, EA/PE = 0-25%) to obtain 6-(*tert*-butyl) 4-methyl (4*S*)-1,1-dichloro-4-methyl-6-azaspiro[2.5]octane-4,6-dicarboxylate (320 mg, yield 98%) as a colorless liquid. LC-MS (m/z): 296.0 (M-56).

**Step B:** (*S*)-(4,6-dimethyl-6-azaspiro[2.5]octan-4-yl)methanol

At room temperature, $LiAlH_4$ (1 M, 2.2 mmol, 2.2 mL) was added dropwise to a solution of 6-(*tert*-butyl) 4-methyl (4*S*)-1,1-dichloro-4-methyl-6-azaspiro[2.5]octane-4,6-dicarboxylate (130 mg, 0.37 mmol) in THF (2 mL). After the addition was completed, the system was heated to 70 °C and stirred overnight. After the reaction was completed (monitored by LCMS), $Na_2SO_4•10H_2O$ was added to quench the reaction until no more gas was generated, and the

resulting mixture was filtered through celite to obtain (*S*)-(4,6-dimethyl-6-azaspiro[2.5]octan-4-yl)methanol (40 mg, yield 62%) as a colorless liquid. LC-MS (m/z): 170.1 (M + H).

## Intermediate a13A-2

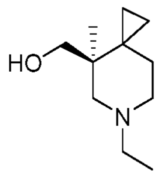

(*S*)-(6-ethyl-4-methyl-6-azaspiro[2.5]octan-4-yl)methanol

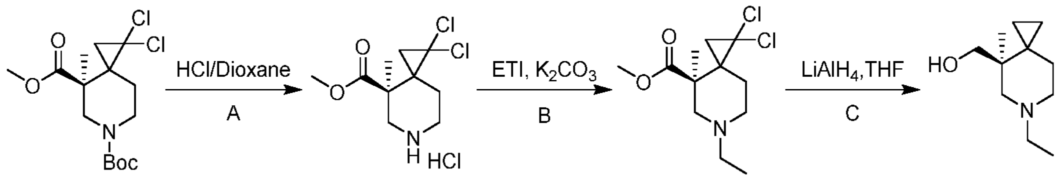


a13A-1-1 a13A-2-1 a13A-2-2 a13A-2

**Step A:** methyl (4*S*)-1,1-dichloro-4-methyl-6-azaspiro[2.5]octane-4-carboxylate hydrochloride

At room temperature, 6-(*tert*-butyl) 4-methyl (4*S*)-1,1-dichloro-4-methyl-6-azaspiro[2.5]octane-4,6-dicarboxylate (200 mg, 0.58 mmol) was added to HCl/dioxane (4M, 5 mL), and the resulting mixture was stirred 1 h at room temperature. After the reaction was completed (monitored by LCMS), solvent was concentrated under reduced pressure to give methyl (4*S*)-1,1-dichloro-4-methyl-6-azaspiro[2.5]octane-4-carboxylate hydrochloride (140 mg, yield 100%) as a white solid. LC-MS (m/z): 252.0 (M + H).

**Step B:** methyl (4*S*)-1,1-dichloro-6-ethyl-4-methyl-6-azaspiro[2.5]octane-4-carboxylate

At room temperature, potassium carbonate (230 mg, 1.67 mmol) was added to a mixture of methyl (4*S*)-1,1-dichloro-4-methyl-6-azaspiro[2.5]octane-4-carboxylate hydrochloride (140 mg, 0.56 mmol) and iodoethane (95 mg, 0.61 mmol) in ACN (5 mL). The resulting mixture was heated to 90 °C and stirred overnight. After the reaction was completed (monitored by LCMS), the reaction was filtered. The filtrate was collected, and the filter cake was washed with EA (20 mL). The combined filtrate was concentrated and purified by FCC ($SiO_2$, EA/PE = 0-80%) to afford methyl (4S)-1,1-dichloro-6-ethyl-4-methyl-6-azaspiro[2.5]octane-4-carboxylate (120 mg, yield 77%) as a pale-yellow liquid. LC-MS (m/z): 280.0 (M + H) and 282.0 (M + H).

**Step C:** (*S*)-(6-ethyl-4-methyl-6-azaspiro[2.5]octan-4-yl)methanol

At room temperature, $LiAlH_4$ (2.1 mL in 1M THF solution, 2.1 mmol) was added dropwise to a solution of methyl (4*S*)-1,1-dichloro-6-ethyl-4-methyl-6-azaspiro[2.5]octane-4-carboxylate (120 mg, 0.43 mmol) in THF (5 mL). After the addition, the reaction was heated to 70 °C and stirred for 16h. After the reaction was completed (monitored by LCMS), the reaction was quenched with $Na_2SO_4$•$10H_2O$ until no gas was generated, the reaction was filtered through celite and the resulting filtrate was concentrated at 35°C to obtain (*S*)-(6-ethyl-4-methyl-6-azaspiro[2.5]octan-4-yl)methanol (70 mg, yield 89%) as a colorless liquid. LC-MS (m/z): 184.1 (M + H).

## Intermediate a14A-1

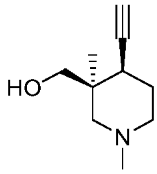

((3*S*,4*R*)-4-ethynyl-1,3-dimethylpiperidin-3-yl)methanol

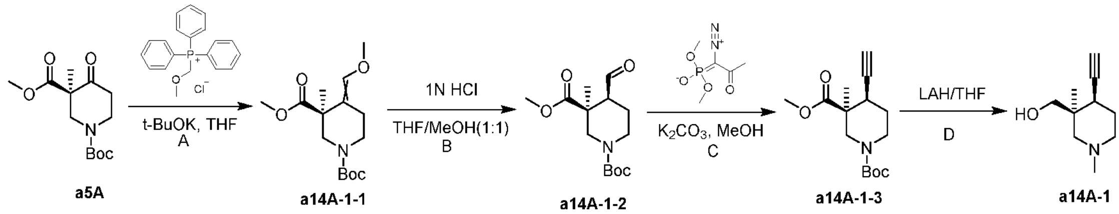


a5A a14A-1-1 a14A-1-2 a14A-1-3 a14A-1

**Step A:** 1-(*tert*-butyl) 3-methyl (*S*)-4-(methoxymethylene)-3-methylpiperidine-1,3-dicarboxylate

At room temperature, potassium *tert*-butoxide (22.1 mL, 1 M in THF, 22.1 mmol) was added dropwise to a solution of (methoxymethyl)triphenylphosphonium chloride (6.95 g, 20.3 mmol) in anhydrous THF (25 mL). The resulting mixture was stirred 1 h at room temperature. *1-(tert-butyl)* 2-ethyl (*R*)-2-methyl-3-oxopyrrolidine-1,2-dicarboxylate (5.00 g, 18.4 mmol) was added to the above mixture in one portion and the reaction was stirred overnight at room temperature. After the reaction was completed (monitored by LCMS), the reaction was concentrated under reduced pressure to dryness to give crude product, which was purified by FCC ($SiO_2$, EA/PE = 0-10%) to afford 1-(*tert*-butyl) 3-methyl (*S*)-4-(methoxymethylene)-3-methylpiperidine-1,3-dicarboxylate (2.15g, yield 39%) as a colorless oil. LCMS (m/z): 244.1 (M-56 + H), 322.1 (M + Na).

**Step B:** 1-(tert-butyl) 3-methyl (3*S*,4*S*)-4-formyl-3-methylpiperidine-1,3-dicarboxylate

At room temperature, 1-(tert-butyl) 3-methyl (*S*)-4-(methoxymethylene)-3-methylpiperidine-1,3-dicarboxylate (2.15g, 7.18 mmol) was dissolved into THF/MeOH (v/v = 1:1, 22 mL). 1N HCl aqueous solution (21.5 mL, 21.5 mmol) was then added dropwise to the above solution, and the resulting mixture was stirred for 8 h at room temperature. After the reaction was completed (monitored by LCMS), water (50 mL) was added to the reaction and then extracted with ethyl acetate (50 mL × 3). The organic phase was combined and concentrated to dryness to give the crude product which was purified by FCC ($SiO_2$, EA/PE = 0-50%) to give 1-(tert-butyl) 3-methyl (3*S*,4*S*)-4-formyl-3-methylpiperidine-1,3-dicarboxylate (850 mg, yield 41%) as a colorless oil. LCMS (m/z): 230.1 (M-56 + H), 308.1 (M + Na).

**Step C:** 1-(*tert*-butyl) 3-methyl (3*S*,4*R*)-4-ethynyl-3-methylpiperidine-1,3-dicarboxylate

At room temperature, dimethyl (1-diazo-2-oxopropyl) phosphonate (454 mg, 2.37 mmol) and potassium carbonate (437 mg, 3.16 mmol) were added to a solution of 1-(*tert*-butyl) 3-methyl (3S,4S)-4-formyl-3-methylpiperidine-1,3-dicarboxylate (450 mg, 1.58 mmol) in methanol (5 mL). After the reaction was completed (monitored by LCMS), it was concentrated under reduced pressure to obtain the crude product which was purified by FCC ($SiO_2$, EA/PE = 0-50%) to afford 1-(*tert*-butyl) 3-methyl (3*S*,4*R*)-4-ethynyl-3-methylpiperidine-1,3-dicarboxylate (70 mg, yield 17%) as a colorless oil. LCMS (m/z): 226.1 (M-56 + H), 304.1 (M + Na).

**Step D:** ((3*S*,4*R*)-4-ethynyl-1,3-dimethylpiperidin-3-yl)methanol

At room temperature, $LiAlH_4$-THF (0.8 mmol, 1 M, 0.8 mL) was added dropwise to a solution of 1-(*tert*-butyl) 3-methyl (3*S*,4*R*)-4-ethynyl-3-methylpiperidine-1,3-dicarboxylate (75 mg, 0.26 mmol) in THF (0.8 mL), and the resulting mixture was heated to 70 °C and stirred for 3 h. After the reaction was completed (monitored by LCMS), the reaction was quenched by adding $Na_2SO_4$•$10H_2O$ until no more gas was generated. The reaction solution was filtered through celite, and the filtrate was concentrated at 35 °C to give ((3S,4R)-4-ethynyl-1,3-dimethylpiperidin-3-yl)methanol (35 mg, yield

84%) as a colorless liquid. LC-MS (m/z): 168.1 (M + H).

## Intermediate a15A-1

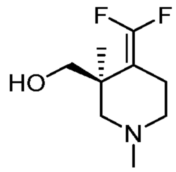

(*S*)-(4-(difluoromethylene)-1,3-dimethylpiperidin-3-yl)methanol

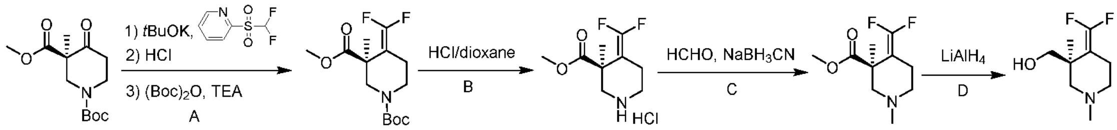


a5A a15A-1-1 a15A-1-2 a15A-1-3 a15A-1

**Step A:** 1-(tert-butyl) 3-methyl (S)-4-(difluoromethylene)-3-methylpiperidine-1,3-dicarboxylate

1-(tert-butyl) 3-methyl (R)-3-methyl-4-oxopiperidine-1,3-dicarboxylate (3.00g, 11.06 mmol) and 2-((difluoromethyl)sulfonyl)pyridine (3.20 g, 16.59 mmol) were dissolved in 30 mL of anhydrous DMF. The reaction was degassed with nitrogen three times. The resulting mixture was cooled with a dry-ice/ethanol bath, potassium tert-butoxide in THF solution (19.90 mL, 1 M, 19.90 mmol) was dropped into the reaction, and the reaction was stirred at this temperature for 1 h. A saturated aqueous ammonium chloride solution (30 mL) was added dropwise to the reaction and then warmed to -40 °C. Hydrochloric acid (6 M, 20 mL) was added dropwise to the reaction. After completion of dropping, the mixture was slowly heated to 70 °C and stirred for 2 days. After the reaction was completed (monitored by LCMS), saturated aqueous potassium carbonate was slowly added to the reaction to adjust the pH to 9. The resulting mixture was extracted three times with a mixture of dichloromethane and methanol (= 10/1 by volume). The combined organic phase was washed with brine, dried over anhydrous sodium sulfate, filtered, concentrated to dryness, and a crude product was obtained. The crude product was dissolved in 20 ml of dichloromethane, and di-*tert*-butyl dicarbonate (5ml) and triethylamine (5ml) were added at room temperature. The resulting mixture was stirred for 1h at room temperature. After the reaction was completed (monitored by TLC), the reaction was concentrated to dryness and the resulting crude product was purified by FCC ($SiO_2$, EA/PE = 0-7%) to afford 1-(*tert*-butyl) 3-methyl (*S*)-4-(difluoromethylene)-3-methylpiperidine-1,3-dicarboxylate (1.0 g, yield 30%) as a colorless solid. LCMS (m/z): 250.1 (M-56 + H).

**Step B:** methyl (*S*)-4-(difluoromethylene)-3-methylpiperidine-3-carboxylate hydrochloride

At room temperature, 4 M HCl-dioxane (10 mL) was added to 1-(*tert*-butyl) 3-methyl (*S*)-4-(difluoromethylene)-3-methylpiperidine-1,3-dicarboxylate (400 mg, 1.31 mmol). The mixture was stirred at room temperature for 1 h, and the acid solution was removed by concentration to obtain methyl (*S*)-4-(difluoromethylene)-3-methylpiperidine-3-carboxylate hydrochloride (300 mg, yield 95%) as a white solid. LCMS (m/z): 206.1 (M + H).

**Step C:** methyl (*S*)-4-(difluoromethylene)-1,3-dimethylpiperidine-3-carboxylate

At room temperature, methyl (*S*)-4-(difluoromethylene)-3-methylpiperidine-3-carboxylate hydrochloride (300 mg, 1.46 mmol) was dissolved in methanol (5 mL) at room temperature, triethylamine was added dropwise then until the pH of the reaction was about 10. The reaction was stirred for 10 min, and glacial acetic acid was added dropwise to adjust the pH to about 4. Aqueous formaldehyde solution (355.9 mg, 4.39 mmol) was added, and the resulting mixture was stirred at room temperature for 30 min. Sodium cyanoborohydride (101.1 mg, 1.61 mmol) was added and the resulting mixture was stirred for 2 h at room temperature. After the reaction was completed (monitored by LCMS), the reaction was concentrated to dryness. The residue was co-evaporated with anhydrous tetrahydrofuran twice to obtain methyl (*S*)-4-(difluoromethylene)-1,3-dimethylpiperidine-3-carboxylate (320 mg, yield 100%) as a white solid. LCMS (m/z): 220.1 (M + H).

**Step D:** (*S*)-(4-(difluoromethylene)-1,3-dimethylpiperidin-3-yl)methanol

Under an ice-bath condition, 1M $LiAlH_4$-THF (2.19 mL, 2.19 mmol) was added dropwise to a solution of methyl (*S*)-4-(difluoromethylene)-1,3-dimethylpiperidine-3-carboxylate (320 mg, 1.46 mmol) in anhydrous THF (10 mL). The resulting mixture was stirred for 15 min at room temperature. After the reaction was completed (monitored by LCMS), the sodium sulfate decahydrate was added to quench the reaction until no bubbles were generated. The mixture was dried over about 5 g anhydrous sodium sulfate, filtered with celite. The filter cake was washed with anhydrous tetrahydrofuran three times. The filtrate was collected and concentrated to dryness to obtain (*S*)-(4-(difluoromethylene)-1,3-dimethylpiperidin-3-yl)methanol (240 mg, yield 86%) as a colorless oil. LCMS (m/z): 192.1 (M + H).

## Intermediate a15A-2

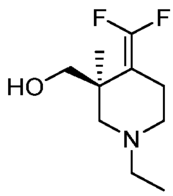

(*S*)-(4-(difluoromethylene)-1-ethyl-3-methylpiperidin-3-yl)methanol

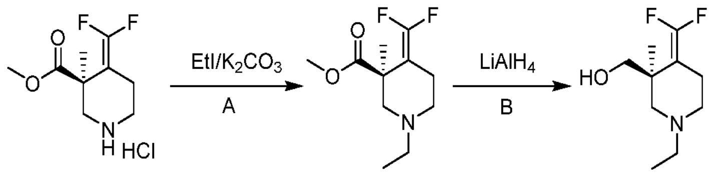


**a15A-1-2** **a15A-2-1** **a15A-2**

**Step A:** methyl (*S*)-4-(difluoromethylene)-1-ethyl-3-methylpiperidine-3-carb oxyl ate

At room temperature, iodoethane (258 mg, 1.66 mmol) was added to a mixture of methyl (S)-4-(difluoromethylene)-3-methylpiperidine-3-carboxylate hydrochloride (200 mg, 0.828 mmol) and $K_2CO_3$ (343 mg, 2.48 mmol) in ACN (5 mL). The reaction was heated to 90 °C and stirred overnight. After the reaction was completed (monitored by TLC), the reaction was filtered through celite. The collected filtrate was concentrated and purified by FCC ($SiO_2$, EA/PE = 0-20%) to obtain methyl (*S*)-4-(difluoromethylene)-1-ethyl-3-methylpiperidine-3-carboxylate (80 mg, yield 41%) as a colorless oil. LCMS (m/z): 234.1 (M + H).

**Step B:** (*S*)-(4-(difluoromethylene)-1-ethyl-3-methylpiperidin-3-yl)methanol

Under ice-bath conditions, $LiAlH_4$ (0.69 mL, 0.69 mmol, 1 M in THF) was added to methyl (*S*)-4-(difluoromethylene)-1-ethyl-3-methylpiperidine-3-carboxylate (80mg, 0.343mmol) and the mixture was stirred for 10 min. After the reaction was completed (monitored by TLC), $Na_2SO_4$•$10H_2O$ was slowly added to the reaction until no more gas was generated. The resulting mixture was diluted with EA, then dried over anhydrous sodium sulfate, filtered. The filtrate was concentrated to obtain (*S*)-(4-(difluoromethylene)-1-ethyl-3-methylpiperidin-3-yl)methanol (60 mg, yield 86%) as a colorless oil. LCMS (m/z): 206.1 (M + H).

## Intermediate a16A-1

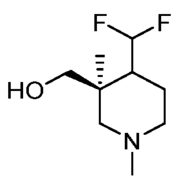

((3*S*)-4-(difluoromethyl)-1,3-dimethylpiperidin-3-yl)methanol

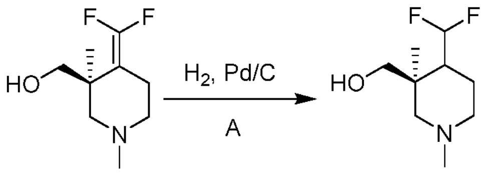

a15A-1 a16A-1

**Step A:** ((3*S*)-4-(difluoromethyl)-1,3-dimethylpiperidin-3-yl)methanol

At room temperature, (*S*)-(4-(difluoromethylene)-1,3-dimethylpiperidin-3-yl)methanol (240 mg, 1.26 mmol) was dissolved in methanol (20 mL), and the system was degassed with nitrogen three times. Palladium carbon (267 mg, 0.126 mmol) was added and degassed with hydrogen, and the reaction was stirred overnight at room temperature under a hydrogen atmosphere at 0.4 MPa. After the reaction was completed (monitored by LCMS), the reaction was filtered with celite, and the filter cake was washed with methanol three times. The filtrate was collected, concentrated to dryness to obtain ((3*S*)-4-(difluoromethyl)-1,3-dimethylpiperidin-3-yl)methanol (240 mg, yield 99%) as a colorless oil. LCMS (m/z): 194.1 (M + H).

## Intermediate a16A-2

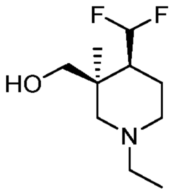

((3*S*,4*S*)-(4-(difluoromethyl)-1-ethyl-3-methylpiperidin-3-yl)methanol

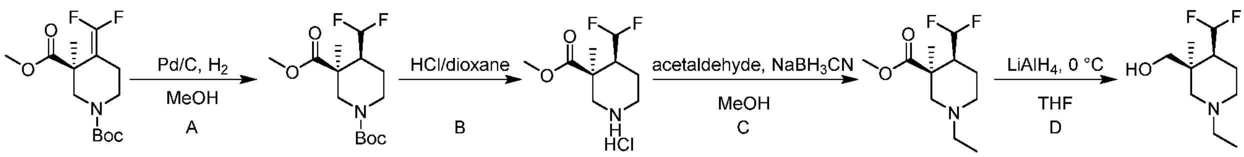

a15A-1-1 a16A-2-1 a16A-2-2 a16A-2-3 a16A-2

**Step A:** 1-(tert-butyl) 3-methyl (3*S*,4*S*)-4-(difluoromethyl)-3-methylpiperidine-1,3-dicarboxylate

At room temperature, 1-(*tert*-butyl) 3-methyl (*S*)-4-(difluoromethylene)-3-methylpiperidine-1,3-dicarboxylate (250 mg, 0.82 mmol) was dissolved in methanol (20 mL), and the system was degassed three times with nitrogen. Palladium carbon (87 mg, 0.08 mmol) was added, and the system was degassed three times with hydrogen. The reaction was stirred overnight at room temperature under hydrogen atmosphere at 15 Psi. After the reaction was completed (monitored by LCMS), the reaction was filtered with celite and the filter cake was washed three times with methanol. The filtrate was collected and concentrated to dryness to obtain 1-(tert-butyl) 3-methyl (3*S*,4*S*)-4-(difluoromethyl)-3-methylpiperidine-1,3-dicarboxylate (200 mg, yield 79%) as a colorless oil. LCMS (m/z): 252.1 (M + H).

**Step B:** methyl (3*S*,4*S*)-4-(difluoromethyl)-3-methylpiperidine-3-carboxylate hydrochloride

At room temperature, 4 M HCl-dioxane (5 mL) was added to 1-(tert-butyl) 3-methyl (3*S*,4*S*)-4-(difluoromethyl)-3-methylpiperidine-1,3-dicarboxylate (190 mg, 0.62 mmol). The resulting mixture was stirred at room temperature for 30 min, and the acid solution was removed by concentration to obtain methyl (3*S*,4*S*)-4-(difluoromethyl)-3-methylpiperidine-3-carboxylate hydrochloride (150 mg, yield 100%) as a white solid. LCMS (m/z): 208.1 (M + H).

**Step C:** methyl (3*S*,4*S*)-4-(difluoromethyl)-1-ethyl-3-methylpiperidine-3-carboxylate

At room temperature, methyl (3*S*,4*S*)-4-(difluoromethyl)-3-methylpiperidine-3-carboxylate hydrochloride (150 mg, 0.62 mmol) was dissolved in methanol (5 mL), and aqueous acetaldehyde solution (184 mg, 1.85 mmol, 40%) was added. The resulting mixture was stirred at room temperature for 30 min. Sodium cyanoborohydride (46 mg, 0.74 mmol) was added and the resulting mixture stirred for 3 h at room temperature. After the reaction was completed (monitored by LCMS), the system was concentrated to dryness and co-evaporated with anhydrous tetrahydrofuran twice to obtain methyl (3*S*,4*S*)-4-(difluoromethyl)-1-ethyl-3-methylpiperidine-3-carboxylate (140 mg, yield 97%) as a colorless oil. LCMS (m/z): 236.1 (M + H).

**Step D:** ((3*S*,4*S*)-(4-(difluoromethyl)-1-ethyl-3-methylpiperidin-3-yl)methanol

Under ice-bath conditions, 1 M $LiAlH_4$-THF (0.9 mL, 0.89 mmol) was added dropwise to methyl (3*S*,4*S*)-4-(difluoromethyl)-1-ethyl-3-methylpiperidine-3-carboxylate (140 mg, 0.60 mmol) in anhydrous THF (5 mL). The resulting mixture was stirred for 15 min at room temperature. After the reaction was completed (monitored by LCMS), the sodium sulfate decahydrate was added to quench the reaction until no bubbles were generated. The resulting mixture was dried over ~5 g anhydrous sodium sulfate, filtered through celite and the filter cake was washed three times with anhydrous tetrahydrofuran. The filtrate was collected and concentrated to dryness to give ((3*S*,4*S*)-(4-(difluoromethyl)-1-ethyl-3-methylpiperidin-3-yl)methanol (120 mg, yield 97%) as a colorless oil. LCMS (m/z): 208.1 (M + H).

## Intermediate a16A-3

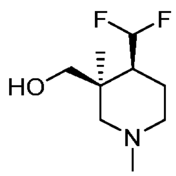

((3*S*,4*S*)-4-(difluoromethyl)-1,3-dimethylpiperidin-3-yl)methanol

Synthesis of intermediate a16A-3 was carried out according to the protocol described for intermediate a16A-2, using aqueous formaldehyde instead of aqueous acetaldehyde in Step D.
Determination of Crystal Structure of Intermediate **a16A-3**:
The sample was obtained by allowing the concentrated THF solution of compound **a16A-3** to stand for two days.
Detection Instrument: Brooke D8 Venture, Germany
Instrument Parameters:

| | |
|---|---|
| Source: Cu target | X-Ray: Cu-Kα (λ = 1.54178 Å) |
| Detector: CMOS Surface detector | Resolution: 0.80 Å |
| Current and voltage: 50 kV, 1.2 mA | Exposure time: 10s |
| Distance face detector to sample: 40 mm | Test temperature: 100 (2) K |

Structure elucidation and refinement process:

After integrating and reducing the diffraction data using the SAINT program, the data were empirically absorption-corrected using the SADABS program. The single-crystal structure was solved by the direct method using SHELXT2014, and the structure was refined by the least-squares method. The hydrogen atoms were obtained by isotropic calculation during the refinement process. The hydrogen atoms on C-H were added by calculation and refined using the riding model. The Flack constant is 0.02(9), and the configurations of C3 and C8 are S.

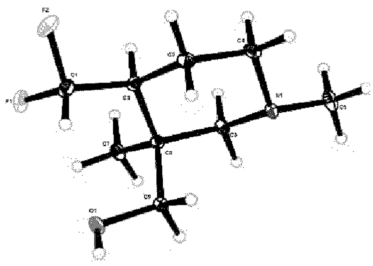

Single Crystal Data:

| | |
|---|---|
| $C_9H_{17}F_2NO$ | F(000) = 416 |
| $M_r$ = 193.23 | $D_x$ = 1.292 Mg $m^{-3}$ |
| Orthorhombic, $P2_12_12_1$ | Cu *K*a radiation, l = 1.54178 Å |
| *a* = 8.3870 (2) Å | Cell parameters from 5188 reflections |
| *b* = 10.1415 (2) Å | q = 5.8 - 72.3° |
| *c* = 11.6792 (3) Å | m = 0.92 $mm^{-1}$ |
| *V* = 993.39 (4) $Å^3$ | *T* = 100 K |
| *Z* = 4 | |

Data collection:

| | |
|---|---|
| Bruker APEX-II CCD diffractometer | $R_{int}$ = 0.064 |
| f and w scans | $q_{max}$ = 74.4 °, $q_{min}$ = 5.8° |
| 6966 measured reflections | *h* =-10®9 |
| 1969 independent reflections | *k* =-12®12 |
| 1887 reflections with *I* > 2s(*I*) | *l* =-14®12 |

Refinement:

| | |
|---|---|
| Refinement on $F^2$ | Hydrogen site inferred: location from sites |
| Least-squares matrix: full | H-atom constrained parameters |
| $R$ [$F^2$ > 2s($F^2$)]= 0.042 | $w = 1/[s^2(F_o^2) + (0.0605 P)^2 + 0.2541 P]$where $P = (F_o^2 + 2F_c^2)/3$ |
| $wR(F^2)$ = 0.111 | $(D/s)_{max}$ = 0.002 |
| *S* = 1.06 | $D_{ñmax}$ = 0.27 e $Å^{-3}$ |
| 1969 reflections | $D_{ñmin}$ =-0.29 e $Å^{-3}$ |
| 121 parameters | Absolute structure: Flack x determined using 746 quotients [(I+)-(I-)]/[(I+)+(I-)](Parsons, Flack and Wagner, Acta Cryst. B69 (2013) 249-259). |

(continued)

| | |
|---|---|
| 0 restraints | Absolute structure parameter: 0.01 (8) |
| Atomic fractional coordinates and isotropic or equivalent isotropic displacement parameters *(Å$^2$) for (cu 20231347 0m)* | |

| | *x* | *y* | *z* | $U_{iso}$*/$U_{eq}$ |
|---|---|---|---|---|
| F1 | 0.76298 (19) | 0.60599 (15) | 0.59005 (13) | 0.0295 (4) |
| F2 | 0.5960 (2) | 0.70504 (14) | 0.47647 (14) | 0.0327 (4) |
| O1 | 0.7102 (2) | 0.31035 (17) | 0.54075 (15) | 0.0217 (4) |
| H1 | 0.718014 | 0.262822 | 0.482116 | 0.033 * |
| N1 | 0.2151 (2) | 0.36986 (18) | 0.64304 (16) | 0.0153 (4) |
| C4 | 0.1932 (3) | 0.4965 (2) | 0.5840 (2) | 0.0191 (5) |
| H4A | 0.170185 | 0.566348 | 0.640814 | 0.023 * |
| H4B | 0.101165 | 0.490333 | 0.531158 | 0.023 * |
| C7 | 0.6333 (3) | 0.4271 (2) | 0.7624 (2) | 0.0200 (5) |
| H7A | 0.740340 | 0.435845 | 0.729641 | 0.030 * |
| H7B | 0.629692 | 0.349595 | 0.812423 | 0.030 * |
| H7C | 0.607655 | 0.506209 | 0.806865 | 0.030 * |
| C2 | 0.3424 (3) | 0.5324 (2) | 0.51676 (19) | 0.0183 (5) |
| H2A | 0.326345 | 0.618688 | 0.478787 | 0.022 * |
| H2B | 0.360853 | 0.465488 | 0.456509 | 0.022 * |
| C3 | 0.4896 (3) | 0.5400 (2) | 0.59515 (19) | 0.0154 (5) |
| H3 | 0.468387 | 0.611800 | 0.651816 | 0.018 * |
| C1 | 0.6311 (3) | 0.5839 (2) | 0.5244 (2) | 0.0212 (5) |
| H1A | 0.654879 | 0.517744 | 0.463232 | 0.025 * |
| C9 | 0.5575 (3) | 0.2921 (2) | 0.5912 (2) | 0.0162 (5) |
| H9A | 0.558771 | 0.211587 | 0.639084 | 0.019 * |
| H9B | 0.477089 | 0.279606 | 0.530070 | 0.019 * |
| C6 | 0.3504 (3) | 0.3809 (2) | 0.72256 (19) | 0.0162 (5) |
| H6A | 0.360074 | 0.297185 | 0.765643 | 0.019 * |
| H6B | 0.327017 | 0.451618 | 0.778512 | 0.019 * |
| C8 | 0.5107 (3) | 0.4107 (2) | 0.66504 (18) | 0.0141 (5) |
| C5 | 0.0708 (3) | 0.3351 (3) | 0.7059 (2) | 0.0241 (5) |
| H5A | 0.048455 | 0.402996 | 0.763395 | 0.036 * |
| H5B | 0.085988 | 0.249903 | 0.743978 | 0.036 * |
| H5C | -0.019098 | 0.328993 | 0.652521 | 0.036 * |
| Atomic displacement parameter *(Å$^2$) for (cu_20231347_0m)* | | | | |

| | $U^{11}$ | $U^{22}$ | $U^{33}$ | $U^{12}$ | $U^{13}$ | $U^{23}$ |
|---|---|---|---|---|---|---|
| F1 | 0.0226 (9) | 0.0322 (8) | 0.0336 (8) | -0.0116 (6) | 0.0014 (6) | -0.0025 (7) |
| F2 | 0.0415 (10) | 0.0209 (7) | 0.0357 (9) | -0.0089 (6) | 0.0031 (7) | 0.0099 (6) |

(continued)

| | $U^{11}$ | $U^{22}$ | $U^{33}$ | $U^{12}$ | $U^{13}$ | $U^{23}$ |
|---|---|---|---|---|---|---|
| O1 | 0.0139 (9) | 0.0260 (8) | 0.0254 (9) | -0.0004 (7) | 0.0034 (7) | -0.0107 (7) |
| N1 | 0.0114 (10) | 0.0190 (9) | 0.0154 (9) | 0.0000 (7) | 0.0007 (7) | 0.0008 (7) |
| C4 | 0.0167 (12) | 0.0205 (11) | 0.0201 (11) | 0.0053 (9) | -0.0039 (9) | -0.0001 (9) |
| C7 | 0.0152 (12) | 0.0269 (11) | 0.0181 (11) | 0.0013 (9) | -0.0033 (9) | -0.0030 (9) |
| C2 | 0.0200 (13) | 0.0185 (10) | 0.0165 (11) | 0.0027 (9) | -0.0020 (9) | 0.0027 (8) |
| C3 | 0.0183 (12) | 0.0123 (9) | 0.0156 (10) | -0.0013 (8) | 0.0015 (8) | -0.0016 (8) |
| C1 | 0.0234 (13) | 0.0177 (10) | 0.0224 (11) | -0.0049 (9) | 0.0035 (10) | 0.0008 (9) |
| C9 | 0.0146 (11) | 0.0160 (9) | 0.0179 (10) | 0.0008 (8) | 0.0013 (9) | -0.0012 (8) |
| C6 | 0.0150 (12) | 0.0196 (10) | 0.0140 (10) | 0.0012 (8) | 0.0010 (8) | 0.0000 (8) |
| C8 | 0.0139 (12) | 0.0147 (10) | 0.0136 (10) | 0.0001 (8) | 0.0001 (8) | -0.0011 (8) |
| C5 | 0.0145 (13) | 0.0395 (13) | 0.0184 (11) | -0.0014 (10) | 0.0014 (9) | -0.0034 (10) |
| Geometric parameters *(Å, °) for (cu_20231347_0m)* | | | | | | |

| | | | |
|---|---|---|---|
| F1 - C1 | 1.365 (3) | C2 - H2B | 0.9900 |
| F2 - C1 | 1.382 (3) | C2 - C3 | 1.539 (3) |
| O1 - H1 | 0.8400 | C3 - H3 | 1.0000 |
| O1 - C9 | 1.421 (3) | C3 - C1 | 1.513 (3) |
| N1 - C4 | 1.469 (3) | C3 - C8 | 1.555 (3) |
| N1 - C6 | 1.470 (3) | C1 - H1A | 1.0000 |
| N1 - C5 | 1.459 (3) | C9 - H9A | 0.9900 |
| C4 - H4A | 0.9900 | C9 - H9B | 0.9900 |
| C4 - H4B | 0.9900 | C9 - C8 | 1.531 (3) |
| C4 - C2 | 1.521 (3) | C6 - H6A | 0.9900 |
| C7 - H7A | 0.9800 | C6 - H6B | 0.9900 |
| C7 - H7B | 0.9800 | C6 - C8 | 1.534 (3) |
| C7 - H7C | 0.9800 | C5 - H5A | 0.9800 |
| C7 - C8 | 1.541 (3) | C5 - H5B | 0.9800 |
| C2 - H2A | 0.9900 | C5 - H5C | 0.9800 |
| | | | |
| C9 - O1 - H1 | 109.5 | F1 - C1 - C3 | 112.16 (19) |
| C4 - N1 - C6 | 109.07 (18) | F1 - C1 - H1A | 110.5 |
| C5 - N1 - C4 | 110.10 (19) | F2 - C1 - C3 | 108.4 (2) |
| C5 - N1 - C6 | 109.91 (17) | F2 - C1 - H1A | 110.5 |
| N1 - C4 - H4A | 109.6 | C3 - C1 -H1A | 110.5 |
| N1 - C4 - H4B | 109.6 | O1 - C9 - H9A | 109.4 |
| N1 - C4 - C2 | 110.42 (18) | O1 - C9 - H9B | 109.4 |
| H4A - C4 - H4B | 108.1 | O1 - C9 - C8 | 111.22 (18) |
| C2 - C4 - H4A | 109.6 | H9A - C9 - H9B | 108.0 |
| C2 - C4 - H4B | 109.6 | C8 - C9 - H9A | 109.4 |

(continued)

| | | | |
|---|---|---|---|
| H7A - C7 - H7B | 109.5 | C8 - C9 - H9B | 109.4 |
| H7A - C7 - H7C | 109.5 | N1 - C6 -H6A | 108.6 |
| H7B - C7 - H7C | 109.5 | N1 - C6 -H6B | 108.6 |
| C8 - C7 - H7A | 109.5 | N1-C6-C8 | 114.51 (17) |
| C8-C7-H7B | 109.5 | H6A - C6 - H6B | 107.6 |
| C8-C7-H7C | 109.5 | C8 - C6 - H6A | 108.6 |
| C4 - C2 - H2A | 109.3 | C8 - C6 - H6B | 108.6 |
| C4 - C2 - H2B | 109.3 | C7-C8-C3 | 111.86 (18) |
| C4-C2-C3 | 111.41 (17) | C9-C8-C7 | 109.22 (18) |
| H2A - C2 - H2B | 108.0 | C9 - C8 - C3 | 113.32 (17) |
| C3 - C2 - H2A | 109.3 | C9 - C8 - C6 | 108.45 (17) |
| C3 - C2 - H2B | 109.3 | C6 - C8 - C7 | 106.44 (18) |
| C2 - C3 - H3 | 106.7 | C6 - C8 - C3 | 107.25 (18) |
| C2 - C3 - C8 | 111.20 (18) | N1 - C5 - H5A | 109.5 |
| C1 - C3 - C2 | 108.60 (18) | N1 - C5 -H5B | 109.5 |
| C1 - C3 - H3 | 106.7 | N1 - C5 - H5C | 109.5 |
| C1 - C3 - C8 | 116.5 (2) | H5A - C5 - H5B | 109.5 |
| C8 - C3 - H3 | 106.7 | H5A - C5 - H5C | 109.5 |
| F1-C1-F2 | 104.72 (18) | H5B - C5 - H5C | 109.5 |
| | | | |
| O1 - C9 - C8 - C7 | -60.9 (2) | C2 - C3 - C8 - C7 | -167.36 (18) |
| O1 - C9 - C8 - C3 | 64.5 (2) | C2 - C3 -C8-C9 | 68.6 (2) |
| O1 - C9 - C8 - C6 | -176.49 (17) | C2 - C3 -C8-C6 | -51.0 (2) |
| N1-C4-C2-C3 | -57.9 (2) | C1 - C3 - C8 - C7 | 67.5 (2) |
| N1-C6-C8-C7 | 176.46 (18) | C1 - C3 - C8 - C9 | -56.5 (3) |
| N1-C6-C8-C3 | 56.6 (2) | C1 - C3 - C8 - C6 | -176.13 (18) |
| N1-C6-C8-C9 | -66.1 (2) | C6 - N1 - C4 - C2 | 60.2 (2) |
| C4 - N1 - C6 - C8 | -62.0 (2) | C8-C3-C1-F1 | -59.8 (2) |
| C4 - C2 - C3 - C1 | -176.68 (17) | C8-C3-C1-F2 | -174.95 (18) |
| C4 - C2 - C3 - C8 | 53.9 (2) | C5 -N1-C4-C2 | -179.15 (18) |
| C2 - C3 - C1 - | 173.76 (17) | C5 - N1 - C6 - | 177.22 (18) |
| F1 | | C8 | |
| C2 - C3 - C1 - F2 | 58.6 (2) | | |

# Intermediate a11A-1-D3

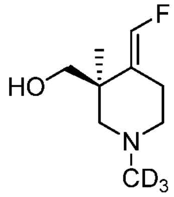

**a11A-1-D3**

(*S,E*)-(4-(fluoromethylene)-3-methyl-1-(methyl-ds)piperidin-3-yl)methanol

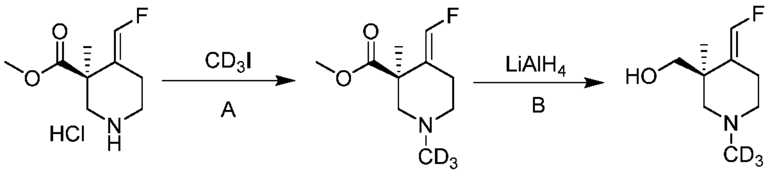


**a11A-1-2** **a11A-1-3-D3** **a11A-1-D3**

**Step A:** methyl (*S*,*E*)-4-(fluoromethylene)-3-methyl-1-(methyl-*d*$_3$)piperidine-3-carboxylate

At room temperature, potassium carbonate (5.55 g, 40.2 mmol) was added to a mixture of methyl (*S,E*)-4-(fluoromethylene)-3-methylpiperidine-3-carboxylate hydrochloride (3.00 g, 13.4 mmol) and deuterated methyl iodide (2.33 g, 16.1 mmol) in ACN (100 mL). The reaction was heated to 0 °C and stirred overnight. After the reaction was completed (monitored by LCMS), the reaction was filtered and the filter cake was washed with EA (50 mL). The filtrate was collected, concentrated, and further purified by FCC ($SiO_2$, EA/PE = 0-20%) to obtain methyl (*S,E*)-4-(fluoromethylene)-3-methyl-1-(methyl-*d*$_3$)piperidine-3-carboxylate (1.9 g, yield 69%) as a colorless liquid. LC-MS (m/z): 205.1 (M + H).

**Step B:** (*S,E*)-(4-(fluoromethylene)-3-methyl-1-(methyl-ds)piperidin-3-yl)methanol

Under ice-bath conditions, $LiAlH_4$ (1M-THF, 9.3 mmol, 9.3 mL) was added dropwise to a solution of methyl (*S*, *E*)-4-(fluoromethylene)-3-methyl-1-(methyl-*d*$_3$)piperidine-3-carboxylate (1.9 g, 9.3 mmol) in THF (50 mL). After the addition, the mixture was stirred in an ice-bath at 0 °C for 0.5 h. After the reaction was completed (monitored by LCMS), $Na_2SO_4$•$10H_2O$ was added to quench the reaction until no more gas was generated. The reaction solution was filtered through celite, and the obtained filtrate was concentrated at a low temperature (35 °C) to obtain (*S,E*)-(4-(fluoromethylene)-3-methyl-1-(methyl-*d*$_3$)piperidin-3-yl)methanol (1.3 g, yield 79%) as a colorless liquid. LC-MS (m/z): 177.1 (M + H).

# Intermediate a11A-1-D5

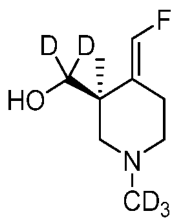

**a11A-1-D5**

(*S,E*)-(4-(fluoromethylene)-3-methyl-1-(methyl-*d*$_3$)piperidin-3-yl)methylene-*d*$_2$-ol

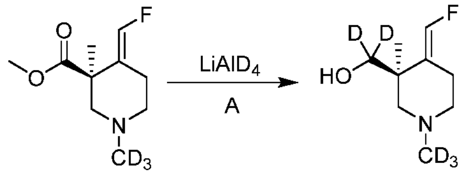


**a11A-1-3-D3** **a11A-1-D5**

**Step A:** (*S*,*E*)-(4-(fluoromethylene)-3-methyl-1-(methyl-*d*$_3$)piperidin-3-yl)methylene-*d*$_2$-ol

Under ice-bath conditions, $LiAlD_4$ powder (271.28 mg, 6.46 mmol) was added to a solution of methyl (*S*, *E*)-4-(fluoromethylene)-3-methyl-1-(methyl-*d*$_3$)piperidine-3-carboxylate (1.10 g, 5.39 mmol) in anhydrous tetrahydrofuran (15 mL). The resulting mixture was stirred for 15 min at room temperature. After the reaction was completed (monitored by LCMS), the reaction was quenched with sodium sulfate decahydrate until no bubbles were generated. About 5 g of anhydrous sodium sulfate was added to the resulting mixture to remove water. The mixture was filtered through celite and the filter cake was washed three times with anhydrous tetrahydrofuran. The filtrate was collected and concentrated to dryness to give (*S*,E)-(4-(fluoromethylene)-3-methyl-1-(methyl-ds)piperidin-3-yl)methylene-*d*$_2$-ol (951 mg, yield 99%) as a colorless oil. This product was used directly in the next step without purification. LCMS (m/z): 179.1 (M + H).

The following intermediates were prepared according to the above synthesis scheme:

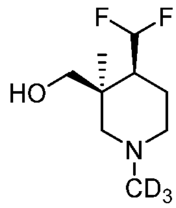

**a16A-3-D3**

((3*S*,4*S*)-4-(difluoromethyl)-3-methyl-1-(methyl-*d*$_3$)piperidin-3-yl)methanol

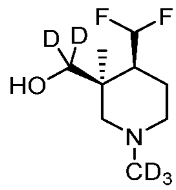

**a16A-3-D5**

((3*S*,4*S*)-4-(difluoromethyl)-3-methyl-1-(methyl-*d*$_3$)piperidin-3-yl)methylene-*d*$_2$-ol

# Intermediate a11A-4

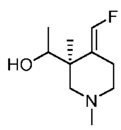

**a11A-4**

1-((*S*,*E*)-4-fluoromethylene-1,3-dimethylpiperidin-3-yl)ethan-1-ol

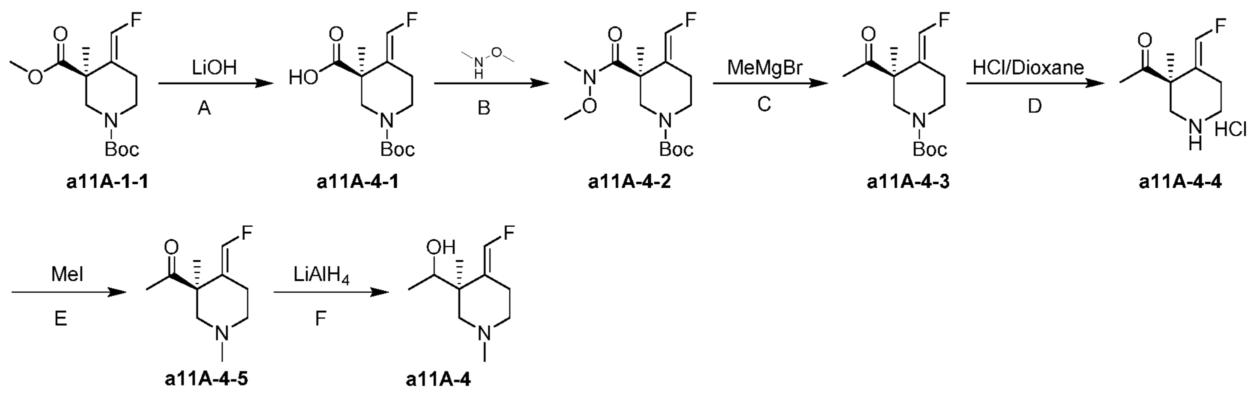


**Step A:** (*S*,*E*)-1-(*tert*-butoxycarbonyl)-4-(fluoromethylene)-3-methylpiperidine-3-carboxylic acid

At room temperature, $LiOH{\cdot}H_2O$ (1.32 g, 31.4 mmol) was added to a solution of 1-(*tert*-butyl) 3-methyl (*S,E*)-4-(fluoromethylene)-3-methylpiperidine-1,3-dicarboxylate (3.00g, 10.5 mmol) in $MeOH/H_2O$ (30 mL/30 mL). The reaction was stirred for 16 hours at room temperature. After the reaction was completed (monitored by LCMS), the reaction concentrated and adjusted to pH≈5 with HCl (1N). The mixture was extracted with DCM (50 mL × 3). The combined organic phase was washed with brine (50 mL), dried over anhydrous $Na_2SO_4$, filtered, and concentrated to give (*S,E*)-1-(tert-butoxycarbonyl)-4-(fluoromethylene)-3-methylpiperidine-3-carboxylic acid (2.7 g, yield 95%) as a white solid. LCMS (m/z): 296.1 (M + Na).

**Step B:** *tert*-butyl (*S,F*)-4-(fluoromethylene)-3-(methoxy(methyl)carbamoyl)-3-methylpiperidine-1-carboxylate

At room temperature, TEA (2.52 g, 24.9 mmol) was added to a mixture of (*S,E*)-1-(*tert*-butoxycarbonyl)-4-(fluoromethylene)-3-methylpiperidine-3-carboxylic acid (1.70 g, 6.22 mmol), dimethylhydroxylamine hydrochloride (0.91 g, 9.33 mmol), HATU (4.73 g, 12.4 mmol) in DCM (50 mL). The reaction was stirred for 16 hours at room temperature. After the reaction was completed (monitored by LCMS), the crude product obtained after concentrating the reaction solution was purified by FCC ($SiO_2$, EA/PE = 0-25%) to obtain *tert*-butyl (*S,E*)-4-(fluoromethylene)-3-(methoxy(methyl)carbamoyl)-3-methylpiperidine-1-carboxylate (2.0 g, yield 100%) as a colorless oil. LCMS (m/z): 261.0 (M-56).

**Step C:** *(S,E)-tert-butyl* 3-acetyl-4-(fluoromethylene)-3-methylpiperidine-1-carboxylate

Under a dry ice-ethanol bath, MeMgBr (6.30 mL, 19.0 mmol) was added dropwise to a mixture of *tert*-butyl (*S*, *E*)-4-(fluoromethylene)-3-(methoxy(methyl)carbamoyl)-3-methylpiperidine-1-carboxylate (2.00 g, 6.33 mmol) in THF (50 mL). After addition, the reaction was warmed to room temperature and continue stirring for 16 h. After the reaction was completed (monitored by LCMS), the reaction was poured into saturated aqueous $NH_4Cl$ (30 mL) solution and extracted with EA (30 mL × 3). The organic phase was combined, washed with brine (70 mL), dried over anhydrous $Na_2SO_4$, filtered, and the concentrated organic phase was purified by FCC ($SiO_2$, EA/PE = 0-25%) to obtain *tert*-butyl (*S,E*)-3-acetyl-4-(fluoromethylene)-3-methylpiperidine-1-carboxylate (1.4g, yield 81%) as a colorless oil. LCMS (m/z): 216.0 (M-56).

**Step D:** (*S*,*E*)-1-(4-(fluoromethylene)-3-methylpiperidin-3-yl)ethan-1-one hydrochloride

At room temperature, HCl/dioxane (4M) (28 mL, 20V) was added to *tert*-butyl (*S,E*)-3-acetyl-4-(fluoromethylene)-3-methylpiperidine-1-carboxylate (1.40 g, 5.16 mmol). The reaction was stirred for 1 h at room temperature. After the reaction was completed (monitored by LCMS), the reaction was concentrated to give (*S,E*)-1-(4-(fluoromethylene)-3-methylpiperidin-3-yl)ethan-1-one hydrochloride (1.08 g, yield 100%) as a white solid. LCMS (m/z): 172.1 (M + H).

**Step E:** (*S*,*E*)-1-(4-fluoromethylene)-1,3-dimethylpiperidin-3-yl*)*ethan-1-one

At room temperature, $K_2CO_3$ (2.87 g, 20.8 mmol) was added to a mixture of (*S,E*)-1-(4-(fluoromethylene)-3-methylpiperidin-3-yl)ethan-1-one hydrochloride (1.08 g, 5.20 mmol) and $CH_3I$ (812 mg, 5.72 mmol) in ACN (20 mL). After the addition, the reaction was heated to 60 °C and stirred 3 h. After the reaction was completed (monitored by LCMS), the reaction was filtered and washed with EA (10 mL). The filtrate was collected, concentrated, and purified by FCC ($SiO_2$, EA/PE = 0-25%) to obtain (*S,E*)-1-(4-fluoromethylene)-1,3-dimethylpiperidin-3-yl)ethan-1-one (560 mg, yield 58%) as a colorless oil. LCMS (m/z): 186.1 (M + H).

**Step F:** 1-((*S,E*)-4-fluoromethylene-1,3-dimethylpiperidin-3-yl)ethan-1-ol

In an ice-water bath, $LiAlH_4$ (2.5 M/THF, 1.1 mmol, 0.43 mL) was added dropwise to a solution of (*S*,*E*)-1-(4-fluoromethylene)-1,3-dimethylpiperidin-3-yl)ethan-1-one (200 mg, 1.08 mmol) in THF (5 mL). After the addition, the mixture was stirred in the ice-water bath for another 1 h. After the reaction was completed (monitored by LCMS), the reaction was quenched by $Na_2SO_4 \cdot 10H_2O$ until no gas was generated. The resulting mixture was filtered through celite, and the filtrate was concentrated at 35°C to obtain 1-((*S*,*E*)-4-fluoromethylene-1,3-dimethylpiperidin-3-yl)ethan-1-ol (150 mg, yield 74%) as a colorless oil. LCMS (m/z): 188.1 (M + H).

The following intermediates were prepared referring to the synthesis scheme of **a11A-4** above:

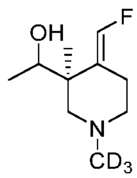

**a11A-4-D3**

1-(*S,E*)-4-(fluoromethylene)-3-methyl-1-(methyl-*d*$_3$)piperidin-3-yl)ethan-1-ol

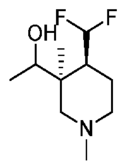

**a16A-4**

1-((3*S*,4*S*)-4-(difluoromethyl)-1,3-dimethylpiperidin-3-yl)ethan-1-ol

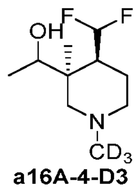

**a16A-4-D3**

1-((3*S*,4*S*)-4-(difluoromethyl)-3-methyl-1-(methyl-*d*$_3$)piperidin-3-yl)ethan-1-ol

## Intermediate a16-5

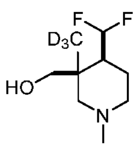

**a16-5**

(($\pm$)-(3*S*,4*S*)-4-(difluoromethyl)-1-methyl-3-(methyl-*d*$_3$)piperidin-3-yl)methanol

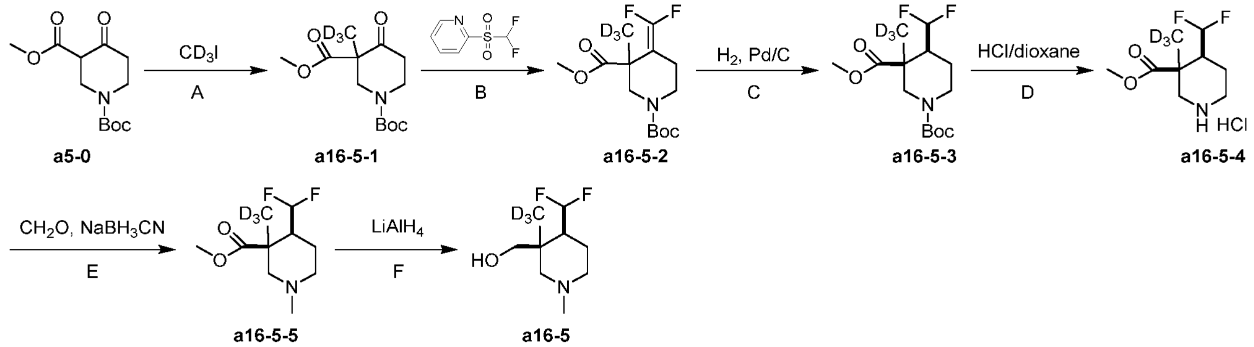


**Step A:** 1-*tert*-butyl 3-methyl 3-(methyl-$d_3$)-4-oxypiperidine-1,3-dicarboxylate

At room temperature, cesium carbonate (15.2 g, 46.64 mmol) and deuterated methyl iodide (8.4 g, 58.30 mmol) were successively added to a solution of 1-tert-butyl 3-methyl 4-oxopiperidine-1,3-dicarboxylate (10 g, 38.87 mmol) in acetonitrile (100 mL). The resulting mixture was stirred for 2 h at 35 °C. After the reaction was completed (monitored by LCMS), the reaction was filtered with celite and the filter cake was washed twice with acetonitrile. The filtrate was collected, concentrated, and dried to obtain the crude product which was subjected to FCC ($SiO_2$, EA/PE = 0-20%) purification to afford 1-*tert*-butyl 3-methyl 3-(methyl-$d_3$)-4-oxypiperidine-1,3-dicarboxylate (7.4 g, yield 69%) as a colorless oil. LCMS (m/z): 219.1 (M-56 + H).

**Step B:** 1-*tert*-butyl 3-methyl 4-(difluoromethylene)-3-(methyl-$d_3$)piperidine-1,3-dicarboxylate

*1-tert-butyl* 3-*methyl* 3-(methyl-$d_3$)-4-oxypiperidine-1,3-dicarboxylate (2.0g, 7.29 mmol) and 2-((difluoromethyl) sulfonyl)pyridine (2.11 g, 10.94 mmol) were dissolved in anhydrous DMF (20 mL), and the reaction system was degassed with nitrogen three times. Under dry ice-ethanol cooling, the reaction was cooled to -60 °C, and potassium *tert*-butyl alcohol tetrahydrofuran solution (14.6 mL, 14.6 mmol, 1 M in THF) was added dropwise. The resulting mixture was stirred at -60 °C for 1 h, slowly warmed to room temperature and stirred overnight. After the reaction was completed (monitored by LCMS), the reaction was quenched with saturated aqueous ammonium chloride (50 mL) and extracted 5 times with ethyl acetate. The combined organic phase was washed with LiCl aqueous solution (30 mL × 3, 4 wt%) and brine, dried over anhydrous sodium sulfate, filtered, and concentrated to dryness. The resulting crude product was purified by FCC ($SiO_2$, EA/PE = 0-20%) to obtain a yellow oil. This product was dissolved in methanol (20 mL), activated carbon (840 mg) was added, and the mixture was stirred at 30 °C overnight. The reaction solution was filtered through celite, and the filtrate was concentrated to dryness to obtain 1-*tert*-butyl 3-methyl 4-(difluoromethylene)-3-(methyl-$d_3$)piperidine-1,3-dicarboxylate (1.1 g, yield 49%) as a pale-yellow oil. LCMS (m/z): 253.1 (M-56 + H).

**Step C:** 1-*tert*-butyl 3-methyl (±)-(3*S*,4*S*)-4-(difluoromethyl)-3-(methyl-$d_3$)piperidine-1,3-dicarboxylate

At room temperature, 1-*tert*-butyl 3-methyl 4-(difluoromethylene)-3-(methyl-$d_3$)piperidine-1,3-dicarboxylate (1.1 g, 3.57 mmol) was dissolved in methanol (20 mL) and degassed with nitrogen. Palladium carbon (760 mg, 0.71 mmol, 10 wt%) was added and degassed three times with hydrogen. The reaction was stirred for 2 hours at 30 °C under a hydrogen atmosphere at 15 Psi. After the reaction was completed (monitored by LCMS), the reaction was filtered with celite. The filtrate was collected, concentrated, and dried to obtain 1-*tert*-butyl 3-methyl (±)-(3*S*,4*S*)-4-(difluoromethyl)-3-(methyl-$d_3$) piperidine-1,3-dicarboxylate (600 mg, yield 54%) as a colorless oil. LCMS (m/z): 255.1 (M-56 + H).

**Step D:** methyl (±)-(3*S*,4*S*)-4-(difluoromethyl)-3-(methyl-$d_3$)piperidine-3-carboxylate hydrochloride

At room temperature, 1-*tert*-butyl 3-methyl (±)-(3*S*,4*S*)-4-(difluoromethyl)-3-(methyl-$d_3$)piperidine-1,3-dicarboxylate (600 mg, 1.93 mmol) was dissolved in 4 M hydrochloric acid-dioxane (10 mL). The resulting mixture was stirred at room temperature for 30 min, and the acid solution was removed by concentration to obtain methyl (±)-(3*S*,4*S*)-4-(difluoromethyl)-3-(methyl-$d_3$)piperidine-3-carboxylate hydrochloride (475 mg, yield 100%) as a white solid. LCMS (m/z): 211.1 (M + H).

**Step E:** methyl (±)-(3*S*,4*S*)-4-(difluoromethyl)-1-methyl-3-(methyl-$d_3$)piperidine-3-carboxylate

At room temperature, methyl (±)-(3*S*,4*S*)-4-(difluoromethyl)-3-(methyl-$d_3$)piperidine-3-carboxylate hydrochlor-

ide (475 mg, 1.93 mmol) was dissolved in methanol (10 mL), and aqueous formaldehyde solution (469 mg, 5.78 mmol, 37 wt%) was added. The resulting mixture was stirred for 10 min at room temperature. Sodium cyanoborohydride (157 mg, 2.50 mmol) was added and the resulting mixture was stirred for 1 h at room temperature. After the reaction was completed (monitored by LCMS), the reaction was concentrated to dryness. The crude product was dissolved in ethyl acetate and filtered through celite. The filtrate was purified by FCC ($SiO_2$, MeOH/DCM = 0-5%), concentrated, and co-evaporated with anhydrous tetrahydrofuran twice to obtain methyl (±)-(3*S*,4*S*)-4-(difluoromethyl)-1-methyl-3-(methyl-*d*$_3$)piperidine-3-carboxylate (180 mg, yield 42%) as a colorless oil. LCMS (m/z): 225.1 (M + H).

**Step F:** ((±)-(3*S*,4*S*)-4-(difluoromethyl)-1-methyl-3-(methyl-*d*$_3$)piperidin-3-yl)methanol

Under an ice-bath condition, 1 M $LiAlH_4$-THF (0.80 mL, 0.80 mmol) was added dropwise to a solution of methyl (±)-(3*S*,4*S*)-4-(difluoromethyl)-1-methyl-3-(methyl-*d*$_3$)piperidine-3-carboxylate (180 mg, 0.80 mmol) in anhydrous THF (10 mL). The resulting mixture was stirred for 15 min at 0 °C. After the reaction was completed (monitored by LCMS), the reaction was quenched by adding sodium sulfate decahydrate until no bubbles were generated. The resulting mixture was dried over ~5 g anhydrous sodium sulfate and filtered with celite. The filter cake was washed 3 times with anhydrous tetrahydrofuran. The filtrate was collected, concentrated, and dried to give ((±)-(3*S*,4*S*)-4-(difluoromethyl)-1-methyl-3-(methyl-*d*$_3$)piperidin-3-yl)methanol (150 mg, yield 95%) as a colorless crystalline solid. LCMS (m/z): 197.1 (M + H).

The following intermediates were prepared according to the synthesis scheme of intermediate **a16-5** described above:

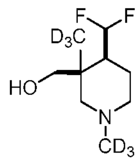

**a16-5**

((±)-(3*S*,4*S*)-4-(difluoromethyl)-1-(methyl-*d*$_3$)-3-(methyl-*d*$_3$)piperidin-3-yl)methanol

## Intermediate a16-6

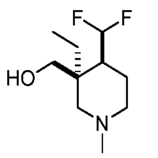

**a16-6** ((±)-(3*S*,4*S*)-4-(difluoromethyl)-3-ethyl-1-methylpiperidin-3-yl)methanol

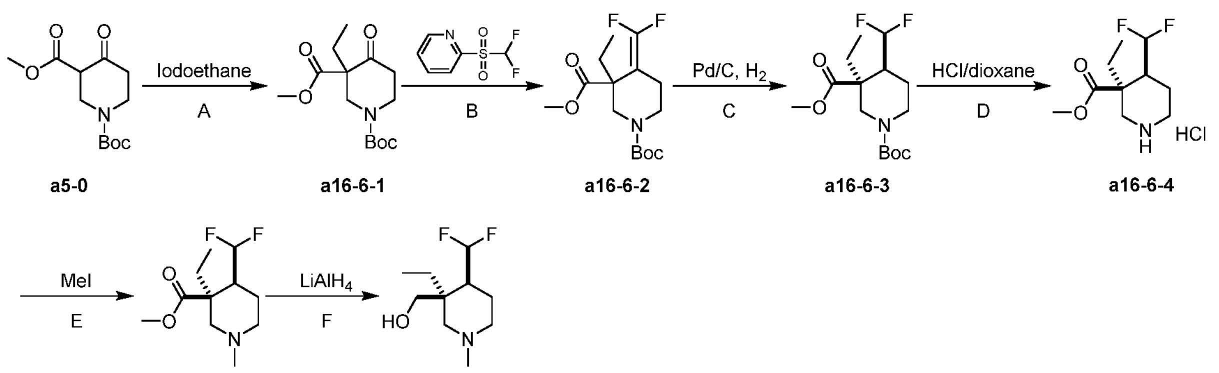


**a5-0** **a16-6-1** **a16-6-2** **a16-6-3** **a16-6-4** **a16-6-5** **a16-6**

Step **A:** 1-*tert*-butyl 3-methyl 3-ethyl-4-oxopiperidine-1,3-dicarboxylate

At room temperature, to a mixture of 1-*tert*-butyl 3-methyl 4-oxopiperidine-1,3-dicarboxylate (10.0 g, 38.9 mmol) and cesium carbonate (15.2 g, 46.6 mmol) in acetonitrile (100 mL), iodoethane (9.1g, 58.3 mmol) was added. The reaction

was heated to 45 °C for and stirred overnight. After the reaction was completed (monitored by LCMS), the reaction was filtered by celite. The filtrate was concentrated to dryness, and the resulting crude product was purified by FCC ($SiO_2$, EA/PE = 0-20%) to give 1-*tert*-butyl 3-methyl 3-ethyl-4-oxopiperidine-1,3-dicarboxylate (7.0 g, yield 63%) as a colorless oil. LCMS (m/z): 230.0 (M-56 + H). $^1$H NMR (400 MHz, Chloroform-d) δ 4.52 - 4.29 (m, 1H), 4.04 - 3.89 (m, 1H), 3.74 (s, 3H), 3.53 - 3.37 (m, 1H), 3.37 - 3.18 (m, 1H), 2.76 - 2.57 (m, 1H), 2.57 - 2.40 (m, 1H), 1.99 - 1.85 (m, 1H), 1.75 - 1.62 (m, 1H), 1.49 (s, 9H), 0.93 (t, *J* = 7.5 Hz, 3H).

**Step B:** 1-tert-butyl 3-methyl 4-(difluoromethylene)-3-ethylpiperidine-1,3-dicarboxylate

At room temperature, 1-*tert*-butyl 3-methyl 3-ethyl-4-oxopiperidine-1,3-dicarboxylate (7.0 g, 24.5 mmol) and 2-((difluoromethyl)sulfonyl)pyridine (7.11 g, 36.8 mmol) were dissolved in anhydrous DMF (70 mL), and the system was degassed with nitrogen three times. Under dry ice-ethanol cooling, the resulting mixture was cooled to -60 °C and potassium *tert*-butoxide (49.1 mL, 49.1 mmol, 1 M in THF) was added dropwise. The resulting mixture was stirred at -60 °C for 1 h and then slowly warmed to room temperature and stirred overnight. After the reaction was completed (monitored by LCMS), the reaction was quenched with saturated aqueous ammonium chloride (150 mL) and extracted with ethyl acetate (200 mL × 4). The combined organic layers were washed twice with LiCl aqueous solution (100 mL, 4 wt%) and brine, dried over anhydrous sodium sulfate, filtered, concentrated. The resulting crude product was purified by FCC ($SiO_2$, EA/PE = 0-50%) to obtain 1-*tert*-butyl 3-methyl 4-(difluoromethylene)-3-ethylpiperidine-1,3-dicarboxylate (3.5 g, yield 45%) as a colorless oil. LCMS (m/z): 264.0 (M-56 + H).

**Step C:** 1-*tert*-butyl 3-methyl (±)-(3*S*,4*S*)-4-difluoromethyl-3-ethylpiperidine-1,3-dicarboxylate

At room temperature, 1-*tert*-butyl 3-methyl 4-(difluoromethylene)-3-ethylpiperidine-1,3-dicarboxylate (3.50 g, 11.0 mmol) was dissolved in methanol (50 mL), and the mixture was degassed with nitrogen. Wet palladium carbon (5.0 g, 2.35 mmol, 10 wt%) was added and degassed three times with hydrogen. The reaction was stirred for 2 hours at 30 °C under a hydrogen atmosphere at 15 Psi. After the reaction was completed (monitored by LCMS), the reaction was filtered with celite, the filtrate was collected, and concentrated to dryness. The resulting crude product was purified by FCC ($SiO_2$, EA/PE = 0-10%) to obtain 1-*tert*-butyl 3-methyl (±)-(3*S*,4*S*)-4-difluoromethyl-3-ethylpiperidine-1,3-dicarboxylate (3.50 g, yield 99%) as a colorless oil. LCMS (m/z): 266.0 (M-56 + H).

**Step D:** methyl (±)-(3*S*,4*S*)-4-difluoromethyl-3-ethylpiperidine-3-carboxylate hydrochloride

At room temperature, 1-*tert*-butyl 3-methyl (±)-(3*S*,4*S*)-4-(difluoromethyl)-3-ethylpiperidine-1,3-dicarboxylate (3.50 g, 10.9 mmol) was dissolved in 4 M hydrochloric acid in dioxane (50 mL). The resulting mixture was stirred at room temperature for 1 h, concentrated to remove the acid solution to afford methyl (±)-(3*S*,4*S*)-4-difluoromethyl-3-ethylpiperidine-3-carboxylate hydrochloride (2.81 g, yield 100%) as a white solid. LCMS (m/z): 222.1 (M + H).

**Step E:** methyl (±)-(3*S*,4*S*)-4-(difluoromethyl)-3-ethyl-1-methylpiperidine-3-carboxylate

At room temperature, to a mixture of methyl (±)-(3*S*,4*S*)-4-(difluoromethyl)-3-ethylpiperidine-3-carboxylate hydrochloride (500 mg, 1.94 mmol) and potassium carbonate (804 mg, 5.82 mmol) in acetonitrile (5 mL), methyl iodide (413 mg, 2.91 mmol) was added. The reaction was heated to 50 °C and stirred for 3 h. After the reaction was completed (monitored by LCMS), the reaction was filtered by celite, the filtrate was concentrated to dryness, and the resulting crude product was purified by FCC ($SiO_2$, EA/PE = 0-50%) to obtain methyl (±)-(3*S*,4*S*)-4-(difluoromethyl)-3-ethyl-1-methylpiperidine-3-carboxylate (150 mg, yield 33%) as a colorless oil. LCMS (m/z): 236.1 (M + H).

**Step F:** ((±)-(3*S*,4*S*)-4-(difluoromethyl)-3-ethyl-1-methylpiperidin-3-yl)methanol

Under ice-bath conditions, 1 M $LiAlH_4$-THF (0.64 mL, 0.64 mmol) was added dropwise to a solution of methyl (±)-(3*S*,4*S*)-4-(difluoromethyl)-3-ethyl-1-methylpiperidine-3-carboxylate (150 mg, 0.638 mmol) in anhydrous THF (2 mL). The resulting mixture was stirred for 30 min at 0 °C. At the end of the reaction monitored by LCMS, sodium sulfate decahydrate was added to quench the reaction until no bubbles were generated. The resulting mixture was dried over anhydrous sodium sulfate and filtered with celite. The filter cake was washed 3 times with anhydrous tetrahydrofuran. The filtrate was collected and concentrated to dryness to obtain ((±)-(3*S*,4*S*)-4-(difluoromethyl)-3-ethyl-1-methylpiperidin-3-yl)methanol (90 mg, yield 68%) as a colorless oil. LCMS (m/z): 208.1 (M + H).

The following intermediates were prepared according to the above synthesis scheme:

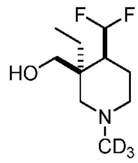

**a16-6-D3**

((±)-(3*S*,4*S*)-4-(difluoromethyl)-3-ethyl-1-(methyl-$d_3$)piperidin-3-yl)methanol

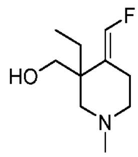

**a11-5**

(*E*)-(3-ethyl-4-(fluoromethylene)-1-methylpiperidin-3-yl)methanol

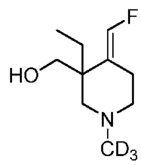

**a11-5-D3**

(*E*)-(3-ethyl-4-(fluoromethylene)-1-(methyl-$d_3$)piperidin-3-yl)methanol

## Intermediate a16A-3-D7

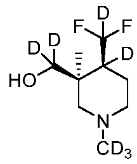

**a16A-3-D7**

((3*S*,4*S*)-4-(difluoromethyl-*d*)-3-methyl-1-(methyl-$d_3$)piperidin-3-yl-4-*d*)methylene-$d_2$-ol

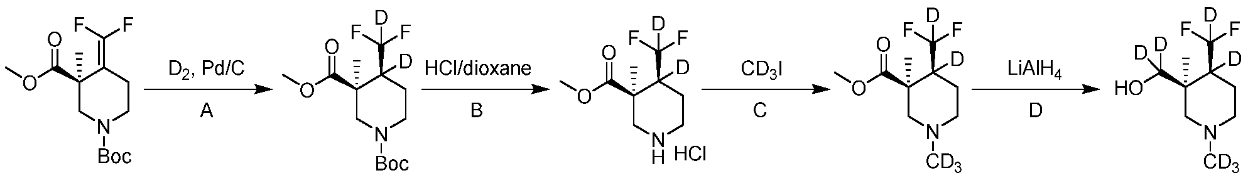


**a15A-1-1** **a16A-2-1-D2** **a16A-2-2-D2** **a16A-3-1-D5** **a16A-3-D7**

**Step A:** 1-*tert*-butyl 3-methyl (3*S*,4*S*)-4-(difluoromethyl-*d*)-3-methylpiperidine-1,3-dicarboxylate-4-*d*

At room temperature, 1-*tert*-butyl 3-methyl (*S*)-4-(difluoromethylene)-3-methylpiperidine-1,3-dicarboxylate (1.20 g, 3.93 mmol) was dissolved in deuterated methanol (12 mL) and the resulting mixture was degassed with nitrogen. Palladium carbon (1.25 g, 1.18 mmol, 10 wt%) was added and degassed with deuterium three times. The reaction was stirred for 2 h at 30 °C in a deuterium atmosphere at 15 Psi. After the reaction was completed (monitored by LCMS), the reaction was filtered with celite, the filtrate was collected, concentrated, and dried to give the crude product. The crude product was purified by FCC ($SiO_2$, EtOAc/PE = 0-20%) to obtain 1-*tert*-butyl 3-methyl (3*S*,4*S*)-4-(difluoromethyl-*d*)-3-methylpiperidine-1,3-dicarboxylate-4-*d* (936 mg, yield 77%) as a pale yellow oil. LCMS (m/z): 254.1 (M-56 + H).

**Step B:** methyl (3*S*,4*S*)-4-(difluoromethyl-*d*)-3-methylpiperidine-3-carboxylate-4-*d* hydrochloride

At room temperature, 1-*tert*-butyl 3-methyl (3S,4S)-4-(difluoromethyl-*d*)-3-methylpiperidine-1,3-dicarboxylate-4-*d* (936 mg, 3.03 mmol) was dissolved in 4 M hydrochloric acid in dioxane (10 mL). The resulting mixture was stirred at room temperature for 30 min and concentrated to remove the acid solution to obtain methyl (3*S*,4*S*)-4-(difluoromethyl-d)-3-methylpiperidine-3-carboxylate-4-d hydrochloride (740 mg, yield 100%) as a white solid. LCMS (m/z): 210.1 (M + H).

**Step C:** methyl (3*S*,4*S*)-4-(difluoromethyl-*d*)-3-methyl-1-(methyl-$d_3$)piperidine-3-carboxylate-4-*d*

At room temperature, potassium carbonate (2.08 g, 15.06 mmol) and deuterated methyl iodide (524 mg, 3.61 mmol) were successively added to a solution of methyl (3*S*,4*S*)-4-(difluoromethyl-*d*)-3-methylpiperidine-3-carboxylate-4-*d* hydrochloride (740 mg, 3.01 mmol) in acetonitrile (10 mL), the resulting mixture was stirred at 50 °C for 5 h. After the reaction was completed (monitored by LCMS), the reaction was filtered with celite and the filter cake was washed twice with acetonitrile. The filtrate was collected and concentrated to obtain the crude product. The crude product was purified by FCC ($SiO_2$, MeOH/DCM = 0-5%) to obtain methyl (3S,4S)-4-(difluoromethyl-*d*)-3-methyl-1-(methyl-$d_3$)piperidine-3-carboxylate-4-*d* (410 mg, yield 60%) as a pale-yellow oil. LCMS (m/z): 227.1 (M + H).

**Step D:** ((3*S*,4*S*)-4-(difluoromethyl-*d*)-3-methyl-1-(methyl-$d_3$)piperidin-3-yl-4-*d*)methylene-$d_2$-ol

Under ice-bath conditions, $LiAlD_4$ (76 mg, 1.81 mmol) was added to a solution of methyl (3*S*,4*S*)-4-(difluoromethyl-*d*)-3-methyl-1-(methyl-$d_3$)piperidine-3-carboxylate-4-*d* (410 mg, 1.81 mmol) in anhydrous THF (10 mL). The resulting mixture was stirred at 0 °C for 15 min. After the reaction was completed (monitored by LCMS), the sodium sulfate decahydrate was added to quench the reaction until no bubbles were generated. The resulting mixture was dried over ~5 g of anhydrous sodium sulfate and filtered with celite. The filter cake was washed 3 times with anhydrous tetrahydrofuran. The filtrate was collected, concentrated, and dried to give ((3*S*,4*S*)-4-(difluoromethyl-*d*)-3-methyl-1-(methyl-$d_3$)piperidin-3-yl-4-*d*)methylene-$d_2$-ol (280 mg, yield 77%) as colorless oil. GCMS (m/z): 200 ($M^{\cdot +}$).

## Intermediate b1

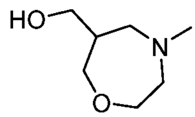

(4-methyl-1,4-oxazepan-6-yl)methanol

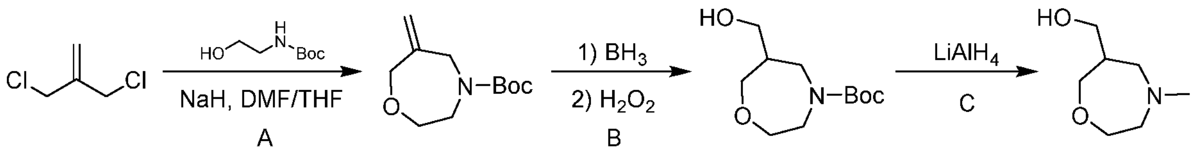


**Step A:** tert-butyl 6-methylene-1,4-oxacyclopentan-4-carboxylate

NaH (3.52 g, 60 w/w%, 0.088 mol) was added to a solution of 3-chloro-2-(chloromethyl) propyl-1-ene (5.0 g, 0.04 mol) in DMF (50 mL) at 0 °C. With stirring, a solution of *tert*-butyl-(2-hydroxyethyl)carbamate (6.44 g, 0.04 mol) in THF (50 mL) was added to the reaction, and the resulting mixture was stirred for 3 h at room temperature. The mixture was poured into $H_2O$ (100 mL) and extracted with EA (100 mL × 3). The organic phase was washed with brine (200 mL), dried over anhydrous $Na_2SO_4$, and purified by FCC ($SiO_2$, EA/PE = 0-100%) to give *tert*-butyl 6-methylene-1,4-oxacyclopentan-4-carboxylate (3.5g, yield 41%) as a colorless liquid. $^1H$ NMR (400 MHz, $CDCl_3$) δ 5.10 - 4.91 (m, 2H), 4.20 - 4.05 (m, 4H), 3.79 - 3.67 (m, 2H), 3.56 - 3.43 (m, 2H), 1.47 (s, 9H).

**Step B:** *tert*-butyl 6-(hydroxymethyl)-1,4-oxacyclopentan-4-carboxylate

At room temperature, a borane-tetrahydrofuran solution (5.0 mL, 1.0 M, 5.0 mmol) was added to a solution of *tert*-butyl 6-methylene-1,4-oxacyclopentan-4-carboxylate (1.0 g, 4.69 mmol) in THF (10 mL). The reaction was stirred at room temperature for 3 hours. Then the reaction was cooled in an ice bath, and a 3 M aqueous NaOH solution (1.6 mL, 4.8 mmol) and hydrogen peroxide (0.7 mL, ~30 w/w%, 6.86 mmol) were added successively. The resulting mixture was allowed to stir at room temperature for 16 hours. After the reaction was completed (monitored by TLC, Rf = 0.4, EA/PE = 1: 1), the mixture was extracted with n-pentane (20 mL × 3). The organic phase was washed with brine (50 mL), dried over anhydrous $Na_2SO_4$, and concentrated under reduced pressure to obtain *tert*-butyl 6-(hydroxymethyl)-1,4-oxacyclopentan-4-carboxylate (crude) as a colorless liquid, which was used directly in the subsequent reaction.

**Step C:** (4-methyl-1,4-oxazepan-6-yl)methanol

At room temperature, to a solution of *tert*-butyl 6-(hydroxymethyl)-1,4-oxacyclopentan-4-carboxylate (crude, 4.69 mmol) in THF (5.0 mL), $LiAlH_4$-tetrahydrofuran (2.3 mL, 3 M, 6.9 mmol) was added. The resulting mixture was heated to 80 °C and stirred for 1 hour. After the reaction was completed (monitored by TLC, Rf = 0.2, EA/PE = 2:1), the system was cooled to room temperature, and excess sodium sulfate decahydrate was added until no bubbles were generated. The solid was removed by filtration. The filtrate was concentrated under reduced pressure to give (4-methyl-1,4-oxazepan-6-yl)methanol (600 mg, crude) as a colorless liquid, which was directly used in the subsequent reaction.

## Intermediate b2

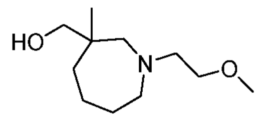

(1-(2-methoxyethyl)-3-methylazepan-3-yl)methanol

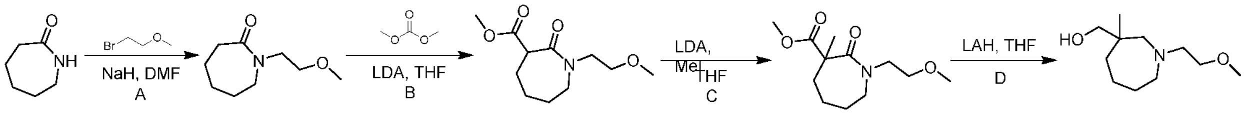


**Step A:** 1-(2-methoxyethyl)azepan-2-one

Under an ice bath, caprolactam (10.0 g, 88 mmol) was dissolved in 100 mL anhydrous DMF. Then sodium hydride (7.07 g, 176 mmol) was slowly added to the reaction system. After stirring the reaction at this temperature for half an hour, 1-bromo-2-methoxyethane (18.4 g, 132 mmol) was added to the reaction, and the resulting mixture was allowed to react at room temperature for additional 12 hours. After the reaction was completed (monitored by TLC, 100% EtOAc), the reaction solution was poured into 200 mL of ice water to quench the reaction. It was then extracted with ethyl acetate three times. The combined organic phases were washed with brine, dried over anhydrous sodium sulfate, filtered, and concentrated to dryness. The resulting crude product was purified by FCC ($SiO_2$, EtOAc/PE = 30 - 100%) to give 1-(2-methoxyethyl) azepan-2-one (9.0 g, yield 59%) as a yellow solid. $^1H$ NMR (400 MHz, DMSO-$d_6$) δ 3.47 - 3.33 (m, 6H), 3.23 (s, 3H), 2.44 - 2.35 (m, 2H), 1.68 - 1.45 (m, 6H).

**Step B:** methyl 1-(2-methoxyethyl)-2-oxoazepane-3-carboxylate

Tetrahydrofuran (70 mL) was added to 1-(2-methoxyethyl)azepan-2-one (7.0 g, 41 mmol) under $N_2$. LDA (30.5 mL, 61 mmol, 2.0 mol/L in tetrahydrofuran solution) was then slowly added dropwise at -78°C. The resulting reaction solution was stirred at -78 °C for 0.5 h followed by addition of dimethyl carbonate (5.5 g, 61 mmol), and the resulting mixture was stirred at room temperature for 5 hours. After the reaction was completed (monitored by TLC, 100% EtOAc), the reaction was quenched by pouring into 50 mL of ice-cold water. The mixture was extracted with ethyl acetate three times. The combined organic phases were washed with brine, dried over anhydrous sodium sulfate, filtered, and concentrated to dryness. The resulting crude product was purified by FCC ($SiO_2$, EtOAc/PE = 10 - 40%) to methyl 1-(2-methoxyethyl)-2-oxoazepane-3-carboxylate (2.8 g, yield 30%) as a yellow oil. $^1H$ NMR (400 MHz, DMSO-$d_6$) δ 3.85 - 3.77 (m, 1H), 3.60 (s, 3H), 3.58 - 3.45 (m, 2H), 3.42 - 3.34 (m, 3H), 3.30 - 3.24 (m, 1H), 3.24 (s, 3H), 1.95 - 1.76 (m, 2H), 1.73 - 1.64 (m, 1H), 1.62 - 1.44 (m, 2H), 1.42 - 1.29 (m, 1H).

**Step C:** Methyl 1-(2-methoxyethyl)-3-methyl-2-oxoazepane-3-carboxylate

Tetrahydrofuran (5 mL) was added to methyl 1-(2-methoxyethyl)-2-oxoazepane-3-carboxylate (0.5 g, 2.2 mmol) under $N_2$. LDA (2.2 mL, 4.4 mmol, 2.0 mol/L in tetrahydrofuran solution) was then slowly added dropwise at 0 °C. The resulting mixture was stirred at 0 °C for 0.5 h, then methyl iodide (625 mg, 4.4 mmol) was added. The resulting reaction solution was stirred at room temperature for 3 hours. After the reaction was completed (monitored by TLC, EtOAc/PE = 1/1), the reaction was quenched by pouring into 30 mL ice-cold water and the mixture was extracted with ethyl acetate three times. The combined organic phases were washed with brine, dried over anhydrous sodium sulfate, filtered, and concentrated to dryness. The resulting crude product was purified by FCC ($SiO_2$, EtOAc/PE = 20-50%) to give methyl 1-(2-methoxyethyl)-3-methyl-2-oxoazepane-3-carboxylate (150 mg, yield 28%) as a yellow oil. LCMS (m/z): 244.1 (M + H).

**Step D:** (1-(2-methoxyethyl)-3-methylazepan-3-yl)methanol

Tetrahydrofuran (2 mL) was added to methyl 1-(2-methoxyethyl)-3-methyl-2-oxoazepane-3-carboxylate (150 mg, 0.62 mmol) under $N_2$. lithium aluminum hydride (3.0 mL, 3.08 mmol, 1.0 mol/L in tetrahydrofuran solution) was then slowly added dropwise at 0 °C. The resulting mixture was stirred at 70 °C for 12 hours. After the reaction was completed (monitored by LCMS), 1 mL water and 30 mL ethyl acetate were successively added to the reaction solution to quench the reaction. The resulting mixture was filtered through celite, then the filtrate was concentrated and dried to give (1-(2-methoxyethyl)-3-methylazepan-3-yl)methanol (120 mg, yield 96%) as a colorless oil. LCMS (m/z): 202.2 (M + H).

**Example 1**

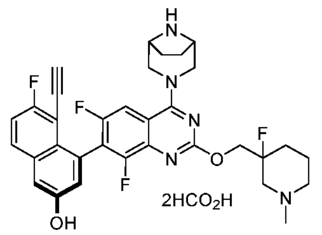

(*Ra*)-4-(4-((1*R*,5*S*)-3,8-diazabicyclo[3.2.1]octan-3-yl)-6,8-difluoro-2-(3-fluoro-1-methylpiperidin-3-yl)methoxy)quinazolin-7-yl)-5-ethynyl-6-fluoronaphthalen-2-ol•diformate

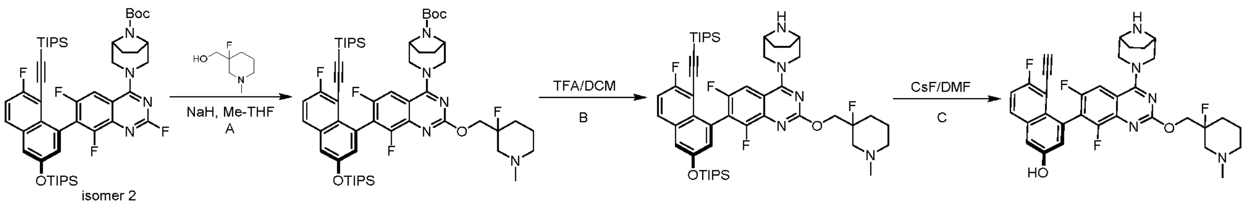


isomer 2
**A-1b-I2**

**Step A:** *tert*-butyl (*Ra*)-(1*R*,5*S*)-3-(6,8-difluoro-2-((3-fluoro-1-methylpiperidin-3-yl)methoxy)-7-(7-fluoro-8-(triisopropylsilyl))ethynyl)-3-((triisopropylsilyl)oxy)naphthalen-1-yl)quinazolin-4-yl)-3,8-diazabicyclo[3.2.1]octane-8-carboxylate

(3-Fluoro-1-methylpiperidin-3-yl)methanol (50 mg, 0.34 mmol) was dissolved into 3 mL anhydrous 2-methyltetrahydrofuran in an ice bath, then sodium hydride (13 mg, 0.34 mmol) was slowly added to the reaction. After stirring at the same temperature for half an hour, *tert*-butyl (*Ra*)-(1*R*,5*S*)-3-(2,6,8-trifluoro-7-(7-fluoro-8-(triisopropylsilyl)ethynyl)-3-((triisopropylsilyl)oxy)naphthalen-1-yl)quinazolin-4-yl)-3,8-diazabicyclo[3.2.1]octane-8-carboxylate (150 mg, 0.17 mmol) was added to the reaction. The resulting mixture was stirred at room temperature for 3 hours. After the reaction was completed (monitored by LCMS), the reaction was quenched by pouring into 30 mL of ice-cold water, and the mixture was extracted with ethyl acetate three times. The combined organic phase was washed with brine, dried over anhydrous sodium sulfate, filtered, and concentrated to dryness to obtain crude *tert*-butyl (*Ra*)-(1*R*,5*S*)-3-(6,8-difluoro-2-((3-fluoro-1-methylpiperidin-3-yl)methoxy)-7-(7-fluoro-8-(triisopropylsilyl))ethynyl)-3-((triisopropylsilyl)oxy)naphthalen-1-yl)quinazolin-4-yl)-3,8-diazabicyclo[3.2.1]octane-8-carboxylate (200 mg) as a yellow solid. LCMS (m/z): 1018.5 (M + H).

**Step B:** (*Ra*)-4-((1*R*,5*S*)-3,8-diazabicyclo[3.2.1]octane-3-yl)-6,8-difluoro-2-((3-fluoro-1-methylpiperidin-3-yl)methoxy)-7-(7-fluoro-8-(triisopropylsilyl)ethynyl)-3-((triisopropylsilyl)oxy)naphthalen-1-yl)quinazoline

At room temperature, dichloromethane (2 mL) and trifluoroacetic acid (1 mL) were added to *tert*-butyl (*Ra*)-(1*R*,5*S*)-3-(6,8-difluoro-2-((3-fluoro-1-methylpiperidin-3-yl)methoxy)-7-(7-fluoro-8-(triisopropylsilyl))ethynyl)-3-((triisopropylsilyl)oxy)naphthalene-1-yl)quinazolin-4-yl)-3,8-diazabicyclo[3.2.1]octane-8-carboxylate (200 mg). The resulting mixture was stirred at room temperature for 2 hours. After the reaction was completed (monitored by LCMS), 30 mL of saturated aqueous sodium bicarbonate solution was added, and the mixture was extracted with ethyl acetate three times. The combined organic phase was washed with brine, dried over anhydrous sodium sulfate, filtered, and concentrated to dryness to obtain crude product (*Ra*)-4-(((1*R*,5*S*)-3,8-diazabicyclo[3.2.1]octane-3-yl)-6,8-difluoro-2-((3-fluoro-1-methylpiperidin-3-yl)methoxy)-7-(7-fluoro-8-(triisopropylsilyl)ethynyl)-3-((triisopropylsilyl)oxy) naphthalen-1-yl)quinazoline (190 mg) as a yellow solid. LCMS (m/z): 918.5 (M + H).

**Step C:** (*Ra*)-4-(4-((1*R*,5*S*)-3,8-diazabicyclo[3.2.1]octan-3-yl)-6,8-difluoro-2-(3-fluoro-1-methylpiperidin-3-yl)methoxy) quinazolin-7-yl)-5-ethynyl-6-fluoronaphthalen-2-ol•diformate

At room temperature, DMF (2 mL) and cesium fluoride (314 mg, 2.0 mmol) were added in sequence to (*Ra*)-4-((1*R*,5*S*)-3,8-diazabicyclo[3.2.1]octane-3-yl)-6,8-difluoro-2-((3-fluoro-1-methylpiperidin-3-yl)methoxy)-7-(7-fluoro-8-(triisopropylsilyl)ethynyl)-3-((triisopropylsilyl)oxy)naphthalen-1-yl)quinazoline (190 mg). The reaction was stirred at 50 °C for 12 hours. After the reaction was completed (monitored by LCMS), the reaction was purified by Pre-HPLC (C18, CAN/(0.1% FA/$H_2O$) = 20-40%) to afford (*Ra*)-4-(4-(((1*R*,5*S*)-3,8-diazabicyclo[3.2.1]octan-3-yl)-6,8-difluoro-2-(3-fluoro-1-methylpiperidin-3-yl)methoxy)quinazolin-7-yl)-5-ethynyl-6-fluoronaphthalen-2-ol•diformate (67.2 mg) as a yellow solid. $^1$H NMR (400 MHz, DMSO-$d_6$) δ 8.27 (s, 2H), 7.99 (dd, *J* = 9.2, 5.9 Hz, 1H), 7.59 (d, *J* = 9.8 Hz, 1H), 7.52 - 7.45 (m, 1H), 7.42 (d, *J* = 2.5 Hz, 1H), 7.16 (d, *J* = 2.5 Hz, 1H), 4.62 - 4.18 (m, 6H), 3.99 - 3.92 (m, 4H), 2.64 - 2.52 (m, 1H), 2.49 - 2.41 (m, 1H), 2.39 - 2.30 (m, 1H), 2.19 (s, 3H), 1.98 - 1.62 (m, 8H), 1.59 - 1.46 (m, 1H). $^{19}$F NMR (376 MHz, DMSO-$d_6$) δ -73.19 --73.84,-73.52,-110.20,-117.90 --118.50,-124.35. LCMS (m/z): 606.3 (M + H).

Example 2

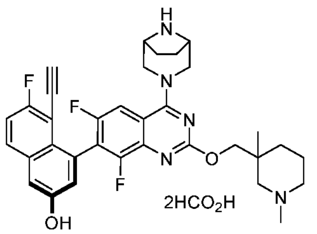

(*Ra*)-4-(4-((1*R*,5*S*)-3,8-diazabicyclo[3.2.1]octan-3-yl)-2-((1,3-dimethylpiperidin-3-yl)methoxy)-6,8-difluoroquinazolin-7-yl)-5-ethynyl-6-fluoronaphthalen-2-oledicarboxylate

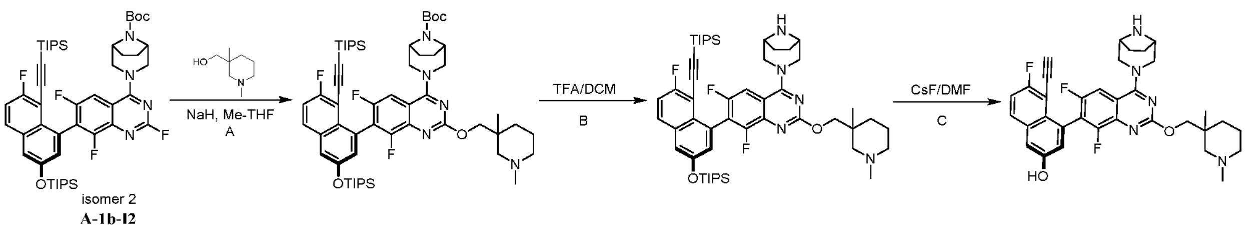

**Step A:** *tert*-butyl (*Ra*)-(1*R*,5*S*)-3-(2-((1,3-dimethylpiperidin-3-yl)methoxy)-6,8-difluoro-7-(7-fluoro-8-(triisopropylsilyl)ethynyl)-3-((triisopropylsilyl)oxy)naphthalen-1-yl)quinazolin-4-yl)-3,8-diazabicyclo[3.2.1]octane-8-carboxylate

(3-fluoro-1-methylpiperidin-3-yl) methanol (50 mg, 0.34 mmol) was dissolved into 3 mL anhydrous tetrahydrofuran in an ice bath, then sodium hydride (14 mg, 0.34 mmol) was slowly added to the reaction. After stirring at the same temperature for half an hour, *tert*-butyl (*Ra*)-(1*R*,5*S*)-3-(2,6,8-trifluoro-7-(7-fluoro-8-(triisopropylsilyl)ethynyl)-3-((triisopropylsilyl)oxy)naphthalen-1-yl)quinazolin-4-yl)-3,8-diazabicyclo[3.2.1]octane-8-carboxylate (150 mg, 0.17 mmol) was added to the reaction. The resulting mixture was stirred at room temperature for 2 hours. After the reaction was completed (monitored by LCMS), the reaction was quenched by pouring into 30 mL of ice-cold water. The mixture was extracted with ethyl acetate three times. The combined organic phase was washed with brine, dried over anhydrous sodium sulfate, filtered, and concentrated to dryness to obtain crude product *tert*-butyl (*Ra*)-(1*R*,5*S*)-3-(2-((1,3-dimethylpiperidin-3-yl)methoxy)-6,8-difluoro-7-(7-fluoro-8-(triisopropylsilyl)ethynyl)-3-((triisopropylsilyl)oxy)naphthalen-1-yl)quinazolin-4-yl)-3,8-diazabicyclo[3.2.1]octane-8-carboxylate (130 mg) as a yellow solid. LCMS (m/z): 1014.5 (M + H).

**Step B:** (*Ra*)-4-((1*R*,5*S*)-3,8-diazabicyclo[3.2.1]octane-3-yl)-2-(1,3-dimethylpiperidin-3-yl)methoxy)-6,8-difluoro-7-(7-fluoro-8-(triisopropylsilyl)ethynyl)-3-((triisopropylsilyl)oxy)naphthalen-1-yl)quinazoline

At room temperature, dichloromethane (1 mL) and trifluoroacetic acid (1 mL) were added in sequence to *tert*-butyl (*Ra*)-(1*R*,5*S*)-3-(2-((1,3-dimethylpiperidin-3-yl)methoxy)-6,8-difluoro-7-(7-fluoro-8-(triisopropylsilyl)ethynyl)-3-((triisopropylsilyl)oxy)naphthalen-1-yl)quinazolin-4-yl)-3,8-diazabicyclo[3.2.1]octane-8-carboxylate (130 mg). The reaction was stirred at room temperature for 1 hour. After the reaction was completed (monitored by LCMS), 20 mL of saturated aqueous sodium bicarbonate solution was added, and the mixture was extracted with ethyl acetate three times. The combined organic phase was washed with brine, dried over anhydrous sodium sulfate, filtered, and concentrated to obtain the crude product (*Ra*)-4-(((1*R*,5*S*)-3,8-diazabicyclo[3.2.1]octane-3-yl)-2-((1,3-dimethylpiperidin-3-yl)methoxy)-6,8-difluoro-7-(7-fluoro-8-(triisopropylsilyl)ethynyl)-3-((triisopropylsilyl)oxy)naphthalen-1-yl)quinazoline (120 mg) as a yellow solid. LCMS (m/z): 914.5 (M + H).

**Step C:** (*Ra*)-4-(4-((1*R*,5*S*)-3,8-diazabicyclo[3.2.1]octane-3-yl)-2-(1,3-dimethylpiperidin-3-yl)methoxy)-6,8-difluoroquinazolin-7-yl)-5-ethynyl-6-fluoronaphthalen-2-ol

At room temperature, DMF (2 mL) and cesium fluoride (199 mg, 1.3 mmol) were added in sequence to (*Ra*)-4-(((1*R*,5*S*)-3,8-diazabicyclo[3.2.1]octane-3-yl)-2-((1,3-dimethylpiperidin-3-yl)methoxy)-6,8-difluoro-7-(7-fluoro-8-(triisopropylsilyl)ethynyl)-3-((triisopropylsilyl)oxy)naphthalen-1-yl)quinazoline (120 mg). The resulting mixture was stirred at 50 °C for 2 hours. After the reaction was completed (monitored by LCMS), the reaction was purified by Pre-HPLC (C18, CAN/(0.1% FA/$H_2O$) = 15-45%) to afford (*Ra*)-4-(4-(((1*R*,5*S*)-3,8-diazabicyclo[3.2.1]octane-3-yl)-2-(1,3-dimethylpiperidin-3-yl)methoxy)-6,8-difluoroquinazolin-7-yl)-5-ethynyl-6-fluoronaphthalen-2-ol•diformate (30 mg) as a yellow solid. $^1$H NMR (400 MHz, DMSO-$d_6$) δ 8.28 (s, 2H), 7.98 (dd, 1H), 7.55 (d, *J* = 10.4 Hz, 1H), 7.51 - 7.44 (m, 1H), 7.41 (d, *J* = 2.6 Hz, 1H), 7.15 (d, *J* = 2.5 Hz, 1H), 4.32 - 4.23 (m, 4H), 4.14 - 4.10 (m, 1H), 3.99 - 3.97 (m, 1H), 3.74 - 3.68 (m, 2H), 3.59 - 3.49 (m, 2H), 2.40 - 1.96 (m, 7H), 1.91 - 1.41 (m, 8H), 1.02 (s, 3H). $^{19}$F NMR (376 MHz, DMSO-$d_6$) δ-73.45,-110.26,-110.27,-110.29,-118.68 --118.98,-124.40 --124.70. LCMS (m/z): 602.3 (M + H).

**Examples 2-1 and 2-2**

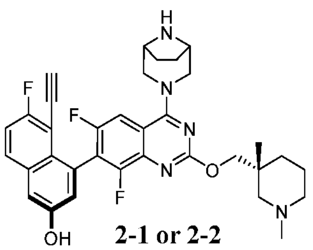

2-1 or 2-2

and

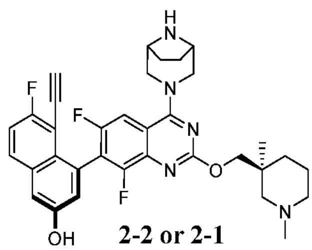

2-2 or 2-1

Resolution of (*Ra*)-4-(4-((1*R*,5*S*)-3,8-diazabicyclo[3.2.1]octane-3-yl)-2-((1,3-dimethylpiperidin-3-yl)methoxy)-6,8-difluoroquinazolin-7-yl)-5-ethynyl-6-fluoronaphthalen-2-ol (Example 2, 50 mg) was carried out by chiral HPLC (BRIX-2860; separation column: DAICEL CHIRALPAK ® IG, 250*25 mm, 10μm; Mobile phase: n-hexane/(EtOH/DCM = 7/3) = 75/25; flow rate: 40 mL/min) . The first-eluted isomer 1 was obtained as example 2-1 (20 mg, with a relatively shorter

retention time). Chiral analytical method-2, Rt = 2.319 min. LCMS (m/z): 602.3 (M + H). The subsequently eluted isomer 2 was obtained as example 2-2 (15 mg, with a relatively longer retention time). Chiral analytical method-2, Rt = 4.176 min. LCMS (m/z): 602.3 (M + H).

**Example 3**

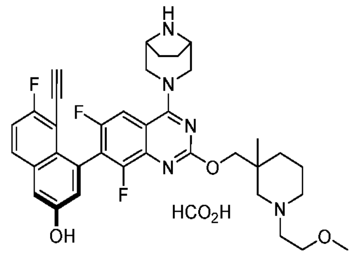

(*Ra*)-4-(4-((1*R*,5*S*)-3,8-diazabicyclo[3.2.1]octan-3-yl)-6,8-difluoro-2-(1-(2-methoxyethyl)-3-methylpiperidin-3-yl)methoxy)quinazolin-7-yl)-5-ethynyl-6-fluoronaphthalen-2-ol•formate

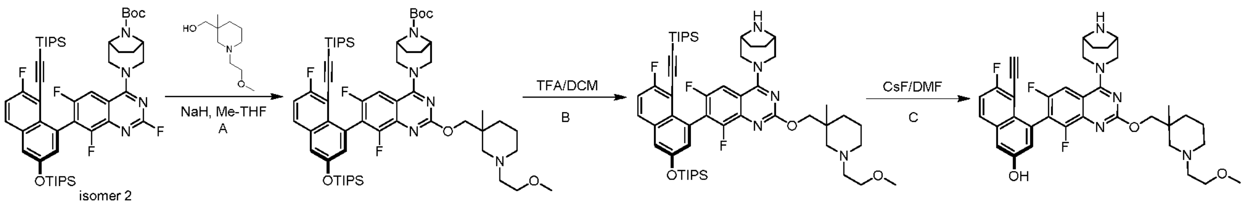


isomer 2
A-1b-I2

**Step A:** *tert*-butyl (*Ra*)-(1*R*,5*S*)-3-(6,8-difluoro-7-(7-fluoro-8-(triisopropylsilyl)ethynyl)-3-((triisopropylsilyl)oxy)naphthalen-1-yl)-2-((1-(2-methoxyethyl)-3-methylpiperidin-3-yl)methoxy)quinazolin-4-yl)-3,8-diazabicyclo[3.2.1]octane-8-carboxylate

(1-(2-methoxyethyl)-3-methylpiperidin-3-yl) methanol (47 mg, 0.25 mmol) was dissolved into 3 mL of anhydrous tetrahydrofuran in an ice bath, then sodium hydride (13 mg, 0.34 mmol) was slowly added, and the reaction was stirred at the same temperature for 0.5 h., *tert*-butyl (*Ra*)-(1*R*,5*S*)-3-(2,6,8-trifluoro-7-(7-fluoro-8-(triisopropylsilyl)ethynyl)-3-((triisopropylsilyl)oxy)naphthalen-1-yl)quinazolin-4-yl)-3,8-diazabicyclo[3.2.1]octane-8-carboxylate (150mg, 0.17 mmol) was added, and the reaction was allowed to stirred for 2 hours at room temperature. After the reaction was completed (monitored by LCMS), the reaction was quenched by pouring into 30 mL of ice-cold water. The resulting mixture was extracted three times with ethyl acetate. The organic phase was combined, washed with brine, dried over anhydrous sodium sulfate, filtered, and concentrated to dryness to obtain the crude yellow solid product *tert*-butyl (*Ra*)-(1*R*,5*S*)-3-(6,8-difluoro-7-(7-fluoro-8-(triisopropylsilyl)ethynyl)-3-((triisopropylsilyl)oxy)naphthalen-1-yl)-2-((1-(2-methoxyethyl)-3-methylpiperidin-3-yl)methoxy)quinazolin-4-yl)-3,8-diazabicyclo[3.2.1]octane-8-carboxylate (100 mg). LCMS (m/z): 1058 .5 (M + H).

**Step B:** (*Ra*)-4-((1*R*,5*S*)-3,8-diazabicyclo[3.2.1]octan-3-yl)-6,8-difluoro-7-(7-fluoro-8-(triisopropylsilyl)ethynyl)-3-((triisopropylsilyl)oxy)naphthalen-1-yl)-2-((1-(2-methoxyethyl)-3-methylpiperidin-3-yl)methoxy)quinazoline

At room temperature, dichloromethane (1 mL) and trifluoroacetic acid (1 mL) were added in sequence to *tert*-butyl (*Ra*)-(1*R*,5*S*)-3-(6,8-difluoro-7-(7-fluoro-8-((triisopropylsilyl)ethynyl)-3-((triisopropylsilyl)oxy)naphthalen-1-yl)-2-((1-(2-methoxyethyl)-3-methylpiperidin-3-yl)methoxy)quinazolin-4-yl)-3,8-diazabicyclo[3.2.1]octane-8-carboxylate(100 mg). The resulting mixture was stirred at room temperature for 1 hour. After the reaction was completed (monitored by LCMS), saturated aqueous sodium bicarbonate 30 mL was added to the reaction. The resulting mixture was extracted three times with ethyl acetate. The combined organic phase was washed with brine, dried over anhydrous sodium sulfate, filtered, concentrated to afford product (*Ra*)-4-((1*R*,5*S*)-3,8-diazabicyclo[3.2.1]octan-3-yl)-6,8-difluoro-7-(7-fluoro-8-((triisopropylsilyl)ethynyl)-3-(triisopropylsilyl)oxy)naphthalen-1-yl)-2-((1-(2-methoxyethyl)-3-methylpiperidin-3-yl)methoxy)quinazoline (110 mg) as a crude yellow solid. LCMS (m/z): 958.5 (M + H).

**Step C:** (*Ra*)-4-(4-((1*R*,5*S*)-3,8-diazabicyclo[3.2.1]octane-3-yl)-6,8-difluoro-2-(1-(2-methoxyethyl)-3-methylpiperidin-3-yl)methoxy)quinazolin-7-yl)-5-ethynyl-6-fluoronaphthalen-2-ol•formate

At room temperature, DMF (2 mL) and cesium fluoride (174 mg, 1.1 mmol) were added in sequence to (*Ra*)-4-((1*R*,5*S*)-3,8-diazabicyclo[3.2.1]octan-3-yl)-6,8-difluoro-7-(7-fluoro-8-(triisopropylsilyl)ethynyl)-3-((triisopropylsilyl)oxy)naphthalen-1-yl)-2-((1-(2-methoxyethyl)-3-methylpiperidin-3-yl)methoxy)quinazoline (110 mg). The resulting mixture was stirred at 50 °C for 2 hours. After the reaction was completed (monitored by LCMS), the reaction was purified by Pre-HPLC (C18,CAN/(0.1% FA/$H_2O$) = 15-40%) to afford (*Ra*)-4-(4-((1*R*,5*S*)-3,8-diazabicyclo[3.2.1]octane-3-yl)-6,8-difluoro-2-(1-(2-methoxyethyl)-3-methylpiperidin-3-yl)methoxy)quinazolin-7-yl)-5-ethynyl-6-fluoronaphthalen-2-ol•formate (20 mg) as a yellow solid. $^1$H NMR (400 MHz, DMSO-$d_6$) δ 8.27 (s, 1H), 7.99 (dd, *J* = 9.2, 6.0 Hz, 1H), 7.56 (d, *J* = 10.0 Hz, 1H), 7.51 - 7.44 (m, 1H), 7.42 (d, *J* = 2.5 Hz, 1H), 7.15 (d, *J* = 2.4 Hz, 1H), 4.30 - 4.25 (m, 3H), 4.17 - 4.13 (m, 2H), 4.00 - 3.96 (m, 2H), 3.89 - 3.82 (m, 2H), 3.63 - 3.52 (m, 2H), 3.39 (t, *J* = 5.9 Hz, 2H), 3.22 - 3.15 (m, 3H), 2.44 - 2.38 (m, 3H), 2.30 - 2.08 (m, 2H), 1.95 - 1.71 (m, 4H), 1.60 - 1.44 (m, 3H), 1.26 - 1.11 (m, 1H), 1.01 (s, 3H). $^{19}$F NMR (376 MHz, DMSO-$d_6$) δ-73.50,-110.24,-118.55,-124.32 --124.47. LCMS (m/z): 646.3 (M + H).

**Example 4**

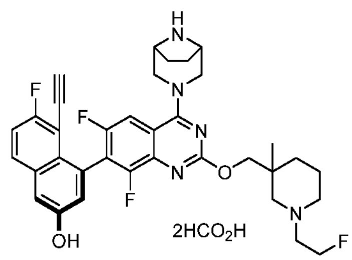

(*Ra*)-4-(4-((1*R*,5*S*)-3,8-diazabicyclo[3.2.1]octan-3-yl)-6,8-difluoro-2-(1-(2-fluoroethyl)-3-methylpiperidin-3-yl)methoxy) quinazolin-7-yl)-5-ethynyl-6-fluoronaphthalen-2-ol•diformate

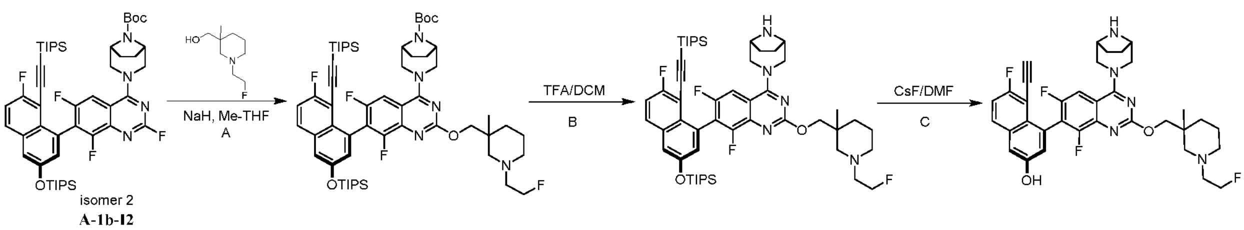


**Step A:** *tert*-butyl (*Ra*)-(1*R*,5*S*)-3-(6,8-difluoro-7-(7-fluoro-8-(triisopropylsilyl)ethynyl)-3-((triisopropylsilyl)oxy)naphthalen-1-yl)-2-(1-(2-fluoroethyl)-3-methylpiperidin-3-yl)methoxy)quinazolin-4-yl)-3,8-diazabicyclo[3.2.1]octane-8-carboxylate

(1-(2-Fluoroethyl)-3-methylpiperidin-3-yl) methanol (59 mg, 0.34 mmol) was dissolved into 3 mL of anhydrous tetrahydrofuran in an ice bath, then sodium hydride (13 mg, 0.34 mmol) was slowly added to the reaction. After stirring the reaction mixture at the same temperature for 0.5 h. *tert*-butyl (*Ra*)-(1*R*,5*S*)-3-(2,6,8-trifluoro-7-(7-fluoro-8-(triisopropylsilyl)ethynyl)-3-((triisopropylsilyl)oxy)naphthalen-1-yl)quinazolin-4-yl)-3,8-diazabicyclo[3.2.1]octane-8-carboxylate (150mg, 0.17 mmol) was added to the reaction. The resulting mixture was stirred for 2 hours at room temperature. After the reaction was completed (monitored by LCMS), the reaction solution was poured into 30 mL of ice-water to quench the reaction. The resulting mixture was extracted three times with ethyl acetate. The combined organic phase was washed with brine, dried over anhydrous sodium sulfate, filtered, and concentrated to dryness to obtain *tert*-butyl (*Ra*)-(1*R*,5*S*)-3-(6,8-difluoro-7-(7-fluoro-8-(triisopropylsilyl)ethynyl)-3-((triisopropylsilyl)oxy)naphthalen-1-yl)-2-((1-(2-fluoroethyl)-3-methylpiperidin-3-yl)methoxy)quinazolin-4-yl)-3,8-diazabicyclo[3.2.1]octane-8-carboxylate (110 mg) as a

yellow solid crude product. LCMS (m/z): 1046.7 (M + H).

**Step B:** (*Ra*)-4-((1*R*,5*S*)-3,8-diazabicyclo[3.2.1]octan-3-yl)-6,8-difluoro-7-(7-fluoro-8-(triisopropylsilyl)ethynyl)-3-((triisopropylsilyl)oxy)naphthalen-1-yl)-2-((1-(2-fluoroethyl)-3-methylpiperidin-3-yl)methoxy)quinazoline

At room temperature, dichloromethane (1 mL) and trifluoroacetic acid (1 mL) were added in sequence to *tert*-butyl (*Ra*)-(1*R*,5*S*)-3-(6,8-difluoro-7-(7-fluoro-8-(triisopropylsilyl)ethynyl)-3-((triisopropylsilyl)oxy)naphthalen-1-yl)-2-(1-(2-fluoroethyl)-3-methylpiperidin-3-yl)methoxy)quinazolin-4-yl)-3,8-diazabicyclo[3.2.1]octane-8-carboxylate (110 mg). The resulting mixture was stirred at room temperature for 1 hour. After the reaction was completed (monitored by LCMS), saturated aqueous sodium bicarbonate 30 mL was added to the reaction, and the mixture was extracted three times with ethyl acetate. The combined organic phase was washed with brine, dried over anhydrous sodium sulfate, filtered, and concentrated afford crude yellow solid (*Ra*)-4-((1*R*,5*S*)-3,8-diazabicyclo[3.2.1]octan-3-yl)-6,8-difluoro-7-(7-fluoro-8-(triisopropylsilyl)ethynyl)-3-((triisopropylsilyl)oxy)naphthalen-1-yl)-2-((1-(2-fluoroethyl)-3-methylpiperidin-3-yl)methoxy)quinazoline (100 mg). LCMS (m/z): 946.5 (M + H).

**Step C:** (*Ra*)-4-(4-((1*R*,5*S*)-3,8-diazabicyclo[3.2.1]octane-3-yl)-6,8-difluoro-2-(1-(2-fluoroethyl)-3-methylpiperidin-3-yl)methoxy)quinazolin-7-yl)-5-ethynyl-6-fluoronaphthalen-2-ol•diformate

At room temperature, DMF (2 mL) and cesium fluoride (152 mg, 1.0mmol) were added in sequence to (*Ra*)-4-((1*R*,5*S*)-3,8-diazabicyclo[3.2.1]octan-3-yl)-6,8-difluoro-7-(7-fluoro-8-(triisopropylsilyl)ethynyl)-3-((triisopropylsilyl)oxy)naphthalen-1-yl)-2-((1-(2-fluoroethyl)-3-methylpiperidin-3-yl)methoxy)quinazoline (100 mg). The resulting mixture was stirred at 50 °C for 2 hours. After the reaction was completed (monitored by LCMS), the reaction was purified by Pre-HPLC (C18, CAN/(0.1% FA/$H_2O$) = 15-45%) to afford (*Ra*)-4-(4-((1*R*,5*S*)-3,8-diazabicyclo[3.2.1]octane-3-yl)-6,8-difluoro-2-(1-(2-fluoroethyl)-3-methylpiperidin-3-yl)methoxy)quinazolin-7-yl)-5-ethynyl-6-fluoronaphthalen-2-ol•diformate (8 mg) as a yellow solid. $^1$H NMR (400 MHz, DMSO-$d_6$) δ 8.28 (s, 2H), 7.98 (dd, *J* = 9.2, 6.0 Hz, 1H), 7.59 - 7.52 (m, 1H), 7.52 - 7.44 (m, 1H), 7.41 (d, *J* = 2.5 Hz, 1H), 7.15 (d, *J* = 2.5 Hz, 1H), 4.55 (t, *J* = 5.0 Hz, 1H), 4.43 (t, *J* = 4.9 Hz, 1H), 4.34 - 4.20 (m, 4H), 4.17 - 4.11 (m, 1H), 4.00 - 3.96 (m, 1H), 3.79 - 3.70 (m, 2H), 3.61 - 3.50 (m, 2H), 2.62 - 2.52 (m, 1H), 2.45 - 2.28 (m, 3H), 2.24 - 2.13 (m, 1H), 1.88 - 1.69 (m, 4H), 1.59 - 1.44 (m, 3H), 1.30 - 1.18 (m, 1H), 1.03 (s, 3H). $^{19}$F NMR (376 MHz, DMSO-$d_6$) δ-73.47,-110.27,-118.75,-124.46. LCMS (m/z): 634.3 (M + H).

### Example 5

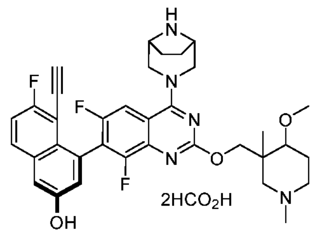

(*Ra*)-4-(4-(((1*R*,5*S*)-3,8-diazabicyclo[3.2.1]octan-3-yl)-6,8-difluoro-2-(4-methoxy-1,3-dimethylpiperidin-3-yl)methoxy) quinazolin-7-yl)-5-ethynyl-6-fluoronaphthalen-2-ol•diformate

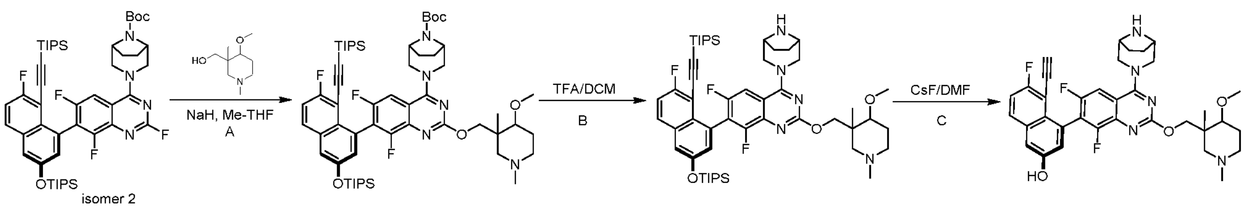


isomer 2
**A-1b-I2**

**Step A:** *tert*-butyl (*Ra*)-(1*R*,5*S*)-3-(6,8-difluoro-7-(7-fluoro-3-hydroxy-8-(triisopropylsilyl)ethynyl)naphthalen-1-yl)-2-((4-methoxy-1,3-dimethylpiperidin-3-yl)methoxy)quinazolin-4-yl)-3,8-diazabicyclo[3.2.1]octane-8-carboxylate

At room temperature, NaH (27 mg, 0.67 mmol, 60%) was added to a mixture of (4-methoxy-1,3-dimethylpiperidin-3-yl) methanol (58 mg, 0.34 mmol) in THF (3 mL). The mixture was stirred at room temperature for 0.5 h. Then, *tert*-butyl (*Ra*)-(1*R*,5*S*)-3-(2,6,8-trifluoro-7-(7-fluoro-8-(triisopropylsilyl)ethynyl)-3-(triisopropylsilyl)oxy)naphthalen-1-yl)quinazolin-4-yl)-3,8-diazobicyclo[3.2.1]octane-8-carboxylate (200 mg, 0.22 mmol) was added, and the reaction was stirred overnight at room temperature. After the reaction was completed (monitored by LCMS), the mixture was poured into $NH_4Cl$ (20 mL) and extracted with EA (30 mL × 3). The combined organic phase was washed with brine (20 mL), concentrated to obtain *tert*-butyl (*Ra*)-(1*R*,5*S*)-3-(6,8-difluoro-7-(7-fluoro-3-hydroxy-8-(triisopropylsilyl)ethynyl)naphthalen-1-yl)-2-((4-methoxy-1,3-dimethylpiperidin-3-yl)methoxy)quinazolin-4-yl)-3,8-diazabicyclo[3.2.1]octane-8-carboxylate (190 mg) as a yellow solid. LCMS (m/z): 888.5 (M + H).

**Step B:** (*Ra*)-4-(4-((1*R*,5*S*)-3,8-diazabicyclo[3.2.1]octane-3-yl)-6,8-difluoro-2-((4-methoxy-1,3-dimethylpiperidin-3-yl) methoxy)quinazolin-7-yl)-6-fluoro-5-((triisopropylsilyl)ethynyl)naphthalene-2-ol

At room temperature, $CF_3COOH$ (15 mL) was added to *tert*-butyl (*Ra*)-(1*R*,5*S*)-3-(6,8-difluoro-7-(7-fluoro-3-hydroxy-8-(triisopropylsilyl)ethynyl)naphthalen-1-yl)-2-((4-methoxy-1,3-dimethylpiperidin-3-yl)methoxy)quinazolin-4-yl)-3,8-diazabicyclo[3.2.1]octane-8-carboxylate (190mg). The reaction was stirred for 1 hour and then concentrated to remove the acid solution. The residue was diluted with EA (30 mL), saturated $NaHCO_3$ solution (20 mL) was added to adjust pH to about 8, and then water (30 mL) was added. The resulting mixture was extracted with EA (30 mL × 2). The organic phases were collected and washed with brine to afford (*Ra*)-4-(4-((1*R,5S*)-3,8-diazabicyclo[3.2.1]octane-3-yl)-6,8-difluoro-2-((4-methoxy-1,3-dimethylpiperidin-3-yl)methoxy)quinazolin-7-yl)-6-fluoro-5-((triisopropylsilyl)ethynyl) naphthalene-2-ol (160 mg) as a yellow solid.

**Step C:** (*Ra*)-4-(4-((1*R*,5*S*)-3,8-diazabicyclo[3.2.1]octane-3-yl)-6,8-difluoro-2-((4-methoxy-1,3-dimethylpiperidin-3-yl) methoxy)quinazolin-7-yl)-5-ethynyl-6-fluoronaphthalen-2-ol

(*Ra*)-4-(4-((1*R*,5*S*)-3,8-diazabicyclo[3.2.1]octane-3-yl)-6,8-difluoro-2-((4-methoxy-1,3-dimethylpiperidin-3-yl) methoxy)quinazolin-7-yl)-6-fluoro-5-((triisopropylsilyl)ethynyl)naphthalen-2-ol (160 m g) and CsF (308 mg, 2.03 mmol) were added in sequence to DMF (3 mL). The reaction was heated to 50°C and stirred for 1 h. After the reaction was completed (monitored by LCMS), the crude product was purified by Pre-HPLC to afford (*Ra*)-4-(4-(((1*R,5S*)-3,8-diazabicyclo[3.2.1]octane-3-yl)-6,8-difluoro-2-(4-methoxy-1,3-dimethylpiperidin-3-yl)methoxy)quinazolin-7-yl)-5-ethynyl-6-fluoronaphthalen-2-ol•diformate (70 mg) as a yellow solid. LCMS (m/z): 632.4 (M + H). $^1H$ NMR (400 MHz, DMSO-$d_6$) δ 8.26 (s, 2H), 7.99 (dd, *J* = 9.2, 5.9 Hz, 1H), 7.56 (dd, *J* = 10.3, 1.7 Hz, 1H), 7.53 - 7.44 (m, 1H), 7.42 (d, *J* = 2.6 Hz, 1H), 7.16 (d, *J* = 2.5 Hz, 1H), 4.47 - 4.08 (m, 4H), 3.99 (dd, *J* = 4.2*,* 1.1 Hz, 1H), 3.76 (s, 2H), 3.56 (dd, *J* = 28.4, 12.7 Hz, 2H), 3.33 - 3.18 (m, 3 H), 3.14 - 2.94 (m, 1H), 2.53 (s, 2 H), 2.25 - 2.02 (m, 4 H), 1.98 - 1.70 (m, 6H), 1.59 (s, 1H), 1.16 - 0.97 (m, 3H). $^{19}F$ NMR (376 MHz, DMSO-$d_6$) δ-110.25,-118.73,-124.44.

**Examples 5-1, 5-2, 5-3 and 5-4**

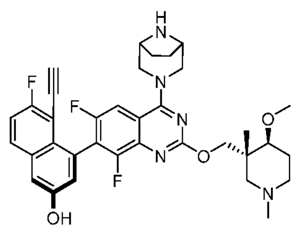
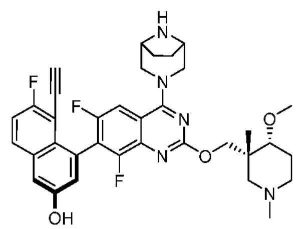
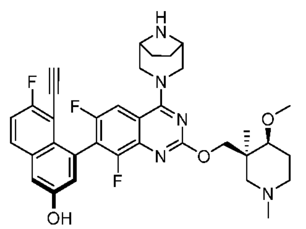
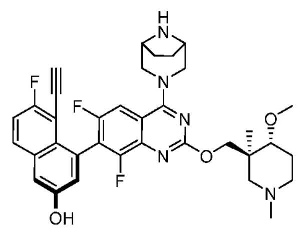

Examples 5-1, 5-2, 5-3 and 5-4 were obtained by chiral resolution from example 5.
The synthesis of example 5-4 and example 5-3 can also be achieved according to the following scheme.

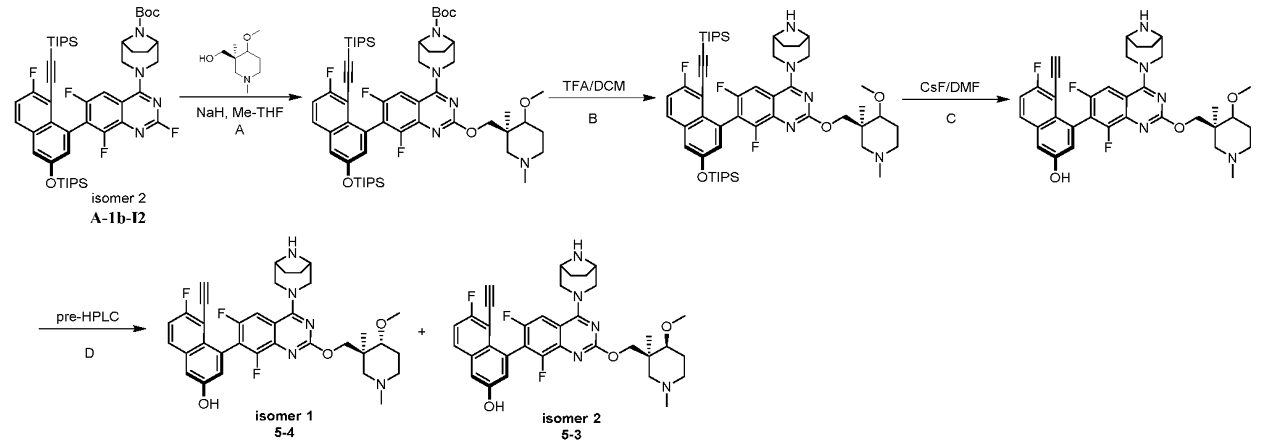

isomer 2
A-1b-I2

isomer 1
5-4

isomer 2
5-3

**Step A:** *tert*-butyl (*Ra*)-(1*R*,5*S*)-3-(6,8-difluoro-7-(7-fluoro-8-(triisopropylsilyl)ethynyl)-3-(triisopropylsilyl)oxy)naphthalen-1-yl)-2-(((3*S*)-4-methoxy-1,3-dimethylpiperidin-3-yl)methoxy)quinazolin-4-yl)-3,8-diazabicyclo[3.2.1]octane-8-carboxylate

At room temperature, NaH (27 mg, 673 μmol, 60%) was added to a mixture of (3*S*)-(4-methoxy-1,3-dimethylpiperidin-3-yl)methanol (intermediate **a5A-1,** 43 mg, 247 umol) in THF (3 mL), and the reaction was stirred at room temperature for 0.5 h, then *tert*-butyl (*Ra*)-(1*R*,5*S*)-3-(2,6,8-trifluoro-7-(7-fluoro-8-(triisopropylsilyl)ethynyl)-3-((triisopropylethylsilyl)oxy)naphthalen-1-yl)quinazolin-4-yl)-3,8-diazabicyclo[3.2.1]octane-8-carboxylate (intermediate **A-1b-I2,** 200 mg, 0.224 mmol) was added. The resulting mixture was stirred overnight at room temperature. After the reaction was completed (monitored by LCMS), the mixture was poured into saturated $NH_4Cl$ aqueous (30 mL) solution and extracted with EA (30 mL × 3). The combined organic phase was washed with brine (20 mL) and concentrated to afford *tert*-butyl (*Ra*)-(1*R*,5*S*)-3-(6,8-difluoro-7-(7-fluoro-8-(triisopropylsilyl)ethynyl)-3-(triisopropylsilyl)oxy)naphthalen-1-yl)-2-(((3*S*)-4-methoxy-1,3-dimethylpiperidin-3-yl)methoxy)quinazolin-4-yl)-3,8-diazabicyclo[3.2.1]octane-8-carboxylate (200 mg, yield 85%) as a yellow solid.

**Step B:** (*Ra*)-4-((1*R*,5*S*)-3,8-diazabicyclo[3.2.1]octan-3-yl)-6,8-difluoro-7-(7-fluoro-8-(triisopropylsilyl)ethynyl)-3-((triisopropylsilyl)oxy)naphthalen-1-yl)-2-(((3*S*)-4-methoxy-1,3-dimethylpiperidin-3-yl)methoxy)quinazoline

At room temperature, trifluoroacetic acid (10 mL) was added to *tert*-butyl (*Ra*)-(1*R*,5*S*)-3-(6,8-difluoro-7-(7-fluoro-8-(triisopropylsilyl)ethynyl)-3-(triisopropylsilyl)oxy)naphthalen-1-yl)-2-(((3*S*)-4-methoxy-1,3-dimethylpiperidin-3-yl)methoxy)quinazolin-4-yl)-3,8-diazabicyclo[3.2.1]octane-8-carboxylate (200 mg, 0.191 mmol). The resulting mixture was stirred at room temperature for 1 h. The acid was removed by concentration under reduced pressure. The residue was diluted with EA (30 mL), and the pH was adjusted to about 8 by adding saturated aqueous $NaHCO_3$ solution (20 mL). Then, water (30 mL) was added. The resulting mixture was extracted with EA (30 mL × 2). The combined organic phases were washed with saturated brine. After separation, the organic phase was concentrated to obtain (*Ra*)-4-((1*R*,5*S*)-3,8-diazabicyclo[3.2.1]octan-3-yl)-6,8-difluoro-7-(7-fluoro-8-(triisopropylsilyl)ethynyl)-3-((triisopropylsilyl)oxy)naphthalen-1-yl)-2-(((3*S*)-4-methoxy-1,3-dimethylpiperidin-3-yl)methoxy)quinazoline (170 mg, yield 94%) as a yellow solid.

**Step C-D:** Synthesis of compounds 5-4 and 5-3

(*Ra*)-4-((1*R*,5*S*)-3,8-diazabicyclo[3.2.1]octan-3-yl)-6,8-difluoro-7-(7-fluoro-8-(triisopropylsilyl)ethynyl)-3-((triisopropylsilyl)oxy)naphthalen-1-yl)-2-(((3*S*)-4-methoxy-1,3-dimethylpiperidin-3-yl)methoxy)quinazoline (170 mg, 0.180 mmol) and CsF (273 mg, 1.8 mmol) were added to DMF (3 mL). The resulting mixture was heated to 50°C and stirred for 1 h. After the reaction was completed (monitored by LCMS), insoluble solid was removed by filtration, and the filtrate was purified by Pre-HPLC (C18, CAN/(10 mmol $NH_4HCO_3/H_2O$) = 30-50%) to obtain the first-eluted isomer 1 (with a shorter retention time) as compound 5-4, LCMS (m/z): 632.4 (M + H). $^1H$ NMR (400 MHz, DMSO-*d*$_6$) δ 10.22 (brs, 1H), 7.99 (dd, *J* = 9.2, 6.0 Hz, 1H), 7.54 (dd, *J* = 10.4, 1.6 Hz, 1H), 7.51 - 7.45 (m, 1H), 7.41 (d, *J* = 2.6 Hz, 1H), 7.15 (d, *J* = 2.5 Hz, 1H), 4.36 - 4.13 (m, 4H), 3.98 (s, 1H), 3.60 - 3.41 (m, 4H), 3.27 - 3.14 (m, 4H), 2.75 - 2.58 (m, 1H), 2.21 - 2.03 (m, 4H), 2.00 - 1.48 (m, 8H), 1.02 (s, 3H). $^{19}F$ NMR (376 MHz, DMSO-*d*$_6$) δ-110.20,-119.21,-124.67; and the subsequently eluted isomer 2 (with a longer retention time) was compound 5-3, LCMS (m/z): 632.4 (M + H). $^1H$ NMR (400 MHz, DMSO-*d*$_6$) δ 10.21 (brs, 1H), 7.99 (dd, *J* = 9.2, 5.9 Hz, 1H), 7.53 (dd, *J* = 10.4, 1.6 Hz, 1H), 7.51 - 7.44 (m, 1H), 7.41 (d, *J* = 2.5 Hz, 1H), 7.15 (d, *J* = 2.5 Hz, 1H), 4.48 - 4.31 (m, 1H), 4.28 - 4.14 (m, 3H), 3.99 (s, 1H), 3.56 - 3.37 (m, 4H), 3.30 - 3.13 (m, 4H), 3.10 - 2.96 (m, 1H), 2.21 -

2.04 (m, 4H), 2.03 - 1.34 (m, 8H), 1.08 (s, 3H). $^{19}F$ NMR (376 MHz, DMSO-$d_6$) $\delta$-110.19,-119.34,-124.70.

Compounds 5-1 and 5-2 can be synthesized according to the above protocol.

**Example 6**

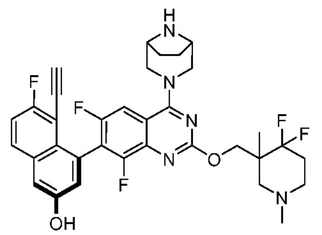

(*Ra*)-4-(4-((1*R*,5*S*)-3,8-diazabicyclo[3.2.1]octan-3-yl)-2-((4,4-difluoro-1,3-dimethylpiperidin-3-yl)methoxy)-6,8-difluoroquinazolin-7-yl)-5-ethynyl-6-fluoronaphthalen-2-ol

The synthesis of example 6 was carried out according to the protocol described in Example 1 using (4,4-difluoro-1,3-dimethylpiperidin-3-yl) methanol (Intermediate a6) instead of (3-fluoro-1-methylpiperidin-3-yl) methanol (Intermediate a1) in Step A. LCMS (m/z): 638.3 (M + H). $^1H$ NMR (400 MHz, DMSO-$d_6$) δ 7.99 (dd, *J* = 9.2, 5.9 Hz, 1H), 7.56 (dd, *J* = 10.4, 1.6 Hz, 1H), 7.52 - 7.45 (m, 1H), 7.41 (d, *J* = 2.6 Hz, 1H), 7.15 (d, *J* = 2.5 Hz, 1H), 4.55 - 4.36 (m, 2H), 4.32 - 4.17 (m, 2H), 3.98 (dd, *J* = 7.4, 1.1 Hz, 1H), 3.61 - 3.41 (m, 4H), 2.50 - 2.24 (m, 4H), 2.19 (d, *J* = 2.4 Hz, 3H), 2.13 - 1.97 (m, 2H), 1.85 - 1.57 (m, 4H), 1.18 (s, 3H). $^{19}F$ NMR (376 MHz, DMSO-$d_6$) δ-105.72,-106.37,-110.21,-119.02,-124.68.

**Examples 6-1 and 6-2**

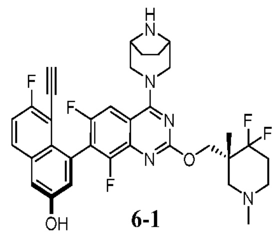

6-1 and

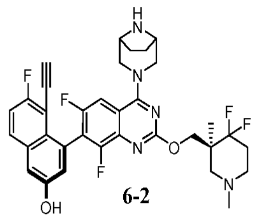

6-2

Examples 6-1 and 6-2 were obtained from Example 6 by chiral column preparation. (*Ra*)-4-(4-((1*R*,5*S*)-3,8-diazabicyclo[3.2.1]octan-3-yl)-2-((4,4-difluoro-1,3-dimethylpiperidin-3-yl)methoxy)-6,8-difluoroquinazolin-7-yl)-5-ethynyl-6-fluoronaphthalen-2-ol (Example 6, 50 mg) was separated by chiral Waters SFC150 (separation column: DAICEL CHIRALPAK® IG, 250*25 mm, 10 μm; mobile phase: $CO_2$/MeOH = 60/40; flow rate: 120 g/min). The first eluted isomer 1 was example 6-1 (20 mg, with a shorter retention time). Chiral analytical method SFC-4, Rt = 3.782 min. LCMS (m/z): 638.3 (M + H). The subsequently eluted isomer 2 was example 6-2 (20 mg, with a longer retention time). Chiral analytical method SFC-4, Rt = 5.653 min. LCMS (m/z): 638.3 (M + H).

The synthesis of Example 6-2 can also be carried out according to the protocol described in Example 1 using (*S*)-(4,4-difluoro-1,3-dimethylpiperidin-3-yl)methanol (Intermediate a6A-1) instead of (3-fluoro-1-methylpiperidin-3-yl) methanol (Intermediate a1) in Step A. LCMS (m/z): 638.3 (M + H). $^1H$ NMR (400 MHz, DMSO-$d_6$) δ 10.23 (brs, 1H), 7.99 (dd, *J* = 9.2, 6.0 Hz, 1H), 7.56 (dd, *J* = 10.4, 1.7 Hz, 1H), 7.52 - 7.45 (m, 1H), 7.41 (d, *J* = 2.5 Hz, 1H), 7.15 (d, *J* = 2.5 Hz, 1H), 4.49 - 4.34 (m, 2H), 4.31 - 4.17 (m, 2H), 4.00 (s, 1H), 3.60 - 3.39 (m, 4H), 2.69 - 2.54 (m, 1H), 2.47 - 2.24 (m, 3H), 2.19 (s, 3H), 2.13 - 1.98 (m, 2H), 1.84 - 1.59 (m, 4H), 1.18 (s, 3H). $^{19}F$ NMR (376 MHz, DMSO-$d_6$) δ-105.69,-106.37,-110.19,-119.04,-124.69.

**Example 7**

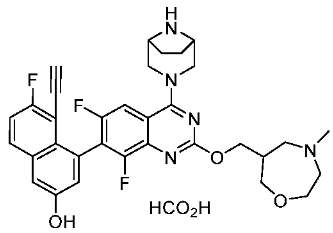

4-(4-((1*R*,5*S*)-3,8-diazabicyclo[3.2.1]octan-3-yl)-6,8-difluoro-2-((4-methyl-1,4-oxazolidin-6-yl)methoxy)quinazolin-7-yl)-5-ethynyl-6-fluoronaphthalen-2-ol-formate

The synthesis of Example 7 was carried out according to the protocol described in Example 1 using (4-methyl-1,4-oxazine-6-yl)methanol (Intermediate b1) instead of (3-fluoro-1-methylpiperidin-3-yl)methanol (intermediate a1), and intermediate A-1b-I instead of intermediate A-1b-I2 in Step A. $^1$H NMR (400 MHz, DMSO-$d_6$) δ 8.28 (s, 1H), 8.01 - 7.94 (m, 1H), 7.56 (d, *J*= 10.1 Hz, 1H), 7.51 - 7.44 (m, 1H), 7.41 (d, *J* = 2.4 Hz, 1H), 7.15 (d, *J* = 2.4 Hz, 1H), 4.34 - 4.26 (m, 2H), 4.20 (d, *J* = 6.3 Hz, 2H), 3.97 - 3.95 (m, 1H), 3.91 - 3.74 (m, 8H), 2.78 - 2.71 (m, 1H), 2.66 - 2.59 (m, 1H), 2.54 - 2.51 (m, 1H), 2.48 - 2.39 (m, 2H), 2.31 (s, 3H), 1.89 - 1.74 (m, 4H). LCMS (m/z): 604.0 (M + H).

## Example 8

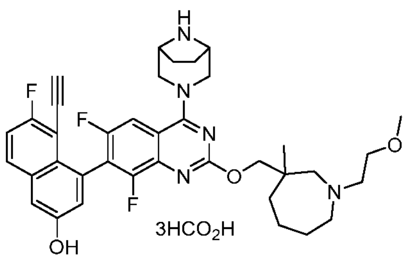

4-(4-((1*R*,5*S*)-3,8-diazabicyclo[3.2.1]octane-3-yl)-6,8-difluoro-2-((1-(2-methoxyethyl)-3-methylazepan-3-yl)methoxy) quinazolin-7-yl)-5-ethynyl-6-fluoronaphthalen-2-oletriformate

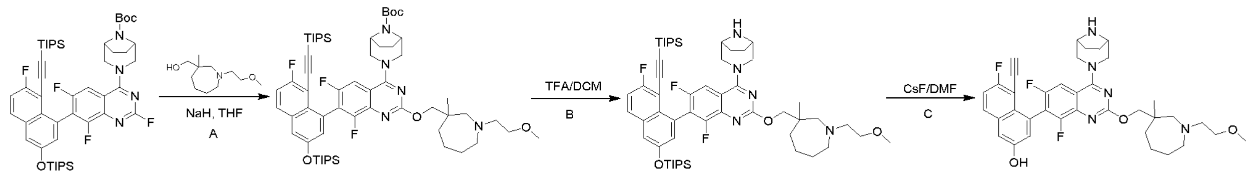


**Step A:** *tert-butyl* (1R,5S)-3-(6,8-difluoro-7-(7-fluoro-8-(triisopropylsilyl)ethynyl)-3-((triisopropylsilyl)oxy)naphthalen-1-yl)-2-((1-(2-methoxyethyl)-3-methylazepan-3-yl)methoxy)quinazolin-4-yl)-3,8-diazabicyclo[3.2.1]octane-8-carboxylate

(1-(2-methoxyethyl)-3-methylazetidin-3-yl)methanol (100 mg, 0.5 mmol) was dissolved into 3 mL of anhydrous tetrahydrofuran in an ice bath, then sodium hydride (25 mg, 0.67 mmol) was slowly added into the reaction. The reaction was stirred at same temperature for half an hour and tert-butyl (1*R*,5*S*)-3-(2,6,8-trifluoro-7-(7-fluoro-8-((triisopropylsilyl) ethynyl)-3-((triisopropylsilyl)oxy)naphthalen-1-yl)quinazolin-4-yl)-3,8-diazabicyclo[3.2.1]octane-8-carboxylate (A-1b-I) (150mg, 0.17 mmol) was added then. The resulting mixture was stirred at room temperature for 3 hours. After the reaction was completed (monitored by LCMS), the reaction was quenched by pouring into 30 mL ice-cold water. The resulting mixture was extracted three times with ethyl acetate. The combined organic phase was washed with brine, dried over anhydrous sodium sulfate, filtered, concentrated to dryness. The resulting crude product was purified by preparation silica gel plates (EtOAc/PE = 50%) to afford tert-butyl (1R,5S)-3-(6,8-difluoro-7-(7-fluoro-8-(triisopropylsilyl)ethynyl)-3-((triisopropylsilyl)oxy)naphthalen-1-yl)-2-((1-(2-methoxyethyl)-3-methylazepan-3-yl)methoxy)quinazolin-4-yl)-3,8-diazabicyclo[3.2.1]octane-8-carboxylate (130 mg, yield 72%) as a yellow solid. LCMS (m/z): 1072.5 (M + H).

**Step B:** 4-((1R,5S)-3,8-diazabicyclo[3.2.1]octan-3-yl)-6,8-difluoro-7-(7-fluoro-8-(triisopropylsilyl)ethynyl)-3-((triisopropylsilyl)oxy)naphthalen-yl)-2-((1-(2-methoxyethyl)-3-methylazepan-3-yl)methoxy)quinazoline

At room temperature, dichloromethane (1 mL) and trifluoroacetic acid (1 mL) were added in sequence to *tert-butyl* (1*R*,5*S*)-3-(6,8-difluoro-7-(7-fluoro-8-(triisopropylsilyl)ethynyl)-3-((triisopropylsilyl)oxy)naphthalen-1-yl)-2-((1-(2-methoxyethyl)-3-methylazepan-3-yl)methoxy)quinazolin-4-yl)-3,8-diazabicyclo[3.2.1]octane-8-carboxylate (130mg, 0.15 mmol). The resulting mixture was stirred for 1 h at room temperature. After the reaction was completed (monitored by LCMS), saturated aqueous sodium bicarbonate solution (20 mL) was added to the reaction. The resulting mixture was extracted with ethyl acetate three times. The combined organic phase was washed with brine, dried over anhydrous sodium sulfate, filtered, and concentrated to dryness to obtain 4-((1*R*,5*S*)-3,8-diazabicyclo[3.2.1]octan-3-yl)-6,8-difluoro-7-(7-fluoro-8-(triisopropylsilyl)ethynyl)-3-((triisopropylsilyl)oxy)naphthalen-yl)-2-((1-(2-methoxyethyl)-3-methylazepan-3-yl)methoxy)quinazoline (100 mg) as a yellow solid crude product. LCMS (m/z): 972.5 (M + H).

**Step C:** 4-(4-((1*R*,5*S*)-3,8-diazabicyclo[3.2.1]octan-3-yl)-6,8-difluoro-2-((1-(2-methoxyethyl)-3-methylacridine-3-yl)methoxy)quinazolin-7-yl)-5-ethynyl-6-fluoronaphthalen-2-ol•triformate

At room temperature, DMF (2 mL) and cesium fluoride (156 mg, 1.0 mmol) were added to 4-((1*R*,5*S*)-3,8-diazabicyclo[3.2.1]octan-3-yl)-6,8-difluoro-7-(7-fluoro-8-(triisopropylsilyl)ethynyl)-3-((triisopropylsilyl)oxy)naphthalen-yl)-2-((1-(2-methoxyethyl)-3-methylazepan-3-yl)methoxy)quinazoline (100 mg). The resulting mixture was stirred at 50 °C for 1 hour. At the end of the reaction monitored by LCMS, the reaction was purified by Pre-HPLC (C18, CAN/(0.1% FA/$H_2O$) = 15-35%) to obtain 4-(4-((1*R*,5*S*)-3,8-diazabicyclo[3.2.1]octan-3-yl)-6,8-difluoro-2-((1-(2-methoxyethyl)-3-methylacridine-3-yl)methoxy)quinazolin-7-yl)-5-ethynyl-6-fluoronaphthalen-2-ol•triformate (40 mg) as a white solid. $^1$H NMR (400 MHz, DMSO-$d_6$) δ 8.19 (s, 3H), 8.02 - 7.95 (m, 1H), 7.58 - 7.52 (m, 1H), 7.51 - 7.45 (m, 1H), 7.41 (d, *J* = 2.6 Hz, 1H), 7.14 (d, *J* = 2.6 Hz, 1H), 4.33 - 4.22 (m, 3H), 4.18 - 4.09 (m, 2H), 4.07 - 3.99 (m, 2H), 3.96 - 3.90 (m, 2H), 3.57 - 3.53 (m, 2H), 3.12 - 3.09 (m, 3H), 2.70 - 2.63 (m, 3H), 2.61 - 2.55 (m, 2H), 2.35 - 2.27 (m, 2H), 1.82 - 1.66 (m, 4H), 1.59 - 1.32 (m, 6H), 0.93 (s, 3H). LCMS (m/z): 660.3 (M + H).

**Example 9**

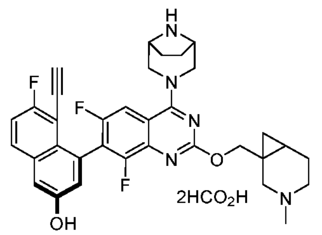

(*Ra*)-4-(4-((1*R*,5*S*)-3,8-diazabicyclo[3.2.1]octan-3-yl)-6,8-difluoro-2-((3-methyl-3-azabicyclo[4.1.0]heptan-1-yl)methoxy)quinazolin-7-yl)-5-ethynyl-6-fluoronaphthalen-2-ol•diformate

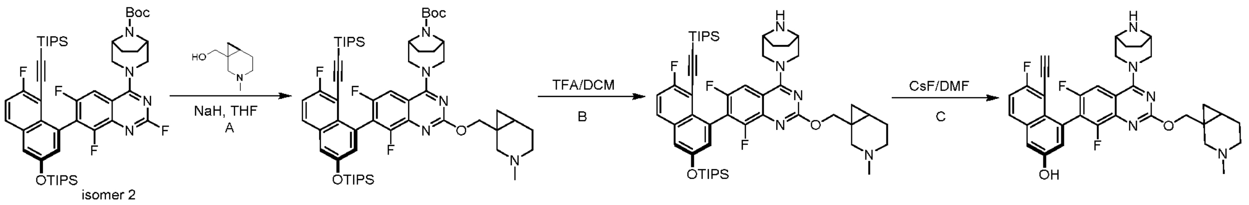

**A-1b-I2**

**Step A:** *tert-butyl* (*Ra*)-(1*R*,5*S*)-3-(6,8-difluoro-7-(7-fluoro-8-(triisopropylsilyl)ethynyl)-3-(triisopropylethylsilyl)oxy)naphthalen-1-yl)-2-((3-methyl-3-azabicyclo[4.1.0]heptan-1-methyl)methoxy)quinazolin-4-yl)-3,8-diazabicyclo[3.2.1]octane-8-carboxylate

NaH (26.9 mg, 0.673 mmol) was added to a mixture of (3-methyl-3-azabicyclo[4.1.0]heptane-1-yl) methanol

(38.0 mg, 0.269 mmol) in THF (5 mL) at room temperature and stirred for 0.5 h at the same temperature. *tert*-butyl (1*R*,5*S*)-3-(2,6,8-trifluoro-7-(7-fluoro-8-(triisopropylsilyl)ethynyl)-3-(triisopropylethylsilyl)oxy)naphthalen-1-yl)quinazolin-4-yl)-3,8-diazobicyclo[3.2.1]octane-8-carboxylate (120 mg, 0.13 mmol) was added and the mixture was stirred for 1 h at room temperature. After the reaction was completed (monitored by LCMS), the reaction was poured into saturated aqueous ammonium chloride solution (50 mL). The resulting mixture was extracted with EA (50 mL × 2). The combined organic phase was washed with brine (70 mL), concentrated, and further purified with FCC ($SiO_2$, EA: PE = 0% to 80%) to obtain *tert*-butyl (*Ra*)-(1*R*,5*S*)-3-(6,8-difluoro-7-(7-fluoro-8-(triisopropylsilyl)ethynyl)-3-(triisopropylethylsilyl)oxy) naphthalen-1-yl)-2-((3-methyl-3-azabicyclo[4.1.0]heptane-1-methyl)methoxy)quinazolin-4-yl)-3,8-diazabicyclo[3.2.1] octane-8-carboxylate (60mg, yield 44%) as a brown solid. LCMS (m/z): 1012.5 (M + H).

**Step B:** (*Ra*)-4-((1R,5S)-3,8-diazabicyclo[3.2.1]octane-3-yl)-6,8-difluoro-7-(7-fluoro-8-((triisopropylsilyl)ethynyl)-3-(triisopropylsilyl)oxy)naphthalen-1-yl)-2-((3-methyl-3-azabicyclo[4.1.0]heptane-1-yl]methoxy)quinazoline

At room temperature, a solution of *tert*-butyl (*Ra*)-(1R,5S)-3-(6,8-difluoro-7-(7-fluoro-8-(triisopropylsilyl)ethynyl)-3-(triisopropylethylsilyl)oxy)naphthalen-1-yl)-2-((3-methyl-3-azabicyclo[4.1.0]heptane-1-methyl)methoxy)quinazolin-4-yl)-3,8-diazabicyclo[3.2.1]octane-8-carboxylate (60mg, 0.059 mmol) in DCM/TFA (V/V = 1:1, 2 mL) was stirred for 1 hour at room temperature. After the reaction was completed (monitored by LCMS), the reaction was concentrated and saturated aqueous sodium bicarbonate solution (70 mL) was added. The resulting mixture was extracted with EA (70 mL × 2). The combined organic phase was washed with brine (70 mL), dried over anhydrous $Na_2SO_4$, and concentrated to obtain (*Ra*)-4-((1*R*,5*S*)-3,8-diazabicyclo[3.2.1]octane-3-yl)-6,8-difluoro-7-(7-fluoro-8-((triisopropylsilyl)ethynyl)-3-(triisopropylsilyl)oxy)naphthalen-1-yl)-2-((3-methyl-3-azabicyclo[4.1.0]heptane-1-yl]methoxy)quinazoline (60 mg, crude) as a brown solid. LCMS (m/z): 912.5 (M + H).

**Step C:** (*Ra*)-4-(4-((1*R*,5*S*)-3,8-diazabicyclo[3.2.1]octan-3-yl)-6,8-difluoro-2-((3-methyl-3-azabicyclo[4.1.0]heptane-1-yl)methoxy)quinazolin-7-yl)-5-ethynyl-6-fluoronaphthalen-2-ol•diformate

At room temperature, CsF (50 mg, 0.33 mmol) was added to a solution of 4-((1*R*,5*S*)-3,8-diazabicyclo[3.2.1] octane-3-yl)-6,8-difluoro-7-(7-fluoro-8-((triisopropylsilyl)ethynyl)-3-(triisopropylsilyl)oxy)naphthalen-1-yl)-2-((3-methyl-3-azabicyclo[4.1.0]heptane-1-yl]methoxy)quinazoline (60 mg) in DMF (2 mL). The reaction was heat to 50 °C and stirred for 1 hour. After cooling to room temperature, the mixture was filtered. The filtrate was purified by Pre-HPLC (formic acid system) to obtain (*Ra*)-4-(4-((1*R*,5*S*)-3,8-diazabicyclo[3.2.1]octan-3-yl)-6,8-difluoro-2-((3-methyl-3-azabicyclo [4.1.0]heptane-1-yl)methoxy)quinazolin-7-yl)-5-ethynyl-6-fluoronaphthalen-2-ol•diformate (5.1 mg) as a white solid. $^1H$ NMR (400 MHz, DMSO-$d_6$) δ 8.30 (s, 2H), 8.03 - 7.94 (m, 1H), 7.61 - 7.51 (m, 1H), 7.51 - 7.44 (m, 1H), 7.41 (d, *J* = 2.5 Hz, 1H), 7.15 (d, *J*= 2.6 Hz, 1H), 4.33 - 4.19 (m, 3H), 4.08 - 4.01 (m, 1H), 4.01 - 3.88 (m, 2H), 3.62 - 3.55 (m, 3H), 2.38 - 2.34 (m, 1H), 2.27 - 2.22 (m, 1H), 2.11 (s, 3H), 1.98 - 1.81 (m, 2H), 1.80 - 1.52 (m, 6H), 1.06 - 0.92 (m, 1H), 0.73 - 0.59 (m, 1H), 0.58 - 0.44 (m, 1H). $^{19}F$ NMR (376 MHz, DMSO-$d_6$) δ-110.27,-119.05,-124.82. LCMS (m/z): 600.3 (M + H).

**Examples 10-1/10-4 and examples 10-2/10-3**

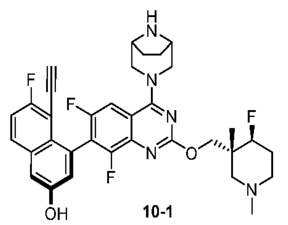
10-1

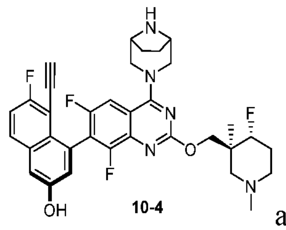
10-4

and

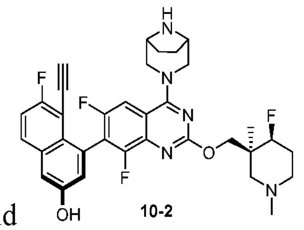
10-2

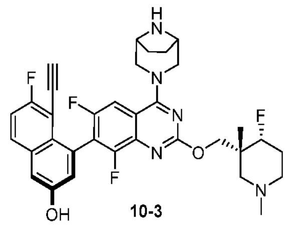
10-3

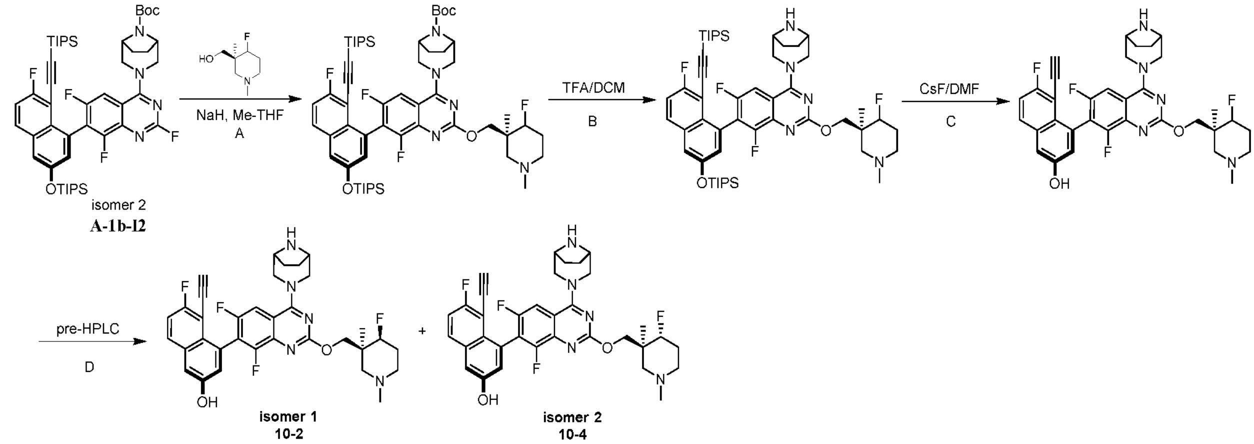

isomer 2
A-1b-I2
isomer 1
10-2
isomer 2
10-4

**Step A:** *tert-butyl* (*Ra*)-(1*R*,5*S*)-3-(6,8-difluoro-2-((3*S*)-4-fluoro-1,3-dimethylpiperidin-3-yl)methoxy)-7-(7-fluoro-8-(triisopropylsilyl)ethynyl)-3-((triisopropylsilyl)oxy)naphthalen-1-yl)quinazolin-4-yl)-3,8-diazabicyclo[3.2.1]octane-8-carboxylate

At room temperature, NaH (162 mg, 4.04 mmol, 60%) was added to a mixture of (3S)-(4-fluoro-1,3-dimethylpiperidin-3-yl)methanol (Intermediate **a8A-1,** 217 mg, 1.35 mmol) in anhydrous THF (20 mL). After stirring for 0.5 h at room temperature, tert-butyl (*Ra*)-(1*R*,5*S*)-3-(2,6,8-trifluoro-7-(7-fluoro-8-(triisopropylsilyl)ethynyl)-3-((triisopropylsilyl)oxy) naphthalen-1-yl)quinazolin-4-yl)-3,8-diazabicyclo[3.2.1]octane-8-carboxylate (intermediate **A-1b-I2,** 1.2g, 1.35 mmol) was added, and the resulting mixture was stirred at room temperature overnight. After the reaction was completed (monitored by LCMS), the reaction was poured into saturated aqueous $NH_4Cl$ solution (60 mL). The resulting mixture was extracted with EA (50 mL × 3), and the combined organic phase was washed with brine (40 mL), concentrated under reduced pressure. The crude product was purified by FCC ($SiO_2$, EA/PE = 0 ~ 40%) to obtain *tert*-butyl (*Ra*)-(1*R*,5*S*)-3-(6,8-difluoro-2-((3*S*)-4-fluoro-1,3-dimethylpiperidin-3-yl)methoxy)-7-(7-fluoro-8-(triisopropylsilyl)ethynyl)-3-((triisopropylsilyl)oxy)naphthalen-1-yl)quinazolin-4-yl)-3,8-diazabicyclo[3.2.1]octane-8-carboxylate (940 mg, yield 68%) as a yellow solid. LCMS (m/z): 516.8 (M/2 + H).

**Step B:** (*Ra*)-4-((1*R*,5*S*)-3,8-diazabicyclo[3.2.1]octan-3-yl)-6,8-difluoro-2-((3S)-4-fluoro-1,3-dimethylpiperidin-3-yl) methoxy)-7-(7-fluoro-8-(triisopropylsilyl)ethynyl)-3-((triisopropylsilyloxy)naphthalen-1-yl)quinazoline

At room temperature, trifluoroacetic acid (30 mL) was added to *tert-butyl* (*Ra*)-(*1R,5S*)-3-(6,8-difluoro-2-((3*S*)-4-fluoro-1,3-dimethylpiperidin-3-yl)methoxy)-7-(7-fluoro-8-(triisopropylsilyl)ethynyl)-3-((triisopropylsilyl)oxy)naphthalen-1-yl)quinazolin-4-yl)-3,8-diazabicyclo[3.2.1]octane-8-carboxylate (700 mg, 0.678 mmol). The resulting mixture was stirred at room temperature for 1 h. The acid was removed by concentration under reduced pressure. The residue was diluted with EA (30 mL), and the pH was adjusted to about 8 with saturated $NaHCO_3$ solution (20 mL). Then, water (30 mL) was added, and the mixture was extracted with EA (30 mL × 2). The combined organic phases were washed with saturated brine and concentrated under reduced pressure to obtain (*Ra*)-4-((1*R*,5*S*)-3,8-diazabicyclo[3.2.1]octan-3-yl)-6,8-difluoro-2-((3S)-4-fluoro-1,3-dimethylpiperidin-3-yl)methoxy)-7-(7-fluoro-8-(triisopropylsilyl)ethynyl)-3-((triisopropylsilyloxy)naphthalen-1-yl)quinazoline (600 mg, yield 95%) as a yellow solid. LCMS (m/z): 466.8 (M/2 + H).

**Step C-D:** Synthesis of compounds 10-2 and 10-4

At room temperature, (*Ra*)-4-((1*R*,5*S*)-3,8-diazabicyclo[3.2.1]octan-3-yl)-6,8-difluoro-2-((3R or 3*S*)-4-fluoro-1,3-dimethylpiperidin-3-yl)methoxy)-7-(7-fluoro-8-(triisopropylsilyl)ethynyl)-3-((triisopropylsilyloxy)naphthalen-1-yl)quinazoline (500 mg, 0.536 mmol) and CsF (815 mg, 5.36 mmol) were added to DMF (6 mL). The reaction was heated to

50 °C and stirred for 1 h. After the reaction was completed (monitored by LCMS), the insoluble solid was removed by filtration. The filtrate was purified by Pre-HPLC (C18, CAN/(10 mmol $NH_4HCO_3/H_2O$) = 35-55%) to obtain isomer 1 (with a shorter retention time) as compound 10-2 (160 mg, yield 50%), LCMS (m/z): 620.1 (M + H). $^1$H NMR (400 MHz, DMSO-*d*$_6$) δ 10.25 (s, 1H), 7.99 (dd, *J* = 9.2, 5.9 Hz, 1H), 7.55 (d, *J* = 10.4 Hz, 1H), 7.51 - 7.45 (m, 1H), 7.41 (d, *J* = 2.4 Hz, 1H), 7.15 (d, *J* = 2.4 Hz, 1H), 4.74 - 4.45 (m, 1H), 4.38 - 4.15 (m, 4H), 3.99 (s, 1H), 3.64 - 3.40 (m, 4H), 2.44 - 2.20 (m, 4H), 2.15 (s, 3H), 2.01 - 1.59 (m, 6H), 1.14 (s, 3H). $^{19}$F NMR (376 MHz, DMSO-*d*$_6$) δ-110.21,-119.17,-124.74,-190.42; And isomer 2 (with a longer retention time) was compound 10-4 (100 mg, yield 30%), LCMS (m/z): 620.1 (M + H). $^1$H NMR (400 MHz, DMSO-*d*$_6$) δ 10.25 (s, 1H), 8.09 - 7.93 (m, 1H), 7.55 (d, *J* = 9.9 Hz, 1H), 7.52 - 7.45 (m, 1H), 7.41 (d, *J* = 2.4 Hz, 1H), 7.15 (d, *J* = 2.4 Hz, 1H), 4.74 - 4.45 (m, 1H), 4.41 - 4.13 (m, 4H), 3.99 (s, 1H), 3.59 - 3.41 (m, 4H), 2.44 - 2.20 (m, 4H), 2.14 (s, 3H), 1.97 - 1.59 (m, 6H), 1.09 (s, 3H). $^{19}$F NMR (376 MHz, DMSO-*d*$_6$) δ-110.19,-119.09,-124.71.

Compounds 10-1 and 10-3 can be synthesized according to the above protocol.

**Referring to the above synthesis methods or appropriate variations, this invention also synthesized the following examples:**

| Example | Structure | Name | Characterization data |
|---|---|---|---|
| 10 | 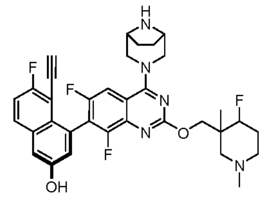 | (*Ra*)-4-(4-((1*R*,5*S*)-3,8-diazabicyclo[3.2.1]octan-3-yl)-6,8-difluoro-2-(4-fluoro-1,3-dimethylpiperidin-3-yl)methoxy)quinazolin-7-yl)-5-ethynyl-6-fluoronaphthalen-2-ol | LCMS (m/z): 620.1 (M + H). |
| 15 | 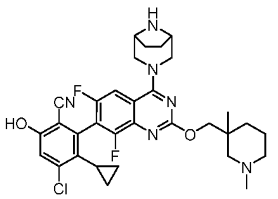 | 2-(4-((1*R*,5*S*)-3,8-diazabicyclo[3.2.1]octan-3-yl)-2-((1,3-dimethylpiperidin-3-yl)methoxy)-6,8-difluoroquinazolin-7-yl)-4-chloro-3-cyclopropyl-6-hydroxybenzonitrile | LCMS (m/z): 609.2 (M + H). |
| 15-1 | 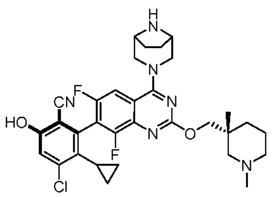 | (*Sa*)-2-(4-((1*R*,5*S*)-3,8-diazabicyclo[3.2.1]octan-3-yl)-2-((*R*)-1,3-dimethylpiperidin-3-yl)methoxy)-6,8-difluoroquinazolin-7-yl)-4-chloro-3-cyclopropyl-6-hydroxybenzonitrile | LCMS (m/z): 609.2 (M + H). |
| 15-2 | 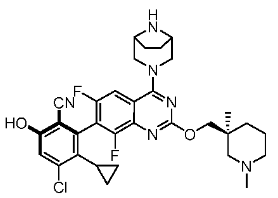 | (*Sa*)-2-(4-((1*R*,5*S*)-3,8-diazabicyclo[3.2.1]octan-3-yl)-2-(((*S*)-1,3-dimethylpiperidin-3-yl)methoxy)-6,8-difluoroquinazolin-7-yl)-4-chloro-3-cyclopropyl-6-hydroxybenzonitrile | LCMS (m/z): 609.2 (M + H). |
| 16 | 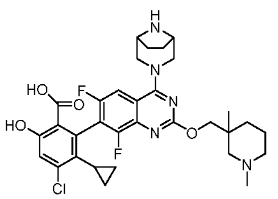 | 2-(4-((1*R*,5*S*)-3,8-diazabicyclo[3.2.1]octan-3-yl)-2-((1,3-dimethylpiperidin-3-yl)methoxy)-6,8-difluoroquinazolin-7-yl)-4-chloro-3-cyclopropyl-6-hydroxybenzoic acid | LCMS (m/z): 627.2 (M + H). |
| 16-1 | 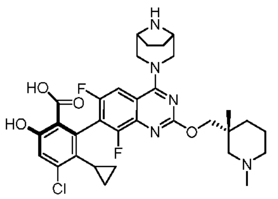 | (*Sa*)-2-(4-((1*R*,5*S*)-3,8-diazabicyclo[3.2.1]octan-3-yl)-2-((*R*)-1,3-dimethylpiperidin-3-yl)methoxy)-6,8-difluoroquinazolin-7-yl)-4-chloro-3-cyclopropyl-6-hydroxybenzoic acid | LCMS (m/z): 627.2 (M + H). |

(continued)

| Example | Structure | Name | Characterization data |
|---|---|---|---|
| 16-2 | 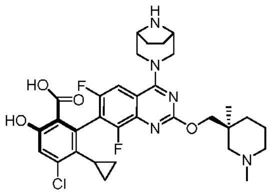 | (*Sa*)-2-(4-((1*R*,5*S*)-3,8-diazabicyclo[3.2.1]octan-3-yl)-2-(((*S*)-1,3-dimethylpiperidin-3-yl)methoxy)-6,8-difluoroquinazolin-7-yl)-4-chloro-3-cyclopropyl-6-hydroxybenzoic acid | LCMS (m/z): 627.2 (M + H). |
| 17 | 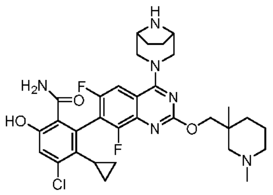 | 2-(4-((1*R*,5*S*)-3,8-diazabicyclo [3.2.1]octan-3-yl)-2-((1,3-dimethylpiperidin-3-yl)methoxy)-6,8-difluoroquinazolin-7-yl)-4-chloro-3-cyclopropyl-6-hydroxybenzamide | LCMS (m/z): 626.3 (M + H). |
| 17-1 | 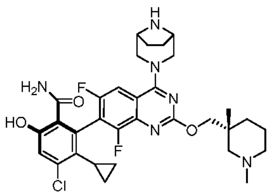 | (*Sa*)-2-(4-((1*R*,5*S*)-3,8-diazabicyclo[3.2.1]octan-3-yl)-2-(((*R*)-1,3-dimethylpiperidin-3-yl)methoxy)-6,8-difluoroquinazolin-7-yl)-4-chloro-3-cyclopropyl-6-hydroxybenzamide | LCMS (m/z): 626.3 (M + H). |
| 17-2 | 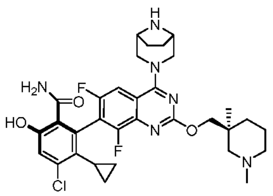 | (*Sa*)-2-(4-((1*R*,5*S*)-3,8-diazabicyclo[3.2.1]octan-3-yl)-2-(((*S*)-1,3-dimethylpiperidin-3-yl)methoxy)-6,8-difluoroquinazolin-7-yl)-4-chloro-3-cyclopropyl-6-hydroxybenzamide | LCMS (m/z): 626.3 (M + H). |
| 18 | 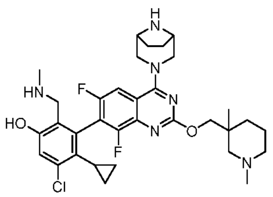 | 2-(4-((1*R*,5*S*)-3,8-diazabicyclo[3.2.1]octan-3-yl)-2-((1,3-dimethylpiperidin-3-yl)methoxy)-6,8-difluoroquinazolin-7-yl)-4-chloro-3-cyclopropyl-6-hydroxybenzamide | LCMS (m/z): 627.3 (M + H). |
| 19 | 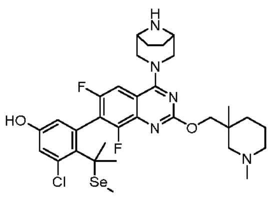 | 3-(4-((1*R*,5*S*)-3,8-diazabicyclo[3.2.1]octan-3-yl)-2-((1,3-dimethylpiperidin-3-yl)methoxy)-6,8-difluoroquinazolin-7-yl)-5-chloro-4-(2-(methylselenyl)prop-2-yl) phenol | LCMS (m/z): 680.2 (M + H). |
| 20 | 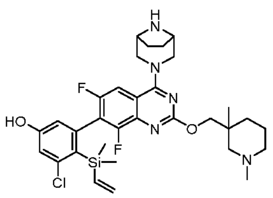 | 3-(4-((1*R*,5*S*)-3,8-diazabicyclo[3.2.1]octan-3-yl)-2-((1,3-dimethylpiperidin-3-yl)methoxy)-6,8-difluoroquinazolin-7-yl)-5-chloro-4-(dimethyl(vinyl) methylsilyl) phenol | LCMS (m/z): 628.3 (M + H). |

(continued)

| Example | Structure | Name | Characterization data |
|---|---|---|---|
| 21 | 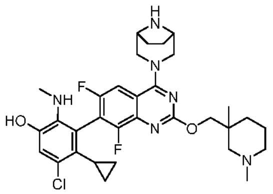 | 3-(4-((1*R*,5*S*)-3,8-diazabicyclo[3.2.1]octan-3-yl)-2-((1,3-dimethylpiperidin-3-yl)methoxy)-6,8-difluoroquinazolin-7-yl)-5-chloro-4-cyclopropyl-2-(methylamino)phenol | LCMS (m/z): 613.3 (M + H). |
| 21-1 | 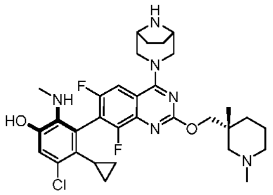 | (*Sa*)-3-(4-((1*R*,5*S*)-3,8-diazabicyclo[3.2.1]octan-3-yl)-2-(((R)-1,3-dimethylpiperidin-3-yl)methoxy)-6,8-difluoroquinazolin-7-yl)-5-chloro-4-cyclopropyl-2-(methylamino) phenol | LCMS (m/z): 613.3 (M + H). |
| 21-2 | 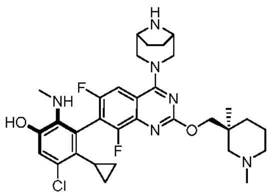 | (*Sa*)-3-(4-((1*R*,5*S*)-3,8-diazabicyclo[3.2.1]octan-3-yl)-2-(((*S*)-1,3-dimethylpiperidin-3-yl)methoxy)-6,8-difluoroquinazolin-7-yl)-5-chloro-4-cyclopropyl-2-(methylamino) phenol | LCMS (m/z): 613.3 (M + H). |
| 22 | 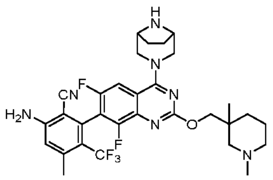 | 2-(4-((1*R*,5*S*)-3,8-diazabicyclo[3.2.1]octan-3-yl)-2-((1,3-dimethylpiperidin-3-yl)methoxy)-6,8-difluoroquinazolin-7-yl)-6-amino-4-methyl-3-(trifluoromethyl)benzonitrile | LCMS (m/z): 616.3 (M + H). |
| 22-1 | 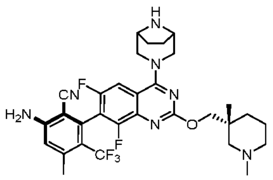 | (*Ra*)-2-(4-((1*R*,5*S*)-3,8-diazabicyclo[3.2.1]octan-3-yl)-2-(((*R*)-1,3-dimethylpiperidin-3-yl)methoxy)-6,8-difluoroquinazolin-7-yl)-6-amino-4-methyl-3-(trifluoromethyl)benzonitrile | LCMS (m/z): 616.3 (M + H). |
| 22-2 | 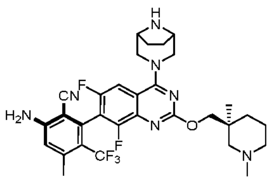 | (*Ra*)-2-(4-((1*R*,5*S*)-*3,8*-diazabicyclo[3.2.1]octan-3-yl)-2-(((*S*)-1,3-dimethylpiperidin-3-yl)methoxy)-6,8-difluoroquinazolin-7-yl)-6-amino-4-methyl-3-(trifluoromethyl)benzonitrile | LCMS (m/z): 616.3 (M + H). |
| 23 | 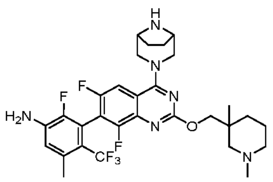 | 3-(4-((1*R*,5*S*)-3,8-diazabicyclo[3.2.1]octan-3-yl)-2-((1,3-dimethylpiperidin-3-yl)methoxy)-6,8-difluoroquinazolin-7-yl)-2-fluoro-5-methyl-4-(trifluoromethyl)aniline | LCMS (m/z): 609.2 (M + H). |
| 23-1 | 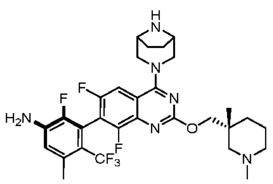 | (*Sa*)-3-(4-((1*R*,5*S*)-3,8-diazabicyclo[3.2.1]octan-3-yl)-2-(((*R*)-1,3-dimethylpiperidin-3-yl)methoxy)-6,8-difluoroquinazolin-7-yl)-2-fluoro-5-methyl-4-(trifluoromethyl)aniline | LCMS (m/z): 609.2 (M + H). |

(continued)

| Example | Structure | Name | Characterization data |
|---|---|---|---|
| 23-2 | 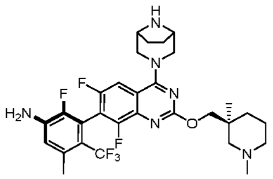 | (*Sa*)-3-(4-((1*R*,5*S*)-3,8-diazabicyclo[3.2.1]octan-3-yl)-2-(((*S*)-1,3-dimethylpiperidin-3-yl)methoxy)-6,8-difluoroquinazolin-7-yl)-2-fluoro-5-methyl-4-(trifluoromethyl)aniline | LCMS (m/z): 609.2 (M + H). |
| 24 | 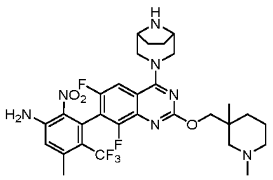 | 3-(4-((1*R*,5*S*)-3,8-diazabicyclo[3.2.1]octan-3-yl)-2-((1,3-dimethylpiperidin-3-yl)methoxy)-6,8-difluoroquinazolin-7-yl)-5-methyl-2-nitro-4-(trifluoromethyl)aniline | LCMS (m/z): 636.3 (M + H). |
| 24-1 | 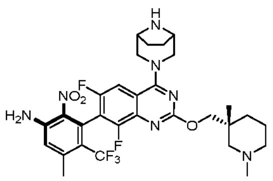 | (*Sa*)-3-(4-((1*R*,5*S*)-3,8-diazabicyclo[3.2.1]octan-3-yl)-2-(((*R*)-1,3-dimethylpiperidin-3-yl)methoxy)-6,8-difluoroquinazolin-7-yl)-5-methyl-2-nitro-4-(trifluoromethyl)aniline | LCMS (m/z): 636.3 (M + H). |
| 24-2 | 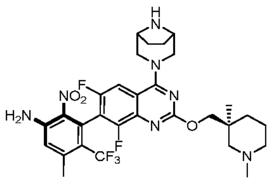 | (*Sa*)-3-(4-((1*R*,5*S*)-3,8-diazabicyclo[3.2.1]octan-3-yl)-2-(((*S*)-1,3-dimethylpiperidin-3-yl)methoxy)-6,8-difluoroquinazolin-7-yl)-5-methyl-2-nitro-4-(trifluoromethyl)aniline | LCMS (m/z): 636.3 (M + H). |
| 25 | 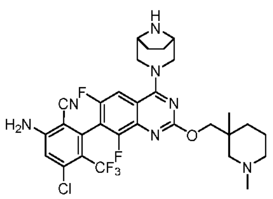 | 2-(4-((1*R*,5*S*)-3,8-diazabicyclo[3.2.1]octan-3-yl)-2-((1,3-dimethylpiperidin-3-yl)methoxy)-6,8-difluoroquinazolin-7-yl)-6-amino-4-chloro-3-(trifluoromethyl)benzonitrile | LCMS (m/z): 636.2 (M + H). |
| 25-1 | 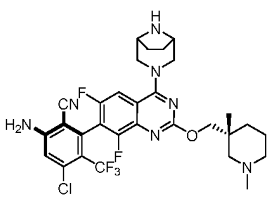 | (*Ra*)-2-(4-((1*R*,5*S*)-3,8-diazabicyclo[3.2.1]octan-3-yl)-2-((*R*)-1,3-dimethylpiperidin-3-yl)methoxy)-6,8-difluoroquinazolin-7-yl)-6-amino-4-chloro-3-(trifluoromethyl)benzonitrile | LCMS (m/z): 636.3 (M + H). |
| 25-2 | 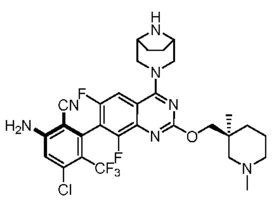 | (*Ra*)-2-(4-((1*R*,5*S*)-3,8-diazabicyclo[3.2.1]octan-3-yl)-2-(((*S*)-1,3-dimethylpiperidin-3-yl)methoxy)-6,8-difluoroquinazolin-7-yl)-6-amino-4-chloro-3-(trifluoromethyl)benzonitrile | LCMS (m/z): 636.2 (M + H). |
| 26 | 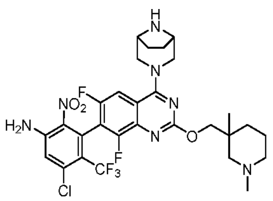 | 3-(4-((1*R*,5*S*)-3,8-diazabicyclo[3.2.1]octan-3-yl)-2-((1,3-dimethylpiperidin-3-yl)methoxy)-6,8-difluoroquinazolin-7-yl)-5-chloro-2-nitro-4-(trifluoromethyl)aniline | LCMS (m/z): 656.2 (M + H). |

(continued)

| Example | Structure | Name | Characterization data |
|---|---|---|---|
| 26-1 | 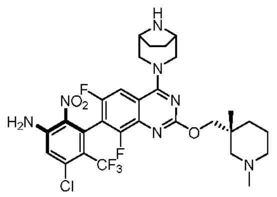 | (*Sa*)-3-(4-((1*R*,5*S*)-3,8-diazabicyclo[3.2.1]octan-3-yl)-2-((*R*)-1,3-dimethylpiperidin-3-yl)methoxy)-6,8-difluoroquinazolin-7-yl)-5-chloro-2-nitro-4-(trifluoromethyl)aniline | LCMS (m/z): 656.2 (M + H). |
| 26-2 | 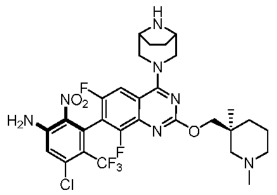 | (*Sa*)-3-(4-((1*R*,5*S*)-3,8-diazabicyclo[3.2.1]octan-3-yl)-2-((*S*)-1,3-dimethylpiperidin-3-yl)methoxy)-6,8-difluoroquinazolin-7-yl)-5-chloro-2-nitro-4-(trifluoromethyl)aniline | LCMS (m/z): 656.2 (M + H). |
| 27 | 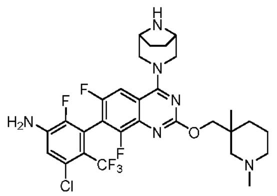 | 3-(4-((1*R*,5*S*)-3,8-diazabicyclo[3.2.1]octan-3-yl)-2-((1,3-dimethylpiperidin-3-yl)methoxy)-6,8-difluoroquinazolin-7-yl)-5-chloro-2-fluoro-4-(trifluoromethyl)aniline | LCMS (m/z): 629.2 (M + H). |
| 27-1 | 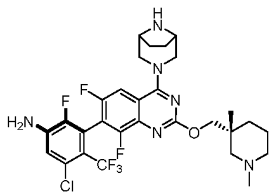 | (*Sa*)-3-(4-((1*R*,5*S*)-3,8-diazabicyclo[3.2.1]octan-3-yl)-2-(((*R*)-1,3-dimethylpiperidin-3-yl)methoxy)-6,8-difluoroquinazolin-7-yl)-5-chloro-2-fluoro-4-(trifluoromethyl)aniline | LCMS (m/z): 629.2 (M + H). |
| 27-2 | 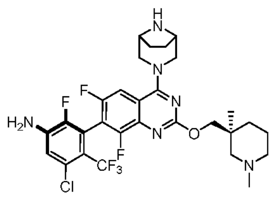 | (*Sa*)-3-(4-((1*R*,5*S*)-3,8-diazabicyclo[3.2.1]octan-3-yl)-2-((*S*)-1,3-dimethylpiperidin-3-yl)methoxy)-6,8-difluoroquinazolin-7-yl)-5-chloro-2-fluoro-4-(trifluoromethyl)aniline | LCMS (m/z): 629.2 (M + H). |
| 28 | 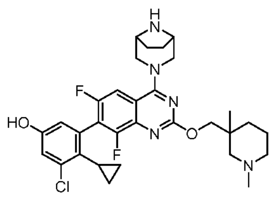 | 3-(4-((1*R*,5*S*)-3,8-diazabicyclo[3.2.1]octan-3-yl)-2-((1,3-dimethylpiperidin-3-yl, methoxy)-6,8-difluoroquinolin-7-yl)-5-chloro-4-cyclopropylphenol | LCMS (m/z): 584.2 (M + H). |
| 29 | 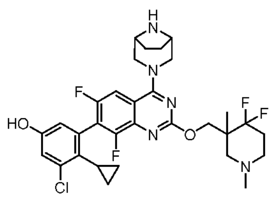 | 3-(4-((1*R*,5*S*)-3,8-diazabicyclo[3.2.1]octan-3-yl)-2-((4,4-difluoro-1,3-dimethylpiperidin-3-yl]methoxy)-6,8-difluoroquinolin-7-yl)-5-chloro-4-cyclopropylphenol | LCMS (m/z): 620.2 (M + H). |

(continued)

| Example | Structure | Name | Characterization data |
|---|---|---|---|
| 29-1 | 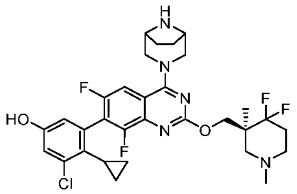 | 3-(4-(((1*R*,5*S*)-3,8-diazabicyclo[3.2.1]octan-3-yl)-2-(((*S*)-4,4-difluoro-1,3-dimethylpiperidin-3-yl]methoxy)-6,8-difluoroquinolin-7-yl)-5-chloro-4-cyclopropylphenol | LCMS (m/z): 620.2 (M + H). |

**Example 29-1A**

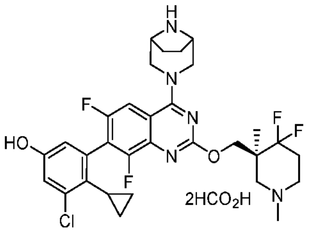

3-(4-((1*R*,5*S*)-3,8-diazabicyclo[3.2.1]octane-3-yl)-2-(((*S*)-4,4-difluoro-1,3-dimethylpiperidin-3-yl)methoxy)-6,8-difluoroquinolin-7-yl)-5-chloro-4-cyclopropylphenol·diformate

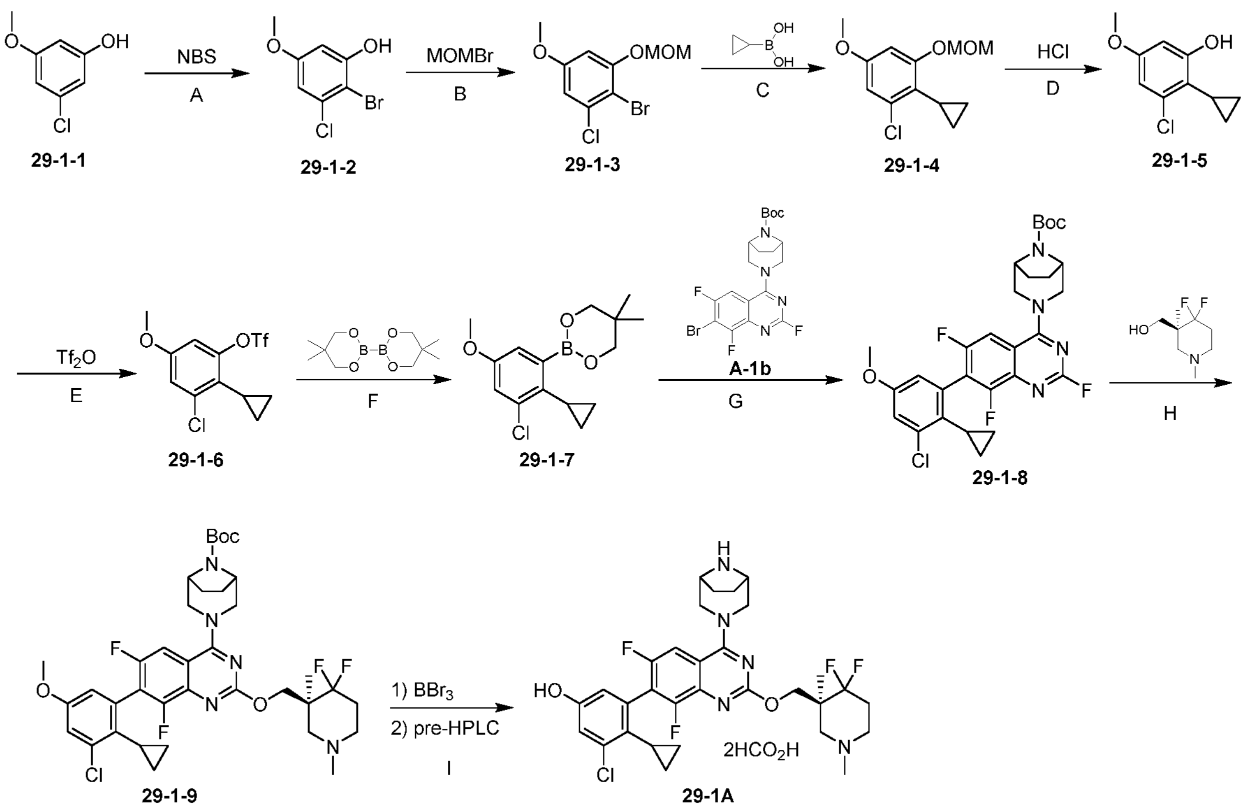

**Step A:** 2-bromo-3-chloro-5-methoxyphenol

3-chloro-5-methoxyphenol (14.0 g, 88.3 mmol) and dichloromethane (200 mL) were added to a round bottom flask with a stir bar under an ice-water bath. While keeping the ice-water bath and stirring, NBS (24.0g, 132 mmol) was added. The resulting mixture was warmed to room temperature and stirred overnight. After the reaction was completed (monitored by TLC), saturated sodium thiosulfate (100 mL) was added, the resulting mixture was extracted with dichloromethane (100 mL × 2). The combined organic phase was washed with brine (170 mL), dried over anhydrous $Na_2SO_4$, and purified with FCC ($SiO_2$, EA/PE = 0-15%) to obtain 2-bromo-3-chloro-5-methoxyphenol (7.0 g, yield 33%). GCMS (m/z): 238.

**Step B:** 2-bromo-1-chloro-5-methoxy-3-(methoxy methoxy) benzene

2-bromo-3-chloro-5-methoxyphenol (9.0 g, 37.9 mmol), *N,N*-diisopropylethylamine (9.8 g, 75.8 mmol) and dichloromethane (100 mL) were added to a round bottom flask with a stir bar under an ice-water bath. While maintaining the temperature, bromomethyl ether (5.2 g, 41.7 mmol) was added dropwise and the resulting mixture was stirred for 30 min. After the reaction was completed (monitored by TLC), the reaction was dried and the crude product was purified by FCC ($SiO_2$, EA/PE = 0-25%) to give 2-bromo-1-chloro-5-methoxy-3-(methoxy methoxy) benzene (7.5 g, yield 70%).

**Step C:** 1-chloro-2-cyclopropyl-5-methoxy-3-(methoxy methoxy)benzene

2-bromo-1-chloro-5-methoxy-3-(methoxy methoxy) benzene (2.0 g, 7.1 mmol), cyclopropylboronic acid (920 mg, 10.7 mmol), potassium phosphate (4.5 g, 21.3 mmol), and dioxane (20 mL) were added at room temperature in a round bottom flask with a stir bar. The mixture was degassed with nitrogen three times, and Pd(dppf)$Cl_2$ (256 mg, 0.35 mmol) was added. The reaction was heated to 110 °C and stirred for 16 h. After the reaction was completed (monitored by GCMS), the reaction solution was evaporated to dryness. The resulting crude product was purified by FCC ($SiO_2$, EA/PE = 0-50%) to give 1-chloro-2-cyclopropyl-5-methoxy-3-(methoxy methoxy) benzene (1.6 g, yield 92%). GCMS (m/z): 242.

**Step D:** 3-chloro-2-cyclopropyl-5-methoxyphenol

Concentrated hydrochloric acid (4 mL) was added dropwise to a solution of 1-chloro-2-cyclopropyl-5-methoxy-3-(methoxy methoxy) benzene (1.0 g, 4.12 mmol) in methanol (20 mL) and dichloromethane (10 mL) at room temperature under stirring, and the resulting mixture was stirred at room temperature for 6 h. After the reaction was completed (monitored by TLC), saturated sodium bicarbonate was added, and the mixture was extracted with dichloromethane (50 mL × 3). The combined organic phase was washed with brine (50 mL) and dried over anhydrous $Na_2SO_4$, filtrated, concentrated, and purified by FCC ($SiO_2$, EA/PE = 0-50%) to obtain 3-chloro-2-cyclopropyl-5-methoxyphenol (680 mg, yield 83%).

**Step E:** 3-chloro-2-cyclopropyl-5-methoxyphenyl trifluoromethanesulfonate

Under stirring in an ice-water bath, trifluoromethanesulfonate anhydride (0.64 mL, 3.82 mmol) was added dropwise to a solution of 3-chloro-2-cyclopropyl-5-methoxyphenol (680 mg, 3.5 mmol), N,N-diisopropylethylamine (1.1 mL, 6.9 mmol) in dichloromethane (6 mL), and the resulting mixture was stirred for 30 min. After the reaction was completed (monitored by LCMS), and the reaction was concentrated to give the crude product which was purified by FCC ($SiO_2$, EA/PE = 0-33%) to give 3-chloro-2-cyclopropyl-5-methoxyphenyl trifluoromethanesulfonate (620 mg, yield 55%). LCMS (m/z): 331.0 (M + H).

**Step F:** 2-(3-chloro-2-cyclopropyl-5-methoxyphenyl)-5,5-dimethyl-1,3,2-dioxaborinane

At room temperature, 3-chloro-2-cyclopropyl-5-methoxyphenyl trifluoromethanesulfonate (200 mg, 0.60 mmol), 5,5,5',5'-tetramethyl-2,2'-bis(1,3,2-dioxaborinane) (250 mg, 1.11 mmol), potassium phosphate (380 mg, 1.8 mmol), and dioxane (2 mL) were added to a round bottom flask with a stir bar. The mixture was degassed with nitrogen three times, Pd(dppf)$Cl_2$ (40 mg, 0.06 mmol) was added. The resulting mixture was heated to 110 °C and stirred for 16 h. After the reaction was completed (monitored by LCMS), the reaction was concentrated and the resulting crude product was purified with FCC ($SiO_2$, EA/PE = 0-50%) to give 2-(3-chloro-2-cyclopropyl-5-methoxyphenyl)-5,5-dimethyl-1,3,2-dioxaborinane (120 mg, yield 67%). GCMS (m/z): 294.

**Step G:** *tert*-butyl (1*R*,5*S*)-3-(7-(3-chloro-2-cyclopropyl-5-methoxyphenyl)-2,6,8-trifluoroquinazolin-4-yl)-3,8-diazabicyclo[3.2.1]octane-8-carboxylate

At room temperature, *tert*-butyl (1*R*,5*S*)-3-(7-bromo-2,6,8-trifluoroquinazolin-4-yl)-3,8-diazabicyclo[3.2.1]octane-8-carboxylate (120 mg, 0.254 mmol), 2-(3-chloro-2-cyclopropyl-5-methoxyphenyl)-5,5-dimethyl-1,3,2-dioxaborinane (120 mg, 0.41 mmol), potassium phosphate (160 mg, 0.76 mmol), and dioxane (2 mL) were added in round-bottom flask with a stir bar. The mixture was degassed with nitrogen three times, and Pd(dtbpf)$Cl_2$ (15 mg, 0.02 mmol) was added. The resulting mixture was heated to 110 °C and stirred for 30 min. After the reaction was completed (monitored by LCMS), the reaction was concentrated to give the crude product which was purified with FCC ($SiO_2$, EA/PE = 0-50%) to obtain *tert*-butyl (1*R*,5*S*)-3-(7-(3-chloro-2-cyclopropyl-5-methoxyphenyl)-2,6,8-trifluoroquinazolin-4-yl)-3,8-diazabicyclo[3.2.1]octane-8-carboxylate (110 mg, yield 75%). LCMS (m/z): 575.0 (M + H).

**Step H:** *tert*-butyl (1*R*,5*S*)-3-(7-(3-chloro-2-cyc1opropyl-5-methoxyphenyl)-2-(((*S*)-4,4-difluoro-1,3-dimethylpiperidin-3-yl)methoxy)-6,8-difluoroquinazolin-4-yl)-3,8-diazabicyclo[3.2.1]octane-8-carboxylate

At room temperature, sodium hydride (10 mg, 0.25 mmol, 60% dispersed in mineral oil) was added to a solution of (*S*)-(4,4-difluoro-1,3-dimethylpiperidin-3-yl) methanol (40 mg, 0.22 mmol) in tetrahydrofuran (0.5 mL) in a round bottom flask with a stir bar. At the same temperature, *tert*-butyl (1*R*,5*S*)-3-(7-(3-chloro-2-cyclopropyl-5-methoxyphenyl)-2,6,8-trifluoroquinazolin-4-yl)-3,8-diazabicyclo[3.2.1]octane-8-carboxylate (60 mg, 0.10 mmol) was added, and the resulting mixture was stirred for 1 h. After the reaction was completed (monitored by LCMS), the reaction was concentrated under reduced pressure. The crude product was purified with FCC ($SiO_2$, EA/PE = 0-100%) to obtain *tert*-butyl (1*R*,5*S*)-3-(7-(3-chloro-2-cyclopropyl-5-methoxyphenyl)-2-(((*S*)-4,4-difluoro-1,3-dimethylpiperidin-3-yl)methoxy)-6,8-difluoroquinazolin-4-yl)-3,8-diazabicyclo[3.2.1]octane-8-carboxylate (60 mg, yield 78%). LCMS (m/z): 734.0 (M + H).

**Step I:** 3-(4-((1*R*,5*S*)-3,8-diazabicyclo[3.2.1]octan-3-yl)-2-(((*S*)-4,4-difluoro-1,3-dimethylpiperidin-3-yl)methoxy)-6,8-difluoroquinazolin-7-yl)-5-chloro-4-cyclopropylphenol·diformate

Boron tribromide (200 mg, 0.82 mmol) was added dropwise to a solution of *tert-butyl* (1*R*,5*S*)-3-(7-(3-chloro-2-cyclopropyl-5-methoxyphenyl)-2-(((*S*)-4,4-difluoro-1,3-dimethylpiperidin-3-yl)methoxy)-6,8-difluoroquinazolin-4-yl)-3,8-diazabicyclo[3.2.1]octane-8-carboxylate (60 mg, 0.08 mmol) in dichloromethane (1 mL) with stirring in an ice-water bath. The resulting mixture was stirred at the same temperature for 30 min. After the reaction was completed (monitored by LCMS), methanol (1 mL) and saturated $NaHCO_3$ solution (2 mL) were added in sequence, and the mixture was extracted with dichloromethane (5 mL × 3). The combined organic phase was washed with brine (5 mL), dried over anhydrous $Na_2SO_4$, filtered, and concentrated to obtain the crude product which was purified by prep-HPLC (C18, CAN/(0.1% FA/$H_2O$) = 20 - 95%) to obtain 3-(4-((1*R*,5*S*)-3,8-diazabicyclo[3.2.1]octan-3-yl)-2-(((S)-4,4-difluoro-1,3-dimethylpiperidin-3-yl)methoxy)-6,8-difluoroquinazolin-7-yl)-5-chloro-4-cyclopropylphenol·diformate (5.8 mg, yield 11%). $^1H$ NMR (400 MHz, Methanol-$d_4$) δ 8.47 (s, 2H), 7.60 (d, *J* = 10.0 Hz, 1H), 6.96 (d, *J* = 2.5 Hz, 1H), 6.66 (d, *J* = 2.6 Hz, 1H), 4.63 - 4.57 (m, 2H), 4.55 - 4.47 (m, 2H), 4.15 (s, 2H), 3.83 - 3.67 (m, 2H), 2.83 - 2.39 (m, 4H), 2.31 (s, 3H), 2.22 - 2.08 (m, 6H), 1.80 - 1.69 (m, 1H), 1.27 (s, 3H), 0.67 - 0.55 (m, 2H), 0.26 - 0.11 (m, 2H). LCMS (m/z): 620.0 (M + H).

| Examples | Structure | Name | CHARACTERIZATION DATA |
|---|---|---|---|
| 30 | 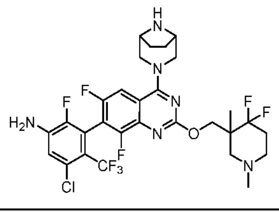 | 3-(4-((1*R*,5*S*)-3,8-diazabicyclo[3.2.1]octan-3-yl)-2-((4,4-difluoro-1,3-dimethylpiperidin-3-yl]methoxy)-6,8-difluoroquinolin-7-yl)-5-chloro-2-fluoro-4-(trifluoromethyl)aniline | LCMS (m/z): 665.2 (M + H). |
| 30-1 | 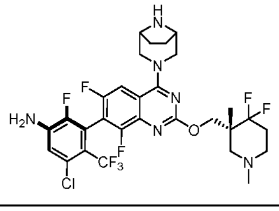 | (*Sa*)-3-(4-((1*R*,5*S*)-3,8-diazabicyclo[3.2.1]octan-3-yl)-2-(((*R*)-4,4-difluoro-1,3-dimethylpiperidin-3-yl)methoxy)-6,8-difluoroquinazolin-7-yl)-5-chloro-2-fluoro-4-(trifluoromethyl) aniline | LCMS (m/z): 665.2 (M + H). |

(continued)

| Examples | Structure | Name | CHARACTERIZATION DATA |
|---|---|---|---|
| 30-2 | 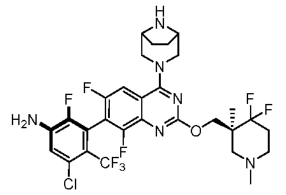 | (*Sa*)-3-(4-((1*R*,5*S*)-3,8-diazabicyclo[3.2.1]octan-3-yl)-2-(((*S*)-4,4-difluoro-1,3-dimethylpiperidin-3-yl)methoxy)-6,8-difluoroquinazolin-7-yl)-5-chloro-2-fluoro-4-(trifluoromethyl) aniline | LCMS (m/z): 665.2 (M + H). |

**Example 30-3**

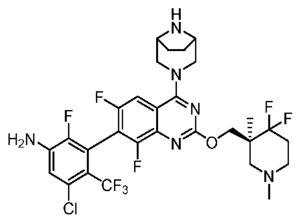

3-(4-((1*R*,5*S*)-3,8-diazabicyclo[3.2.1]octan-3-yl)-2-(((*S*)-4,4-difluoro-1,3-dimethylpiperidin-3-yl)methoxy)-6,8-difluoroquinazolin-7-yl)-5-chloro-2-fluoro-4-(trifluoromethyl)aniline

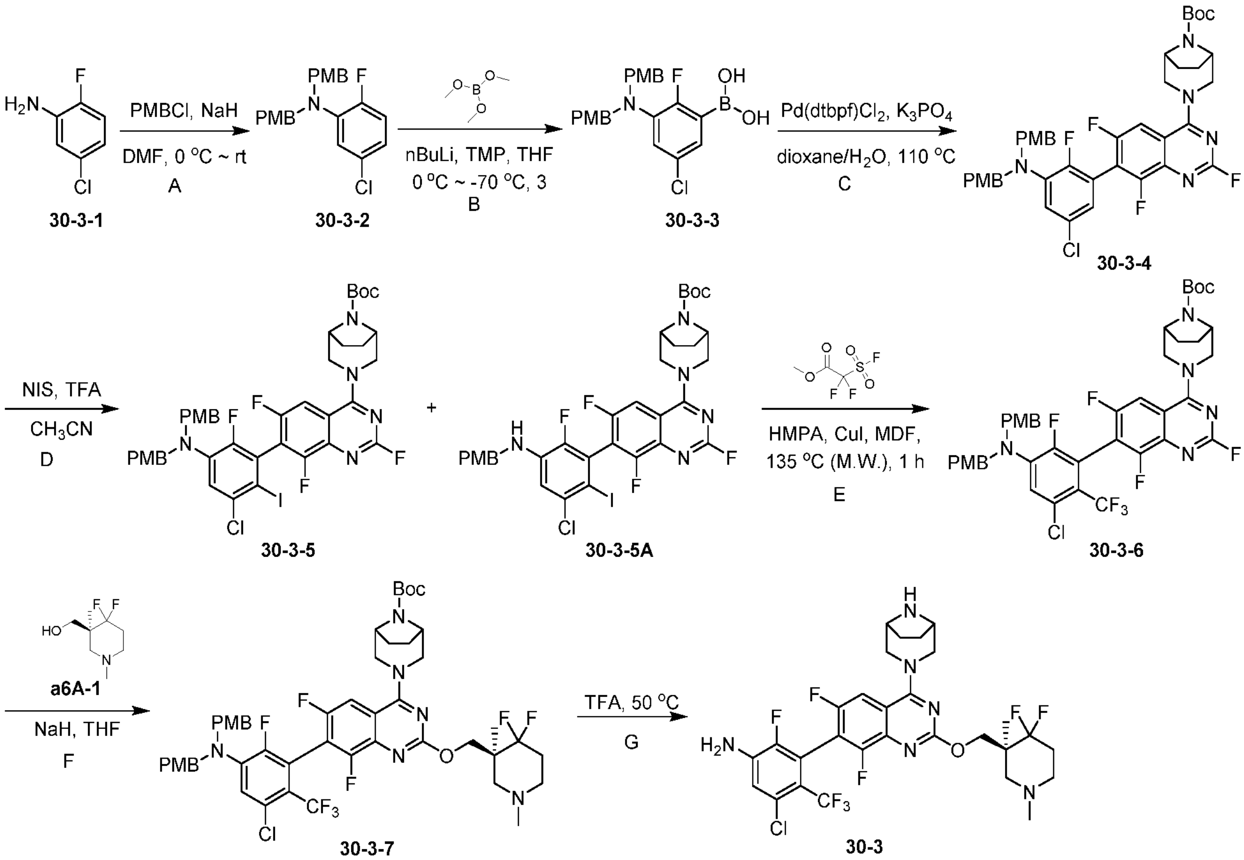

**Step A:** 5-chloro-2-fluoro-N,N-bis(4-methoxybenzyl)aniline

Sodium hydride (3.4 g, 85.8 mmol) and PMBCl (13.5 g, 85.8 mmol) were successively added to a solution of 5-chloro-2-fluoroaniline (5.0 g, 34.4 mmol) in DMF (50 mL) at 0 °C. The reaction was stirred at room temperature overnight. After the reaction was completed (monitored by LCMS), the reaction solution was poured into 100 mL of ice water to quench the reaction. The mixture was extracted with ethyl acetate. The combined organic phases were washed with saturated brine, dried over anhydrous sodium sulfate, filtered, and concentrated to dryness. The resulting crude product was purified by FCC ($SiO_2$, EtOAc/PE = 0 - 10%) to obtain 5-chloro-2-fluoro-N,N-bis(4-methoxybenzyl)aniline (7.8 g, yield 59%) as a brown oil. LCMS (m/z): 386.1 (M + H).

**Step B:** (3-(bis(4-methoxybenzyl)amino)-5-chloro-2-fluorophenyl)boric acid

To a solution of 2,2,6,6-tetramethylpiperidine (5.7 g, 40.0 mmol) in tetrahydrofuran (25 mL), n-butyllithium (16 mL, 40.0 mmol, 2.5 M in n-hexane) was added at -70 °C and the reaction was stirred for 20 min. Then, a solution of 5-chloro-2-fluoro-*N,N*-bis(4-methoxybenzyl)aniline (7.8 g, 20.0 mmol) in tetrahydrofuran was added to the above solution, and the resulting mixture was stirred at -70 °C for 1 h. Then trimethyl borate (4.2 g, 40.0 mmol) was added, and the resulting mixture was stirred at -70 °C for 1 h. After the reaction was completed (monitored by LCMS), the reaction solution was poured into 80 mL of saturated aqueous ammonium chloride solution to quench the reaction. The mixture was extracted with ethyl acetate. The combined organic phases were washed with saturated brine, dried over anhydrous sodium sulfate, filtered, and concentrated to dryness. The resulting crude product was purified by FCC ($SiO_2$, EtOAc/PE = 30 - 100%) to obtain (3-(bis(4-methoxybenzyl)amino)-5-chloro-2-fluorophenyl)boric acid (2.4 g, yield 28%) as a yellow solid. $^1$H NMR (400 MHz, DMSO-$d_6$) δ 7.26 - 7.13 (m, 4H), 7.03 - 6.65 (m, 6H), 4.19 (s, 4H), 3.75 - 3.63 (m, 6H), 3.17 (s, 2H). LCMS (m/z): 430.2 (M + H).

**Step C:** *tert*-butyl (1*R*,5*S*)-3-(7-(3-(bis(4-methoxybenzyl)amino)-5-chloro-2-fluorophenyl)-2,6,8-trifluoroquinazolin-4-yl)-3,8-diazabicyclo[3.2.1]octane-8-carboxylate

Under $N_2$, *tert*-butyl (1*R*,5*S*)-3-(7-bromo-2,6,8-trifluoroquinazolin-4-yl)-3,8-diazabicyclo[3.2.1]octane-8-carboxylate (2.2 g, 4.65 mmol), potassium phosphate (1.97 g, 9.3 mmol), and Pd(dtbpf)$Cl_2$ (537 mg, 0.46 mmol) were added to a solution of (3-(bis(4-methoxybenzyl)amino)-5-chloro-2-fluorophenyl) boric acid (2.4 g, 5.58 mmol) in 1,4-dioxane (20 mL) and water (4 mL). The resulting mixture was stirred at 110 °C for 2h. After the reaction was completed (monitored by LCMS), the reaction was filtered through celite, and the filtrate was concentrated to dryness. The resulting crude product was purified by FCC ($SiO_2$, EA/PE = 0 - 15%) to give *tert*-butyl (1R,5S)-3-(7-(3-(bis(4-methoxybenzyl)amino)-5-chloro-2-fluorophenyl)-2,6,8-trifluoroquinazolin-4-yl)-3,8-diazabicyclo[3.2.1]octane-8-carboxylate (1.9 g, yield 52%) as a yellow solid. $^1$H NMR (400 MHz, DMSO-$d_6$) δ 7.96 - 7.83 (m, 1H), 7.26 - 7.18 (m, 4H), 7.13 - 7.04 (m, 2H), 6.94 - 6.81 (m, 4H), 4.48 - 4.21 (m, 8H), 3.70 - 3.52 (m, 2H), 1.84 - 1.62 (m, 4H), 1.46 (s, 9H). LCMS (m/z): 778.3 (M + H).

**Step D:** tert-butyl (1R,5S)-3-(7-(3-(bis(4-methoxybenzyl)amino)-5-chloro-2-fluoro-6-iodophenyl)-2,6,8-trifluoroquinazoline-4-yl)-3,8-diazabicyclo[3.2.1]octane-8-carboxylate and *tert*-butyl (1*R*,5*S*)-3-(7-(3-((4-methoxybenzyl)amino)-5-chloro-2-fluoro-6-iodophenyl)-2,6,8-trifluoroquinazolin-4-yl)-3,8-diazabicyclo[3.2.1]octane-8-carboxylate

At room temperature, trifluoroacetic acid (21 mg, 0.18 mmol) was added to a solution of *tert*-butyl (1*R*,5*S*)-3-(7-(3-(bis(4-methoxybenzyl)amino)-5-chloro-2-fluorophenyl)-2,6,8-trifluoroquinazolin-4-yl)-3,8-diazabicyclo [3.2.1]octane-8-carboxylate (1.4g, 1.8 mmol) and NIS (1.62g, 7.2 mmol) in acetonitrile (20 mL). The resulting mixture was stirred at room temperature for 2h. After the reaction was completed (monitored by LCMS), water (50 mL) was added, and the resulting mixture was extracted with ethyl acetate. The combined organic phase was washed with brine, dried over anhydrous sodium sulfate, filtered, and concentrated to dryness. The resulting crude product was purified by FCC ($SiO_2$, EA/PE = 0 - 20%) to obtain a yellow solid mixture of *tert-butyl* (1*R*,5*S*)-3-(7-(3-(bis(4-methoxybenzyl)amino)-5-chloro-2-fluoro-6-iodophenyl)-2,6,8-trifluoroquinazolin-4-yl)-3,8-diazabicyclo[3.2.1]octane-8-carboxylate and *tert-butyl (1R,5S)-3-(7-*(3-((4-methoxybenzyl)amino)-5-chloro-2-fluoro-6-iodophenyl)-2,6,8-trifluoroquinazolin-4-yl)-3,8-diazabicyclo[3.2.1]octane-8-carboxylate (1.1 g). LCMS (m/z): 904.3 & 784.2 (M + H).

**Step E:** *tert-butyl* (1R,5S)-3-(7-(3-(bis(4-methoxybenzyl)amino)-5-chloro-2-fluoro-6-(trifluoromethyl)phenyl)-2,6,8-trifluoroquinazolin-4-yl)-3,8-diazabicyclo[3.2.1]octane-8-carboxylate

Under $N_2$, methyl 2,2-difluoro-2-(fluorosulfonyl)acetate (159 mg, 0.83 mmol), cuprous iodide (16 mg, 0.08 mmol), and hexamethylphosphotriamine (89 mg, 0.49 mmol) were added to a solution of *tert*-butyl (1*R*,5*S*)-3-(7-(3-(bis(4-methoxybenzyl)amino)-5-chloro-2-fluoro-6-iodophenyl)-2,6,8-trifluoroquinazolin-4-yl)-3,8-diazabicyclo[3.2.1]octane-8-carboxylate and *tert-butyl (1R,5S)-3-(7*-(3-((4-methoxybenzyl)amino)-5-chloro-2-fluoro-6-iodophenyl)-2,6,8-trifluoroquinazolin-4-yl)-3,8-diazabicyclo[3.2.1]octane-8-carboxylate (150 mg) in DMF (3 mL). The reaction was heated at 135°C under microwave for 1 h. After the reaction was completed (monitored by LCMS), the reaction was poured into 30 mL of ice-cold water, and the resulting mixture was extraction with ethyl acetate. The combined organic phase was washed with brine, dried over anhydrous sodium sulfate, filtered, and concentrated to dryness. The resulting crude product was purified by pre-TLC (EtOAc/PE = 1/3) to give *tert*-butyl (1*R*,5*S*)-3-(7-(3-(bis(4-methoxybenzyl)amino)-5-chloro-2-fluoro-6-(trifluoromethyl)phenyl)-2,6,8-trifluoroquinazolin-4-yl)-3,8-diazabicyclo[3.2.1]octane-8-carboxylate (60 mg) as a yellow solid. LCMS (m/z): 846.0 (M + H).

**Step F:** *tert*-butyl (1*R*,5*S*)-3-(7-(3-(bis(4-methoxybenzyl)amino)-5-chloro-2-fluoro-6-(trifluoromethyl)phenyl)-2-(((*S*)-4,4-difluoro-1,3-dimethylpiperidin-3-yl)methoxy)-6,8-difluoroquinazolin-4-yl)-3,8-diazabicyclo[3.2.1]octane-8-carboxylate

(*S*)-(4,4-difluoro-1,3-dimethylpiperidin-3-yl)methanol (26 mg, 0.14 mmol) was dissolved into anhydrous tetrahydrofuran (2 mL) in an ice bath, then sodium hydride (6 mg, 0.14 mmol) was added and the reaction was stirred at the same temperature for 0.5 h. *tert*-butyl (1*R*,5*S*)-3-(7-(3-(bis(4-methoxybenzyl)amino)-5-chloro-2-fluoro-6-(trifluoromethyl) phenyl)-2,6,8-trifluoroquinazolin-4-yl)-3,8-diazabicyclo[3.2.1]octane-8-carboxylate (60 mg, 0.07 mmol) was added to the reaction. The reaction was stirred at room temperature for 1 h. After the reaction was completed (monitored by LCMS), the reaction solution was poured into 30 mL of ice water to quench the reaction. The mixture was extracted with ethyl acetate. The combined organic phases were washed with saturated brine, dried over anhydrous sodium sulfate, filtered, and concentrated to dryness to obtain *tert*-butyl (1*R*,5*S*)-3-(7-(3-(bis(4-methoxybenzyl)amino)-5-chloro-2-fluoro-6-(trifluoromethyl)phenyl)-2-(((*S*)-4,4-difluoro-1,3-dimethylpiperidin-3-yl)methoxy)-6,8-difluoroquinazolin-4-yl)-3,8-diazabicyclo [3.2.1]octane-8-carboxylate (50 mg, yield 70%). LCMS (m/z): 885.4 (M + H).

**Step G:** 3-(4-((1*R*,5*S*)-3,8-diazabicyclo[3.2.1]octan-3-yl)-2-(((*S*)-4,4-difluoro-1,3-dimethylpiperidin-3-yl)methoxy)-6,8-Difluoroquinazolin-7-yl)-5-chloro-2-fluoro-4-(trifluoromethyl)aniline

Trifluoroacetic acid (2 mL) was added to *tert-butyl* (1*R*,5*S*)-3-(7-(3-(bis(4-methoxybenzyl)amino)-5-chloro-2-fluoro-6-(trifluoromethyl)phenyl)-2-(((*S*)-4,4-difluoro-1,3-dimethylpiperidin-3-yl)methoxy)-6,8-difluoroquinazolin-4-yl)-3,8-diazabicyclo[3.2.1]octane-8-carboxylate (50 mg, 0.05 mmol). The reaction was stirred at 50 °C for 0.5 h. After the reaction was completed (monitored by LCMS), the reaction was purified by pre-HPLC (C18, CAN/(10 mmol $NH_4HCO_3/H_2O$) = 20-50%) to obtain 3-(4-((1*R*,5*S*)-3,8-diazabicyclo[3.2.1]octan-3-yl)-2-(((*S*)-4,4-difluoro-1,3-dimethylpiperidin-3-yl)methoxy)-6,8-difluoroquinazolin-7-yl)-5-chloro-2-fluoro-4-(trifluoromethyl)aniline (12.8 mg, yield 38%) as a white solid. $^1$H NMR (400 MHz, DMSO-$d_6$) δ 7.73 (d, *J* = 10.3 Hz, 1H), 7.14 (d, *J* = 8.0 Hz, 1H), 6.55 (s, 2H), 4.54 - 4.04 (m, 6H), 3.50 - 3.25 (m, 6H), 2.18 (s, 3H), 2.13 - 2.01 (m, 2H), 1.80 - 1.54 (m, 4H), 1.17 (s, 3H). $^{19}$F NMR (376 MHz, DMSO-$d_6$) δ-54.22,-106.35,-119.89,-123.79,-133.94. LCMS (m/z): 665.3 (M + H).

| Examples | Structure | Name | Characterization data |
|---|---|---|---|
| 31 | 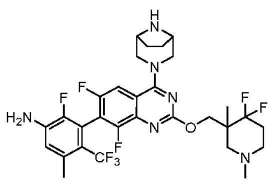 | 3-(4-((1*R*,5*S*)-3,8-diazabicyclo [3.2.1]octan-3-yl)-2-((4,4-difluoro-1,3-dimethylpiperidin-3-yl)methoxy)-6,8-difluoroquinolin-7-yl)-2-fluoro-5-methyl-4-(trifluoromethyl)aniline | LCMS (m/z): 645.2 (M + H). |
| 31-1 | 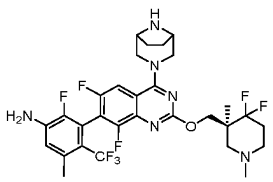 | 3-(4-((1*R*,5*S*)-3,8-diazabicyclo [3.2.1]octan-3-yl)-2-(((*S*)-4,4-difluoro-1,3-dimethylpiperidin-3-yl)methoxy)-6,8-difluoroquinolin-7-yl)-2-fluoro-5-methyl-4-(trifluoromethyl)aniline | LCMS (m/z): 645.3 (M + H). $^1$H NMR (400 MHz, DMSO-$d_6$) δ 7.68 (d, *J* = 10.2 Hz, 1H), 6.86 (d, *J* = 8.9 Hz, 1H), 6.07 (s, 2H), 4.50 - 4.40 (m, 2H), 4.29 - 4.20 (m, 2H), 3.57 - 3.43 (m, 4H), 2.46 - 2.25 (m, 7H), 2.18 (s, 3H), 2.15 - 2.01 (m, 2H), 1.70 - 1.57 (m, 4H), 1.20 - 1.11 (m, 3H). |

(continued)

| Examples | Structure | Name | Characterization data |
|---|---|---|---|
| 32 | 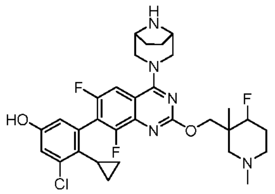 | 3-(4-((1*R*,5*S*)-3,8-diazabicyclo[3.2.1]octan-3-yl)-6,8-difluoro-2-((4-fluoro-1,3-dimethylpiperidin-3-yl)methoxy)quinazolin-7-yl)-5-chloro-4-cyclopropylphenol | LCMS (m/z): 602.2 (M + H). |
| 33 | 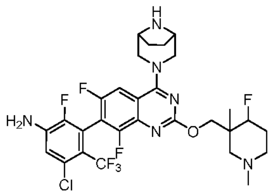 | 3-(4-((1*R*,5*S*)-3,8-diazabicyclo[3.2.1]octan-3-yl)-6,8-difluoro-2-((4-fluoro-1,3-dimethylpiperidin-3-yl)methoxy)quinazolin-7-yl)-5-chloro-2-fluoro-4-(trifluoromethyl)aniline | LCMS (m/z): 647.2 (M + H). |
| 33-1 | 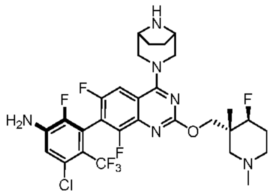 | (*Sa*)-3-(4-((1*R,*5*S*)-3,8-diazabicyclo[3.2.1]octan-3-yl)-6,8-difluoro-2-(((3*R*,4*S*)-4-fluoro-1,3-dimethylpiperidin-3-yl)methoxy)quinazolin-7-yl)-5-chloro-2-fluoro-4-(trifluoromethyl)aniline | LCMS (m/z): 647.2 (M + H). |
| 33-2 | 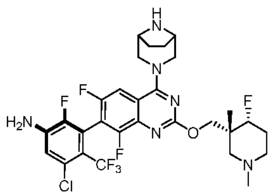 | (*Sa*)-3-(4-((1*R*,5*S*)-3,8-diazabicyclo[3.2.1]octan-3-yl)-6,8-difluoro-2-(((3*R*,4*R*)-4-fluoro-1,3-dimethylpiperidin-3-yl)methoxy)quinazolin-7-yl)-5-chloro-2-fluoro-4-(trifluoromethyl)aniline | LCMS (m/z): 647.2 (M + H). |
| 33-3 | 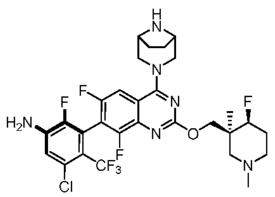 | (*Sa*)-3-(4-((1*R,5*S)-3,8-diazabicyclo[3.2.1]octan-3-yl)-6,8-difluoro-2-(((3*S*,4*S*)-4-fluoro-1,3-dimethylpiperidin-3-yl)methoxy)quinazolin-7-yl)-5-chloro-2-fluoro-4-(trifluoromethyl)aniline | LCMS (m/z): 647.2 (M + H). |
| 33-4 | 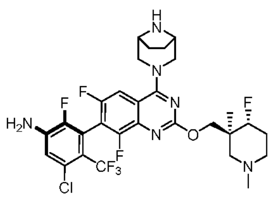 | (*Sa*)-3-(4-((1*R,*5S)-3,8-diazabicyclo[3.2.1]octan-3-yl)-6,8-difluoro-2-(((3*S*,4*R*)-4-fluoro-1,3-dimethylpiperidin-3-yl)methoxy)quinazolin-7-yl)-5-chloro-2-fluoro-4-(trifluoromethyl)aniline | **LCMS** (m/z): 647.2 (M + H). |
| 34 | 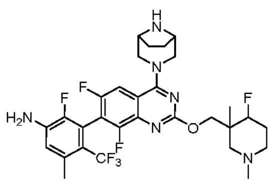 | 3-(4-((1*R*,5*S*)-3,8-diazabicyclo[3.2.1]octan-3-yl)-6,8-difluoro-2-((4-fluoro-1,3-dimethylpiperidin-3-yl)methoxy)quinazolin-7-yl)-2-fluoro-5-methyl-4-(trifluoromethyl)aniline | LCMS (m/z): 627.2 (M + H). |

(continued)

| Examples | Structure | Name | Characterization data |
|---|---|---|---|
| 34-1 | 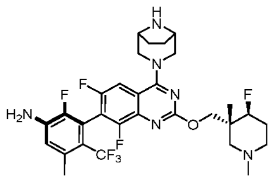 | (*Sa*)-3-(4-((1*R*,5*S*)-3,8-diazabicyclo[3.2.1]octan-3-yl)-6,8-difluoro-2-(((3*R*,4*S*)-4-fluoro-1,3-dimethylpiperidin-3-yl methoxy)quinazolin-7-yl)-2-fluoro-5-methyl-4-(trifluoromethyl)aniline | LCMS (m/z): 627.2 (M + H). |
| 34-2 | 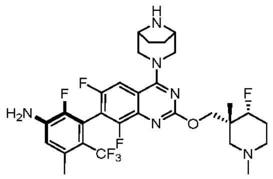 | (*Sa*)-3-(4-((1*R*,5*S*)-3,8-diazabicyclo[3.2.1]octan-3-yl)-6,8-difluoro-2-(((3*R*,4*R*)-4-fluoro-1,3-dimethylpiperidin-3-yl)methoxy)quinazolin-7-yl)-2-fluoro-5-methyl-4-(trifluoromethyl)aniline | LCMS (m/z): 627.2 (M + H). |
| 34-3 | 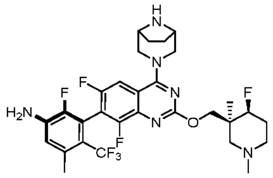 | (*Sa*)-3-(4-((1*R*,5*S*)-3,8-diazabicyclo[3.2.1]octan-3-yl)-6,8-difluoro-2-(((3*S*,4*S*)-4-fluoro-1,3-dimethylpiperidin-3-yl)methoxy)quinazolin-7-yl)-2-fluoro-5-methyl-4-(trifluoromethyl)aniline | **LCMS** (m/z): 627.2 (M + H). |
| 34-4 | 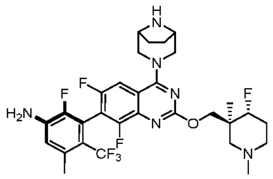 | (*Sa*)-3-(4-((1*R*,5*S*)-3,8-diazabicyclo[3.2.1]octan-3-yl)-6,8-difluoro-2-(((3*S*,4*R*)-4-fluoro-1,3-dimethylpiperidin-3-yl)methoxy)quinazolin-7-yl)-2-fluoro-5-methyl-4-(trifluoromethyl)aniline | LCMS (m/z): 627.2 (M + H). |
| 35 | 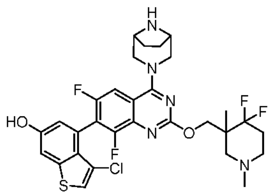 | 4-(4-((1*R*,5*S*)-3,8-diazabicyclo[3.2.1]octan-3-yl)-2-((4,4-difluoro-1,3-dimethylpiperidin-3-yl)methoxy)-6,8-difluoroquinolin-7-yl)-3-chlorobenzo[b]thiophen-6-ol | LCMS (m/z): 636.2 (M + H). |
| 36 | 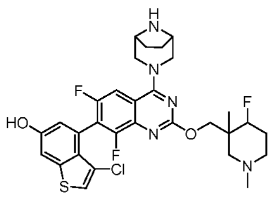 | 4-(4-((1*R*,5*S*)-3,8-diazabicyclo[3.2.1]octan-3-yl)-6,8-difluoro-2-((4-fluoro-1,3-dimethylpiperidin-3-yl)methoxy) quinazolin-7-yl)-3-chlorobenzo[*b*]thiophen-6-ol | LCMS (m/z): 618.2 (M + H). |
| 37 | 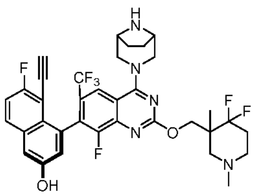 | (*Ra*)-4-(4-((1*R,5*S)-3,8-diazabicyclo[3.2.1]octan-3-yl)-2-((4,4-difluoro-1,3-dimethylpiperidin-3-yl)methoxy)-8-fluoro-6-(trifluoromethyl)quinazolin-7-yl)-5-ethynyl-6-fluoronaphthalen-2-ol | **LCMS** (m/z): 688.2 (M + H). |

(continued)

| Examples | Structure | Name | Characterization data |
|---|---|---|---|
| 38 | 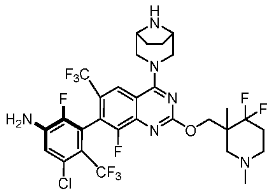 | (*Sa*)-(3*S*)-3-(4-((1*R*,5*S*)-3,8-diazabicyclo[3.2.1]octan-3-yl)-2-((4,4-difluoro-1,3-dimethylpiperidin-3-yl)methoxy)-8-fluoro-6-(trifluoromethyl)quinazolin-7-yl)-5-chloro-2-fluoro-4-(trifluoromethyl)aniline | LCMS (m/z): 715.2 (M + H). |

**Example 39-1 and example 39-2**

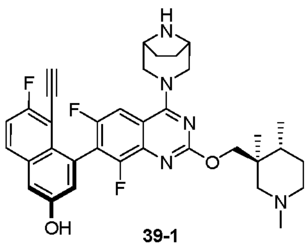

39-1

and

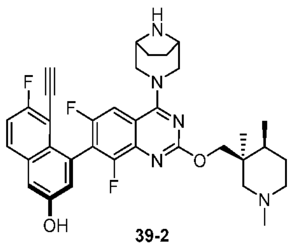

39-2

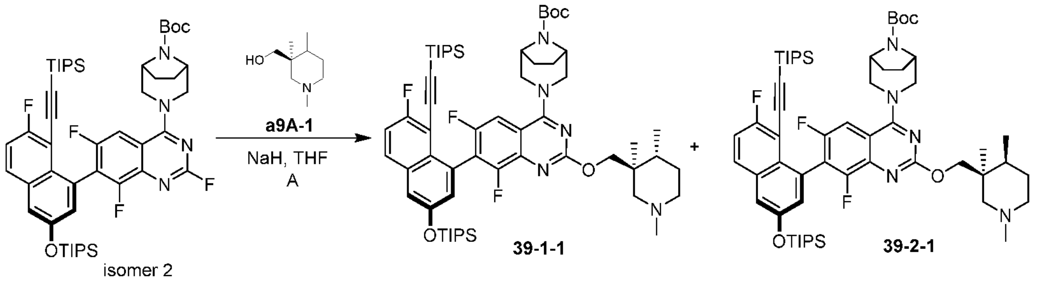


isomer 2

A-1b-I2

39-1-1

39-2-1

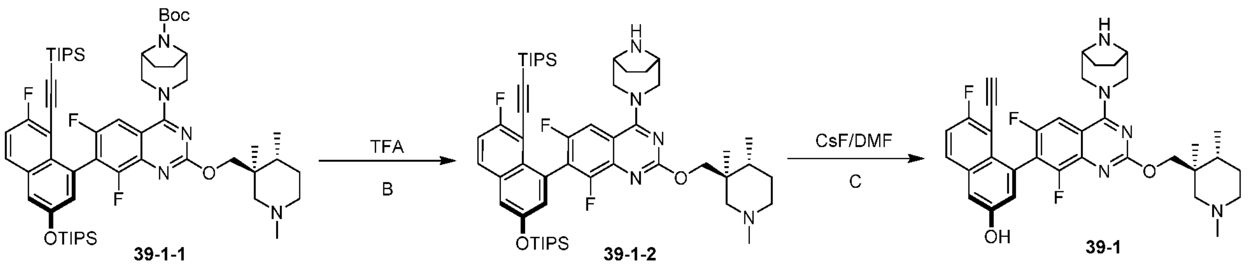


39-1-1

39-1-2

39-1

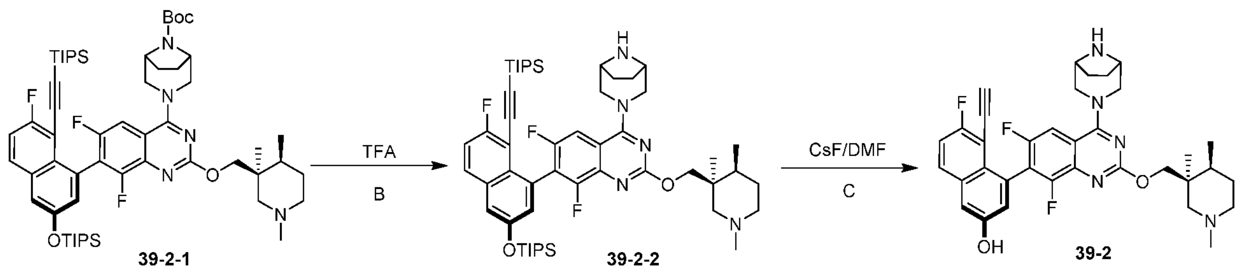


39-2-1

39-2-2

39-2

**Step A: Synthesis of compounds 39-1-1 and 39-2-1**

NaH (27.0 mg, 0.674 mmol, 60%) was added to a mixture of (3*S*)-(1,3,4-trimethylpiperidin-3-yl)methanol (Intermediate **a9A-1,** 79.4 mg, 0.505 mmol) in THF (5 mL) in an ice bath, and the reaction was stirred for 0.5 h at the same temperature. Then, *tert*-butyl (*R*a)-(1*R,5*S)-3-(2,6,8-trifluoro-7-(7-fluoro-8-(triisopropylsilyl)ethynyl)-3-((triisopropylsilyl)oxy)naphthalen-1-yl)quinazolin-4-yl)-3,8-diazabicyclo[3.2.1]octane-8-carboxylate (Intermediate **A-1b-I2,** 300 mg, 0.357 mmol) was added. The resulting mixture was stirred at room temperature for 1 h. After the reaction was completed (monitored by LCMS), the reaction was poured into saturated aqueous ammonium chloride (50 mL). The resulting mixture was extracted with EA (50 mL × 2). The combined organic phase was washed with brine (30 mL), concentrated and further purified by FCC ($SiO_2$, MeOH/DCM = 0 ~ 5%) to obtain **39-1-1** (90 mg, yield 26%) as a white solid. LCMS (m/z): 514.9 (M/2 + H); and **39-2-1** (120 mg, yield 35%) as a white solid. LCMS (m/z): 514.9 (M/2 + H).

**Step B:** Synthesis of compounds **39-1-2** and **39-2-2**

Compound **39-1-1** (90 mg, 0.088 mmol) was dissolved in TFA (10 mL) at room temperature, and the resulting mixture stirred for 0.5 h at room temperature. After the reaction was completed (monitored by LCMS), the reaction was concentrated, and saturated aqueous sodium bicarbonate (50 mL) was added. The reaction was extracted with EA (50 mL × 2). The combined organic phase was washed with brine (30 mL), dried over anhydrous $Na_2SO_4$, filtered, and concentrated under reduced pressure to give **39-1-2** (60 mg, yield 74%) as a brown solid. LCMS (m/z): 928.6 (M + H).

Using the same method as described above, compound **39-2-2** (71 mg, yield 66%) as a brown solid was prepared from compound **39-2-1** (120 mg, 0.129 mmol). LCMS (m/z): 928.6 (M + H).

**Step C:** Syntheses of compounds 39-1 and 39-2

CsF (49.1 mg, 0.32 mmol) was added to a mixture of **39-1-2** (60 mg, 0.065 mmol) in DMF (2 mL) at room temperature. The reaction was heated to 50°C and stirred for 1h. After cooling to room temperature and filtration, the filtrate was purified by Pre-HPLC (basic system) to give **39-1** (14.1 mg, yield 37%) as a white solid. $^1$H NMR (400 MHz, DMSO-$d_6$) δ 10.21 (brs, 1H), 7.99 (dd, *J* = 9.2, 5.9 Hz, 1H), 7.54 (dd, *J* = 10.4, 1.6 Hz, 1H), 7.51 - 7.45 (m, 1H), 7.41 (d, *J* = 2.6 Hz, 1H), 7.15 (d, *J* = 2.5 Hz, 1H), 4.30 - 4.17 (m, 2H), 4.17 - 4.08 (m, 2H), 3.97 (s, 1H), 3.58 - 3.41 (m, 4H), 2.74 - 2.61 (m, 2H), 2.12 (s, 3H), 1.98 - 1.91 (m, 1H), 1.88 - 1.78 (m, 1H), 1.78 - 1.71 (m, 1H), 1.71 - 1.61 (m, 3H), 1.60 - 1.50 (m, J 1H), 1.49 - 1.37 (m, 2H), 0.94 (s, 3H), 0.83 (d, *J* = 6.7 Hz, 3H). $^{19}$F NMR (376 MHz, DMSO-$d_6$) δ-110.19,-119.24,-124.73. LCMS (m/z): 616.4 (M + H).

Using the same procedure described above, compound **39-2** (21.0 mg, yield 45%) as A white solid was prepared from compound **39-2-2** (71.0 mg, 0.076 mmol). $^1$H NMR (400 MHz, DMSO-$d_6$) δ 10.20 (brs, 1H), 7.99 (dd, *J* = 9.2, 5.9 Hz, 1H), 7.57 - 7.44 (m, 2H), 7.41 (d, *J* = 2.6 Hz, 1H), 7.15 (d, *J* = 2.6 Hz, 1H), 4.43 - 4.33 (m, 1H), 4.33 - 4.18 (m, 3H), 3.99 (s, 1H), 3.58 - 3.40 (m, 4H), 2.84 - 2.64 (m, 2H), 2.09 (s, 3H), 1.92 - 1.81 (m, 1H), 1.79 - 1.72 (m, 1H), 1.72 - 1.61 (m, 3H), 1.61 - 1.53 (m, 1H), 1.52 - 1.40 (m, 2H), 1.38 - 1.27 (m, 1H), 0.98 (s, 3H), 0.93 (d, *J* = 6.8 Hz, 3H). $^{19}$F NMR (376 MHz, DMSO-$d_6$) δ-110.20,-119.35,-124.69. LCMS (m/z): 616.4 (M + H).

**Example 40**

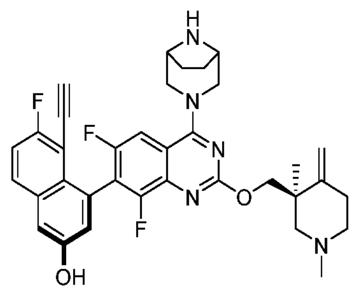

(*Ra*)-4-(4-((1*R*,5*S*)-3,8-diazabicyclo[3.2.1]octan-3-yl)-2-(((*S*)-1,3-dimethyl-4-methylenepiperidin-3-yl)methoxy)-6,8-difluoroquinazolin-7-yl)-5-ethynyl-6-fluoronaphthalen-2-ol

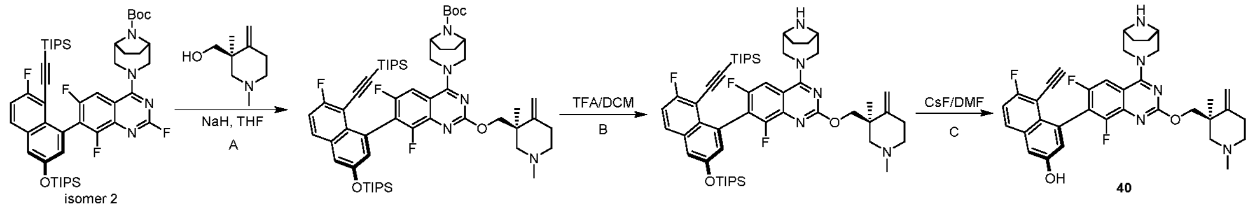


isomer 2
A-1b-I2

40

**Step A:** *tert*-butyl (*Ra*)-(1*R*,5*S*)-3-(2-(((*S*)-1,3-dimethyl-4-methylenepiperidin-3-yl)methoxy)-6,8-difluoro-7-(7-fluoro-8-((triisopropylsilyl)ethynyl)-3-(triisopropylsilyl)oxy)naphthalen-1-yl)quinazolin-4-yl)-3,8-diazabicyclo[3.2.1]octane-8-carboxylate

NaH (20 mg, 0.34 mmol, 60%) was added to a solution of (S)-(1,3-dimethyl-4-methylenepiperidin-3-yl)methanol (Intermediate **a10A-1,** 22 mg, 0.14 mmol) in anhydrous THF (2 mL), and the reaction was stirred at room temperature for 0.5 h. Then, *tert*-butyl (*Ra*)-(1*R*,5*S*)-3-(2,6,8-trifluoro-7-(7-fluoro-8-(triisopropylsilyl)ethynyl)-3-((triisopropylsilyl)oxy) naphthalen-1-yl)quinazolin-4-yl)-3,8-diazabicyclo[3.2.1]octane-8-carboxylate (intermediate **A-1b-I2,** 100 mg, 0.11 mmol) was added, and the resulting mixture was stirred at room temperature overnight. After the reaction was completed (monitored by TLC), the reaction was poured into saturated aqueous $NH_4Cl$ solution (5 mL), and the resulting mixture was extracted with EA (5 mL × 3). The combined organic phase was washed with brine (10 mL), and concentrated under reduced pressure to obtain *tert*-butyl (*Ra)*-(1*R*,5*S*)-3-(2-(((*S*)-1,3-dimethyl-4-methylenepiperidin-3-yl)methoxy)-6,8-difluoro-7-(7-fluoro-8-((triisopropylsilyl)ethynyl)-3-(triisopropylsilyl)oxy)naphthalen-1-yl)quinazolin-4-yl)-3,8-diazabicyclo [3.2.1]octane-8-carboxylate (100 mg, yield 89%) as a yellow solid, which was directly used in the subsequent reaction.

**Step B:** (*Ra*)-4-((1*R*,5*S*)-3,8-diazabicyclo[3.2.1]octan-3-yl)-2-(((S)-1,3-dimethyl-4-methylenepiperidin-3-yl)methoxy)-6,8-difluoro-7-(7-fluoro-8-((triisopropylsilyl)ethynyl)-3-((triisopropylsilyl)oxy)naphthalen-1-yl)quinazoline

At room temperature, $CF_3COOH$ (3 mL) was added to *tert*-butyl (*Ra*)-(1*R*,5*S*)-3-(2-(((*S*)-1,3-dimethyl-4-methylenepiperidin-3-yl)methoxy)-6,8-difluoro-7-(7-fluoro-8-((triisopropylsilyl)ethynyl)-3-(triisopropylsilyl)oxy)naphthalen-1-yl)quinazolin-4-yl)-3,8-diazabicyclo[3.2.1]octane-8-carboxylate (100 mg, 0.0975 mmol). The reaction was stirred for 1 h and concentrated then under reduced pressure to remove the acid solution. The resulting residue was diluted with EA (20 mL), and saturated $NaHCO_3$ solution (20 mL) was added to adjust pH ~ 8. Water (20 mL) was added, and the resulting mixture was extracted with EA (30 mL × 2). The combined organic phase was washed with brine and concentrated to afford (*Ra*)-4-((1*R*,5*S*)-3,8-diazabicyclo[3.2.1]octan-3-yl)-2-(((*S*)-1,3-dimethyl-4-methylenepiperidin-3-yl)methoxy)-6,8-difluoro-7-(7-fluoro-8-((triisopropylsilyl)ethynyl)-3-((triisopropylsilyl)oxy)naphthalen-1-yl)quinazoline (60 mg, yield 37%) as a yellow solid.

**Step C:** (*Ra*)-4-(4-((1*R*,5*S*)-3,8-diazabicyclo[3.2.1]octan-3-yl)-2-(((*S*)-1,3-dimethyl-4-methylenepiperidin-3-yl)methoxy)-6,8-difluoroquinazolin-7-yl)-5-ethynyl-6-fluoronaphthalen-2-ol

At room temperature, (*Ra*)-4-((1*R*,5*S*)-3,8-diazabicyclo[3.2.1]octan-3-yl)-2-(((*S*)-1,3-dimethyl-4-methylenepiperidin-3-yl)methoxy)-6,8-difluoro-7-(7-fluoro-8-((triisopropylsilyl)ethynyl)-3-((triisopropylsilyl)oxy)naphthalen-1-yl)quinazoline (60 mg , 0.065 mmol) and CsF (60 mg, 397 umol) were added to DMF (2 mL). The resulting mixture was heated to 50 °C and stirred for 1 h. After the reaction was completed (monitored by LCMS), insoluble material was removed by filtration and the filtrate was purified by Pre-HPLC to give (*Ra*)-4-(4-((1R,5S)-3,8-diazabicyclo[3.2.1]octan-3-yl)-2-(((*S*)-1,3-dimethyl-4-methylenepiperidin-3-yl)methoxy)-6,8-difluoroquinazolin-7-yl)-5-ethynyl-6-fluoronaphthalen-2-ol (3 mg, yield 8%) as a white solid. LCMS (m/z): 614.4 (M + H). $^1$H NMR (400 MHz, DMSO-$d_6$) δ 10.21 (s, 1H), 8.01 - 7.96 (m, 1H), 7.56 - 7.51 (m, 1H), 7.51 - 7.45 (m, 1H), 7.41 (d, *J* = 2.6 Hz, 1H), 7.14 (d, *J* = 2.6 Hz, 1H), 4.84 (s, 1H), 4.78 (s, 1H), 4.53 (d, *J* = 10.5 Hz, 1H), 4.28 (d, *J*= 10.6 Hz, 1H), 4.24 - 4.17 (m, 2H), 3.99 (s, 1H), 3.53 - 3.39 (m, 4H), 2.66 - 2.60 (m, 2H), 2.47 - 2.39 (m, 2H), 2.24 - 2.18 (m, 1H), 2.14 (s, 3H), 2.05 - 1.94 (m, 2H), 1.88 - 1.82 (m, 1H), 1.77 - 1.66 (m, 2H), 1.14 (s, 3H). $^{19}$F NMR (376 MHz, DMSO-$d_6$) δ-110.20,-119.29,-124.74.

| Examples | Structure | Name | Characterization data |
|---|---|---|---|
| 41 | 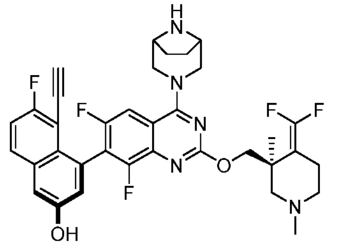 | (*Ra*)-4-(4-((1*R,5*S)-3,8-diazabicyclo[3.2.1]octan-3-yl)-2-(((*S*)-4-(difluoromethylene)-1,3-dimethylpiperidin-3-yl)methoxy)-6,8-difluoroquinazolin-7-yl)-5-ethynyl-6-fluoronaphthalen-2-ol | LCMS (m/z): 650.2 (M + H). |
| 42 | 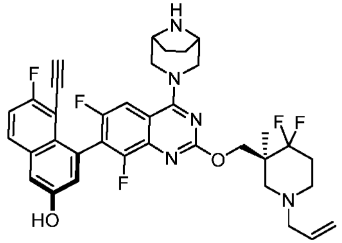 | (*Ra*)-4-(2-((*S*)-1-allyl-4,4-difluoro-3-methylpiperidin-3-yl)methoxy)-4-((1*R*,5*S*)-3,8-diazabicyclo[3.2.1]octan-3-yl)-6,8-difluoroquinazolin-7-yl)-5-ethynyl-6-fluoronaphthalen-2-ol | LCMS (m/z): 664.3 (M + H). $^1$H NMR (400 MHz, chloroform-d) δ 7.68 (dd, *J* = 9.1, 5.8 Hz, 1H), 7.31 - 7.27 (m, 2H), 7.23 - 7.13 (m, 2H), 5.71 - 5.50 (m, 1H), 4.92 (d, *J* = 17.0 Hz, 1H), 4.75 (d, *J* = 10.2 Hz, 1H), 4.61 - 4.25 (m, 5H), 3.80 - 3.55 (m, 4H), 2.93 - 2.80 (m, 2H), 2.78 (s, 1H), 2.72 - 2.31 (m, 2H), 2.20 - 1.67 (m, 7H), 1.30 (s, 3H). $^{19}$F NMR (376 MHz, Chloroform-*d*) δ-107.12,-107.78,-109.74,-116.25,-121.97. |
| 43 | 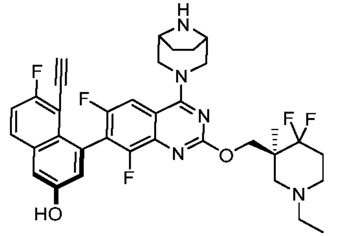 | (*Ra*)-4-(4-((1*R*,5*S*)-3,8-diazabicyclo[3.2.1]octan-3-yl)-2-(((*S*)-1-ethyl-4,4-difluoro-3-methylpiperidin-3-yl)methoxy)-6,8-difluoroquinazolin-7-yl)-5-ethynyl-6-fluoronaphthalen-2-ol | LCMS (m/z): 652.4 (M + H). $^1$H NMR (400 MHz, DMSO-$d_6$) δ 10.22 (s, 1H), 7.99 (dd, *J* = 9.2, 6.0 Hz, 1H), 7.61 - 7.53 (m, 1H), 7.53 - 7.45 (m, 1H), 7.41 (d, *J* = 2.6 Hz, 1H), 7.15 (d, *J* = 2.5 Hz, 1H), 4.55 - 4.37 (m, 2H), 4.32 - 4.16 (m, 2H), 3.99 (s, 1H), 3.68 - 3.41 (m, 4H), 2.68 - 2.58 (m, 2H), 2.45 - 2.26 (m, 4H), 2.19 - 1.95 (m, 2H), 1.85 - 1.55 (m, 4H), 1.17 (s, 3H), 0.95 (t, *J* = 7.1 Hz, 3H). $^{19}$F NMR (376 MHz, DMSO-$d_6$) δ-105.28,-105.91,-110.18,-119.05,-124.72. |
| 44 | 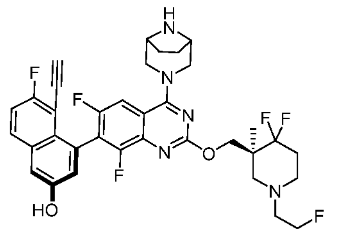 | (*Ra*)-4-(4-((1*R*,5*S*)-3,8-diazabicyclo[3.2.1]octan-3-yl)-2-(((*S*)-4,4-difluoro-1-(2-fluoroethyl)-3-methylpiperidin-3-yl)methoxy)-6,8-difluoroquinazolin-7-yl)-5-ethynyl-6-fluoronaphthalen-2-ol | LCMS (m/z): 670.3 (M + H). $^1$H NMR (400 MHz, DMSO-$d_6$) δ 10.24 (s, 1H), 7.99 (dd, *J* = 9.2, 5.9 Hz, 1H), 7.56 (dd, *J* = 10.4, 1.6 Hz, 1H), 7.52 - 7.45 (m, 1H), 7.42 (d, *J* = 2.6 Hz, 1H), 7.15 (d, *J* = 2.5 Hz, 1H), 4.56 (t, *J* = 4.8 Hz, 1H), 4.49 - 4.36 (m, 3H), 4.34 - 4.16 (m, 2H), 3.98 (s, 1H), 3.61 - 3.40 (m, 4H), 2.82 - 2.55 (m, 5H), 2.48 - 2.30 (m, 1H), 2.18 - 1.94 (m, 2H), 1.86 - 1.54 (m, 4H), 1.18 (s, 3H). $^{19}$F NMR (376 MHz, DMSO-$d_6$) δ-105.42,-106.06,-110.19,-119.05,-124.69,-216.86. |

(continued)

| Examples | Structure | Name | Characterization data |
|---|---|---|---|
| 45 | 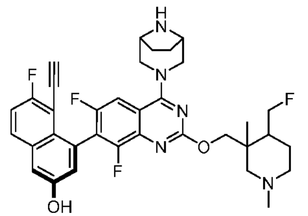 | (*Ra*)-4-(4-((1*R*,5*S*)-3,8-diazabicyclo[3.2.1]octan-3-yl)-6,8-difluoro-2-(4-(fluoromethyl)-1,3-dimethylpiperidin-3-yl)methoxy)quinazolin-7-yl)-5-ethynyl-6-fluoronaphthalen-2-ol | LCMS (m/z): 634.3 (M + H). |
| 45-1 | 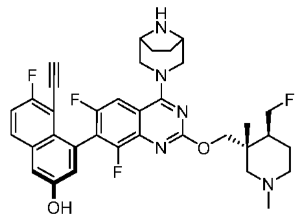 | (*Ra*)-4-(4-(1*R*,5*S*)-3,8-diazabicyclo[3.2.1]octan-3-yl)-6,8-difluoro-2-(3*R*,4*S*)-4-(fluoromethyl)-1,3-dimethylpiperidin-3-yl)methoxy) quinazolin-7-yl)-5-ethynyl-6-fluoronaphthalen-2-ol | LCMS (m/z): 634.3 (M + H). |
| 45-2 | 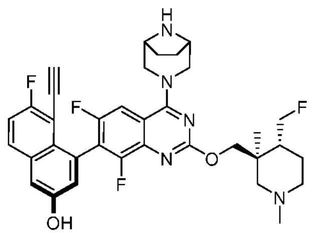 | (*Ra*)-4-(4-(1*R*,5S)-3,8-diazabicyclo[3.2.1]octan-3-yl)-6,8-difluoro-2-(3*S*,4*R*)-4-(fluoromethyl)-1,3-dimethylpiperidin-3-yl)methoxy) quinazolin-7-yl)-5-ethynyl-6-fluoronaphthalen-2-ol | LCMS (m/z): 634.3 (M + H). |
| 45-3 | 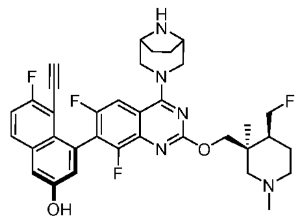 | (*Ra*)-4-(4-(1*R*,5S)-3,8-diazabicyclo[3.2.1]octan-3-yl)-6,8-difluoro-2-(3*S*,4*S*)-4-(fluoromethyl)-1,3-dimethylpiperidin-3-yl)methoxy) quinazolin-7-yl)-5-ethynyl-6-fluoronaphthalen-2-ol | LCMS (m/z): 634.3 (M + H). $^1$H NMR (400 MHz, DMSO-$d_6$) δ 10.30 (brs, 1H), 7.99 (dd, *J* = 9.2, 5.9 Hz, 1H), 7.57 - 7.45 (m, 2H), 7.41 (d, *J* = 2.5 Hz, 1H), 7.15 (d, *J* = 2.5 Hz, 1H), 4.77 - 4.71 (m, 0.5 H), 4.66 - 4.59 (m, 0.5 H), 4.53 - 4.47 (m, 0.5 H), 4.47 - 4.41 (m, 1H), 4.41 - 4.34 (m, 0.5 H), 4.31 - 4.16 (m, 3H), 3.98 (s, 1H), 3.55 - 3.40 (m, 4H), 2.86 - 2.71 (m, 2H), 2.10 (s, 3H), 1.88 - 1.50 (m, 8H), 1.06 (s, 3H). $^{19}$F NMR (376 MHz, DMSO-$d_6$) δ-110.21,-119.22,-124.59,-218.87. |

(continued)

| Examples | Structure | Name | Characterization data |
|---|---|---|---|
| 45-4 | 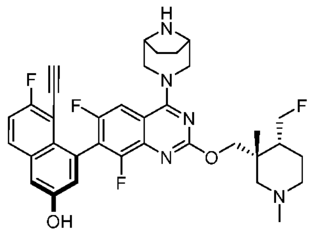 | (*Ra*)-4-(4-(1*R*,5*S*)-3,8-diazabicyclo[3.2.1]octan-3-yl)-6,8-difluoro-2-(3*R*,4*R*)-4-(fluoromethyl)-1,3-dimethylpiperidin-3-yl)methoxy)quinazolin-7-yl)-5-ethynyl-6-fluoronaphthalen-2-ol | LCMS (m/z): 634.3 (M + H). |
| 46 | 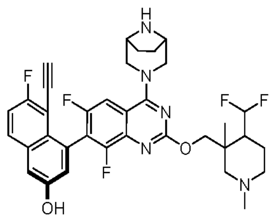 | (*Ra*)-4-(4-((1*R*,5*S*)-3,8-diazabicyclo[3.2.1]octan-3-yl)-2-((4-(difluoromethyl)-1,3-dimethylpiperidin-3-yl)methoxy)-6,8-difluoroquinazolin-7-yl)-5- | LCMS (m/z): 652.3 (M + H). |
| | | ethynyl-6-fluoronaphthalen-2-ol | |

**Examples 46-1 through 46-4**

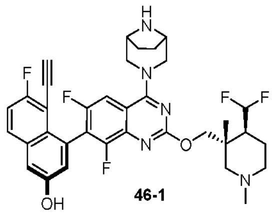
46-1,
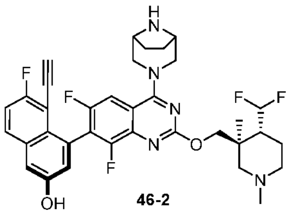
46-2,
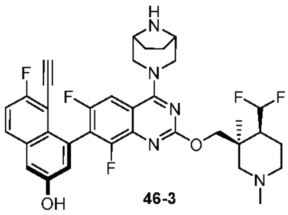
46-3 and
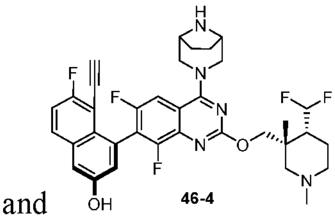
46-4

**Synthesis of 46-2 and 46-3:**

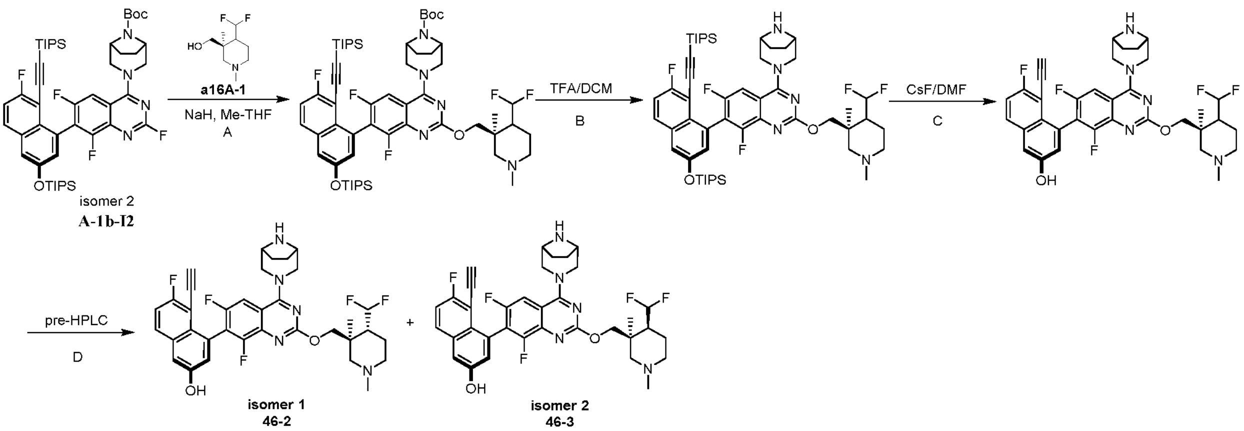


The synthesis of steps A ~ C was carried out by referring to the synthesis described in Example 10-2/10-4. In step A, ((3*S*)-4-(difluoromethyl)-1,3-Dimethylpiperidin-3-yl) methanol **(Intermediate a16A-1)** was used instead of (3S)-(4-fluoro-1,3-dimethylpiperidin-3-yl) methanol **(Intermediate a8A-1).**

Step D: (*Ra*)-4-(4-((1*R*,5*S*)-3,8-diazabicyclo[3.2.1]octan-3-yl)-2-(((3*S*,4*R*)-4-(difluoromethyl)-1,3-dimethylpiperidin-3-yl)methoxy)-6,8-difluoroquinazolin-7-yl)-5-ethynyl-6-fluoronaphthalen-2-ol and (*Ra*)-4-(4-((1*R*,5*S*)-3,8-diazabicyclo[3.2.1]octan-3-yl)-2-(((3*S*,4*S*)-4-(difluoromethyl)-1,3-dimethylpiperidin-3-yl)methoxy)-6,8-difluoroquinazolin-7-yl)-5-ethynyl-6-fluoronaphthalen-2-ol

The crude product (*Ra*)-4-(4-((1*R*,5*S*)-3,8-diazabicyclo[3.2.1]octan-3-yl)-2-(((3*S*,4*R*)-4-(difluoromethyl)-1,3-dimethylpiperidin-3-yl)methoxy)-6,8-difluoroquinazolin-7-yl)-5-ethynyl-6-fluoronaphthalen-2-ol obtained from Step C was subjected to Pre-HPLC (C18, CAN/(10 mmol $NH_4HCO_3/H_2O$) = 35-55%). The isomer 1 (with a shorter retention time) was compound 46-2 (19 mg), LCMS (m/z): 652.3 (M + H). $^1$H NMR (400 MHz, DMSO-$d_6$) δ 7.99 (dd, *J* = 9.3, 6.0 Hz, 1H), 7.59 - 7.52 (m, 1H), 7.51 - 7.45 (m, 1H), 7.41 (d, *J* = 2.6 Hz, 1H), 7.15 (d, *J* = 2.6 Hz, 1H), 6.36 - 6.02 (m, 1H), 4.28 - 4.15 (m, 4H), 3.97 (s, 1H), 3.55 - 3.48 (m, 3H), 3.46 - 3.43 (m, 1H), 2.81 - 2.75 (m, 1H), 2.49 - 2.46 (m, 1H), 2.14 (s, 3H), 2.07 - 1.94 (m, 2H), 1.89 - 1.79 (m, 1H), 1.78 - 1.68 (m, 2H), 1.68 - 1.58 (m, 4H), 1.09 (s, 3H). $^{19}$F NMR (376 MHz, DMSO-$d_6$) δ-110.21, -115.79, -119.11, -120.78, -124.72. The isomer 2 (with a longer retention time) was compound 46-3 (30 mg), LCMS (m/z): LCMS (m/z): 652.3 (M + H). $^1$H NMR (400 MHz, DMSO-$d_6$) δ 10.22 (s, 1H), 7.99 (dd, *J* = 9.2, 6.0 Hz, 1H), 7.58 - 7.52 (m, 1H), 7.52 - 7.45 (m, 1H), 7.41 (d, *J* = 2.6 Hz, 1H), 7.15 (d, *J* = 2.5 Hz, 1H), 6.44 - 6.13 (m, 1H), 4.46 - 4.39 (m, 1H), 4.38 - 4.31 (m, 1H), 4.30 - 4.19 (m, 2H), 3.99 (s, 1H), 3.56 - 3.48 (m, 3H), 3.47 - 3.42 (m, 1H), 2.87 - 2.78 (m, 2H), 2.11 (s, 3H), 1.89 - 1.76 (m, 2H), 1.74 - 1.54 (m, 7H), 1.11 (s, 3H). $^{19}$F NMR (376 MHz, DMSO-$d_6$) δ-110.19,-115.57,-118.92,-119.19,-124.59.

The syntheses of Examples 46-1 and 46-4 were carried out according to the above protocol.

| Examples | Structure | Name | Characterization data |
|---|---|---|---|
| 47 | 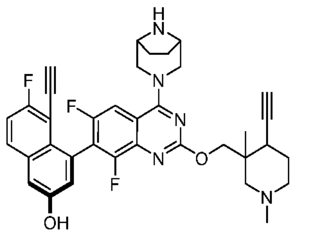 | (*Ra*)-4-(4-((1*R*,5S)-3,8-diazabicyclo[3.2.1]octan-3-yl)-2-(4-ethynyl-1,3-dimethylpiperidin-3-yl)methoxy)-6,8-difluoroquinazolin-7-yl)-5-ethynyl-6-fluoronaphthalen-2-ol | LCMS (m/z): 626.3 (M + H). |
| 47-1 | 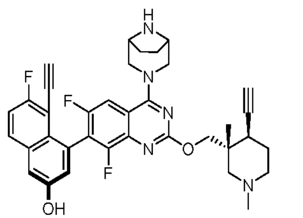 | (*Ra*)-4-(4-((1*R*,5*S*)-3,8-diazabicyclo[3.2.1]octan-3-yl)-2-(((3*R*,4*R*)-4-ethynyl-1,3-dimethylpiperidin-3-yl)methoxy)-6,8-difluoroquinazolin-7-yl)-5-ethynyl-6-fluoronaphthalen-2-ol | LCMS (m/z): 626.3 (M + H). |
| 47-2 | 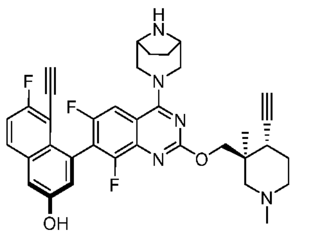 | (*Ra*)-4-(4-((1*R*,5*S*)-3,8-diazabicyclo[3.2.1]octan-3-yl)-2-(((3*S*,4*S*)-4-ethynyl-1,3-dimethylpiperidin-3-yl)methoxy)-6,8-difluoroquinazolin-7-yl)-5-ethynyl-6-fluoronaphthalen-2-ol | LCMS (m/z): 626.3 (M + H). |
| 47-3 | 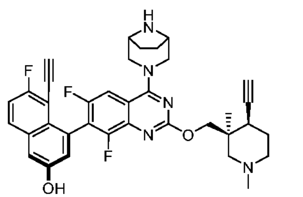 | (*Ra*)-4-(4-((1*R*,5*S*)-3,8-diazabicyclo[3.2.1]octan-3-yl)-2-(((3*S*,4*R*)-4-ethynyl-1,3-dimethylpiperidin-3-yl)methoxy)-6,8-difluoroquinazolin-7-yl)-5-ethynyl-6-fluoronaphthalen-2-ol | LCMS (m/z): 626.3 (M + H). LCMS (m/z): 626.3 (M + H). $^1$H NMR (400 MHz, DMSO-$d_6$) δ 10.23 (s, 1H), 7.99 (dd, *J* = 9.2, 5.9 Hz, 1H), 7.55 (d, *J* = 10.2 Hz, 1H), 7.52 - 7.46 (m, 1H), 7.42 (d, *J* = 2.5 Hz, 1H), 7.15 (d, *J* = 2.5 Hz, 1H), 4.45 - 4.20 (m, 3H), 4.19 - 4.10 (m, 1H), 3.99 (s, 1H), 3.63 - 3.55 (m, 2H), 3.55 - 3.44 (m, 2H), 3.00 - 2.96 (m, 1H), 2.67 - 2.53 (m, 2H), 2.13 (s, 3H), 2.09 - 1.87 (m, 2H), 1.85 - 1.57 (m, 7H), 1.12 (s, 3H). $^{19}$F NMR (376 MHz, DMSO-$d_6$) δ-110.18,-119.04,-124.65. |

(continued)

| Examples | Structure | Name | Characterization data |
|---|---|---|---|
| 47-4 | 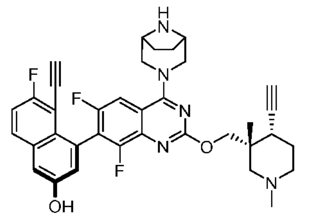 | (*Ra*)-4-(4-((*1R,5S*)-3,8-diazabicyclo[3.2.1]octan-3-yl)-2-(((3*R*,4*S)*-4-ethynyl-1,3-dimethylpiperidin-3-yl)methoxy)-6,8-difluoroquinazolin-7-yl)-5-ethynyl-6-fluoronaphthalen-2-ol | LCMS (m/z): 626.3 (M + H). |
| 48 | 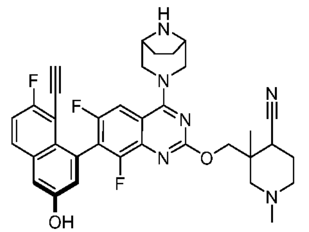 | (*Ra*)-3-(((4-((1*R*,S)-3,8-diazabicyclo[3.2.1]octan-3-yl)-7-(8-ethynyl-7-fluoro-3-hydroxynaphthalen-1-yl)-6,8-difluoroquinazolin-2-yl)oxy)methyl)-1,3-dimethylpiperidin-4-carbonitrile | LCMS (m/z): 627.3 (M + H). |
| 48-1 | 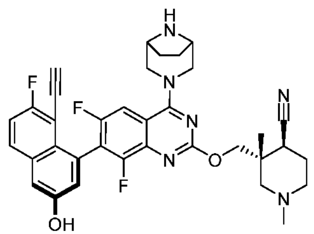 | (*Ra*)-(3*R*,4*S*)-3-(((4-((1*R*,5*S*)-3,8-diazabicyclo[3.2.1]octan-3-yl)-7-(8-ethynyl-7-fluoro-3-hydroxynaphthalen-1-yl)-6,8-difluoroquinazolin-2-yl)oxy)methyl)-1,3-dimethylpiperidin-4-carbonitrile | **LCMS** (m/z): 627.3 (M + H). |
| 48-2 | 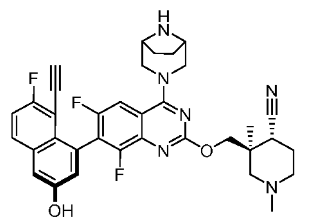 | (*Ra*)-(3*S,*4*R*)-3-(((4-((1*R*,5*S*)-3,8-diazabicyclo[3.2.1]octan-3-yl)-7-(8-ethynyl-7-fluoro-3-hydroxynaphthalen-1-yl)-6,8-difluoroquinazolin-2-yl)oxy)methyl)-1,3-dimethylpiperidin-4-carbonitrile | LCMS (m/z): 627.3 (M + H). |

(continued)

| Examples | Structure | Name | Characterization data |
|---|---|---|---|
| 48-3 | 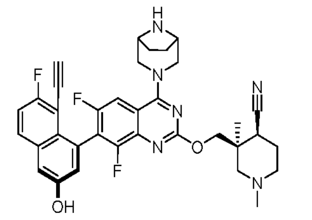 | (*Ra*)-(3*S*,4*S*)-3-(((4-((1*R*,5*S*)-3,8-diazabicyclo [3.2.1]octan-3-yl)-7-(8-ethynyl-7-fluoro-3-hydroxynaphthalen-1-yl)-6,8-difluoroquinazolin-2-yl) oxy)methyl)-1,3-dimethylpiperidin-4-carbonitrile | LCMS (m/z): 627.3 (M + H). |
| 48-4 | 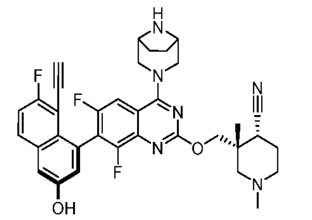 | (*Ra*)-(3*R*,4R)-3-(((4-((1*R*,5*S*)-3,8-diazabicyclo [3.2.1]octan-3-yl)-7-(8-ethynyl-7-fluoro-3-hydroxynaphthalen-1-yl)-6,8-difluoroquinazolin-2-yl) oxy)methyl)-1,3-dimethylpiperidin-4-carbonitrile | LCMS (m/z): 627.3 (M + H). |
| 49 | 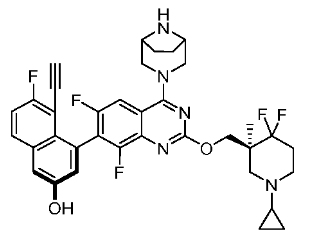 | (*Ra*)-4-(4-((1*R*,5*S*)-3,8-diazabicyclo[3.2.1]octan-3-yl)-2-(((*S*)-1-cyclopropyl-4,4-difluoro-3-methylpiperidin-3-yl)methoxy)-6,8-difluoroquinazolin-7-yl)-5-ethynyl-6-fluoronaphthalen-2-ol | LCMS (m/z): 664.3 (M + H). $^{1}$H NMR (400 MHz, DMSO-$d_6$) δ 7.99 (dd, *J* = 9.2, 5.9 Hz, 1H), 7.55 (d, *J* = 10.3 Hz, 1H), 7.52 - 7.44 (m, 1H), 7.41 (d, *J* = 2.5 Hz, 1H), 7.15 (d, *J* = 2.5 Hz, 1H), 4.49 - 4.33 (m, 2H), 4.31 - 4.17 (m, 2H), 3.97 (s, 1H), 3.60 - 3.43 (m, 4H), 2.84 - 2.55 (m, 3H), 2.11 - 1.92 (m, 3H), 1.81 - 1.62 (m, 5H), 1.13 (s, 3H), 0.50 - 0.34 (m, 2H), 0.32 - 0.13 (m, 2H). $^{19}$F NMR (376 MHz, DMSO-$d_6$) δ-105.33,-105.96,-110.18,-118.99,-124.74 |
| 50 | 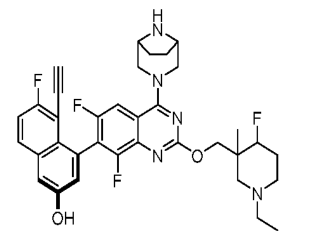 | (*Ra*)-4-(4-((1*R*,5*S*)-3,8-diazabicyclo[3.2.1]octan-3-yl)-2-((1-ethyl-4-fluoro-3-methylpiperidin-3-yl)methoxy)-6,8-difluoroquinazolin-7-yl)-5-ethynyl-6-fluoronaphthalen-2-ol | LCMS (m/z): 634.3 (M + H). |

(continued)

| Examples | Structure | Name | Characterization data |
|---|---|---|---|
| 50-1 | 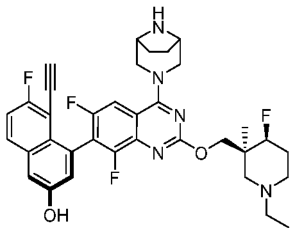 | (*Ra*)-4-(4-((1*R*,5*S*)-3,8-diazabicyclo[3.2.1]octan-3-yl)-2-(((3*S*,4*S*)-1-ethyl-4-fluoro-3-methylpiperidin-3-yl)methoxy)-6,8-difluoroquinazolin-7-yl)-5-ethynyl-6-fluoronaphthalen-2-ol | LCMS (m/z): 634.3 (M + H). $^1$H NMR (400 MHz, Methanol-$d_4$) δ 7.87 (dd, *J*= 9.2, 5.7 Hz, 1H), 7.57 - 7.49 (m, 1H), 7.39 - 7.30 (m, 2H), 7.14 (d, *J* = 2.6 Hz, 1H), 4.77 - 4.56 (m, 1H), 4.54 - 4.31 (m, 4H), 4.03 - 3.83 (m, 2H), 3.69 (dd, *J* = 25.5, 13.2 Hz, 2H), 2.78 - 2.47 (m, 4H), 2.19 - 1.94 (m, 6H), 1.39 - 1.29 (m, 2H), 1.27 (s, 3H), 1.15 (t, *J* = 7.2 Hz, 3H). $^{19}$F NMR (376 MHz, Methanol-$d_4$) δ-76.93,-111.52,-118.70,-125.07 |
| 50-2 | 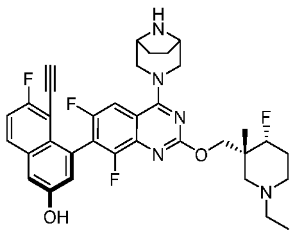 | (*Ra*)-4-(4-((1*R*,5*S*)-3,8-diazabicyclo[3.2.1]octan-3-yl)-2-(((3*R*,4*R*)-1-ethyl-4-fluoro-3-methylpiperidin-3-yl)methoxy)-6,8-difluoroquinazolin-7-yl)-5-ethynyl-6-fluoronaphthalen-2-ol | LCMS (m/z): 634.3 (M + H). |
| 50-3 | 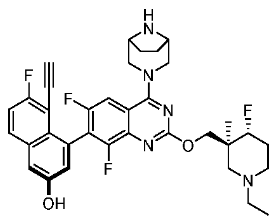 | (*Ra*)-4-(4-((1*R*,5S)-3,8-diazabicyclo[3.2.1]octan-3-yl)-2-(((3*S*,4*R*)-1-ethyl-4-fluoro-3-methylpiperidin-3-yl)methoxy)-6,8-difluoroquinazolin-7-yl)-5-ethynyl-6-fluoronaphthalen-2-ol | LCMS (m/z): 634.3 (M + H). $^1$H NMR (400 MHz, Methanol-$d_4$) δ 7.87 (dd, *J* = 9.2, 5.7 Hz, 1H), 7.51 (d, *J* = 9.9 Hz, 1H), 7.38 - 7.29 (m, 2H), 7.14 (d, *J* = 2.5 Hz, 1H), 4.79 - 4.62 (m, 1H) 1H), 4.55 - 4.30 (m, 4H), 3.78 - 3.52 (m, 4H), 2.82 - 2.21 (m, 3H), 2.13 - 1.74 (m, 7H), 1.40 - 1.29 (m, 2H), 1.20 (s, 3H), 1.10 (t, *J* = 7.2 Hz, 3H). $^{19}$F NMR (376 MHz, Methanol-$d_4$) δ-111.58,-119.32,-125.28 |
| 50-4 | 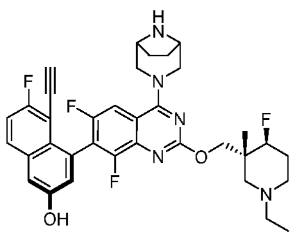 | (*Ra*)-4-(4-((1*R*,5*S*)-3,8-diazabicyclo[3.2.1]octan-3-yl)-2-(((3*R*,4*S*)-1-ethyl-4-fluoro-3-methylpiperidin-3-yl)methoxy)-6,8-difluoroquinazolin-7-yl)-5-ethynyl-6-fluoronaphthalen-2-ol | LCMS (m/z): 634.3 (M + H). |

(continued)

| Examples | Structure | Name | Characterization data |
|---|---|---|---|
| 51 | 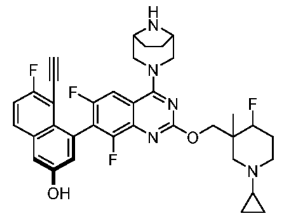 | (*Ra*)-4-(4-((1*R*,5*S*)-3,8-diazabicyclo[3.2.1]octan-3-yl)-2-((1-cyclopropyl-4-fluoro-3-methylpiperidin-3-yl)methoxy)-6,8-difluoroquinazolin-7-yl)-5-ethynyl-6-fluoronaphthalen-2-ol | LCMS (m/z): 646.3 (M + H). |
| 51-1 | 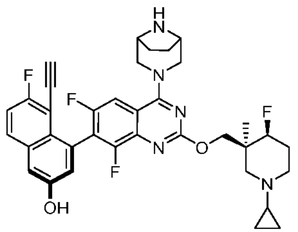 | (*Ra*)-4-(4-((1*R*,5*S*)-3,8-diazabicyclo[3.2.1]octan-3-yl)-2-(((3*S*,4*S*)-1-cyclopropyl-4-fluoro-3-methylpiperidin-3-yl)methoxy)-6,8-difluoroquinazolin-7-yl)-5-ethynyl-6-fluoronaphthalen-2-ol | LCMS (m/z): 646.3 (M + H). |
| 51-2 | 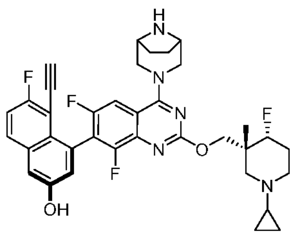 | (*Ra*)-4-(4-((1*R*,5*S*)-3,8-diazabicyclo[3.2.1]octan-3-yl)-2-(((3*R*,4*R*)-1-cyclopropyl-4-fluoro-3-methylpiperidin-3-yl)methoxy)-6,8-difluoroquinazolin-7-yl)-5-ethynyl-6-fluoronaphthalen-2-ol | **LCMS** (m/z): 646.3 (M + H). |
| 51-3 | 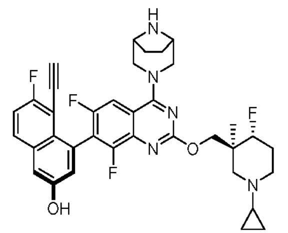 | (*Ra*)-4-(4-((1*R*,5S)-3,8-diazabicyclo[3.2.1]octan-3-yl)-2-(((3*S*,4*R*)-1-cyclopropyl-4-fluoro-3-methylpiperidin-3-yl)methoxy)-6,8-difluoroquinazolin-7-yl)-5-ethynyl-6-fluoronaphthalen-2-ol | **LCMS** (m/z): 646.3 (M + H). |

(continued)

| Examples | Structure | Name | Characterization data |
|---|---|---|---|
| 51-4 | 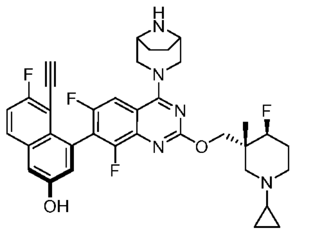 | (*Ra*)-4-(4-((1*R*,5*S*)-3,8-diazabicyclo[3.2.1]octan-3-yl)-2-(((3R,4S)-1-cyclopropyl-4-fluoro-3-methylpiperidin-3-yl)methoxy)-6,8-difluoroquinazolin-7-yl)-5-ethynyl-6-fluoronaphthalen-2-ol | **LCMS** (m/z): 646.3 (M + H). |
| 52 | 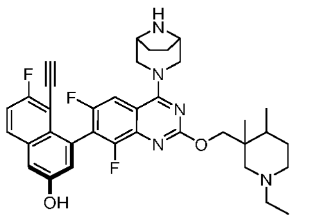 | (*Ra*)-4-(4-((1*R*,5*S*)-3,8-diazabicyclo[3.2.1]octan-3-yl)-2-((1-ethyl-3,4-dimethylpiperidin-3-yl)methoxy)-6,8-difluoroquinazolin-7-yl)-5-ethynyl-6-fluoronaphthalen-2-ol | LCMS (m/z): 630.3 (M + H). |
| 52-1 | 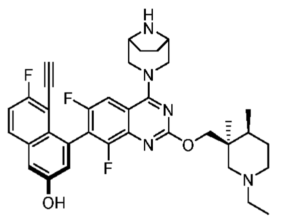 | (*Ra*)-4-(4-((1*R*,5*S*)-3,8-diazabicyclo[3.2.1]octan-3-yl)-2-(((3*S*,4*S*)-1-ethyl-3,4-dimethylpiperidin-3-yl)methoxy)-6,8-difluoroquinazolin-7-yl)-5-ethynyl-6-fluoronaphthalen-2-ol | LCMS (m/z): 630.3 (M + H). $^1$H NMR (400 MHz, DMSO-$d_6$) $\delta$ 10.26 (s, 1H), 8.05 - 7.94 (m, 1H), 7.62 - 7.54 (m, 1H), 7.53 - 7.45 (m, 1H), 7.42 (d, *J* = 2.4 Hz, 1H), 7.16 (d, *J* = 2.3 Hz, 1H), 4.49 - 4.38 (m, 1H), 4.34 - 4.21 (m, 3H), 3.98 (s, 1H), 3.81 - 3.75 (m, 2H), 3.62 - 3.53 (m, 2H), 2.96 - 2.86 (m, 1H), 2.85 - 2.77 (m, 1H), 2.36 - 2.20 (m, 2H), 1.99 - 1.72 (m, 5H), 1.71 - 1.57 (m, 1H), 1.56 - 1.43 (m, 2H), 1.42 - 1.33 (m, 1H), 1.01 (s, 3H), 0.97 - 0.86 (m, 6H). $^{19}$F NMR (376 MHz, DMSO-$d_6$) $\delta$-110.17,-118.76,-124.42. |
| 52-2 | 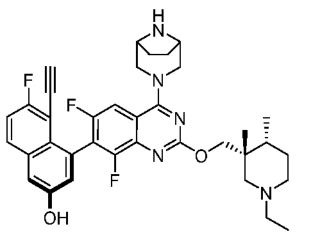 | (*Ra*)-4-(4-((1*R*,5*S*)-3,8-diazabicyclo[3.2.1]octan-3-yl)-2-(((3*R*,4*R*)-1-ethyl-3,4-dimethylpiperidin-3-yl)methoxy)-6,8-difluoroquinazolin-7-yl)-5-ethynyl-6-fluoronaphthalen-2-ol | **LCMS** (m/z): 630.3 (M + H). |

(continued)

| Examples | Structure | Name | Characterization data |
|---|---|---|---|
| 52-3 | 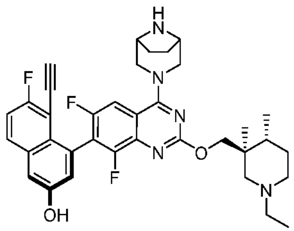 | (*Ra*)-4-(4-((1*R*,5*S*)-3,8-diazabicyclo[3.2.1]octan-3-yl)-2-(((3*S*,4*R*)-1-ethyl-3,4-dimethylpiperidin-3-yl)methoxy)-6,8-difluoroquinazolin-7-yl)-5-ethynyl-6-fluoronaphthalen-2-ol | **LCMS** (m/z): 630.3 (M + H). |
| 52-4 | 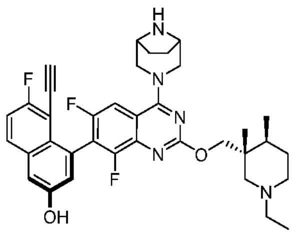 | (*Ra*)-4-(4-((1*R*,5*S*)-3,8-diazabicyclo[3.2.1]octan-3-yl)-2-(((3*R*,4*S*)-1-ethyl-3,4-dimethylpiperidin-3-yl)methoxy)-6,8-difluoroquinazolin-7-yl)-5-ethynyl-6-fluoronaphthalen-2-ol | **LCMS** (m/z): 630.3 (M + H). |
| 53 | 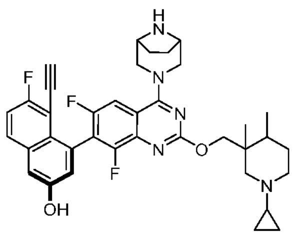 | (*Ra*)-4-(4-((1*R*,5*S*)-3,8-diazabicyclo[3.2.1]octan-3-yl)-2-((1-cyclopropyl-3,4-dimethylpiperidin-3-yl)methoxy)-6,8-difluoroquinazolin-7-yl)-5-ethynyl-6-fluoronaphthalen-2-ol | LCMS (m/z): 642.3 (M + H). |
| 53-1 | 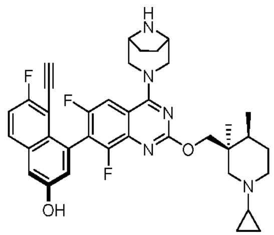 | (*Ra*)-4-(4-((1*R*,5*S*)-3,8-diazabicyclo[3.2.1]octan-3-yl)-2-(((3*S*,4*S*)-1-cyclopropyl-3,4-dimethylpiperidin-3-yl)methoxy)-6,8-difluoroquinazolin-7-yl)-5-ethynyl-6-fluoronaphthalen-2-ol | LCMS (m/z): 642.3 (M + H). |

(continued)

| Examples | Structure | Name | Characterization data |
|---|---|---|---|
| 53-2 | 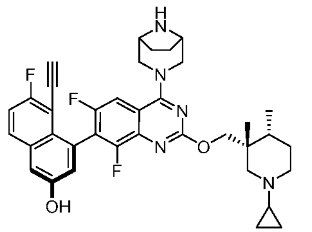 | (*Ra*)-4-(4-((1*R*,5*S*)-3,8-diazabicyclo[3.2.1]octan-3-yl)-2-(((3*R*,4*R*)-1-cyclopropyl-3,4-dimethylpiperidin-3-yl)methoxy)-6,8-difluoroquinazolin-7-yl)-5-ethynyl-6-fluoronaphthalen-2-ol | **LCMS** (m/z): 642.3 (M + H). |
| 53-3 | 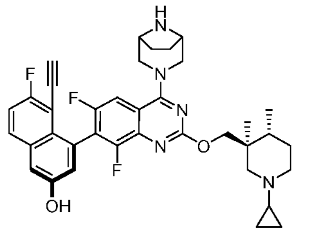 | (*Ra*)-4-(4-((1*R*,5*S*)-3,8-diazabicyclo[3.2.1]octan-3-yl)-2-(((3*S*,4*R*)-1-cyclopropyl-3,4-dimethylpiperidin-3-yl)methoxy)-6,8-difluoroquinazolin-7-yl)-5-ethynyl-6-fluoronaphthalen-2-ol | LCMS (m/z): 642.3 (M + H). |
| 53-4 | 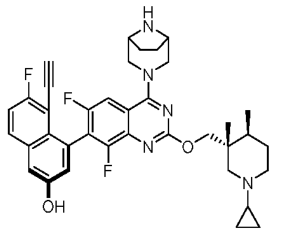 | (*Ra*)-4-(4-((1*R*,5*S*)-3,8-diazabicyclo[3.2.1]octan-3-yl)-2-(((3*R*,4*S*)-1-cyclopropyl-3,4-dimethylpiperidin-3-yl)methoxy)-6,8-difluoroquinazolin-7-yl)-5-ethynyl-6-fluoronaphthalen-2-ol | LCMS (m/z): 642.3 (M + H). |
| 54 | 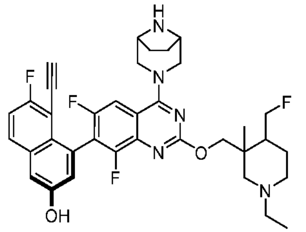 | (*Ra*)-4-(4-((1*R*,5*S*)-3,8-diazabicyclo[3.2.1]octan-3-yl)-2-((1-ethyl-4-(fluoromethyl)-3-methylpiperidin-3-yl)methoxy)-6,8-difluoroquinazolin-7-yl)-5-ethynyl-6-fluoronaphthalen-2-ol | LCMS (m/z): 648.3 (M + H). |

(continued)

| Examples | Structure | Name | Characterization data |
|---|---|---|---|
| 54-1 | 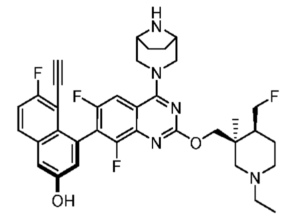 | (*Ra*)-4-(4-((1*R*,5*S*)-3,8-diazabicyclo[3.2.1]octan-3-yl)-2-(((3*S*,4*S*)-1-ethyl-4-(fluoromethyl)-3-methylpiperidin-3-yl)methoxy)-6,8-difluoroquinazolin-7-yl)-5-ethynyl-6-fluoronaphthalen-2-ol | **LCMS** (m/z): 648.3 (M + H). |
| 54-2 | 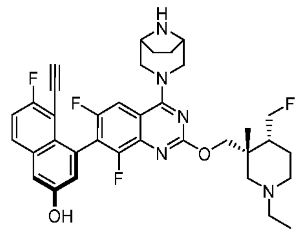 | (*Ra*)-4-(4-((1*R*,5*S*)-3,8-diazabicyclo[3.2.1]octan-3-yl)-2-(((3*R*,4*R*)-1-ethyl-4-(fluoromethyl)-3-methylpiperidin-3-yl)methoxy)-6,8-difluoroquinazolin-7-yl)-5-ethynyl-6-fluoronaphthalen-2-ol | **LCMS** (m/z): 648.3 (M + H). |
| 54-3 | 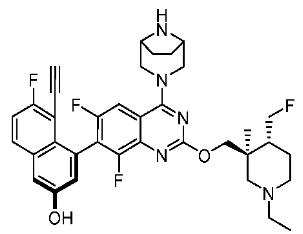 | (*Ra*)-4-(4-((1*R*,5*S*)-3,8-diazabicyclo[3.2.1]octan-3-yl)-2-(((3*S*,4*R*)-1-ethyl-4-(fluoromethyl)-3-methylpiperidin-3-yl)methoxy)-6,8-difluoroquinazolin-7-yl)-5-ethynyl-6-fluoronaphthalen-2-ol | LCMS (m/z): 648.3 (M + H). |
| 54-4 | 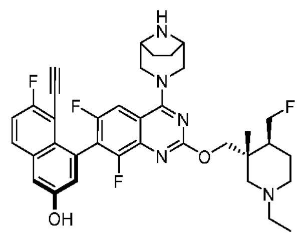 | (*Ra*)-4-(4-((1*R*,5*S*)-3,8-diazabicyclo[3.2.1]octan-3-yl)-2-(((3*R*,4*S*)-1-ethyl-4-(fluoromethyl)-3-methylpiperidin-3-yl)methoxy)-6,8-difluoroquinazolin-7-yl)-5-ethynyl-6-fluoronaphthalen-2-ol | LCMS (m/z): 648.3 (M + H). |

(continued)

| Examples | Structure | Name | Characterization data |
|---|---|---|---|
| 55 | 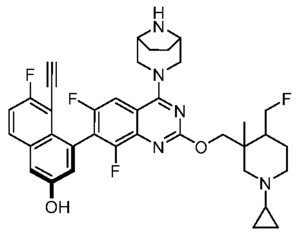 | (*Ra*)-4-(4-((1*R*,5*S*)-3,8-diazabicyclo[3.2.1]octan-3-yl)-2-((1-cyclopropyl-4-(fluoromethyl)-3-methylpiperidin-3-yl)methoxy)-6,8-difluoroquinazolin-7-yl)-5-ethynyl-6-fluoronaphthalen-2-ol | **LCMS** (m/z): 660.3 (M + H). |
| 55-1 | 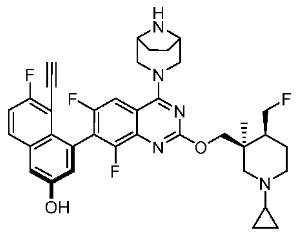 | (*Ra*)-4-(4-((1*R*,5*S*)-3,8-diazabicyclo[3.2.1]octan-3-yl)-2-(((3S,4S)-1-cyclopropyl-4-(fluoromethyl)-3-methylpiperidin-3-yl)methoxy)-6,8-difluoroquinazolin-7-yl)-5-ethynyl-6-fluoronaphthalen-2-ol | LCMS (m/z): 660.3 (M + H). |
| 55-2 | 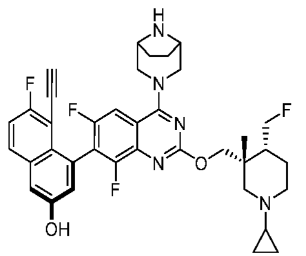 | (*Ra*)-4-(4-((1*R*,5*S*)-3,8-diazabicyclo[3.2.1]octan-3-yl)-2-(((3*R*,4*R*)-1-cyclopropyl-4-(fluoromethyl)-3-methylpiperidin-3-yl)methoxy)-6,8-difluoroquinazolin-7-yl)-5-ethynyl-6-fluoronaphthalen-2-ol | **LCMS** (m/z): 660.3 (M + H). |
| 55-3 | 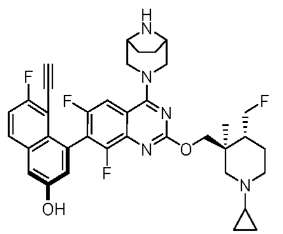 | (*Ra*)-4-(4-((1*R*,5*S*)-3,8-diazabicyclo[3.2.1]octan-3-yl)-2-(((3*S*,4*R*)-1-cyclopropyl-4-(fluoromethyl)-3-methylpiperidin-3-yl)methoxy)-6,8-difluoroquinazolin-7-yl)-5-ethynyl-6-fluoronaphthalen-2-ol | **LCMS** (m/z): 660.3 (M + H). |

(continued)

| Examples | Structure | Name | Characterization data |
|---|---|---|---|
| 55-4 | 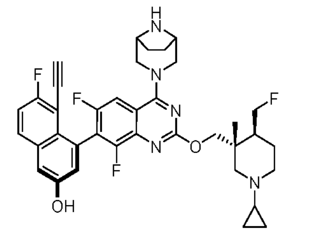 | (*Ra*)-4-(4-((1*R*,5*S*)-3,8-diazabicyclo[3.2.1]octan-3-yl)-2-(((3R,4S)-1-cyclopropyl-4-(fluoromethyl)-3-methylpiperidin-3-yl)methoxy)-6,8-difluoroquinazolin-7-yl)-5-ethynyl-6-fluoronaphthalen-2-ol | LCMS (m/z): 660.3 (M + H). |
| 56 | 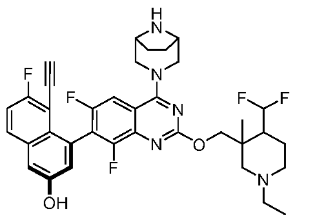 | (*Ra*)-4-(4-((1*R*,5*S*)-3,8-diazabicyclo[3.2.1]octan-3-yl)-2-((4-(difluoromethyl)-1-ethyl-3-methylpiperidin-3-yl)methoxy)-6,8-difluoroquinazolin-7-yl)-5-ethynyl-6-fluoronaphthalen-2-ol | LCMS (m/z): 666.3 (M + H). |
| 56-1 | 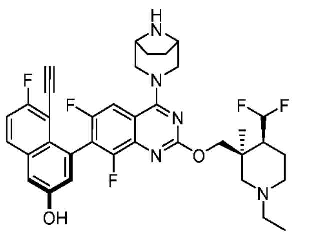 | (*Ra*)-4-(4-((1*R*,5*S*)-3,8-diazabicyclo[3.2.1]octan-3-yl)-2-(((3*S*,4*S*)-4-(difluoromethyl)-1-ethyl-3-methylpiperidin-3-yl)methoxy)-6,8-difluoroquinazolin-7-yl)-5-ethynyl-6-fluoronaphthalen-2-ol | LCMS (m/z): 666.3 (M + H). $^{1}$H NMR (400 MHz, methanol-$d_4$) δ 7.84 (dd, *J* = 9.2, 5.7 Hz, 1H), 7.48 (dd, *J* = 10.0, 1.8 Hz, 1H), 7.34 - 7.27 (m, 2H), 7.11 (d, *J* = 2.6 Hz, 1H), 6.37 - 6.02 (m, 1H), 4.63 - 4.56 (m, 1H), 4.54 - 4.45 (m, 2H), 4.44 - 4.37 (m, 1H), 3.66 - 3.59 (m, 3H), 3.58 - 3.52 (m, 1H), 3.21 - 3.12 (m, 1H), 3.07 - 2.99 (m, 1H), 2.45 - 2.30 (m, 2H), 2.05 - 1.95 (m, 1H), 1.95 - 1.75 (m, 7H), 1.74 - 1.68 (m, 1H), 1.21 (s, 3H), 1.04 (t, *J* = 7.2 Hz, 3H). $^{19}$F NMR (376 MHz, Methanol-$d_4$) δ-111.60,-118.55,-119.56,-121.63,-125.36. |
| 56-2 | 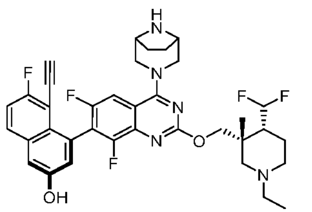 | (*Ra*)-4-(4-((1*R*,5*S*)-3,8-diazabicyclo[3.2.1]octan-3-yl)-2-(((3*R*,4*R*)-4-(difluoromethyl)-1-ethyl-3-methylpiperidin-3-yl)methoxy)-6,8-difluoroquinazolin-7-yl)-5-ethynyl-6-fluoronaphthalen-2-ol | **LCMS** (m/z): 666.3 (M + H). |

(continued)

| Examples | Structure | Name | Characterization data |
|---|---|---|---|
| 56-3 | 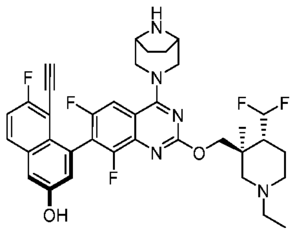 | (*Ra*)-4-(4-((1*R*,5*S*)-3,8-diazabicyclo[3.2.1]octan-3-yl)-2-(((3*S*,4*R*)-4-(difluoromethyl)-1-ethyl-3-methylpiperidin-3-yl)methoxy)-6,8-difluoroquinazolin-7-yl)-5-ethynyl-6-fluoronaphthalen-2-ol | **LCMS** (m/z): 666.3 (M + H). |
| 56-4 | 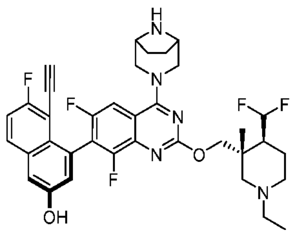 | (*Ra*)-4-(4-((1*R*,5*S*)-3,8-diazabicyclo[3.2.1]octan-3-yl)-2-((3*R*,4*S*)-4-(difluoromethyl)-1-ethyl-3-methylpiperidin-3-yl)methoxy)-6,8-difluoroquinazolin-7-yl)-5-ethynyl-6-fluoronaphthalen-2-ol | **LCMS** (m/z): 666.3 (M + H). |
| 57 | 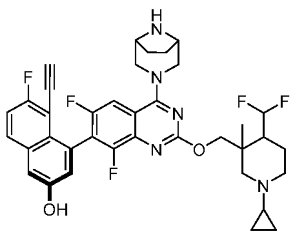 | (*Ra*)-4-(4-((1*R*,5*S*)-3,8-diazabicyclo[3.2.1]octan-3-yl)-2-((1-cyclopropyl-4-(difluoromethyl)-3-methylpiperidin-3-yl)methoxy)-6,8-difluoroquinazolin-7-yl)-5-ethynyl-6-fluoronaphthalen-2-ol | **LCMS** (m/z): 678.3 (M + H). |
| 57-1 | 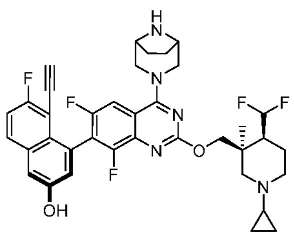 | (*Ra*)-4-(4-((1*R*,5*S*)-3,8-diazabicyclo[3.2.1]octan-3-yl)-2-(((3*S*,4*S*)-1-cyclopropyl-4-(difluoromethyl)-3-methylpiperidin-3-yl)methoxy)-6,8-difluoroquinazolin-7-yl)-5-ethynyl-6-fluoronaphthalen-2-ol | **LCMS** (m/z): 678.3 (M + H). |

(continued)

| Examples | Structure | Name | Characterization data |
|---|---|---|---|
| 57-2 | 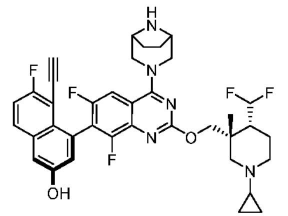 | (*Ra*)-4-(4-((1*R*,5S)-3,8-diazabicyclo[3.2.1]octan-3-yl)-2-(((3*R*,4*R*)-1-cyclopropyl-4-(difluoromethyl)-3-methylpiperidin-3-yl)methoxy)-6,8-difluoroquinazolin-7-yl)-5-ethynyl-6-fluoronaphthalee-2-ol | LCMS (m/z): 678.3 (M + H). |
| 57-3 | 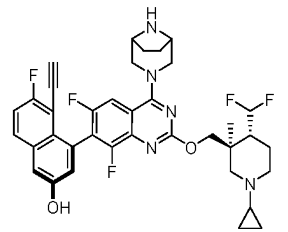 | (*Ra*)-4-(4-((1*R*,5*S*)-3,8-diazabicyclo[3.2.1]octan-3-yl)-2-(((3*S*,4*R*)-1-cyclopropyl-4-(difluoromethyl)-3-methylpiperidin-3-yl)methoxy)-6,8-difluoroquinazolin-7-yl)-5-ethynyl-6-fluoronaphthalen-2-ol | LCMS (m/z): 678.3 (M + H). |
| 57-4 | 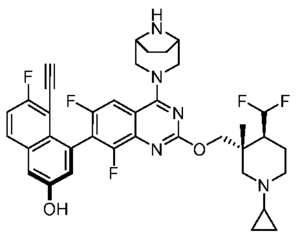 | (*Ra*)-4-(4-((1*R*,5S)-3,8-diazabicyclo[3.2.1]octan-3-yl)-2-(((3*R*,4*S*)-1-cyclopropyl-4-(difluoromethyl)-3-methylpiperidin-3-yl)methoxy)-6,8-difluoroquinazolin-7-yl)-5-ethynyl-6-fluoronaphthalen-2-ol | LCMS (m/z): 678.3 (M + H). |
| 58 | 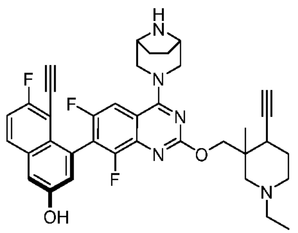 | (*Ra*)-4-(4-((1*R*,5*S*)-3,8-diazabicyclo[3.2.1]octan-3-yl)-2-((1-ethyl-4-ethynyl-3-methylpiperidin-3-yl)methoxy)-6,8-difluoroquinazolin-7-yl)-5-ethynyl-6-fluoronaphthalen-2-ol | **LCMS** (m/z): 640.3 (M + H). |

(continued)

| Examples | Structure | Name | Characterization data |
|---|---|---|---|
| 58-1 | 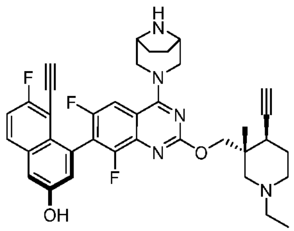 | (*Ra*)-4-(4-((1*R*,5*S*)-3,8-diazabicyclo[3.2.1]octan-3-yl)-2-(((3*R*,4*R*)-1-ethyl-4-ethynyl-3-methylpiperidin-3-yl)methoxy)-6,8-difluoroquinazolin-7-yl)-5-ethynyl-6-fluoronaphthalen-2-ol | LCMS (m/z): 640.3 (M + H). |
| 58-2 | 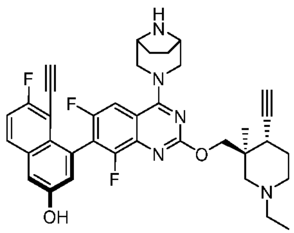 | (*Ra*)-4-(4-((1*R*,5S)-3,8-diazabicyclo[3.2.1]octan-3-yl)-2-(((3*S*,4*S*)-1-ethyl-4-ethynyl-3-methylpiperidin-3-yl)methoxy)-6,8-difluoroquinazolin-7-yl)-5-ethynyl-6-fluoronaphthalen-2-ol | LCMS (m/z): 640.3 (M + H). |
| 58-3 | 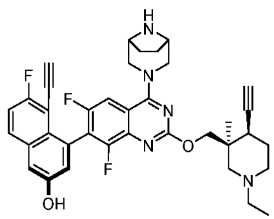 | (*Ra*)-4-(4-((1*R*,5S)-3,8-diazabicyclo[3.2.1]octan-3-yl)-2-(((3*S*,4*R*)-1-ethyl-4-ethynyl-3-methylpiperidin-3-yl)methoxy)-6,8-difluoroquinazolin-7-yl)-5-ethynyl-6-fluoronaphthalen-2-ol | LCMS (m/z): 640.3 (M + H). |
| 58-4 | 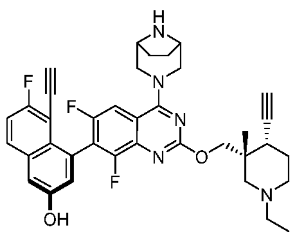 | (*Ra*)-4-(4-((1*R*,5*S*)-3,8-diazabicyclo[3.2.1]octan-3-yl)-2-(((3*R*,4*S*)-1-ethyl-4-ethynyl-3-methylpiperidin-3-yl)methoxy)-6,8-difluoroquinazolin-7-yl)-5-ethynyl-6-fluoronaphthalen-2-ol | **LCMS** (m/z): 640.3 (M + H). |

(continued)

| Examples | Structure | Name | Characterization data |
|---|---|---|---|
| 59 | 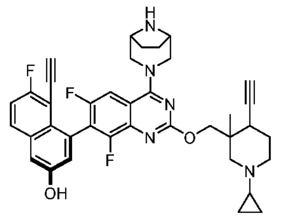 | (*Ra*)-4-(4-((1*R*,5*S*)-3,8-diazabicyclo[3.2.1]octan-3-yl)-2-((1-cyclopropyl-4-ethynyl-3-methylpiperidin-3-yl)methoxy)-6,8-difluoroquinazolin-7-yl)-5-ethynyl-6-fluoronaphthalen-2-ol | **LCMS** (m/z): 652.3 (M + H). |
| 59-1 | 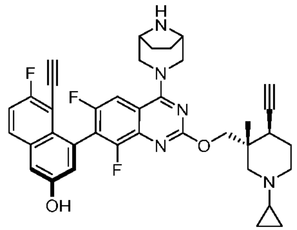 | (*Ra*)-4-(4-((1*R*,5*S*)-3,8-diazabicyclo[3.2.1]octan-3-yl)-2-(((3*R*,4*R*)-1-cyclopropyl-4-ethynyl-3-methylpiperidin-3-yl)methoxy)-6,8-difluoroquinazolin-7-yl)-5-ethynyl-6-fluoronaphthalen-2-ol | **LCMS** (m/z): 652.3 (M + H). |
| 59-2 | 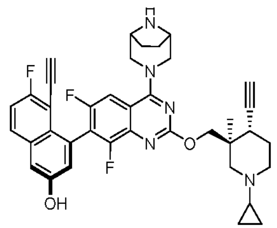 | (*Ra*)-4-(4-((1*R*,5*S*)-3,8-diazabicyclo[3.2.1]octan-3-yl)-2-(((3S,4S)-1-cyclopropyl-4-ethynyl-3-methylpiperidin-3-yl)methoxy)-6,8-difluoroquinazolin-7-yl)-5-ethynyl-6-fluoronaphthalen-2-ol | **LCMS** (m/z): 652.3 (M + H). |
| 59-3 | 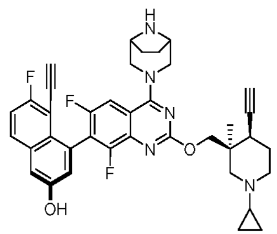 | (*Ra*)-4-(4-((1*R*,5*S*)-3,8-diazabicyclo[3.2.1]octan-3-yl)-2-(((3*S*,4*R*)-1-cyclopropyl-4-ethynyl-3-methylpiperidin-3-yl)methoxy)-6,8-difluoroquinazolin-7-yl)-5-ethynyl-6-fluoronaphthalen-2-ol | LCMS (m/z): 652.3 (M + H). |

(continued)

| Examples | Structure | Name | Characterization data |
|---|---|---|---|
| 59-4 | 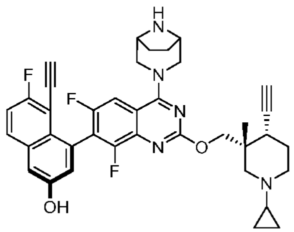 | (*Ra*)-4-(4-((1*R*,5*S*)-3,8-diazabicyclo[3.2.1]octan-3-yl)-2-(((3*R*,4*S*)-1-cyclopropyl-4-ethynyl-3-methylpiperidin-3-yl)methoxy)-6,8-difluoroquinazolin-7-yl)-5-ethynyl-6-fluoronaphthalen-2-ol | LCMS (m/z): 652.3 (M + H). |
| 60 | 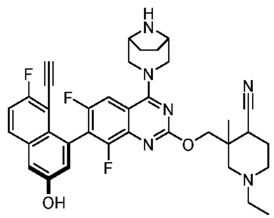 | (*Ra*)-3-(((4-((1R,5*S*)-3,8-diazabicyclo[3.2.1]octan-3-yl)-7-(8-ethynyl-7-fluoro-3-hydroxynaphthalen-1-yl)-6,8-difluoroquinazolin-2-yl)oxy)methyl)-1-ethyl-3-methylpiperidin-4-carbonitrile | LCMS (m/z): 641.3 (M + H). |
| 60-1 | 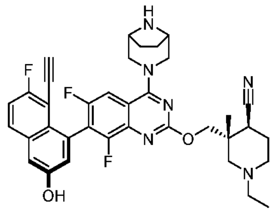 | (*Ra*)-(3*R*,4*S*)-3-(((4-((1*R*,5*S*)-3,8-diazabicyclo[3.2.1]octan-3-yl)-7-(8-ethynyl-7-fluoro-3-hydroxynaphthalen-1-yl)-6,8-difluoroquinazolin-2-yl)oxy)methyl)-1-ethyl-3-methylpiperidin-4-carbonitrile | LCMS (m/z): 641.3 (M + H). |
| 60-2 | 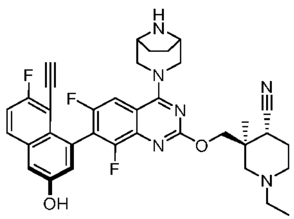 | (*Ra*)-(3*S*,4*R*)-3-(((4-((1*R*,5*S*)-3,8-diazabicyclo[3.2.1]octan-3-yl)-7-(8-ethynyl-7-fluoro-3-hydroxynaphthalen-1-yl)-6,8-difluoroquinazolin-2-yl)oxy)methyl)-1-ethyl-3-methylpiperidin-4-carbonitrile | LCMS (m/z): 641.3 (M + H). |

(continued)

| Examples | Structure | Name | Characterization data |
|---|---|---|---|
| 60-3 | 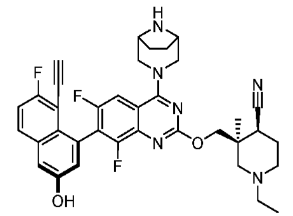 | (*Ra*)-(*3S,4S*)-3-(((4-((1*R*,5*S*)-3,8-diazabicyclo [3.2.1]octan-3-yl)-7-(8-ethynyl-7-fluoro-3-hydroxynaphthalen-1-yl)-6,8-difluoroquinazolin-2-yl) oxy)methyl)-1-ethyl-3-methylpiperidin-4-carbonitrile | LCMS (m/z): 641.3 (M + H). |
| 60-4 | 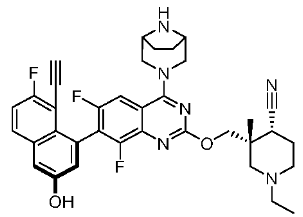 | (*Ra*)-(*3R,4R*)-3-(((4-((1*R*,5*S*)-3,8-diazabicyclo [3.2.1]octan-3-yl)-7-(8-ethynyl-7-fluoro-3-hydroxynaphthalen-1-yl)-6,8-difluoroquinazolin-2-yl) oxy)methyl)-1-ethyl-3-methylpiperidin-4-carbonitrile | LCMS (m/z): 641.3 (M + H). |
| 61 | 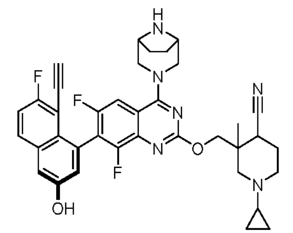 | (*Ra*)-3-(((4-((1*R*,5*S*)-3,8-diazabicyclo[3.2.1]octan-3-yl)-7-(8-ethynyl-7-fluoro-3-hydroxynaphthalen-1-yl)-6,8-difluoroquinazolin-2-yl)oxy) methyl)-1-cyclopropyl-3-methylpiperidin-4-carbonitrile | LCMS (m/z): 653.3 (M + H). |
| 61-1 | 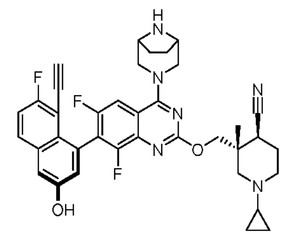 | (*Ra*)-(*3R,4*S)-3-(((4-((1*R*,5*S*)-3,8-diazabicyclo [3.2.1]octan-3-yl)-7-(8-ethynyl-7-fluoro-3-hydroxynaphthalen-1-yl)-6,8-difluoroquinazolin-2-yl) oxy)methyl)-1-cyclopropyl-3-methylpiperidin-4-carbonitrile | LCMS (m/z): 653.3 (M + H). |

(continued)

| Examples | Structure | Name | Characterization data |
|---|---|---|---|
| 61-2 | 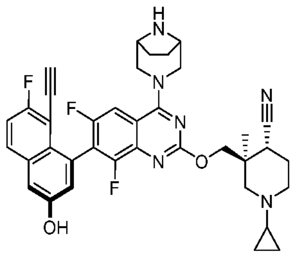 | (*Ra*)-(3*S*,4*R*)-3-(((4-((1*R*,5*S*)-3,8-diazabcyclo [3.2.1]octan-3-yl)-7-(8-ethynyl-7-fluoro-3-hydroxynaphthalen-1-yl)-6,8-difluoroquinazolin-2-yl) oxy)methyl)-1-cyclopropyl-3-methylpiperidin-4-carbonitrile | LCMS (m/z): 653.3 (M + H). |
| 61-3 | 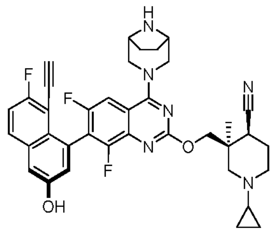 | (*Ra*)-(3*S*,4*S*)-3-(((4-((1*R*,5*S*)-3,8-diazabicyclo [3.2.1]octan-3-yl)-7-(8-ethynyl-7-fluoro-3-hydroxynaphthalen-1-yl)-6,8-difluoroquinazolin-2-yl) oxy)methyl)-1-cyclopropyl-3-methylpiperidin-4-carbonitrile | LCMS (m/z): 653.3 (M + H). |
| 61-4 | 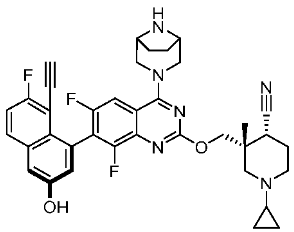 | (*Ra*)-(3*R*,4*R*)-3-(((4-((1*R*,5*S*)-3,8-diazabicyclo [3.2.1]octan-3-yl)-7-(8-ethynyl-7-fluoro-3-hydroxynaphthalen-1-yl)-6,8-difluoroquinazolin-2-yl) oxy)methyl)-1-cyclopropyl-3-methylpiperidin-4-carbonitrile | LCMS (m/z): 653.3 (M + H). |

(continued)

| Examples | Structure | Name | Characterization data |
|---|---|---|---|
| 62 | 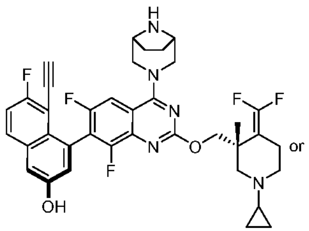 or 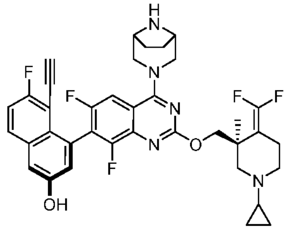 | (*Ra*)-4-(4-((*1R,5S*)-3,8-diazabicyclo[3.2.1]octan-3-yl)-2-(((*R* or *S*)-1-cyclopropyl-4-(difluoromethylene)-3-methylpiperidin-3-yl)methoxy)-6,8-difluoroquinazolin-7-yl)-5-ethynyl-6-fluoronaphthalen-2-ol | **LCMS** (m/z): 676.3 (M + H). |
| 63 | 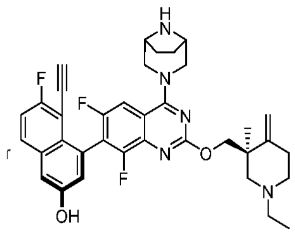 | (*Ra*)-4-(4-((1R,5S)-3,8-diazabicyclo[3.2.1]octan-3-yl)-2-(((*S*)-1-ethyl-3-methyl-4-methylenepiperidin-3-yl)methoxy)-6,8-difluoroquinazolin-7-yl)-5-ethynyl-6-fluoronaphthalen-2-ol | LCMS (m/z): 628.3 (M + H). $^{1}$H NMR (400 MHz, DMSO-$d_6$) δ 10.24 (s, 1H), 8.05 - 7.94 (m, 1H), 7.58 - 7.52 (m, 1H), 7.52 - 7.46 (m, 1H), 7.42 (d, *J* = 2.5 Hz, 1H), 7.15 (d, *J* = 2.4 Hz, 1H), 4.81 (d, *J* = 22.6 Hz, 2H), 4.56 - 4.48 (m, 1H), 4.36 - 4.27 (m, 1H), 4.27 - 4.18 (m, 2H), 3.99 (s, 1H), 3.56 - 3.43 (m, 4H), 2.79 - 2.69 (m, 2H), 2.48 - 2.37 (m, 1H), 2.35 - 2.25 (m, 2H), 2.25 - 2.16 (m, 1H), 2.08 - 1.96 (m, 1H), 1.91 - 1.82 (m, 1H), 1.80 - 1.71 (m, 1H), 1.71 - 1.60 (m, J 3H), 1.15 (s, 3H), 0.97 (t, *J* = 7.1 Hz, 3H). $^{19}$F NMR (376 MHz, DMSO-$d_6$) δ-110.18,-119.26,-124.68. |
| 64 | 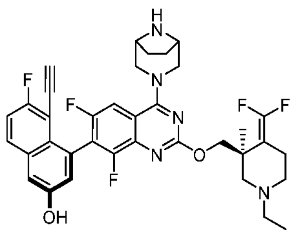 | (*Ra*)-4-(4-((1*R*,5*S*)-3,8-diazabicyclo[3.2.1]octan-3-yl)-2-(((*S*)-1-ethyl-4-(difluoromethylene)-3-methylpiperidin-3-yl)methoxy)-6,8-difluoroquinazolin-7-yl)-5-ethynyl-6-fluoronaphthalen-2-ol | LCMS (m/z): 664.3 (M + H). $^{1}$H NMR (400 MHz, DMSO-$d_6$) δ 8.07 - 7.94 (m, 1H), 7.61 - 7.46 (m, 2H), 7.45 - 7.37 (m, 1H), 7.20 - 7.10 (m, 1H), 4.75 - 4.61 (m, 1H), 4.35 - 4.13 (m, 3H), 4.05 - 3.94 (m, 1H), 3.61 - 3.43 (m, 5H), 2.79 - 2.64 (m, 2H), 2.39 - 2.19 (m, 4H), 2.11 - 1.92 (m, 3H), 1.81 - 1.60 (m, 5H), 1.06 - 0.91 (m, 3H). $^{19}$F NMR (376 MHz, DMSO-$d_6$) δ-90.20,-90.63,-110.18,-119.14,-124.64. |

(continued)

| Examples | Structure | Name | Characterization data |
|---|---|---|---|
| 65 | 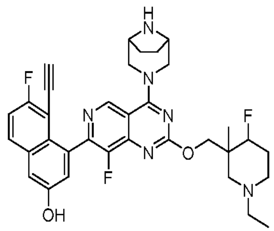 | 4-(4-((1*R*,5*S*)-3,8-diazabicyclo[3.2.1]octan-3-yl)-2-((1-ethyl-4-fluoro-3-methylpiperidin-3-yl)methoxy)-8-fluoropyrido[4,3-*d*]pyrimidin-7-yl)-5-ethynyl-6-fluoronaphthalen-2-ol | LCMS (m/z): 617.3 (M + H). |
| 65-1 | 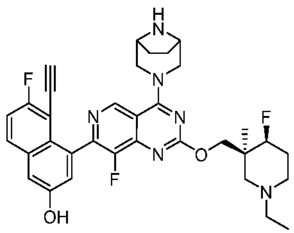 | 4-(4-((1*R*,5*S*)-3,8-diazabicyclo[3.2.1]octan-3-yl)-2-(((3*S*,4*S*)-1-ethyl-4-fluoro-3-methylpiperidin-3-yl)methoxy)-8-fluoropyrido[4,3-*d*]pyrimidin-7-yl)-5-ethynyl-6-fluoronaphthalen-2-ol | LCMS (m/z): 617.3 (M + H). |
| 65-2 | 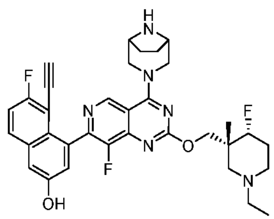 | 4-(4-((1*R*,5*S*)-3,8-diazabicyclo[3.2.1]octan-3-yl)-2-(((3*R*,4*R*)-1-ethyl-4-fluoro-3-methylpiperidin-3-yl)methoxy)-8-fluoropyrido[4,3-*d*]pyrimidin-7-yl)-5-ethynyl-6-fluoronaphthalen-2-ol | LCMS (m/z): 617.3 (M + H). |
| 65-3 | 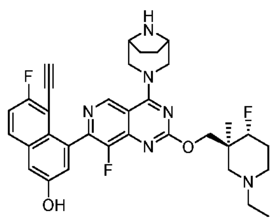 | 4-(4-((1*R*,5*S*)-3,8-diazabicyclo[3.2.1]octan-3-yl)-2-(((3*S*,4*R*)-1-ethyl-4-fluoro-3-methylpiperidin-3-yl)methoxy)-8-fluoropyrido[4,3-*d*]pyrimidin-7-yl)-5-ethynyl-6-fluoronaphthalen-2-ol | LCMS (m/z): 617.3 (M + H). |

(continued)

| Examples | Structure | Name | Characterization data |
|---|---|---|---|
| 65-4 | 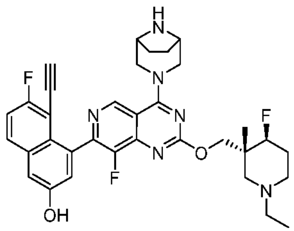 | 4-(4-((1*R*,5*S*)-3,8-diazabicyclo[3.2.1]octan-3-yl)-2-(((3*R*,4*S*)-1-ethyl-4-fluoro-3-methylpiperidin-3-yl)methoxy)-8-fluoropyrido[4,3-*d*]pyrimidin-7-yl)-5-ethynyl-6-fluoronaphthalen-2-ol | LCMS (m/z): 617.3 (M + H). |
| 66 | 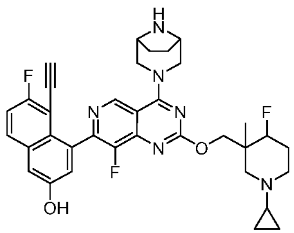 | 4-(4-((1*R*,5*S*)-3,8-diazabicyclo[3.2.1]octan-3-yl)-2-((1-cyclopropyl-4-fluoro-3-methylpiperidin-3-yl)methoxy)-8-fluoropyrido[4,3-*d*]pyrimidin-7-yl)-5-ethynyl-6-fluoronaphthalen-2-ol | LCMS (m/z): 629.3 (M + H). |
| 66-1 | 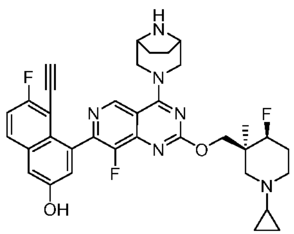 | 4-(4-((1*R*,5*S*)-3,8-diazabicyclo[3.2.1]octan-3-yl)-2-(((3*S*,4*S*)-1-cyclopropyl-4-fluoro-3-methylpiperidin-3-yl)methoxy)-8-fluoropyrido[4,3-*d*]pyrimidin-7-yl)-5-ethynyl-6-fluoronaphthalen-2-ol | LCMS (m/z): 629.3 (M + H). |
| 66-2 | 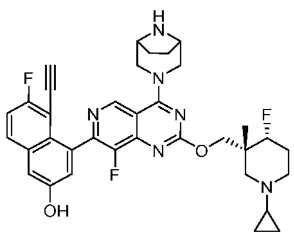 | 4-(4-((1*R*,5*S*)-3,8-diazabicyclo[3.2.1]octan-3-yl)-2-(((3*R*,4*R*)-1-cyclopropyl-4-fluoro-3-methylpiperidin-3-yl)methoxy)-8-fluoropyrido[4,3-*d*]pyrimidin-7-yl)-5-ethynyl-6-fluoronaphthalen-2-ol | LCMS (m/z): 629.3 (M + H). |

(continued)

| Examples | Structure | Name | Characterization data |
|---|---|---|---|
| 66-3 | 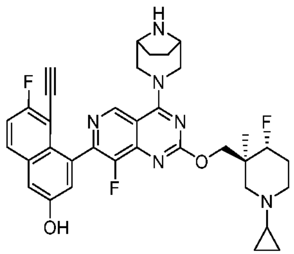 | 4-(4-((1*R*,5*S*)-3,8-diazabicyclo[3.2.1]octan-3-yl)-2-(((3*S*,4*R*)-1-cyclopropyl-4-fluoro-3-methylpiperidin-3-yl)methoxy)-8-fluoropyrido[4,3-*d*]pyrimidin-7-yl)-5-ethynyl-6-fluoronaphthalen-2-ol | LCMS (m/z): 629.3 (M + H). |
| 66-4 | 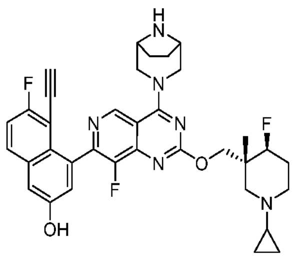 | 4-(4-((1*R*,5*S*)-3,8-diazabicyclo[3.2.1]octan-3-yl)-2-(((3*R*,4*S*)-1-cyclopropyl-4-fluoro-3-methylpiperidin-3-yl)methoxy)-8-fluoropyrido[4,3-*d*]pyrimidin-7-yl)-5-ethynyl-6-fluoronaphthalen-2-ol | LCMS (m/z): 629.3 (M + H). |
| 67 | 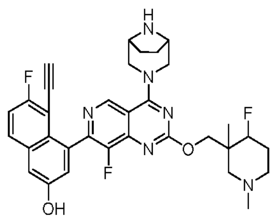 | 4-(4-((1*R*,5*S*)-3,8-diazabicyclo[3.2.1]octan-3-yl)-8-fluoro-2-(4-fluoro-1,3-dimethylpiperidin-3-yl)methoxy)pyrido[4,3-*d*]pyrimidin-7-yl)-5-ethynyl-6-fluoronaphthalen-2-ol | LCMS (m/z): 603.3 (M + H). |
| 67-1 | 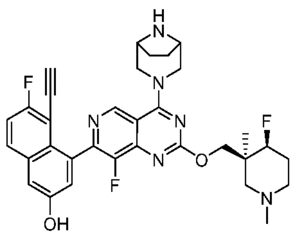 | 4-(4-((1*R*,5*S*)-3,8-diazabicyclo[3.2.1]octan-3-yl)-8-fluoro-2-((((3*S*,4*S*)-4-fluoro-1,3-dimethylpiperidin-3-yl)methoxy))pyrido[4,3-*d*]pyrimidin-7-yl)-5-ethynyl-6-fluoronaphthalen-2-ol | LCMS (m/z): 603.3 (M + H). |

(continued)

| Examples | Structure | Name | Characterization data |
|---|---|---|---|
| 67-2 | 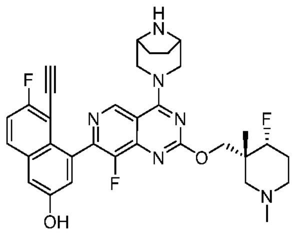 | 4-(4-((1*R*,5*S*)-3,8-diazabicyclo[3.2.1]octan-3-yl)-8-fluoro-2-((((3*R*,4*R*)-4-fluoro-1,3-dimethylpiperidin-3-yl)methoxy)pyrido[4,3-*d*]pyrimidin-7-yl)-5-ethynyl-6-fluoronaphthalen-2-ol | LCMS (m/z): 603.3 (M + H). |
| 67-3 | 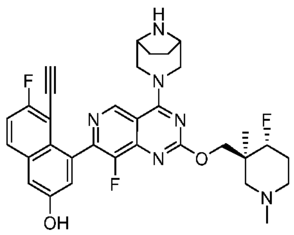 | 4-(4-((1*R*,5*S*)-3,8-diazabicyclo[3.2.1]octan-3-yl)-8-fluoro-2-((((3*S*,4*R*)-4-fluoro-1,3-dimethylpiperidin-3-yl)methoxy)pyrido[4,3-*d*]pyrimidin-7-yl)-5-ethynyl-6-fluoronaphthalen-2-ol | LCMS (m/z): 603.3 (M + H). |
| 67-4 | 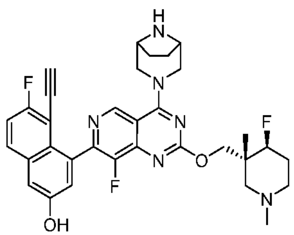 | 4-(4-((1*R*,5*S*)-3,8-diazabicyclo[3.2.1]octan-3-yl)-8-fluoro-2-((((3*R*,4*S*)-4-fluoro-1,3-dimethylpiperidin-3-yl)methoxy)pyrido[4,3-*d*]pyrimidin-7-yl)-5-ethynyl-6-fluoronaphthalen-2-ol | LCMS (m/z): 603.3 (M + H). |

(continued)

| Examples | Structure | Name | Characterization data |
|---|---|---|---|
| 68-1 | 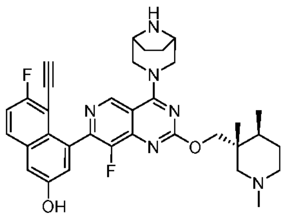 or 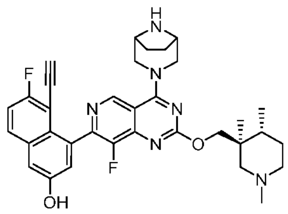 | 4-(4-((1*R*,5*S*)-3,8-diazabicyclo[3.2.1]octan-3-yl)-8-fluoro-2-(((3*R*,4*S*)-1,3,4-trimethylpiperidin-3-yl)methoxy)pyrido[4,3-*d*]pyrimidin-7-yl)-5-ethynyl-6-fluoronaphthalen-2-ol or 4-(4-((1*R*,5*S*)-3,8-diazabicyclo[3.2.1]octan-3-yl)-8-fluoro-2-(((3*S*,4*R*)-1,3,4-trimethylpiperidin-3-yl)methoxy)pyrido[4,3-*d*]pyrimidin-7-yl)-5-ethynyl-6-fluoronaphthalen-2-ol | LCMS (m/z): 599.3 (M + H). |
| 68-2 | 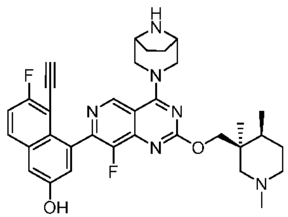 | 4-(4-((1*R*,5*S*)-3,8-diazabicyclo[3.2.1]octan-3-yl)-8-fluoro-2-(((3*R*,4*R*)-1,3,4-trimethylpiperidin-3-yl)methoxy)pyrido[4,3-*d*]pyrimidin-7-yl)-5-ethynyl-6-fluoronaphthalen-2-ol or 4-(4-((1*R*,5*S*)-3,8-diazabicyclo[3.2.1]octan-3-yl)-8-fluoro-2-(((3*S*,4*S*)-1,3,4-trimethylpiperidin-3-yl)methoxy)pyrido[4,3-*d*]pyrimidin-7-yl)-5-ethynyl-6-fluoronaphthalen-2-ol | LCMS (m/z): 599.3 (M + H). $^1$H NMR (400 MHz, Methanol-$d_4$) $\delta$ 9.02 (d, *J* = 1.5 Hz, 1H), 7.88 (dd, *J* = 9.1, 5.7 Hz, 1H), 7.38 - 7.31 (m, 2H), 7.23 (d, J = 2.6 Hz, 1H), 4.69 - 4.53 (m, 3H), 4.47 - 4.40 (m, 1H), 3.78 - 3.66 (m, 4H), 3.37 (dd, *J* = 7.5, 1.0 Hz, 1H), 3.18 - 3.11 (m, 1H), 2.95 - 2.88 (m, 1H), 2.29 (s, 3H), 2.14 - 2.05 (m, 1H), 1.93 - 1.77 (m, 5H), 1.68 - 1.61 (m, 2H), 1.52 - 1.45 (m, 1H), 1.12 (s, 3H), 1.05 (d, *J* = 7.0 Hz, 3H). |

(continued)

| Examples | Structure | Name | Characterization data |
|---|---|---|---|
| 69-1 | 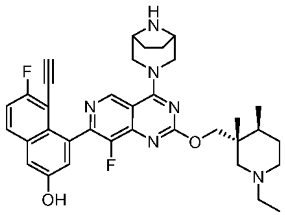 or 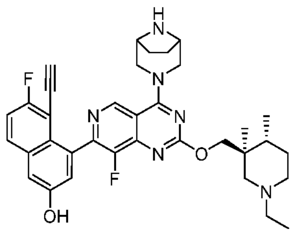 | 4-(4-((1*R*,5*S*)-3,8-diazabicyclo[3.2.1]octan-3-yl)-2-(((3*R*,4*S*)-1-ethyl-3,4-dimethylpiperidin-3-yl)methoxy)-8-fluoropyrido[4,3-*d*]pyrimidin-7-yl)-5-ethynyl-6-fluoronaphthalen-2-ol or 4-(4-((1R,5S)-3,8-diazabicyclo[3.2.1]octan-3-yl)-2-(((3S,4R)-1-ethyl-3, 4-dimethylpiperidin-3-yl)methoxy)-8-fluoropyrido[4,3-*d*]pyrimidin-7-yl)-5-ethynyl-6-fluoronaphthalen-2-ol | LCMS (m/z): 613.3 (M + H). |
| 69-2 | 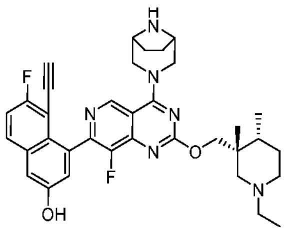 or 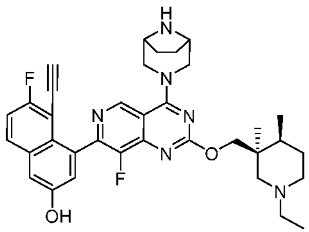 | 4-(4-((1*R*,5*S*)-3,8-diazabicyclo[3.2.1]octan-3-yl)-2-(((3*R*,4*R*)-1-ethyl-3,4-dimethylpiperidin-3-yl)methoxy)-8-fluoropyrido[4,3-*d*]pyrimidin-7-yl)-5-ethynyl-6-fluoronaphthalen-2-ol or 4-(4-((1*R*,5*S*)-3,8-diazabicyclo[3.2.1]octan-3-yl)-2-(((3*S*,4*S*)-1-ethyl-3,4-dimethylpiperidin-3-yl)methoxy)-8-fluoropyrido[4,3-*d*]pyrimidin-7-yl)-5-ethynyl-6-fluoronaphthalen-2-ol | LCMS (m/z): 613.3 (M + H). |

(continued)

| Examples | Structure | Name | Characterization data |
|---|---|---|---|
| 70-1 | 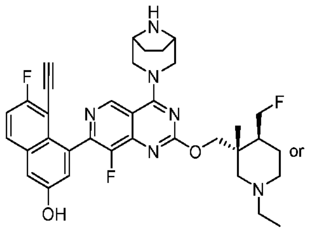 or 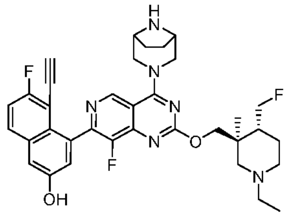 | 4-(4-((1*R*,5*S*)-3,8-diazabicyclo[3.2.1]octan-3-yl)-2-(((3*R*,4*S*)-1-ethyl-4-(fluoromethyl)-3-methylpiperidin-3-yl)methoxy)-8-fluoropyrido[4,3-*d*]pyrimidin-7-yl)-5-ethynyl-6-fluoronaphthalen-2-ol or 4-(4-((1*R*,5*S*)-3,8-diazabicyclo[3.2.1]octan-3-yl)-2-(((3*S*,4*R*)-1-ethyl-4-(fluoromethyl)-3-methylpiperidin-3-yl)methoxy)-8-fluoropyrido[4,3-*d*]pyrimidin-7-yl)-5-ethynyl-6-fluoronaphthalen-2-ol | LCMS (m/z): 631.3 (M + H). |
| 70-2 | 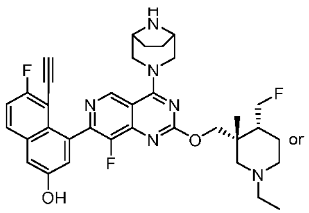 or 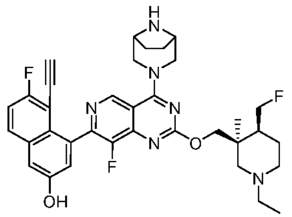 | 4-(4-((1*R*,5*S*)-3,8-diazabicyclo[3.2.1]octan-3-yl)-2-(((3*R*,4*R*)-1-ethyl-4-(fluoromethyl)-3-methylpiperidin-3-yl)methoxy)-8-fluoropyrido[4,3-*d*]pyrimidin-7-yl)-5-ethynyl-6-fluoronaphthalen-2-ol or 4-(4-((1*R*,5*S*)-3,8-diazabicyclo[3.2.1]octan-3-yl)-2-((3*S*,4*S*)-1-ethyl-4-(fluoromethyl)-3-methylpiperidin-3-yl)methoxy)-8-fluoropyrido[4,3-*d*]pyrimidin-7-yl)-5-ethynyl-6-fluoronaphthalen-2-ol | LCMS (m/z): 631.3 (M + H). |

(continued)

| Examples | Structure | Name | Characterization data |
|---|---|---|---|
| 71-1 | 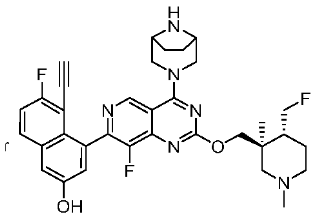 | 4-(4-((1*R*,5*S*)-3,8-diazabicyclo[3.2.1]octan-3-yl)-8-fluoro-2-((((3*R*,4*S*)-4-(fluoromethyl)-1,3-dimethylpiperidin-3-yl)methoxy)pyrido[4,3-*d*]pyrimidin-7-yl)-5-ethynyl-6-fluoronaphthalen-2-ol or 4-(4-((1*R*,5*S*)-3,8-diazabicyclo[3.2.1]octan-3-yl)-8-fluoro-2-((((3S,4R)-4-(fluoromethyl)-1,3-dimethylpiperidin-3-yl)methoxy)pyrido[4,3-*d*]pyrimidin-7-yl)-5-ethynyl-6-fluoronaphthalen-2-ol | LCMS (m/z): 617.3 (M + H). |

**Example 71-2**

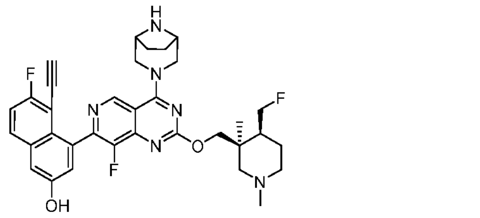

4-(4-((1*R*,5*S*)-3,8-diazabicyclo[3.2.1]octan-3-yl)-8-fluoro-2-((((3*S*,4*S*)-4-(fluoromethyl)-1,3-dimethylpiperidin-3-yl)methoxy)pyrido[4,3-*d*]pyrimidin-7-yl)-5-ethynyl-6-fluoronaphthalen-2-ol

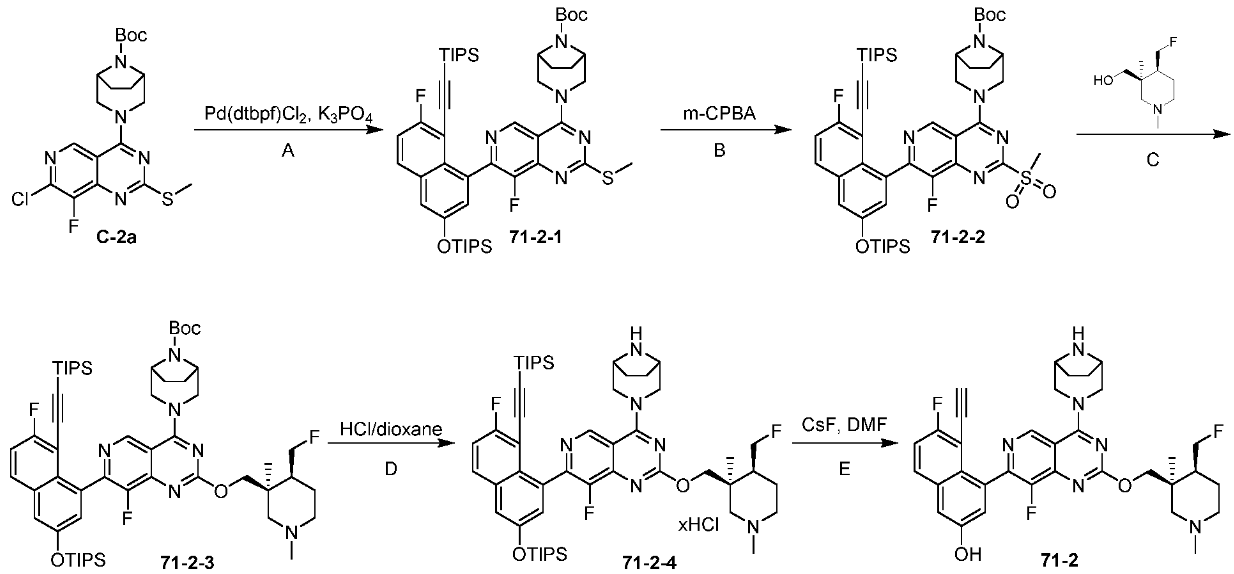


**Step A:** *tert*-butyl (1*R*,5*S*)-3-(8-fluoro-7-(7-fluoro-8-(triisopropylsilyl)ethynyl)-3-((triisopropylsilyl)oxy)naphthalen-1-yl)-2-(methylthio)pyrido[4,3-*d*]pyrimidin-4-yl)-3,8-diazabicyclo[3.2.1]octane-8-carboxylate

Under $N_2$, (7-fluoro-8-((triisopropylsilyl)ethynyl)-3-((triisopropylsilyl)oxy)naphthalen-1-yl) boric acid (925 mg, 1.7 mmol), potassium phosphate (482 mg, 2.3 mmol), and Pd(dtbpf)$Cl_2$ (127 mg, 0.11 mmol) were added to a solution of tert-butyl (1R,5S)-3-(7-chloro-8-fluoro-2-(methylthio)pyrido[4,3-d]pyrimidin-4-yl)-3,8-diazabicyclo[3.2.1]octane-8-carboxylate (500 mg, 1.14 mmol) in 1,4-dioxane (10 mL) and water (2 mL). The reaction was stirred at 110 °C for 2 h. After the reaction was completed (monitored by LCMS), the reaction was filtered by celite. The filtrate was concentrated and dried, and the resulting crude product was purified by FCC ($SiO_2$, EA/PE = 0 - 30%) to obtain *tert*-butyl (1*R*,5*S*)-3-(8-fluoro-7-(7-fluoro-8-(triisopropylsilyl)ethynyl)-3-((triisopropylsilyl)oxy)naphthalen-1-yl)-2-(methylthio)pyrido[4,3-*d*]pyrimidin-4-yl)-3,8-diazabicyclo[3.2.1]octane-8-carboxylate (220 mg, yield 20%) as a yellow solid. LCMS (m/z): 902.0 (M + H).

**Step B:** *tert*-butyl (1*R*,5*S*)-3-(8-fluoro-7-(7-fluoro-8-((triisopropylsilyl)ethynyl)-3-((triisopropylsilyl)oxy)naphthalen-1-yl)-2-(methylsulfonyl)pyrido[4,3-*d*]pyrimidin-4-yl)-3,8-diazabicyclo[3.2.1]octane-8-carboxylate

At room temperature, *m*-chloroperoxybenzoic acid (123 mg, 0.61 mmol) was added to a solution of *tert*-butyl (1*R*,5*S*)-3-(8-fluoro-7-(7-fluoro-8-(triisopropylsilyl)ethynyl)-3-((triisopropylsilyl)oxy)naphthalen-1-yl)-2-(methylthio)pyrido[4,3-*d*]pyrimidin-4-yl)-3,8-diazabicyclo[3.2.1]octane-8-carboxylate (220 mg, 0.24 mmol) in dichloromethane (5 mL). The reaction was stirred at the same temperature for 1 h. After the reaction was completed (monitored by LCMS), 20 mL of saturated sodium bicarbonate solution was added and then the resulting mixture was extracted with dichloromethane. The combined organic phase was washed with brine, dried over anhydrous sodium sulfate, filtered, concentrated, and dried to

obtain a crude product tert-butyl (1*R*,5*S*)-3-(8-fluoro-7-(7-fluoro-8-((triisopropylsilyl)ethynyl)-3-((triisopropylsilyl)oxy) naphthalen-1-yl)-2-(methylsulfonyl)pyrido[4,3-*d*]pyrimidin-4-yl)-3,8-diazabicyclo[3.2.1]octane-8-carboxylate (230 mg, yield 99%). LCMS (m/z): 934.4 (M + H).

**Step C:** *tert*-butyl (1*R*,5*S*)-3-(8-fluoro-7-(7-fluoro-8-((triisopropylsilyl)ethynyl)-3-((triisopropylsilyl)oxy)naphthalen-1-yl)-2-(((3*S*,4*S*)-4-(fluoromethyl)-1,3-dimethylpiperidin-3-yl)methoxy)pyrido[4,3-*d*]pyrimidin-4-yl)-3, 8-diazabicyclo [3.2.1]octane-8-carboxylate

Under an ice bath, sodium hydride (7 mg, 0.17 mmol) was added to a solution of ((3*S*,4*S*)-4-(fluoromethyl)-1,3-dimethylpiperidin-3-yl) methanol (18 mg, 0.10 mol) in anhydrous tetrahydrofuran (2 mL). After stirring the reaction mixture at this temperature for 0.5 h, *tert*-butyl (1*R*,5*S*)-3-(8-fluoro-7-(7-fluoro-8-((triisopropylsilyl)ethynyl)-3-((triisopropylsilyl) oxy)naphthalen-1-yl)-2-(methylsulfonyl)pyrido[4,3-*d*]pyrimidin-4-yl)-3,8-diazabicyclo[3.2.1]octane-8-carboxylate (80 mg, 0.08 mmol) was added to the reaction. The reaction was stirred at room temperature for 3 h. After the reaction was completed (monitored by LCMS), the reaction mixture was poured into 50 mL of saturated ammonium chloride solution to quench the reaction. The mixture was extracted with ethyl acetate. The combined organic phase was washed with brine, dried over anhydrous sodium sulfate, filtered, concentrated to dryness to give a crude product *tert*-butyl (1*R*,5*S*)-3-(8-fluoro-7-(7-fluoro-8-((triisopropylsilyl)ethynyl)-3-((triisopropylsilyl)oxy)naphthalen-1-yl)-2-(((3*S*,4S)-4-(fluoromethyl)-1,3-dimethylpiperidin-3-yl)methoxy)pyrido[4,3-*d*]pyrimidin-4-yl)-3,8-diazabicyclo[3.2.1] octane-8-carboxylate (82 mg, yield 93%). LCMS (m/z): 1029.5 (M + H).

**Step D:** 4-((1*R*,5*S*)-3,8-diazabicyclo[3.2.1]octan-3-yl)-8-fluoro-7-(7-fluoro-8-((triisopropylsilyl)ethynyl)-3-((triisopropylsilyl)oxy)naphthalen-1-yl)-2-(((3*S*,4*S*)-4-(fluoromethyl)-1,3-dimethylpiperidin-3-yl)methoxy)pyrido[4,3-*d*]pyrimidine-hydrochloride

At room temperature, 1.0 mL HCl (4 N in 1,4-dioxane solution) was added to a solution of *tert*-butyl (1*R*,5*S*)-3-(8-fluoro-7-(7-fluoro-8-((triisopropylsilyl)ethynyl)-3-((triisopropylsilyl)oxy)naphthalen-1-yl)-2-(((3*S*,4*S*)-4-(fluoromethyl)-1,3-dimethylpiperidin-3-yl)methoxy)pyrido[4,3-*d*]pyrimidin-4-yl)-3,8-diazabicyclo[3.2.1]octane-8-carboxylate (82 mg, 0.05 mmol) in dichloromethane (1.0 mL). The reaction was stirred for 1h at room temperature. After the reaction was completed (monitored by LCMS), the reaction was concentrated and dried to give the crude product 4-((1*R*,5*S*)-3,8-diazabicyclo[3.2.1]octan-3-yl)-8-fluoro-7-(7-flu1oro-8-((triisopropylsilyl)ethynyl)-3-((triisopropylsilyl)oxy)naphthalen-1-yl)-2-(((3*S*,4*S*)-4-(fluoromethyl)-1,3-dimethylpiperidin-3-yl)methoxy)pyrido[4,3-*d*]pyrimidine·hydrochloride (110 mg). LCMS (m/z): 929.5 (M + H).

**Step E:** 4-(4-((1*R*,5*S*)-3,8-diazabicyclo[3.2.1]octan-3-yl)-8-fluoro-2-(2-((3S,4S)-4-(fluoromethyl)-1,3-dimethylpiperidin-3-yl)ethyl)pyrido[4,3-*d*]pyrimidin-7-yl)-5-ethynyl-6-fluoronaphthalen-2-ol

At room temperature, cesium fluoride (333 mg, 2.19 mmol) was added to a solution of 4-((1R,55)-3,8-diazabicyclo[3.2.1]octan-3-yl)-8-fluoro-7-(7-fluoro-8-((triisopropylsilyl)ethynyl)-3-((triisopropylsilyl)oxy)naphthalen-1-yl)-2-(((3*S*,4*S*)-4-(fluoromethyl)-1,3-dimethylpiperidin-3-yl)methoxy)pyrido[4,3-*d*]pyrimidine·hydrochloride (110 mg) in DMF (2 mL). The reaction was stirred at 50 °C for 1 h. After the reaction was completed (monitored by LCMS), the reaction was purified by pre-HPLC (C18, CAN/(10 mmol $NH_4HCO_3/H_2O$) = 30-70%) to afford 4-(4-((1*R*,5*S*)-3,8-diazabicyclo[3.2.1]octan-3-yl)-8-fluoro-2-(2-((3*S*,4*S*)-4-(fluoromethyl)-1,3-dimethylpiperidin-3-yl)ethyl)pyrido[4,3-d]pyrimidin-7-yl)-5-ethynyl-6-fluoronaphthalen-2-ol (17 mg, yield 25%) as a yellow solid. $^1$H NMR (400 MHz, DMSO-*d*$_6$) δ 10.24 (s, 1H), 9.03 (s, 1H), 7.97 (dd, *J* = 9.2, 5.9 Hz, 1H), 7.50 - 7.42 (m, 1H), 7.38 (d, *J* = 2.5 Hz, 1H), 7.17 (d, *J* = 2.5 Hz, 1H), 4.76 - 4.60 (m, 1H), 4.54 - 4.37 (m, 3H), 4.35 - 4.25 (m, 2H), 3.96 - 3.87 (m, 1H), 3.66 - 3.52 (m, 4H), 2.85 - 2.72 (m, 2H), 2.67 - 2.56 (m, 1H), 2.11 (s, 3H), 1.90 - 1.81 (m, 1H), 1.69 - 1.54 (m, 7H), 1.06 (s, 3H). $^{19}$F NMR (376 MHz, DMSO-*d*$_6$) δ-110.85,-139.99,-219.06. LCMS (m/z): 617.3 (M + H).

| | | | |
|---|---|---|---|
| 72 | 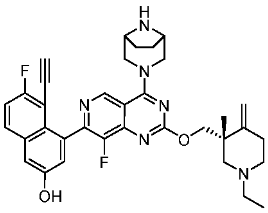 or 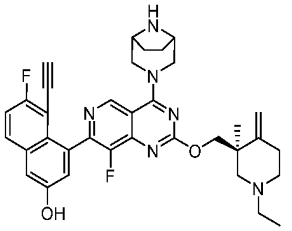 | 4-(4-(4-((1*R*,5S)-3,8-diazabicyclo[3.2.1]octan-3-yl)-2-(((*R* or *S*)-1-ethyl-3-methyl-4-methylenepiperidin-3-yl)methoxy)-8-fluoropyrido [4,3-*d*]pyrimidin-7-yl)-5-ethynyl-6-fluoronaphthalen-2-ol | LCMS (m/z): 611.3 (M + H). |
| 73 | 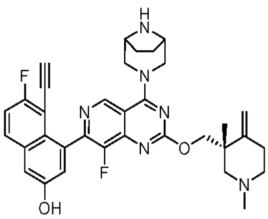 or 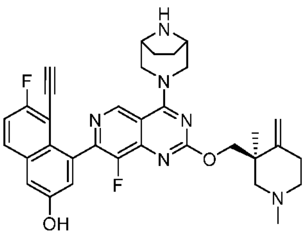 | 4-(4-(4-((1*R*,5S)-3,8-diazabicyclo[3.2.1]octan-3-yl)-2-(((*R* or *S*)-1,3-dimethyl-4-methylenepiperidin-3-yl)methoxy)-8-fluoropyrido[4,3-*d*]pyrimidin-7-yl)-5-ethynyl-6-fluoronaphthalen-2-ol | LCMS (m/z): 597.3 (M + H). |
| 74 | 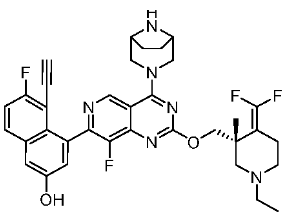 or 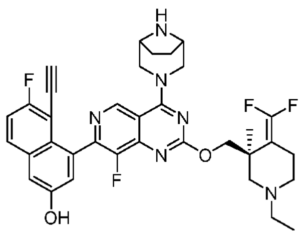 | 4-(4-(4-((1*R*,5*S*)-3,8-diazabicyclo[3.2.1]octan-3-yl)-2-(((*R* or *S*)-4-(difluoromethylene)-1-ethyl-3-methylpiperidin-3-yl)methoxy)-8-fluoropyrido[4,3-*d*]pyrimidine-7-yl)-5-ethynyl-6-fluoronaphthalen-2-ol | LCMS (m/z): 647.3 (M + H). |

(continued)

| | | | |
|---|---|---|---|
| 75 | 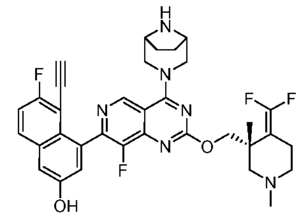 or 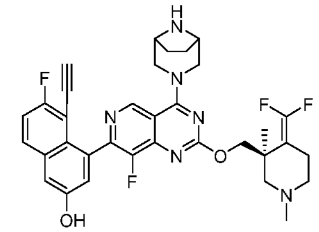 | 4-(4-(4-((1*R*,5*S*)-3,8-diazabicyclo[3.2.1]octan-3-yl)-2-(((*R* or *S*)-4-(difluoromethylene)-1,3-dimethylpiperidin-3-yl)methoxy)-8-fluoropyrido[4,3-*d*]pyrimidin-7-yl)-5-ethynyl-6-fluoronaphthalen-2-ol | LCMS (m/z): 633.3 (M + H). |
| 76 | 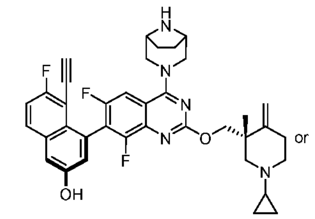 or 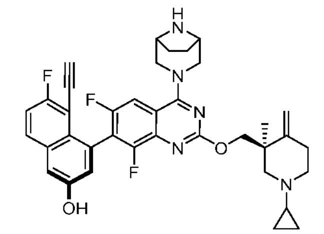 | (*Ra*)-4-(4-((*1R,5S*)-3,8-diazabicyclo[3.2.1]octan-3-yl)-2-(((*R* or *S*)-1-cyclopropyl-3-methyl-4-methylenepiperidin-3-yl)methoxy)-6,8-difluoroquinazolin-7-yl)-5-ethynyl-6-fluoronaphthalen-2-ol | LCMS (m/z): 640.3 (M + H). |

**Example 77**

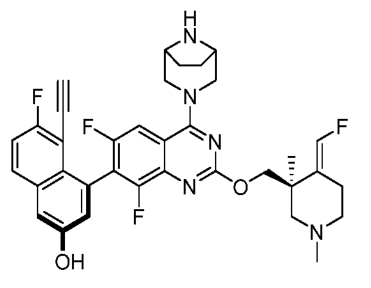

(*Ra*)-4-(4-((1*R*,5*S*)-3,8-diazabicyclo[3.2.1]octan-3-yl)-6,8-difluoro-2-(((*S*,*E*)-4-(fluoromethylene)-1,3-dimethylpiperidin-3-yl)methoxy)quinazolin-7-yl)-5-ethynyl-6-fluoronaphthalen-2-ol

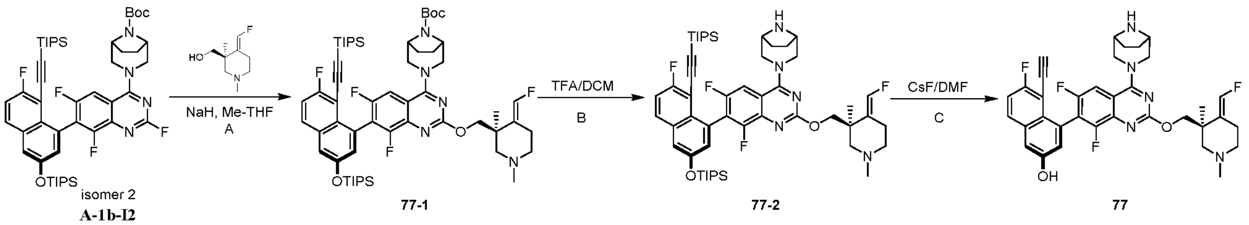


isomer 2 A-1b-I2 77-1 77-2 77

**Step A:** (*Ra*)-(1*R*,5*S*)-3-(6,8-difluoro-7-(7-fluoro-8-((triisopropylsilyl)ethynyl)-3-((triisopropylsilyl)oxy)naphthalen-1-yl)-2-(((*S,E*)-4-(fluoromethylene)-1,3-dimethylpiperidin-3-yl)methoxy)quinazolin-4-yl)-3,8-diazabicyclo[3.2.1]octane-8-carboxylate

Under an ice bath, NaH (67.3 mg, 1.68 mmol) was added to a solution of 1-*tert*-butyl 3-methyl (E)-4-(fluoromethylene)-3-methylpiperidin-1,3-dicarboxylate (intermediate **a-11A-1,** 116.6 mg, 0.673 mmol) in anhydrous THF (10 mL), the reaction was stirred at the same temperature for 20 min. Then, *tert*-butyl (*Ra*)-(1*R*,5*S*)-3-(2,6,8-trifluoro-7-(7-fluoro-8-((triisopropylsilyl)ethynyl)-3-((triisopropylsilyl)oxy)naphthalen-1-yl)quinazolin-4-yl)-3,8-diazabicyclo[3.2.1]octane-8-carboxylate (A-1b-I2, 500 mg, 0.56 mmol) was added, and the reaction was stirred at room temperature overnight. After the reaction was completed (monitored by LCMS), 20 mL of saturated aqueous ammonium chloride was added to the reaction and the resulting mixture was extracted with ethyl acetate. The combined organic phase was dried by anhydrous sodium sulfate, filtered, and concentrated to give (*Ra*)-(1*R*,5*S*)-3-(6,8-difluoro-7-(7-fluoro-8-((triisopropylsilyl)ethynyl)-3-((triisopropylsilyl)oxy)naphthalen-1-yl)-2-(((*S,E*)-4-(fluoromethylene)-1,3-dimethylpiperidin-3-yl)methoxy)quinazolin-4-yl)-3,8-diazabicyclo[3.2.1]octane-8-carboxylate (625 mg, yield 73%) as a yellow oil crude product. LCMS (m/z): 1044.5 (M + H).

**Step B:** (*Ra*)-4-((1*R*,5*S*)-3,8-diazabicyclo[3.2.1]octan-3-yl)-6,8-difluoro-7-(7-fluoro-8-((triisopropylsilyl)ethynyl)-3-((triisopropylsilyl)oxy)naphthalen-1-yl)-2-(((*S,E*)-4-(fluoromethylene)-1,3-dimethylpiperidin-3-yl)methoxy)quinazoline

(*Ra*)-(1*R*,5*S*)-3-(6,8-difluoro-7-(7-fluoro-8-((triisopropylsilyl)ethynyl)-3-((triisopropylsilyl)oxy)naphthalen-1-yl)-2-(((*S,E*)-4-(fluoromethylene)-1,3-dimethylpiperidin-3-yl)methoxy)quinazolin-4-yl)-3,8-diazabicyclo[3.2.1]octane-8-carboxylate (623 mg, 0.311 mmol) was dissolved in trifluoroacetic acid (10 mL), and the reaction was stirred at room temperature for 1 h. After the reaction was completed (monitored by LCMS), the reaction was concentrated to dryness. Ethyl acetate (20 mL) and water (20 mL) were added to the residue, and saturated sodium bicarbonate solution was added to adjust the aqueous phase to neutral. The resulting mixture was extracted with ethyl acetate, and the combined organic phase was dried over anhydrous sodium sulfate, filtered, and concentrated to obtain (*Ra*)-4-((1*R*,5*S*)-3,8-diazabicyclo[3.2.1]octan-3-yl)-6,8-difluoro-7-(7-fluoro-8-((triisopropylsilyl)ethynyl)-3-((triisopropylsilyl)oxy)naphthalen-1-yl)-2-(((*S,E*)-4-(fluoromethylene)-1,3-dimethylpiperidin-3-yl)methoxy)quinazoline (600 mg, yield 100%) as a yellow solid. LCMS (m/z): 944.5 (M + H).

**Step C:** (*Ra*)-4-(4-((1*R*,5*S*)-3,8-diazabicyclo[3.2.1]octan-3-yl)-6,8-difluoro-2-(((*S*,*E*)-4-(fluoromethylene)-1,3-dimethylpiperidin-3-yl)methoxy)quinazolin-7-yl)-5-ethynyl-6-fluoronaphthalen-2-ol

A solution of (*Ra*)-4-((1*R*,5S)-3,8-diazabicyclo[3.2.1]octan-3-yl)-6,8-difluoro-7-(7-fluoro-8-((triisopropylsilyl)ethynyl)-3-((triisopropylsilyl)oxy)naphthalen-1-yl)-2-(((*S,E*)-4-(fluoromethylene)-1,3-dimethylpiperidin-3-yl)methoxy)quinazoline (600 mg, 0.635 mmol) and cesium fluoride (500 mg, 3.29 mmol) in DMF (5 mL) was heated to 50 °C and stirred for 1.5 h. After the reaction was completed (monitored by LCMS), the reaction was purified by pre-HPLC (C18, CAN/(10 mM $NH_4HCO_3/H_2O$)=5-95%) to obtain (*Ra*)-4-(4-((1*R*,5*S*)-3,8-diazabicyclo[3.2.1]octan-3-yl)-6,8-difluoro-2-(((S,E)-4-(fluoromethylene)-1,3-dimethylpiperidin-3-yl)methoxy)quinazolin-7-yl)-5-ethynyl-6-fluoronaphthalen-2-ol (108 mg, yield 27%). LCMS (m/z): 632.3 (M + H). $^1$H NMR (400 MHz, DMSO-$d_6$) δ 10.21 (s, 1H), 7.99 (dd, *J* = 9.2, 5.9 Hz, 1H), 7.55 (d, *J* = 10.2 Hz, 1H), 7.52 - 7.45 (m, 1H), 7.41 (d, *J* = 2.6 Hz, 1H), 7.15 (d, *J* = 2.5 Hz, 1H), 6.82 - 6.58 (m, 1H), 4.57 (d, *J* = 10.6 Hz, 1H), 4.33 - 4.13 (m, 3H), 3.98 (s, 1H), 3.63 - 3.55 (m, 2H), 3.54 - 3.42 (m, 2H), 2.76 - 2.60 (m, 2H), 2.53 - 2.51 (m, 1H), 2.27 - 2.18 (m, 1H), 2.13 (s, 3H), 1.95 - 1.54 (m, 6H), 1.10 (s, 3H). $^{19}$F NMR (376 MHz, DMSO-$d_6$) δ-110.18,-119.08,-124.64,-138.94.

**Example 78**

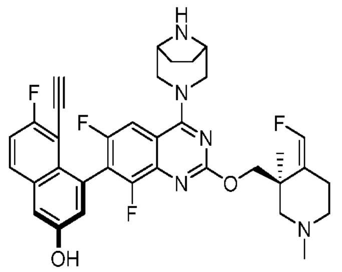

4-(4-((1*R*,5*S*)-3,8-diazabicyclo[3.2.1]octan-3-yl)-6,8-difluoro-2-(((*S*,Z)-4-(fluoromethylene)-1,3-dimethylpiperidin-3-yl) methoxy)quinazolin-7-yl)-5-ethynyl-6-fluoronaphthalen-2-ol

The synthesis of Example 78 was carried out according to the protocol described in Example 77 using (*S,Z*)-(4-(fluoromethylene)-1,3-dimethylpiperidin-3-yl)methanol (intermediate **a11A-2**) in Step A to replace 1-*tert*-butyl 3-methyl (*E*)-4-(fluoromethylene)-3-methylpiperidine-1,3-dicarboxylate (intermediate **a-11A-1**). LCMS (m/z): 632.3 (M + H). $^{1}$H NMR (400 MHz, DMSO-*$d_6$*) δ 10.22 (s, 1H), 7.99 (dd, J = 9.2, 5.9 Hz, 1H), 7.54 (d, J = 10.4, 1.7 Hz, 1H), 7.51 - 7.45 (m, 1H), 7.41 (d, J = 2.5 Hz, 1H), 7.15 (d, J = 2.5 Hz, 1H), 6.75 - 6.49 (m, 1H), 4.65 (d, J = 10.6 Hz, 1H), 4.33 - 4.13 (m, 3H), 3.99 (s, 1H), 3.56 - 3.41 (m, 4H), 2.49 - 2.46 (m, 2H), 2.28 - 2.11 (m, 4H), 2.10 - 1.98 (m, 3H), 1.81 - 1.58 (m, 4H), 1.34 - 1.25 (m, 3H). $^{19}$F NMR (376 MHz, DMSO-*$d_6$*) δ-110.18,-119.19,-124.71,-131.01.

**Example 79**

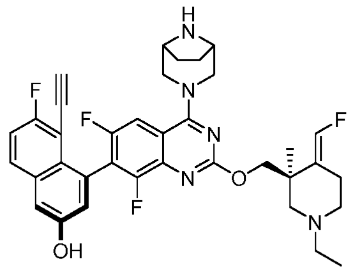

4-(4-((1*R*,5*S*)-3,8-diazabicyclo[3.2.1]octan-3-yl)-2-(((*S*,*E*)-1-ethyl-4-(fluoromethylene)-3-methylpiperidin-3-yl)methoxy)-6,8-difluoroquinazoline-7-yl)-5-ethynyl-6-fluoronaphthalene-2-ol

The synthesis of Example 79 was performed according to the protocol described in Example 77 using (*S,E*)-(1-ethyl-4-(fluoromethylene)-3-methylpiperidin-3-yl) methanol (intermediate **a11A-3)** in Step A instead of compound 1-*tert*-butyl 3-methyl (*E*)-4-(fluoromethylene)-3-methylpiperidin-1,3-dicarboxylate (intermediate **a-11A-1).** LCMS (m/z): 646.3 (M + H). $^{1}$H NMR (400 MHz, DMSO-*$d_6$*) δ 10.21 (s, 1H), 7.99 (dd, *J* = 9.2, 6.0 Hz, 1H), 7.58 - 7.52 (m, 1H), 7.51 - 7.45 (m, 1H), 7.41 (d, *J* = 2.6 Hz, 1H), 7.15 (d, *J* = 2.5 Hz, 1H), 6.80 - 6.56 (m, 1H), 4.56 (d, *J* = 10.6 Hz, 1H), 4.32 - 4.18 (m, 3H), 3.98 (s, 1H), 3.65 - 3.45 (m, 4H), 2.83 - 2.72 (m, 2H), 2.55 - 2.51 (m, 1H), 2.29 (q, *J* = 7.1 Hz, 2H), 2.24 - 2.14 (m, 1H), 1.96 - 1.88 (m, 1H), 1.87 - 1.81 (m, 1H), 1.80 - 1.74 (m, 1H), 1.74 - 1.63 (m, 3H), 1.11 (s, 3H), 0.96 (t, *J* = 7.1 Hz, 3H). $^{19}$F NMR (376 MHz, DMSO-*$d_6$*) δ 12.70,-110.17,-119.08,-124.56,-138.20.

**Example 80**

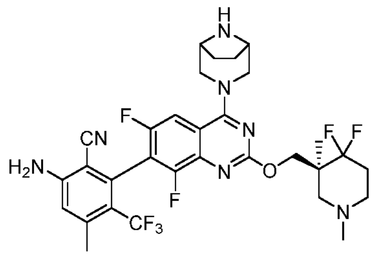

2-(4-((1*R*,5*S*)-3,8-diazabicyclo[3.2.1]octan-3-yl)-2-(((*S*)-4,4-difluoro-1,3-dimethylpiperidin-3-yl)methoxy)-6,8-difluoroquinazolin-7-yl)-6-amino-4-methyl-3-(trifluoromethyl)benzonitrile

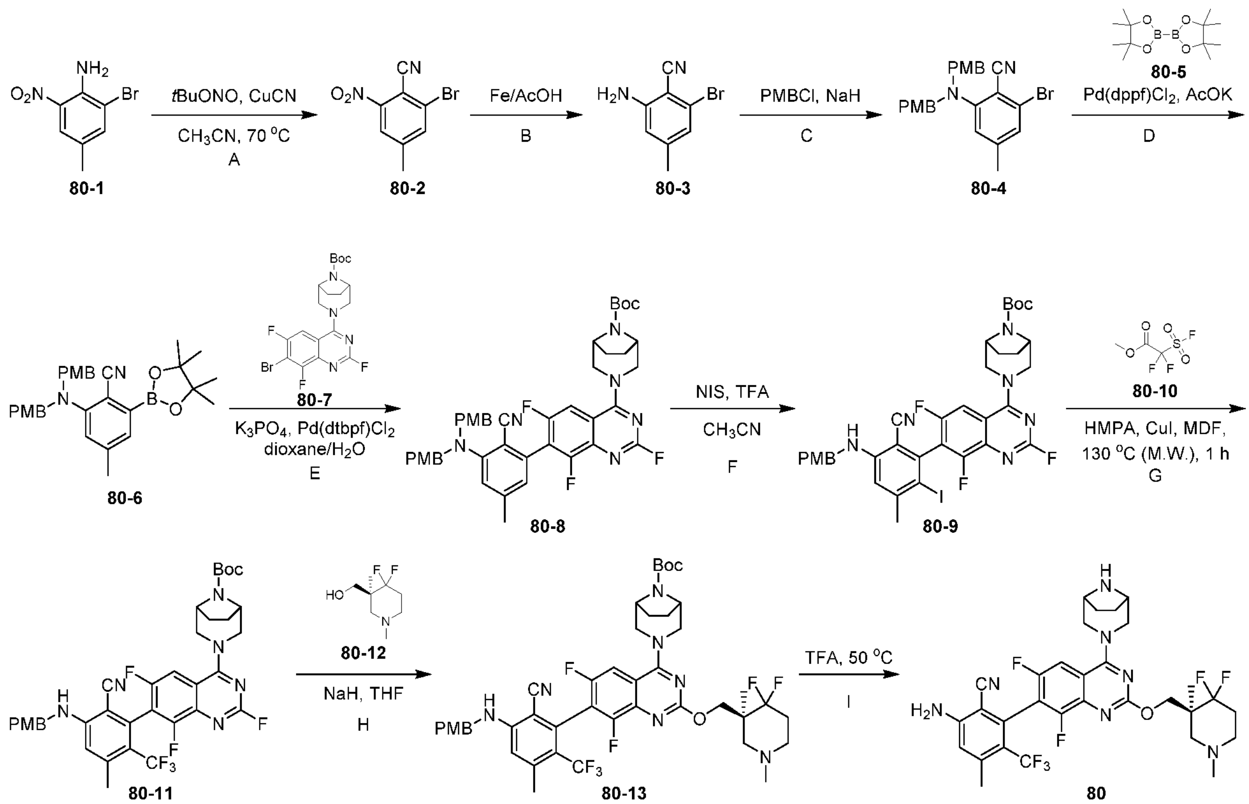


**Step A:** 2-bromo-4-methyl-6-nitrobenzonitrile

*tert*-Butyl nitrite (13.4 g, 130 mmol) was slowly added to a solution of cuprous cyanide (11.6 g, 130 mmol) in acetonitrile (150 mL) at 70 °C. After the reaction was stirred at same temperature for 20 min, a solution of 2-bromo-4-methyl-6-nitroaniline (15.0 g, 65 mmol) in acetonitrile (80 mL) was slowly added. The resulting mixture was stirred at 70 °C for 12 h. After the reaction was completed (monitored by LCMS), the reaction was filtered with celite. The filtrate was concentrated. The residue obtained was dissolved in ethyl acetate (200 mL), washed with saturated ammonium chloride and brine, dried over anhydrous sodium sulfate, filtered and concentrated to dryness. The crude product obtained was purified by FCC ($SiO_2$, EtOAc/PE = 0-10%) to 2-bromo-4-methyl-6-nitrobenzonitrile (8.5 g, yield 54%) as a yellow solid. LCMS (m/z): 240.9 (M + H).

**Step B:** 2-amino-6-bromo-4-methylbenzonitrile

Reduced iron powder (6.3g, 112 mmol) was added to a solution of 2-bromo-4-methyl-6-nitrobenzonitrile (2.7g, 11.2 mmol) in acetic acid (50 mL) at room temperature. The reaction was stirred at room temperature for 1 h. After the reaction was completed (monitored by LCMS), the reaction was filtered with celite and the filter cake was washed with ethyl acetate. The filtrate was neutralized with saturated sodium bicarbonate, washed with brine, dried over anhydrous sodium

sulfate, filtered and concentrated. The crude product obtained was purified by FCC ($SiO_2$, EtOAc/PE = 0-60%) to afford 2-amino-6-bromo-4-methylbenzonitrile (2.0 g, yield 84%) as a yellow solid. LCMS (m/z): 212.1 (M + H).

**Step C:** (2-(bis(4-methoxybenzyl)amino)-6-bromo-4-methylbenzonitrile

Under an ice bath, sodium hydride (0.4 g, 10 mmol) and PMBCl (1.42 g, 9.1 mmol) were successively added to a solution of 2-amino-6-bromo-4-methylbenzonitrile (0.96 g, 4.55 mmol) in DMF (15 mL). The reaction mixture was stirred at room temperature overnight. After the reaction was completed (monitored by LCMS), the reaction mixture was poured into 30 mL of ice water to quench the reaction. The mixture was extracted with ethyl acetate three times. The combined organic phases were washed with saturated brine, dried over anhydrous sodium sulfate, filtered, and concentrated to dryness. The resulting crude product was purified by FCC ($SiO_2$, EtOAc/PE = 0 - 10%) to afford (2-(bis(4-methoxybenzyl)amino)-6-bromo-4-methylbenzonitrile (1.7 g, yield 83%) as a yellow solid. LCMS (m/z): 451.0 (M + H).

**Step D:** 2-(bis(4-methoxybenzyl)amino)-4-methyl-6-(4,4,5,5-tetramethyl-1,3,2-dioxaborolan-2-yl) benzonitrile

Under $N_2$, bis(pinacolato)diboron (1.99 g, 7.54 mmol), potassium acetate (924 mg, 9.42 mmol), and Pd(dppf)$Cl_2$ (276 mg, 0.38 mmol) were added to a solution of (2-(bis(4-methoxybenzyl)amino)-6-bromo-4-methylbenzonitrile (1.7 g, 3.77 mmol) in 1,4-dioxane (25 mL). The resulting reaction mixture was stirred at 100 °C for 3 h. After the reaction was completed (monitored by LCMS), the reaction was filtered with celite and the filtrate was concentrated to dryness. The resulting crude product was purified by FCC ($SiO_2$, EtOAc/PE = 0-10%) to obtain 2-(bis(4-methoxybenzyl)amino)-4-methyl-6-(4,4,5,5-tetramethyl-1,3,2-dioxaborolan-2-yl) benzonitrile (2.0g, yield 99%) as a yellow solid. LCMS (m/z): 499.2 (M + H).

**Step E:** *tert*-butyl (1*R*,5*S*)-3-(7-(3-(bis(4-methoxybenzyl)amino)-2-cyano-5-methylphenyl)-2,6,8-trifluoroquinazolin-4-yl)-3,8-diazabicyclo[3.2.1]octane-8-carboxylate

Under $N_2$, *tert*-butyl (1*R*,5*S*)-3-(7-bromo-2,6,8-trifluoroquinazolin-4-yl)-3,8-diazabicyclo[3.2.1]octane-8-carboxylate (1.32 g, 3.2 mmol), potassium phosphate (2.13 g, 10.0 mmol), and Pd(dtbpf)$Cl_2$ (262 mg, 0.4 mmol) were added to a solution of 2-(bis(4-methoxybenzyl)amino)-4-methyl-6-(4,4,5,5-tetramethyl-1,3,2-dioxaborolan-2-yl) benzonitrile (2.0g, 4.0 mmol) in 1,4-dioxane (20 mL) and water (5 mL). The reaction was stirred at 100 °C for 2 h. After the reaction was completed (monitored by LCMS), the reaction was filtered through celite and the filtrate was concentrated to dryness. The resulting crude product was purified by FCC ($SiO_2$, EA/PE = 0-10%) to afford *tert*-butyl (1*R,5S*)-3-(7-(3-(bis(4-methoxybenzyl)amino)-2-cyano-5-methylphenyl)-2,6,8-trifluoroquinazolin-4-yl)-3,8-diazabicyclo[3.2.1]octane-8-carboxylate (2.2 g, yield 72%) as a yellow solid. LCMS (m/z): 765.2 (M + H).

**Step F:** *tert*-butyl (1*R*,5*S*)-3-(7-(3-((4-methoxybenzyl)amino)-2-cyano-5-methylphenyl-6-iodo)-2,6,8-trifluoroquinazolin-4-yl)-3,8-diazabicyclo[3.2.1]octane-8-carboxylate

At room temperature, trifluoroacetic acid (0.25 mL) was added to a solution of *tert*-butyl (1*R*,5*S*)-3-(7-(3-(bis(4-methoxybenzyl)amino)-2-cyano-5-methylphenyl)-2,6,8-trifluoroquinazolin-4-yl)-3,8-diazabicyclo[3.2.1]octane-8-carboxylate (1.5g, 1.96 mmol) and NIS (662 mg, 2.94 mmol) in acetonitrile. The reaction was stirred at room temperature for 2 h. After the reaction was completed (monitored by LCMS), saturated aqueous sodium bicarbonate (20 mL) was added, and the resulting mixture was extraction with ethyl acetate. The combined organic phase was washed with brine, dried over anhydrous sodium sulfate, filtered and concentrated to dryness. The resulting crude product was purified by FCC ($SiO_2$, EA/PE = 0 - 40%) to afford *tert*-butyl (1*R*,5*S*)-3-(7-(3-((4-methoxybenzyl)amino)-2-cyano-5-methylphenyl-6-iodo)-2,6,8-trifluoroquinazolin-4-yl)-3,8-diazabicyclo[3.2.1]octane-8-carboxylate (1.2 g, yield 69%) as a yellow solid. LCMS (m/z): 771.3 (M + H).

**Step G:** *tert*-butyl (1*R*,5*S*)-3-(7-(3-((4-methoxybenzyl)amino)-2-cyano-5-methylphenyl-6-trifluoromethyl)-2,6,8-trifluoroquinazolin-4-yl)-3,8-diazabicyclo[3.2.1]octane-8-carboxylate

Under $N_2$, methyl 2,2-difluoro-2-(fluorosulfonyl)acetate (150 mg, 0.78 mmol), cuprous iodide (74 mg, 0.39 mmol), and hexamethylphosphotriamine (0.2 mL) were added to a solution of *tert*-butyl (1*R*,5*S*)-3-(7-(3-((4-methoxybenzyl)amino)-2-cyano-5-methylphenyl-6-iodo)-2,6,8-trifluoroquinazolin-4-yl)-3,8-diazabicyclo[3.2.1]octane-8-carboxylate (200 mg, 0.26 mmol) in DMF (3 mL). The reaction heated to 130°C with microwave for 1 h. After the reaction was completed (monitored by LCMS), the reaction was poured into 30 mL of ice-cold water, and the resulting mixture was extracted with ethyl acetate. The combined organic phase was washed with brine, dried over anhydrous sodium sulfate, filtered and concentrated to dryness. The resulting crude product was purified by FCC ($SiO_2$, EtOAc/PE = 0 - 40%) to afford

*tert*-butyl (1*R*,5*S*)-3-(7-(3-((4-methoxybenzyl)amino)-2-cyano-5-methylphenyl-6-trifluoromethyl)-2,6,8-trifluoroquinazolin-4-yl)-3,8-diazabicyclo[3.2.1]octane-8-carboxylate (100 mg, yield 54%) as a yellow solid. LCMS (m/z): 713.3 (M + H).

**Step H:** *tert*-butyl (1*R*,5*S*)-3-(7-(2-cyano-3-((4-methoxybenzyl)amino)-5-methyl-6-(trifluoromethyl)phenyl)-2-(((*S*)-4,4-difluoro-1,3-dimethylpiperidin-3-yl)methoxy)-6,8-difluoroquinazolin-4-yl)-3,8-diazabicyclo[3.2.1]octane-8-carboxylate

Under an ice bath, (*S*)-(4,4-difluoro-1,3-dimethylpiperidin-3-yl) methanol (50 mg, 0.28 mmol) was dissolved into 3 mL of anhydrous tetrahydrofuran, then sodium hydride (11 mg, 0.28 mmol) was added, and the resulting reaction was stirred at the same temperature for 0.5 h. *tert*-butyl (1*R*,5*S*)-3-(7-(3-((4-methoxybenzyl)amino)-2-cyano-5-methylphenyl-6-trifluoromethyl)-2,6,8-trifluoroquinazolin-4-yl)-3,8-diazabicyclo[3.2.1]octane-8-carboxylate (100 mg, 0.14 mmol) was added to the reaction. The resulting mixture was stirred at room temperature for 1 h. After the reaction was completed (monitored by LCMS), the reaction mixture was poured into 30 mL of ice water to quench the reaction. The mixture was then extracted with ethyl acetate. The combined organic phases were washed with brine, dried over anhydrous sodium sulfate, filtered, and concentrated to dryness to obtain the crude product *tert*-butyl (1*R*,5*S*)-3-(7-(2-cyano-3-((4-methoxybenzyl)amino)-5-methyl-6-(trifluoromethyl)phenyl)-2-(((*S*)-4,4-difluoro-1,3-dimethylpiperidin-3-yl)methoxy)-6,8-difluoroquinazolin-4-yl)-3,8-diazabicyclo[3.2.1]octane-8-carboxylate (110 mg, yield 90%). LCMS (m/z): 873.2 (M + H).

**Step I:** 2-(4-((1*R*,5*S*)-3,8-diazabicyclo[3.2.1]octan-3-yl)-2-(((*S*)-4,4-difluoro-1,3-dimethylpiperidin-3-yl)methoxy)-6,8-difluoroquinazolin-7-yl)-6-amino-4-methyl-3-(trifluoromethyl) benzonitrile

Trifluoroacetic acid (2 mL) was added to *tert*-butyl (1*R*,5*S*)-3-(7-(2-cyano-3-((4-methoxybenzyl)amino)-5-methyl-6-(trifluoromethyl)phenyl)-2-(((*S*)-4,4-difluoro-1,3-dimethylpiperidin-3-yl)methoxy)-6,8-difluoroquinazolin-4-yl)-3,8-diazabicyclo[3.2.1]octane-8-carboxylate (100 mg, 0.11 mmol). The reaction was stirred at 50 °C for 0.5 h. After the reaction was completed (monitored by LCMS), the reaction was purified by pre-HPLC (C18, CAN/(10 mmol $NH_4HCO_3/H_2O$) = 35-65%) to obtain 2-(4-((1*R*,5*S*)-3,8-diazabicyclo[3.2.1]octan-3-yl)-2-(((S)-4,4-difluoro-1,3-dimethylpiperidin-3-yl)methoxy)-6,8-difluoroquinazolin-7-yl)-6-amino-4-methyl-3-(trifluoromethyl)benzonitrile (25 mg, yield 33%) as a white solid. $^1$H NMR (400 MHz, DMSO-$d_6$) δ 7.74 (d, *J* = 10.5 Hz, 1H), 6.97 (s, 2H), 6.91 (s, 1H), 4.51 - 4.39 (m, 2H), 4.24 (dd, *J* = 28.3, 12.2 Hz, 2H), 3.57 - 3.44 (m, 6H), 2.77 - 2.65 (m, 2H), 2.42 (s, 3H), 2.18 (s, 3H), 2.15 - 2.00 (m, 3H), 1.72 - 1.57 (m, 4H), 1.17 (s, 3H). LCMS (m/z): 652.3 (M + H).

**Intermediate 80-11-1 and 80-11-2**

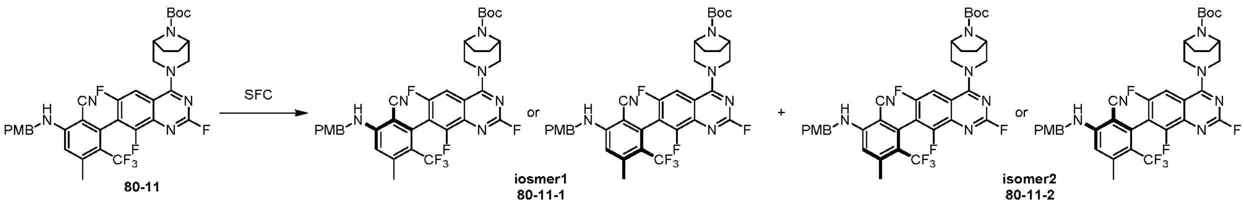


80-11 iosmer1 80-11-1 isomer2 80-11-2

The resolution of *tert*-butyl (1*R*,5*S*)-3-(7-(3-((4-methoxybenzyl)amino)-2-cyano-5-methylphenyl-6-trifluoromethyl)-2,6,8-trifluoroquinazolin-4-yl)-3,8-diazabicyclo[3.2.1]octane-8-carboxylate (Intermediate 80-11, 910 mg) was carried out with Chiral HPLC (SHIMADZU LC-20AP). Separation column: DAICEL CHIRALPAK®IA, 250*25 mm 10μm; Mobile phase: n-Hexane/ETOH (+0.1% 7.0 mol/l Ammonia in MEOH) = 60/40; flow rate 40 mL/min. The isomer 1was intermediate 80-11-1 (346 mg, with a shorter retention time), Chiral analysis method 5, Rt = 11.208, LCMS (m/z): 713.3 (M + H). The isomer 2 was intermediate 80-11-2 (362 mg, with a longer retention time), Chiral analysis method 5, Rt = 15.041, LCMS (m/z): 713.3 (M + H).

**Starting from intermediates 80-11, 80-11-1, and 80-11-2, the following compounds were prepared and characterized according to the synthetic scheme of Example 80.**

| Example | Structure | Name | Intermediate | CHARACTERIZA TION DATA |
|---|---|---|---|---|
| 80-1 | 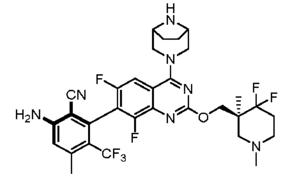 or 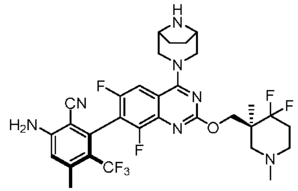 | 2-(4-((1*R*,5*S*)-3,8-diazabicyclo[3.2.1] octan-3-yl)-2-(((*S*)-4,4-difluoro-1,3-dimethylpiperidin-3-yl)methoxy)-6,8-difluoroquinazolin-7-yl)-6-amino-4-methyl-3-(trifluoromethyl)be nzonitrile | 80-11-1 | LCMS (m/z): 652.3 (M + H). |
| 80-2 | 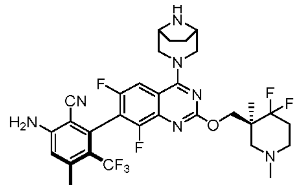 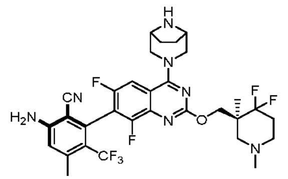 or | 2-(4-((1*R*,5*S*)-3,8-diazabicyclo[3.2.1] octan-3-yl)-2-(((*S*)-4,4-difluoro-1,3-dimethylpiperidin-3-yl)methoxy)-6,8-difluoroquinazolin-7-yl)-6-amino-4-methyl-3-(trifluoromethyl) benzonitrile | 80-11-2 | LCMS (m/z): 652.3 (M + H). |
| 81 | 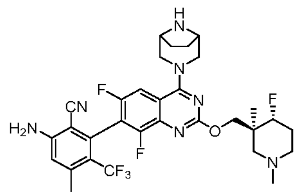 | 2-(4-((1*R*,5*S*)-3,8-diazabicyclo[3.2.1] octan-3-yl)-6,8-difluoro-2-((((3*S*,4*R*)-4-fluoro-1,3-dimethylpiperidin-3-yl)methoxy)quinaz olin-7-yl)-6-amino-4-methyl-3-(trifluoromethyl) benzonitrile | 80-11 | LCMS (m/z): 634.3 (M + H). |

(continued)

| Example | Structure | Name | Intermediate | CHARACTERIZA TION DATA |
|---|---|---|---|---|
| 81-1 | 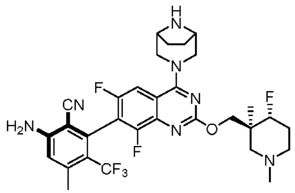 or 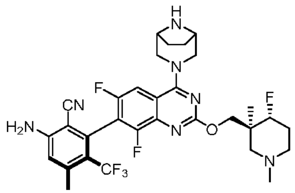 | 2-(4-((1*R*,5*S*)-3,8-diazabicyclo[3.2.1] octan-3-yl)-6,8-difluoro-2-((((3*S*,4*R*)-4-fluoro-1,3-dimethylpiperidin-3-yl)methoxy)quinaz olin-7-yl)-6-amino-4-methyl-3-(trifluoromethyl) benzonitrile | 80-11-1 | LCMS (m/z): 634.3 (M + H). |
| 81-2 | 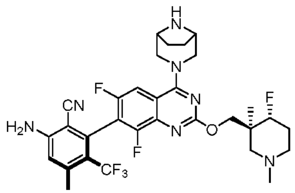 or 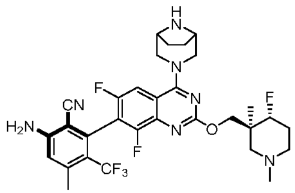 | 2-(4-((1*R*,5*S*)-3,8-diazabicyclo[3.2.1] octan-3-yl)-6,8-difluoro-2-((((3*S*,4*R*)-4-fluoro-1,3-dimethylpiperidin-3-yl)methoxy)quinaz olin-7-yl)-6-amino-4-methyl-3-(trifluoromethyl) benzonitrile | 80-11-2 | LCMS (m/z): 634.3 (M + H). |

(continued)

| Example | Structure | Name | Intermediate | CHARACTERIZA TION DATA |
|---|---|---|---|---|
| 81-3 | 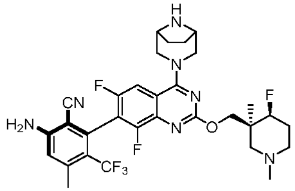 or 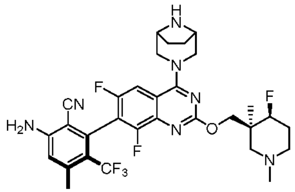 | 2-(4-((1*R*,5*S*)-3,8-diazabicyclo[3.2.1] octan-3-yl)-6,8-difluoro-2-((((3*S*,4*S*)-4-fluoro-1,3-dimethylpiperidin-3-yl)methoxy)quinaz olin-7-yl)-6-amino-4-methyl-3-(trifluoromethyl) benzonitrile | 80-11-1 | LCMS (m/z): 634.3 (M + H). |
| 81-4 | 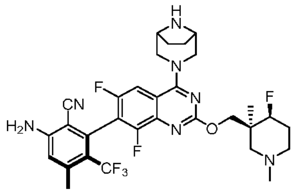 or 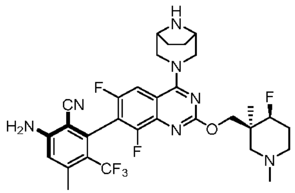 | 2-(4-((1*R*,5*S*)-3,8-diazabicyclo[3.2.1] octan-3-yl)-6,8-difluoro-2-((((3*S*,4*S*)-4-fluoro-1,3-dimethylpiperidin-3-yl)methoxy)quinaz olin-7-yl)-6-amino-4-methyl-3-(trifluoromethyl) benzonitrile | 80-11-2 | LCMS (m/z): 634.3 (M + H). |
| 82 | 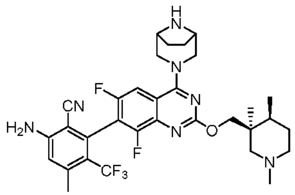 | 2-(4-(4-((1*R*,5*S*)-3,8-diazabicyclo [3.2.1] octan-3-yl)-6,8-difluoro-2-(((3*S*,4*S*)-1,3,4-trimethylpiperidin-3-yl)methoxy)quinaz olin-7-yl)-6-amino-4-methyl-3-(trifluoromethyl) benzonitrile | 80-11 | LCMS (m/z): 630.3 (M + H). |

(continued)

| Example | Structure | Name | Intermediate | CHARACTERIZA TION DATA |
|---|---|---|---|---|
| 82-1 | 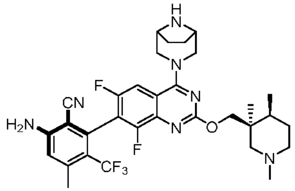 or 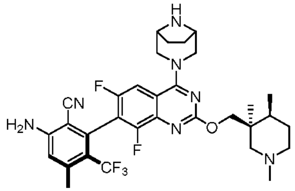 | 2-(4-(4-((1*R*,5*S*)-3,8-diazabicyclo [3.2.1] octan-3-yl)-6,8-difluoro-2-(((3*S*,4*S*)-1,3,4-trimethylpiperidin-3-yl)methoxy)quinaz olin-7-yl)-6-amino-4-methyl-3-(trifluoromethyl) benzonitrile | 80-11-1 | LCMS (m/z): 630.3 (M + H). |
| 82-2 | 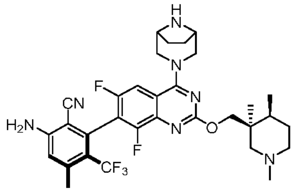 or 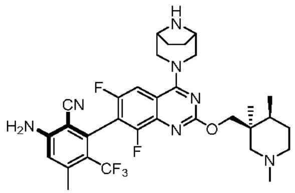 | 2-(4-(4-((1*R*,5*S*)-3,8-diazabicyclo [3.2.1] octan-3-yl)-6,8-difluoro-2-(((3*S*,4*S*)-1,3,4-trimethylpiperidin-3-yl)methoxy)quinaz olin-7-yl)-6-amino-4-methyl-3-(trifluoromethyl) benzonitrile | 80-11-2 | LCMS (m/z): 630.3 (M + H). |
| 83 | 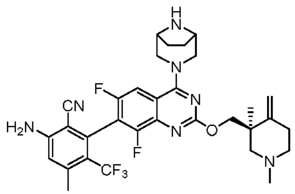 | 2-(4-((1*R*,5*S*)-3,8-diazabicyclo[3.2.1] octan-3-yl)-2-(((*S*)-1,3-dimethyl-4-methylene piperidin-3-yl)methoxy)-6,8-difluoroquinazolin-7-yl)-6-amino-4-methyl-3-(trifluoromethyl) benzonitrile | 80-11 | LCMS (m/z): 628.3 (M + H). |

(continued)

| Example | Structure | Name | Intermediate | CHARACTERIZA TION DATA |
|---|---|---|---|---|
| 83-1 | 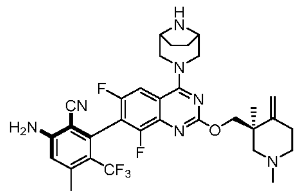 or 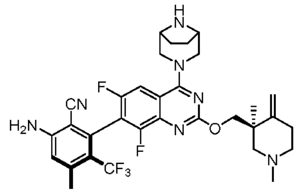 | 2-(4-((1R,5S)-3,8-diazabicyclo[3.2.1] octan-3-yl)-2-(((*S*)-1,3-dimethyl-4-methylene piperidin-3-yl)methoxy)-6,8-difluoroquinazolin-7-yl)-6-amino-4-methyl-3-(trifluoromethyl) benzonitrile | 80-11-1 | LCMS (m/z): 628.3 (M + H). |
| 83-2 | 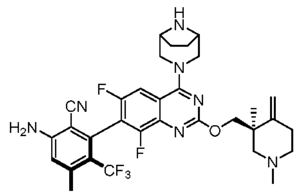 or 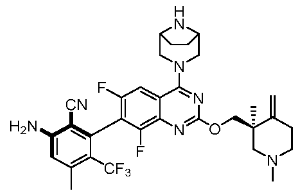 | 2-(4-((1*R*,5*S*)-3,8-diazabicyclo[3.2.1] octan-3-yl)-2-(((S)-1,3-dimethyl-4-methylene piperidin-3-yl)methoxy)-6,8-difluoroquinazolin-7-yl)-6-amino-4-methyl-3-(trifluoromethyl) benzonitrile | 80-11-2 | LCMS (m/z): 628.3 (M + H). |
| 84 | 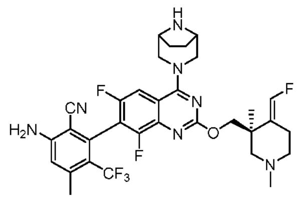 | 2-(4-((1*R*,5*S*)-3,8-diazabicyclo[3.2.1] octan-3-yl)-6,8-difluoro-2-(((*S*, *E*)-4-(fluoromethylene)-1,3-dimethylpiperidin-3-yl)methoxy)quinaz olin-7-yl)-6-amino-4-methyl-3-(trifluoromethyl) benzonitrile | 80-11 | LCMS (m/z): 646.3 (M + H). |

(continued)

| Example | Structure | Name | Intermediate | CHARACTERIZA TION DATA |
|---|---|---|---|---|
| 84-1 | 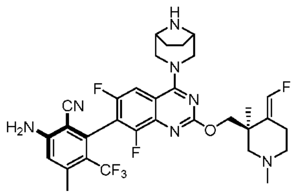 or 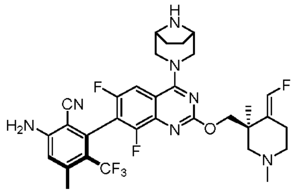 | 2-(4-((1*R*,5*S*)-3,8-diazabicyclo[3.2.1] octan-3-yl)-6,8-difluoro-2-(((*S*,*E*)-4-(fluoromethylene)-1,3-dimethylpiperidin-3-yl)methoxy)quinaz olin-7-yl)-6-amino-4-methyl-3-(trifluoromethyl) benzonitrile | 80-11-1 | LCMS (m/z): 646.3 (M + H). $^1$H NMR (400 MHz, DMSO-$d_6$) $\delta$ 7.76 (d, *J* = 10.3 Hz, 1H), 6.98 (s, 2H), 6.92 (s, 1H), 6.71 (d, *J* = 86.7 Hz, 1H), 4.59 (d, *J* = 10.5 Hz, 1H), 4.38 - 4.23 (m, 3H), 3.93 (s, 2H), 3.65 - 3.59 (m, 2H), 2.71 - 2.65 (m, 2H), 2.43 (s, 3H), 2.26 - 2.20 (m, 1H), 2.15 (s, 3H), 1.99 - 1.76 (m, 7H), 1.17 - 1.04 (m, 3H). |
| 84-2 | 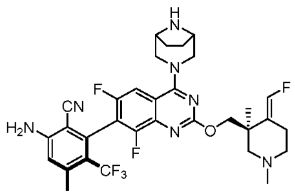 or 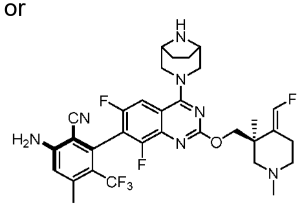 | 2-(4-((1*R*,5*S*)-3,8-diazabicyclo[3.2.1] octan-3-yl)-6,8-difluoro-2-(((*S*,*E*)-4-(fluoromethylene)-1,3-dimethylpiperidin-3-yl)methoxy)quinaz olin-7-yl)-6-amino-4-methyl-3-(trifluoromethyl) benzonitrile | 80-11-2 | LCMS (m/z): 646.3 (M + H). $^1$H NMR (400 MHz, DMSO-$d_6$) $\delta$ 7.74 (d, *J* = 10.3 Hz, 1H), 6.97 (s, 2H), 6.91 (s, 1H), 6.81 (s, 0.5 H), 6.60 (s, 0.5 H), 4.61 - 4.54 (m, 1H), 4.34 - 4.19 (m, 3H), 3.79 - 3.67 (m, 2H), 3.60 - 3.47 (m, 2H), 2.71 - 2.65 (m, 2H), 2.44 - 2.39 (m , 3H), 2.26 - 2.19 (m, 1H), 2.14 (s, 3H), 2.04 - 1.72 (m, 7H), 1.10 (s, 3H). $^{19}$F NMR (376 MHz, DMSO-$d_6$) $\delta$-53.45,-120.11,-124.26,-138.85. |
| 85 | 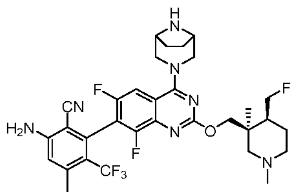 | 2-(4-((1*R*,5*S*)-3,8-diazabicyclo[3.2.1] octan-3-yl)-6,8-difluoro-2-(((3*S*,4*S*)-4-(fluoromethyl)-1,3-dimethylpiperidin-3-yl)methoxy)quinaz olin-7-yl)-6-amino-4-methyl-3-(trifluoromethyl) benzonitrile | 80-11 | LCMS (m/z): 648.3 (M + H). |

(continued)

| Example | Structure | Name | Intermediate | CHARACTERIZA TION DATA |
|---|---|---|---|---|
| 85-1 | 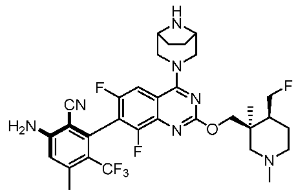 or 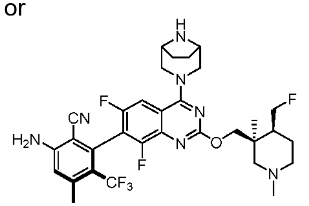 | 2-(4-((1*R*,5*S*)-3,8-diazabicyclo[3.2.1] octan-3-yl)-6,8-difluoro-2-(((3*S*,4*S*)-4-(fluoromethyl)-1,3-dimethylpiperidin-3-yl)methoxy)quinaz olin-7-yl)-6-amino-4-methyl-3-(trifluoromethyl) benzonitrile | 80-11-1 | LCMS (m/z): 648.3 (M + H). |
| 85-2 | 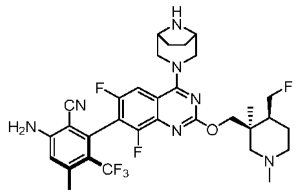 or 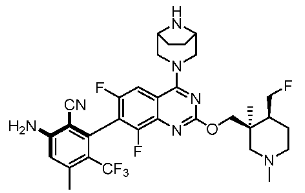 | 2-(4-((1*R*,5*S*)-3,8-diazabicyclo[3.2.1] octan-3-yl)-6,8-difluoro-2-(((3*S*,4*S*)-4-(fluoromethyl)-1,3-dimethylpiperidin-3-yl)methoxy)quinaz olin-7-yl)-6-amino-4-methyl-3-(trifluoromethyl) benzonitrile | 80-11-2 | LCMS (m/z): 648.3 (M + H). $^{1}H$ NMR (400 MHz, DMSO-$d_6$) δ 7.79 - 7.71 (m, 1H), 6.97 (s, 2H), 6.91 (s, 1H), 4.76 - 4.60 (m, 1H), 4.54 - 4.36 (m, 2H), 4.36 - 4.19 (m, 3H), 3.88 - 3.75 (m, 2H), 3.65 - 3.52 (m, 2H), 3.40 - 3.33 (m, 1H), 2.86 - 2.70 (m, 2H), 2.42 (s, 3H), 2.12 (s, 3H), 1.88 - 1.57 (m, 8H), 1.06 (s, 3H). $^{19}F$ NMR (376 MHz, DMSO-$d_6$) δ-53.42 ~-53.49,-73.42 ~-73.43,-119.89,-124.09. |
| 86 | 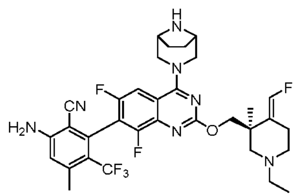 | 2-(4-((1*R*,5*S*)-3,8-diazabicyclo[3.2.1] octan-3-yl)-2-(((*S,E*)-1-ethyl-4-(fluoromethylene)-3-methylpiperidin-3-yl) methoxy)-6,8-difluoroquinazolin-7-yl)-6-amino-4-methyl-3-(trifluoromethyl) benzonitrile | 80-11 | LCMS (m/z): 660.3 (M + H). |

(continued)

| Example | Structure | Name | Intermediate | CHARACTERIZA TION DATA |
|---|---|---|---|---|
| 86-1 | 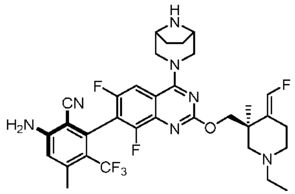 or 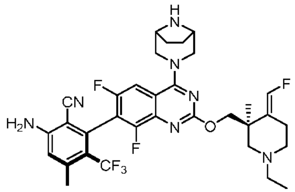 | 2-(4-((1*R*,5*S*)-3,8-diazabicyclo[3.2.1] octan-3-yl)-2-(((*S*,*E*)-1-ethyl-4-(fluoromethylene)-3-methylpiperidin-3-yl) methoxy)-6,8-difluoroquinazolin-7-yl)-6-amino-4-methyl-3-(trifluoromethyl) benzonitrile | 80-11-1 | LCMS (m/z): 660.3 (M + H). |
| 86-2 | 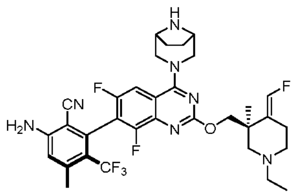 or 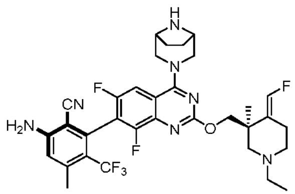 | 2-(4-((1R,55)-3,8-diazabicyclo[3.2.1] octan-3-yl)-2-(((S,E)-1-ethyl-4-(fluoromethylene)-3-methylpiperidin-3-yl) methoxy)-6,8-difluoroquinazolin-7-yl)-6-amino-4-methyl-3-(trifluoromethyl) benzonitrile | 80-11-2 | LCMS (m/z): 660.3 (M + H). |
| 87 | 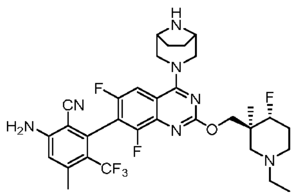 | 2-(4-((1*R*,5*S*)-3,8-diazabicyclo[3.2.1] octan-3-yl)-2-(((3*S*,4*R*)-1-ethyl-4-fluoro-3-methylpiperidin-3-yl)methoxy)-6,8-difluoroquinazolin-7-yl)-6-amino-4-methyl-3-(trifluoromethyl) benzonitrile | 80-11 | LCMS (m/z): 648.3 (M + H). |

(continued)

| Example | Structure | Name | Intermediate | CHARACTERIZA TION DATA |
|---|---|---|---|---|
| 87-1 | 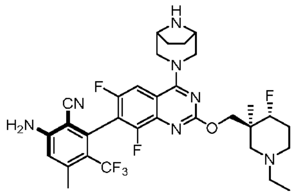 or 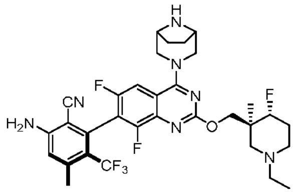 | 2-(4-((1*R*,5*S*)-3,8-diazabicyclo[3.2.1] octan-3-yl)-2-(((3*S*,4R)-1-ethyl-4-fluoro-3-methylpiperidin-3-yl)methoxy)-6,8-difluoroquinazolin-7-yl)-6-amino-4-methyl-3-(trifluoromethyl) benzonitrile | 80-11-1 | LCMS (m/z): 648.3 (M + H). |
| 87-2 | 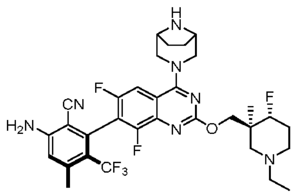 or 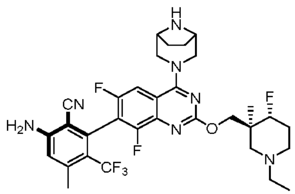 | 2-(4-((1*R*,5*S*)-3,8-diazabicyclo[3.2.1] octan-3-yl)-2-(((3*S*,4*R*)-1-ethyl-4-fluoro-3-methylpiperidin-3-yl)methoxy)-6,8-difluoroquinazolin-7-yl)-6-amino-4-methyl-3-(trifluoromethyl) benzonitrile | 80-11-2 | LCMS (m/z): 648.3 (M + H). |

(continued)

| Example | Structure | Name | Intermediate | CHARACTERIZA TION DATA |
|---|---|---|---|---|
| 87-3 | 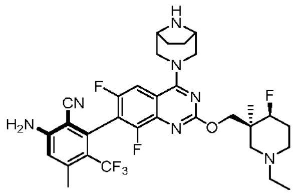 or 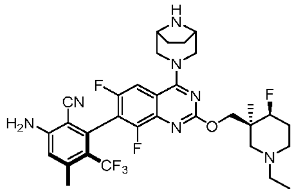 | 2-(4-((1*R*,5*S*)-3,8-diazabicyclo[3.2.1] octan-3-yl)-2-(((3*S*,4*S*)-1-ethyl-4-fluoro-3-methylpiperidin-3-yl)methoxy)-6,8-difluoroquinazolin-7-yl)-6-amino-4-methyl-3-(trifluoromethyl) benzonitrile | 80-11-1 | LCMS (m/z): 648.3 (M + H). |
| 87-4 | 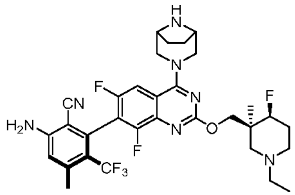 or 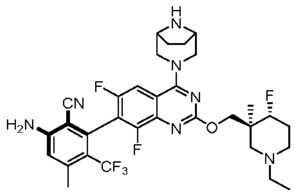 | 2-(4-((1*R*,5*S*)-3,8-diazabicyclo[3.2.1] octan-3-yl)-2-(((3*S*,4*S*)-1-ethyl-4-fluoro-3-methylpiperidin-3-yl)methoxy)-6,8-difluoroquinazolin-7-yl)-6-amino-4-methyl-3-(trifluoromethyl) benzonitrile | 80-11-2 | LCMS (m/z): 648.3 (M + H). |

(continued)

| Example | Structure | Name | Intermediate | CHARACTERIZA TION DATA |
|---|---|---|---|---|
| 288-1 | 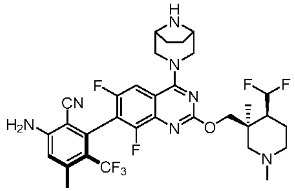 or 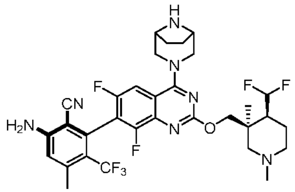 | 2-(4-((1*R*,5*S*)-3,8-diazabicyclo[3.2.1] octan-3-yl)-2-(((3*S*,4*S*)-4-(difluoromethyl)-1,3-dimethylpiperidin-3-yl) methoxy)-6,8-difluoroquinazolin-7-yl)-6-amino-4-methyl-3-(trifluoromethyl) benzonitrile | 80-11-1 | LCMS (m/z): 666.3 (M + H). |
| 288-2 | 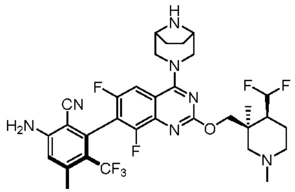 or 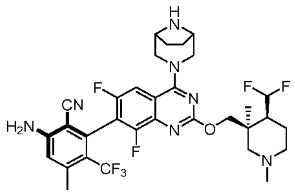 | 2-(4-((1*R*,5*S*)-3,8-diazabicyclo[3.2.1] octan-3-yl)-2-(((3*S*,4*S*)-4-(difluoromethyl)-1,3-dimethylpiperidin-3-yl) methoxy)-6,8-difluoroquinazolin-7-yl)-6-amino-4-methyl-3-(trifluoromethyl) benzonitrile | 80-11-2 | LCMS (m/z): 666.3 (M + H). $^1$H NMR (400 MHz, Methanol-$d_4$) $\delta$ 7.63 (d, $J$ = 10.0 Hz, 1H), 6.91 (s, 1H), 6.22 (t, $J$ = 55.5 Hz, 1H), 4.60 - 4.46 (m, 4H), 3.89 - 3.75 (m, 2H), 3.72 - 3.59 (m, 2H), 3.23 - 3.04 (m, 2H), 2.53 - 2.43 (m, 3H), 2.36 (s, 3H), 2.24 - 2.11 (m, 1H), 2.00 - 1.83 (m, 8H), 1.23 (s, 3H). |

Referring to the above synthetic method or appropriate variants, this invention also incorporated and characterized the following compounds:

| | | | |
|---|---|---|---|
| 88 | 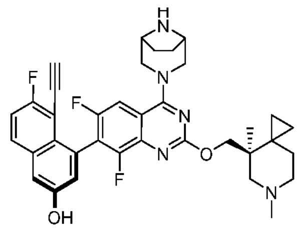 | 4-(4-((1*R*,5*S*)-3,8-diazabicyclo[3.2.1]octan-3-yl)-2-(((*S*)-4,6-dimethyl-6-azaspiro[2.5]octan-4-yl)methoxy)-6,8-difluoroquinazolin-7-yl)-5-ethynyl-6-fluoronaphthalen-2-ol | LCMS (m/z): 628.3 (M + H). |
| 89 | 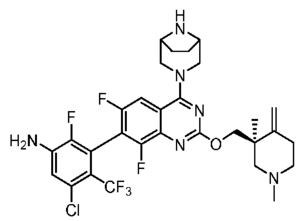 | 3-(4-((1*R*,5*S*)-3,8-diazabicyclo[3.2.1]octan-3-yl)-2-(((*S*)-1,3-dimethyl-4-methylenepiperidin-3-yl)methoxy)-6,8-difluoroquinazolin-7-yl)-5-chloro-2-fluoro-4-(trifluoromethyl)aniline | LCMS (m/z): 641.2 (M + H). |
| 90 | 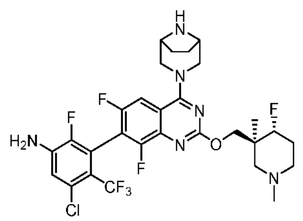 | 3-(4-((1*R*,5*S*)-3,8-diazabicyclo[3.2.1]octan-3-yl)-6,8-difluoro-2-(((3*S*,4*R*)-4-fluoro-1,3-dimethylpiperidin-3-yl)methoxy)quinazolin-7-yl)-5-chloro-2-fluoro-4-(trifluoromethyl)aniline | LCMS (m/z): 647.2 (M + H). |
| 91 | 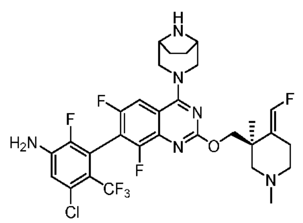 | 3-(4-((1*R*,5*S*)-3,8-diazabicyclo[3.2.1]octan-3-yl)-6,8-difluoro-2-(((*S,E*)-4-(fluoromethylene)-1,3-dimethylpiperidin-3-yl)methoxy)quinazolin-7-yl)-5-chloro-2-fluoro-4-(trifluoromethyl)aniline | LCMS (m/z): 659.2 (M + H). |
| 92 | 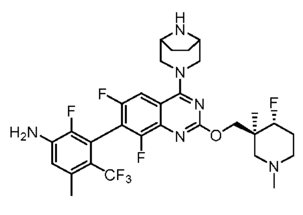 | 3-(4-((1*R*,5*S*)-3,8-diazabicyclo[3.2.1]octan-3-yl)-6,8-difluoro-2-(((3*S*,4*R*)-4-fluoro-1,3-dimethylpiperidin-3-yl)methoxy)quinazolin-7-yl)-2-fluoro-5-methyl-4-(trifluoromethyl)aniline | LCMS (m/z): 627.3 (M + H). |
| 93 | 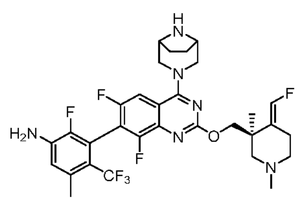 | 3-(4-((1*R*,5*S*)-3,8-diazabicyclo[3.2.1]octan-3-yl)-6,8-difluoro-2-(((*S,E*)-4-(fluoromethylene)-1,3-dimethylpiperidin-3-yl)methoxy)quinazolin-7-yl)-2-fluoro-5-methyl-4-(trifluoromethyl)aniline | LCMS (m/z): 639.3 (M + H). |
| 94 | 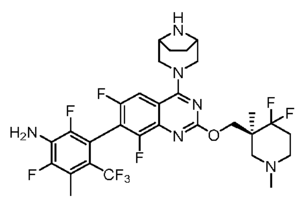 | 3-(4-((1*R*,5*S*)-3,8-diazabicyclo[3.2.1]octan-3 -yl)-2-(((*S*)-4,4-difluoro-1,3-dimethylpiperidin-3-yl)methoxy)-6,8-difluoroquinazolin-7-yl)-2,6-difluoro-5-methyl-4-(trifluoromethyl)aniline | LCMS (m/z): 663.0 (M + H). $^1$H NMR (400 MHz, Methanol-$d_4$) δ 7.64 - 7.50 (m, 1H), 4.64 - 4.55 (m, 1H), 4.52 - 4.36 (m, 3H), 3.69 - 3.54 (m, 4H), 2.81 - 2.30 (m, 7H), 2.27 (s, 3H), 2.19 - 2.04 (m, 2H), 1.93 - 1.76 (m, 4H), 1.26 (s, 3H). |

(continued)

| | | | |
|---|---|---|---|
| 95 | 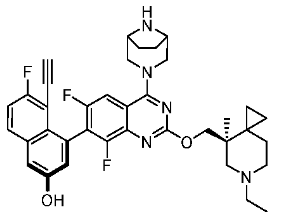 | 4-(4-((1*R*,5*S*)-3,8-diazabicyclo [3.2.1]octan-3-yl)-2-(((*S*)-6-ethyl-4-methyl-6-azaspiro[2.5] octan-4-yl)methoxy)-6,8-difluoroquinazolin-7-yl)-5-ethynyl-6-fluoronaphthalen-2-ol | LCMS (m/z): 642.3 (M + H). $^1$H NMR (400 MHz, DMSO-$d_6$) δ 10.24 (s, 1H), 8.05 - 7.93 (m, 1H), 7.59 - 7.44 (m, 2H), 7.41 (d, *J* = 2.4 Hz, 1H), 7.14 (d, J = 2.3 Hz, 1H), 4.53 - 4.40 (m, 1H), 4.36 - 4.27 (m, 1H), 4.27 - 4.16 (m, 2H), 3.97 (s, 1H), 3.55 - 3.42 (m, 4H), 2.83 - 2.59 (m, 2H), 2.28 (q, *J* = 7.1 Hz, 2H), 2.16 - 1.79 (m, 3H), 1.78 - 1.72 (m, 1H), 1.71 - 1.54 (m, 3H), 0.94 (t, *J* = 7.1 Hz, 3H), 0.88 - 0.74 (m, 1H), 0.70 (s, 3H), 0.64 - 0.58 (m, 1H), 0.58 - 0.48 (m, 1H), 0.18 - 0.07 (m, 1H), 0.05- -0.04 (m, 1H). $^{19}$F NMR (376 MHz, DMSO-$d_6$) δ-110.17,-119.34,-124.72. |
| 96 | 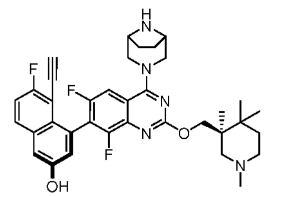 | 4-(4-((1*R*,5*S*)-3,8-diazabicyclo [3.2.1]octan-3-yl)-6,8-difluoro-2-(((*S*)-1,3,4,4-tetramethylpiperidin-3-yl)methoxy) quinazolin-7-yl)-5-ethynyl-6-fluoronaphthalen-2-ol | LCMS (m/z): 644.3 (M + H). |
| 97 | 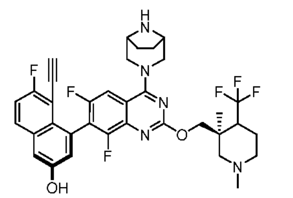 | 4-(4-((1*R*,5*S*)-3,8-diazabicyclo [3.2.1]octan-3-yl)-2-(((3*S*)-1,3-dimethyl-4-(trifluoromethyl) piperidin-3-yl)methoxy)-6,8-difluoroquinazolin-7-yl)-5-ethynyl-6-fluoronaphthalen-2-ol | LCMS (m/z): 670.3 (M + H). |
| 98 | 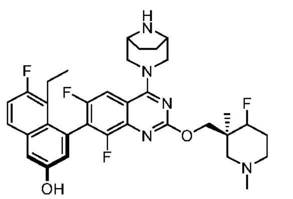 | 4-(4-((1*R*,5*S*)-3,8-diazabicyclo [3.2.1]octan-3-yl)-6,8-difluoro-2-(((3*S*)-4-fluoro-1,3-dimethylpiperidin-3-yl)methoxy) quinazolin-7-yl)-5-ethyl-6-fluoronaphthalen-2-ol | LCMS (m/z): 624.3 (M + H). |
| 99 | 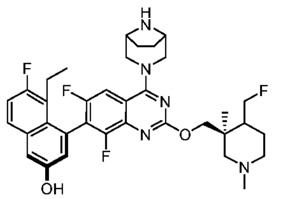 | 4-(4-((1*R*,5*S*)-3,8-diazabicyclo [3.2.1]octan-3-yl)-6,8-difluoro-2-(((3*S*)-4-(fluoromethyl)-1,3-dimethylpiperidin-3-yl)methoxy)quinazolin-7-yl)-5-ethyl-6-fluoronaphthalen-2-ol | LCMS (m/z): 638.3 (M + H). |
| 99-1 | 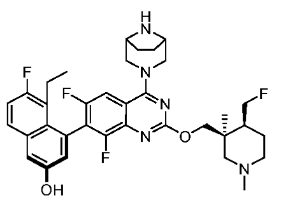 | 4-(4-(4-((1*R,*5S)-3,8-diazabicyclo[3.2.1]octan-3-yl)-6,8-difluoro-2-(((3*S*,4*S*)-4-(fluoromethyl)-1,3-dimethylpiperidin-3-yl) methoxy)quinazolin-7-yl)-5-ethyl-6-fluoronaphthalen-2-ol | LCMS (m/z): 638.3 (M + H). |
| 100 | 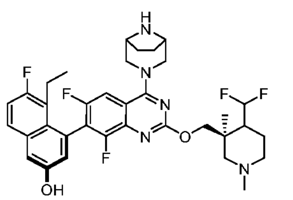 | 4-(4-((1*R*,5*S*)-3,8-diazabicyclo [3.2.1]octan-3-yl)-2-(((3*S*)-4-(difluoromethyl)-1,3-dimethylpiperidin-3-yl) methoxy)-6,8-difluoroquinazolin-7-yl)-5-ethyl-6-fluoronaphthalen-2-ol | LCMS (m/z): 656.3 (M + H). |

(continued)

| | | | |
|---|---|---|---|
| 101 | 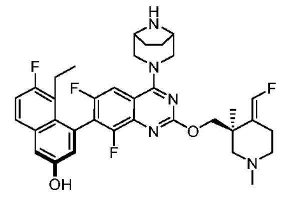 | 4-(4-((1*R*,5*S*)-3,8-diazabicyclo[3.2.1]octan-3-yl)-6,8-difluoro-2-(((*S,E*)-4-(fluoromethylene)-1,3-dimethylpiperidin-3-yl)methoxy)quinazolin-7-yl)-5-ethyl-6-fluoronaphthalen-2-ol | LCMS (m/z): 636.3 (M + H). |
| 102 | 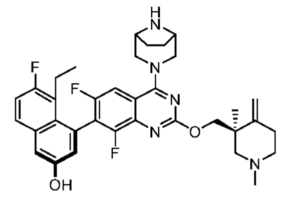 | 4-(4-((1*R*,5*S*)-3,8-diazabicyclo[3.2.1]octan-3-yl)-2-(((*S*)-1,3-dimethyl-4-methylenepiperidin-3-yl)methoxy)-6,8-difluoroquinazolin-7-yl)-5-ethyl-6-fluoronaphthalen-2-ol | LCMS (m/z): 618.3 (M + H). |

**Example 103**

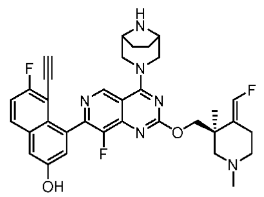

4-(4-((1*R*,5*S*)-3,8-diazabicyclo[3.2.1]octan-3-yl)-8-fluoro-2-(((*S*,*E*)-4-(fluoromethylene)-1,3-dimethylpiperidin-3-yl)methoxy)pyrido[4,3-*d*]pyrimidin-7-yl)-5-ethynyl-6-fluoronaphthalen-2-ol

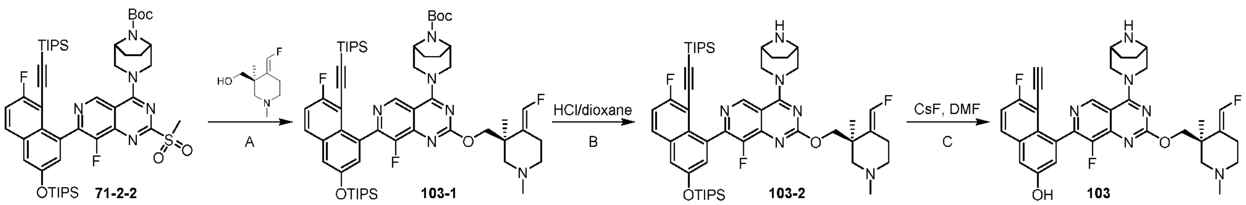


71-2-2 103-1 103-2 103

**Step A:** *tert*-butyl (1*R*,5*S*)-3-(8-fluoro-7-(7-fluoro-8-((triisopropylsilyl)ethynyl)-3-((triisopropylsilyl)oxy)naphthalen-1-yl)-2-(((*S,E*)-4-(fluoromethylene)-1,3-dimethylpiperidin-3-yl)methoxy)pyrido[4,3-*d*]pyrimidin-4-yl)-3,8-diazabicyclo[3.2.1]octane-8-carboxylate

Under an ice bath, sodium hydride (5 mg, 0.13 mmol) was added to a solution of (*S,E*)-(4-(fluoromethylene)-1,3-dimethylpiperidin-3-yl) methanol (17 mg, 0.09 mol) in anhydrous tetrahydrofuran (2 mL). After stirring the reaction mixture at this temperature for 0.5 h, *tert-butyl* (1*R*,5*S*)-3-(8-fluoro-7-(7-fluoro-8-((triisopropylsilyl)ethynyl)-3-((triisopropylsilyl)oxy)naphthalen-1-yl)-2-(methylsulfonyl)pyrido[4,3-*d*]pyrimidin-4-yl)-3,8-diazabicyclo[3.2.1]octan-8-carboxylate (60mg, 0.06 mmol) was added to the reaction. The resulting mixture was stirred at room temperature for 2 h. After the reaction was completed (monitored by LCMS), the reaction mixture was poured into 30 mL of ice water to quench the reaction. The mixture was then extracted with ethyl acetate. The combined organic phases were washed with saturated brine, dried over anhydrous sodium sulfate, filtered, and concentrated to dryness to obtain a crude product *tert*-butyl (1*R*,5*S*)-3-(8-fluoro-7-(7-fluoro-8-((triisopropylsilyl)ethynyl)-3-((triisopropylsilyl)oxy)naphthalen-1-yl)-2-(((*S,E*)-4-(fluoromethylene)-1,3-dimethylpiperidin-3-yl)methoxy)pyrido[4,3-*d*]pyrimidin-4-yl)-3,8-diazabicyclo[3.2.1]octane-8-carboxylate (50 mg, yield 48%). LCMS (m/z): 1027.5 (M + H).

**Step B:** 4-((1*R*,5*S*)-3,8-diazabicyclo[3.2.1]octan-3-yl)-8-fluoro-7-(7-fluoro-8-((triisopropylsilyl)ethynyl)-3-((triisopropylsilyl)oxy)naphthalen-1-yl)-2-(((*S*,*E*)-4-(fluoromethylene)-1,3-dimethylpiperidin-3-yl)methoxy)pyrido[4,3-d]pyrimidine

At room temperature, 0.5 mL of HCl (4 N in 1,4-dioxane solution) was added to a solution of *tert*-butyl (1*R*,5*S*)-3-(8-fluoro-7-(7-fluoro-8-((triisopropylsilyl)ethynyl)-3-((triisopropylsilyl)oxy)naphthalen-1-yl)-2-(((S,E)-4-(fluoromethylene)-1,3-dimethylpiperidin-3-yl)methoxy)pyrido[4,3-*d*]pyrimidin-4-yl)-3,8-diazabicyclo[3.2.1]octane-8-carboxylate (50 mg, 0.05 mmol) in dichloromethane (0.5 mL). The reaction was stirred at room temperature for 1 h. After the reaction was completed (monitored by LCMS), the reaction was concentrated to dryness to give a crude product 4-((1*R,*5*S*)-3,8-diazabicyclo[3.2.1]octan-3-yl)-8-fluoro-7-(7-fluoro-8-((triisopropylsilyl)ethynyl)-3-((triisopropylsilyl)oxy)naphthalen-1-yl)-2-(((*S,E*)-4-(fluoromethylene)-1,3-dimethylpiperidin-3-yl)methoxy)pyrido[4,3-d]pyrimidine (30 mg). LCMS (m/z): 927.4 (M + H).

**Step C:** 4-(4-((1*R*,5*S*)-3,8-diazabicyclo[3.2.1]octan-3-yl)-8-fluoro-2-(((*S*,*E*)-4-(fluoromethylene)-1,3-dimethylpiperidin-3-yl)methoxy)pyrido[4,3-*d*]pyrimidin-7-yl)-5-ethynyl-6-fluoronaphthalen-2-ol

At room temperature, cesium fluoride (49 mg, 0.33 mmol) was added to a solution of 4-((1*R*,5*S*)-3,8-diazabicyclo[3.2.1]octan-3-yl)-8-fluoro-7-(7-fluoro-8-((triisopropylsilyl)ethynyl)-3-((triisopropylsilyl)oxy)naphthalen-1-yl)-2-(((*S,E*)-4-(fluoromethylene)-1,3-dimethylpiperidin-3-yl)methoxy)pyrido[4,3-d]pyrimidine (30 mg, 0.03 mmol) in DMF (2 mL). The reaction was stirred at 50 °C for 2 hours. After the reaction was completed (monitored by LCMS), the reaction was purified by pre-HPLC (C18, CAN/(10 mmol $NH_4HCO_3/H_2O$) = 30-70%) to obtain 4-(4-((1*R*,5*S*)-3,8-diazabicyclo[3.2.1]octan-3-yl)-8-fluoro-2-(((*S,E*)-4-(fluoromethylene)-1,3-dimethylpiperidin-3-yl)methoxy)pyrido[4,3-*d*]pyrimidin-7-yl)-5-ethynyl-6-fluoronaphthalen-2-ol (2 mg, yield 10%) as a white solid. $^1$H NMR (400 MHz, Methanol-$d_4$) δ 8.99 (s, 1H), 7.85 (dd, *J* = 9.2, 5.7 Hz, 1H), 7.38 - 7.27 (m, 2H), 7.24 - 7.15 (m, 1H), 6.64 (d, *J* = 86.3 Hz, 1H), 4.68 - 4.55 (m, 3H), 4.44 - 4.32 (m, 1H), 3.75 - 3.63 (m, 3H), 3.33 - 3.31 (m, 1H), 2.93 - 2.83 (m, 1H), 2.83 - 2.74 (m, 1H), 2.71 - 2.62 (m, 1H), 2.38 - 2.27 (m, 1H), 2.25 (s, 3H), 2.03 - 1.77 (m, 6H), 1.20 (s, 3H). $^{19}$F NMR (376 MHz, Methanol-$d_4$) δ-111.74, -139.28 ~-139.52; LCMS (m/z): 616.2 (M + H).

**Referring to the above synthesis method or appropriate variants, this invention also synthesized and characterized the following compounds:**

| Example | Structure | Name | CHARACTERIZATION DATA |
|---|---|---|---|
| 104 | 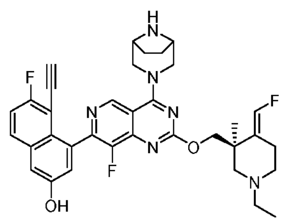 | 4-(4-((1*R*,5*S*)-3,8-diazabicyclo[3.2.1]octan-3-yl)-2-(((*S*,*E*)-1-ethyl-4-(fluoromethylene)-3-methylpiperidin-3-yl)methoxy)-8-fluoropyrido[4,3-*d*]pyrimidin-7-yl)-5-ethynyl-6-fluoronaphthalen-2-ol | LCMS (m/z): 629.3 (M + H). |
| 105 | 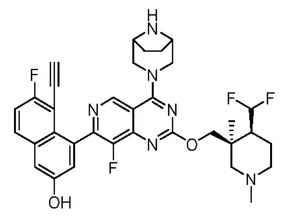 | 4-(4-((1*R*,5*S*)-3,8-diazabicyclo[3.2.1]octan-3-yl)-2-(((3*S*,4*S*)-4-(difluoromethyl)-1,3-dimethylpiperidin-3-yl)methoxy)-8-fluoropyrido[4,3-*d*]pyrimidin-7-yl)-5-ethynyl-6-fluoronaphthalen-2-ol | LCMS (m/z): 635.3 (M + H). $^1$H NMR (400 MHz, DMSO-$d_6$) δ 10.19 (s, 1H), 9.07 (s, 1H), 7.98 (dd, *J* = 9.2, 5.9 Hz, 1H), 7.51 - 7.43 (m, 1H), 7.40 (d, *J* = 2.6 Hz, 1H), 7.19 (d, *J* = 2.6 Hz, 1H), 6.48 - 6.14 (m, 1H), 4.59 - 4.51 (m, 1H), 4.50 - 4.34 (m, 3H), 3.97 - 3.93 (m, 1H), 3.91 - 3.79 (m, 2H), 3.78 - 3.65 (m, 2H), 2.90 - 2.76 (m, 2H), 2.12 (s, 3H), 1.90 - 1.58 (m, 9H), 1.12 (s, 3H). $^{19}$F NMR (376 MHz, DMSO-$d_6$) δ-110.77,-115.81,-118.91,-139.76. |

(continued)

| Example | Structure | Name | CHARACTERIZATION DATA |
|---|---|---|---|
| 106 | 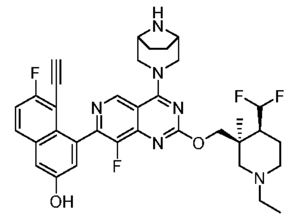 | 4-(4-((1*R*,5*S*)-3,8-diazabicyclo [3.2.1]octan-3-yl)-2-(((3*S*,4*S*)-4-(difluoromethyl)-1-ethyl-3-methylpiperidin-3-yl)methoxy)-8-fluoropyrido[4,3-*d*] pyrimidin-7-yl)-5-ethynyl-6-fluoronaphthalen-2-ol | LCMS (m/z): 649.3 (M + H). |
| 107 | 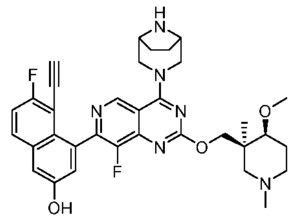 | 4-(4-((1*R*,5*S*)-3,8-diazabicyclo [3.2.1]octan-3-yl)-8-fluoro-2-(((3*S*,4*S*)-4-methoxy-1,3-dimethylpiperidin-3-yl)methoxy)pyrido[4,3-*d*]pyrimidin-7-yl)-5-ethynyl-6-fluoronaphthalen-2-ol | LCMS (m/z): 615.3 (M + H). |
| 108 | 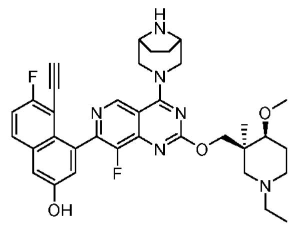 | 4-(4-((1*R*,5*S*)-3,8-diazabicyclo [3.2.1]octan-3-yl)-2-(((3*S*,4*S*)-1-ethyl-4-methoxy-3-methylpiperidin-3-yl)methoxy)-8-fluoropyrido [4,3-*d*]pyrimidin-7-yl)-5-ethynyl-6-fluoronaphthalen-2-ol | LCMS (m/z): 629.3 (M + H). |
| 109 | 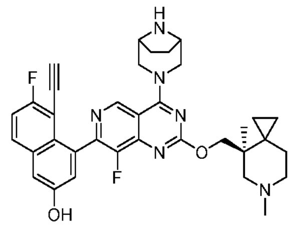 | 4-(4-(4-((1*R*,5*S*)-3,8-diazabicyclo [3.2.1]octan-3-yl)-2-(((*S*)-4,6-dimethyl-6-azaspiro[2.5]octan-4-yl) methoxy)-8-fluoropyrido[4,3-*d*]pyrimidin-7-yl)-5-ethynyl-6-fluoronaphthalen-2-ol | LCMS (m/z): 611.3 (M + H). |
| 110 | 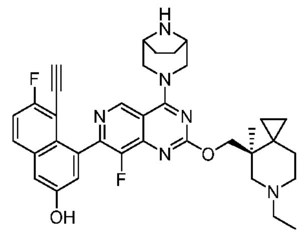 | 4-(4-((1*R*,5*S*)-3,8-diazabicyclo [3.2.1]octan-3-yl)-2-(((*S*)-6-ethyl-4-methyl-6-azaspiro[2.5]octan-4-yl) methoxy)-8-fluoropyrido[4,3-*d*]pyrimidin-7-yl)-5-ethynyl-6-fluoronaphthalen-2-ol | LCMS (m/z): 625.3 (M + H). |
| 111 | 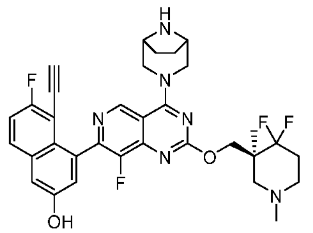 | 4-(4-(4-((1*R*,5*S*)-3,8-diazabicyclo [3.2.1]octan-3-yl)-2-(((*S*)-4,4-difluoro-1,3-dimethylpiperidin-3-yl) methoxy)-8-fluoropyrido[4,3-*d*]pyrimidin-7-yl)-5-ethynyl-6-fluoronaphthalen-2-ol | LCMS (m/z): 621.3 (M + H). |
| 112 | 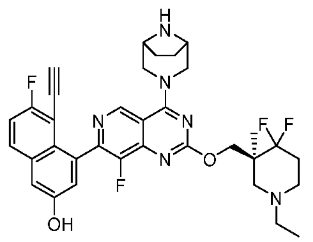 | 4-(4-(4-((1*R*,5*S*)-3,8-diazabicyclo [3.2.1]octan-3-yl)-2-(((*S*)-1-ethyl-4,4-difluoro-3-methylpiperidin-3-yl)methoxy)-8-fluoropyrido [4,3-*d*]pyrimidin-7-yl)-5-ethynyl-6-fluoronaphthalen-2-ol | LCMS (m/z): 635.3 (M + H). |

(continued)

| Example | Structure | Name | CHARACTERIZATION DATA |
|---|---|---|---|
| 113 | 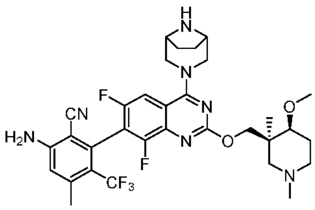 | 2-(4-(1*R*,5*S*)-3,8-diazabicyclo [3.2.1]octan-3-yl)-6,8-difluoro-2-(3*S*,4*S*)-4-methoxy-1,3-dimethylpiperidin-3-yl)methoxy)quinazolin-7-yl)-6-amino-4-methyl-3-(trifluoromethyl) benzonitrile | LCMS (m/z): 646.3 (M + H). |
| 114 | 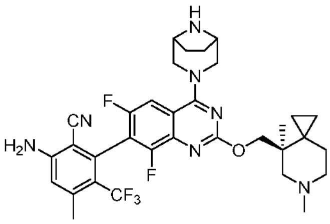 | 2-(4-((1*R*,5*S*)-3,8-diazabicyclo [3.2.1]octan-3-yl)-2-(((*S*)-4,6-dimethyl-6-azaspiro[2.5]octan-4-yl) methoxy)-6,8-difluoroquinazolin-7-yl)-6-amino-4-methyl-3-(trifluoromethyl) benzonitrile | LCMS (m/z): 642.3 (M + H). |
| 115 | 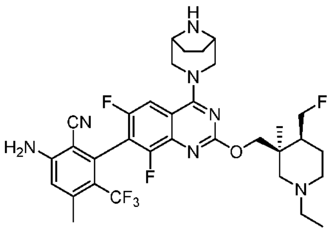 | 2-(4-((1*R*,5*S*)-3,8-diazabicyclo [3.2.1]octan-3-yl)-2-(((3*S*,4*S*)-1-ethyl-4-(fluoromethyl)-3-methylpiperidin-3-yl)methoxy)-6,8-difluoroquinazolin-7-yl)-6-amino-4-methyl-3-(trifluoromethyl) benzonitrile | LCMS (m/z): 662.3 (M + H). |
| 116 | 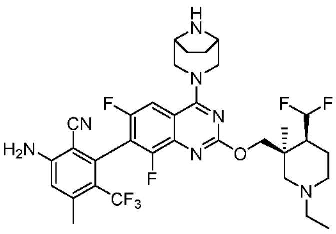 | 2-(4-((1*R*,5*S*)-3,8-diazabicyclo [3.2.1]octan-3-yl)-2-(((3*S*,4*S*)-4-(difluoromethyl)-1-ethyl-3-methylpiperidin-3-yl)methoxy)-6,8-difluoroquinazolin-7-yl)-6-amino-4-methyl-3-(trifluoromethyl) benzonitrile | LCMS (m/z): 680.3 (M + H). |
| 117 | 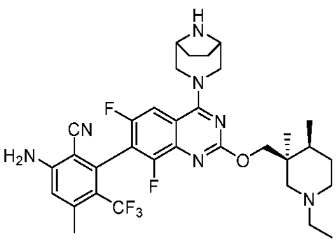 | 2-(4-((1*R*,5*S*)-3,8-diazabicyclo [3.2.1]octan-3-yl)-2-(((3*S*,4*S*)-1-ethyl-3,4-dimethylpiperidin-3-yl) methoxy)-6,8-difluoroquinazolin-7-yl)-6-amino-4-methyl-3-(trifluoromethyl) benzonitrile | LCMS (m/z): 644.3 (M + H). |
| 118 | 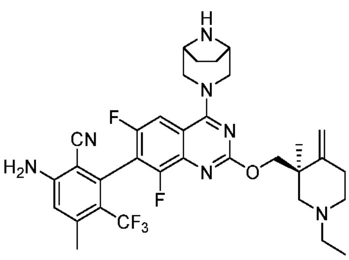 | 2-(4-((1*R*,5*S*)-3,8-diazabicyclo [3.2.1]octan-3-yl)-2-(((*S*)-1-ethyl-3-methyl-4-methylenepiperidin-3-yl) methoxy)-6,8-difluoroquinazolin-7-yl)-6-amino-4-methyl-3-(trifluoromethyl) benzonitrile | LCMS (m/z): 642.3 (M + H). |

(continued)

| Example | Structure | Name | CHARACTERIZATION DATA |
|---|---|---|---|
| 119 | 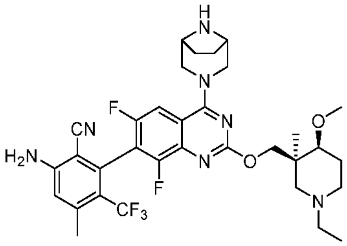 | 2-(4-((1*R*,5*S*)-3,8-diazabicyclo [3.2.1]octan-3-yl)-2-(((3*S*,4*S*)-1-ethyl-4-methoxy-3-methylpiperidin-3-yl)methoxy)-6,8-difluoroquinazolin-7-yl)-6-amino-4-methyl-3-(trifluoromethyl) benzonitrile | LCMS (m/z): 660.3 (M + H). |
| 120 | 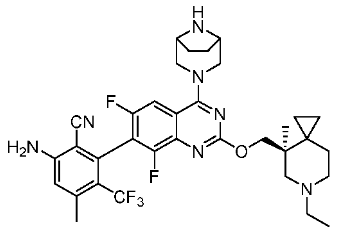 | 2-(4-((1*R*,5*S*)-3,8-diazabicyclo [3.2.1]octan-3-yl)-2-(((*S*)-6-ethyl-4-methyl-6-azaspiro[2.5]octan-4-yl) methoxy)-6,8-difluoroquinazolin-7-yl)-6-amino-4-methyl-3-(trifluoromethyl) benzonitrile | LCMS (m/z): 656.3 (M + H). |
| 121 | 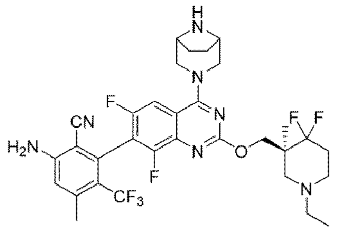 | 2-(4-((1*R*,5*S*)-3,8-diazabicyclo [3.2.1]octan-3-yl)-2-(((*S*)-1-ethyl-4,4-difluoro-3-methylpiperidin-3-yl)methoxy)-6,8-difluoroquinazolin-7-yl)-6-amino-4-methyl-3-(trifluoromethyl) benzonitrile | LCMS (m/z): 666.3 (M + H). |

**Example 122-1 and Example 122-2**

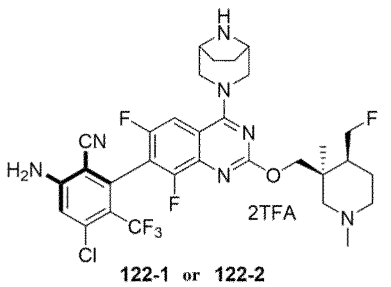

2TFA

**122-1 or 122-2**

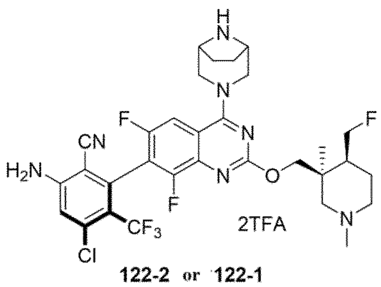

2TFA

**122-2 or 122-1**

(*Ra* or *Sa*)-2-(4-((1R,5*S*)-3,8-diazabicyclo[3.2.1]octan-3-y1)-6,8-difluoro-2-(((3*S*,4*S*)-4-(fluoromethyl)-1,3-dimethylpiperidin-3-yl)methoxy)quinazolin-7-yl)-6-amino-4-chloro-3-(trifluoromethyl) benzonitrile·trifluoroacetate

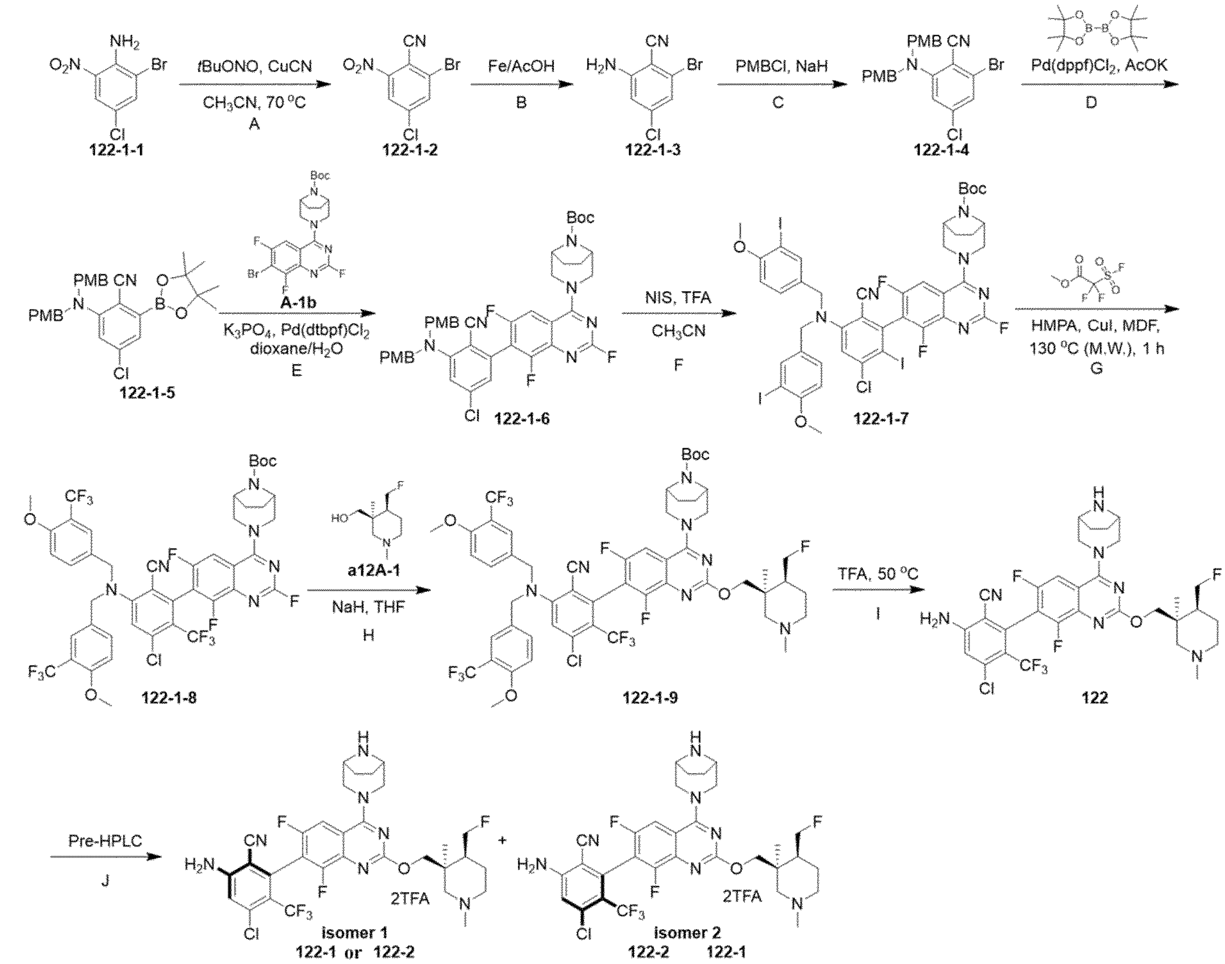


**Step A:** 2-bromo-4-chloro-6-nitrobenzonitrile

A mixture of tert-butyl nitrite (4.92 g, 47.72 mmol) and cuprous cyanide (4.27 g, 47.72 mmol) in acetonitrile (50 mL) was stirred at 70 °C for 15 min. Then, a solution of 2-bromo-4-chloro-6-nitroaniline in acetonitrile (20 mL) was added dropwise to the above mixture, and the resulting mixture was stirred at 70 °C for 16 h. After the reaction was completed (monitored by TLC), the reaction was cooled to room temperature and filtered with celite. The filtrate was concentrated and the crude product obtained was purified by FCC ($SiO_2$, EA/PE = 0-50%) to give 2-bromo-4-chloro-6-nitrobenzonitrile (3.1 g, yield 50%) as a yellow solid.

**Step B:** 2-amino-6-bromo-4-chlorobenzonitrile

At room temperature, iron powder (3.2 g, 57.37 mmol) was slowly added to a solution of 2-bromo-4-chloro-6-nitrobenzonitrile (3.0 g, 11.47 mmol) in acetic acid (50 mL). The resulting mixture was stirred 1 h at room temperature. Acetic acid was removed by distillation under reduced pressure. EA (100 mL) was added to the residue and sonicated. The resulting mixture was filtered with celite. The filtrate was successively washed with saturated sodium bicarbonate solution (50 mL) and brine (30 mL), then concentrated. The crude product was purified by FCC ($SiO_2$, EA/PE = 0-50%) to obtain 2-amino-6-bromo-4-chlorobenzonitrile (2.37 g, yield 89%) as a yellow solid. LCMS (m/z): 231.9/233.9 (M + H).

**Step C:** 2-(bis(4-methoxybenzyl)amino)-6-bromo-4-chlorobenzonitrile

NaH (1.02 g, 25.6 mmol) was added to a solution of 2-amino-6-bromo-4-chlorobenzonitrile (2.37 g, 10.24 mmol) in DMF (25 mL) at room temperature, and the reaction was stirred for 15 min. PMBCl (3.53 g, 22.52 mmol) was added dropwise, and the resulting mixture was stirred at room temperature for 16 h. Saturated ammonium chloride solution (25 mL) was added to quench the reaction. The reaction system was extracted with EA (30 mL × 3). The combined organic phases were washed with saturated brine (30 mL) and concentrated. The obtained crude product was purified by FCC ($SiO_2$, EA/PE = 0-40%) to obtain 2-(bis(4-methoxybenzyl)amino)-6-bromo-4-chlorobenzonitrile (4.7 g, yield 97%) as a

pale yellow solid. LCMS (m/z): 493.0/495.0 (M + Na).

**Step D:** 2-(bis(4-methoxybenzyl)amino)-4-chloro-6-(4,4,5,5-tetramethyl-1,3,2-dioxaborolan-2-yl) benzonitrile

Under nitrogen atmosphere, a solution of 2-(bis(4-methoxybenzyl)amino)-6-bromo-4-chlorobenzonitrile (2.0 g, 4.24 mmol), potassium acetate (1.25 g, 12.72 mmol), bis(pinacolato)diboron (5.38 g, 21.2 mmol), and Pd(dppf)$Cl_2$ (307 mg, 0.42 mmol) in dioxane (30 mL) was heated to 100 °C and stirred for 1 h. After the reaction was completed (monitored by TLC), the reaction was cooled to room temperature, and solvent was removed by distillation under vacuum to obtain the crude product which was purified by FCC ($SiO_2$, EA/PE = 0-30%) to afford 2-(bis(4-methoxybenzyl)amino)-4-chloro-6-(4,4,5,5-tetramethyl-1,3,2-dioxaborolan-2-yl) benzonitrile (2.0 g, yield 91%) as a brown solid.

**Step E:** *tert*-butyl (1*R*,5*S*)-3-(7-(3-(bis(4-methoxybenzyl)amino)-5-chloro-2-cyanophenyl)-2,6,8-trifluoroquinazolin-4-yl)-3,8-diazabicyclo[3.2.1]octane-8-carboxylate

Under nitrogen atmosphere, a solution of *tert*-butyl (1*R*,5*S*)-3-(7-bromo-2,6,8-trifluoroquinazolin-4-yl)-3,8-diazabicyclo[3.2.1]octane-8-carboxylate (2.0 g, 4.23 mmol), 2-(bis(4-methoxybenzyl)amino)-4-chloro-6-(4,4,5,5-tetramethyl-1,3,2-dioxaborolan-2-yl) benzonitrile (3 g , 5.92 mmol), potassium phosphate (2.21 g, 10.56 mmol) and Pd(dtbpf)$Cl_2$ (276 mg, 0.42mol) in dioxane (25 mL)/water (6 mL) was heated to 100 °C and stirred for 2 h. After the reaction was completed (monitored by LCMS), the reaction was cooled to room temperature, and the solvent was removed by distillation under reduced pressure. The resulting crude product was purified by FCC ($SiO_2$, EA/PE = 0-30%) to afford *tert*-butyl (1*R*,5*S*)-3-(7-(3-(bis(4-methoxybenzyl)amino)-5-chloro-2-cyanophenyl)-2,6,8-trifluoroquinazolin-4-yl)-3,8-diazabicyclo[3.2.1]octane-8-carboxylate (1.3g, yield 39.2%) as a pale yellow solid. LCMS (m/z): 785.3 (M + H).

**Step F:** *tert*-butyl (1*R*,5*S*)-3-(7-(3-(bis(3-iodo-4-methoxybenzyl)amino)-5-chloro-2-cyano-6-iodophenyl)-2,6,8-trifluoroquinazolin-4-yl)-3,8-diazabicyclo[3.2.1]octane-8-carboxylate

Silver sulfate (2.38 g, 7.64 mmol) was added to a solution of *tert*-butyl (1*R*,5*S*)-3-(7-(3-(bis(4-methoxybenzyl) amino)-5-chloro-2-cyanophenyl)-2,6,8-trifluoroquinazolin-4-yl)-3,8-diazabicyclo[3.2.1]octane-8-carboxylate (1.2g, 1.53 mmol) and iodine (1.94g, 7.64 mmol) in DMF (15 mL). The resulting mixture was stirred at room temperature in the dark for 16 h. The reaction was filtered with celite, and ethyl acetate (30 mL) was added to the filtrate. The resulting solution was successively washed with saturated sodium bisulfite saturated solution, brine, and concentrated under reduced pressure to obtain the crude product which was purified by FCC ($SiO_2$, EA/PE = 0 - 40%) to obtain *tert*-butyl (1*R*,5*S*)-3-(7-(3-(bis(3-iodo-4-methoxybenzyl)amino)-5-chloro-2-cyano-6-iodophenyl)-2,6,8-trifluoroquinazolin-4-yl)-3,8-diazabicyclo[3.2.1] octane-8-carboxylate (0.6 g, yield 4%) as a yellow solid. LCMS (m/z): 1163.0 (M + H).

**Step G:** (1*R*,5*S*)-3-(7-(3-(bis(4-methoxy-3-(trifluoromethyl)benzyl)amino)-5-chloro-2-cyano-6-(trifluoromethyl)phenyl)-2,6,8-trifluoroquinazolin-4-yl)-3,8-diazabicyclo[3.2.1]octane-8-carboxylate

At room temperature, *tert*-butyl (1*R*,5*S*)-3-(7-(3-(bis(3-iodo-4-methoxybenzyl)amino)-5-chloro-2-cyano-6-iodophenyl)-2,6,8-trifluoroquinazolin-4-yl)-3,8-diazabicyclo[3.2.1]octane-8-carboxylate (600 mg, 0.52 mmol), methyl fluorosulfonyl difluoroacetate (500 mg, 2.58 mmol), CuI (200 mg, 1.03 mmol), and HMPA (0.2 mL) were dissolved in DMF (3 mL), the resulting mixture was heated by microwave to 130 °C for 1 h. After the reaction was completed (monitored by LCMS), the reaction was filtered with celite. The filtrate was diluted with EA (20 mL), washed with brine (15 mL × 2), concentrated under reduced pressure. The crude product was purified by FCC ($SiO_2$, EA/PE = 0-30%) to obtain (1*R*,5*S*)-3-(7-(3-(bis(4-methoxy-3-(trifluoromethyl)benzyl)amino)-5-chloro-2-cyano-6-(trifluoromethyl)phenyl)-2,6,8-trifluoroquinazolin-4-yl)-3,8-diazabicyclo[3.2.1]octane-8-carboxylate (400 mg, yield 78%) as a yellow solid. LCMS (m/z): 989.2 (M + H).

**Step H:** (1*R*,5*S*)-3-(7-(3-(bis(4-methoxy-3-(trifluoromethyl)benzyl)amino)-5-chloro-2-cyano-6-(trifluoromethyl)phenyl)-6,8-difluoro-2-(((3*S*,4*S*)-4-(fluoromethyl)-1,3-dimethylpiperidin-3-yl)methoxy)quinazolin-4-yl)-3,8-diazabicyclo [3.2.1]octane-8-carboxylate

At room temperature, NaH (8 mg, 0.20 mmol) was added to a solution of ((3*S*,4*S*)-4-(fluoromethyl)-1,3-dimethylpiperidin-3-yl) methanol (35 mg, 0.20 mmol) in THF (3 mL), and the resulting mixture was stirred for 20 min at room temperature. Then, (1*R*,5*S*)-3-(7-(3-(bis(4-methoxy-3-(trifluoromethyl)benzyl)amino)-5-chloro-2-cyano-6-(trifluoromethyl)phenyl)-2,6,8-trifluoroquinazolin-4-yl)-3,8-diazabicyclo[3.2.1]octane-8-carboxylate (100 mg, 0.10 mmol) was added, and the reaction was stirred at room temperature for 2 h. Saturated aqueous $NH_4Cl$ solution (1 mL) was added to quench the reaction, and the resulting mixture was extracted with ethyl acetate (10 mL × 2). The combined

organic phase was washed with brine, dried over anhydrous $Na_2SO_4$, filtered, concentrated under reduced pressure to give (1*R*,5*S*)-3-(7-(3-(bis(4-methoxy-3-(trifluoromethyl)benzyl)amino)-5-chloro-2-cyano-6-(trifluoromethyl)phenyl)-6,8-difluoro-2-(((3*S*,*4*S)-4-(fluoromethyl)-1,3-dimethylpiperidin-3-yl)methoxy)quinazolin-4-yl)-3,8-diazabicyclo[3.2.1]octane-8-carboxylate (50 mg, yield 43%), which was used directly in the next step. LCMS (m/z): 1144.3 (M + H).

**Steps I and J**: (*Ra* or *Sa*)-2-(4-(1*R*,5*S*)-3,8-diazabicyclo[3.2.1]octan-3-yl)-6,8-difluoro-2-(((3*S*,4S)-4-(fluoromethyl)-1,3-dimethylpiperidin-3-yl)methoxy)quinazolin-7-yl)-6-amino-4-chloro-3-(trifluoromethyl) benzonitrile·trifluoroacetate

At room temperature, (1*R*,5*S*)-3-(7-(3-(bis(4-methoxy-3-(trifluoromethyl)benzyl)amino)-5-chloro-2-cyano-6-(trifluoromethyl)phenyl)-6,8-difluoro-2-(((3*S*,4*S*)-4-(fluoromethyl)-1,3-dimethylpiperidin-3-yl)methoxy)quinazolin-4-yl)-3,8-diazabicyclo[3.2.1]octane-8-carboxylate (50 mg, 0.044 mmol) was dissolved in 2 mL TFA, and the mixture was stirred at 50 °C for 1 h. The solvent was removed by under reduced pressure to obtain crude product 2-(4-((1*R*,5*S*)-3,8-diazabicyclo[3.2.1]octan-3-yl)-6,8-difluoro-2-(((3*S*,4*S*)-4-(fluoromethyl)-1,3-dimethylpiperidin-3-yl) methoxy)quinazolin-7-yl)-6-amino-4-chloro-3-(trifluoromethyl)benzonitrile (compound 122) which was further purified by Pre-HPLC (C18, CAN/(0.1% FA/$H_2O$) = 25 - 40%) to obtain the first eluted isomer as 122-1 (with a shorter retention time, 5.5 mg), LCMS (m/z): 668.2 (M + H), $^1$H NMR (400 MHz, Methanol-$d_4$) δ 7.78 (dd, *J* = 9.9, 1.8 Hz, 1H), 7.23 (s, 1H), 4.80 - 4.62 (m, 5H), 4.35 (d, *J* = 11.4 Hz, 1H), 4.31 - 4.24 (m, 2H), 3.95 - 3.85 (m, 2H), 3.75 - 3.64 (m, 2H), 3.17 - 3.09 (m, 1H), 3.02 (d, *J* = 12.9 Hz, 1H), 2.94 (s, 3H), 2.27 - 2.13 (m, 5H), 2.05 - 1.96 (m, 2H), 1.27 (s, 3H). And the later eluted isomer as 122-2 (with a longer retention time, 6.1 mg), LCMS (m/z): 668.2 (M + H), $^1$H NMR (400 MHz, Methanol-$d_4$) δ 7.77 (dd, *J* = 9.9, 1.8 Hz, 1H), 7.23 (s, 1H), 4.81 - 4.61 (m, 5H), 4.34 (d, *J* = 11.4 Hz, 1H), 4.31 - 4.22 (m, 2H), 3.96 - 3.85 (m, 2H), 3.75 - 3.64 (m, 2H), 3.17 - 3.09 (m, 1H), 3.01 (d, *J* = 12.9 Hz, 1H), 2.94 (s, 3H), 2.29 - 2.21 (m, 1H), 2.18 (s, 4H), 2.08 - 1.96 (m, 2H), 1.27 (s, 3H).

Compounds 122-1 and 122-2 can also be obtained by the resolution of compound 122 with an appropriate chiral HPLC or SFC method.

**Referring to the above synthesis method or appropriate variants, this invention also synthesized and characterized the following compounds:**

| Example | Structure | Name | CHARACTERIZATION DATA |
|---|---|---|---|
| 123-1 | 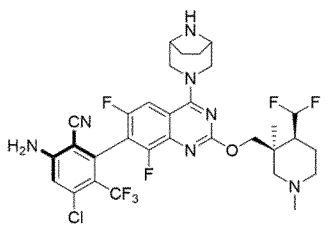 or 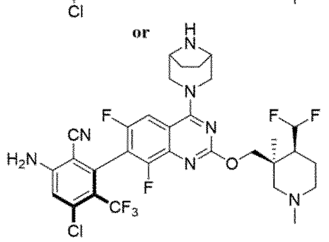 | (*Ra* or *Sa*)-2-(4-((1*R*,5*S*)-3,8-diazabicyclo[3.2.1]octan-3-yl)-2-(((3*S*,4*S*)-4-(difluoromethyl)-1,3-dimethylpiperidin-3-yl) methoxy)-6,8-difluoroquinazolin-7-yl)-6-amino-4-chloro-3-(trifluoromethyl) benzonitrile | LCMS (m/z): 686.3 (M + H). $^1$H NMR (400 MHz, Methanol-$d_4$) δ 7.78 (d, *J* = 9.8 Hz, 1H), 7.23 (s, 1H), 6.30 (t, *J* = 54.9 Hz, 1H), 4.79 (d, *J* = 11.5 Hz, 1H), 4.76 - 4.62 (m, 2H), 4.37 (d, *J* = 11.5 Hz, 1H), 4.31 - 4.23 (m, 2H), 3.94 - 3.83 (m, 2H), 3.78 - 3.67 (m, 2H), 3.17 - 3.09 (m, 1H), 3.04 (d, *J* = 13.2 Hz, 1H), 2.95 (s, 3H), 2.34 - 2.23 (m, 2H), 2.22 - 2.03 (m, 5H), 1.32 (s, 3H). |

(continued)

| Example | Structure | Name | CHARACTERIZATION DATA |
|---|---|---|---|
| 123-2 | 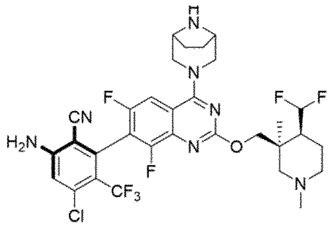 or 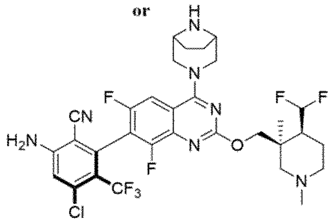 | (*Ra* or *Sa*)-2-(4-((1*R*,5*S*)-3,8-diazabicyclo[3.2.1]octan-3-yl)-2-(((3*S*,4*S*)-4-(difluoromethyl)-1,3-dimethylpiperidin-3-yl)methoxy)-6,8-difluoroquinazolin-7-yl)-6-amino-4-chloro-3-(trifluoromethyl) benzonitrile | LCMS (m/z): 686.3 (M + H). $^1$H NMR (400 MHz, Methanol-$d_4$) δ 7.78 (d, *J* = 10.0 Hz, 1H), 7.23 (s, 1H), 6.30 (t, *J* = 54.8 Hz, 1H), 4.81 (d, *J* = 11.6 Hz, 1H), 4.70 (t, *J* = 12.6 Hz, 2H), 4.35 (d, *J* = 11.5 Hz, 1H), 4.27 (s, 2H), 3.94 - 3.83 (m, 2H), 3.72 (d, *J* = 12.8 Hz, 2H), 3.14 - 2.99 (m, 2H), 2.95 (s, 3H), 2.28 (d, *J* = 13.8 Hz, 2H), 2.18 (s, 4H), 2.10 (d, *J* = 10.5 Hz, 1H), 1.32 (s, 3H). |
| 124-1 | 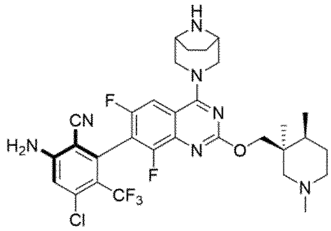 or 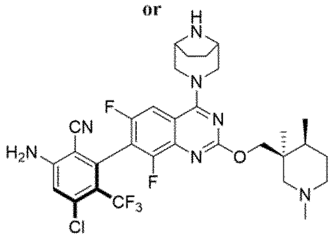 | (*Ra* or *Sa*)-2-(4-((1*R*,5S)-3,8-diazabicyclo[3.2.1]octan-3-yl)-6,8-difluoro-2-(((3*S*,4*S*)-1,3,4-trimethylpiperidin-3-yl)methoxy)quinazolin-7-yl)-6-amino-4-chloro-3-(trifluoromethyl) benzonitrile | LCMS (m/z): 650.2 (M + H). |
| 124-2 | 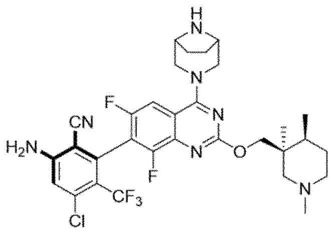 or 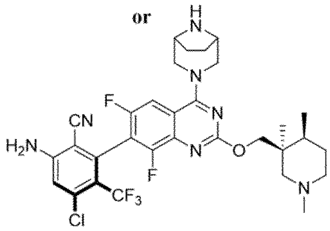 | (*Ra* or *Sa*)-2-(4-((1 *R*,5*S*)-3,8-diazabicyclo[3.2.1]octan-3-yl)-6,8-difluoro-2-(((3*S*,4*S*)-1,3,4-trimethylpiperidin-3-yl)methoxy)quinazolin-7-yl)-6-amino-4-chloro-3-(trifluoromethyl) benzonitrile | LCMS (m/z): 650.2 (M + H). |

(continued)

| Example | Structure | Name | CHARACTERIZATION DATA |
|---|---|---|---|
| 125-1 | 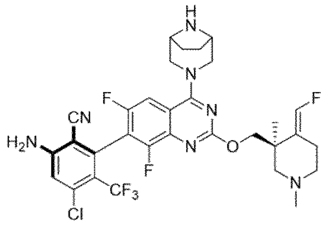 or 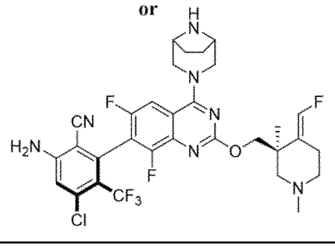 | (*Ra* or *Sa*)-2-(4-((1*R*,5*S*)-3,8-diazabicyclo[3.2.1]octan-3-yl)-6,8-difluoro-2-(((*S,E*)-4-(fluoromethylene)-1,3-dimethylpiperidin-3-yl) methoxy)quinazolin-7-yl)-6-amino-4-chloro-3-(trifluoromethyl) benzonitrile | LCMS (m/z): 666.2 (M + H). |
| 125-2 | 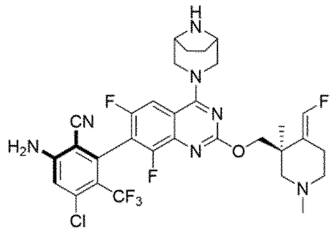 or 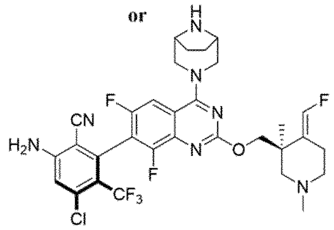 | (*Ra* or *Sa*)-2-(4-((1*R*,5*S*)-3,8-diazabicyclo[3.2.1]octan-3-yl)-6,8-difluoro-2-(((*S,E*)-4-(fluoromethylene)-1,3-dimethylpiperidin-3-yl) methoxy)quinazolin-7-yl)-6-amino-4-chloro-3-(trifluoromethyl) benzonitrile | LCMS (m/z): 666.2 (M + H). |
| 126-1 | 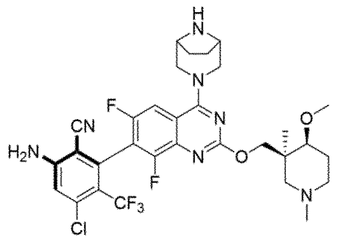 or 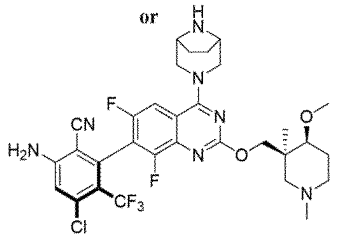 | (*Ra* or *Sa*)-2-(4-((1*R*,5*S*)-3,8-diazabicyclo[3.2.1]octan-3-yl)-6,8-difluoro-2-(((3*S*,4*S*)-4-methoxy-1,3-dimethylpiperidin-3-yl)methoxy) quinazolin-7-yl)-6-amino-4-chloro-3-(trifluoromethyl) benzonitrile | LCMS (m/z): 666.2 (M + H). |

(continued)

| Example | Structure | Name | CHARACTERIZATION DATA |
|---|---|---|---|
| 126-2 | 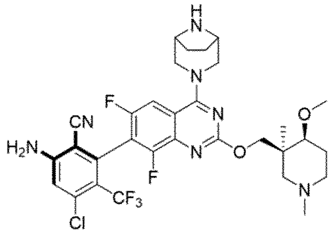 or 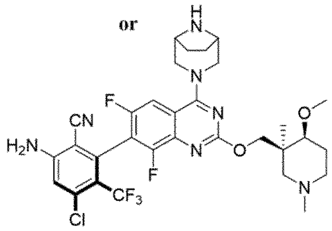 | (*Ra* or *Sa*)-2-(4-((1*R*,5*S*)-3,8-diazabicyclo[3.2.1]octan-3-yl)-6,8-difluoro-2-(((3*S*,4*S*)-4-methoxy-1,3-dimethylpiperidin-3-yl)methoxy) quinazolin-7-yl)-6-amino-4-chloro-3-(trifluoromethyl) benzonitrile | LCMS (m/z): 666.2 (M + H). |
| 127-1 | 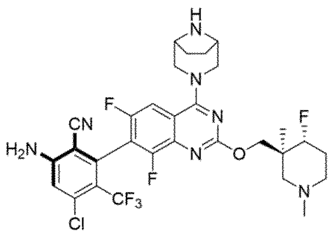 or 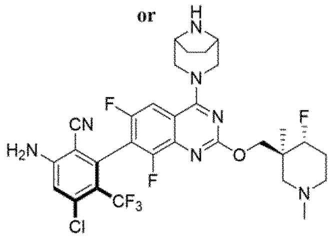 | (*Ra* or *Sa*)-2-(4-((1*R*,5*S*)-3,8-diazabicyclo[3.2.1]octan-3-yl)-6,8-difluoro-2-(((3*S*,4*R*)-4-fluoro-1,3-dimethylpiperidin-3-yl)methoxy)quinazolin-7-yl)-6-amino-4-chloro-3-(trifluoromethyl) benzonitrile | LCMS (m/z): 654.2 (M + H). |
| 127-2 | 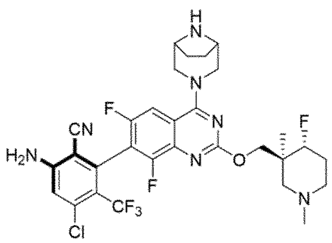 or 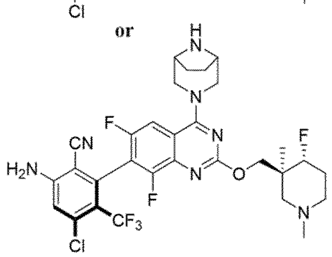 | (*Ra* or *Sa*)-2-(4-((1*R*,5*S*)-3,8-diazabicyclo[3.2.1]octan-3-yl)-6,8-difluoro-2-(((3*S*,4*R*)-4-fluoro-1,3-dimethylpiperidin-3-yl)methoxy)quinazolin-7-yl)-6-amino-4-chloro-3-(trifluoromethyl) benzonitrile | LCMS (m/z): 654.2 (M + H). |

(continued)

| Example | Structure | Name | CHARACTERIZATION DATA |
|---|---|---|---|
| 127-3 | 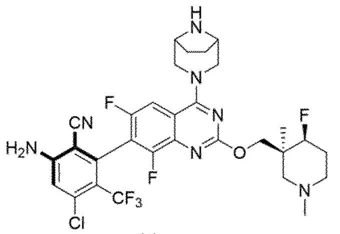 or 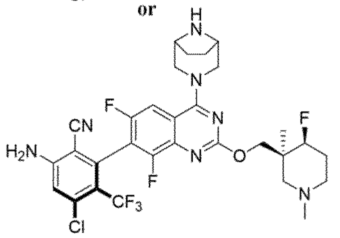 | (*Ra* or *Sa*)-2-(4-((1*R*,5*S*)-3,8-diazabicyclo[3.2.1]octan-3-yl)-6,8-difluoro-2-(((3*S*,4*S*)-4-fluoro-1,3-dimethylpiperidin-3-yl)methoxy)quinazolin-7-yl)-6-amino-4-chloro-3-(trifluoromethyl) benzonitrile | LCMS (m/z): 654.2 (M + H). |
| 127-4 | 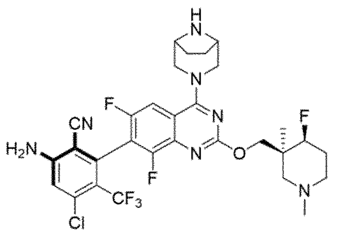 or 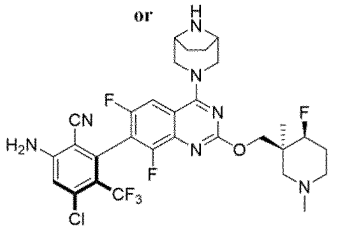 | (*Ra* or *Sa*)-2-(4-((1*R*,5*S*)-3,8-diazabicyclo[3.2.1]octan-3-yl)-6,8-difluoro-2-(((3S,4S)-4-fluoro-1,3-dimethylpiperidin-3-yl)methoxy)quinazolin-7-yl)-6-amino-4-chloro-3-(trifluoromethyl) benzonitrile | LCMS (m/z): 654.2 (M + H). |
| 128-1 | 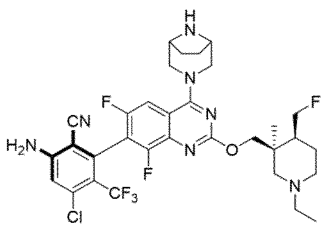 or 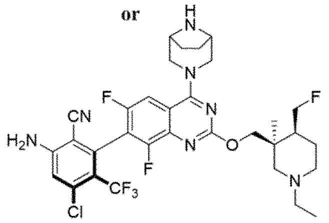 | (*Ra* or *Sa*)-2-(4-((1*R*,5*S*)-3,8-diazabicyclo[3.2.1]octan-3-yl)-2-(((3*S*,4*S*)-1-ethyl-4-(fluoromethyl)-3-methylpiperidin-3-yl)methoxy)-6,8-difluoroquinazolin-7-yl)-6-amino-4-chloro-3-(trifluoromethyl) benzonitrile | LCMS (m/z): 682.2 (M + H). |

(continued)

| Example | Structure | Name | CHARACTERIZATION DATA |
|---|---|---|---|
| 128-2 | 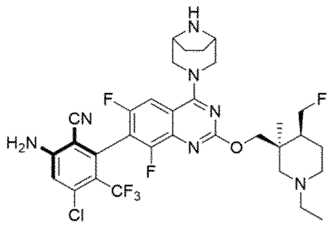 or 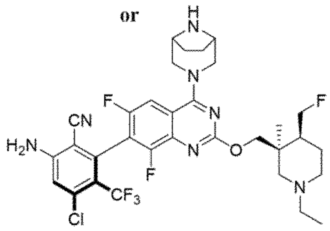 | (*Ra* or *Sa*)-2-(4-((1*R*,5*S*)-3,8-diazabicyclo[3.2.1]octan-3-yl)-2-(((3*S*,4*S*)-1-ethyl-4-(fluoromethyl)-3-methylpiperidin-3-yl) methoxy)-6,8-difluoroquinazolin-7-yl)-6-amino-4-chloro-3-(trifluoromethyl) benzonitrile | LCMS (m/z): 682.2 (M + H). |
| 129-1 | 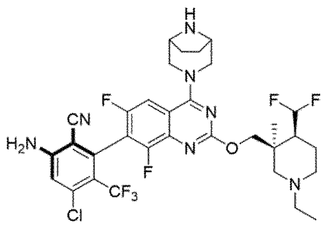 or 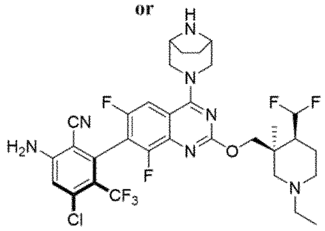 | (*Ra* or *Sa*)-2-(4-((1*R*,5*S*)-3,8-diazabicyclo[3.2.1]octan-3-yl)-2-(((3*S*,4*S*)-4-(difluoromethyl)-1-ethyl-3-methylpiperidin-3-yl)methoxy)-6,8-difluoroquinazolin-7-yl)-6-amino-4-chloro-3-(trifluoromethyl) benzonitrile | LCMS (m/z): 700.2 (M + H). |
| 129-2 | 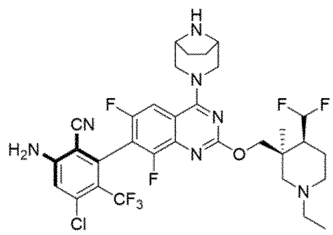 or 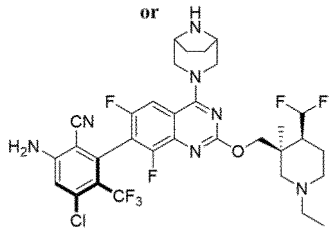 | (*Ra* or *Sa*)-2-(4-((1*R*,5*S*)-3,8-diazabicyclo[3.2.1]octan-3-yl)-2-(((3*S*,4*S*)-4-(difluoromethyl)-1-ethyl-3-methylpiperidin-3-yl)methoxy)-6,8-difluoroquinazolin-7-yl)-6-amino-4-chloro-3-(trifluoromethyl) benzonitrile | LCMS (m/z): 700.2 (M + H). |

(continued)

| Example | Structure | Name | CHARACTERIZATION DATA |
|---|---|---|---|
| 130-1 | 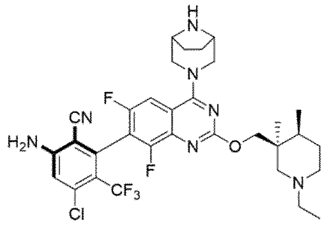 or 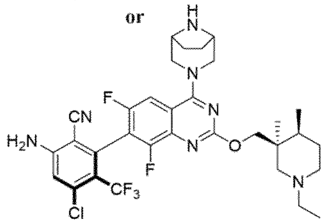 | (*Ra* or *Sa*)-2-(4-((1*R*,5*S*)-3,8-diazabicyclo[3.2.1]octan-3-yl)-2-(((3*S*,4*S*)-1-ethyl-3,4-dimethylpiperidin-3-yl)methoxy)-6,8-difluoroquinazolin-7-yl)-6-amino-4-chloro-3-(trifluoromethyl) benzonitrile | LCMS (m/z): 664.3 (M + H). |
| 130-2 | 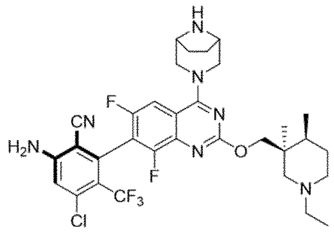 or 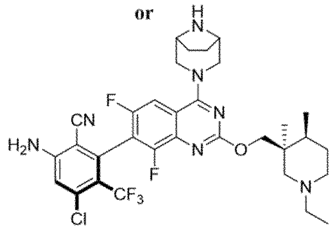 | (*Ra* or *Sa*)-2-(4-((1*R*,5*S*)-3,8-diazabicyclo[3.2.1]octan-3-yl)-2-(((3*S*,4*S*)-1-ethyl-3,4-dimethylpiperidin-3-yl)methoxy)-6,8-difluoroquinazolin-7-yl)-6-amino-4-chloro-3-(trifluoromethyl) benzonitrile | LCMS (m/z): 664.3 (M + H). |
| 131-1 | 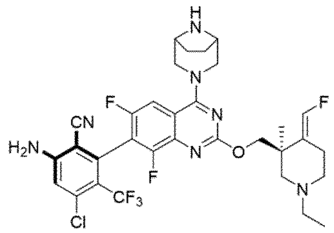 or 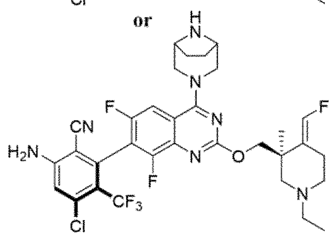 | (*Ra* or *Sa*)-2-(4-((1*R*,5*S*)-3,8-diazabicyclo[3.2.1]octan-3-yl)-2-(((*S*,*E*)-1-ethyl-4-(fluoromethylene)-3-methylpiperidin-3-yl)methoxy)-6,8-difluoroquinazolin-7-yl)-6-amino-4-chloro-3-(trifluoromethyl) benzonitrile | LCMS (m/z): 680.2 (M + H). |

(continued)

| Example | Structure | Name | CHARACTERIZATION DATA |
|---|---|---|---|
| 131-2 | 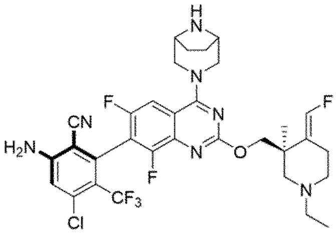 or 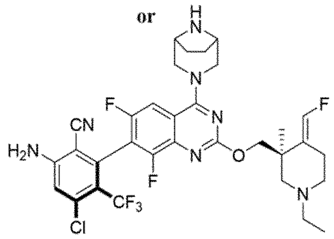 | (*Ra* or *Sa*)-2-(4-((1*R*,5*S*)-3,8-diazabicyclo[3.2.1]octan-3-yl)-2-(((*S*,*E*)-1-ethyl-4-(fluoromethylene)-3-methylpiperidin-3-yl)methoxy)-6,8-difluoroquinazolin-7-yl)-6-amino-4-chloro-3-(trifluoromethyl) benzonitrile | LCMS (m/z): 680.2 (M + H). |
| 132-1 | 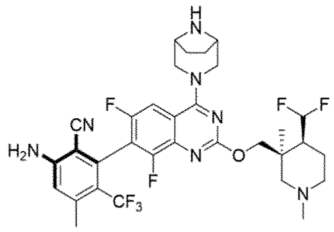 or 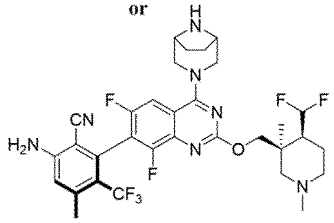 | (*Ra* or *Sa*)-2-(4-((1*R*,5*S*)-3,8-diazabicyclo[3.2.1]octan-3-yl)-2-(((3S,4S)-4-(difluoromethyl)-1,3-dimethylpiperidin-3-yl) methoxy)-6,8-difluoroquinazolin-7-yl)-6-amino-4-methyl-3-(trifluoromethyl) benzonitrile | LCMS (m/z): 666.3 (M + H). |
| 132-2 | 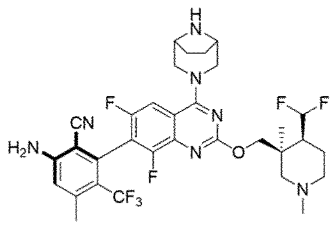 or 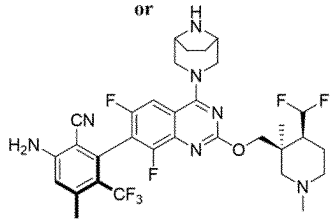 | (*Ra* or *Sa*)-2-(4-((1*R*,5*S*)-3,8-diazabicyclo[3.2.1]octan-3-yl)-2-(((3*S*,4*S*)-4-(difluoromethyl)-1,3-dimethylpiperidin-3-yl) methoxy)-6,8-Difluoroquinazolin-7-yl)-6-amino-4-methyl-3-(trifluoromethyl) benzonitrile | LCMS (m/z): 666.3 (M + H). |

(continued)

| Example | Structure | Name | CHARACTERIZATION DATA |
|---|---|---|---|
| 133-1 | 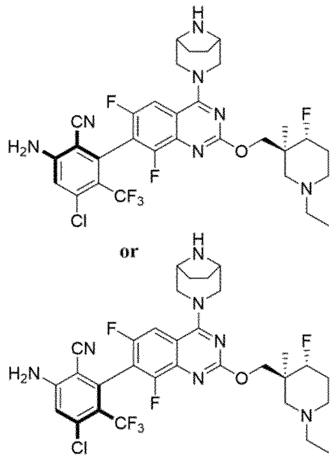 or | (*Ra* or *Sa*)-2-(4-((*R*,5*S*)-3,8-diazabicyclo[3.2.1]octan-3-yl)-2-(((3*S*,4*R*)-1-ethyl-4-fluoro-3-methylpiperidin-3-yl)methoxy)-6,8-difluoroquinazolin-7-yl)-6-amino-4-chloro-3-(trifluoromethyl) benzonitrile | LCMS (m/z): 668.2 (M + H). |
| 133-2 | 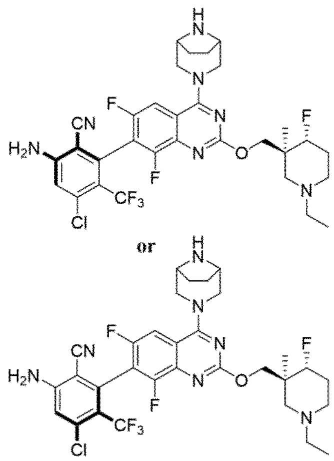 or | (*Ra* or *Sa*)-2-(4-((1*R*,5*S*)-3,8-diazabicyclo[3.2.1]octan-3-yl)-2-(((3*S*,4*R*)-1-ethyl-4-fluoro-3-methylpiperidin-3-yl)methoxy)-6,8-difluoroquinazolin-7-yl)-6-amino-4-chloro-3-(trifluoromethyl) benzonitrile | LCMS (m/z): 668.2 (M + H). |
| 133-3 | 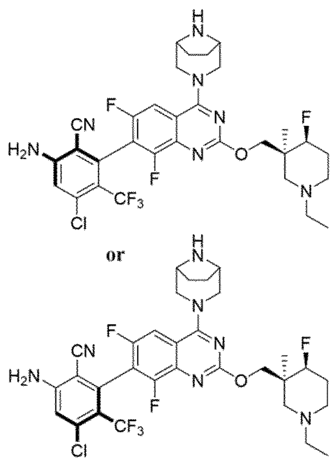 or | (*Ra* or *Sa*)-2-(4-((1*R*,5*S*)-3,8-diazabicyclo[3.2.1]octan-3-yl)-2-(((3*S*,4*S*)-1-ethyl-4-fluoro-3-methylpiperidin-3-yl)methoxy)-6,8-difluoroquinazolin-7-yl)-6-amino-4-chloro-3-(trifluoromethyl) benzonitrile | LCMS (m/z): 668.2 (M + H). |

(continued)

| Example | Structure | Name | CHARACTERIZATION DATA |
|---|---|---|---|
| 133-4 | 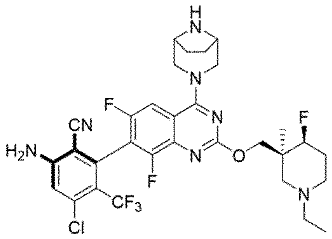 or 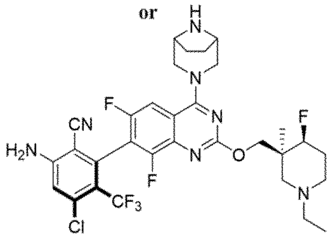 | (*Ra* or *Sa*)-2-(4-((1*R*,5*S*)-3,8-diazabicyclo[3.2.1]octan-3-yl)-2-(((3*S*,4*S*)-1-ethyl-4-fluoro-3-methylpiperidin-3-yl)methoxy)-6,8-difluoroquinazolin-7-yl)-6-amino-4-chloro-3-(trifluoromethyl) benzonitrile | LCMS (m/z): 668.2 (M + H). |
| 134-1 | 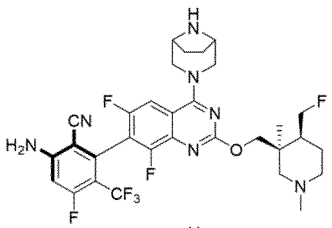 or 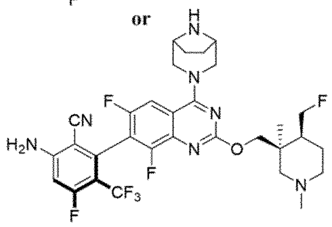 | (*Ra* or *Sa*)-2-(4-((1*R*,5*S*)-3,8-diazabicyclo[3.2.1]octan-3-yl)-6,8-difluoro-2-(((3S,4S)-4-(fluoromethyl)-1,3-dimethylpiperidin-3-yl) methoxy)quinazolin-7-yl)-6-amino-4-fluoro-3-(trifluoromethyl) benzonitrile | LCMS (m/z): 652.3 (M + H). |
| 134-2 | 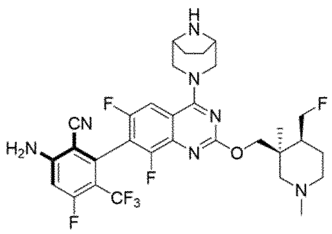 or 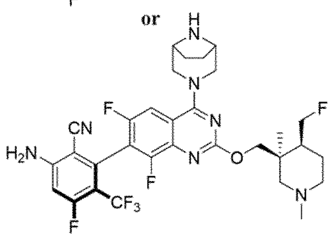 | (*Ra* or *Sa*)-2-(4-((1*R*,5*S*)-3,8-diazabicyclo[3.2.1]octan-3-yl)-6,8-difluoro-2-(((3*S*,4*S*)-4-(fluoromethyl)-1,3-dimethylpiperidin-3-yl) methoxy)quinazolin-7-yl)-6-amino-4-fluoro-3-(trifluoromethyl) benzonitrile | LCMS (m/z): 652.3 (M + H). |

(continued)

| Example | Structure | Name | CHARACTERIZATION DATA |
|---|---|---|---|
| 135-1 | 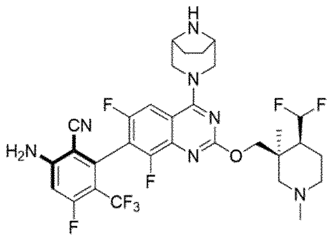 or 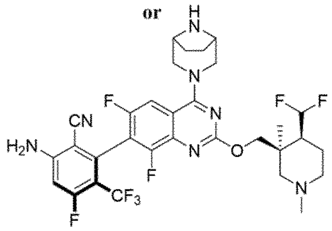 | (*Ra* or *Sa*)-2-(4-((1*R*,5*S*)-3,8-diazabicyclo[3.2.1]octan-3-yl)-2-(((3*S*,4*S*)-4-(difluoromethyl)-1,3-dimethylpiperidin-3-yl) methoxy)-6,8-difluoroquinazolin-7-yl)-6-amino-4-fluoro-3-(trifluoromethyl) benzonitrile | LCMS (m/z): 670.3 (M + H). |
| 135-2 | 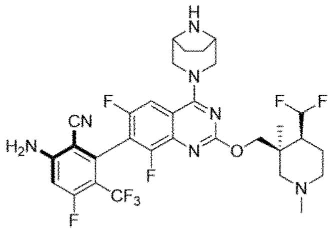 or 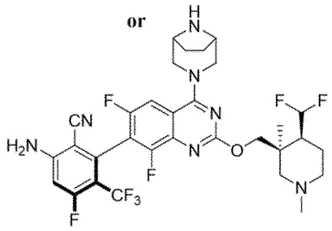 | (*Ra* or *Sa*)-2-(4-((1*R*,5*S*)-3,8-diazabicyclo[3.2.1]octan-3-yl)-2-(((3*S*,4*S*)-4-(difluoromethyl)-1,3-dimethylpiperidin-3-yl) methoxy)-6,8-difluoroquinazolin-7-yl)-6-amino-4-fluoro-3-(trifluoromethyl) benzonitrile | LCMS (m/z): 670.3 (M + H). |
| 136-1 | 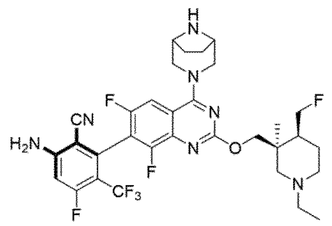 or 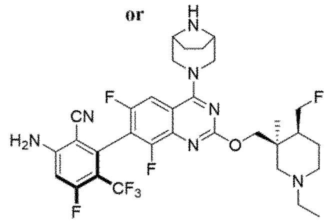 | (*Ra* or *Sa*)-2-(4-((1*R*,5*S*)-3,8-diazabicyclo[3.2.1]octan-3-yl)-2-(((3*S*,4*S*)-1-ethyl-4-(fluoromethyl)-3-methylpiperidin-3-yl) methoxy)-6,8-difluoroquinazolin-7-yl)-6-amino-4-fluoro-3-(trifluoromethyl) benzonitrile | LCMS (m/z): 666.3 (M + H). |

(continued)

| Example | Structure | Name | CHARACTERIZATION DATA |
|---|---|---|---|
| 136-2 | 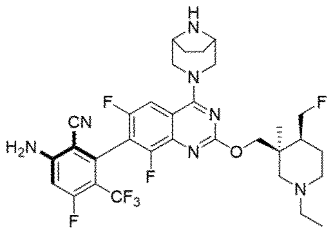 or 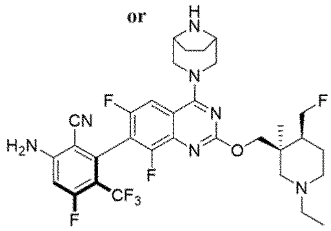 | (*Ra* or *Sa*)-2-(4-((1*R*,5*S*)-3,8-diazabicyclo[3.2.1]octan-3-yl)-2-(((3*S*,4*S*)-1-ethyl-4-(fluoromethyl)-3-methylpiperidin-3-yl) methoxy)-6,8-difluoroquinazolin-7-yl)-6-amino-4-fluoro-3-(trifluoromethyl) benzonitrile | LCMS (m/z): 666.3 (M + H). |
| 137-1 | 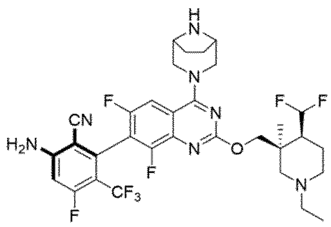 or 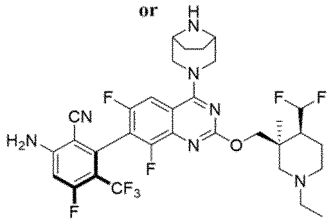 | (*Ra* or *Sa*)-2-(4-((1*R*,5*S*)-3,8-diazabicyclo[3.2.1]octan-3-yl)-2-(((3*S*,4*S*)-4-(difluoromethyl)-1-ethyl-3-methylpiperidin-3-yl)methoxy)-6,8-difluoroquinazolin-7-yl)-6-amino-4-fluoro-3-(trifluoromethyl) benzonitrile | LCMS (m/z): 684.3 (M + H). |
| 137-2 | 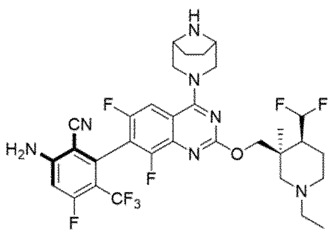 or 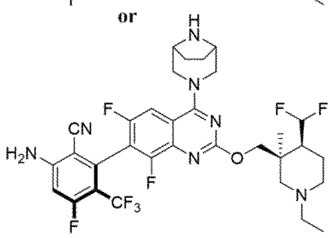 | (*Ra* or *Sa*)-2-(4-((1*R*,5*S*)-3,8-diazabicyclo[3.2.1]octan-3-yl)-2 (((3*S*,4*S*)-4-(difluoromethyl)-1-ethyl-3-methylpiperidin-3-yl)methoxy)-6,8-difluoroquinazolin-7-yl)-6-amino-4-fluoro-3-(trifluoromethyl) benzonitrile | LCMS (m/z): 684.3 (M + H). |

(continued)

| Example | Structure | Name | CHARACTERIZATION DATA |
|---|---|---|---|
| 138-1 | 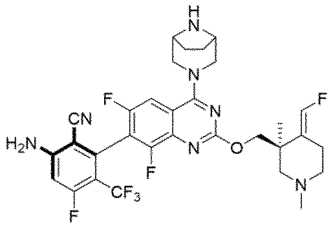 or 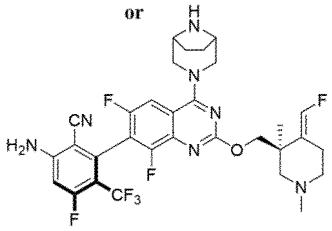 | (*Ra* or *Sa*)-2-(4-((1*R*,5*S*)-3,8-diazabicyclo[3.2.1]octan-3-yl)-6,8-difluoro-2-(((*S*,*E*)-4-(fluoromethylene)-1,3-dimethylpiperidin-3-yl) methoxy)quinazolin-7-yl)-6-amino-4-fluoro-3-(trifluoromethyl) benzonitrile | LCMS (m/z): 650.2 (M + H). |
| 138-2 | 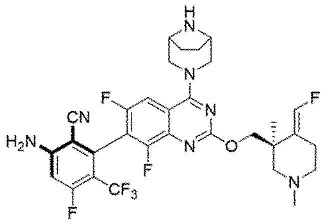 or 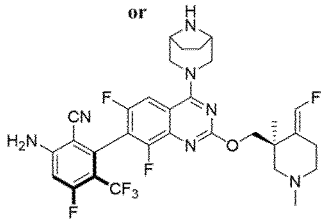 | (*Ra* or *Sa*)-2-(4-((1*R*,5*S*)-3,8-diazabicyclo[3.2.1]octan-3-yl)-6,8-difluoro-2-(((*S*,*E*)-4-(fluoromethylene)-1,3-dimethylpiperidin-3-yl) methoxy)quinazolin-7-yl)-6-amino-4-fluoro-3-(trifluoromethyl) benzonitrile | LCMS (m/z): 650.2 (M + H). |
| 139-1 | 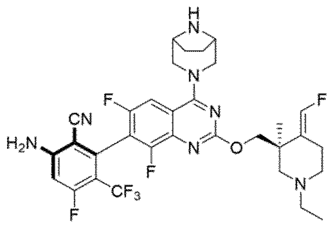 or 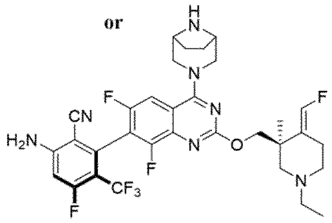 | (*Ra* or *Sa*)-2-(4-((1*R*,5*S*)-3,8-diazabicyclo[3.2.1]octan-3-yl)-2-(((*S,E*)-1-ethyl-4-(fluoromethylene)-3-methylpiperidin-3-yl) methoxy)-6,8-difluoroquinazolin-7-yl)-6-amino-4-fluoro-3-(trifluoromethyl) benzonitrile | LCMS (m/z): 664.3 (M + H). |

(continued)

| Example | Structure | Name | CHARACTERIZATION DATA |
|---|---|---|---|
| 139-2 | 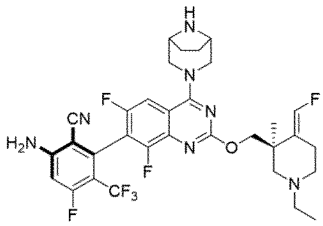 or 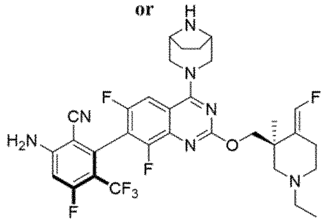 | (*Ra* or *Sa*)-2-(4-((1*R*,5*S*)-3,8-diazabicyclo[3.2.1]octan-3-yl)-2-(((*S,E*)-1-ethyl-4-(fluoromethylene)-3-methylpiperidin-3-yl)methoxy)-6,8-difluoroquinazolin-7-yl)-6-amino-4-fluoro-3-(trifluoromethyl) benzonitrile | LCMS (m/z): 664.3 (M + H). |

The compounds listed in the following table can be synthesized with referring to the above synthesis method and by using appropriate intermediates.

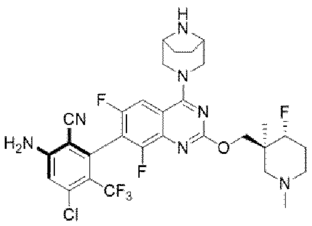
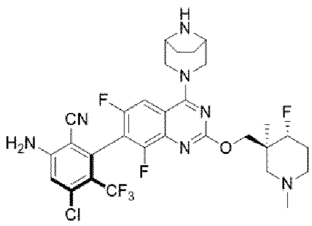
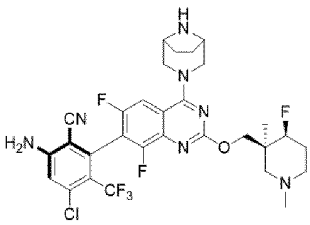
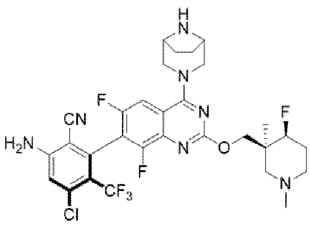

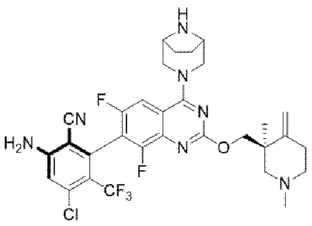
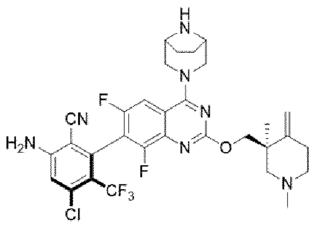
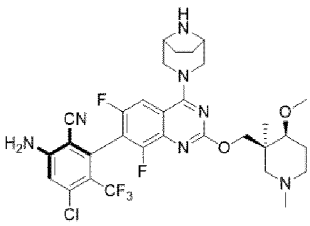
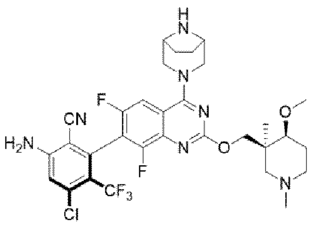

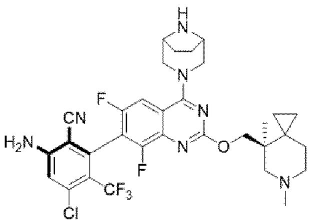
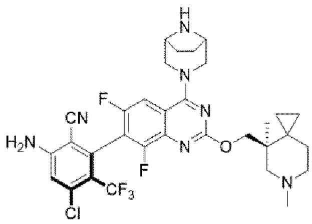
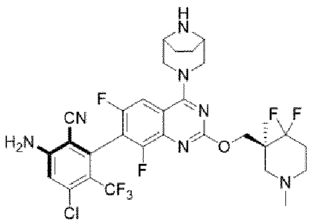
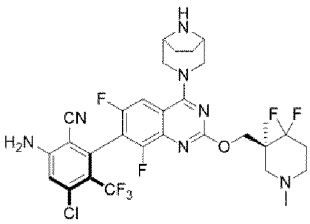

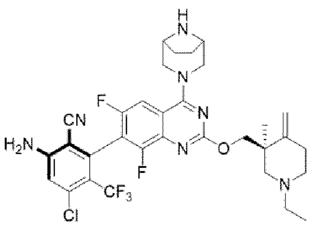
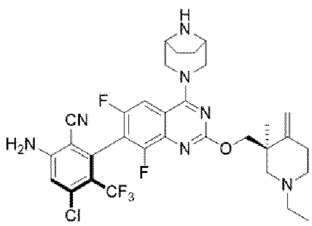
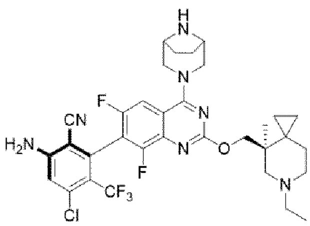
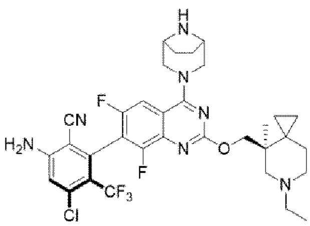

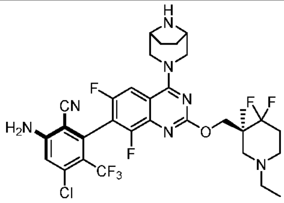
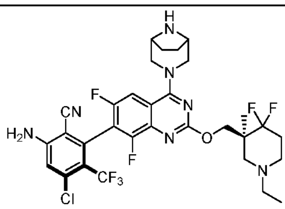
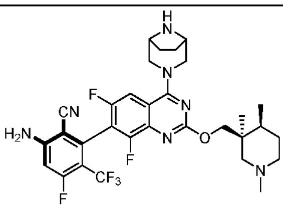
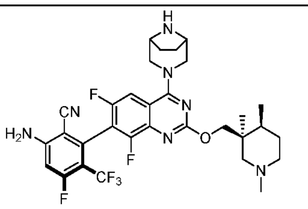
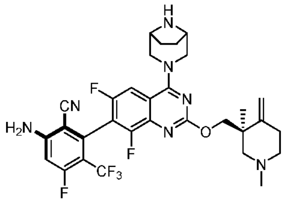
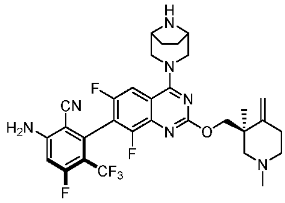
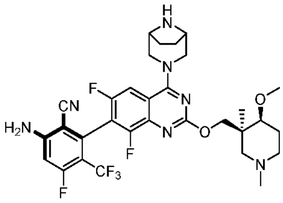
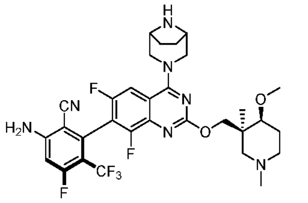
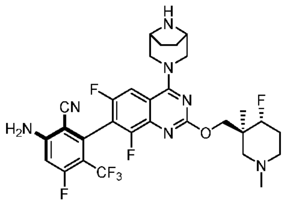
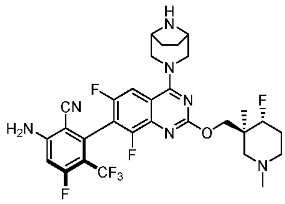
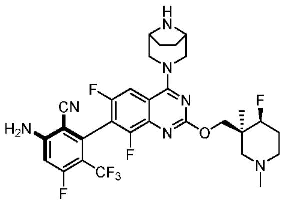
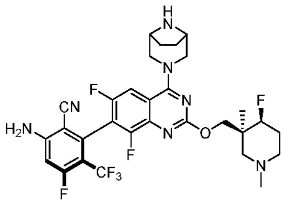
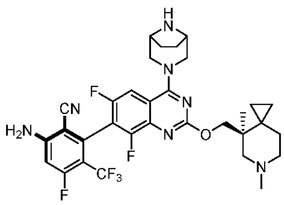
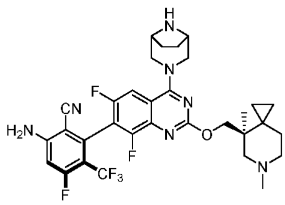
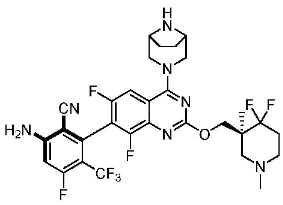
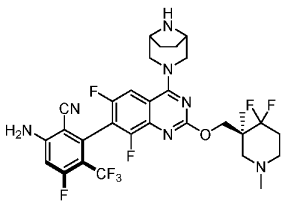
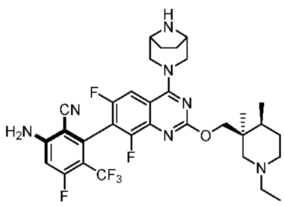
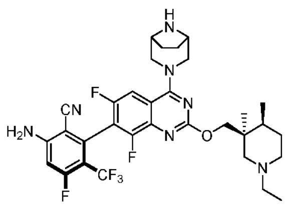
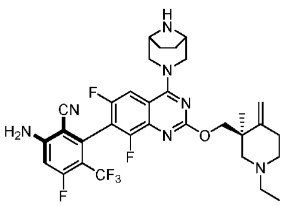
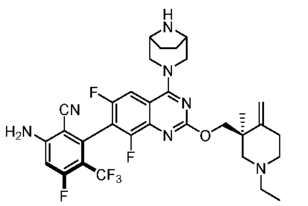
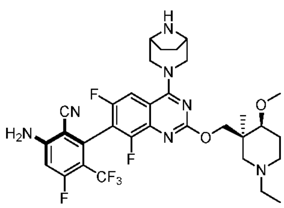
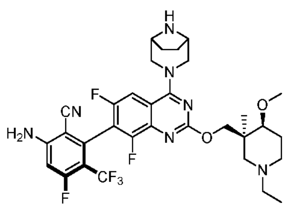
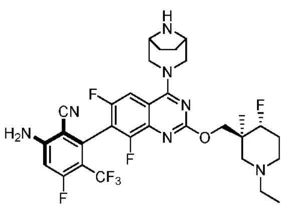
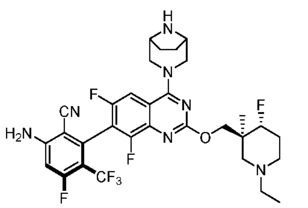
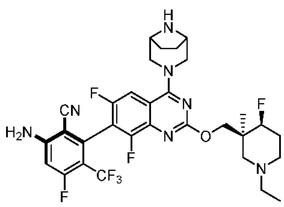
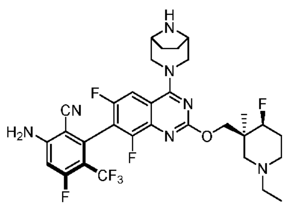
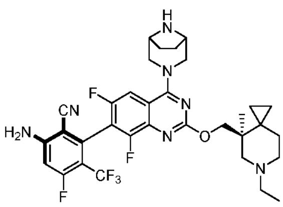
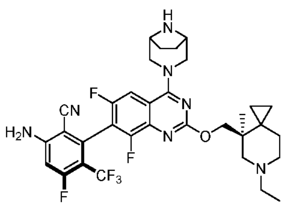

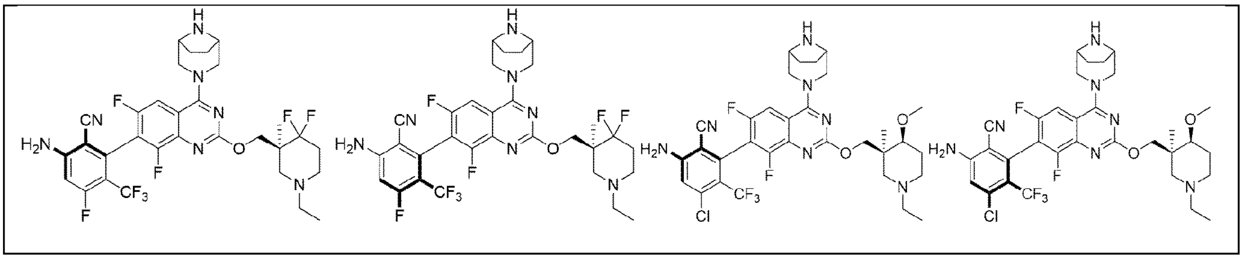

**Example 140-1 and Example 140-2**

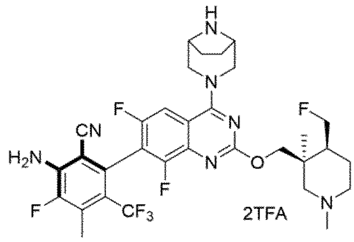

**140-1 or 140-2**

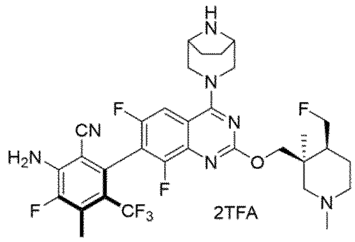

**140-2 or 140-1**

*(Ra* or *Sa*)-2-(4-((1*R*,5*S*)-3,8-diazabicyclo[3.2.1]octan-3-yl)-6,8-difluoro-2-(((3*S*,4*S*)-4-(fluoromethyl)-1,3-dimethylpiperidin-3-yl)methoxy)quinazolin-7-yl)-6-amino-5-fluoro-4-methyl-3-(trifluoromethyl) benzonitrile·trifluoroacetate

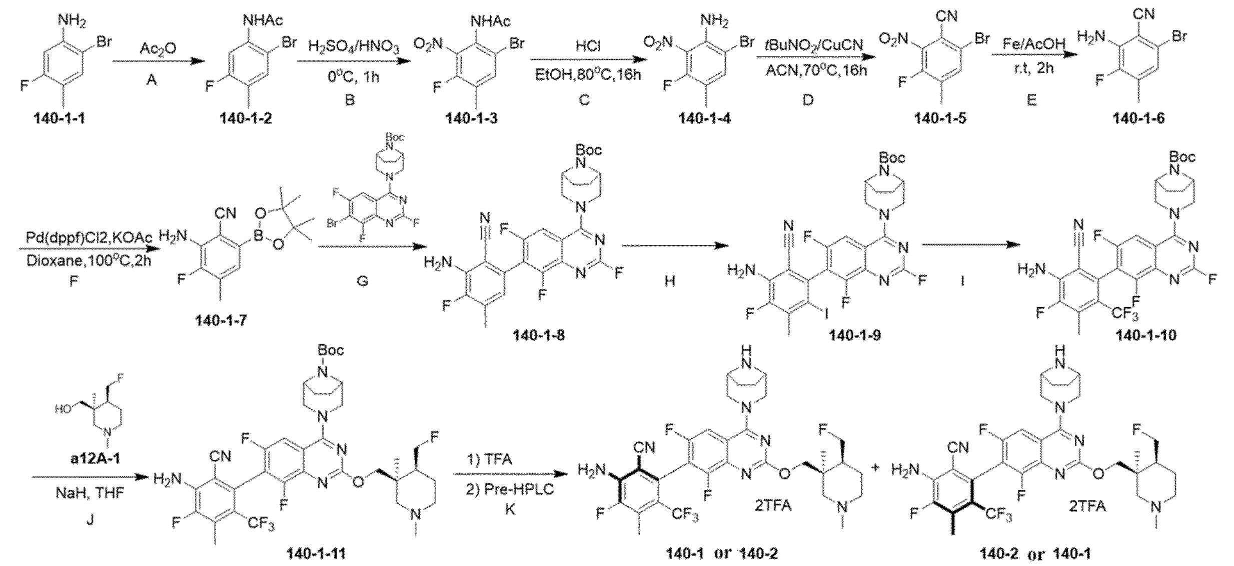


140-1-1, 140-1-2, 140-1-3, 140-1-4, 140-1-5, 140-1-6, 140-1-7, 140-1-8, 140-1-9, 140-1-10, 140-1-11, 140-1 or 140-2, 140-2 or 140-1

**Step A:** N-(2-bromo-5-fluoro-4-methylphenyl) acetamide

Acetic anhydride (5.0 g, 49.0 mmol) was added dropwise into a solution of 2-bromo-5-fluoro-4-methylaniline (10.0 g, 49.0 mmol) in dichloromethane (150 mL) at room temperature. The reaction was stirred at room temperature for 5 h. After the reaction was completed (monitored by LCMS), the reaction was concentrated directly to give N-(2-bromo-5-fluoro-4-methylphenyl) acetamide (12.7 g, crude) as white solid. LC-MS (m/z): 245.8 (M + H) and 247.8 (M + H).

**Step B:** *N*-(6-bromo-3-fluoro-4-methyl-2-nitrophenyl) acetamide

Concentrated sulfuric acid (39 mL, 98%) was added dropwise into a solution of N-(2-bromo-5-fluoro-4-methylphenyl) acetamide (12.7 g) in concentrated nitric acid (39 mL, 68%) in an ice-water bath. After the addition was completed, the mixture was stirred under an ice-water bath for 1 h. After the reaction was completed (monitored by LCMS), the reaction was quenched with saturated aqueous sodium bicarbonate (300 mL) and the resulting mixture was extracted with ethyl acetate (100 mL × 3). The combined organic phase was washed with brine (200 mL), dried over anhydrous $Na_2SO_4$, filtered and concentrated to obtain N-(6-bromo-3-fluoro-4-methyl-2-nitrophenyl) acetamide (12.0 g , crude) as a brown solid. LC-MS (m/z): 290.9 (M + H) and 292.9 (M + H).

**Step C:** 6-bromo-3-fluoro-4-methyl-2-nitroaniline

Concentrated hydrochloric acid (120 mL, 12 M) was added to a solution of N-(6-bromo-3-fluoro-4-methyl-2-nitrophenyl) acetamide (12.0 g) in ethanol (120 mL) at room temperature. The reaction was heated to 80 °C and stirred overnight after addition. After the reaction was completed (monitored by LCMS), the reaction was directly concentrated, quenched with saturated aqueous sodium bicarbonate (300 mL), and extracted with ethyl acetate (100 mL × 3). The combined organic phase was washed with brine (200 mL), dried over anhydrous $Na_2SO_4$, filtered, concentrated, and purified by FCC ($SiO_2$, EA/PE = 0-30%) to obtain 6-bromo-3-fluoro-4-methyl-2-nitroaniline (6.1 g, overall yield 50% for 3 steps) as a brown solid. LC-MS (m/z): 248.9 (M + H) and 250.9 (M + H).

**Step D:** 6-bromo-3-fluoro-4-methyl-2-nitrobenzonitrile

*tert*-Butyl nitrite (5.0 g, 49.0 mmol) was added to a mixture of CuCN (4.4 g, 49.0 mmol) in acetonitrile (120 mL) at room temperature, and the reaction was heated to 70 °C and stirred for 20 min. A solution of 6-bromo-3-fluoro-4-methyl-2-nitroaniline (6.1 g, 24.5 mmol) in acetonitrile (20 mL) was added dropwise to the reaction at 70 °C and stirred for 16 h. After the reaction was completed (monitored by LCMS), the reaction was filtered, and the filtrate was concentrated and purified with FCC ($SiO_2$, EA/PE = 0-25%) to give 6-bromo-3-fluoro-4-methyl-2-nitrobenzonitrile (2.5 g, yield 39%) as a brown oily liquid. LC-MS (m/z): 259.0 (M + H) and 261.0 (M + H).

**Step E:** 2-amino-6-bromo-3-fluoro-4-methylbenzonitrile

Iron powder (4.3 g, 77.2 mmol) was added to a solution of 6-bromo-3-fluoro-4-methyl-2-nitrobenzonitrile (2.5 g, 9.65 mmol) in acetic acid (50 mL) at room temperature. After the addition was completed, the mixture was stirred at room temperature for 2 h. After the reaction was completed (monitored by LCMS), the reaction was filtered, quenched with saturated aqueous sodium bicarbonate solution, and extracted with ethyl acetate (100 mL × 3). The combined organic phase was washed with brine (200 mL), dried over anhydrous $Na_2SO_4$, filtered and concentrated. The crude product was purified by FCC ($SiO_2$, EA/PE = 0-25%) to obtain 2-amino-6-bromo-3-fluoro-4-methylbenzonitrile (1.7 g, yield 77%) as a yellow solid. LC-MS (m/z): 228.9 (M + H) and 230.9 (M + H).

**Step F:** 2-amino-3-fluoro-4-methyl-6-(4,4,5,5-tetramethyl-1,3,2-dioxaborolan-2-yl) benzonitrile

A room temperature, Pd(dppf)$Cl_2$ (282 mg, 0.39 mmol) was added to a solution of 2-amino-6-bromo-3-fluoro-4-methylbenzonitrile (1.0 g, 3.86 mmol), 4,4,4',4',5,5,5',5'-octamethyl-2,2'-bis(1,3,2-dioxaborolane) (1.96 g, 7.72 mmol), potassium acetate (1.14 g, 11.6 mmol) in dioxane (50 mL). The reaction was degassed with nitrogen three times, heated to 100 °C, and stirred for 2 h. After the reaction was completed (monitored by LCMS), the reaction was dried, and the crude product was purified with FCC ($SiO_2$, EA/PE = 0-30%) to obtain 2-amino-3-fluoro-4-methyl-6-(4,4,5,5-tetramethyl-1,3,2-dioxaborolan-2-yl) benzonitrile (1.1 g, yield 90%) as an oil substance. LC-MS (m/z): 278.0 (M + H).

**Step G:** *tert-butyl* (1R,5S)-3-(7-(3-amino-2-cyano-4-fluoro-5-methylphenyl)-2,6,8-trifluoroquinazolin-4-yl)-3,8-diazabicyclo[3.2.1]octane-8-carboxylate

At room temperature, Pd(dtppf)$Cl_2$ (260 mg, 0.40 mmol) was added to a solution of 2-amino-3-fluoro-4-methyl-6-(4,4,5,5-tetramethyl-1,3,2-dioxaborolan-2-yl) benzonitrile (1.1 g, 3.98 mmol), *tert-butyl* (1*R*,5*S*)-3-(7-bromo-2,6,8-trifluoroquinazolin-4-yl)-3,8-diazabicyclo[3.2.1]octane-8-carboxylate (1.3g, 2.78 mmol), potassium phosphate (2.5g, 12.0 mmol) in dioxane/water (V/V = 5:1, 60 mL). The reaction was degassed with nitrogen, heated to 110 °C and stirred for 2 h. After the reaction was completed (monitored by LCMS), the reaction was filtered, and the filtrate was concentrated. The resulting crude product was purified with FCC ($SiO_2$, EA/PE = 0 - 50%) to give *tert-butyl* (1*R*,5*S*)-3-(7-(3-amino-2-cyano-4-fluoro-5-methylphenyl)-2,6,8-trifluoroquinazoline-4-yl)-3,8-diazabicyclo[3.2.1]octane-8-carboxylate (1.0 g, yield 49%) as a yellow solid. LC-MS (m/z): 543.2 (M + H).

**Step H:** *tert-butyl* (1R,5S)-3-(7-(3-amino-2-cyano-4-fluoro-6-iodo-5-methylphenyl)-2,6,8-trifluoroquinazolin-4-yl)-3,8-diazabicyclo[3.2.1]octane-8-carboxylate

At room temperature, silver sulfate (3.0 g, 9.68 mmol) was added to a solution of *tert-butyl* (1*R*,5*S*)-3-(7-(3-amino-2-cyano-4-fluoro-5-methylphenyl)-2,6,8-trifluoroquinazoline-4-yl)-3,8-diazabicyclo[3.2.1]octane-8-carboxylate (1.0g, 1.94 mmol) and iodine (2.5g, 9.68 mmol) in DMF (30 mL). The reaction was stirred at room temperature in the dark for 16 h. After the reaction was completed (monitored by LCMS), the reaction was filtered, quenched with saturated aqueous sodium bisulfite (200 mL), and extracted with ethyl acetate (50 mL × 3). The organic phase was washed with brine (100 mL), dried over anhydrous $Na_2SO_4$, filtered and concentrated. The crude product was purified by FCC ($SiO_2$, EA/PE = 0 - 50%) to give tert-butyl (1R,5S)-3-(7-(3-amino-2-cyano-4-fluoro-6-iodo-5-methylphenyl)-2,6,8-trifluoroquinazolin-4-yl)-3,8-diazabicyclo[3.2.1]octane-8-carboxylate (1.1 g, yield 85%) as a yellow solid. LC-MS (m/z): 669.1 (M + H).

**Step I:** *tert*-butyl (1*R*,5*S*)-3-(7-(3-amino-2-cyano-4-fluoro-5-methyl-6-(trifluoromethyl)phenyl)-2,6,8-trifluoroquinazolin-4-yl)-3,8-diazabicyclo[3.2.1]octane-8-carboxylate

At room temperature, CuI (1.57 g, 8.23 mmol) was added to a solution of *tert*-butyl (1*R*,5*S*)-3-(7-(3-amino-2-cyano-4-fluoro-6-iodo-5-methylphenyl)-2,6,8-trifluoroquinazolin-4-yl)-3,8-diazabicyclo[3.2.1]octane-8-carboxylate (1.1 g, 1.65 mmol), methyl 2,2-difluoro-2-(fluorosulfonyl)acetate (3.16 g, 16.5 mmol) in DMA (30 mL). After the system was evacuated and backfilled with nitrogen three times, the reaction system was heated to 100 °C and stirred for 1 h. After the reaction was completed (monitored by LCMS), the reaction was filtered, quenched with water (200 mL), and extracted with ethyl acetate (50 mL × 3). The organic phase was washed with brine (150 mL), dried over anhydrous $Na_2SO_4$, filtered and concentrated. The crude product was purified by FCC ($SiO_2$, EA/PE = 0 - 40%) to give *tert*-butyl (1R,5S)-3-(7-(3-amino-2-cyano-4-fluoro-5-methyl-6-(trifluoromethyl)phenyl)-2,6,8-trifluoroquinazolin-4-yl)-3,8-diazabicyclo[3.2.1]octane-8-carboxylate (210 mg, yield 20%) as a yellow solid. LC-MS (m/z): 611.0 (M + H).

**Step J:** *tert*-butyl (1*R*,5*S*)-3-(7-(3-amino-2-cyano-4,5-dimethyl-6-trifluoromethylphenyl)-6,8-difluoro-2-(3S,4S)-4-fluoromethyl-1,3-dimethylpiperidin-3-methylmethoxy)quinazolin-4-yl)-3,8-diazabicyclo[3.2.1]octane-8-carboxylate

Sodium hydride (12 mg, 0.29 mmol, 60 wt%) was added to a solution of (3S,4S)-4-fluoromethyl-1,3-dimethylpiperidin-3-methanol (26 mg, 0.15 mmol) in THF (2 mL) at room temperature. After the addition was completed, the mixture was stirred at room temperature for 0.5 h. *tert*-butyl (1*R*,5*S*)-3-(7-(3-amino-2-cyano-4-fluoro-5-methyl-6-(trifluoromethyl)phenyl)-2,6,8-trifluoroquinazolin-4-yl)-3,8-diazabicyclo[3.2.1]octane-8-carboxylate (60 mg, 0.098 mmol) was added, and the reaction was stirred at room temperature after addition for 16 h. After the reaction was completed (monitored by LCMS), the reaction was quenched with saturated aqueous ammonium chloride (50 mL) and extracted with ethyl acetate (30 mL × 3). The combined organic phase was washed with brine (70 mL), dried over anhydrous $Na_2SO_4$, filtered and concentrated to give tert-butyl (1*R*,5*S*)-3-(7-(3-amino-2-cyano-4,5-dimethyl-6-trifluoromethylphenyl)-6,8-difluoro-2-(3S,4S)-4-fluoromethyl-1,3-dimethylpiperidin-3-methylmethoxy)quinazolin-4-yl)-3,8-diazabicyclo[3.2.1]octane-8-carboxylate (80 mg crude) as a brown solid. LC-MS (m/z): 766.2 (M + H).

**Step K:** *(Ra* or *Sa*)-2-(4-((1*R*,5*S*)-3,8-diazabicyclo[3.2.1]octan-3-yl)-6,8-difluoro-2-(((3*S*,4*S*)-4-(fluoromethyl)-1,3-dimethylpiperidin-3-yl)methoxy)quinazolin-7-yl)-6-amino-5-fluoro-4-methyl-3-(trifluoromethyl) benzonitrile·trifluoroacetate

TFA (1 mL) was added to a solution of *tert*-butyl (1R,5S)-3-(7-(3-amino-2-cyano-4,5-dimethyl-6-trifluoromethylphenyl)-6,8-difluoro-2-(3S,4S)-4-fluoromethyl-1,3-dimethylpiperidin-3-methylmethoxy)quinazolin-4-yl)-3,8-diazabicyclo[3.2.1]octane-8-carboxylate (80 mg, 0.11 mmol) in DCM (2 mL). After the addition was completed, the mixture was stirred at room temperature for 0.5 h. After the reaction was completed (monitored by LCMS), the reaction was concentrated, and the crude product was purified by pre-HPLC (C18, CAN/(0.1% FA/$H_2O$) = 20-40%) to obtain compound **140-1** eluted first (with a shorter retention time, 4.05 mg, yield 5.8%), LCMS (m/z): 666.2 (M + H); and compound **140-2** eluted later (with a longer retention time 5.98 mg, yield 8.6%), LCMS (m/z): 666.2 (M + H).

**Example 141-1 and example 141-2**

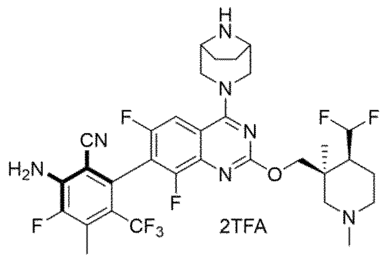

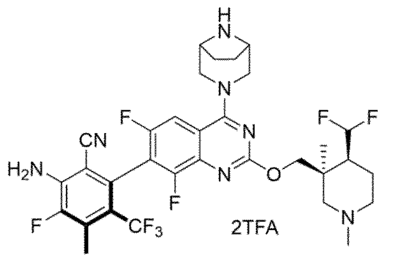

**141-1** or **141-2** **141-2** or **141-1**

*(Ra* or *Sa*)-2-(4-((1*R*,5*S*)-3,8-diazabicyclo[3.2.1]octan-3-yl)-2-(((3*S*,4*S*)-4-(difluoromethyl)-1,3-dimethylpiperidin-3-yl) methoxy)-6,8-difluoroquinazolin-7-yl)-6-amino-5-fluoro-4-methyl-3-(trifluoromethyl) benzonitrile·trifluoroacetate

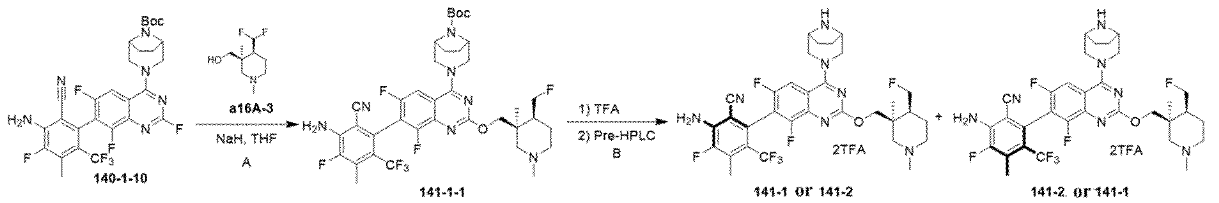


**Step A:** *tert-butyl* (1*R*,5*S*)-3-(7-(3-amino-2-cyano-4,5-dimethyl-6-trifluoromethylphenyl)-2-((3S,4S)-4-difluoromethyl-1,3-dimethylpiperidin-3-yl)methoxy)-6,8-difluoroquinazolin-4-yl)-3,8-diazabicyclo[3.2.1]octane-8-carboxylate

Sodium hydride (11 mg, 0.29 mmol, 60% wt) was added to a solution of (3S,4S)-4-difluoromethyl-1,3-dimethylpiperidin-3-methanol (28 mg, 0.15 mmol) in THF (2 mL). After the addition, the mixture was stirred at room temperature for 0.5 h. Tert-butyl (1R,5S)-3-(7-(3-amino-2-cyano-4-fluoro-5-methyl-6-(trifluoromethyl)phenyl)-2,6,8-trifluoroquinazolin-4-yl)-3,8-diazabicyclo[3.2.1]octane-8-carboxylate (60 mg, 0.098 mmol) was added then. After the addition, the reaction was stirred at room temperature for 16 h. After the reaction was completed (monitored by LCMS), the reaction was quenched with saturated aqueous ammonium chloride (50 mL) and extracted with ethyl acetate (30 mL × 3). The organic phase was washed with brine (70 mL), dried over anhydrous $Na_2SO_4$, filtered, and concentrated to give tert-butyl (1R,5S)-3-(7-(3-amino-2-cyano-4,5-dimethyl-6-trifluoromethylphenyl)-2-((3S,4S)-4-difluoromethyl-1,3-dimethylpiperidin-3-yl)methoxy)-6,8-difluoroquinazolin-4-yl)-3,8-diazabicyclo[3.2.1]octane-8-carboxylate (80 mg crude) as a brown solid. LC-MS (m/z): 784.3 (M + H).

**Step B:** (1R,55)-3-(7-(3-amino-2-cyano-4,5-dimethyl-6-trifluoromethylphenyl)-2-((3.5,4.5)-4-difluoromethyl-1,3-dimethylpiperidin-3-yl)methoxy)-6,8-difluoroquinazolin-4-yl)-3,8-diazabicyclo[3.2.1]octane-8-carboxylate

TFA (1 mL) was added to a solution of tert-butyl (1R,5S)-3-(7-(3-amino-2-cyano-4,5-dimethyl-6-trifluoromethylphenyl)-2-((3S,4S)-4-difluoromethyl-1,3-dimethylpiperidin-3-yl)methoxy)-6,8-difluoroquinazolin-4-yl)-3,8-diazabicyclo [3.2.1]octane-8-carboxylate (80.0 mg, 0.10 mmol) in DCM (2 mL). After the addition, the mixture was stirred at room temperature for 0.5 h. After the reaction was completed (monitored by LCMS), the reaction was concentrated, and the residue was purified by pre-HPLC (C18, CAN/(0.1% FA/$H_2O$) = 15-35%) to obtain compound **141-1** eluted first (with a shorter retention time, 9.40 mg, yield 13.5%), LCMS (m/z): 684.2 (M + H); and compound **141-2** eluted later (with a longer retention time, 12.34 mg, yield 17.7%), LCMS (m/z): 684.2 (M + H).

With reference to the above synthesis methods or appropriate variants, the following compounds were also synthesized and characterized in the present invention:

| Example | Structure | Name | Characterization data |
|---|---|---|---|
| 142-1 | 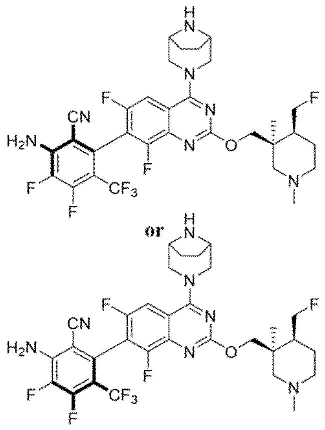 or | *(Ra* or *Sa*)-2-(4-((1*R*,5*S*)-3,8-diazabicyclo [3.2.1]octan-3-yl)-6,8-difluoro-2-(((3*S*,4*S*)-4-(fluoromethyl)-1,3-dimethylpiperidin-3-yl methoxy)quinazolin-7-yl)-6-amino-4, 5-difluoro-3-(trifluoromethyl) benzonitrile | LCMS (m/z): 670.3 (M + H). |
| 142-2 | 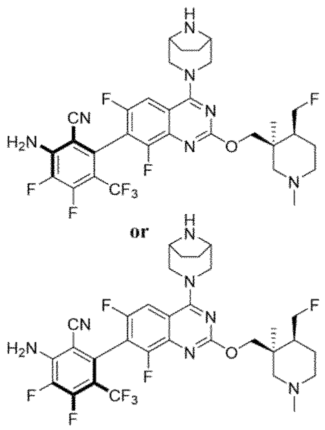 or | *(Ra* or *Sa*)-2-(4-((1*R*,5*S*)-3,8-diazabicyclo [3.2.1]octan-3-yl)-6,8-difluoro-2-(((3S,4S)-4-(fluoromethyl)-1,3-dimethylpiperidin-3-yl methoxy)quinazolin-7-yl)-6-amino-4, 5-difluoro-3-(trifluoromethyl) benzonitrile | LCMS (m/z): 670.3 (M + H). |
| 143-1 | 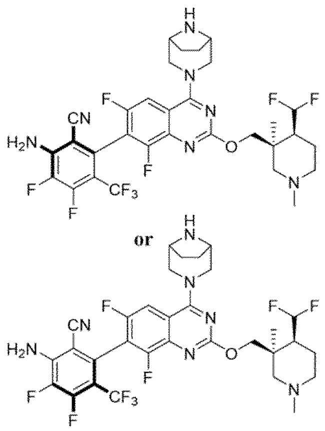 or | *(Ra* or *Sa)-2-(4-((1R,55)-3,8*-diazabicyclo [3.2.1]octan-3-yl)-2-(((3S,4S)-4-(difluoromethyl)-1,3-dimethylpiperidin-3-yl)methoxy)-6,8-difluoroquinazolin-7-yl)-6-amino-4,5-difluoro-3-(trifluoromethyl) benzonitrile | LCMS (m/z): 688.2 (M + H). |

(continued)

| Example | Structure | Name | Characterization data |
|---|---|---|---|
| 143-2 | 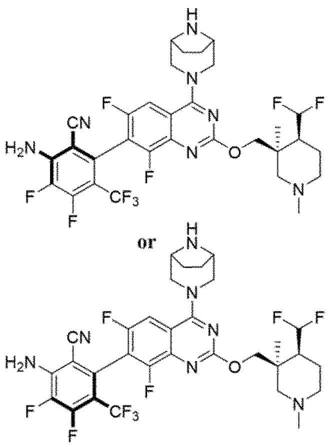 or | *(Ra* or *Sa*)-2-(4-((1*R*,5*S*)-3,8-diazabicyclo [3.2.1]octan-3-yl)-2-(((3*S*,4*S*)-4-(difluoromethyl)-1,3-dimethylpiperidin-3-yl)methoxy)-6,8-difluoroquinazolin-7-yl)-6-amino-4,5-difluoro-3-(trifluoromethyl) benzonitrile | LCMS (m/z): 688.2 (M + H). |

The compounds listed in the following table can be synthesized by referring to the above synthesis method or appropriate variants:

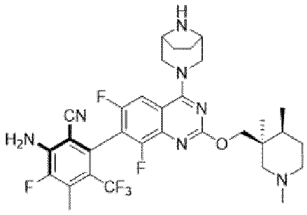
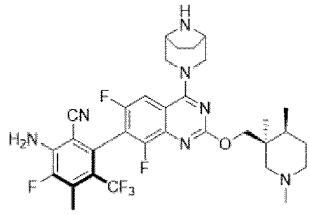
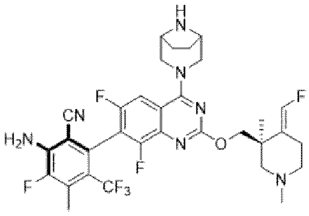
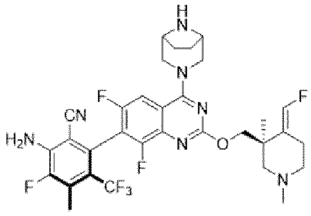

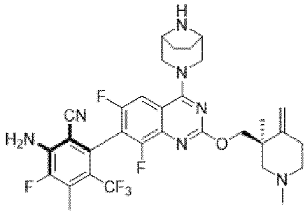
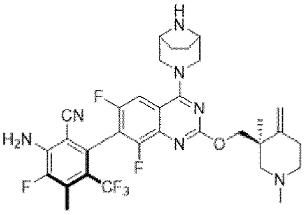
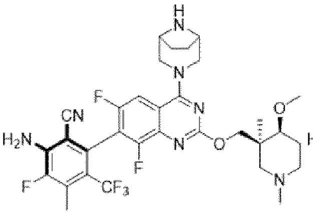
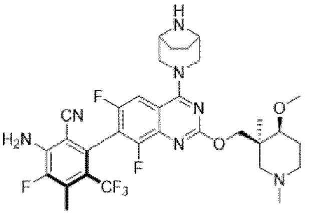

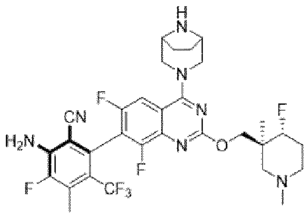
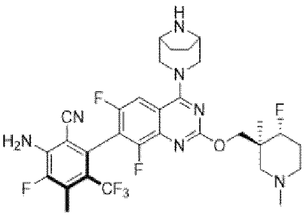
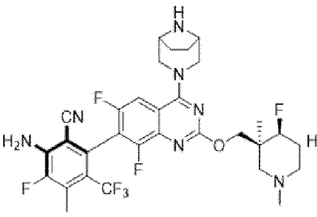
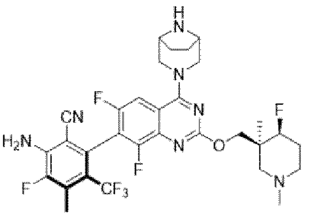

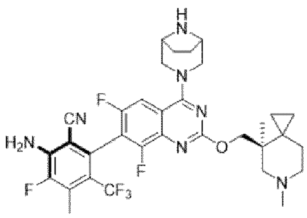
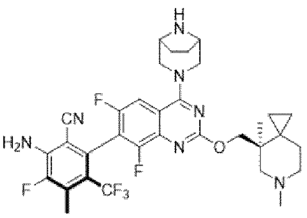
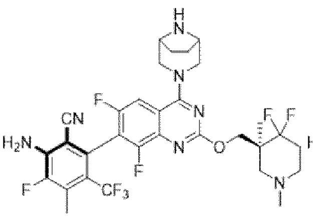
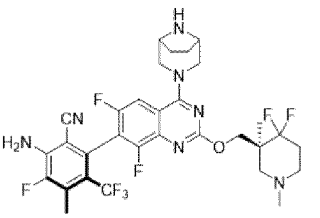

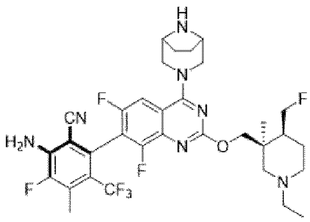
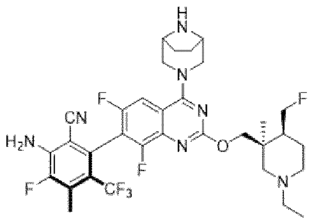
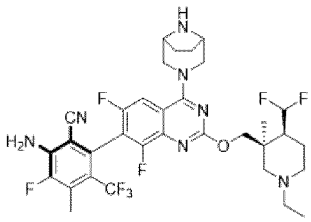
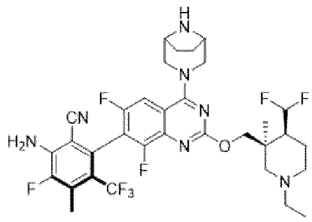

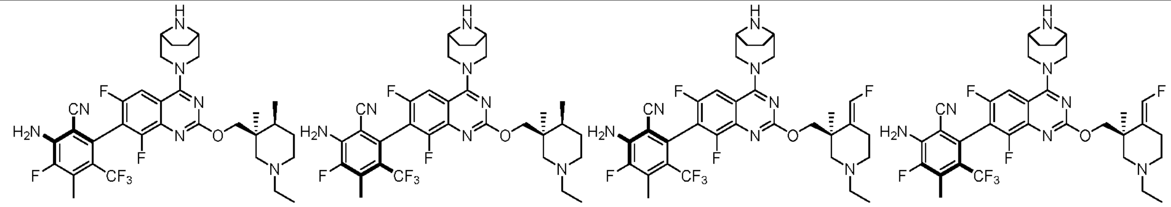

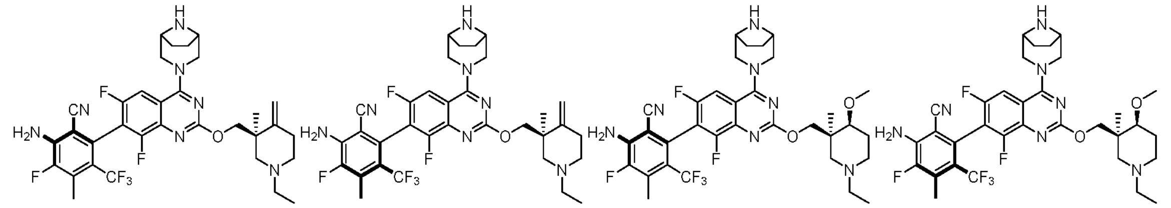

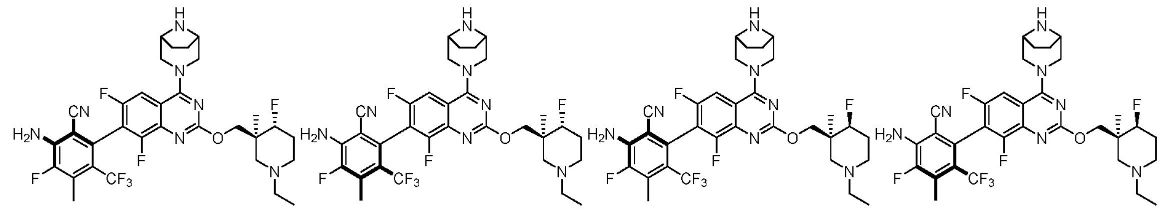

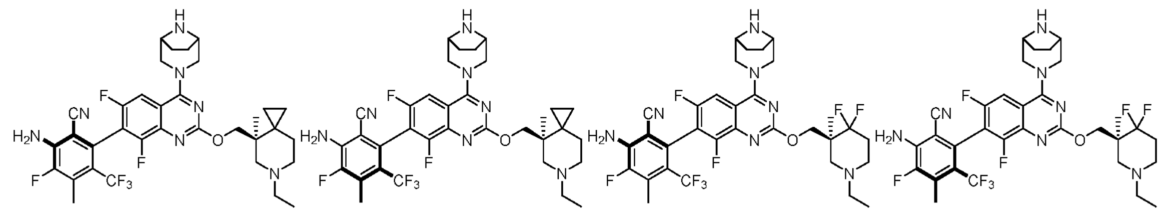

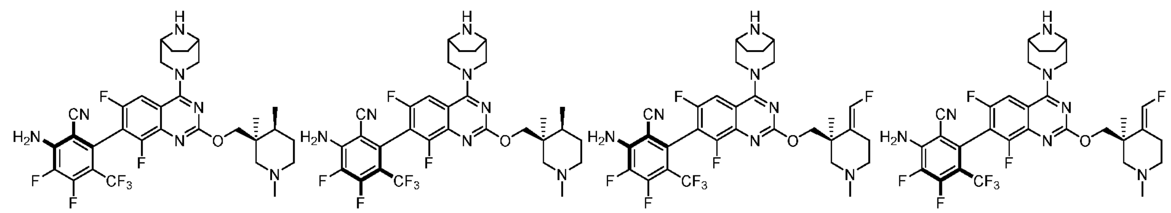

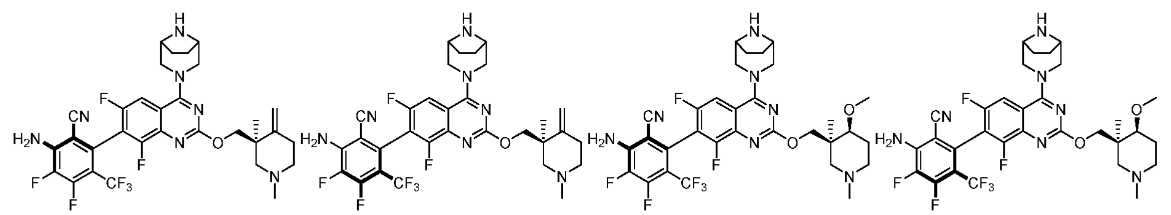

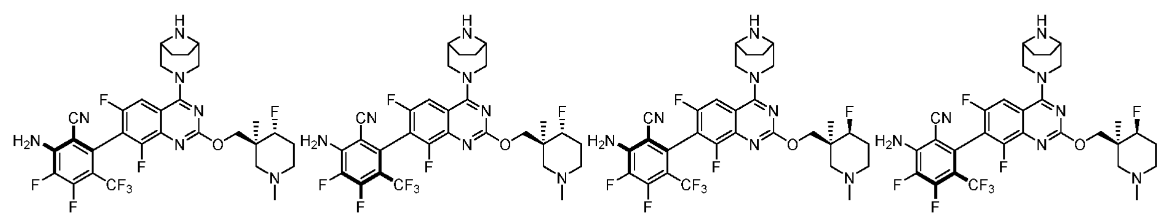

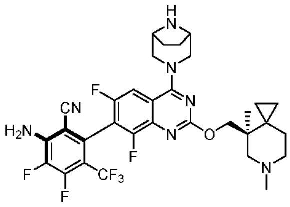
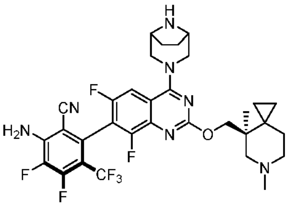
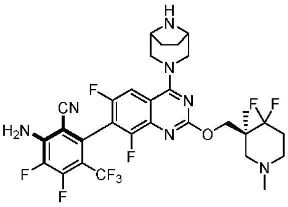
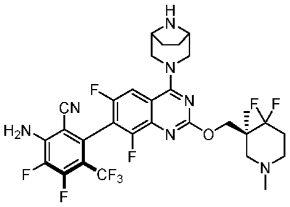
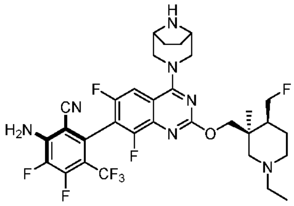
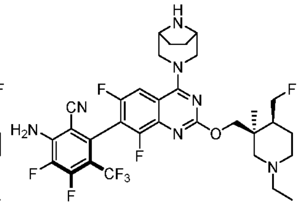
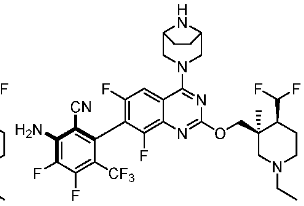
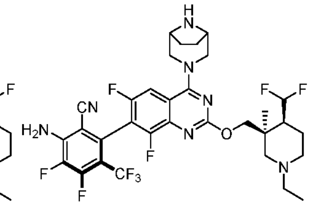
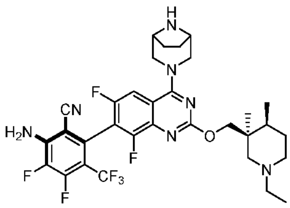
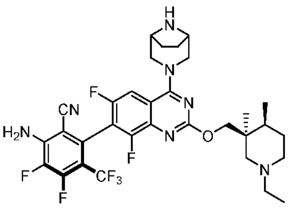
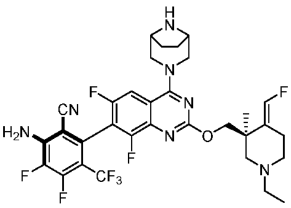
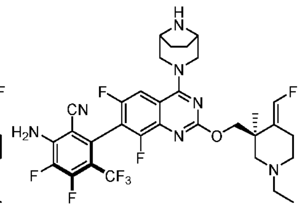
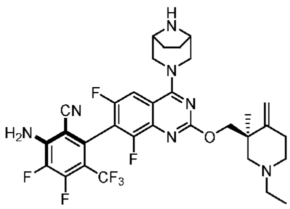
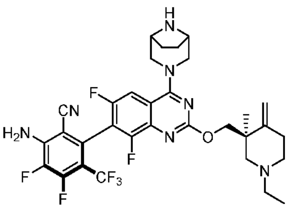
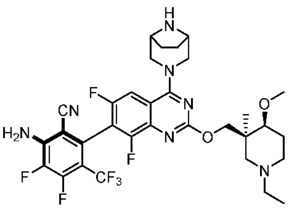
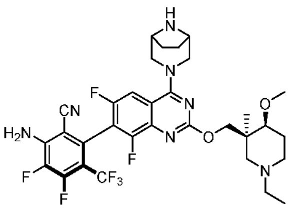
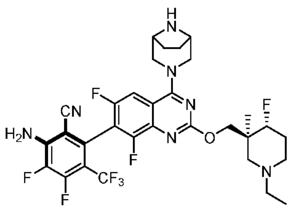
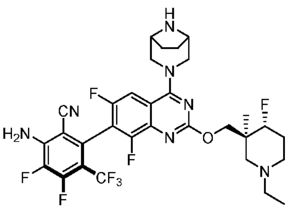
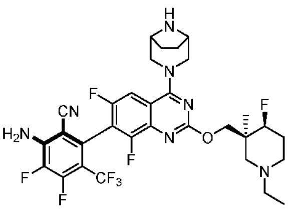
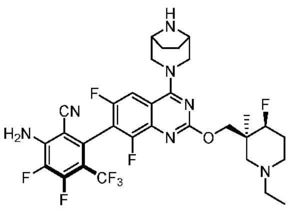
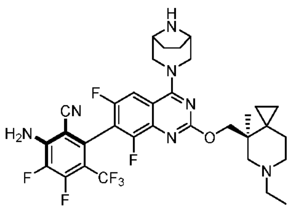
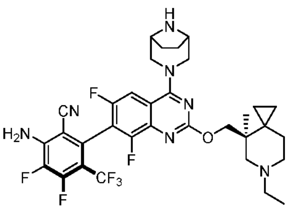
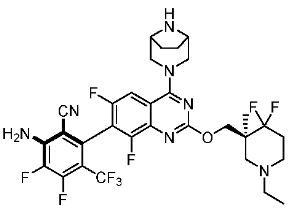
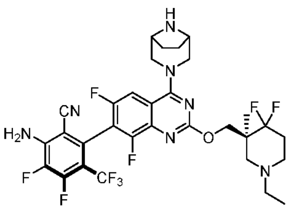
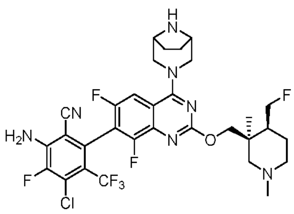
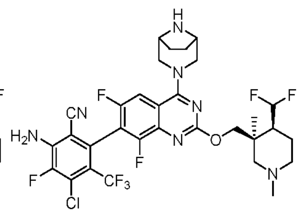
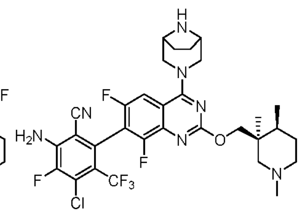
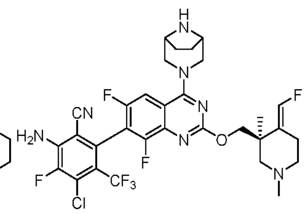

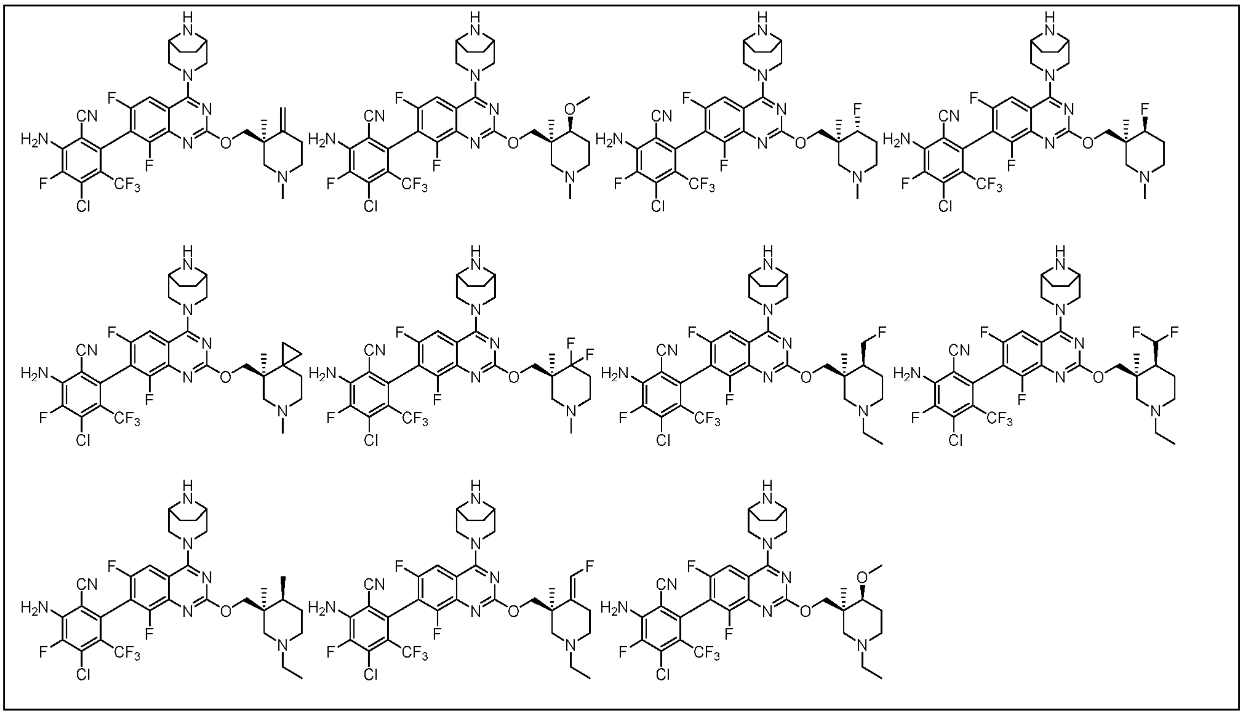

## Example 144

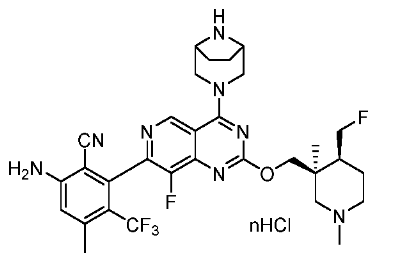

2-(4-((1R,5S)-3,8-diazabicyclo[3.2.1]octan-3-yl)-8-fluoro-2-(((3S,4S)-4-(fluoromethyl)-1,3-dimethylpiperidin-3-yl)methoxy)pyrido[4,3-d]pyrimidin-7-yl)-6-amino-4-methyl-3-(trifluoromethyl) benzonitrile·hydrochloride

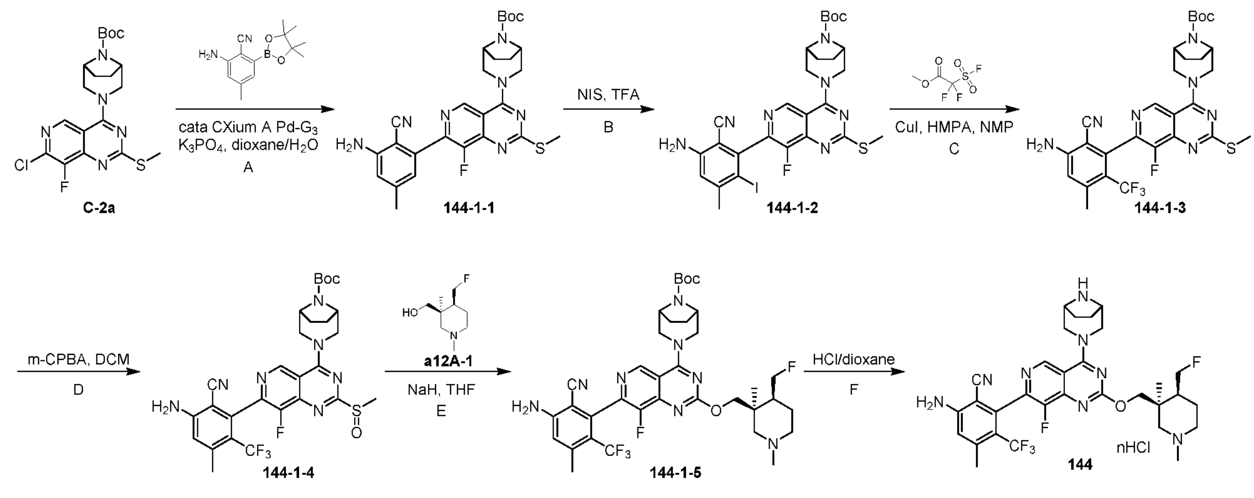

C-2a
144-1-1
144-1-2
144-1-3
144-1-4
144-1-5
144

**Step** A: *tert-butyl* (1R,5S)-3-(7-(3-amino-2-cyano-5-methylphenyl)-8-fluoro-2-(methylthio)pyrido[4,3-d]pyrimidin-4-yl)-3,8-diazabicyclo[3.2.1]octane-8-carboxylate

Under $N_2$ at room temperature, 2-amino-4-methyl-6-(4,4,5,5-tetramethyl-1,3,2-dioxaborolan-2-yl) benzonitrile (968 mg, 3.75 mmol), potassium phosphate (1.59 g, 7.5 mmol), and cataCXium-A-Pd-G3 (182 mg, 0.25 mmol) were added to a solution of tert-butyl (1R,5S)-3-(7-chloro-8-fluoro-2-(methylthio)pyrido[4,3-d]pyrimidin-4-yl)-3,8-diazabicyclo[3.2.1] octane-8-carboxylate (1.1 g, 2.5 mmol) in 1,4-dioxane (15 mL) and water (3 mL). The reaction was heated to 100 °C and stirred for 2 h. After the reaction was completed (monitored by LCMS), the reaction was filtered with celite and the filtrate was concentrated to dryness. The resulting crude product was purified by FCC ($SiO_2$, EA/PE = 0 - 50%) to obtain tert-butyl (1R,5S)-3-(7-(3-amino-2-cyano-5-methylphenyl)-8-fluoro-2-(methylthio)pyrido[4,3-d]pyrimidin-4-yl)-3,8-diazabicyclo [3.2.1]octane-8-carboxylate (1.0 g, yield 74%) as a yellow solid. LCMS (m/z): 536.2 (M + H).

**Step B:** *tert-butyl* (1R,5S)-3-(7-(3-amino-2-cyano-6-iodo-5-methylphenyl)-8-fluoro-2-(methylthio)pyrido[4,3-*d*]pyrimidin-4-yl)-3,8-diazabicyclo[3.2.1]octane-8-carboxylate

At room temperature, trifluoroacetic acid (21 mg, 0.18 mmol) was added to a solution of *tert*-butyl (1R,5S)-3-(7-(3-amino-2-cyano-5-methylphenyl)-8-fluoro-2-(methylthio)pyrido[4,3-d]pyrimidin-4-yl)-3,8-diazabicyclo [3.2.1]octane-8-carboxylate (1.0 g, 1.87 mmol) and NIS (1.7 g, 7.47 mmol) in acetonitrile. The reaction was stirred at room temperature for 0.5 h. After the reaction was completed (monitored by LCMS), saturated aqueous sodium bicarbonate (100 mL) was added to the reaction. The resulting mixture was extraction with ethyl acetate. The combined organic phase was washed with brine, dried overover anhydrous sodium sulfate, filtrated and concentrated. The crude product obtained was purified by FCC ($SiO_2$, EA/PE = 0-40%) to afford *tert*-butyl (1*R,5S*)-3-(7-(3-amino-2-cyano-6-iodo-5-methylphenyl)-8-fluoro-2-(methylthio)pyrido[4,3-d]pyrimidin-4-yl)-3,8-diazabicyclo[3.2.1]octane-8-carboxylate (1.1 g, yield 89%) as a yellow solid. LCMS (m/z): 662.0 (M + H).

**Step C:** *tert*-butyl (1R,5S)-3-(7-(3-amino-2-cyano-5-methyl-6-(trifluoromethyl)phenyl)-8-fluoro-2-(methylthio)pyrido [4,3-d]pyrimidin-4-yl)-3,8-diazabicyclo[3.2.1]octane-8-carboxylate

Under $N_2$ at room temperature, methyl 2,2-difluoro-2-(fluorosulfonyl)acetate (1.16 g, 6.0 mmol), cuprous iodide (431 mg, 2.2 mmol), and hexamethylphosphoric triamide (1.08 g, 6.0 mmol) were added to a solution of tert-butyl (1R,5S)-3-(7-(3-amino-2-cyano-6-iodo-5-methylphenyl)-8-fluoro-2-(methylthio)pyrido[4,3-d]pyrimidin-4-yl)-3,8-diazabicyclo[3.2.1]octane-8-carboxylate (1.0 g, 1.5 mmol) in NMP (20 mL).The reaction was stirred at 100 °C for 10 min. After the reaction was completed (monitored by LCMS), the reaction was poured into 50 mL of ice-cold water and extracted with ethyl acetate. The combined organic phases was washed with brine, dried over anhydrous sodium sulfate, filtered and concentrated to dryness. The resulting crude product was purified by FCC ($SiO_2$, EtOAc/PE = 0 - 50%) to give *tert*-butyl (1*R*,5*S*)-3-(7-(3-amino-2-cyano-5-methyl-6-(trifluoromethyl)phenyl)-8-fluoro-2-(methylthio)pyrido[4,3-d]pyrimidin-4-yl)-3,8-diazabicyclo[3.2.1]octane-8-carboxylate (150 mg, yield 16%) as a yellow solid. LCMS (m/z): 604.2 (M + H).

**Step D:** *tert*-butyl (1R,5S)-3-(7-(3-amino-2-cyano-5-methyl-6-(trifluoromethyl)phenyl)-8-fluoro-2-(methylsulfinyl)pyrido [4,3-d]Pyrimidine-4-yl)-3,8-diazabicyclo[3.2.1]octane-8-carboxylate

At room temperature, m-chloroperbenzoic acid (51 mg, 0.3 mmol) was added to a solution of *tert-butyl* (1R,5S)-3-(7-(3-amino-2-cyano-5-methyl-6-(trifluoromethyl)phenyl)-8-fluoro-2-(methylthio)pyrido[4,3-d]pyrimidin-4-yl)-3,8-diazabicyclo[3.2.1]octane-8-carboxylate (150 mg, 0.24 mmol) in dichloromethane (5 mL) , and the resulting mixture was stirred at same temperature for 1 h. After the reaction was completed (monitored by LCMS), 20 mL of saturated sodium bicarbonate solution was added, and the resulting mixture was extracted with dichloromethane. The combined organic phase was washed with brine, dried over anhydrous sodium sulfate, filtered and concentrated to obtain the crude product tert-butyl (1*R*,5*S*)-3-(7-(3-amino-2-cyano-5-methyl-6-(trifluoromethyl)phenyl)-8-fluoro-2-(methylsulfinyl)pyrido[4,3-d]pyrimidine-4-yl)-3,8-diazabicyclo[3.2.1]octane-8-carboxylate (160 mg). LCMS (m/z): 621.2 (M + H).

**Step E:** *tert*-butyl (1R,5S)-3-(7-(3-amino-2-cyano-5-methyl-6-(trifluoromethyl)phenyl)-8-fluoro-2-(((3S,4S)-4-(fluoromethyl)-1,3-dimethylpiperidin-3-yl)methoxy)pyrido[4,3-d]pyrimidin-4-yl)-3,8-diazabicyclo[3.2.1]octane-8-carboxylate

Under an ice-bath, sodium hydride (20 mg, 0.52 mmol) was added to a solution of ((3S,4S)-4-(fluoromethyl)-1,3-dimethylpiperidin-3-yl)methanol (17 mg, 0.09 mol) in anhydrous tetrahydrofuran (2 mL), and the resulting mixture was stirred at the same temperature for 0.5 h. Then, tert-butyl (1*R*,5*S*)-3-(7-(3-amino-2-cyano-5-methyl-6-(trifluoromethyl) phenyl)-8-fluoro-2-(methylsulfinyl)pyrido[4,3-d]pyrimidine-4-yl)-3,8-diazabicyclo[3.2.1]octane-8-carboxylate (160 mg, 0.26 mmol) was added. The resulting mixture was stirred at room temperature for 2 h. After the completion of the

reaction was monitored by LCMS, the reaction solution was poured into 30 mL of ice-water to quench the reaction. The mixture was then extracted with ethyl acetate. The combined organic phases were washed with saturated brine, dried over anhydrous sodium sulfate, filtered and concentrated to dryness. The obtained crude product was purified by FCC ($SiO_2$, MeOH/DCM = 0 - 5%) to obtain yellow solid product tert-butyl (1*R*,5*S*)-3-(7-(3-amino-2-cyano-5-methyl-6-(trifluoromethyl) phenyl)-8-fluoro-2-(((3S,4S)-4-(fluoromethyl)-1,3-dimethylpiperidin-3-yl)methoxy)pyrido[4,3-d]pyrimidin-4-yl)-3,8-diazabicyclo[3.2.1]octane-8-carboxylate (20 mg, yield 11%). LCMS (m/z): 731.3 (M + H).

**Step F:** 2-(4-((1R,5S)-3,8-diazabicyclo[3.2.1]octan-3-yl)-8-fluoro-2-(((3S,4S)-4-(fluoromethyl)-1,3-dimethylpiperidin-3-yl)methoxy)pyrido[4,3-*d*]pyrimidin-7-yl)-6-amino-4-methyl-3-(trifluoromethyl) benzonitrile·hydrochloride

At room temperature, HCl in 1,4-dioxane solution (1 mL, 4 M) was added to *tert*-butyl (1*R*,5*S*)-3-(7-(3-amino-2-cyano-5-methyl-6-(trifluoromethyl)phenyl)-8-fluoro-2-(((3S,4S)-4-(fluoromethyl)-1,3-dimethylpiperidin-3-yl)methoxy) pyrido[4,3-*d*]pyrimidin-4-yl)-3,8-diazabicyclo[3.2.1]octane-8-carboxylate (20 mg, 0.03mol), and the reaction was stirred at the same temperature for 1 h. Then the reaction was diluted with EtOAc (2 mL), filtered. The filter cake was washed with EtOAc (1 mL) and lyophilized to afford 2-(4-((1R,5S)-3,8-diazabicyclo[3.2.1]octan-3-yl)-8-fluoro-2-(((3S,4S)-4-(fluoromethyl)-1,3-dimethylpiperidin-3-yl)methoxy)pyrido[4,3-d]pyrimidin-7-yl)-6-amino-4-methyl-3-(trifluoromethyl) benzonitrile·hydrochloride (2 mg) as a yellow solid. LCMS (m/z): 631.3 (M + H).

With reference to the above synthesis methods or appropriate variants, the following compounds were also synthesized and characterized in the present invention:

| Ex. | Structure | Name | Characterization data |
|---|---|---|---|
| 145 | 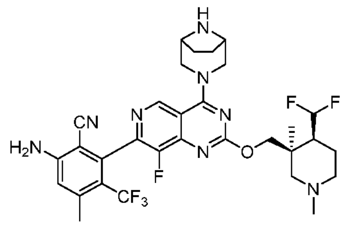 | 2-(4-((1R,5S)-3,8-diazabicyclo[3.2.1]octan-3-yl)-2-(((3S,4S)-4-(difluoromethyl)-1,3-dimethylpiperidin-3-yl)methoxy)-8-fluoropyridino[4,3-*d*]pyrimidin-7-yl)-6-amino-4-methyl-3-(trifluoromethyl) benzonitrile | LCMS (m/z): 649.3 (M + H). |
| 146 | 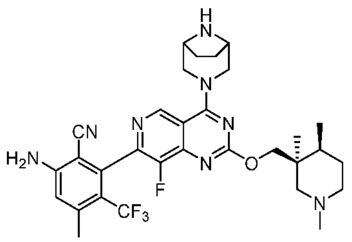 | 2-(4-((1R,5S)-3,8-diazabicyclo[3.2.1]octan-3-yl)-8-fluoro-2-(((3S,4S)-1,3,4-trimethylpiperidin-3-yl)methoxy)pyrido[4,3-d]pyrimidin-7-yl)-6-amino-4-methyl-3-(trifluoromethyl) benzonitrile | LCMS (m/z): 613.3 (M + H). |
| 147 | 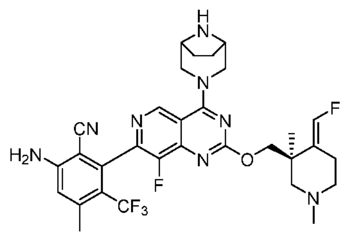 | 2-(4-((1R,5S)-3,8-diazabicyclo[3.2.1]octan-3-yl)-8-fluoro-2-((((S,E)-4-(fluoromethylene)-1,3-dimethylpiperidin-3-yl)methoxy)pyrido[4,3-d]pyrimidin-7-yl)-6-amino-4-methyl-3-(trifluoromethyl) benzonitrile | LCMS (m/z): 629.3 (M + H). |
| 148 | 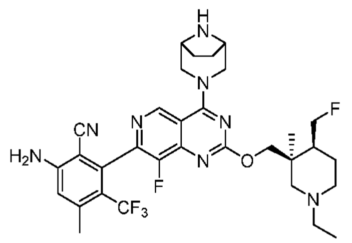 | 2-(4-((1R,5S)-3,8-diazabicyclo[3.2.1]octan-3-yl)-2-(((3S,4S)-1-ethyl-4-(fluoromethyl)-3-methylpiperidin-3-yl)methoxy)-8-fluoropyridino[4,3-*d*]pyrimidin-7-yl)-6-amino-4-methyl-3-(trifluoromethyl) benzonitrile | LCMS (m/z): 645.3 (M + H) |

(continued)

| Ex. | Structure | Name | Characterization data |
|---|---|---|---|
| 149 | 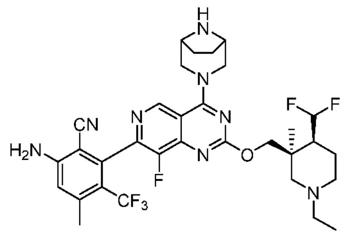 | 2-(4-((1R,5S)-3,8-diazabicyclo[3.2.1]octan-3-yl)-2-(((3S,4S)-4-(difluoromethyl)-1-ethyl-3-methylpiperidin-3-yl)methoxy)-8-fluoropyridino [4,3-*d*]pyrimidin-7-yl)-6-amino-4-methyl-3-(trifluoromethyl) benzonitrile | LCMS (m/z): 663.3 (M + H). |
| 150 | 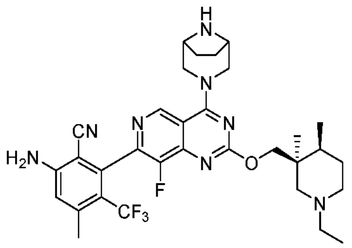 | 2-(4-((1R,5S)-3,8-diazabicyclo[3.2.1]octan-3-yl)-2-(((3S,4S)-1-ethyl-3,4-dimethylpiperidin-3-yl)methoxy)-8-fluoropyridino[4,3-*d*]pyrimidin-7-yl)-6-amino-4-methyl-3-(trifluoromethyl) benzonitrile | LCMS (m/z): 627.3 (M + H). |
| 151 | 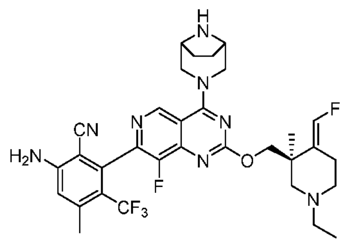 | 2-(4-((1R,5S)-3,8-diazabicyclo[3.2.1]octan-3-yl)-2-(((S,E)-1-ethyl-4-(fluoromethylene)-3-methylpiperidin-3-yl)methoxy)-8-fluoropyridino [4,3-d]pyrimidin-7-yl)-6-amino-4-methyl-3-(trifluoromethyl) benzonitrile | LCMS (m/z): 643.3 (M + H). |
| 152 | 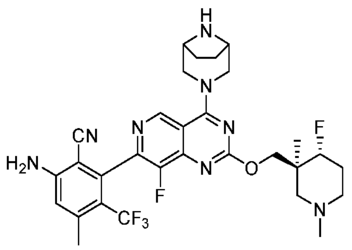 | 2-(4-((1R,5S)-3,8-diazabicyclo[3.2.1]octan-3-yl)-8-fluoro-2-(((3S,4R)-4-fluoro-1,3-dimethylpiperidin-3-yl)methoxy)pyrido[4,3-d]pyrimidin-7-yl)-6-amino-4-methyl-3-(trifluoromethyl) benzonitrile | LCMS (m/z): 617.3 (M + H). |
| 153 | 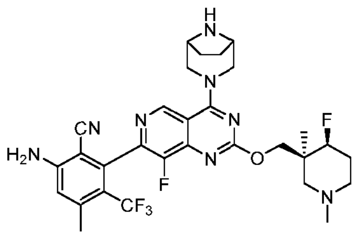 | 2-(4-((1*R*,5*S*)-3,8-diazabicyclo[3.2.1]octan-3-yl)-8-fluoro-2-(((3S,4S)-4-fluoro-1,3-dimethylpiperidin-3-yl)methoxy)pyrido[4,3-d]pyrimidin-7-yl)-6-amino-4-methyl-3-(trifluoromethyl) benzonitrile | LCMS (m/z): 617.3 (M + H). |
| 154 | 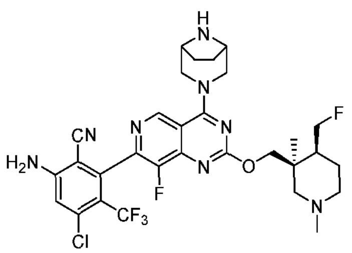 | 2-(4-((1R,5S)-3,8-diazabicyclo[3.2.1]octan-3-yl)-8-fluoro-2-(((3S,4S)-4-(fluoromethyl)-1,3-dimethylpiperidin-3-yl)methoxy)pyrido[4,3-d]pyrimidin-7-yl)-6-amino-4-chloro-3-(trifluoromethyl) benzonitrile | LCMS (m/z): 651.2 (M + H). |

(continued)

| Ex. | Structure | Name | Characterization data |
|---|---|---|---|
| 155 | 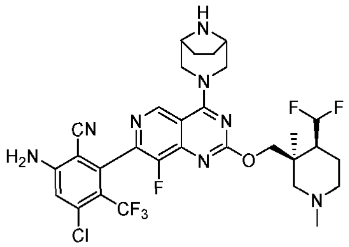 | 2-(4-((1R,5S)-3,8-diazabicyclo[3.2.1]octan-3-yl)-2-(((3S,4S)-4-(difluoromethyl)-1,3-dimethylpiperidin-3-yl)methoxy)-8-fluoropyridino[4,3-d]pyrimidin-7-yl)-6-amino-4-chloro-3-(trifluoromethyl) benzonitrile | LCMS (m/z): 669.2 (M + H). |
| 156 | 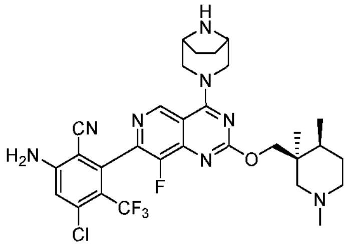 | 2-(4-((1R,5S)-3,8-diazabicyclo[3.2.1]octan-3-yl)-8-fluoro-2-(((3S,4S)-1,3,4-trimethylpiperidin-3-yl)methoxy)pyrido[4,3-*d*]pyrimidin-7-yl)-6-amino-4-chloro-3-(trifluoromethyl) benzonitrile | LCMS (m/z): 633.3 (M + H). |
| 157 | 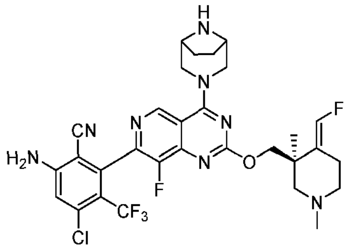 | 2-(4-((1*R*,5*S*)-3,8-diazabicyclo[3.2.1]octan-3-yl)-8-fluoro-2-(((S,E)-4-(fluoromethylene)-1,3-dimethylpiperidin-3-yl)methoxy)pyrido[4,3-d]pyrimidin-7-yl)-6-amino-4-chloro-3-(trifluoromethyl) benzonitrile | LCMS (m/z): 649.2 (M + H). |
| 158 | 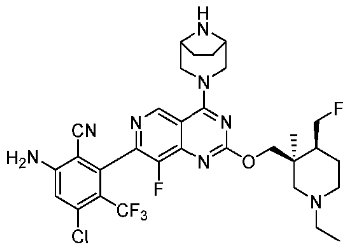 | 2-(4-((1*R*,5*S*)-3,8-diazabicyclo[3.2.1]octan-3-yl)-2-(((3S,4S)-1-ethyl-4-(fluoromethyl)-3-methylpiperidin-3-yl)methoxy)-8-fluoropyridino [4,3-d]pyrimidin-7-yl)-6-amino-4-chloro-3-(trifluoromethyl) benzonitrile | LCMS (m/z): 665.3 (M + H). |
| 159 | 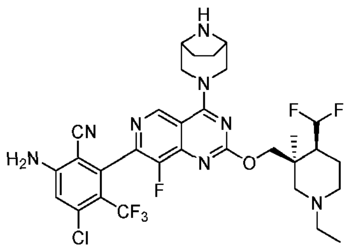 | 2-(4-((1R,5S)-3,8-diazabicyclo[3.2.1]octan-3-yl)-2-(((3S,4S)-4-(difluoromethyl)-1-ethyl-3-methylpiperidin-3-yl)methoxy)-8-fluoropyridino [4,3-d]pyrimidin-7-yl)-6-amino-4-chloro-3-(trifluoromethyl) benzonitrile | LCMS (m/z): 683.2 (M + H). |
| 160 | 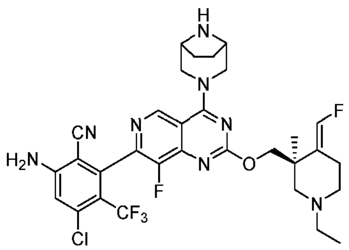 | 2-(4-((1R,5S)-3,8-diazabicyclo[3.2.1]octan-3-yl)-2-(((S,E)-1-ethyl-4-(fluoromethylene)-3-methylpiperidin-3-yl)methoxy)-8-fluoropyridino [4,3-d]pyrimidin-7-yl)-6-amino-4-chloro-3-(trifluoromethyl) benzonitrile | LCMS (m/z): 663.2 (M + H). |

(continued)

| Ex. | Structure | Name | Characterization data |
|---|---|---|---|
| 161 | 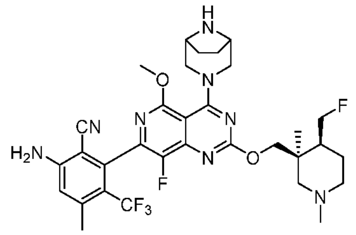 | 2-(4-(1R,5S)-3,8-diazabicyclo[3.2.1]octan-3-yl)-8-fluoro-2-(((3S,4S)-4-(fluoromethyl)-1,3-dimethylpiperidin-3-yl)methoxy)-5-methoxypyrido [4,3-d]pyrimidin-7-yl)-6-amino-4-methyl-3-(trifluoromethyl) benzonitrile | LCMS (m/z): 661.3 (M + H). |
| 162 | 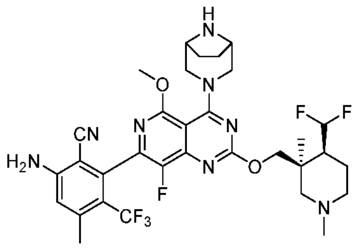 | 2-(4-((1R,5S)-3,8-diazabicyclo[3.2.1]octan-3-yl)-2-(((3S,4S)-4-(difluoromethyl)-1,3-dimethylpiperidin-3-yl)methoxy)-8-fluoro-5-methoxypyrido [4,3-d]pyrimidin-7-yl)-6-amino-4-methyl-3-(trifluoromethyl) benzonitrile | LCMS (m/z): 679.3 (M + H). |
| 163 | 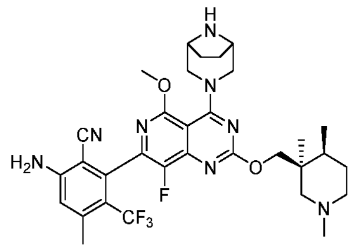 | 2-(4-((1R,5S)-3,8-diazabicyclo[3.2.1]octan-3-yl)-8-fluoro-5-methoxy-2-(((3S,4S)-1,3,4-trimethylpiperidin-3-yl)methoxy)pyrido[4,3-d]pyrimidin-7-yl)-6-amino-4-methyl-3-(trifluoromethyl) benzonitrile | LCMS (m/z): 643.3 (M + H). |
| 164 | 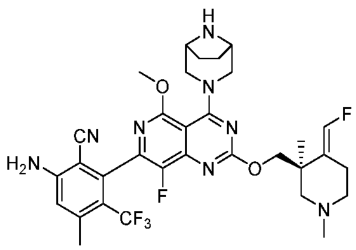 | 2-(4-((1R,5S)-3,8-diazabicyclo[3.2.1]octan-3-yl)-8-fluoro-2-(((S,E)-4-(fluoromethylene)-1,3-dimethylpiperidin-3-yl)methoxy)-5-methoxypyrido [4,3-d]pyrimidin-7-yl)-6-amino-4-methyl-3-(trifluoromethyl) benzonitrile | LCMS (m/z): 659.3 (M + H). |
| 165 | 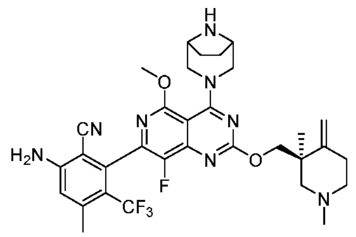 | 2-(4-((1R,5S)-3,8-diazabicyclo[3.2.1]octan-3-yl)-2-(((S)-1,3-dimethyl-4-methylene piperidin-3-yl)methoxy)-8-fluoro-5-methoxypyrido[4,3-d]pyrimidin-7-yl)-6-amino-4-methyl-3-(trifluoromethyl) benzonitrile | LCMS (m/z): 641.3 (M + H). |
| 166 | 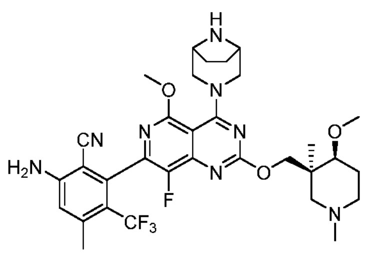 | 2-(4-((1R,5S)-3,8-diazabicyclo[3.2.1]octan-3-yl)-8-fluoro-5-methoxy-2-(((3S,4S)-4-methoxy-1,3-dimethylpiperidin-3-yl)methoxy)pyrido [4,3-d]pyrimidin-7-yl)-6-amino-4-methyl-3-(trifluoromethyl) benzonitrile | LCMS (m/z): 659.3 (M + H). |

(continued)

| Ex. | Structure | Name | Characterization data |
|---|---|---|---|
| 167 | 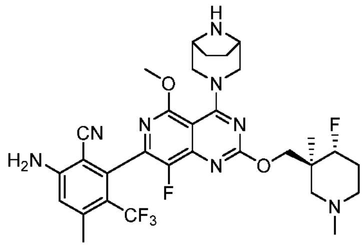 | 2-(4-((1R,5S)-3,8-diazabicyclo[3.2.1]octan-3-yl)-8-fluoro-2-(((3S,4R)-4-fluoro-1,3-dimethylpiperidin-3-yl)methoxy)-5-methoxypyrido[4,3-d]pyrimidin-7-yl)-6-amino-4-methyl-3-(trifluoromethyl) benzonitrile | LCMS (m/z): 647.3 (M + H). |
| 168 | 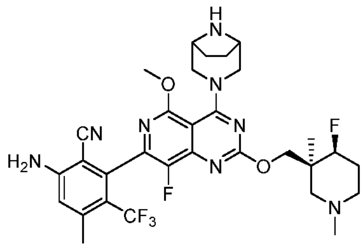 | 2-(4-((1R,5S)-3,8-diazabicyclo[3.2.1]octan-3-yl)-8-fluoro-2-(((3*S*,4*S*)-4-fluoro-1,3-dimethylpiperidin-3-yl)methoxy)-5-methoxypyrido[4,3-d]pyrimidin-7-yl)-6-amino-4-methyl-3-(trifluoromethyl) benzonitrile | LCMS (m/z): 647.3 (M + H). |
| 169 | 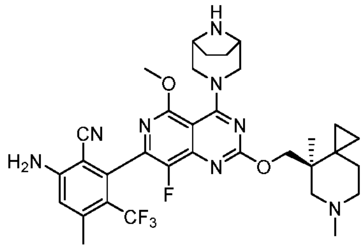 | 2-(4-((1R,5S)-3,8-diazabicyclo[3.2.1]octan-3-yl)-2-(((*S*)-4,6-dimethyl-6-azaspiro[2.5]octan-4-yl)methoxy)-8-fluoro-5-methoxypyrido[4,3-d]pyrimidin-7-yl)-6-amino-4-methyl-3-(trifluoromethyl) benzonitrile | LCMS (m/z): 655.3 (M + H). |
| 170 | 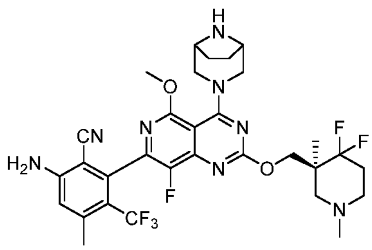 | 2-(4-((1R,5S)-3,8-diazabicyclo[3.2.1]octan-3-yl)-2-(((S)-4,4-difluoro-1,3-dimethylpiperidin-3-yl)methoxy)-8-fluoro-5-methoxypyrido[4,3-d]pyrimidin-7-yl)-6-amino-4-methyl-3-(trifluoromethyl) benzonitrile | LCMS (m/z): 665.3 (M + H). |
| 171 | 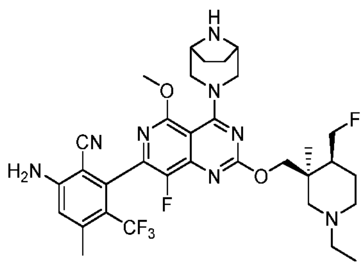 | 2-(4-((1R,5S)-3,8-diazabicyclo[3.2.1]octan-3-yl)-2-(((3S,4S)-1-ethyl-4-(fluoromethyl)-3-methylpiperidin-3-yl)methoxy)-8-fluoro-5-methoxypyrido[4,3-d]pyrimidin-7-yl)-6-amino-4-methyl-3-(trifluoromethyl) benzonitrile | LCMS (m/z): 675.3 (M + H). |
| 172 | 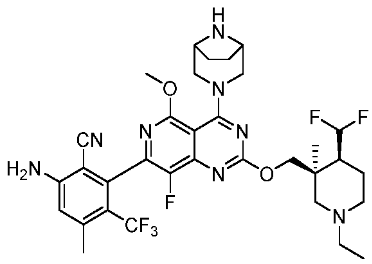 | 2-(4-((1R,5S)-3,8-diazabicyclo[3.2.1]octan-3-yl)-2-(((3S,4S)-4-(difluoromethyl)-1-ethyl-3-methylpiperidin-3-yl)methoxy)-8-fluoro-5-methoxypyrido[4,3-d]pyrimidin-7-yl)-6-amino-4-methyl-3-(trifluoromethyl) benzonitrile | LCMS (m/z): 693.3 (M + H). |

(continued)

| Ex. | Structure | Name | Characterization data |
|---|---|---|---|
| 173 | 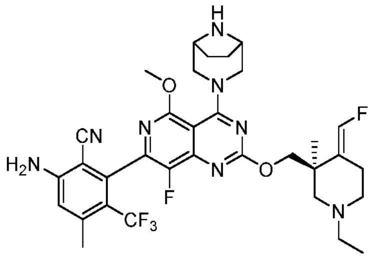 | 2-(4-((1R,5S)-3,8-diazabicyclo[3.2.1]octan-3-yl)-2-(((S,E)-1-ethyl-4-(fluoromethylene)-3-methylpiperidin-3-yl)methoxy)-8-fluoro-5-methoxypyrido[4,3-d]pyrimidin-7-yl)-6-amino-4-methyl-3-(trifluoromethyl) benzonitrile | LCMS (m/z): 673.3 (M + H). |
| 174 | 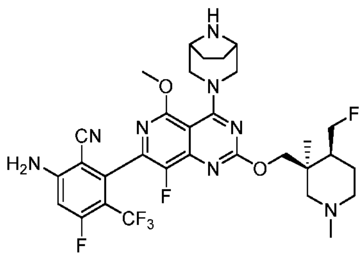 | 2-(4-((1R,5S)-3,8-diazabicyclo[3.2.1]octan-3-yl)-8-fluoro-2-(((3S,4S)-4-(fluoromethyl)-1,3-dimethylpiperidin-3-yl)methoxy)-5-methoxypyrido [4,3-d]pyrimidin-7-yl)-6-amino-4-fluoro-3-(trifluoromethyl) benzonitrile | LCMS (m/z): 665.3 (M + H). |
| 175 | 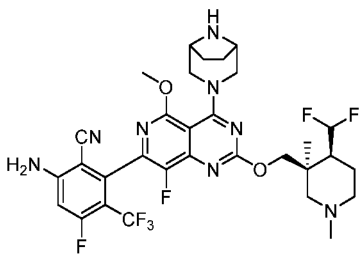 | 2-(4-((1R,5S)-3,8-diazabicyclo[3.2.1]octan-3-yl)-2-(((3S,4S)-4-(difluoromethyl)-1,3-dimethylpiperidin-3-yl)methoxy)-8-fluoro-5-methoxypyrido [4,3-d]pyrimidin-7-yl)-6-amino-4-fluoro-3-(trifluoromethyl) benzonitrile | LCMS (m/z): 683.3 (M + H). |
| 176 | 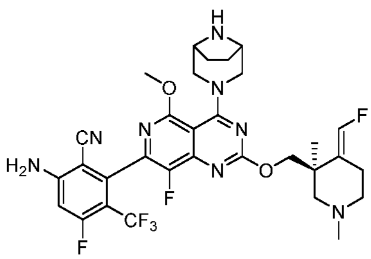 | 2-(4-((1R,5S)-3,8-diazabicyclo[3.2.1]octan-3-yl)-8-fluoro-2-(((S,E)-4-(fluoromethylene)-1,3-dimethylpiperidin-3-yl)methoxy)-5-methoxypyrido [4,3-d]pyrimidin-7-yl)-6-amino-4-fluoro-3-(trifluoromethyl) benzonitrile | LCMS (m/z): 663.3 (M + H). |
| 177 | 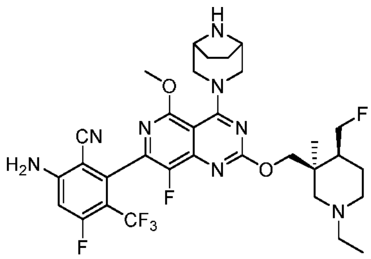 | 2-(4-((1R,5S)-3,8-diazabicyclo[3.2.1]octan-3-yl)-2-(((3S,4S)-1-ethyl-4-(fluoromethyl)-3-methylpiperidin-3-yl)methoxy)-8-fluoro-5-methoxypyrido[4,3-d]pyrimidin-7-yl)-6-amino-4-fluoro-3-(trifluoromethyl) benzonitrile | LCMS (m/z): 679.3 (M + H). |
| 178 | 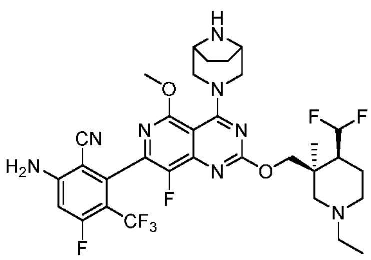 | 2-(4-((1*R*,5*S*)-3,8-diazabicyclo[3.2.1]octan-3-yl)-2-(((3*S*,4*S*)-4-(difluoromethyl)-1-ethyl-3-methylpiperidin-3-yl)methoxy)-8-fluoro-5-methoxypyrido[4,3-d]pyrimidin-7-yl)-6-amino-4-fluoro-3-(trifluoromethyl) benzonitrile | LCMS (m/z): 697.3 (M + H). |

(continued)

| Ex. | Structure | Name | Characterization data |
|---|---|---|---|
| 179 | 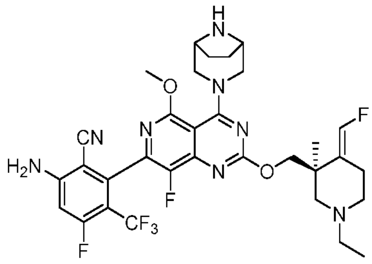 | 2-(4-((1R,5S)-3,8-diazabicyclo[3.2.1]octan-3-yl)-2-(((S,E)-1-ethyl-4-(fluoromethylene)-3-methylpiperidin-3-yl)methoxy)-8-fluoro-5-methoxypyrido[4,3-d]pyrimidin-7-yl)-6-amino-4-fluoro-3-(trifluoromethyl) benzonitrile | LCMS (m/z): 677.3 (M + H). |
| 180 | 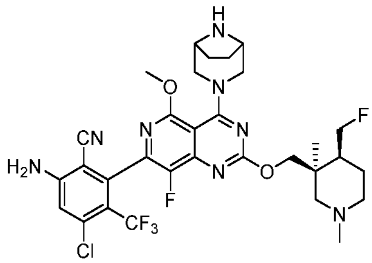 | 2-(4-((1R,5S)-3,8-diazabicyclo[3.2.1]octan-3-yl)-8-fluoro-2-(((3S,4S)-4-(fluoromethyl)-1,3-dimethylpiperidin-3-yl)methoxy)-5-methoxypyrido[4,3-d]pyrimidin-7-yl)-6-amino-4-chloro-3-(trifluoromethyl) benzonitrile | LCMS (m/z): 681.2 (M + H). |
| 181 | 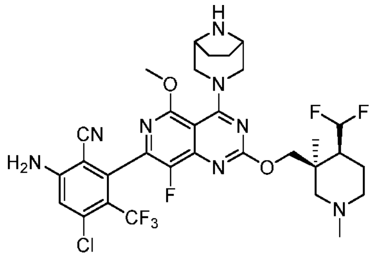 | 2-(4-((1R,5S)-3,8-diazabicyclo[3.2.1]octan-3-yl)-2-(((3S,4S)-4-(difluoromethyl)-1,3-dimethylpiperidin-3-yl)methoxy)-8-fluoro-5-methoxypyrido[4,3-d]pyrimidin-7-yl)-6-amino-4-chloro-3-(trifluoromethyl) benzonitrile | LCMS (m/z): 699.2 (M + H). |
| 182 | 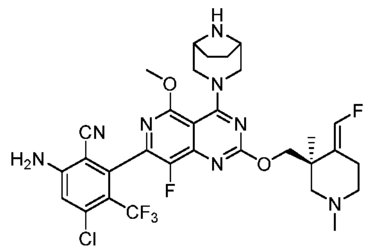 | 2-(4-((1R,5S)-3,8-diazabicyclo[3.2.1]octan-3-yl)-8-fluoro-2-(((S,E)-4-(fluoromethylene)-1,3-dimethylpiperidin-3-yl)methoxy)-5-methoxypyrido[4,3-d]pyrimidin-7-yl)-6-amino-4-chloro-3-(trifluoromethyl) benzonitrile | LCMS (m/z): 679.2 (M + H). |
| 183 | 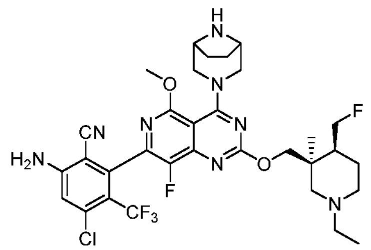 | 2-(4-((1R,5S)-3,8-diazabicyclo[3.2.1]octan-3-yl)-2-(((3S,4S)-1-ethyl-4-(fluoromethyl)-3-methylpiperidin-3-yl)methoxy)-8-fluoro-5-methoxypyrido[4,3-d]pyrimidin-7-yl)-6-amino-4-chloro-3-(trifluoromethyl) benzonitrile | LCMS (m/z): 695.3 (M + H) |
| 184 | 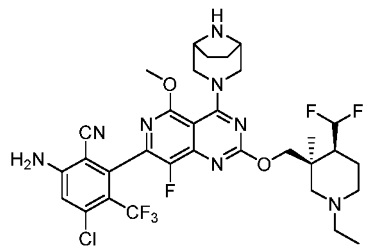 | 2-(4-((1R,5S)-3,8-diazabicyclo[3.2.1]octan-3-yl)-2-(((3S,4S)-4-(difluoromethyl)-1-ethyl-3-methylpiperidin-3-yl)methoxy)-8-fluoro-5-methoxypyrido[4,3-d]pyrimidin-7-yl)-6-amino-4-chloro-3-(trifluoromethyl) benzonitrile | LCMS (m/z): 713.3 (M + H). |

(continued)

| Ex. | Structure | Name | Characterization data |
|---|---|---|---|
| 185 | 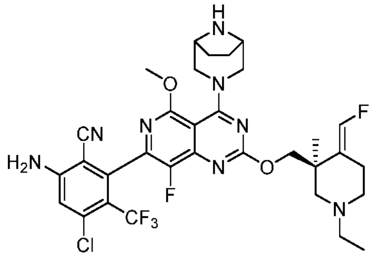 | 2-(4-((1R,5S)-3,8-diazabicyclo[3.2.1]octan-3-yl)-2-(((S,E)-1-ethyl-4-(fluoromethylene)-3-methylpiperidin-3-yl)methoxy)-8-fluoro-5-methoxypyrido[4,3-d]pyrimidin-7-yl)-6-amino-4-chloro-3-(trifluoromethyl) benzonitrile | LCMS (m/z): 693.2 (M + H). |
| 186 | 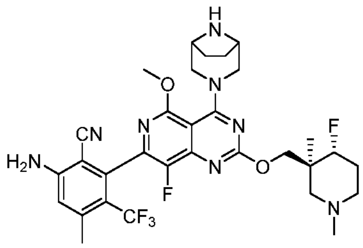 | 2-(4-((1R,5S)-3,8-diazabicyclo[3.2.1]octan-3-yl)-8-fluoro-2-(((3S,4R)-4-fluoro-1,3-dimethylpiperidin-3-yl)methoxy)-5-methoxypyrido[4,3-d] pyrimidin-7-yl)-6-amino-4-methyl-3-(trifluoromethyl) benzonitrile | LCMS (m/z): 647.3 (M + H). |
| 187 | 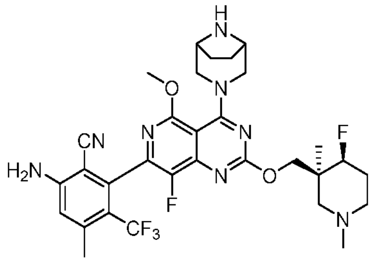 | 2-(4-((1R,5S)-3,8-diazabicyclo[3.2.1]octan-3-yl)-8-fluoro-2-(((3S,4S)-4-fluoro-1,3-dimethylpiperidin-3-yl)methoxy)-5-methoxypyrido[4,3-d] pyrimidin-7-yl)-6-amino-4-methyl-3-(trifluoromethyl) benzonitrile | LCMS (m/z): 647.3 (M + H). |
| 188 | 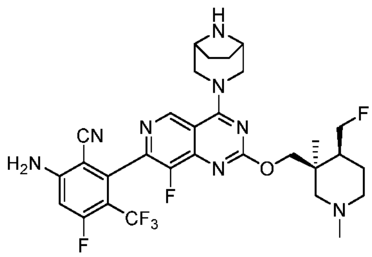 | 2-(4-((1R,5S)-3,8-diazabicyclo[3.2.1]octan-3-yl)-8-fluoro-2-(((3S,4S)-4-(fluoromethyl)-1,3-dimethylpiperidin-3-yl)methoxy)pyrido[4,3-d]pyrimidin-7-yl)-6-amino-4-fluoro-3-(trifluoromethyl) benzonitrile | LCMS (m/z): 635.3 (M + H). |
| 189 | 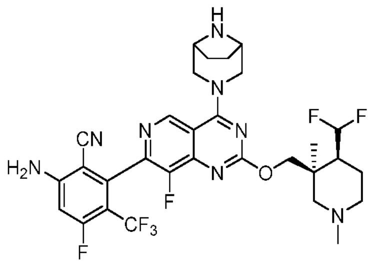 | 2-(4-((1R,5S)-3,8-diazabicyclo[3.2.1]octan-3-yl)-2-(((3S,4S)-4-(difluoromethyl)-1,3-dimethylpiperidin-3-yl)methoxy)-8-fluoropyridino[4,3-d]pyrimidin-7-yl)-6-amino-4-fluoro-3-(trifluoromethyl) benzonitrile | LCMS (m/z): 653.3 (M + H). |

The compounds listed in the following table can be prepared by referring to the above synthesis method or appropriate variants:

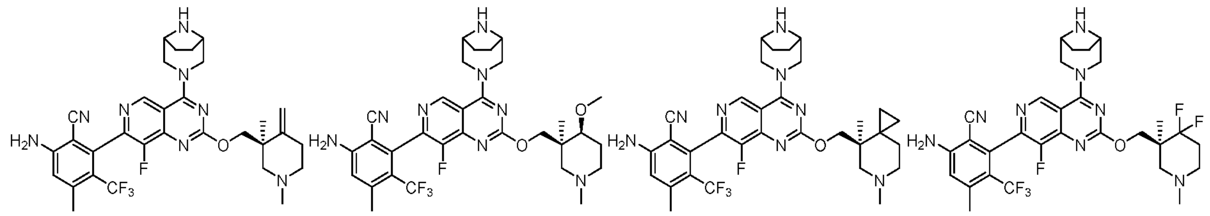

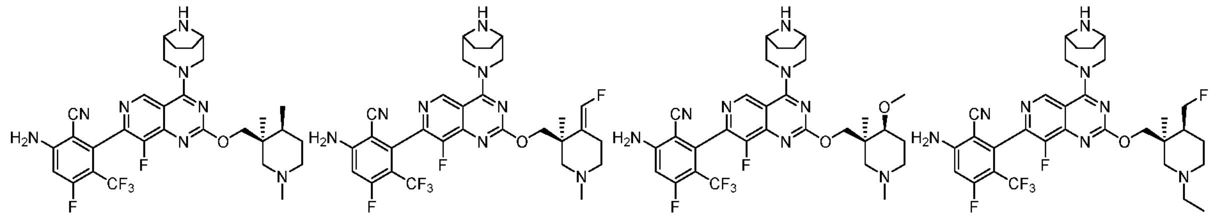

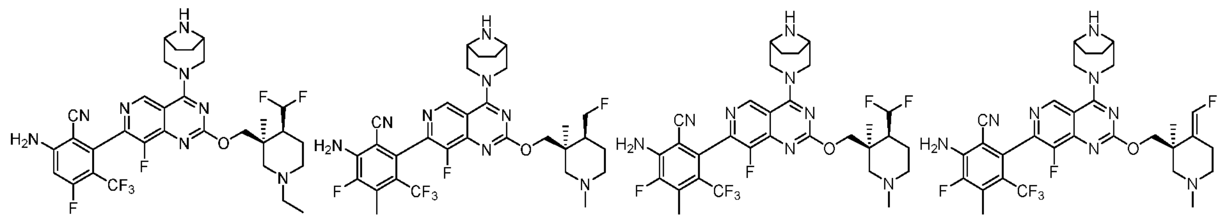

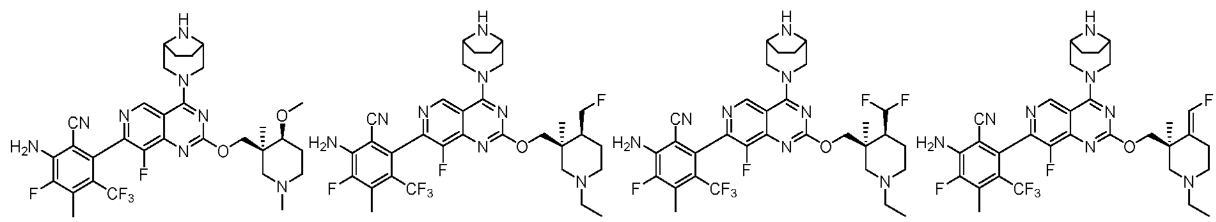

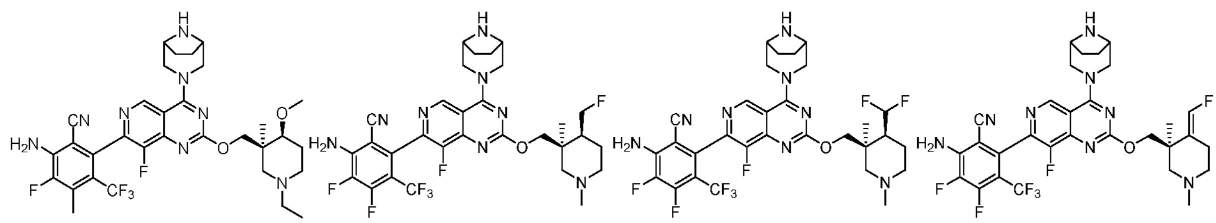

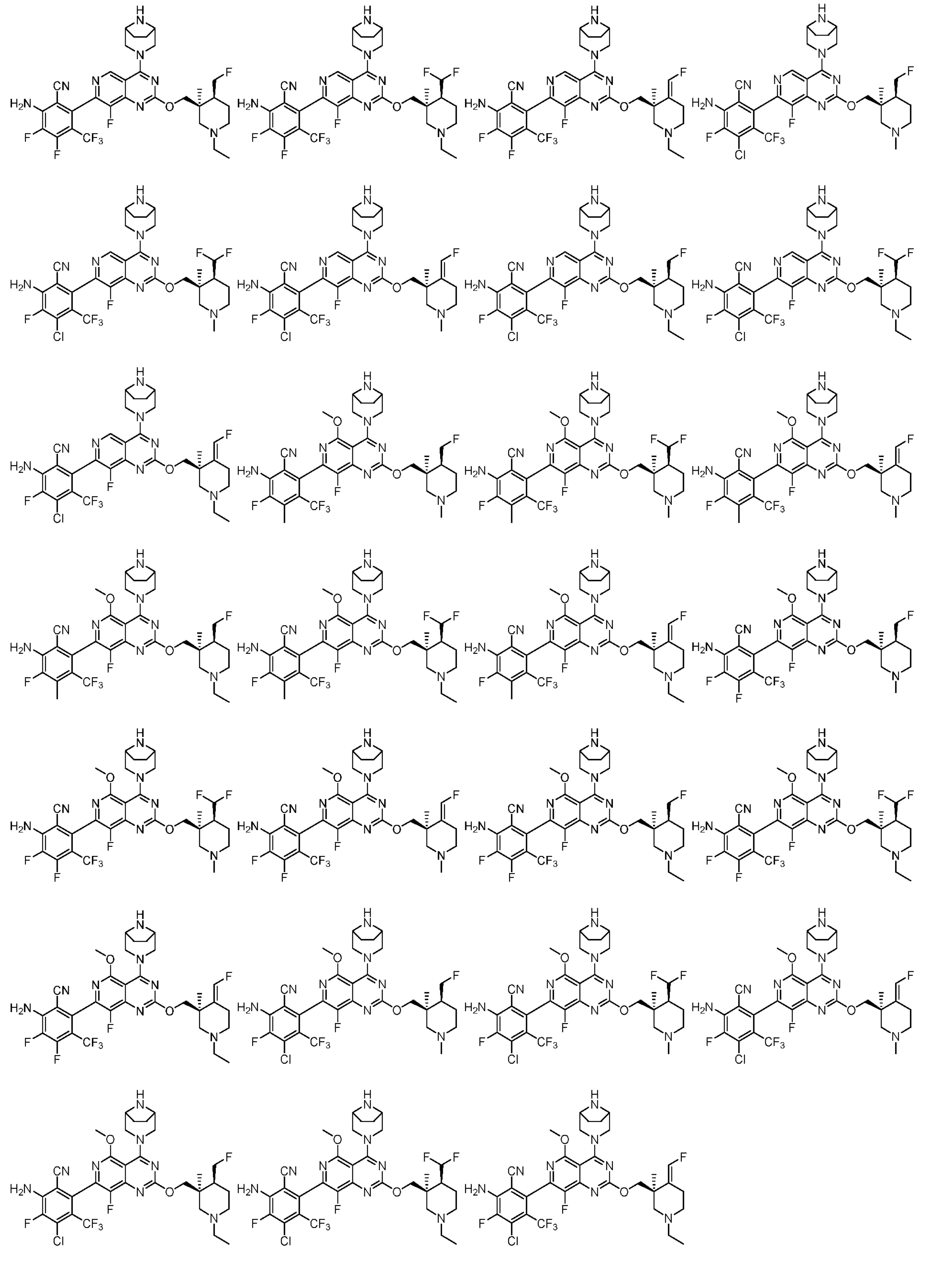

**Example 190**

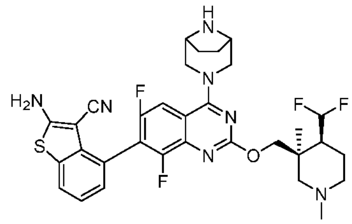

4-(4-((1R,5S)-3,8-diazabicyclo[3.2.1]octan-3-yl)-2-(((3S,4S)-4-(difluoromethyl)-1,3-dimethylpiperidin-3-yl)methoxy)-6,8-difluoroquinazolin-7-yl)-2-aminobenzo[b]thiophene-3-nitrile

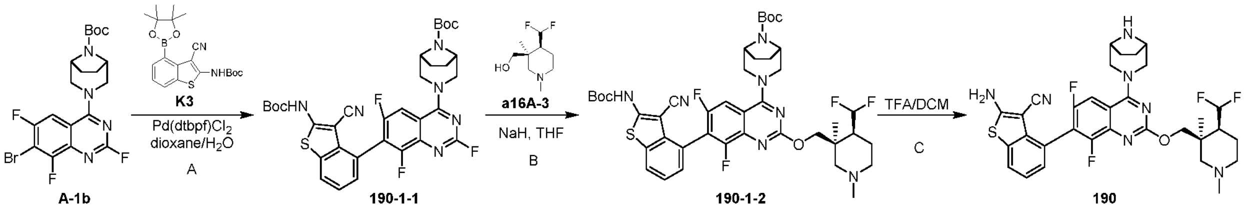


A-1b 190-1-1 190-1-2 190

**Step A:** tert-butyl (1*R*,5*S*)-3-(7-(2-((*tert*-butoxycarbonyl)amino)-3-cyanobenzo[*b*]thiophen-4-yl)-2,6,8-trifluoroquinazolin-4-yl)-3,8-diazabicyclo[3.2.1]octane-8-carboxylate

*tert-butyl* (3-cyano-4-(4,4,5,5-tetramethyl-1,3,2-dioxaborolan-2-yl)benzothiophen-2-yl) carbamate (500 mg, 1.45 mmol), tert-butyl (1R,5S)-3-(7-bromo-2, 6,8-trifluoroquinazolin-4-yl)-3,8-diazabicyclo[3.2.1]octane-8-carboxylate (455 mg, 0.96 mmol), potassium phosphate (611 mg, 2.9 mmol),[1,1'-bis(di-tert-butylphosphino) ferrocene]palladium dichloride (62 mg, 0.1 mmol) were successively added to a 50 mL round-bottom flask. Then, 1,4-dioxane (4 mL) and water (4 mL) were added to the reaction flask. The reaction system was degassed with nitrogen and stirred at 105 °C for 3 h. After the reaction was completed (monitored by LCMS), the reaction was filtered. The filtrate was concentrated, and the resulting residue was purified by FCC ($SiO_2$, THF/PE = 0-20%) to afford *tert-butyl* (1R,5S)-3-(7-(2-((tert-butoxycarbonyl) amino)-3-cyanobenzo[b]thiophen-4-yl)-2,6,8-trifluoroquinazolin-4-yl)-3,8-diazabicyclo[3.2.1]octane-8-carboxylate (170 mg) as a pale-yellow oil. m/z:$[M + H]^+$ 667.3.

**Step B:** tert-butyl (1*R*,5*S*)-3-(7-(2-(((*tert*-butoxycarbonyl)amino)-3-cyanobenzo[*b*]thiophen-4-yl)-2-(((3S,4S)-4-(difluoromethyl)-1,3-dimethylpiperidin-3-yl)methoxy)-6,8-difluoroquinazolin-4-yl)-3, 8-diazabicyclo[3 .2.1]octane-8-carboxylate

At room temperature, sodium hydride (22 mg, 0.54 mmol, 60% dispersed in mineral oil) was added to a solution of (3S,4S)-4-difluoromethyl-1,3-dimethylpiperidin-3-methanol (52 mg, 0.27 mmol) in THF (10 mL). After the addition, the mixture was stirred at room temperature for 0.5 h. Then tert-butyl (1R,5S)-3-(7-(2-((tert-butoxycarbonyl)amino)-3-cyanobenzo[b]thiophen-4-yl)-2, 6,8-trifluoroquinazolin-4-yl)-3,8-diazabicyclo[3.2.1]octane-8-carboxylate (120 mg, 0.18 mmol) was added. After the addition, the mixture was stirred at room temperature for 3 h. After the reaction was completed (monitored by LCMS), the reaction was quenched with saturated aqueous ammonium chloride (70 mL), and the resulting mixture was extracted with ethyl acetate (30 mL × 3). The organic phase was washed with brine (70 mL), dried over anhydrous $Na_2SO_4$, filtered and concentrated to give tert-butyl (1*R*,5*S*)-3-(7-(2-(((tert-butoxycarbonyl)amino)-3-cyanobenzo[b]thiophen-4-yl)-2-(((3S,4S)-4-(difluoromethyl)-1,3-dimethylpiperidin-3-yl)methoxy)-6,8-difluoroquinazolin-4-yl)-3,8-diazabicyclo[3.2.1]octane-8-carboxylate (120 mg, yield 79%) as a brown oil. LC-MS (m/z): 840.3 (M + H).

**Step C:** 4-(4-((1R,5S)-3,8-diazabicyclo[3.2.1]octan-3-yl)-2-(((3S,4S)-4-(difluoromethyl)-1,3-dimethylpiperidin-3-yl) methoxy)-6,8-difluoroquinazolin-7-yl)-2-aminobenzo[b]thiophene-3-nitrile

At room temperature, TFA (1 mL) was added to a solution of *tert*-butyl (1*R*,5*S*)-3-(7-(2-*(((tert*-butoxycarbonyl)amino)-3-cyanobenzo[b]thiophen-4-yl)-2-(((3S,4S)-4-(difluoromethyl)-1,3-dimethylpiperidin-3-yl)methoxy)-6,8-difluoroquinazolin-4-yl)-3,8-diazabicyclo[3.2.1]octane-8-carboxylate (60 mg, 0.071 mmol) in DCM (2 mL), and the reaction was stirred at room temperature after addition for 1 h. After the reaction was completed (monitored by LCMS), the reaction was concentrated, quenched by adding saturated aqueous sodium bicarbonate (50 mL), and extracted with ethyl acetate (30 mL × 3). The combined organic phase was washed with brine (100 mL), dried over anhydrous $Na_2SO_4$, filtered and concentrated. The resulting crude product was purified by pre-HPLC (C18, CAN/(10 mmol $NH_4HCO_3/H_2O$) = 30-50%) to obtained 4-(4-((1R,5S)-3,8-diazabicyclo[3.2.1]octan-3-yl)-2-(((3S,4S)-4-(difluoromethyl)-1,3-dimethylpiperidin-3-yl)methoxy)-6,8-difluoroquinazolin-7-yl)-2-aminobenzo[b]thiophene-3-nitrile (5.7 mg, yield 15.5%) as a white solid. $^1H$ NMR (400 MHz, DMSO-$d_6$) δ 7.86 (s, 2H), 7.83 - 7.79 (m, 1H), 7.59 (d, *J*= 9.6 Hz, 1H), 7.30 - 7.20 (m, 2H), 6.51 - 6.11 (m, 1H), 4.49 - 4.40 (m, 1H), 4.40 - 4.34 (m, 1H), 4.34 - 4.25 (m, 1H), 4.25 - 4.16 (m, 1H), 3.55 - 3.52 (m, 1H), 3.51 - 3.46 (m, 3H), 2.91 - 2.75 (m, 2H), 2.11 (s, 3H), 1.87 - 1.56 (m, 9H), 1.11 (s, 3H). $^{19}F$ NMR (376 MHz, DMSO-$d_6$) δ-115.20,-115.94,-118.47,-119.19,-124.26. LCMS (m/z): 640.3 (M + H).

## Intermediate 190-1-1A and 190-1-1B

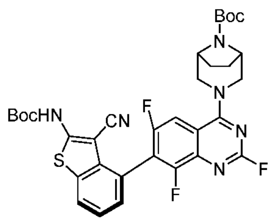

190-1-1A或190-1-1B

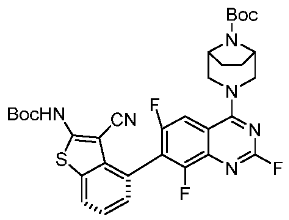

190-1-1B或190-1-1A

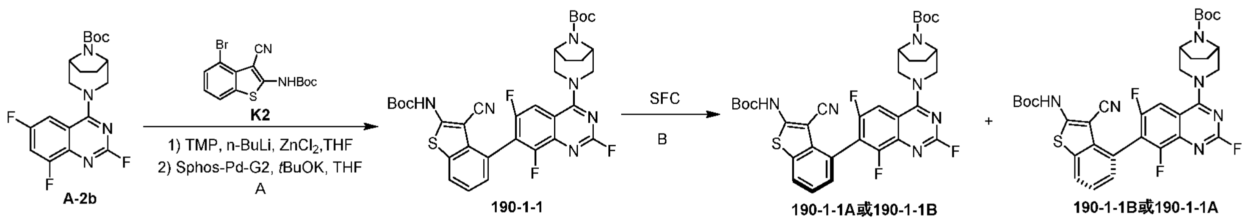


**Step A:** *tert*-butyl (1*R*,5*S*)-3-(7-(2-((*tert*-butoxycarbonyl)amino)-3-cyanobenzo[*b*]thiophen-4-yl)-2,6,8-trifluoroquinazolin-4-yl)-3,8-diazabicyclo[3.2.1]octane-8-carboxylate

Reaction system (A): Under a dry ice-acetonitrile bath, n-butyllithium (7.6 mL, 12.2 mmol, 1.6 M in THF) was added to a solution of 2,2,6,6-tetramethylpiperidine (1.72 g, 12.2 mmol) in THF (6 mL). After the addition, the mixture was continuously stirred at the same temperature for 0.5 h and then transferred to an ice-salt bath. $ZnCl_2$ (12.2 mL, 12.2 mmol, 1 M in THF) was added to the reaction system. After the addition, the mixture was stirred at the same temperature for 0.5 h. Then, at the same temperature, a solution of tert-butyl (1*R*,5*S*)-3-(2,6,8-trifluoroquinazolin-4-yl)-3,8-diazabicyclo[3.2.1]octane-8-carboxylate (3.0 g, 7.6 mmol) in THF (12 mL) was added dropwise to the above reaction system. After the addition, the system was heated to 65 °C and stirred for 0.5 h to obtain Reaction System (A).

Reaction system (B): At room temperature, Sphos-Pd-G2 (548 mg, 0.76 mmol) was added to a solution of *tert-butyl* (4-bromo-3-cyanobenzo[b]thiophen-2-yl)carbamate (2.96 g, 8.40 mmol) in THF (12 mL). The system was purged with nitrogen three times and then stirred at room temperature for 0.5 h to obtain Reaction System (B).

At room temperature, the reaction system (A) was added into the reaction system (B), and the resulting mixture was heated to 65 °C and stirred for 16 h. After the reaction was completed (monitored by LCMS), the reaction was quenched with saturated aqueous ammonium chloride (70 mL), and the resulting mixture was extracted with ethyl acetate (50 mL × 3). The combined organic phases were washed with saturated aqueous NaCl solution (70 mL), dried over anhydrous $Na_2SO_4$, filtered and concentrated. The crude product was purified by FCC ($SiO_2$, THF/PE = 0 - 40%) to give *tert-butyl* (1R,5S)-3-(7-(2-((tert-butoxycarbonyl)amino)-3-cyanobenzo[b]thiophen-4-yl)-2,6,8-trifluoroquinazolin-4-yl)-3,8-diazabicyclo[3.2.1]octane-8-carboxylate (3.8 g, yield 75%) as a yellow solid. LCMS (m/z): 667.2 (M + H).

**Step B: Intermediates 190-1-1A and 190-1-1B**

The resolution of *tert-butyl* (1R,5S)-3-(7-(2-((tert-butoxycarbonyl)amino)-3-cyanobenzo[b]thiophen-4-yl)-2,6,8-trifluoroquinazolin-4-yl)-3,8-diazabicyclo[3.2.1]octane-8-carboxylate (compound 190-1-1, 2 .8 g) was carried out by SFC (Waters SFC 150, Separation column: DAICEL CHIRALPAK ® OZ, 250 * 25 mm, 10 μm; Mobile phase: $CO_2$/MeOH =

60/40; flow rate: 120 mL/min). The first eluted isomer 1 was intermediate 190-1-1A (1.28g, with a shorter retention time). Chiral analytical method SFC-6, Rt = 1.757 min. LCMS (m/z): 667.2 (M + H). The subsequently eluted isomer 2 was Intermediate 190-1-1B (1.18 g, with a longer retention time). Chiral analytical method SFC-6, Rt = 2.716 min. LCMS (m/z): 667.2 (M + H).

**The following compounds were prepared using Intermediates 190-1-1, 190-1-1A or 190-1-1B, or referring to the above synthesis scheme.** It should be noted that for the following Isomer-1 or -2, the naming was based on the principle that the one with a shorter retention time in the chiral resolution of the final compound was designated as -1, and the one with a longer retention time was designated as -2. Or, when Intermediate 190-1-1A was used as the intermediate for synthesis, the resulting isomer was Isomer-1, and when Intermediate 190-1-1B was used as the intermediate for synthesis, the resulting isomer was Isomer-2. When there is a conflict in the two naming methods, the naming after preparation using the latter chiral pure intermediate prevails.

| Ex. | Structure | Name | Characterization data |
|---|---|---|---|
| 190-1 | 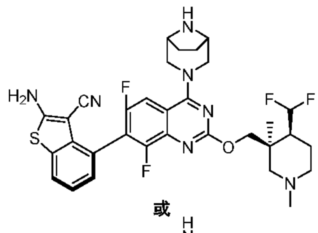 或 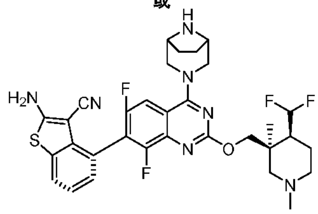 | (*Ra* or *Sa*)-4-(4-((1*R*,5*S*)-3,8-diazabicyclo[3.2.1]octan-3-yl)-2-(((3S,4S)-4-(difluoromethyl)-1,3-dimethylpiperidin-3-yl)methoxy)-6,8-difluoroquinazolin-7-yl)-2-aminobenzo[b]thiophene-3-nitrile | LCMS (m/z): 640.3 (M + H). |
| 190-2 | 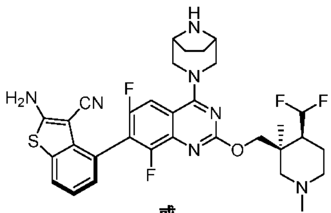 或 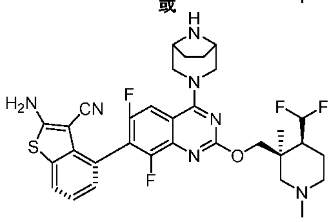 | (*Ra* or *Sa*)-4-(4-((1*R*,5*S*)-3,8-diazabicyclo[3.2.1]octan-3-yl)-2-(((3S,4S)-4-(difluoromethyl)-1,3-dimethylpiperidin-3-yl)methoxy)-6,8-difluoroquinazolin-7-yl)-2-aminobenzo[b]thiophene-3-nitrile | LCMS (m/z): 640.3 (M + H). |
| 191 | 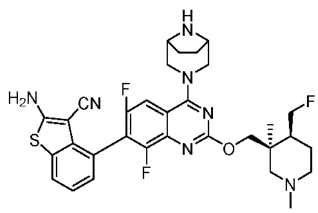 | 4-(4-((1R,5S)-3,8-diazabicyclo[3.2.1]octan-3-yl)-6,8-difluoro-2-(((3S,4S)-4-(fluoromethyl)-1,3-dimethylpiperidin-3-yl)methoxy)quinazolin-7-yl)-2-aminobenzo[b]thiophene-3-nitrile | |

(continued)

| Ex. | Structure | Name | Characterization data |
|---|---|---|---|
| 191-1 | 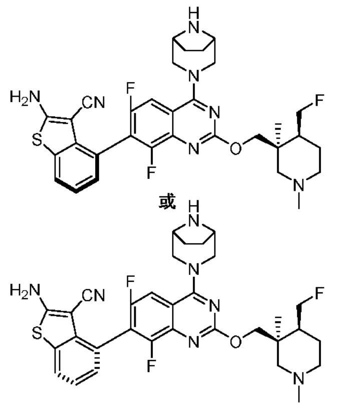 或 | *(Ra* or *Sa*)-4-(4-((1*R*,5*S*)-3,8-diazabicyclo[3.2.1]octan-3-yl)-6,8-difluoro-2-(((3S,4S)-4-(fluoromethyl)-1,3-Dimethylpiperidin-3-yl)methoxy)quinazolin-7-yl)-2-aminobenzo[b]thiophene-3-nitrile | LCMS (m/z): 622.3 (M + H). |
| 191-2 | 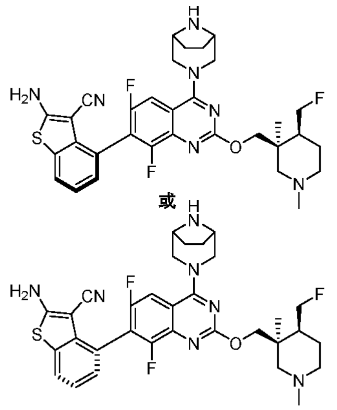 或 | *(Ra* or *Sa*)-4-(4-((1*R*,5*S*)-3,8-diazabicyclo[3.2.1]octan-3-yl)-6,8-difluoro-2-(((3S,4S)-4-(fluoromethyl)-1,3-Dimethylpiperidin-3-yl)methoxy)quinazolin-7-yl)-2-aminobenzo[b]thiophene-3-nitrile | LCMS (m/z): 622.3 (M + H). $^{1}$H NMR (400 MHz, DMSO-$d_6$) $\delta$ 7.87 (s, 2H), 7.84 - 7.79 (m, 1H), 7.60 (d, *J* = 10.3 Hz, 1H), 7.28 - 7.21 (m, 2H), 4.77 - 4.60 (m, 1H), 4.54 - 4.36 (m, 2H), 4.34 - 4.26 (m, 2H), 4.25 - 4.17 (m, 1H), 3.57 - 3.49 (m, 4H), 2.88 - 2.70 (m, 2H), 2.11 (s, 3H), 1.91 - 1.77 (m, 1H), 1.69 - 1.50 (m, 8H), 1.06 (s, 3H). $^{19}$F NMR (376 MHz, DMSO-$d_6$) $\delta$-119.12,-124.17,-218.82. |
| 192 | 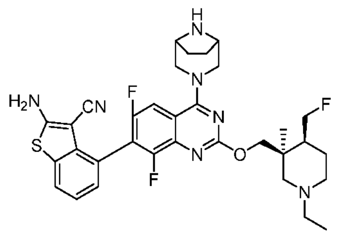 | 4-(4-((1*R*,5*S*)-3,8-diazabicyclo[3.2.1]octan-3-yl)-2-((((3*S*,4*S*)-1-ethyl-4-(fluoromethyl)-3-methylpiperidin-3-yl)methoxy)-6,8-difluoroquinazolin-7-yl)-2-aminobenzo[b]thiophene-3-nitrile | LCMS (m/z): 636.3 (M + H). |
| 192-1 | 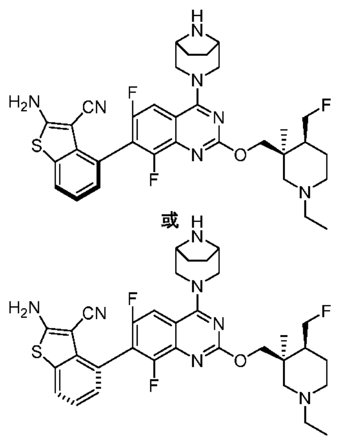 或 | *(Ra* or *Sa*)-4-(4-((1*R*,5*S*)-3,8-diazabicyclo[3.2.1]octan-3-yl)-2-((((3S,4S)-1-ethyl-4-(fluoromethyl)-3-methylpiperidin-3-yl)methoxy)-6,8-difluoroquinazolin-7-yl)-2-aminobenzo[b]thiophene-3-nitrile | LCMS (m/z): 636.3 (M + H). |

(continued)

| Ex. | Structure | Name | Characterization data |
|---|---|---|---|
| 192-2 | 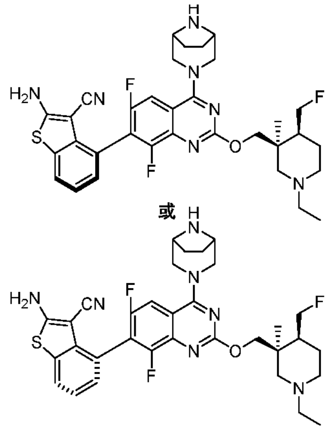 或 | *(Ra* or *Sa*)-4-(4-((1*R*,5*S*)-3,8-diazabicyclo[3.2.1]octan-3-yl)-2-((((3S,4S)-1-ethyl-4-(fluoromethyl)-3-methylpiperidin-3-yl) methoxy)-6,8-difluoroquinazolin-7-yl)-2-aminobenzo[b]thiophene-3-nitrile | LCMS (m/z): 636.3 (M + H). |
| 193 | 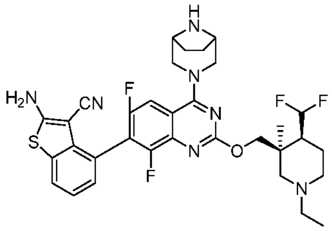 | 4-(4-((1R,5S)-3,8-diazabicyclo[3.2.1]octan-3-yl)-2-(((3S,4S)-4-(difluoromethyl)-1-ethyl-3-methylpiperidin-3-yl) methoxy)-6,8-difluoroquinazolin-7-yl)-2-aminobenzo[b]thiophene-3-nitrile | LCMS (m/z): 654.3 (M + H). |
| 193-1 | 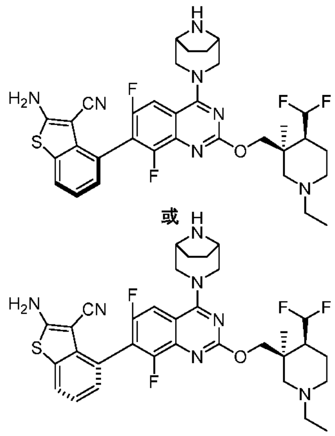 或 | *(Ra* or *Sa*)-4-(4-((1*R*,5*S*)-3,8-diazabicyclo[3.2.1]octan-3-yl)-2-(((3S,4S)-4-(difluoromethyl)-1-ethyl-3-methylpiperidin-3-yl) methoxy)-6,8-difluoroquinazolin-7-yl)-2-aminobenzo[b]thiophene-3-nitrile | LCMS (m/z): 654.3 (M + H). |
| 193-2 | 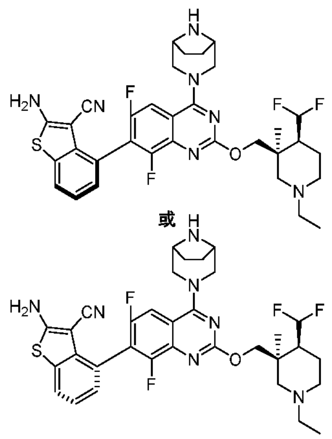 或 | *(Ra* or *Sa*)-4-(4-((1*R*,5*S*)-3,8-diazabicyclo[3.2.1]octan-3-yl)-2-(((3S,4S)-4-(difluoromethyl)-1-ethyl-3-methylpiperidin-3-yl) methoxy)-6,8-difluoroquinazolin-7-yl)-2-aminobenzo[b]thiophene-3-nitrile | LCMS (m/z): 654.3 (M + H). $^1H$ NMR (400 MHz, DMSO-$d_6$) δ 7.86 (s, 2H), 7.83 - 7.79 (m, 1H), 7.59 (d, *J*= 9.9 Hz, 1H), 7.34 - 7.15 (m, 2H), 6.54 - 6.11 (m, 1H), 4.50 - 4.42 (m, 1H), 4.42 - 4.27 (m, 2H), 4.26 - 4.13 (m, 1H), 3.62 - 3.41 (m, 4H), 2.98 - 2.82 (m, 2H), 2.33 - 2.15 (m, 2H), 1.95 - 1.82 (m, 1H), 1.78 - 1.55 (m, 8H), 1.12 (s, 3H), 0.91 (t, *J* = 7.1 Hz, 3H). $^{19}F$ NMR (376 MHz, DMSO-$d_6$) δ-115.35, -116.10, -118.55, -119.17, -119.29, 124.18. |

(continued)

| Ex. | Structure | Name | Characterization data |
|---|---|---|---|
| 194 | 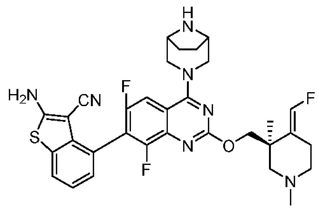 | 4-(4-(1R,5S)-3,8-diazabicyclo[3.2.1]octan-3-yl)-6,8-difluoro-2-(S,E)-4-(fluoromethylene)-1,3-dimethylpiperidin-3-yl)methoxy) quinazolin-7-yl)-2-aminobenzo[b]thiophene-3-nitrile | LCMS (m/z): 620.2 (M + H). $^1$H NMR (400 MHz, Methanol-$d_4$) δ 7.68 (dd, *J* = 5.9, 3.2 Hz, 1H), 7.54 (dd, *J*= 10.1, 1.8 Hz, 1H), 7.30 - 7.19 (m, 2H), 6.66 (dd, *J* = 86.4, 1.7 Hz, 1H), 4.57 - 4.46 (m, 2H), 4.46 - 4.37 (m, 2H), 3.68 - 3.51 (m, 4H), 2.88 (d, *J* = 11.6 Hz, 1H), 2.80 - 2.59 (m, 2H), 2.39 - 2.22 (m, 4H), 2.11 - 1.95 (m, 2H), 1.85 (s, 4H), 1.21 (s, 3H). $^{19}$F NMR (376 MHz, Methanol-$d_4$) δ-119.86 (d, *J* = 6.0 Hz), -123.90-126.65 (m), -139.68. |
| 194-1 | 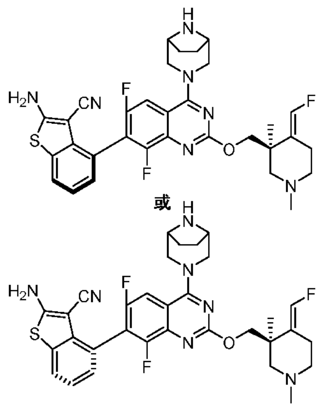 或 | (*Ra* or *Sa*)-4-(4-(1*R*,5*S*)-3,8-diazabicyclo[3.2.1]octan-3-yl)-6,8-difluoro-2-(S,E)-4-(fluoromethylene)-1,3-Dimethylpiperidin-3-yl)methoxy) quinazolin-7-yl)-2-aminobenzo[b]thiophene-3-nitrile | LCMS (m/z): 620.2 (M + H). |
| 194-2 | 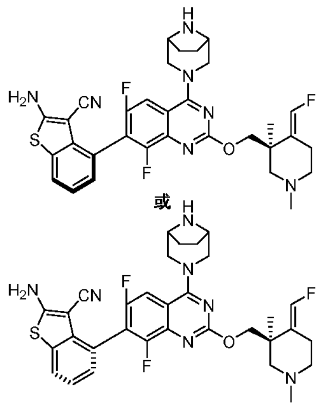 或 | (*Ra* or *Sa*)-4-(4-(1*R*,5*S*)-3,8-diazabicyclo[3.2.1]octan-3-yl)-6,8-difluoro-2-(S,E)-4-(fluoromethylene)-1,3-Dimethylpiperidin-3-yl)methoxy) quinazolin-7-yl)-2-aminobenzo[b]thiophene-3-nitrile | LCMS (m/z): 620.2 (M + H). $^1$H NMR (400 MHz, DMSO-$d_6$) δ 7.87 (brs, 2H), 7.82 (dd, *J*= 6.5, 2.6 Hz, 1H), 7.61 (d, *J* = 10.7 Hz, 1H), 7.30 - 7.18 (m, 2H), 6.82 (s, 0.5H), 6.61 (s, 0.5H), 4.58 - 4.50 (m, 1H), 4.35 - 4.29 (m, 1H), 4.28 - 4.23 (m, 1H), 4.23 - 4.16 (m, 1H), 3.66 - 3.52 (m, 3H), 3.48 - 3.42 (m, 1H), 2.73 - 2.64 (m, 2H), 2.25 - 2.09 (m, 4H), 2.04 - 1.79 (m, 3H), 1.73 - 1.59 (m, 4H), 1.11 (s, 3H). $^{19}$F NMR (376 MHz, DMSO-$d_6$) δ -118.91, - 124.13, -138.87. |
| 195 | 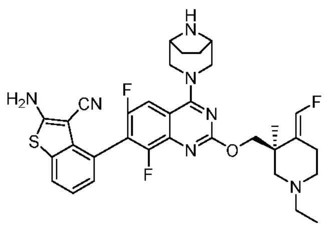 | 4-(4-((1R,5S)-3,8-diazabicyclo[3.2.1]octan-3-yl)-2-(((S,E)-1-ethyl-4-(fluoromethylene)-3-methylpiperidin-3-yl)methoxy)-6,8-difluoroquinazolin-7-yl)-2-aminobenzo[b]thiophene-3-nitrile | LCMS (m/z): 634.3 (M + H). |

(continued)

| Ex. | Structure | Name | Characterization data |
|---|---|---|---|
| 195-1 | 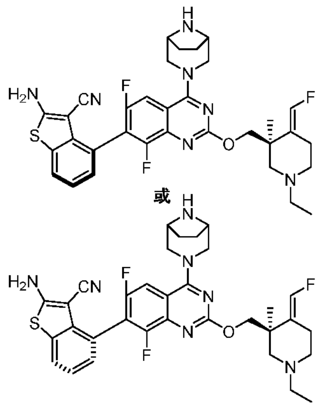 或 | *(Ra* or *Sa*)-4-(4-((1*R*,5*S*)-3,8-diazabicyclo[3.2.1]octan-3-yl)-2-(((S,E)-1-ethyl-4-(fluoromethylene)-3-methylpiperidin-3-yl)methoxy)-6,8-difluoroquinazolin-7-yl)-2-aminobenzo[b] thiophene-3-nitrile | LCMS (m/z): 634.3 (M + H). |
| 195-2 | 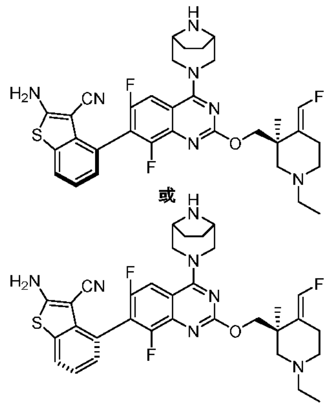 或 | *(Ra* or *Sa*)-4-(4-((1*R*,5*S*)-3,8-diazabicyclo[3.2.1]octan-3-yl)-2-(((S,E)-1-ethyl-4-(fluoromethylene)-3-methylpiperidin-3-yl)methoxy)-6,8-difluoroquinazolin-7-yl)-2-aminobenzo[b] thiophene-3-nitrile | LCMS (m/z): 634.3 (M + H). |
| 196 | 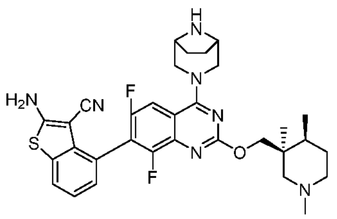 | 4-(4-(1*R*,5*S*)-3,8-diazabicyclo[3.2.1]octan-3-yl)-6,8-difluoro-2-(3*S*,4*S*)-1,3,4-trimethylpiperidin-3-yl) methoxy) quinazolin-7-yl)-2-aminobenzo[b] thiophene-3-nitrile | LCMS (m/z): 604.3 (M + H). |
| 197 | 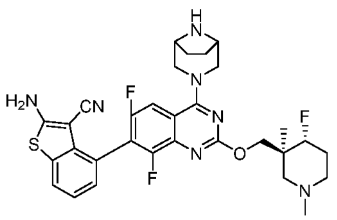 | 4-(4-(1*R*,5*S*)-3,8-diazabicyclo[3.2.1]octan-3-yl)-6,8-difluoro-2-(3S,4R)-4-fluoro-1,3-dimethylpiperidin-3-yl)methoxy) quinazolin-7-yl)-2-aminobenzo[b]thiophene-3-nitrile | LCMS (m/z): 608.2 (M + H). |
| 198 | 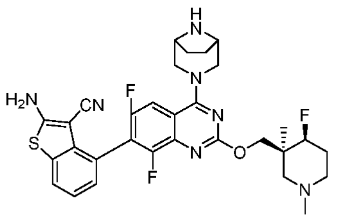 | 4-(4-(1*R*,5*S*)-3,8-diazabicyclo[3.2.1]octan-3-yl)-6,8-difluoro-2-(3S,4S)-4-fluoro-1,3-dimethylpiperidin-3-yl)methoxy) quinazolin-7-yl)-2-aminobenzo[b]thiophene-3-nitrile | LCMS (m/z): 608.2 (M + H). |

## Intermediates 199-1-1, 199-1-1A, and 199-1-1B

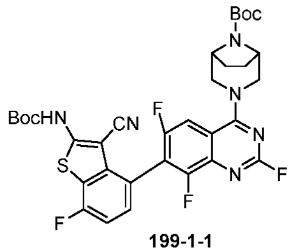
199-1-1

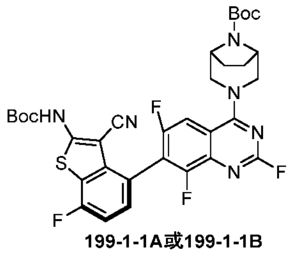
199-1-1A或199-1-1B

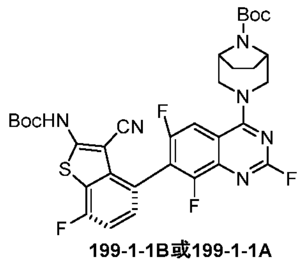
199-1-1B或199-1-1A

The synthesis of Intermediate **199-1-1** was carried according to the synthesis of Intermediate **190-1-1** using *tert-butyl* (3-cyano-4-(5,5-dimethyl-1,3,2-dioxaborolan-2-yl)-7-fluorobenzo[b]thiophen-2-yl) carbamate instead of *tert*-butyl (3-cyano-4-(4,4,5,5-tetramethyl-1,3,2-dioxaborolan-2-yl) benzothiophen-2-yl)carbamate.

Intermediate **199-1-1** (1.0g) was resolved by SFC (Waters SFC 150, separation column: DAICEL CHIRALPAK® OZ, 250 * 25 mm, 10μm; mobile phase: $CO_2$/MeOH (+ 0.1% 7.0 mol/L $NH_3$/MeOH) = 67/33; flow rate: 120 mL/min). The first eluted isomer 1 was Intermediate **199-1-1A** (0.5 g, with a shorter retention time). Chiral analytical method SFC-10, Rt = 1.552 min. LCMS (m/z): 683.2 (M + H). The subsequently eluted isomer 2 was Intermediate **199-1-1B** (0.5 g, with a longer retention time). Chiral analytical method SFC-10, Rt = 2.961 min. LCMS (m/z): 683.2 (M + H).

**The following compounds were prepared using Intermediates 199-1-1, 199-1-1A, or 199-1-1B and referring to the synthesis scheme of Examples 190, 190-1, and 190-2.** It should be noted that for the following Isomer-1 or -2, the naming was based on the principle that the one with a shorter retention time in the chiral resolution of the final compound was designated as -1, and the one with a longer retention time was designated as -2. Or, when Intermediate 199-1-1A was used as the intermediate for synthesis, the resulting isomer was Isomer-1, and when Intermediate 199-1-1B was used as the intermediate for synthesis, the resulting isomer was Isomer-2. When there is a conflict in the two naming methods, the naming after preparation using the latter chiral pure intermediate prevails.

| Ex. | Structural Formula | Name | Characterization data |
|---|---|---|---|
| 199 | 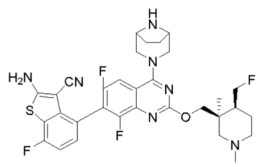 | 4-(4-(1*R*,5*S*)-3,8-diazabicyclo [3.2.1]octan-3-yl)-6,8-difluoro-2-(3*S*,4*S*)-4-(fluoromethyl)-1,3-dimethylpiperidin-3-yl)methoxy) quinazolin-7-yl)-2-amino-7-fluorobenzo[*b*]thiophene-3-nitrile | LCMS (m/z): 640.2 (M + H). |
| 200 | 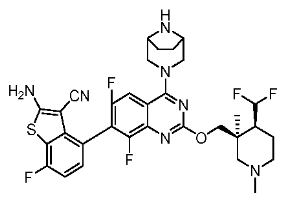 | 4-(4-((1*R*,5*S*)-3,8-diazabicyclo [3.2.1]octan-3-yl)-2-(((3*S*,4*S*)-4-(difluoromethyl)-1,3-dimethylpiperidin-3-yl) methoxy)-6,8-difluoroquinazolin-7-yl)-2-amino-7-fluorobenzo[b]thiophene-3-nitrile | LCMS (m/z): 658.2 (M + H). $^1$H NMR (400 MHz, DMSO-$d_6$) δ 8.15 (s, 2H), 7.61 (d, *J* = 10.0 Hz, 1H), 7.42 - 7.29 (m, 1H), 7.25 - 7.08 (m, 1H), 6.49 - 6.09 (m, 1H), 4.49 - 4.34 (m, 2H), 4.29 (d, *J* = 12.2 Hz, 1H), 4.20 (d, J = 12.1 Hz, 1H), 3.58 - 3.43 (m, 4H), 2.90 - 2.76 (m, 2H), 2.11 (s, 3H), 1.90 - 1.79 (m, 1H), 1.77 - 1.49 (m, 8H), 1.11 (s, 3H). $^{19}$F NMR (376 MHz, DMSO-$d_6$) δ - 73.42, -115.17, -115.23, -115.92, -115.97, - 116.06, -118.48, - 118.55, -119.23, - 119.30. |

(continued)

| Ex. | Structural Formula | Name | Characterization data |
|---|---|---|---|
| 200-1 | 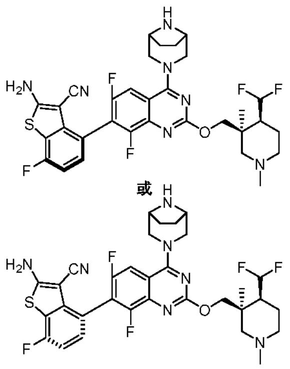 或 | *(Sa* or *Ra*)-4-(4-((1*R*,5*S*)-3,8-diazabicyclo[3.2.1]octan-3-yl)-2-(((3*S*,4*S*)-4-(difluoromethyl)-1,3-dimethylpiperidin-3-yl) methoxy)-6,8-difluoroquinazolin-7-yl)-2-amino-7-fluorobenzo[b]thiophene-3-nitrile | LCMS (m/z): 658.2 (M + H).<br>It can be made from Intermediate **199-1-1A.** |
| 200-2 | 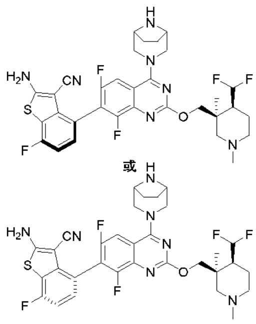 或 | *(Sa* or *Ra*)-4-(4-((1*R*,5*S*)-3,8-diazabicyclo[3.2.1]octan-3-yl)-2-(((3*S*,4*S*)-4-(difluoromethyl)-1,3-dimethylpiperidin-3-yl) methoxy)-6,8-difluoroquinazolin-7-yl)-2-amino-7-fluorobenzo[b]thiophene-3-nitrile | LCMS (m/z): 658.2 (M + H).<br>It can be prepared from Intermediate **199-1-1B.** |
| 201 | 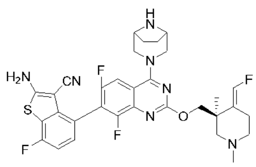 | 4-(4-(1R,5S)-3,8-diazabicyclo [3.2.1]octan-3-yl)-6,8-difluoro-2-(S,E)-4-(fluoromethylene)-1,3-dimethylpiperidin-3-yl)methoxy) quinazolin-7-yl)-2-amino-7-fluorobenzo[b]thiophene-3-nitrile | LCMS (m/z): 638.2 (M + H). $^{1}$H NMR (400 MHz, DMSO-$d_6$) $\delta$ 8.15 (s, 2H), 7.69 - 7.58 (m, 1H), 7.42 - 7.27 (m, 1H), 7.24 - 7.09 (m, 1H), 6.88 - 6.54 (m, 1H), 4.66 - 4.47 (m, 1H), 4.40 - 4.16 (m, 3H), 3.79 - 3.63 (m, 2H), 3.63 - 3.54 (m, 1H), 3.52 - 3.43 (m, 1H), 2.75 - 2.62 (m, 2H), 2.28 - 2.17 (m, 1H), 2.14 (s, 3H), 1.95 - 1.80 (m, 2H), 1.77 - 1.59 (m, 4H), 1.23 (s, 1H), 1.10 (s, 3H). $^{19}$F NMR (376 MHz, DMSO-$d_6$) $\delta$ -73.41, -116.01, -123.85, -138.85. |
| 201-1 | 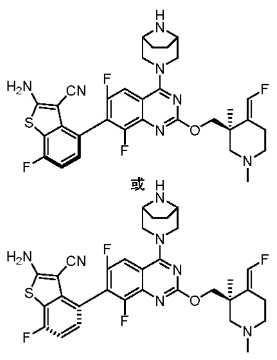 或 | *(Sa* or *Ra*)-4-(4-(1*R*,5*S*)-3,8-diazabicyclo[3.2.1]octan-3-yl)-6,8-difluoro-2-(S,E)-4-(fluoromethylene)-1,3-dimethylpiperidin-3-yl) methoxy) quinazolin-7-yl)-2-amino-7-fluorobenzo[b]thiophene-3-nitrile | LCMS (m/z): 638.3 (M + H).<br>It can be made from Intermediate **199-1-1A.** |

(continued)

| Ex. | Structural Formula | Name | Characterization data |
|---|---|---|---|
| 201-2 | 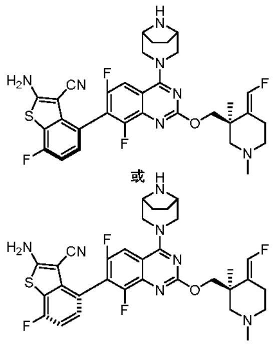 或 | *(Sa* or *Ra*)-4-(4-(1*R*,5*S*)-3,8-diazabicyclo[3.2.1]octan-3-yl)-6,8-difluoro-2-(S,E)-4-(fluoromethylene)-1,3-dimethylpiperidin-3-yl) methoxy) quinazolin-7-yl)-2-amino-7-fluorobenzo[b]thiophene-3-nitrile | LCMS (m/z): 638.3 (M + H). $^{1}$H NMR (400 MHz, DMSO-$d_6$) δ 8.18 (s, 2H), 7.65 (d, *J* = 10.2 Hz, 1H), 7.34 (dd, *J* = 8.4, 5.2 Hz, 1H), 7.20 - 7.14 (m, 1H), 6.83 (s, 0.5H), 6.61 (s, 0.5H), 4.60 - 4.52 (m, 1H), 4.42 - 4.33 (m, 1H), 4.31 - 4.17 (m, 2H), 3.93 - 3.80 (m, 2H), 3.75 - 3.65 (m, 1H), 3.61 - 3.52 (m, 1H), 2.75 - 2.64 (m, 2H), 2.24 - 2.16 (m, 1H), 2.14 (s, 3H), 1.94 - 1.72 (m, 7H), 1.11 (s, 3H). $^{19}$F NMR (376 MHz, DMSO-$d_6$) δ - 115.95, -118.39, -123.57, -138.79. It can be prepared from Intermediate **199-1-1B.** |
| 202 | 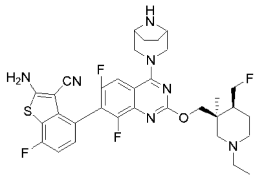 | 4-(4-((1*R*,5*S*)-3,8-diazabicyclo [3.2.1]octan-3-yl)-2-((((3S,4S)-1-ethyl-4-(fluoromethyl)-3-methylpiperidin-3-yl)methoxy)-6,8-difluoroquinazolin-7-yl)-2-amino-7-fluorobenzo[b]thiophene-3-nitrile | LCMS (m/z): 654.3 (M + H). |
| 203 | 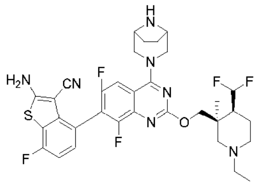 | 4-(4-((1*R*,5*S*)-3,8-diazabicyclo [3.2.1]octan-3-yl)-2-(((3*S*,4*S*)-4-(difluoromethyl)-1-ethyl-3-methylpiperidin-3-yl)methoxy)-6,8-difluoroquinazolin-7-yl)-2-amino-7-fluorobenzo[b] thiophene-3-nitrile | LCMS (m/z): 672.3 (M + H). |
| 203-1 | 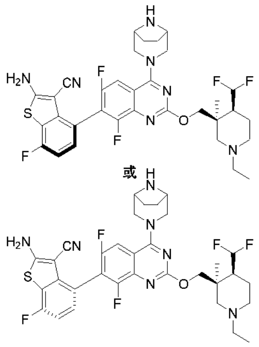 或 | *(Sa* or *Ra*)-4-(4-((1*R*,5*S*)-3,8-diazabicyclo[3.2.1]octan-3-yl)-2-(((3*S*,4*S*)-4-(difluoromethyl)-1-ethyl-3-methylpiperidin-3-yl)methoxy)-6,8-difluoroquinazolin-7-yl)-2-amino-7-fluorobenzo[b] thiophene-3-nitrile | LCMS (m/z): 672.3 (M + H). It can be prepared from Intermediate **199-1-1A.** |
| 203-2 | 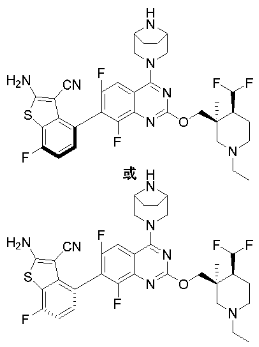 或 | *(Sa* or *Ra*)-4-(4-((1*R*,5*S*)-3,8-diazabicyclo[3.2.1]octan-3-yl)-2-(((3*S*,4*S*)-4-(difluoromethyl)-1-ethyl-3-methylpiperidin-3-yl)methoxy)-6,8-difluoroquinazolin-7-yl)-2-amino-7-fluorobenzo[*b*] thiophene-3-nitrile | LCMS (m/z): 672.3 (M + H). $^{1}$H NMR (400 MHz, DMSO-$d_6$) δ 8.14 (s, 2H), 7.60 (d, *J*= 10.4 Hz, 1H), 7.39 - 7.29 (m, 1H), 7.21 - 7.11 (m, 1H), 6.46 - 6.10 (m, 1H), 4.52 - 4.42 (m, 1H), 4.40 - 4.26 (m, 2H), 4.24 - 4.10 (m, 1H), 3.56 - 3.41 (m, 4H), 2.98 - 2.85 (m, 2H), 2.36 - 2.14 (m, 2H), 1.96 - 1.83 (m, 1H), 1.81 - 1.50 (m, 8H), 1.12 (s, 3H), 0.90 (t, *J* = 7.1 Hz, 3H). $^{19}$F NMR (376 MHz, DMSO-$d_6$) δ -115.38, - 116.10, -118.56, - 119.32, -124.01. It can be prepared from Intermediate **199-1-1B.** |

(continued)

| Ex. | Structural Formula | Name | Characterization data |
|---|---|---|---|
| 204 | 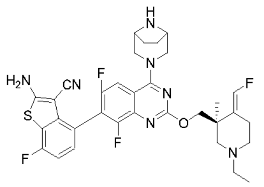 | 4-(4-((1*R*,5*S*)-3,8-diazabicyclo [3.2.1]octan-3-yl)-2-(((S,E)-1-ethyl-4-(fluoromethylene)-3-methylpiperidin-3-yl)methoxy)-6,8-difluoroquinazolin-7-yl)-2-amino-7-fluorobenzo[*b*]thiophene-3-nitrile | LCMS (m/z): 652.2 (M + H). |

Referring to the preparation methods of the above intermediates and examples, the following examples were synthesized.

| Ex. | Structural Formula | Name | Characterization data |
|---|---|---|---|
| 205 | 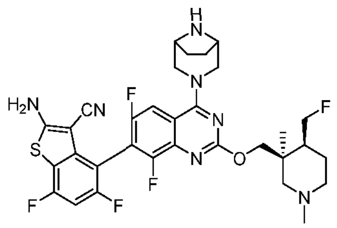 | 4-(4-(1*R*,5*S*)-3,8-diazabicyclo[3.2.1]octan-3-yl)-6,8-difluoro-2-(3S,4S)-4-(fluoromethyl)-1,3-dimethylpiperidin-3-yl)methoxy) quinazolin-7-yl)-2-amino-5,7-difluorobenzo[*b*]thiophene-3-nitrile | LCMS (m/z): 658.2 (M + H). |
| 206 | 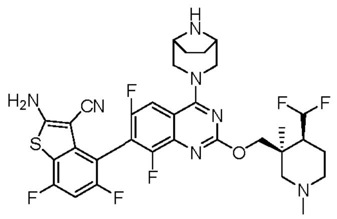 | 4-(4-((1*R*,5*S*)-3,8-diazabicyclo[3.2.1]octan-3-yl)-2-(((3S,4S)-4-(difluoromethyl)-1,3-dimethylpiperidin-3-yl)methoxy)-6,8-difluoroquinazolin-7-yl)-2-amino-5,7-difluorobenzo[*b*]thiophene-3-nitrile | LCMS (m/z): 676.2 (M + H). |
| 207 | 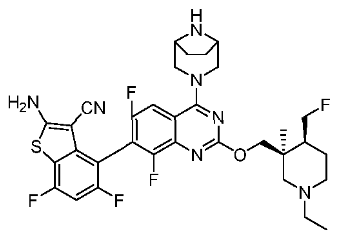 | 4-(4-((1*R*,5*S*)-3,8-diazabicyclo[3.2.1]octan-3-yl)-2-((((3*S*,4*S*)-1-ethyl-4-(fluoromethyl)-3-methylpiperidin-3-yl)methoxy)-6,8-difluoroquinazolin-7-yl)-2-amino-5,7-difluorobenzo[*b*]thiophene-3-nitrile | LCMS (m/z): 672.3 (M + H). |
| 208 | 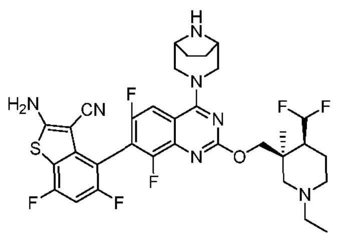 | 4-(4-((1*R*,5*S*)-3,8-diazabicyclo[3.2.1]octan-3-yl)-2-(((3*S*,4*S*)-4-(difluoromethyl)-1-ethyl-3-methylpiperidin-3-yl)methoxy)-6,8-difluoroquinazolin-7-yl)-2-amino-5,7-difluorobenzo[b]thiophene-3-nitrile | LCMS (m/z): 690.2 (M + H). |
| 209 | 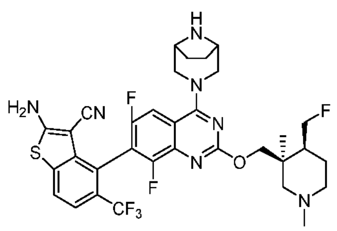 | 4-(4-((1*R*,5*S*)-3,8-diazabicyclo[3.2.1]octan-3-yl)-6,8-difluoro-2-(((3S,4S)-4-(fluoromethyl)-1,3-dimethylpiperidin-3-yl)methoxy) quinazolin-7-yl)-2-amino-5-(trifluoromethyl) benzo[b]thiophene-3-nitrile | LCMS (m/z): 690.2 (M + H). |

(continued)

| Ex. | Structural Formula | Name | Characterization data |
|---|---|---|---|
| 210 | 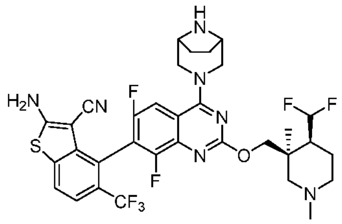 | 4-(4-((1*R*,5*S*)-3,8-diazabicyclo[3.2.1]octan-3-yl)-2-(((3*S*,4*S*)-4-(difluoromethyl)-1,3-dimethylpiperidin-3-yl)methoxy)-6,8-difluoroquinazolin-7-yl)-2-amino-5-(trifluoromethyl) benzo[b]thiophene-3-nitrile | LCMS (m/z): 708.2 (M + H). |
| 211 | 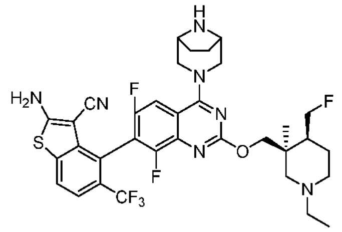 | 4-(4-((1*R*,5*S*)-3,8-diazabicyclo[3.2.1]octan-3-yl)-2-(((3S,4S)-1-ethyl-4-(fluoromethyl)-3-methylpiperidin-3-yl)methoxy)-6,8-difluoroquinazolin-7-yl)-2-amino-5-(trifluoromethyl) benzo[b]thiophene-3-nitrile | LCMS (m/z): 704.3 (M + H). |
| 212 | 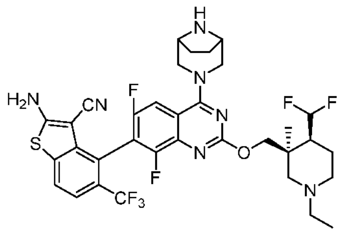 | 4-(4-((1*R*,5*S*)-3,8-diazabicyclo[3.2.1]octan-3-yl)-2-(((3S,4S)-4-(difluoromethyl)-1-ethyl-3-methylpiperidin-3-yl)methoxy)-6,8-difluoroquinazolin-7-yl)-2-amino-5-(trifluoromethyl) benzo[b]thiophene-3-nitrile | LCMS (m/z): 722.2 (M + H). |
| 213 | 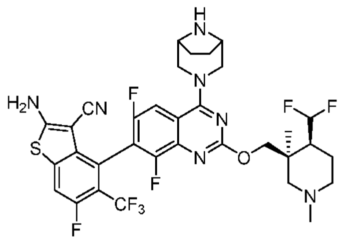 | 4-(4-((1*R*,5*S*)-3,8-diazabicyclo[3.2.1]octan-3-yl)-2-(((3*S*,4*S*)-4-(difluoromethyl)-1,3-dimethylpiperidin-3-yl)methoxy)-6,8-difluoroquinazolin-7-yl)-2-amino-6-fluoro-5-(trifluoromethyl) benzo[b] thiophene-3-nitrile | LCMS (m/z): 726.2 (M + H). |
| 214 | 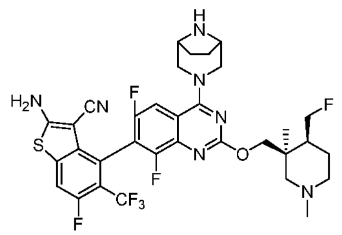 | 4-(4-((1*R*,5*S*)-3,8-diazabicyclo[3.2.1]octan-3-yl)-6,8-difluoro-2-(((3S,4S)-4-(fluoromethyl)-1,3-Dimethylpiperidin-3-yl)methoxy) quinazolin-7-yl)-2-amino-6-fluoro-5-(trifluoromethyl) benzo[b]thiophene-3-nitrile | LCMS (m/z): 708.2 (M + H). |

The compounds listed in the following table can be prepared by reference to the above synthesis method or appropriate variants:

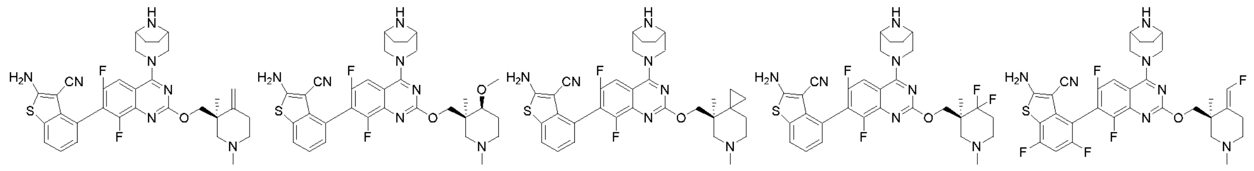

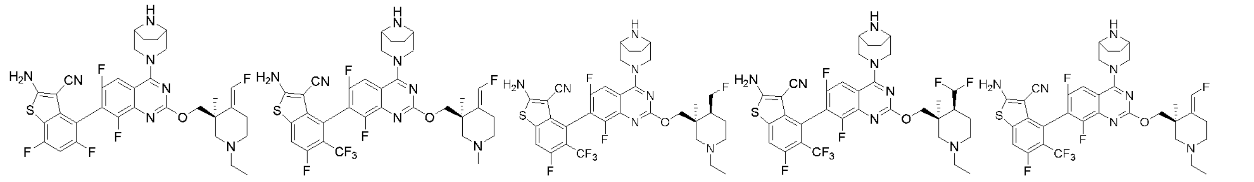

**Example 215**

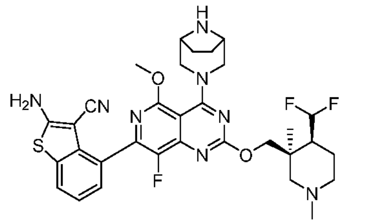

4-(4-((1R,5S)-3,8-diazabicyclo[3.2.1]octan-3-yl)-2-(((3S,4S)-4-(difluoromethyl)-1,3-dimethylpiperidin-3-yl)methoxy)-8-fluoro-5-methoxypyridine[4,3-d]pyrimidin-7-yl)-2-aminobenzo[b]thiophene-3-nitrile

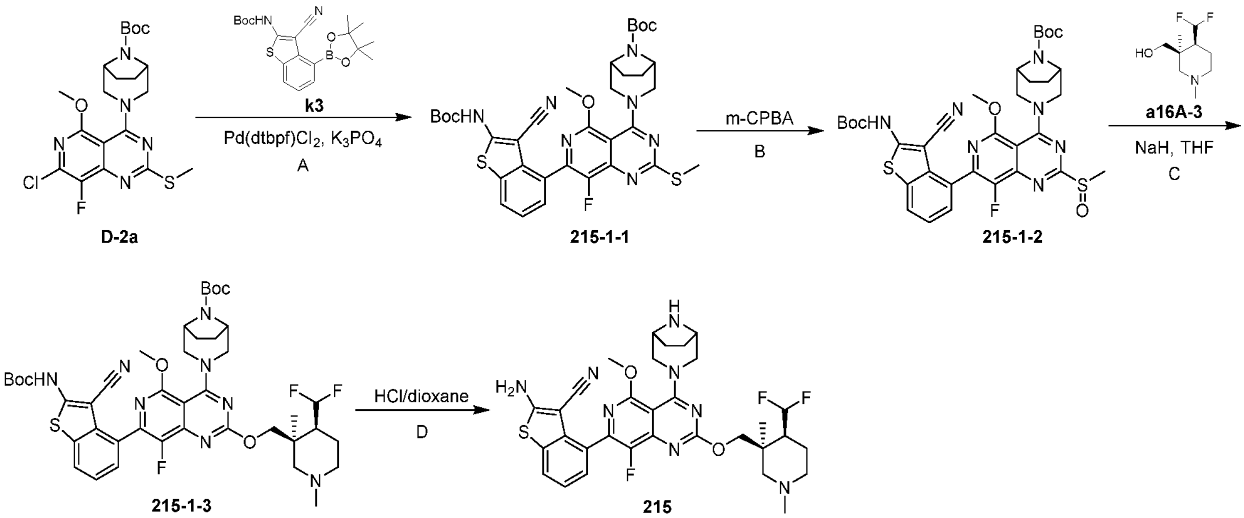


D-2a 215-1-1 215-1-2

215-1-3 215

**Step A:** *tert*-butyl (1*R*,5*S*)-3-(7-(2-((*tert*-butoxycarbonyl)amino)-3-cyanobenzothiophen-4-yl)-8-fluoro-5-methoxy-2-(methylthio)pyrido[4,3-*d*]pyrimidin-4-yl)-3,8-diazabicyclo[3.2.1]octane-8-carboxylate

Under nitrogen protection at room temperature, a solution of *tert*-butyl (3-cyano-4-(4,4,5,5-tetramethyl-1,3,2-dioxaborolan-2-yl)benzothiophen-2-yl)carbamate (1.1 g, 2.8 mmol), potassium phosphate (1.35 g, 6.4 mmol), and Pd(dtbpf)$Cl_2$ (748 mg, 1.1 mmol) were added to a solution of *tert*-butyl (1*R*,5*S*)-3-(7-chloro-8-fluoro-5-methoxy-2-(methylthio)pyrido[4,3-*d*]pyrimidin-4-yl)-3,8-diazabicyclo[3.2.1]octane-8-carboxylate (1.0 g, 2.1 mmol) in 1,4-dioxane (15 mL) and water (3 mL). The reaction was heated to 100 °C and stirred for 2 h. After the reaction was completed (monitored by LCMS), the reaction was filtered with celite and the filtrate was concentrated. The resulting crude product was purified by FCC ($SiO_2$, EA/PE = 0 - 20%) to give *tert*-butyl (1*R*,5*S*)-3-(7-(2-((*tert*-butoxycarbonyl)amino)-3-cyanobenzothiophen-4-yl)-8-fluoro-5-methoxy-2-(methylthio)pyrido[4,3-*d*]pyrimidin-4-yl)-3,8-diazabicyclo[3.2.1]octane-8-carboxylate (1.1 g, yield 73%) as a yellow solid. LCMS (m/z): 708.1 (M + H).

**Step B:** *tert*-butyl (1*R*,5*S*)-3-(7-(2-((*tert*-butoxycarbonyl)amino)-3-cyanobenzothiophen-4-yl)-8-fluoro-5-methoxy-2-(methylsulfonyl)pyrido[4,3-*d*]pyrimidin-4-yl)-3,8-diazabicyclo[3.2.1]octane-8-carboxylate

At room temperature, m-chloroperbenzoic acid (200 mg, 0.99 mmol) was added to a solution of *tert*-butyl (1*R*,5*S*)-3-(7-(2-((*tert*-butoxycarbonyl)amino)-3-cyanobenzothiophen-4-yl)-8-fluoro-5-methoxy-2-(methylthio)pyrido [4,3-*d*]pyrimidin-4-yl)-3,8-diazabicyclo[3.2.1]octane-8-carboxylate (700 mg, 0.99 mmol) in dichloromethane (10 mL), and the reaction was stirred at same temperature for 1 h. After the reaction was completed (monitored by LCMS), 50 mL of saturated aqueous sodium bicarbonate solution was added to the reaction, and the resulting mixture was extracted with dichloromethane. The combined organic phase was washed with brine, dried over anhydrous sodium sulfate, filtered, concentrated to obtain the crude product *tert*-butyl (1*R*,5*S*)-3-(7-(2-((*tert*-butoxycarbonyl)amino)-3-cyanobenzothiophen-4-yl)-8-fluoro-5-methoxy-2-(methylsulfonyl)pyrido[4,3-*d*]pyrimidin-4-yl)-3,8-diazabicyclo[3.2.1]octane-8-carboxylate (760 mg). LCMS (m/z): 724.1 (M + H).

**Step C:** *tert*-butyl (1*R*,5*S*)-3-(7-(2-((*tert*-butoxycarbonyl)amino)-3-cyanobenzo[*b*]thiophen-4-yl)-2-(((3*S*,4*S*)-4-(difluoromethyl)-1,3-dimethylpiperidin-3-yl)methoxy)-8-fluoro-5-methoxypyrido[4,3-*d*]pyrimidin-4-yl)-3,8-diazabicyclo[3.2.1]octane-8-carboxylate

Under an ice-bath, sodium hydride (11 mg, 0.28 mmol) was added to a solution of ((3*S*,4*S*)-4-(difluoromethyl)-1,3-dimethylpiperidin-3-yl) methanol (32 mg, 0.16 mmol) in anhydrous tetrahydrofuran (2 mL), and the reaction was stirred at the same temperature for 0.5 h. Then *tert*-butyl (1*R*,5*S*)-3-(7-(2-((*tert*-butoxycarbonyl)amino)-3-cyanobenzothiophen-4-yl)-8-fluoro-5-methoxy-2-(methylsulfonyl)pyrido[4,3-*d*]pyrimidin-4-yl)-3,8-diazabicyclo[3.2.1]octane-8-carboxylate (100 mg, 0.14 mmol) was added to the reaction. The resulting mixture was stirred for 2 h at room temperature. After the reaction was completed (monitored by LCMS), the reaction was poured into 30 mL of saturated aqueous ammonium chloride solution. The resulting mixture was extracted with ethyl acetate. The combined organic phase was washed with brine, dried over anhydrous sodium sulfate, filtered and concentrated to dryness. The resulting crude product was purified by FCC ($SiO_2$, MeOH/DCM = 0 - 5%) to obtain *tert*-butyl (1R,5S)-3-(7-(2-((*tert*-butoxycarbonyl) amino)-3-cyanobenzo[*b*]thiophen-4-yl)-2-(((3*S*,4*S*)-4-(difluoromethyl)-1,3-dimethylpiperidin-3-yl)methoxy)-8-fluoro-5-methoxypyrido[4,3-*d*]pyrimidin-4-yl)-3,8-diazabicyclo[3.2.1]octane-8-carboxylate (60 mg, yield 51%) as a yellow solid. LCMS (m/z): 853.2 (M + H).

**Step D:** 2-(4-((1*R*,5*S*)-3,8-diazabicyclo[3.2.1]octan-3-yl)-8-fluoro-2-(((3.5,45)-4-(fluoromethyl)-1,3-dimethylpiperidin-3-yl)methoxy)pyrido[4,3-*d*]pyrimidin-7-yl)-6-amino-4-methyl-3-(trifluoromethyl) benzonitrile

At room temperature, HCl in 1,4-dioxane solution (1 mL, 4 N) was added to *tert*-butyl (1R,5S)-3-(7-(2-((*tert*-butoxycarbonyl)amino)-3-cyanobenzo[*b*]thiophen-4-yl)-2-(((3*S*,4*S*)-4-(difluoromethyl)-1,3-dimethylpiperidin-3-yl)methoxy)-8-fluoro-5-methoxypyrido[4,3-*d*]pyrimidin-4-yl)-3,8-diazabicyclo[3.2.1]octane-8-carboxylate (60 mg, 0.03 mol), and the reaction was stirred at the same temperature for 1 h. After the reaction was completed (monitored by LCMS), the reaction was purified by pre-HPLC (C18, CAN/(10 mmol $NH_4HCO_3/H_2O$) = 30-50%) to obtain 2-(4-((1*R*,5*S*)-3, 8-diazabicyclo[3.2.1]octan-3-yl)-8-fluoro-2-(((3*S*,4*S*)-4-(fluoromethyl)-1,3-dimethylpiperidin-3-yl)methoxy)pyrido[4,3-*d*] pyrimidin-7-yl)-6-amino-4-methyl-3-(trifluoromethyl) benzonitrile (5 mg, yield 11%) as a white solid. $^1$H NMR (400 MHz, DMSO-$d_6$) δ 7.87 - 7.72 (m, 3H), 7.38 (d, *J* = 7.4 Hz, 1H), 7.25 - 7.19 (m, 1H), 6.43 - 6.15 (m, 1H), 4.51 - 4.34 (m, 3H), 3.95 (s, 3H), 3.87 - 3.74 (m, 1H), 3.62 - 3.43 (m, 5H), 2.88 - 2.74 (m, 2H), 2.11 (s, 3H), 1.89 - 1.56 (m, 8H), 1.45 - 1.33 (m, 1H), 1.11 (s, 3H). $^{19}$F NMR (376 MHz, DMSO-$d_6$) δ -115.27 ~-116.14, -118.63 ~-119.38,-151.05. LCMS (m/z): 653.3 (M + H).

Referring to the above synthesis method or appropriate variants, this invention synthesized and characterized the following compounds:

| Ex. | Structure | Name | Characterization data |
|---|---|---|---|
| 216 | 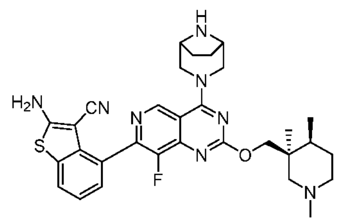 | 4-(4-((1*R*,5*S*)-3,8-diazabicyclo [3.2.1]octan-3-yl)-8-fluoro-2-(((3*S*,4*S*)-1,3,4-trimethylpiperidin-3-yl)methoxy)pyrido[4,3-*d*]pyrimidin-7-yl)-2-aminobenzo[*b*]thiophene-3-nitrile | LCMS (m/z): 605.3 (M + H). |

(continued)

| Ex. | Structure | Name | Characterization data |
|---|---|---|---|
| 217 | 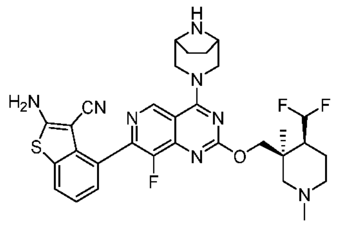 | 4-(4-((1*R*,5*S*)-3,8-diazabicyclo [3.2.1]octan-3-yl)-2-(((3*S*,4*S*)-4-(difluoromethyl)-1,3-dimethylpiperidin-3-yl)methoxy)-8-fluoropyridino [4,3-*d*]pyrimidin-7-yl)-2-aminobenzo[*b*]thiophene-3-nitrile | LCMS (m/z): 623.3 (M + H). |
| 218 | 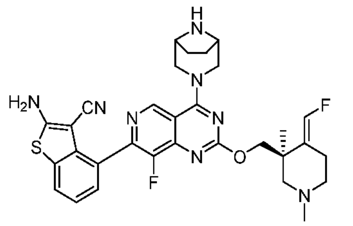 | 4-(4-(1*R*,5*S*)-3,8-diazabicyclo [3.2.1]octan-3-yl)-8-fluoro-2-(*S*, *E*)-4-(fluoromethylene)-1,3-dimethylpiperidin-3-yl)methoxy)pyrido[4,3-*d*]pyrimidin-7-yl)-2-aminobenzo[*b*]thiophene-3-nitrile | LCMS (m/z): 603.2 (M + H). |
| 219 | 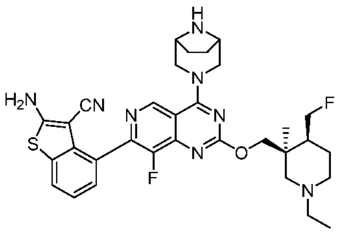 | 4-(4-((1*R*,5*S*)-3,8-diazabicyclo [3.2.1]octan-3-yl)-2-(((3*S*,4*S*)-1-ethyl-4-(fluoromethyl)-3-methylpiperidin-3-yl)methoxy)-8-fluoropyridino[4,3-*d*]pyrimidin-7-yl)-2-aminobenzo[*b*]thiophene-3-nitrile | LCMS (m/z): 619.3 (M + H). |
| 220 | 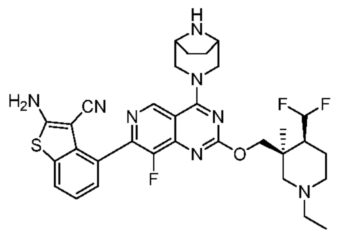 | 4-(4-((1*R*,5*S*)-3,8-diazabicyclo [3.2.1]octan-3-yl)-2-(((3*S*,4*S*)-4-(difluoromethyl)-1-ethyl-3-methylpiperidin-3-yl)methoxy)-8-fluoropyridino[4,3-*d*]pyrimidin-7-yl)-2-aminobenzo[*b*]thiophene-3-nitrile | LCMS (m/z): 637.3 (M + H). |
| 221 | 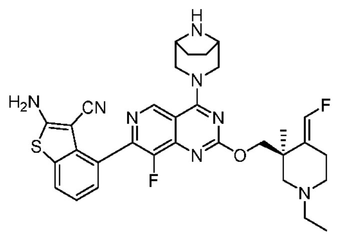 | 4-(4-((1*R*,5*S*)-3,8-diazabicyclo [3.2.1]octan-3-yl)-2-(((*S*,*E*)-1-ethyl-4-(fluoromethylene)-3-methylpiperidin-3-yl)methoxy)-8-fluoropyridino[4,3-*d*]pyrimidin-7-yl)-2-aminobenzo[*b*]thiophene-3-nitrile | LCMS (m/z): 617.3 (M + H). |
| 222 | 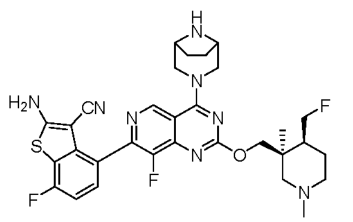 | 4-(4-((1*R*,5*S*)-3,8-diazabicyclo [3.2.1]octan-3-yl)-8-fluoro-2-(((3*S*,4*S*)-4-(fluoromethyl)-1,3-dimethylpiperidin-3-yl)methoxy) pyrido[4,3-*d*]pyrimidin-7-yl)-2-amino-7-fluorobenzo[*b*]thiophene-3-nitrile | LCMS (m/z): 623.3 (M + H). |
| 223 | 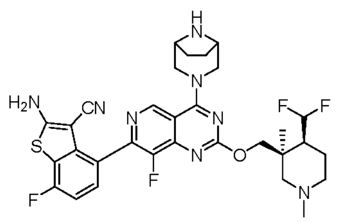 | 4-(4-((1*R*,5*S*)-3,8-diazabicyclo [3.2.1]octan-3-yl)-2-(((3*S*,4*S*)-4-(difluoromethyl)-1,3-dimethylpiperidin-3-yl)methoxy)-8-fluoropyridino [4,3-*d*]pyrimidin-7-yl)-2-amino-7-fluorobenzo[*b*]thiophene-3-nitrile | LCMS (m/z): 641.2 (M + H). |

(continued)

| Ex. | Structure | Name | Characterization data |
|---|---|---|---|
| 224 | 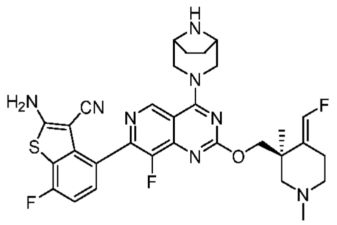 | 4-(4-((1*R*,5*S*)-3,8-diazabicyclo [3.2.1]octan-3-yl)-8-fluoro-2-(((*S*, *E*)-4-(fluoromethylene)-1,3-dimethylpiperidin-3-yl)methoxy)pyrido[4,3-*d*]pyrimidin-7-yl)-2-amino-7-fluorobenzo[*b*]thiophene-3-nitrile | LCMS (m/z): 621.2 (M + H). |
| 225 | 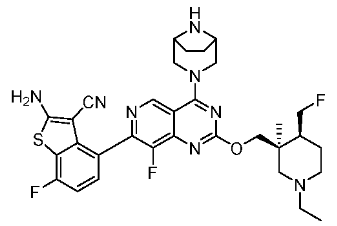 | 4-(4-((1*R*,5*S*)-3,8-diazabicyclo [3.2.1]octan-3-yl)-2-(((3*S*,4*S*)-1-ethyl-4-(fluoromethyl)-3-methylpiperidin-3-yl)methoxy)-8-fluoropyridino[4,3-*d*]pyrimidin-7-yl)-2-amino-7-fluorobenzo[*b*]thiophene-3-nitrile | LCMS (m/z): 637.3 (M + H). |
| 226 | 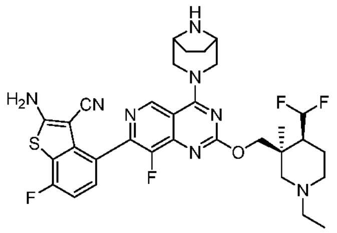 | 4-(4-((1*R*,5*S*)-3,8-diazabicyclo [3.2.1]octan-3-yl)-2-(((3*S*,4*S*)-4-(difluoromethyl)-1-ethyl-3-methylpiperidin-3-yl)methoxy)-8-fluoropyridino[4,3-*d*]pyrimidin-7-yl)-2-amino-7-fluorobenzo[*b*]thiophene-3-nitrile | LCMS (m/z): 655.3 (M + H). |
| 227 | 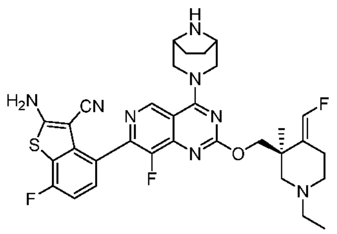 | 4-(4-((1*R*,5*S*)-3,8-diazabicyclo [3.2.1]octan-3-yl)-2-(((*S*,*E*)-1-ethyl-4-(fluoromethylene)-3-methylpiperidin-3-yl)methoxy)-8-fluoropyridino[4,3-*d*]pyrimidin-7-yl)-2-amino-7-fluorobenzo[*b*]thiophene-3-nitrile | LCMS (m/z): 635.3 (M + H). |
| 228 | 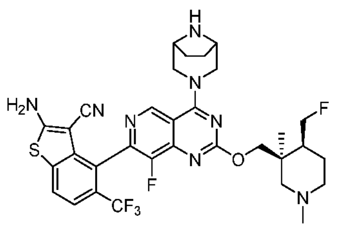 | 4-(4-((1*R*,5*S*)-3,8-diazabicyclo [3.2.1]octan-3-yl)-8-fluoro-2-(((3*S*,4*S*)-4-(fluoromethyl)-1,3-dimethylpiperidin-3-yl)methoxy) pyrido[4,3-*d*]pyrimidin-7-yl)-2-amino-5-(trifluoromethyl) benzo[*b*]thiophene-3-nitrile | LCMS (m/z): 673.2 (M + H). |
| 229 | 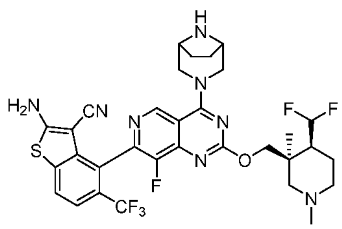 | 4-(4-((1*R*,5*S*)-3,8-diazabicyclo [3.2.1]octan-3-yl)-2-(((3*S*,4*S*)-4-(difluoromethyl)-1,3-dimethylpiperidin-3-yl)methoxy)-8-fluoropyridino [4,3-*d*]pyrimidin-7-yl)-2-amino-5-(trifluoromethyl) benzo[*b*]thiophene-3-nitrile | LCMS (m/z): 691.2 (M + H). |
| 230 | 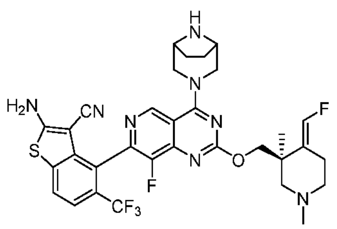 | 4-(4-((1*R*,5*S*)-3,8-diazabicyclo [3.2.1]octan-3-yl)-8-fluoro-2-(((*S*,*E*)-4-(fluoromethylene)-1,3-dimethylpiperidin-3-yl) methoxy)pyrido[4,3-*d*]pyrimidin-7-yl)-2-amino-5-(trifluoromethyl) benzo[*b*]thiophene-3-nitrile | LCMS (m/z): 671.2 (M + H). |

(continued)

| Ex. | Structure | Name | Characterization data |
|---|---|---|---|
| 231 | 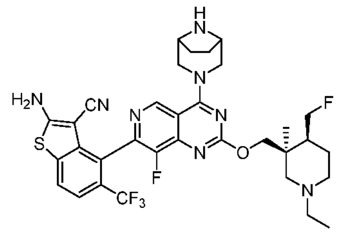 | 4-(4-((1*R*,5*S*)-3,8-diazabicyclo [3.2.1]octan-3-yl)-2-(((3*S*,4*S*)-1-ethyl-4-(fluoromethyl)-3-methylpiperidin-3-yl)methoxy)-8-fluoropyridino[4,3-*d*]pyrimidin-7-yl)-2-amino-5-(trifluoromethyl) benzo[*b*]thiophene-3-nitrile | LCMS (m/z): 687.3 (M + H). |
| 232 | 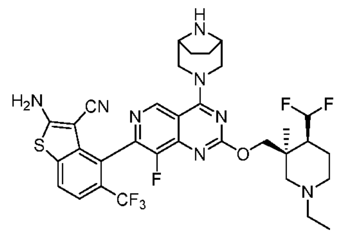 | 4-(4-((1*R*,5*S*)-3,8-diazabicyclo [3.2.1]octan-3-yl)-2-(((3*S*,4*S*)-4-(difluoromethyl)-1-ethyl-3-methylpiperidin-3-yl)methoxy)-8-fluoropyridino[4,3-*d*]pyrimidin-7-yl)-2-amino-5-(trifluoromethyl) benzo[*b*]thiophene-3-nitrile | LCMS (m/z): 705.3 (M + H). |
| 233 | 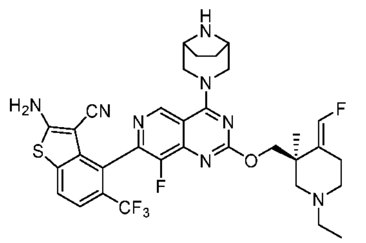 | 4-(4-((1*R*,5*S*)-3,8-diazabicyclo [3.2.1]octan-3-yl)-2-(((*S*,*E*)-1-ethyl-4-(fluoromethylene)-3-methylpiperidin-3-yl)methoxy)-8-fluoropyridino[4,3-*d*]pyrimidin-7-yl)-2-amino-5-(trifluoromethyl) benzo[*b*]thiophene-3-nitrile | LCMS (m/z): 685.2 (M + H). |
| 234 | 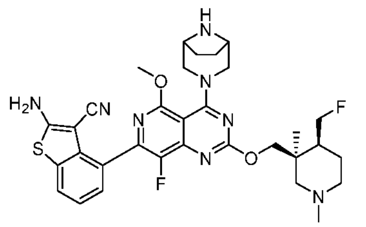 | 4-(4-((1*R*,5*S*)-3,8-diazabicyclo [3.2.1]octan-3-yl)-8-fluoro-2-(((3*S*,4*S*)-4-(fluoromethyl)-1,3-dimethylpiperidin-3-yl)methoxy)-5-methoxypyrido[4,3-*d*]pyrimidin-7-yl)-2-aminobenzo[*b*]thiophene-3-nitrile | LCMS (m/z): 635.3 (M + H). $^1$H NMR (400 MHz, Methanol-$d_4$) δ 7.65 (dd, *J* = 8.0, 1.1 Hz, 1H), 7.41 (d, *J* = 7.5 Hz, 1H), 7.27 - 7.19 (m, 1H), 4.80 - 4.35 (m, 6H), 4.05 (s, 3H), 4.02 - 3.86 (m, 1H), 3.67 - 3.36 (m, 5H), 3.16 - 2.90 (m, 2H), 2.27 (s, 3H), 2.10 - 1.98 (m, 1H), 1.86 - 1.72 (m, 5H), 1.63 - 1.56 (m, 1H), 1.16 (s, 3H). $^{19}$F NMR (376 MHz, methanol-$d_4$) δ -76.93, -152.33. |
| 235 | 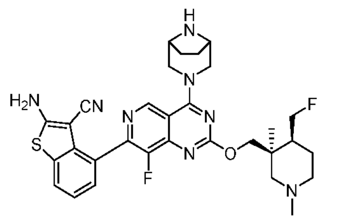 | 4-(4-((1*R*,5*S*)-3,8-diazabicyclo [3.2.1]octan-3-yl)-8-fluoro-2-(((3*S*,4*S*)-4-(fluoromethyl)-1,3-dimethylpiperidin-3-yl)methoxy) pyrido[4,3-*d*]pyrimidin-7-yl)-2-aminobenzo[*b*]thiophene-3-nitrile | LCMS (m/z): 605.3 (M + H). |
| 236 | 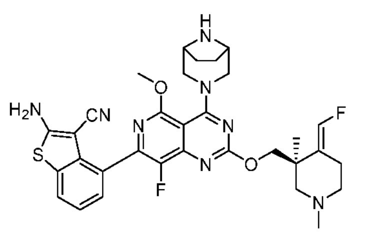 | 4-(4-((1*R*,5*S*)-3,8-diazabicyclo [3.2.1]octan-3-yl)-8-fluoro-2-(((*S*,*E*)-4-(fluoromethylene)-1,3-dimethylpiperidin-3-yl)methoxy)-5-methoxypyrido[4,3-*d*]pyrimidin-7-yl)-2-aminobenzo[*b*]thiophene-3-nitrile | LCMS (m/z): 633.3 (M + H). |

(continued)

| Ex. | Structure | Name | Characterization data |
|---|---|---|---|
| 237 | 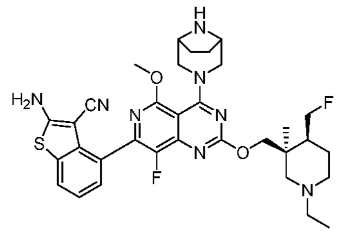 | 4-(4-((1*R*,5*S*)-3,8-diazabicyclo [3.2.1]octan-3-yl)-2-(((3*S*,4*S*)-1-ethyl-4-(fluoromethyl)-3-methylpiperidin-3-yl)methoxy)-8-fluoro-5-methoxypyrido[4,3-*d*]pyrimidin-7-yl)-2-aminobenzo[*b*]thiophene-3-nitrile | LCMS (m/z): 649.3 (M + H). |
| 238 | 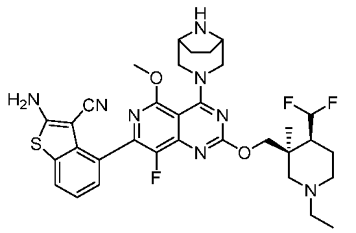 | 4-(4-((1*R*,5*S*)-3,8-diazabicyclo [3.2.1]octan-3-yl)-2-(((3*S*,4*S*)-4-(difluoromethyl)-1-ethyl-3-methylpiperidin-3-yl)methoxy)-8-fluoro-5-methoxypyrido[4,3-*d*]pyrimidin-7-yl)-2-aminobenzo[*b*]thiophene-3-nitrile | LCMS (m/z): 667.3 (M + H). |
| 239 | 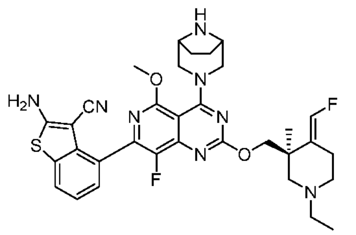 | 4-(4-((1*R*,5*S*)-3,8-diazabicyclo [3.2.1]octan-3-yl)-2-(((*S*,*E*)-1-ethyl-4-(fluoromethylene)-3-methylpiperidin-3-yl)methoxy)-8-fluoro-5-methoxypyrido[4,3-*d*]pyrimidin-7-yl)-2-aminobenzo[*b*]thiophene-3-nitrile | LCMS (m/z): 647.3 (M + H). |
| 240 | 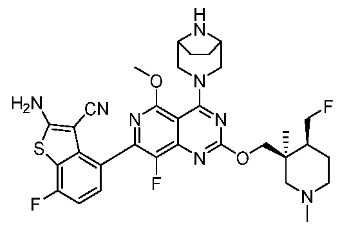 | 4-(4-((1*R*,5*S*)-3,8-diazabicyclo [3.2.1]octan-3-yl)-8-fluoro-2-(((3*S*,4*S*)-4-(fluoromethyl)-1,3-dimethylpiperidin-3-yl)methoxy)-5-methoxypyrido[4,3-*d*]pyrimidin-7-yl)-2-amino-7-fluorobenzo[*b*]thiophene-3-nitrile | LCMS (m/z): 653.3 (M + H). |
| 241 | 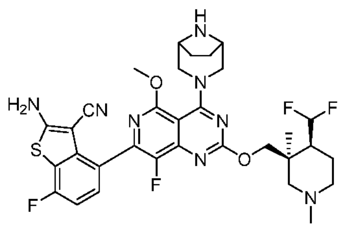 | 4-(4-((1*R*,5*S*)-3,8-diazabicyclo [3.2.1]octan-3-yl)-2-(((3*S*,4*S*)-4-(difluoromethyl)-1,3-dimethylpiperidin-3-yl)methoxy)-8-fluoro-5-methoxypyrido[4,3-*d*]pyrimidin-7-yl)-2-amino-7-fluorobenzo[*b*]thiophene-3-nitrile | LCMS (m/z): 671.3 (M + H). $^1$H NMR (400 MHz, DMSO-$d_6$) δ 8.08 (s, 2H), 7.45 (dd, *J* = 8.4, 5.3 Hz, 1H), 7.13 (dd, *J* = 9.5, 8.4 Hz, 1H), 6.29 (t, *J* = 55.6 Hz, 1H), 4.51 - 4.30 (m, 3H), 3.95 (s, 3H), 3.82 - 3.69 (m, 1H), 3.53 - 3.42 (m, 4H), 2.88 - 2.76 (m, 2H), 2.10 (s, 3H), 1.86 - 1.59 (m, 6H), 1.57 - 1.51 (m, 2H), 1.34 - 1.20 (m, 2H), 1.10 (s, 3H). $^{19}$F NMR (376 MHz, DMSO-$d_6$) δ -116.22, -118.68 ~-119.43, - 151.03. |

(continued)

| Ex. | Structure | Name | Characterization data |
|---|---|---|---|
| 242 | 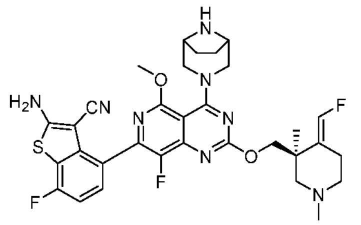 | 4-(4-((1*R*,5*S*)-3,8-diazabicyclo [3.2.1]octan-3-yl)-8-fluoro-2-(((*S*, *E*)-4-(fluoromethylene)-1,3-dimethylpiperidin-3-yl)methoxy)-5-methoxypyrido[4,3-*d*]pyrimidin-7-yl)-2-amino-7-fluorobenzo[*b*]thiophene-3-nitrile | LCMS (m/z): 651.2 (M + H). $^{1}H$ NMR (400 MHz, DMSO-$d_6$) δ 8.08 (s, 2H), 7.44 (dd, *J* = 8.4, 5.3 Hz, 1H), 7.13 (dd, *J* = 9.5, 8.3 Hz, 1H), 6.70 (d, *J* = 86.8 Hz, 1H), 4.61 - 4.22 (m, 2H), 3.94 (s, 3H), 3.51 - 3.38 (m, 2H), 2.74 - 2.59 (m, 4H), 2.25 - 2.11 (m, 5H), 1.92 - 1.81 (m, 2H), 1.59 - 1.49 (m, 3H), 1.34 - 1.25 (m, 1H), 1.09 (s, 3H). $^{19}F$ NMR (376 MHz, DMSO-$d_6$) δ - 116.21, -138.77, - 151.10. |
| 243 | 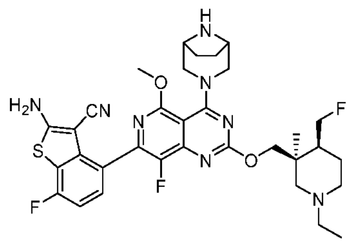 | 4-(4-((1*R*,5*S*)-3,8-diazabicyclo [3.2.1]octan-3-yl)-2-(((3*S*,4*S*)-1-ethyl-4-(fluoromethyl)-3-methylpiperidin-3-yl)methoxy)-8-fluoro-5-methoxypyrido[4,3-*d*]pyrimidin-7-yl)-2-amino-7-fluorobenzo[*b*]thiophene-3-nitrile | LCMS (m/z): 667.3 (M + H). |
| 244 | 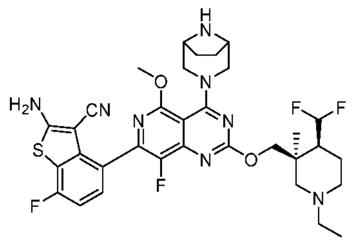 | 4-(4-((1*R*,5*S*)-3,8-diazabicyclo [3.2.1]octan-3-yl)-2-(((3*S*,4*S*)-4-(difluoromethyl)-1-ethyl-3-methylpiperidin-3-yl)methoxy)-8-fluoro-5-methoxypyrido[4,3-*d*]pyrimidin-7-yl)-2-amino-7-fluorobenzo[*b*]thiophene-3-nitrile | LCMS (m/z): 685.3 (M + H). |
| 245 | 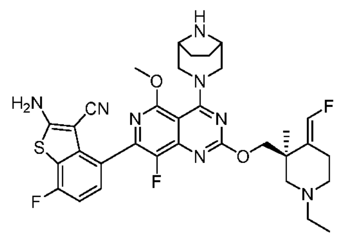 | 4-(4-((1*R*,5*S*)-3,8-diazabicyclo [3.2.1]octan-3-yl)-2-(((*S*,*E*)-1-ethyl-4-(fluoromethylene)-3-methylpiperidin-3-yl)methoxy)-8-fluoro-5-methoxypyrido[4,3-*d*]pyrimidin-7-yl)-2-amino-7-fluorobenzo [*b*]thiophene-3-nitrile | LCMS (m/z): 665.3 (M + H). |
| 246 | 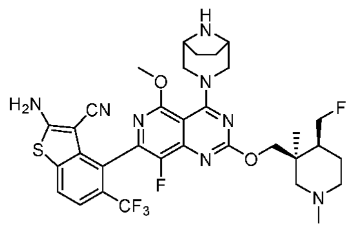 | 4-(4-((1*R*,5*S*)-3,8-diazabicyclo [3.2.1]octan-3-yl)-8-fluoro-2-(((3*S*,4*S*)-4-(fluoromethyl)-1,3-dimethylpiperidin-3-yl)methoxy)-5-methoxypyrido[4,3-*d*]pyrimidin-7-yl)-2-amino-5-(trifluoromethyl) benzo[*b*]thiophene-3-nitrile | LCMS (m/z): 703.3 (M + H). |
| 247 | 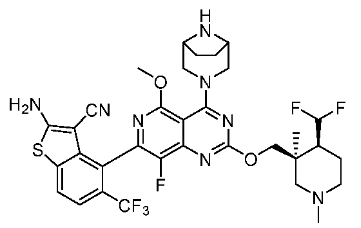 | 4-(4-((1*R*,5*S*)-3,8-diazabicyclo [3.2.1]octan-3-yl)-2-(((3*S*,4*S*)-4-(difluoromethyl)-1,3-dimethylpiperidin-3-yl)methoxy)-8-fluoro-5-methoxypyrido[4,3-*d*]pyrimidin-7-yl)-2-amino-5-(trifluoromethyl) benzo[*b*] thiophene-3-nitrile | LCMS (m/z): 721.2 (M + H). |

(continued)

| Ex. | Structure | Name | Characterization data |
|---|---|---|---|
| 248 | 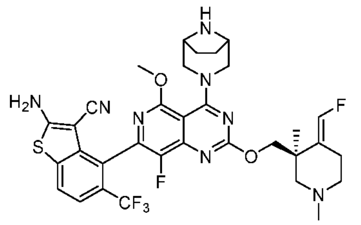 | 4-(4-((1*R*,5*S*)-3,8-diazabicyclo [3.2.1]octan-3-yl)-8-fluoro-2-(((*S*, *E*)-4-(fluoromethylene)-1,3-dimethylpiperidin-3-yl)methoxy)-5-methoxypyrido[4,3-*d*]pyrimidin-7-yl)-2-amino-5-(trifluoromethyl) benzo[*b*]thiophene-3-nitrile | LCMS (m/z): 701.2 (M + H). |
| 249 | 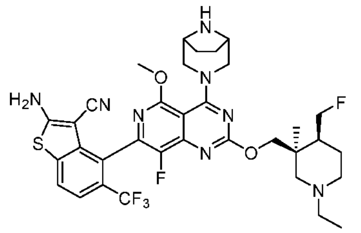 | 4-(4-((1*R*,5*S*)-3,8-diazabicyclo [3.2.1]octan-3-yl)-2-(((3*S*,4*S*)-1-ethyl-4-(fluoromethyl)-3-methylpiperidin-3-yl)methoxy)-8-fluoro-5-methoxypyrido[4,3-*d*]pyrimidin-7-yl)-2-amino-5-(trifluoromethyl) benzo[*b*]thiophene-3-nitrile | LCMS (m/z): 717.3 (M + H). |
| 250 | 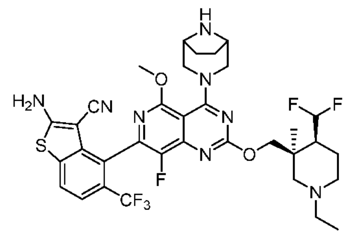 | 4-(4-((1*R*,5*S*)-3,8-diazabicyclo [3.2.1]octan-3-yl)-2-(((3*S*,4*S*)-4-(difluoromethyl)-1-ethyl-3-methylpiperidin-3-yl)methoxy)-8-fluoro-5-methoxypyrido[4,3-*d*]pyrimidin-7-yl)-2-amino-5-(trifluoromethyl) benzo[*b*]thiophene-3-nitrile | LCMS (m/z): 735.3 (M + H). |
| 251 | 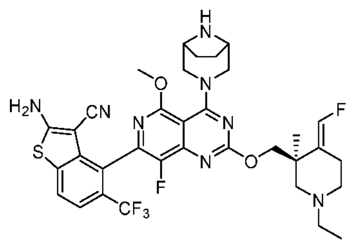 | 4-(4-((1*R*,5*S*)-3,8-diazabicyclo [3.2.1]octan-3-yl)-2-(((*S*,*E*)-1-ethyl-4-(fluoromethylene)-3-methylpiperidin-3-yl)methoxy)-8-fluoro-5-methoxypyrido[4,3-*d*]pyrimidin-7-yl)-2-amino-5-(trifluoromethyl) benzo[*b*]thiophene-3-nitrile | LCMS (m/z): 715.3 (M + H). |
| 252 | 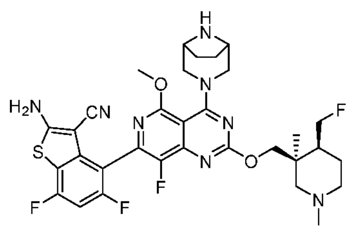 | 4-(4-((1*R*,5*S*)-3,8-diazabicyclo [3.2.1]octan-3-yl)-8-fluoro-2-(((3*S*,4*S*)-4-(fluoromethyl)-1,3-dimethylpiperidin-3-yl)methoxy)-5-methoxypyrido[4,3-*d*]pyrimidin-7-yl)-2-amino-5,7-difluorobenzo[*b*] thiophene-3-nitrile | LCMS (m/z): 671.3 (M + H). |
| 253 | 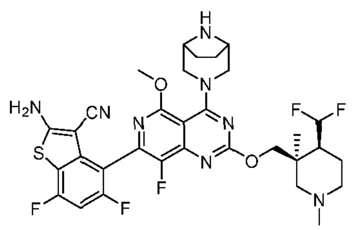 | 4-(4-((1*R*,5*S*)-3,8-diazabicyclo [3.2.1]octan-3-yl)-2-(((3*S*,4*S*)-4-(difluoromethyl)-1,3-dimethylpiperidin-3-yl)methoxy)-8-fluoro-5-methoxypyrido[4,3-*d*]pyrimidin-7-yl)-2-amino-5,7-difluorobenzo[*b*]thiophene-3-nitrile | LCMS (m/z): 689.2 (M + H). |

(continued)

| Ex. | Structure | Name | Characterization data |
|---|---|---|---|
| 254 | 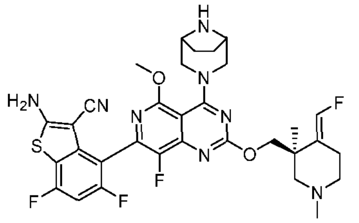 | 4-(4-((1*R*,5*S*)-3,8-diazabicyclo [3.2.1]octan-3-yl)-8-fluoro-2-(((*S*, *E*)-4-(fluoromethylene)-1,3-dimethylpiperidin-3-yl)methoxy)-5-methoxypyrido[4,3-*d*]pyrimidin-7-yl)-2-amino-5,7-difluorobenzo[*b*] thiophene-3-nitrile | LCMS (m/z): 669.2 (M + H). |
| 255 | 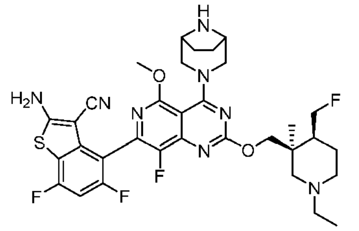 | 4-(4-((1*R*,5*S*)-3,8-diazabicyclo [3.2.1]octan-3-yl)-2-(((3*S*,4*S*)-1-ethyl-4-(fluoromethyl)-3-methylpiperidin-3-yl)methoxy)-8-fluoro-5-methoxypyrido[4,3-*d*]pyrimidin-7-yl)-2-amino-5,7-difluorobenzo[*b*] thiophene-3-nitrile | LCMS (m/z): 685.3 (M + H). |
| 256 | 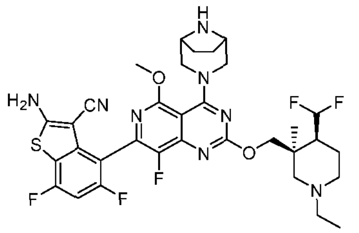 | 4-(4-((1*R*,5*S*)-3,8-diazabicyclo [3.2.1]octan-3-yl)-2-(((3*S*,4*S*)-4-(difluoromethyl)-1-ethyl-3-methylpiperidin-3-yl)methoxy)-8-fluoro-5-methoxypyrido[4,3-*d*]pyrimidin-7-yl)-2-amino-5,7-difluorobenzo[*b*] thiophene-3-nitrile | LCMS (m/z): 703.3 (M + H). |
| 257 | 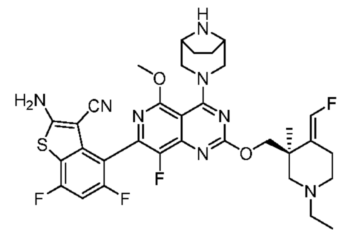 | 4-(4-((1*R*,5*S*)-3,8-diazabicyclo [3.2.1]octan-3-yl)-2-(((*S*,*E*)-1-ethyl-4-(fluoromethylene)-3-methylpiperidin-3-yl)methoxy)-8-fluoro-5-methoxypyrido[4,3-*d*]pyrimidin-7-yl)-2-amino-5,7-difluorobenzo[*b*]thiophene-3-nitrile | LCMS (m/z): 683.3 (M + H). |

Example 258

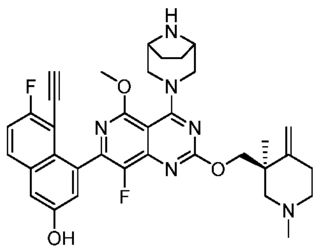

4-(4-((1*R*,5*S*)-3,8-diazabicyclo[3.2.1]octan-3-yl)-2-(((*S*)-1,3-dimethyl-4-methylenepiperidin-3-yl)methoxy)-8-fluoro-5-methoxypyrido[4,3-*d*]pyrimidin-7-yl)-5-ethynyl-6-fluoronaphthalen-2-ol

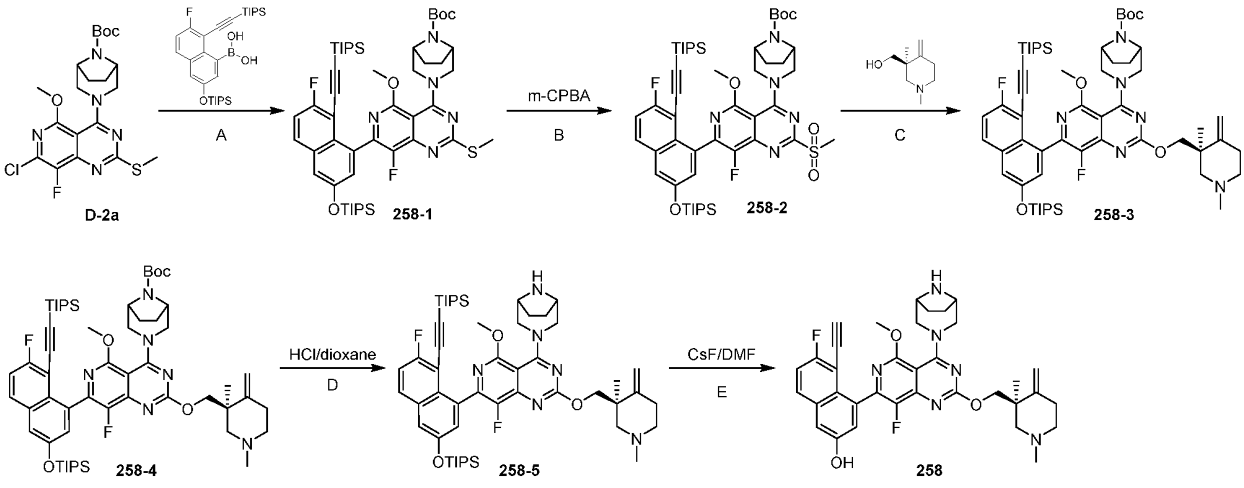


D-2a, 258-1, 258-2, 258-3, 258-4, 258-5, 258

**Step A**: (1*R*,5*S*)-3-(8-fluoro-7-(7-fluoro-8-((triisopropylsilyl)ethynyl)-3-((triisopropylsilyl)oxy)naphthalen-1-yl)-5-methoxy-2-(methylthio)pyrido[4,3-*d*]pyrimidin-4-yl)-3,8-diazabicyclo[3.2.1]octane-8-carboxylate

Under nitrogen atmosphere, a solution of tert-butyl (1*R*,5*S*)-3-(7-chloro-8-fluoro-5-methoxy-2-(methylthio)pyrido[4,3-*d*]pyrimidin-4-yl)-3,8-diazabicyclo[3.2.1]octane-8-carboxylate (500 mg, 1.1 mmol), (7-fluoro-8-((triisopropylsilyl)ethynyl)-3-((triisopropylsilyl)oxy)naphthalen-1-yl) boronic acid (1.2g, 2.2 mmol), potassium phosphate (678 mg, 3.2 mmol), and tetrakis(triphenylphosphine)palladium (60 mg, 0.05 mmol) in dioxane/water (5 mL, V/V = 1: 1) was heated to 100 °C and stirred for 2 h. After the reaction was monitored to be complete by LCMS, the reaction mixture was concentrated under reduced pressure. The resulting crude product was purified by FCC ($SiO_2$, EA/PE = 0-50%) to give (1*R*,5*S*)-3-(8-fluoro-7-(7-fluoro-8-((triisopropylsilyl)ethynyl)-3-((triisopropylsilyl)oxy)naphthalen-1-yl)-5-methoxy-2-(methylthio)pyrido[4,3-*d*]pyrimidin-4-yl)-3,8-diazabicyclo[3.2.1]octane-8-carboxylate (260 mg, yield 28%) as a white solid. LCMS (m/z): 776.4 (M-156 + H).

**Step B:** (1*R*,5*S*)-3-(8-fluoro-7-(7-fluoro-8-((triisopropylsilyl)ethynyl)-3-((triisopropylsilyl)oxy)naphthalen-1-yl)-5-methoxy-2-(methylsulfonyl)pyrido[4,3-*d*]pyrimidin-4-yl)-3,8-diazabicyclo[3.2.1]octane-8-carboxylate

A solution of (1*R*,5*S*)-3-(8-fluoro-7-(7-fluoro-8-((triisopropylsilyl)ethynyl)-3-((triisopropylsilyl)oxy)naphthalen-1-yl)-5-methoxy-2-(methylthio)pyrido[4,3-*d*]pyrimidin-4-yl)-3,8-diazabicyclo[3.2.1]octane-8-carboxylate (100 mg, 0.11 mmol) and m-CPBA (36 mg, 0.22 mmol, 85%) in dichloromethane (3 mL) was stirred at room temperature for 1 h. After the reaction was completed (monitored by LCMS), the reaction was diluted with ethyl acetate (10 mL) and then washed with saturated sodium bicarbonate aqueous solution and brine. The resulting organic phase was concentrated to obtain (1*R*,5*S*)-3-(8-fluoro-7-(7-fluoro-8-((triisopropylsilyl)ethynyl)-3-((triisopropylsilyl)oxy)naphthalen-1-yl)-5-methoxy-2-(methylsulfonyl)pyrido[4,3-*d*]pyrimidin-4-yl)-3,8-diazabicyclo[3.2.1]octane-8-carboxylate (80 mg, yield 77%) as a white solid. LCMS (m/z): 808.3 (M-156 + H).

**Step C:** *tert*-butyl (1*R*,5*S*)-3-(2-(((*S*)-1,3-dimethyl-4-methylene piperidin-3-yl)methoxy)-8-fluoro-7-(7-fluoro-8-((triisopropylsilyl)ethynyl)-3-((triisopropylsilyl)oxy)naphthalen-1-yl)-5-methoxypyrido[4,3-*d*]pyrimidin-4-yl)-3,8-diazabicyclo[3.2.1]octane-8-carboxylate

NaH (14 mg, 0.35 mmol, 60%) was added to a solution of (*S*)-(1,3-dimethyl-4-methylene piperidin-3-yl) methanol (24 mg, 0.15 mmol) in tetrahydrofuran (3 mL) at room temperature and stirred for 0.5 h. Then, (1*R*,5*S*)-3-(8-fluoro-7-(7-fluoro-8-((triisopropylsilyl)ethynyl)-3-((triisopropylsilyl)oxy)naphthalen-1-yl)-5-methoxy-2-(methylsulfonyl)pyrido[4,3-*d*]pyrimidin-4-yl)-3,8-diazabicyclo[3.2.1]octane-8-carboxylate (80 mg, 0.085 mmol) was added, and the resulting mixture was stirred at room temperature for 2 h. After the reaction was completed (monitored by LCMS), the reaction was poured into $NH_4Cl$ (10 mL) and extracted with EA (10 mL × 2). The combined organic phase was washed with brine (20 mL), concentrated to obtained *tert*-butyl (1*R*,5*S*)-3-(2-(((*S*)-1,3-dimethyl-4-methylene piperidin-3-yl)methoxy)-8-fluoro-7-(7-fluoro-8-((triisopropylsilyl)ethynyl)-3-((triisopropylsilyl)oxy)naphthalen-1-yl)-5-methoxypyrido[4,3-*d*]pyrimidin-4-yl)-3,8-diazabicyclo[3.2.1]octane-8-carboxylate (40 mg, yield 45%) as a yellow solid. LCMS (m/z): 883.4 (M-156 + H).

**Step D:** 4-((1*R*,5*S*)-3,8-diazabicyclo[3.2.1]octan-3-yl)-2-(((*S*)-1,3-dimethyl-4-methylene piperidin-3-yl)methoxy)-8-fluoro-7-(7-fluoro-8-((triisopropylsilyl)ethynyl)-3-((triisopropylsilyl)oxy)naphthalen-1-yl)-5-methoxypyrido[4,3-*d*]pyrimidine · hydrochloride

At room temperature, hydrochloric acid-dioxane solution (3 mL, 4 M) was added to *tert*-butyl (1*R*,5*S*)-3-(2-(((*S*)-1,3-dimethyl-4-methylene piperidin-3-yl)methoxy)-8-fluoro-7-(7-fluoro-8-((triisopropylsilyl)ethynyl)-3-((triisopropylsilyl)oxy)naphthalen-1-yl)-5-methoxypyrido[4,3-*d*]pyrimidin-4-yl)-3,8-diazabicyclo[3.2.1]octane-8-carboxylate (40 mg, 0.038 mmol). The resulting mixture was stirred at room temperature for 1 h and concentrate to remove the acid. The residue was diluted with EA (10 mL), and the pH was adjusted to about 8 with saturated $NaHCO_3$ solution (10 mL), then water (10 mL) was added. The mixture was extracted with EA (10 mL × 2). The collected organic phase was washed with saturated brine and then concentrated to obtain 4-((1*R*,5*S*)-3,8-diazabicyclo[3.2.1]octane-3-yl)-2-(((*S*)-1,3-dimethyl-4-methylene piperidin-3-yl)methoxy)-8-fluoro-7-(7-fluoro-8-((triisopropylsilyl)ethynyl)-3-((triisopropylsilyl)oxy) naphthalene-1-yl)-5-methoxypyrido[4,3-*d*]pyrimidine · hydrochloride (30 mg) as a yellow solid, which was used directly in the next reaction.

**Step E:** 4-(4-((1*R*,5*S*)-3,8-diazabicyclo[3.2.1]octan-3-yl)-2-(((*S*)-1,3-dimethyl-4-methylene piperidin-3-yl)methoxy)-8-fluoro-5-methoxypyrido[4,3-*d*]pyrimidin-7-yl)-5-ethynyl-6-fluoronaphthalen-2-ol

A solution of 4-((1*R*,5*S*)-3,8-diazabicyclo[3.2.1]octan-3-yl)-2-(((*S*)-1,3-dimethyl-4-methylenepiperidin-3-yl) methoxy)-8-fluoro-7-(7-fluoro-8-((triisopropylsilyl)ethynyl)-3-((triisopropylsilyl)oxy)naphthalen-1-yl)-5-methoxypyrido [4,3-*d*]pyrimidine · hydrochloride (30 mg) and CsF (50 mg, 0.32 mmol) in DMF (2 mL) was heated to 50 °C and stirred for 1 h. After the reaction was completed (monitored by LCMS), the crude product was purified by Pre-HPLC to afford 4-(4-((1*R*,5*S*)-3,8-diazabicyclo[3.2.1]octan-3-yl)-2-(((*S*)-1,3-dimethyl-4-methylene piperidin-3-yl)methoxy)-8-fluoro-5-methoxypyrido[4,3-*d*]pyrimidin-7-yl)-5-ethynyl-6-fluoronaphthalen-2-ol (10 mg, yield 50%) as a yellow solid. LCMS (m/z): 627.3 (M + H). $^1$H NMR (400 MHz, $CD_3OD$-$d_4$) δ 7.87 - 7.77 (m, 1H), 7.34 - 7.26 (m, 2H), 7.24 - 7.19 (m, 1H), 4.97 - 4.91 (m, 2H), 4.60 - 4.42 (m, 2H), 4.41 - 4.28 (m, 1H), 4.26 - 4.12 (m, 1H), 4.03 - 3.98 (m, 4H), 3.74 - 3.71 (m, 2H), 3.63 - 3.43 (m, 3H), 3.03 - 2.92 (m, 1H), 2.91 - 2.79 (m, 1H), 2.64 - 2.52 (m, 1H), 2.41 - 2.30 (m, 4H), 2.29 - 2.18 (m, 1H), 2.18 - 2.09 (m, 1H), 1.91 - 1.70 (m, 3H), 1.25 (s, 3H).

| Ex. | Structure | Name | Characterization data |
|---|---|---|---|
| 259 | 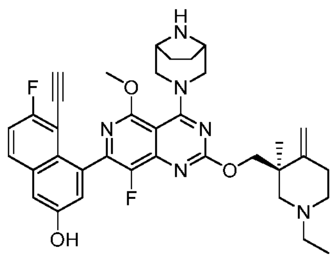 | 4-(4-((1*R*,5*S*)-3,8-diazabicyclo[3.2.1]octan-3-yl)-2-(((*S*)-1-ethyl-3-methyl-4-methylene piperidin-3-yl)methoxy)-8-fluoro-5-methoxypyrido[4,3-*d*]pyrimidin-7-yl)-5-ethynyl-6-fluoronaphthalen-2-ol | LCMS (m/z): 641.3 (M + H). |
| 260 | 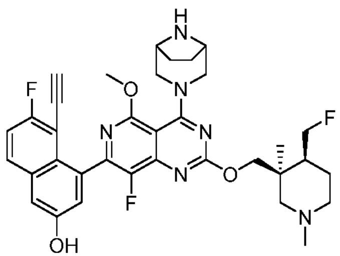 | 4-(4-((1*R*,5*S*)-3,8-diazabicyclo[3.2.1]octan-3-yl)-8-fluoro-2-(((3*S*,4*S*)-4-(fluoromethyl)-1,3-dimethylpiperidin-3-yl)methoxy)-5-methoxypyrido[4,3-*d*]pyrimidin-7-yl)-5-ethynyl-6-fluoronaphthalen-2-ol | LCMS (m/z): 647.3 (M + H). $^1$H NMR (400 MHz, DMSO-$d_6$) $\delta$ 10.17 (s, 1H), 7.97 (dd, *J* = 9.2, 5.9 Hz, 1H), 7.51 - 7.42 (m, 1H), 7.39 (d, *J* = 2.6 Hz, 1H), 7.22 (d, *J* = 2.6 Hz, 1H), 4.77 - 4.69 (m, 0H), 4.65 - 4.57 (m, 0H), 4.55 - 4.36 (m, 2H), 4.35 - 4.29 (m, 1H), 4.18 - 4.04 (m, 1H), 4.01 - 3.91 (m, 2H), 3.89 (s, 3H), 3.67 - 3.55 (m, 2H), 3.47 - 3.39 (m, 2H), 2.85 - 2.71 (m, 2H), 2.11 (s, 3H), 1.91 - 1.81 (m, 1H), 1.72 - 1.55 (m, 8H), 1.06 (s, 3H). |
| 261 | 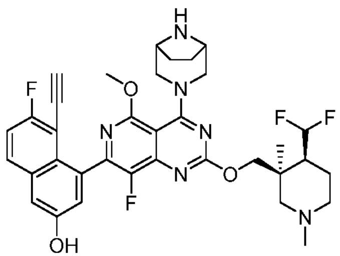 | 4-(4-((1*R*,5*S*)-3,8-diazabicyclo[3.2.1]octan-3-yl)-2-(((3*S*,4*S*)-4-(difluoromethyl)-1,3-dimethylpiperidin-3-yl)methoxy)-8-fluoro-5-methoxypyrido[4,3-*d*]pyrimidin-7-yl)-5-ethynyl-6-fluoronaphthalen-2-ol | LCMS (m/z): 665.3 (M + H). $^1$H NMR (400 MHz, DMSO-$d_6$) $\delta$ 10.17 (s, 1H), 7.97 (dd, *J* = 9.2, 5.9 Hz, 1H), 7.52 - 7.43 (m, 1H), 7.38 (d, *J* = 2.6 Hz, 1H), 7.22 (d, *J* = 2.5 Hz, 1H), 6.46 - 6.12 (m, 1H), 4.49 - 4.35 (m, 2H), 4.17 - 4.02 (m, 1H), 4.01 - 3.77 (m, 5H), 3.52 - 3.45 (m, 2H), 3.45 - 3.38 (m, 2H), 2.91 - 2.75 (m, 2H), 2.11 (s, 3H), 1.90 - 1.49 (m, 9H), 1.11 (s, 3H). $^{19}$F NMR (376 MHz, DMSO-$d_6$) $\delta$ - 110.49, -115.86, - 119.02, -149.80. |
| 262 | 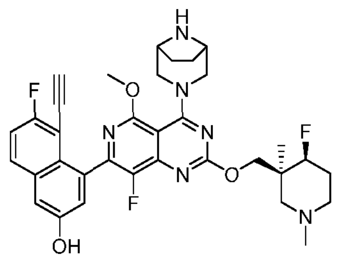 | 4-(4-((1*R*,5*S*)-3,8-diazabicyclo[3.2.1]octan-3-yl)-8-fluoro-2-(((3*S*,4*S*)-4-fluoro-1,3-dimethylpiperidin-3-yl)methoxy)-5-methoxypyrido[4,3-*d*]pyrimidin-7-yl)-5-ethynyl-6-fluoronaphthalen-2-ol | LCMS (m/z): 633.3 (M + H). |

(continued)

| Ex. | Structure | Name | Characterization data |
|---|---|---|---|
| 263 | 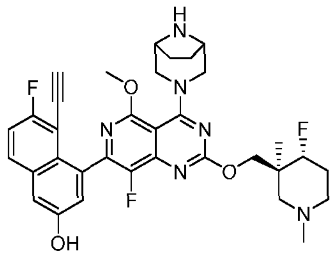 | 4-(4-((1*R*,5*S*)-3,8-diazabicyclo[3.2.1]octan-3-yl)-8-fluoro-2-(((3*S*,4*R*)-4-fluoro-1,3-dimethylpiperidin-3-yl)methoxy)-5-methoxypyrido[4,3-*d*]pyrimidin-7-yl)-5-ethynyl-6-fluoronaphthalen-2-ol | LCMS (m/z): 633.3 (M + H). |
| 264 | 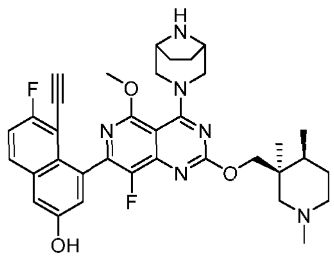 | 4-(4-((1*R*,5*S*)-3,8-diazabicyclo[3.2.1]octan-3-yl)-8-fluoro-5-methoxy-2-(((3*S*,4*S*)-1,3,4-trimethylpiperidin-3-yl)methoxy)pyrido[4,3-*d*]pyrimidin-7-yl)-5-ethynyl-6-fluoronaphthalen-2-ol | LCMS (m/z): 629.3 (M + H). |
| 265 | 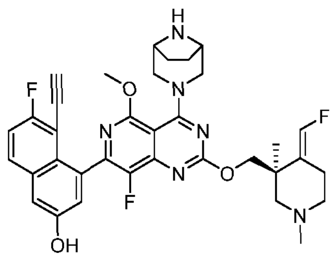 | 4-(4-((1*R*,5*S*)-3,8-diazabicyclo[3.2.1]octan-3-yl)-8-fluoro-2-(((*S,E*)-4-(fluoromethylene)-1,3-dimethylpiperidin-3-yl)methoxy)-5-methoxypyrido[4,3-*d*]pyrimidin-7-yl)-5-ethynyl-6-fluoronaphthalen-2-ol | LCMS (m/z): 645.3 (M + H). $^{1}$H NMR (400 MHz, Methanol-$d_4$) δ 7.82 (dd, *J* = 9.2, 5.7 Hz, 1H), 7.32 - 7.25 (m, 2H), 7.24 - 7.19 (m, 1H), 6.78 - 6.46 (m, 1H), 4.61 (dd, *J* = 10.5, 2.7 Hz, 1H), 4.44 - 4.06 (m, 3H), 3.99 (s, 3H), 3.64 - 3.42 (m, 4H), 3.28 - 3.26 (m, 1H), 2.91 - 2.84 (m, 1H), 2.83 - 2.75 (m, 1H), 2.71 - 2.61 (m, 1H), 2.33 - 2.20 (m, 4H), 2.05 - 1.91 (m, 2H), 1.88 - 1.61 (m, 4H), 1.19 (s, 3H). $^{19}$F NMR (376 MHz, Methanol-$d_4$) δ -111.90, -139.31, - 150.93. |
| 266 | 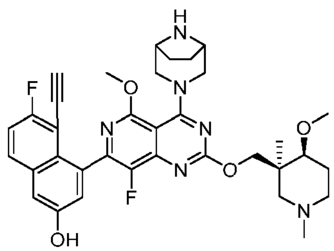 | 4-(4-(1*R*,5*S*)-3,8-diazabicyclo[3.2.1]octan-3-yl)-8-fluoro-5-methoxy-2-(3*S*,4*S*)-4-methoxy-1,3-dimethylpiperidin-3-yl)methoxy)pyrido[4,3-*d*]pyrimidin-7-yl)-5-ethynyl-6-fluoronaphthalen-2-ol | LCMS (m/z): 645.3 (M + H). |

(continued)

| Ex. | Structure | Name | Characterization data |
|---|---|---|---|
| 267 | 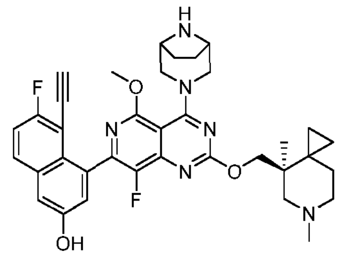 | 4-(4-((1*R*,5*S*)-3,8-diazabicyclo[3.2.1]octan-3-yl)-2-(((*S*)-4,6-dimethyl-6-azaspiro[2.5]octan-4-yl)methoxy)-8-fluoro-5-methoxypyrido[4,3-*d*]pyrimidin-7-yl)-5-ethynyl-6-fluoronaphthalen-2-ol | LCMS (m/z): 641.3 (M + H). |
| 268 | 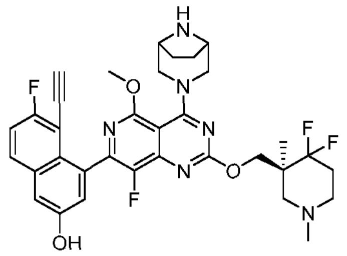 | 4-(4-((1*R*,5*S*)-3,8-diazabicyclo[3.2.1]octan-3-yl)-2-(((*S*)-4,4-difluoro-1,3-dimethylpiperidin-3-yl)methoxy)-8-fluoro-5-methoxypyrido[4,3-*d*]pyrimidin-7-yl)-5-ethynyl-6-fluoronaphthalen-2-ol | LCMS (m/z): 651.3 (M + H). |
| 269 | 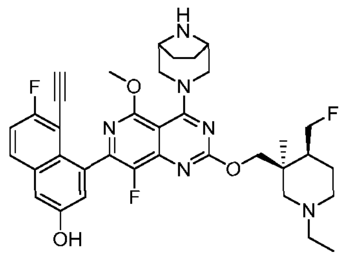 | 4-(4-((1*R*,5*S*)-3,8-diazabicyclo[3.2.1]octan-3-yl)-2-(((3*S*,4*S*)-1-ethyl-4-(fluoromethyl)-3-methylpiperidin-3-yl)methoxy)-8-fluoro-5-methoxypyrido[4,3-*d*]pyrimidin-7-yl)-5-ethynyl-6-fluoronaphthalen-2-ol | LCMS (m/z): 661.3 (M + H). |
| 270 | 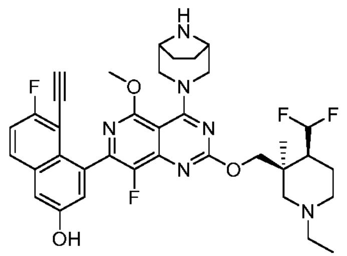 | 4-(4-((1*R*,5*S*)-3,8-diazabicyclo[3.2.1]octan-3-yl)-2-(((3*S*,4*S*)-4-(difluoromethyl)-1-ethyl-3-methylpiperidin-3-yl)methoxy)-8-fluoro-5-methoxypyrido[4,3-*d*]pyrimidin-7-yl)-5-ethynyl-6-fluoronaphthalen-2-ol | LCMS (m/z): 679.3 (M + H). |

(continued)

| Ex. | Structure | Name | Characterization data |
|---|---|---|---|
| 271 | 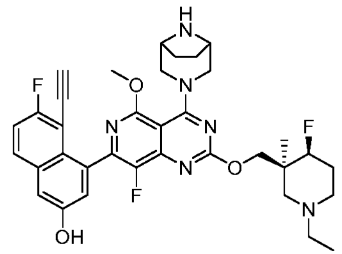 | 4-(4-((1*R*,5*S*)-3,8-diazabicyclo[3.2.1]octan-3-yl)-2-(((3*S*,4*S*)-1-ethyl-4-fluoro-3-methylpiperidin-3-yl)methoxy)-8-fluoro-5-methoxypyrido[4,3-*d*]pyrimidin-7-yl)-5-ethynyl-6-fluoronaphthalen-2-ol | LCMS (m/z): 647.3 (M + H). |
| 272 | 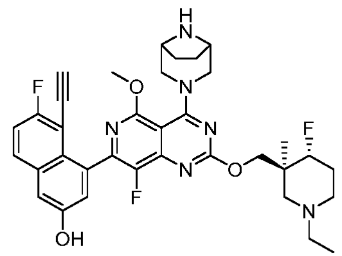 | 4-(4-((1*R*,5*S*)-3,8-diazabicyclo[3.2.1]octan-3-yl)-2-(((3*S*,4*R*)-1-ethyl-4-fluoro-3-methylpiperidin-3-yl)methoxy)-8-fluoro-5-methoxypyrido[4,3-*d*]pyrimidin-7-yl)-5-ethynyl-6-fluoronaphthalen-2-ol | LCMS (m/z): 647.3 (M + H). |
| 273 | 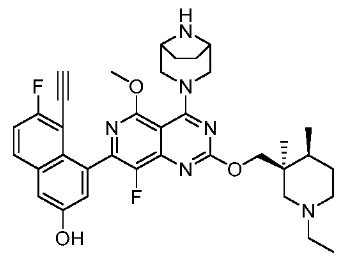 | 4-(4-((1*R*,5*S*)-3,8-diazabicyclo[3.2.1]octan-3-yl)-2-(((3*S*,4*S*)-1-ethyl-3,4-dimethylpiperidin-3-yl)methoxy)-8-fluoro-5-methoxypyrido[4,3-*d*]pyrimidin-7-yl)-5-ethynyl-6-fluoronaphthalen-2-ol | LCMS (m/z): 643.3 (M + H). |
| 274 | 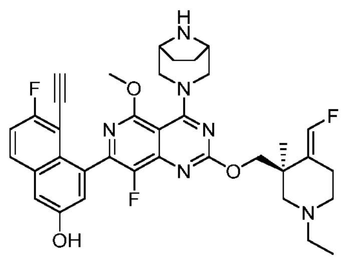 | 4-(4-((1*R*,5*S*)-3,8-diazabicyclo[3.2.1]octan-3-yl)-2-(((*S*,*E*)-1-ethyl-4-(fluoromethylene)-3-methylpiperidin-3-yl)methoxy)-8-fluoro-5-methoxypyrido[4,3-*d*]pyrimidin-7-yl)-5-ethynyl-6-fluoronaphthalen-2-ol | LCMS (m/z): 659.3 (M + H). |

(continued)

| Ex. | Structure | Name | Characterization data |
|---|---|---|---|
| 275 | 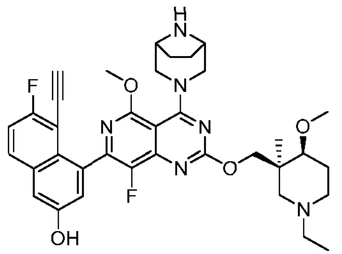 | 4-(4-((1*R*,5*S*)-3,8-diazabicyclo[3.2.1]octan-3-yl)-2-(((3*S*,4*S*)-1-ethyl-4-methoxy-3-methylpiperidin-3-yl)methoxy)-8-fluoro-5-methoxypyrido[4,3-*d*]pyrimidin-7-yl)-5-ethynyl-6-fluoronaphthalen-2-ol | LCMS (m/z): 659.3 (M + H). |
| 276 | 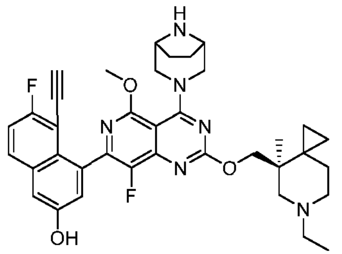 | 4-(4-((1*R*,5*S*)-3,8-diazabicyclo[3.2.1]octan-3-yl)-2-(((*S*)-6-ethyl-4-methyl-6-azaspiro[2.5]octan-4-yl)methoxy)-8-fluoro-5-methoxypyrido[4,3-*d*]pyrimidin-7-yl)-5-ethynyl-6-fluoronaphthalen-2-ol | LCMS (m/z): 655.3 (M + H). |
| 277 | 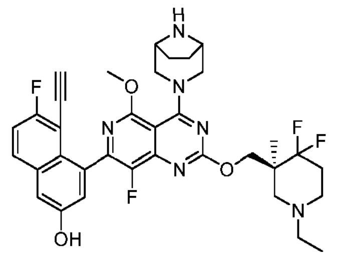 | 4-(4-((1*R*,5*S*)-3,8-diazabicyclo[3.2.1]octan-3-yl)-2-(((*S*)-1-ethyl-4,4-difluoro-3-methylpiperidin-3-yl)methoxy)-8-fluoro-5-methoxypyrido[4,3-*d*]pyrimidin-7-yl)-5-ethynyl-6-fluoronaphthalen-2-ol | LCMS (m/z): 665.3 (M + H). |
| 278 | 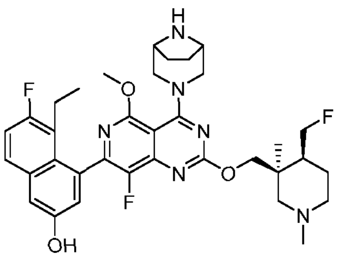 | 4-(4-((1*R*,5*S*)-3,8-diazabicyclo[3.2.1]octan-3-yl)-8-fluoro-2-(((3*S*,4*S*)-4-(fluoromethyl)-1,3-dimethylpiperidin-3-yl)methoxy)-5-methoxypyrido[4,3-*d*]pyrimidin-7-yl)-5-ethyl-6-fluoronaphthalen-2-ol | LCMS (m/z): 651.3 (M + H). |

(continued)

| Ex. | Structure | Name | Characterization data |
|---|---|---|---|
| 279 | 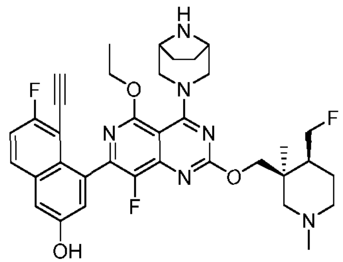 | 4-(4-((1*R*,5*S*)-3,8-diazabicyclo[3.2.1]octan-3-yl)-5-ethoxy-8-fluoro-2-(((3*S*,4*S*)-4-(fluoromethyl)-1,3-dimethylpiperidin-3-yl)methoxy)pyrido[4,3-*d*]pyrimidin-7-yl)-5-ethynyl-6-fluoronaphthalen-2-ol | LCMS (m/z): 661.3 (M + H). |
| 280 | 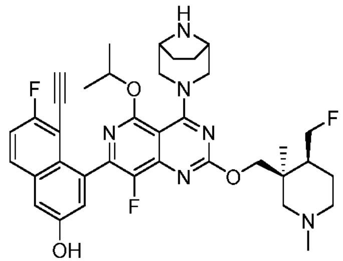 | 4-(4-((1*R*,5*S*)-3,8-diazabicyclo[3.2.1]octan-3-yl)-8-fluoro-2-(((3*S*,4*S*)-4-(fluoromethyl)-1,3-dimethylpiperidin-3-yl)methoxy)-5-isopropoxypyrido[4,3-*d*]pyrimidin-7-yl)-5-ethynyl-6-fluoronaphthalen-2-ol | LCMS (m/z): 675.3 (M + H). |
| 281 | 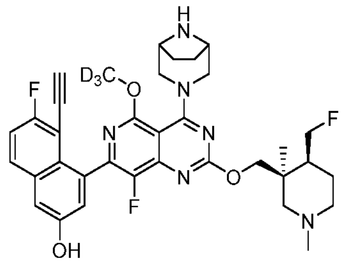 | 4-(4-((1*R*,5*S*)-3,8-diazabicyclo[3.2.1]octan-3-yl)-8-fluoro-2-(((3*S*,4*S*)-4-(fluoromethyl)-1,3-dimethylpiperidin-3-yl)methoxy)-5-(methoxy-$d_3$) pyrido[4,3-*d*]pyrimidin-7-yl)-5-ethynyl-6-fluoronaphthalen-2-ol | LCMS (m/z): 650.3 (M + H). |
| 282 | 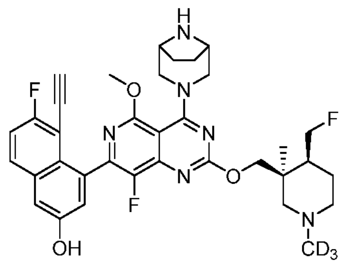 | 4-(4-((1*R*,5*S*)-3,8-diazabicyclo[3.2.1]octan-3-yl)-8-fluoro-2-(((3*S*,4*S*)-4-(fluoromethyl)-3-methyl-1-(methyl-$d_3$)piperidin-3-yl)methoxy)-5-methoxypyrido[4,3-*d*]pyrimidin-7-yl)-5-ethynyl-6-fluoronaphthalen-2-ol | LCMS (m/z): 650.3 (M + H). |

(continued)

| Ex. | Structure | Name | Characterization data |
|---|---|---|---|
| 283 | 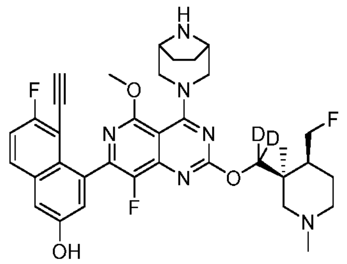 | 4-(4-((1*R*,5*S*)-3,8-diazabicyclo[3.2.1]octan-3-yl)-8-fluoro-2-(((3*S*,4*S*)-4-(fluoromethyl)-1,3-dimethylpiperidin-3-yl)methoxy-$d_2$)-5-methoxypyrido[4,3-*d*]pyrimidin-7-yl)-5-ethynyl-6-fluoronaphthalen-2-ol | LCMS (m/z): 649.3 (M + H). |
| 284 | 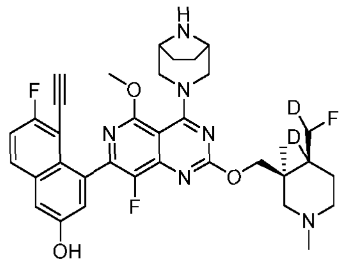 | 4-(4-((1*R*,5*S*)-3,8-diazabicyclo[3.2.1]octan-3-yl)-8-fluoro-2-(((3*S*,4*S*)-4-((*S*)-fluoromethyl-*d*)-1,3-dimethylpiperidin-3-yl-4-*d*) methoxy)-5-methoxypyrido[4,3-*d*] pyrimidin-7-yl)-5-ethynyl-6-fluoronaphthalen-2-ol | LCMS (m/z): 649.3 (M + H). |
| 285 | 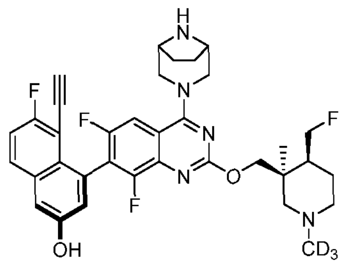 | 4-(4-(1*R*,5*S*)-3,8-diazabicyclo[3.2.1]octan-3-yl)-6,8-difluoro-2-(3*S*,4*S*)-4-(fluoromethyl)-3-methyl-1-(methyl-$d_3$)piperidin-3-yl)methoxy)quinazolin-7-yl)-5-ethynyl-6-fluoronaphthalen-2-ol | LCMS (m/z): 637.3 (M + H). |
| 286 | 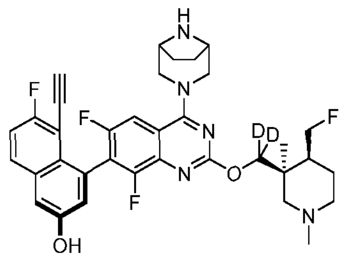 | 4-(4-(1*R*,5*S*)-3,8-diazabicyclo[3.2.1]octan-3-yl)-6,8-difluoro-2-(3*S*,4*S*)-4-(fluoromethyl)-1,3-dimethylpiperidin-3-yl)methoxyl-$d_2$)quinazolin-7-yl)-5-ethynyl-6-fluoronaphthalen-2-ol | LCMS (m/z): 636.3 (M + H). |

(continued)

| Ex. | Structure | Name | Characterization data |
|---|---|---|---|
| 287 | 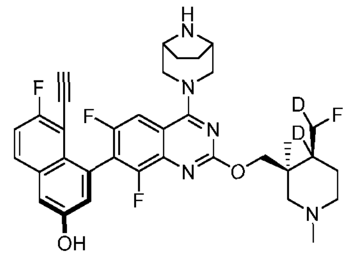 | 4-(4-((1*R*,5*S*)-3,8-diazabicyclo[3.2.1]octan-3-yl)-6,8-difluoro-2-(((3*S*,4*S*)-4-((*S*)-fluoromethyl-d)-1,3-dimethylpiperidin-3-yl-4-*d*) methoxy)quinazolin-7-yl)-5-ethynyl-6-fluoronaphthalen-2-ol | LCMS (m/z): 636.3 (M + H). |
| 289 | 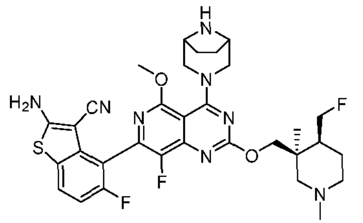 | 4-(4-((1*R*,5*S*)-3,8-diazabicyclo[3.2.1]octan-3-yl)-8-fluoro-2-(((3*S*,4*S*)-4-(fluoromethyl)-1,3-dimethylpiperidin-3-yl)methoxy)-5-methoxypyrido[4,3-*d*]pyrimidin-7-yl)-2-amino-5-fluorobenzo[*b*]thiophen-3-nitrile | LCMS (m/z): 653.3 (M + H). |
| 290 | 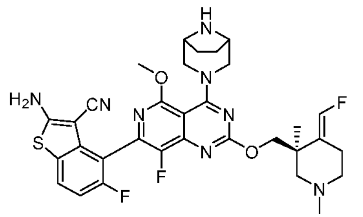 | 4-(4-((1*R*,5*S*)-3,8-diazabicyclo[3.2.1]octan-3-yl)-8-fluoro-2-(((*S*,*E*)-4-(fluoromethylene)-1,3-dimethylpiperidin-3-yl) methoxy)-5-methoxypyrido[4,3-*d*]pyrimidien-7-yl)-2-amino-5-fluorobenzo[*b*]thiophen-3-nitrile | LCMS (m/z): 651.3 (M + H). |
| 291 | 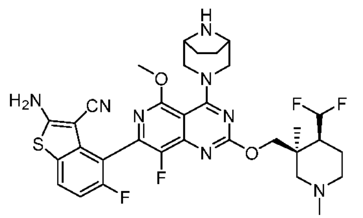 | 4-(4-((1*R*,5*S*)-3,8-diazabicyclo[3.2.1]octan-3-yl)-2-(((3*S*,4*S*)-4-(difluoromethyl)-1,3-dimethylpiperidin-3-yl)methoxy)-8-fluoro-5-methoxypyrido[4,3-*d*]pyrimidin-7-yl)-2-amino-5-fluorobenzo[*b*] thiophen-3-nitrile | LCMS (m/z): 671.3 (M + H). $^1$H NMR (400 MHz, DMSO-$d_6$) δ 7.95 (s, 2H), 7.82 (dd, *J* = 8.7, 5.0 Hz, 1H), 7.16 - 7.07 (m, 1H), 6.30 (t, *J* = 56.0 Hz, 1H), 4.55 - 4.32 (m, 3H), 3.93 (s, 3H), 3.78 - 3.66 (m, 1H), 3.54 - 3.45 (m, 3H), 3.28 - 3.23 (m, 1H), 2.88 - 2.74 (m, 2H), 2.11 (s, 3H), 1.85 - 1.44 (m, 9H), 1.11 (s, 3H). $^{19}$F NMR (376 MHz, DMSO-$d_6$) δ -116.10 ~-119.42, -120.04, - 149.52. |

(continued)

| Ex. | Structure | Name | Characterization data |
|---|---|---|---|
| 292 | 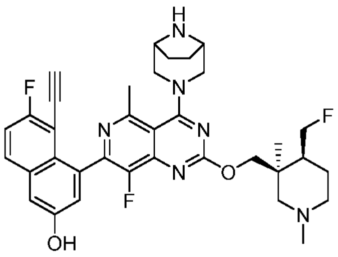 | 4-(4-((1*R*,5*S*)-3,8-diazabicyclo[3.2.1]octan-3-yl)-8-fluoro-2-(((3*S*,4*S*)-4-(fluoromethyl)-1,3-dimethylpiperidin-3-yl)methoxy)-5-methylpyrido[4,3-*d*]pyrimidin-7-yl)-5-ethynyl-6-fluoronaphthalen-2-ol | LCMS (m/z): 631.3 (M + H). |
| 293 | 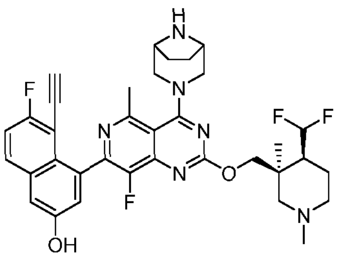 | 4-(4-((1*R*,5*S*)-3,8-diazabicyclo[3.2.1]octan-3-yl)-2-(((3*S*,4*S*)-4-(difluoromethyl)-1,3-dimethylpiperidin-3-yl)methoxy)-8-fluoro-5-methylpyrido[4,3-*d*]pyrimidin-7-yl)-5-ethynyl-6-fluoronaphthalen-2-ol | LCMS (m/z): 649.3 (M + H). |
| 294 | 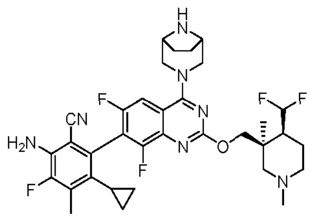 | 2-(4-((1*R*,5*S*)-3,8-diazabicyclo[3.2.1]octan-3-yl)-2-(((3*S*,4*S*)-4-(difluoromethyl)-1,3-dimethylpiperidin-3-yl)methoxy)-6,8-difluoroquinazolin-7-yl)-6-amino-3-cyclopropyl-5-fluoro-4-methylbenzonitrile | LCMS (m/z): 656.3 (M + H). |
| 295 | 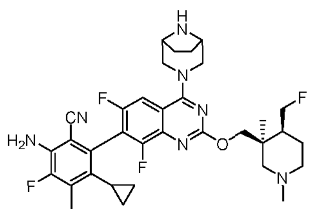 | 2-(4-((1*R*,5*S*)-3,8-diazabicyclo[3.2.1]octan-3-yl)-6,8-difluoro-2-(((3*S*,4*S*)-4-(fluoromethyl)-1,3-dimethylpiperidin-3-yl) methoxy) quinazolin-7-yl)-6-amino-3-cyclopropyl-5-fluoro-4-methylbenzonitrile | LCMS (m/z): 638.3 (M + H). |

(continued)

| Ex. | Structure | Name | Characterization data |
|---|---|---|---|
| 296 | 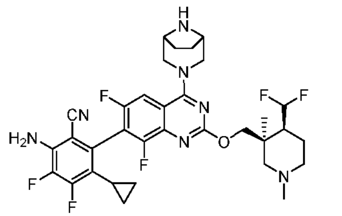 | 2-(4-((1*R*,5*S*)-3,8-diazabicyclo[3.2.1]octan-3-yl)-2-(((3*S*,4*S*)-4-(difluoromethyl)-1,3-dimethylpiperidin-3-yl)methoxy)-6,8-difluoroquinazolin-7-yl)-6-amino-3-cyclopropyl-4,5-difluorobenzonitrile | LCMS (m/z): 660.3 (M + H). |
| 297 | 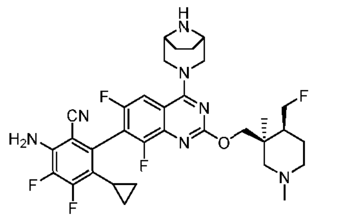 | 2-(4-((1*R*,5*S*)-3,8-diazabicyclo[3.2.1]octan-3-yl)-6,8-difluoro-2-(((3*S*,4*S*)-4-(fluoromethyl)-1,3-dimethylpiperidin-3-yl) methoxy) quinazolin-7-yl)-6-amino-3-cyclopropyl-4,5-difluorobenzonitrile | LCMS (m/z): 642.3 (M + H). |
| 299 | 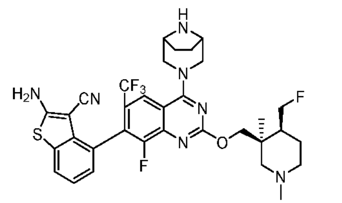 | 4-(4-((1*R*,5*S*)-3,8-diazabicyclo[3.2.1]octan-3-yl)-8-fluoro-2-(((3*S*,4*S*)-4-(fluoromethyl)-1,3-dimethylpiperidin-3-yl)methoxy)-6-(trifluoromethyl)quinazolin-7-yl)-2-aminobenzo[*b*]thiophen-3-nitrile | LCMS (m/z): 672.3 (M + H). |
| 300 | 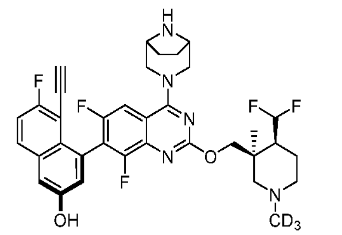 | 4-(4-((1*R*,5*S*)-3,8-diazabicyclo[3.2.1]octan-3-yl)-2-(((3*S*,4*S*)-4-(difluoromethyl)-3-methyl-1-(methyl-$d_3$) piperidin-3-yl)methoxy)-6,8-difluoroquinazolin-7-yl)-5-ethynyl-6-fluoronaphthalen-2-ol | LCMS (m/z): 655.3 (M + H). |

(continued)

| Ex. | Structure | Name | Characterization data |
|---|---|---|---|
| 301 | 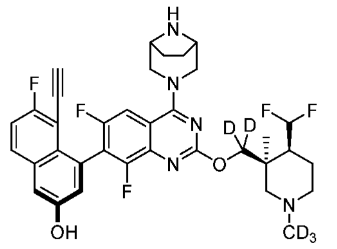 | 4-(4-((1*R*,5*S*)-3,8-diazabicyclo[3.2.1]octan-3-yl)-2-(((3*S*,4*S*)-4-(difluoromethyl)-3-methyl-1-(methyl-$d_3$) piperidin-3-yl)methoxy-$d_2$)-6,8-difluoroquinazolin-7-yl)-5-ethynyl-6-fluoronaphthalen-2-ol | LCMS (m/z): 657.3 (M + H). |
| 302 | 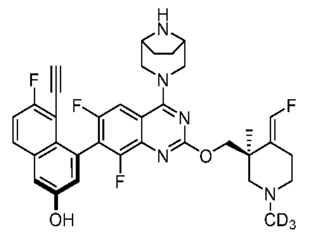 | 4-(4-((1*R*,5*S*)-3,8-diazabicyclo[3.2.1]octan-3-yl)-6,8-difluoro-2-(((*S*,*E*)-4-(fluoromethylene)-3-methyl-1-(methyl-$d_3$)piperidin-3-yl)methoxy)quinazolin-7-yl)-5-ethynyl-6-fluoronaphthalen-2-ol | LCMS (m/z): 635.3 (M + H). |
| 303 | 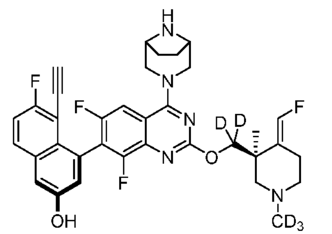 | 4-(4-((1*R*,5*S*)-3,8-diazabicyclo[3.2.1]octan-3-yl)-6,8-difluoro-2-(((*S*,*E*)-4-(fluoromethylene)-3-methyl-1-(methyl-$d_3$)piperidin-3-yl)methoxy-$d_2$)quinazolin-7-yl)-5-ethynyl-6-fluoronaphthalen-2-ol | LCMS (m/z): 637.3 (M + H). |

Example 304

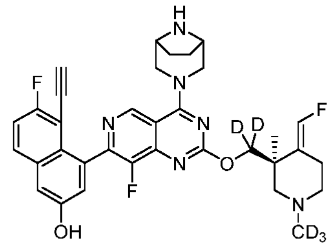

4-(4-((1*R*,5*S*)-3,8-diazabicyclo[3.2.1]octan-3-yl)-8-fluoro-2-(((*S,E*)-4-(fluoromethylene)-3-methyl-1-(methyl-$d_3$)piperidin-3-yl)methoxy-$d_2$)pyrido[4,3-*d*]pyrimidin-7-yl)-5-ethynyl-6-fluoronaphthalen-2-ol

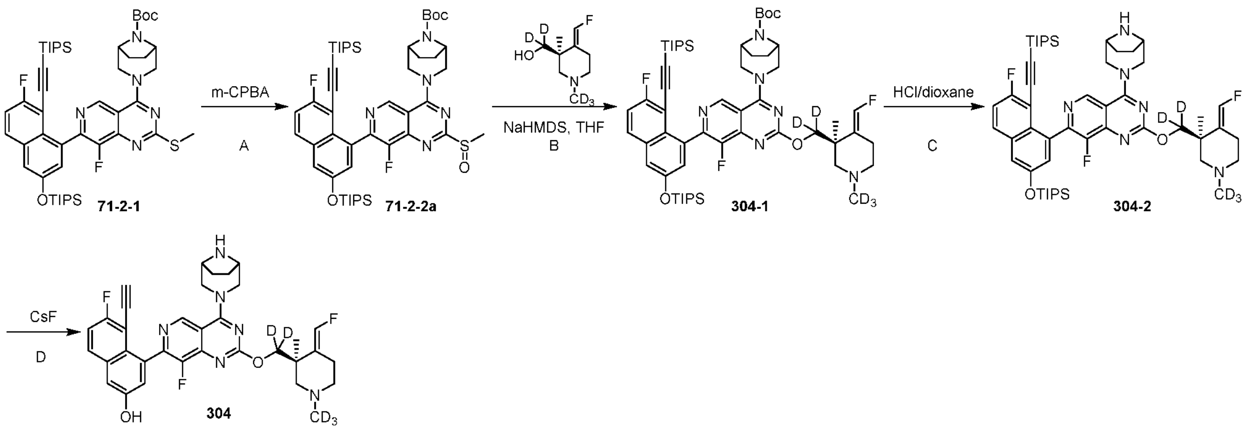


**Step A:** *tert*-butyl (1*R*,5*S*)-3-(8-fluoro-7-(7-fluoro-8-((triisopropylsilyl)ethynyl)-3-((triisopropylsilyl)oxy)naphthalen-1-yl)-2-(methylsulfinyl)pyrido[4,3-*d*]pyrimidin-4-yl)-3,8-diazabicyclo[3.2.1]octane-8-carboxylate

Under an ice-bath, m-chloroperbenzoic acid (1.48 g, 7.3 mmol, 85% purity) was added to a solution of *tert-butyl* (1R,5S)-3-(8-fluoro-7-(7-fluoro-8-((triisopropylsilyl)ethynyl)-3-((triisopropylsilyl)oxy)naphthalen-1-yl)-2-(methylthio)pyrido[4,3-d]pyrimidin-4-yl)-3,8-diazabicyclo[3.2.1]octane-8-carboxylate (6.0 g, 6.65 mmol) in dichloromethane (60.0 mL), and the resulting mixture was stirred at the same temperature for 1.5 h. After the reaction was completed, the system was warmed to room temperature, quenched by adding saturated sodium bicarbonate solution (100 mL), and continuously stirred for 10 min. The mixture was extracted with dichloromethane (50 mL × 3). The combined organic phases were washed with saturated sodium chloride solution (100 mL), dried over anhydrous sodium sulfate, filtered, and concentrated under reduced pressure to obtain *tert*-butyl (1*R*, 5*S*)-3-(8-fluoro-7-(7-fluoro-8-((triisopropylsilyl)ethynyl)-3-((triisopropylsilyl)oxy)naphthalen-1-yl)-2-(methylsulfinyl) pyrido[4,3-*d*]pyrimidin-4-yl)-3,8-diazabicyclo[3.2.1]octane-8-carboxylate (6.1 g , yield 99%). LCMS (m/z):[M + H]918.4.

**Step B:** *tert*-butyl (1*R*,5*S*)-3-(8-fluoro-7-(7-fluoro-8-((triisopropylsilyl)ethynyl)-3-((triisopropylsilyl)**oxy**)naphthalen-1-yl)-2-(((*S,E*)-4-(fluoromethylene)-3-methyl-1-(methyl-$d_3$) piperidin-3-yl)methoxy-$d_2$)pyrido[4,3-*d*]pyrimidin-4-yl)-3,8-diazabicyclo[3.2.1]octane-8-carboxylate

*tert*-butyl (1*R*,5*S*)-3-(8-fluoro-7-(7-fluoro-8-((triisopropylsilyl)ethynyl)-3-((triisopropylsilyl)oxy)naphthalen-1-yl)-2-(methylsulfinyl)pyrido[4,3-*d*]pyrimidin-4-yl)-3,8-diazabicyclo[3.2.1]octane-8-carboxylate (300 mg, 0.33 mmol) and (*S,E*)-(4-(fluoromethylene)-3-methyl-1-(methyl-$d_3$)piperidin-3-yl)methylene-$d_2$-ol (76.13 mg, 0.42 mmol) were dissolved in 5 mL of anhydrous THF. The reaction was cooled to -40 °C in a dry ice/acetonitrile bath, and sodium bis(trimethylsilyl) amide (0.65 mL, 0.65 mmol, 1 M in THF) was added. The resulting mixture was stirred at -40 °C for 30 min. After the reaction was completed (monitored by LCMS), 20 mL of saturated aqueous ammonium chloride was added to quench the reaction, and the resulting mixture was extracted with ethyl acetate. The combined organic phases were dried over anhydrous

sodium sulfate, filtered, and concentrated to obtain the crude product, *tert*-butyl (1*R*,5*S*)-3-(8-fluoro-7-(7-fluoro-8-((triisopropylsilyl)ethynyl)-3-((triisopropylsilyl)oxy)naphthalen-1-yl)-2-(((*S,E*)-4-(fluoromethylene)-3-methyl-1-(methyl-$d_3$) piperidin-3-yl)methoxy-$d_2$) pyrido[4,3-*d*]pyrimidin-4-yl)-3, 8-diazabicyclo[3.2.1]octane-8-carboxylate (375 mg, yield 67%, purity 60%) as a yellow oil, which was used in the next reaction without further purification. LCMS (m/z): 516.8 (0.5M + H).

**Step C:** 4-((1*R*,5*S*)-3,8-diazabicyclo[3.2.1]oct-3-yl)-8-fluoro-7-(7-fluoro-8-((triisopropylsilyl)ethynyl)-3-((triisopropylsilyl)oxy)naphthalen-1-yl)-2-(((*S*,*E*)-4-(fluoromethylene)-3-methyl-1-(methyl-$d_3$)piperidin-3-yl)methoxy-$d_2$)pyrido[4,3-*d*]pyrimidine

At room temperature, *tert*-butyl (1*R*,5*S*)-3-(8-fluoro-7-(7-fluoro-8-((triisopropylsilyl)ethynyl)-3-((triisopropylsilyl)oxy)naphthalen-1-yl)-2-(((*S,E*)-4-(fluoromethylene)-3-methyl-1-(methyl-$d_3$)piperidin-3-yl)methoxy-$d_2$)pyrido[4,3-*d*]pyrimidin-4-yl)-3,8-diazabicyclo[3.2.1]octane-8-carboxylate (375 mg, purity 60%, 0.22 mmol) was dissolved in hydrochloric acid/dioxane solution (5 mL), and the reaction was stirred at room temperature for 1 h. After the reaction was completed (monitored by LCMS), the reaction was concentrated to dryness. The crude product was dissolved by adding 20 mL of ethyl acetate and 20 mL of water. Saturated sodium bicarbonate solution was added to adjust the aqueous phase to neutral. The resulting reaction mixture was extracted with ethyl acetate. The organic phase was dried over anhydrous sodium sulfate, filtered, and concentrated to obtain the crude product 4-((1*R*,5*S*)-3,8-diazabicyclo[3.2.1]oct-3-yl)-8-fluoro-7-(7-fluoro-8-((triisopropylsilyl)ethynyl)-3-((triisopropylsilyl)oxy)naphthalen-1-yl)-2-(((*S,E*)-4-(fluoromethylene)-3-methyl-1-(methyl-$d_3$)piperidin-3-yl)methoxy-$d_2$)pyrido[4,3-*d*]pyrimidine (300 mg, purity 60%, yield 89%) as a yellow solid, which was used directly in the next reaction without further purification. LCMS (m/z): 933.0 (M + H).

**Step D:** 4-(4-((1*R*,5*S*)-3,8-diazabicyclo[3.2.1]octan-3-yl)-8-fluoro-2-((*S*,*E*)-4-(fluoromethylene)-3-methyl-1-(methyl-$d_3$) piperidin-3-yl)methoxy-$d_2$)pyrido[4,3-*d*]pyrimidin-7-yl)-5-ethynyl-6-fluoronaphthalen-2-ol

At room temperature, 4-((1*R*,5*S*)-3,8-diazabicyclo[3.2.1]oct-3-yl)-8-fluoro-7-(7-fluoro-8-((triisopropylsilyl)ethynyl)-3-((triisopropylsilyl)oxy)naphthalen-1-yl)-2-(((*S*,*E*)-4-(fluoromethylene)-3-methyl-1-(methyl-$d_3$)piperidin-3-yl)methoxy-$d_2$)pyrido[4,3-*d*]pyrimidine (300 mg, purity 60%, 0.19 mmol) was dissolved in DMF (5 mL), and CsF (88 mg, 0.58 mmol) was added. The resulting mixture was stirred at 50 °C for 0.5 h. After the reaction was completed (monitored by LCMS), the mixture was purified by pre-HPLC (C18, CAN/(10 mM $NH_4HCO_3$) = 5-95%) to obtain 4-(4-((1*R*,5*S*)-3,8-diazabicyclo[3.2.1]octan-3-yl)-8-fluoro-2-((*S*,*E*)-4-(fluoromethylene)-3-methyl-1-(methyl-$d_3$)piperidin-3-yl)methoxy-$d_2$) pyrido[4,3-*d*]pyrimidin-7-yl)-5-ethynyl-6-fluoronaphthalen-2-ol (44 mg, yield 37%). LCMS (m/z): 620.3 (M + H). $^1H$ NMR (400 MHz, DMSO-$d_6$) δ 10.16 (s, 1H), 9.04 (s, 1H), 7.98 (dd, *J* = 9.2, 5.9 Hz, 1H), 7.51 - 7.43 (m, 1H), 7.39 (d, *J* = 2.6 Hz, 1H), 7.20 - 7.14 (m, 1H), 6.85 - 6.58 (m, 1H), 4.54 - 4.40 (m, 1H), 4.35 - 4.25 (m, 1H), 3.97 - 3.88 (m, 1H), 3.69 - 3.51 (m, 4H), 2.75 - 2.64 (m, 2H), 2.56 - 2.51 (m, 1H), 2.27 - 2.11 (m, 1H), 1.94 - 1.79 (m, 2H), 1.74 - 1.60 (m, 4H), 1.10 (d, *J* = 1.9 Hz, 3H). $^{19}F$ NMR (376 MHz, DMSO-$d_6$) δ-110.75, -138.81, - 140.01.

| Ex. | Structure | Name | Characterization data |
|---|---|---|---|
| 305 | 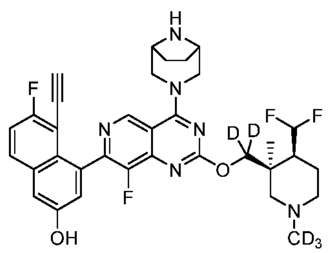 | 4-(4-((1*R*,5*S*)-3,8-diazabicyclo [3.2.1]octan-3-yl)-2-(((3*S*,4*S*)-4-(difluoromethyl)-3-methyl-1-(methyl-$d_3$) piperidin-3-yl)methoxy-$d_2$)-8-fluoropyrido[4,3-*d*]pyrimidin-7-yl)-5-ethynyl-6-fluoronaphthalen-2-ol | LCMS (m/z): 640.3 (M + H). |

(continued)

| Ex. | Structure | Name | Characterization data |
|---|---|---|---|
| 306 | 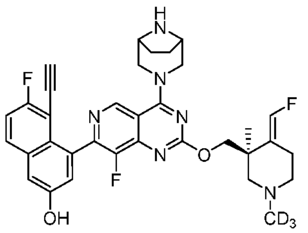 | 4-(4-((1*R*,5*S*)-3,8-diazabicyclo [3.2.1]octan-3-yl)-8-fluoro-2-(((*S,E*)-4-(fluoromethylene)-3-methyl-1-(methyl-$d_3$)piperidin-3-yl) methoxy)pyrido[4,3-*d*]pyrimidin-7-yl)-5-ethynyl-6-fluoronaphthalen-2-ol | LCMS (m/z): 618.3 (M + H). $^1$H NMR (400 MHz, DMSO-$d_6$) δ 10.16 (s, 1H), 9.03 (s, 1H), 7.97 (dd, *J* = 9.2, 5.9 Hz, 1H), 7.50 - 7.43 (m, 1H), 7.39 (d, *J* = 2.6 Hz, 1H), 7.20 - 7.16 (m, 1H), 6.82 (d, *J* = 5.8 Hz, 0.5H), 6.60 (d, *J* = 5.8 Hz, 0.5H), 4.64 - 4.53 (m, 1H), 4.52 - 4.39 (m, 1H), 4.36 - 4.23 (m, 2H), 3.96 - 3.88 (m, 1H), 3.68 - 3.51 (m, 4H), 2.74 - 2.65 (m, 2H), 2.55 - 2.50 (m, 1H), 2.27 - 2.13 (m, 1H), 1.94 - 1.79 (m, 2H), 1.66 (s, 4H), 1.10 (d, *J* = 1.9 Hz, 3H). $^{19}$F NMR (376 MHz, DMSO-$d_6$) δ - 110.76, -138.80, - 140.05. |
| 307 | 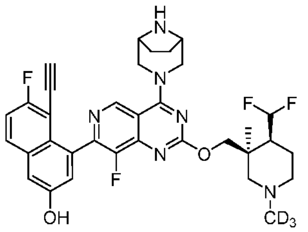 | 4-(4-((1*R*,5*S*)-3,8-diazabicyclo [3.2.1]octan-3-yl)-2-(((3*S*,4*S*)-4-(difluoromethyl)-3-methyl-1-(methyl-$d_3$)piperidin-3-yl)methoxy)-8-fluoropyrido [4,3-*d*]pyrimidin-7-yl)-5-ethynyl-6-fluoronaphthalen-2-ol | LCMS (m/z): 638.3 (M + H). |
| 308 | 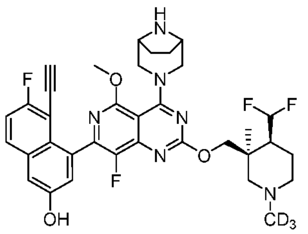 | 4-(4-((1*R*,5*S*)-3,8-diazabicyclo [3.2.1]octan-3-yl)-2-(((3*S*,4*S*)-4-(difluoromethyl)-3-methyl-1-(methyl-$d_3$)piperidin-3-yl)methoxy)-8-fluoro-5-methoxypyrido[4,3-*d*]pyrimidin-7-yl)-5-ethynyl-6-fluoronaphthalen-2-ol | LCMS (m/z): 668.3 (M + H). |
| 309 | 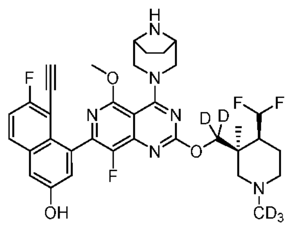 | 4-(4-((1*R*,5*S*)-3,8-diazabicyclo [3.2.1]octan-3-yl)-2-(((3*S*,4*S*)-4-(difluoromethyl)-3-methyl-1-(methyl-$d_3$)piperidin-3-yl)methoxy-$d_2$)-8-fluoro-5-methoxypyrido[4,3-*d*]pyrimidin-7-yl)-5-ethynyl-6-fluoronaphthalen-2-ol | LCMS (m/z): 670.3 (M + H). |
| 310 | 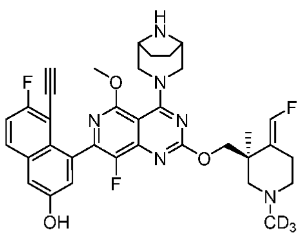 | 4-(4-((1*R*,5*S*)-3,8-diazabicyclo [3.2.1]octan-3-yl)-8-fluoro-2-(((*S,E*)-4-(fluoromethylene)-3-methyl-1-(methyl-$d_3$)piperidin-3-yl) methoxy)-5-methoxyquinazolin-7-yl)-5-ethynyl-6-fluoronaphthalen-2-ol | LCMS (m/z): 648.3 (M + H). |
| 311 | 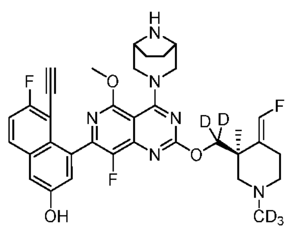 | 4-(4-((1*R*,5*S*)-3,8-diazabicyclo [3.2.1]octan-3-yl)-8-fluoro-2-(((*S*,*E*)-4-(fluoromethylene)-3-methyl-1-(methyl-$d_3$)piperidin-3-yl) methoxy-$d_2$)-5-methoxypyrido [4,3-*d*]pyrimidin-7-yl)-5-ethynyl-6-fluoronaphthalen-2-ol | LCMS (m/z): 650.3 (M + H). |

**Example 312**

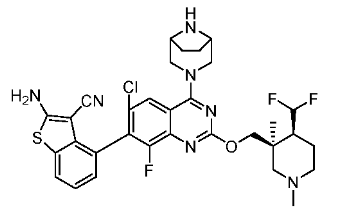

4-(4-((1*R*,5*S*)-3,8-diazabicyclo[3.2.1]octan-3-yl)-6-chloro-2-(((3*S*,4*S*)-4-(difluoromethyl)-1,3-dimethylpiperidin-3-yl) methoxy)-8-fluoroquinazolin-7-yl)-2-aminobenzo[*b*]thiophen-3-nitrile

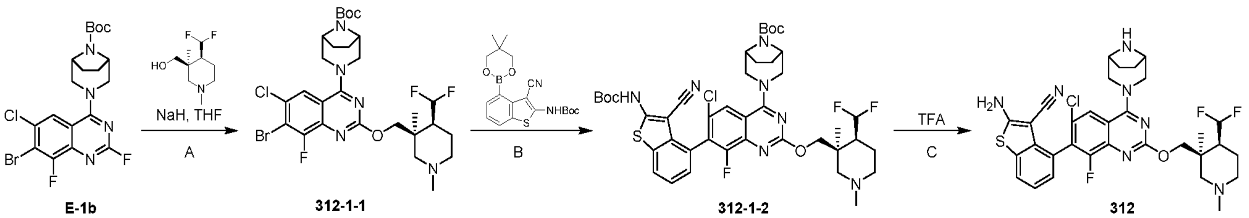


E-1b 312-1-1 312-1-2 312

**Step A:** *tert*-butyl (1*R*,5*S*)-3-(7-bromo-6-chloro-2-(((3*S*,4*S*)-4-(difluoromethyl)-1,3-dimethylpiperidin-3-yl)methoxy)-8-fluoroquinazolin-4-yl)-3,8-diazabicyclo[3.2.1]octane-8-carboxylate

Under stirring at room temperature, sodium hydride (98 mg, 2.45 mmol, 60% w/w) was added to a solution of ((3*S*,4*S*)-4-(difluoromethyl)-1,3-dimethylpiperidin-3-yl)methanol (174 mg, 0.90 mmol) in tetrahydrofuran (10 mL), and the reaction was stirred for 0.5 h at room temperature. Then, *tert*-butyl (1*R*,5*S*)-3-(7-bromo-6-chloro-2,8-difluoroquinazolin-4-yl)-3,8-diazabicyclo[3.2.1]octane-8-carboxylate (400 mg, 0.82 mmol) was added at room temperature, and the resulting mixture was stirred at room temperature for 1 h. After the reaction was completed (monitored by LCMS), saturated aqueous ammonium chloride (70 mL) was added, and the resulting mixture was extracted with EA (50 mL × 3). The combined organic phase was washed with brine (70 mL), dried over anhydrous sodium sulfate, filtered, and concentrated. The crude product was purified by FCC ($SiO_2$, EA/PE = 0-80%) to obtain *tert*-butyl (1*R*,5*S*)-3-(7-bromo-6-chloro-2-(((3*S*,4*S*)-4-(difluoromethyl)-1,3-dimethylpiperidin-3-yl)methoxy)-8-fluoroquinazolin-4-yl)-3,8-diazabicyclo[3.2.1] octane-8-carboxylate (370 mg, yield 68%) as a yellow solid. LC-MS (m/z): 662.1 (M + H) and 664.1 (M + H). **Step B:** tert-butyl (1*R*,5*S*)-3-(7-(2-((*tert*-butoxycarbonyl)amino)-3-cyanobenzo[*b*]thiophen-4-yl)-6-chloro-2-(((3*S*,4*S*)-4-(difluoromethyl)-1,3-dimethylpiperidin-3-yl)methoxy)-8-fluoroquinazoline-4-yl)-3,8-diazabicyclo[3.2.1]octane-8-carboxylate

At room temperature, bis(diphenylphosphophenyl ether) palladium (II) dichloride (18.4 mg, 0.026 mmol) and $Cs_2CO_3$ (251 mg, 0.77 mmol) were added to a solution of *tert*-butyl (1*R*,5*S*)-3-(7-bromo-6-chloro-2-(((3*S*,4*S*)-4-(difluoromethyl)-1,3-dimethylpiperidin-3-yl)methoxy)-8-fluoroquinazolin-4-yl)-3,8-diazabicyclo[3.2.1]octane-8-carboxylate (170 mg, 0.26 mmol) and *tert*-butyl (3-cyano-4-(5, 5-dimethyl-1,3, 2-dioxaborolan-2-yl) benzothiophen-2-yl) carbamate (149, mg, 0.39 mmol) in toluene (5 mL). The reaction was degassed with nitrogen, heated to 120 °C and stirred for 2 h. After the reaction was completed (monitored by LCMS), the reaction was concentrated and further purified by FCC ($SiO_2$, EA/PE = 0-80%) to give *tert-butyl* (1*R*,5*S*)-3-(7-(2-((*tert*-butoxycarbonyl)amino)-3-cyanobenzo[*b*]thiophen-4-yl)-6-chloro-2-(((3.5,4.5)-4-(difluoromethyl)-1,3-dimethylpiperidin-3-yl)methoxy)-8-fluoroquinazoline-4-yl)-3,8-diazabicyclo[3.2.1] octane-8-carboxylate (150 mg, yield 68%) as a brown solid. LC-MS (m/z): 856.3 (M + H).

**Step C:** 4-(4-((1*R*,5*S*)-3,8-diazabicyclo[3.2.1]octan-3-yl)-6-chloro-2-(((3*S*,4*S*)-4-(difluoromethyl)-1,3-dimethylpiperidin-3-yl)methoxy)-8-fluoroquinazolin-7-yl)-2-aminobenzo[*b*]thiophene-3-carbonitrile

A mixture of *tert*-butyl (1*R*,5*S*)-3-(7-(2-((*tert*-butoxycarbonyl)amino)-3-cyanobenzo[*b*]thiophene-4-yl)-6-chloro-2-(((3*S*,4*S*)-4-(difluoromethyl)-1,3-dimethylpiperidin-3-yl)methoxy)-8-fluoroquinazoline-4-yl)-3,8-diazabicyclo[3.2.1] octane-8-carboxylate (150 mg, 0.175 mmol) in DCM/TFA (V/V = 1:1, 4 mL) was stirred at room temperature for 1 h. After the reaction was monitored to be complete by LCMS, saturated aqueous sodium bicarbonate solution (50 mL) was added after concentration, and the mixture was extracted with EA (30 mL × 3). The combined organic phases were washed with

brine (70 mL), dried over anhydrous $Na_2SO_4$, filtered, and concentrated. The crude product was purified by Pre-HPLC (0.1% $NH_4HCO_3$) to obtain 4-(4-((1*R*,5*S*)-3,8-diazabicyclo[3.2.1]octan-3-yl)-6-chloro-2-(((3*S*,4*S*)-4-(difluoromethyl)-1,3-dimethylpiperidin-3-yl)methoxy)-8-fluoroquinazolin-7-yl)-2-aminobenzo[*b*]thiophene-3-nitrile (35.3 mg, yield 31%) as a white solid. $^1$H NMR (400 MHz, DMSO-$d_6$) δ 7.87 - 7.81 (m, 3H), 7.81 - 7.76 (m, 1H), 7.27 - 7.21 (m, 1H), 7.21 - 7.15 (m, 1H), 6.48 - 6.06 (m, 0H), 4.46 - 4.35 (m, 2H), 4.34 - 4.20 (m, 2H), 3.63 - 3.45 (m, 3H), 2.89 - 2.76 (m, 1H), 2.10 (s, 3H), 1.91 - 1.49 (m, 10H), 1.11 (s, 3H). $^{19}$F NMR (376 MHz, DMSO-$d_6$) δ -153.23, -115.98, -118.58, -119.32, -122.63. LCMS (m/z): 656.3 (M + H).

**Examples 312-1 and 312-2**

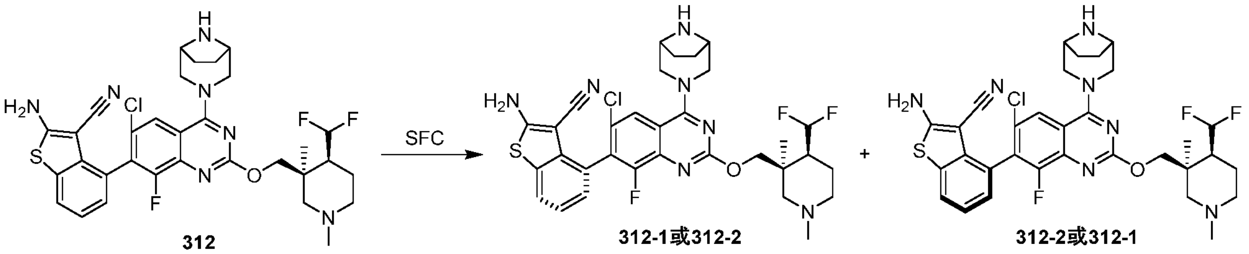


312 312-1或312-2 + 312-2或312-1

4-(4-((1*R*,5*S*)-3,8-diazabicyclo[3.2.1]octan-3-yl)-6-chloro-2-(((3*S*,4*S*)-4-(difluoromethyl)-1,3-dimethylpiperidin-3-yl) methoxy)-8-fluoroquinazolin-7-yl)-2-aminobenzo[*b*]thiophene-3 - carbonitrile (Example 312, 60 mg, 0.091 mmol) was resolved by SFC (Waters SFC 150, separation column: DAICEL CHIRALCEL ® IC, 250 * 25 mm, 10 μm; mobile phase: $CO_2$/MeOH (+ 0.1% 7.0 mol/l Ammonia) = 60/40; flow rate: 120 mL/min). The first eluted white solid isomer 1 was 312-1 (17.8 mg, with a shorter retention time). Chiral analytical method SFC-7, Rt = 2.933 min. $^1$H NMR (400 MHz, Methanol-$d_4$) δ 7.84 (s, 1H), 7.71 - 7.61 (m, 1H), 7.29 - 7.20 (m, 1H), 7.20 - 7.12 (m, 1H), 6.42 - 6.02 (m, 1H), 4.67 - 4.51 (m, 2H), 4.51 - 4.36 (m, 2H), 3.74 - 3.51 (m, 4H), 3.07 (d, *J* = 11.8 Hz, 1H), 2.96 (d, *J* = 11.0 Hz, 1H), 2.24 (s, 3H), 2.06 - 1.93 (m, 1H), 1.90 - 1.68 (m, 8H), 1.21 (s, 3H). $^{19}$F NMR (376 MHz, DMSO-$d_6$) δ-118.14, -118.90, -121.44, -122.20, -123.70. LCMS (m/z): 656.2 (M + H). The subsequently eluted white solid isomer 2 was 312-2 (19.3 mg, with a longer retention time). Chiral analytical method SFC-7, Rt = 4.343 min. $^1$H NMR (400 MHz, Methanol-$d_4$) δ 7.85 (s, 1H), 7.71 - 7.62 (m, 1H), 7.30 - 7.21 (m, 1H), 7.20 - 7.11 (m, 1H), 6.40 - 6.04 (m, 1H), 4.66 - 4.51 (m, 3H), 4.47 (d, *J* = 12.0 Hz, 1H), 3.75 - 3.57 (m, 4H), 3.12 (d, *J*= 11.9 Hz, 1H), 3.03 (d, *J* = 12.2 Hz, 1H), 2.30 (s, 3H), 2.15 - 1.99 (m, 1H), 1.99 - 1.72 (m, 8H), 1.22 (s, 3H). $^{19}$F NMR (376 MHz, DMSO-$d_6$) δ-118.46, -119.22, -121.48, -122.24, -123.76. LCMS (m/z): 656.3 (M + H).

**Example 313**

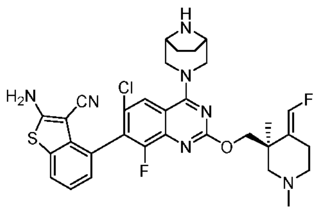

4-(4-((1*R*,5*S*)-3,8-diazabicyclo[3.2.1]octan-3-yl)-6-chloro-8-fluoro-2-(((*S*,*E*)-4-(fluoromethylene)-1,3-dimethylpiperidin-3-yl)methoxy)quinazolin-7-yl)-2-aminobenzo[b]thiophen-3-carbonitrile

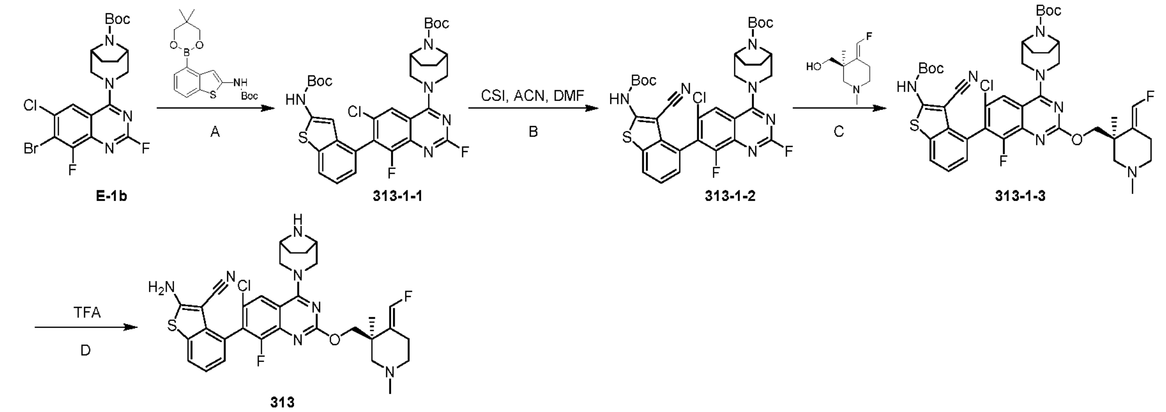


E-1b, 313-1-1, 313-1-2, 313-1-3, 313

**Step A:** *tert*-butyl (1*R*,5*S*)-3-(7-(2-((tert-butoxycarbonyl)amino)benzo[*b*]thiophen-4-yl)-6-chloro-2,8-difluoroquinazolin-4-yl)-3,8-diazabicyclo[3.2.1]octane-8-carboxylate

Under nitrogen atmosphere, a mixture of *tert*-butyl (1*R*,5*S*)-3-(7-bromo-6-chloro-2, 8-difluoroquinazolin-4-yl)-3,8-diazabicyclo[3.2.1]octane-8-carboxylate (5.0 g, 10.2 mmol), *tert*-butyl (4-(5,5-dimethyl-1,3,2-dioxaborinane-2-yl)benzo[*b*]thiophen-2-yl) carbamate (7.38 g, 20.4 mmol), Pd(dppf)$Cl_2$ (740 mg, 1.02 mmol), potassium phosphate (6.5 g, 30.63 mmol) in dioxane/$H_2O$ (V/V = 5:1,30 mL) were stirred at 100 °C for 16 h. After the reaction was completed (monitored by LCMS), the reaction was concentrated and purified with FCC ($SiO_2$, EA/(PE: DCM = 5:1) = 0 - 40%) to give *tert-butyl* (1*R*,5*S*)-3-(7-(2-((*tert*-butoxycarbonyl)amino)benzo[*b*]thiophen-4-yl)-2,8-difluoro-6-(trifluoromethyl)quinazolin-4-yl)-3,8-diazabicyclo[3.2.1]octane-8-carboxylate (580 mg, yield 34%) as a yellow solid. LCMS (m/z): 692.6 (M + H).

**Step B:** *tert*-butyl (1*R*,5*S*)-3-(7-(2-((*tert*-butoxycarbonyl)amino)-3-cyanobenzo[*b*]thiophen-4-yl)-6-chloro-2,8-difluoroquinazolin-4-yl)-3,8-diazabicyclo[3.2.1]octane-8-carboxylate

At 0 °C, CSI (2.9 g, 20.5 mmol) was added to a solution of *tert*-butyl (1*R,5S*)-3-(7-(2-((*tert*-butoxycarbonyl)amino) benzo[*b*]thiophen-4-yl)-6-chloro-2, 8-difluoroquinazolin-4-yl)-3,8-diazabicyclo[3.2.1]octane-8-carboxylate (5.4g, 8.2 mmol) in acetonitrile (200 mL). After stirring for at 0 °C 1.5 h, DMF (28 mL) was added dropwise to the reaction. The resulting mixture was stirred at 0 °C for 1.5 h. After the reaction was completed (monitored by LCMS), the reaction was slowly poured into water, and a solid was precipitated. The solid was collected by filtration to obtain the crude product, which was purified by FCC ($SiO_2$, EA/(PE: DCM = 2:1) = 0-40%) to obtain tert-butyl (1*R*,5*S*)-3-(7-(2-((*tert*-butoxycarbonyl) amino)-3-cyanobenzo[*b*]thiophen-4-yl)-6-chloro-2,8-difluoroquinazolin-4-yl)-3,8-diazabicyclo[3.2.1]octane-8-carboxylate (3 g, yield 53%). LCMS (m/z): 683.2 (M + H).

**Step C:** *tert*-butyl-(1*R*,5*S*)-3-(7-(2-((*tert*-butoxycarbonyl)amino)-3-cyanobenzo[*b*]thiophen-4-yl)-6-chloro-8-fluoro-2-(((*S,E*)-4-(fluoromethylene)-1,3-dimethylpiperidin-3-yl)methoxy) quinazolin-4-yl)-3,8-diazabicyclo[3.2.1]octane-8-carboxylate

At 0 °C, NaH (35 mg, 0.88 mmol) was added to a solution of *tert*-butyl (1*R*,5*S*)-3-(7-(2-((*tert*-butoxycarbonyl) amino)-3-cyanobenzo[*b*]thiophen-4-yl)-6-chloro-2,8-difluoroquinazolin-4-yl)-3,8-diazabicyclo[3.2.1]octane-8-carboxylate (200 mg, 0.293 mmol), (*S,E*)-(4-(fluoromethylene)-1,3-dimethylpiperidin-3-yl)methanol (76 mg, 0.44 mmol) in tetrahydrofuran (5 mL). The reaction was stirred at room temperature for 2 h. After the reaction was completed (monitored by LCMS), the reaction was quenched by adding saturated ammonium chloride solution (60 mL), extracted by ethyl acetate (30 mL x 3). The organic phase was dried over anhydrous sodium sulfate, filtered and concentrated to obtain *tert*-butyl (1*R*,5*S*)-3-(7-(2-((*tert*-butoxycarbonyl)amino)-3-cyanobenzo[*b*]thiophen-4-yl)-6-chloro-8-fluoro-2-(((*S,E*)-4-(fluoromethylene)-1,3-dimethylpiperidin-3-yl)methoxy)quinazolin-4-yl)-3,8-diazabicyclo[3.2.1]octane-8-carboxylate (200 mg, yield 82%) as a yellow solid. LCMS (m/z): 836.3 (M + H).

**Step D:** 4-(4-((1*R*,5*S*)-3,8-diazabicyclo[3.2.1]octan-3-yl)-6-chloro-8-fluoro-2-(((*S*,*E*)-4-(fluoromethylene)-1,3-dimethylpiperidin-3-yl)methoxy)quinazolin-7-yl)-2-aminobenzo[b]thiophene-3-carbonitrile

At room temperature, trifluoroacetic acid (1 mL) was added to a solution of *tert*-butyl-(1*R,5S*)-3-(7-(2-((*tert*-butoxycarbonyl)amino)-3-cyanobenzo[*b*]thiophen-4-yl)-6-chloro-8-fluoro-2-(((*S*,*E*)-4-(fluoromethylene)-1,3-dimethylpiperidin-3-yl)methoxy)quinazolin-4-yl)-3,8-diazabicyclo[3.2.1]octane-8-carboxylate (200 mg, 0.240 mmol) in dichloromethane (1 mL), and the resulting mixture was stirred at room temperature for 1 h. After the reaction was completed (monitored by LCMS), the reaction was concentrated and subjected to Pre-HPLC preparation to obtain 4-(4-((1*R*,5*S*)-3,8-diazabicyclo[3.2.1]octan-3-yl)-6-chloro-8-fluoro-2-(((*S*,*E*)-4-(fluoromethylene)-1,3-dimethylpiperidin-3-yl)methoxy)quinazolin-7-yl)-2-aminobenzo[*b*]thiophene-3-carbonitrile (51.2 mg, yield 34%) as a white solid. LCMS (m/z): 636.3 (M + H). $^1$H NMR (400 MHz, DMSO-$d_6$) δ 7.83 (s, 3H), 7.81 - 7.76 (m, 1H), 7.30 - 7.22 (m, 1H), 7.22 - 7.15 (m, 1H), 6.88 - 6.52 (m, 1H), 4.63 - 4.47 (m, 1H), 4.35 - 4.13 (m, 3H), 3.58 - 3.46 (m, 4H), 2.72 - 2.64 (m, 2H), 2.50 - 2.45 (m, 1H), 2.27 - 2.15 (m, 1H), 2.14 (s, 3H), 1.96 - 1.77 (m, 2H), 1.70 - 1.49 (m, 4H), 1.10 (s, 3H). $^{19}$F NMR (376 MHz, DMSO-$d_6$) δ -122.73, -138.82.

Intermediates **313-1-2A** and **313-1-2B**

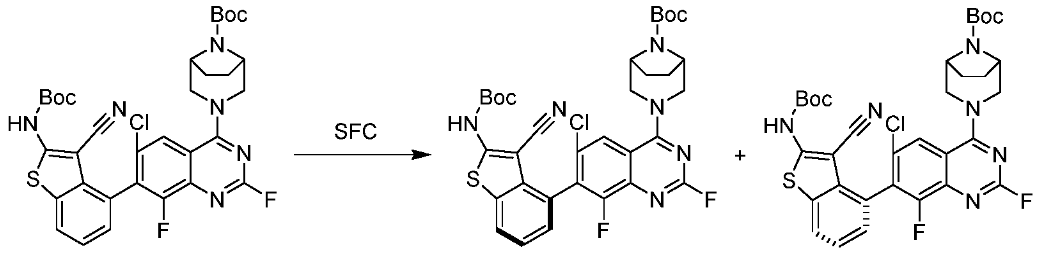


**313-1-2** **313-1-2A或313-1-2B** **313-1-2A或313-1-2B**

Intermediate **313-1-2** (5.0 g) was resolved by SFC (Waters SFC 150, separation column: DAICEL CHIRALPAK® OZ, 250 * 25 mm, 10 μm; mobile phase: $CO_2$/MeOH (+ 0.1% 7.0 mol/L $NH_3$/MeOH) = 60/40; flow rate: 120 mL/min). The first eluted isomer 1 was Intermediate **313-1-2A** (2.0 g, with a shorter retention time). Chiral analytical method SFC-11, Rt = 1.864 min. LCMS (m/z): 683.2 (M + H). The subsequently eluted isomer 2 was the Intermediate **313-1-2B** (2.0 g, with longer retention time). Chiral analytical method SFC-11, Rt = 3.799 min. LCMS (m/z): 683.2 (M + H).

The synthesis of the following compounds can be carried out by referring to the method described in Example **313** to obtain an axial chiral mixture, which can then be resolved to obtain the axial chiral pure compound. Alternatively, the axial chiral pure compound can be prepared from Intermediate **313-1-2A** or **313-1-2B** by referring to the experimental method described in Example **313.** It should be noted that for the following Isomer-1 or -2, the naming was based on the principle that the one with a shorter retention time in the chiral resolution of the final compound was designated as -1, and the one with a longer retention time was designated as -2. Or, when Intermediate **313-1-2A** was used as the intermediate for synthesis, the resulting isomer was Isomer-1, and when Intermediate **313-1-2B** was used as the intermediate for synthesis, the resulting isomer was Isomer-2. When there is a conflict in the two naming methods, the naming after preparation using the latter chiral pure intermediate prevails.

| Ex. | Structure | Name | Characterization data |
|---|---|---|---|
| 313-1 | 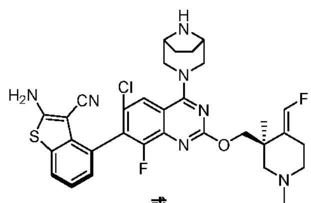 或 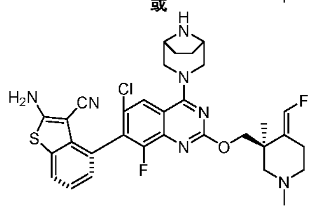 | (*S*)-4-(4-((1*R*,5*S*)-3,8-diazabicyclo[3.2.1]octan-3-yl)-6-chloro-8-fluoro-2-(((*S*,*E*)-4-(fluoromethylene)-1,3-dimethylpiperidin-3-yl methoxy)quinazolin-7-yl)-2-aminobenzo[*b*]thiophene-3-carbonitrile Or (*R*)-4-(4-((1*R*,5*S*)-3,8-diazabicyclo[3.2.1]octan-3-yl)-6-chloro-8-fluoro-2-(((*S*,*E*)-4-(fluoromethylene)-1,3-dimethylpiperidin-3-yl methoxy) quinazolin-7-yl)-2-aminobenzo[*b*]thiophene-3-carbonitrile | LCMS (m/z): 636.2 (M + H). |

(continued)

| Ex. | Structure | Name | Characterization data |
|---|---|---|---|
| 313-2 | 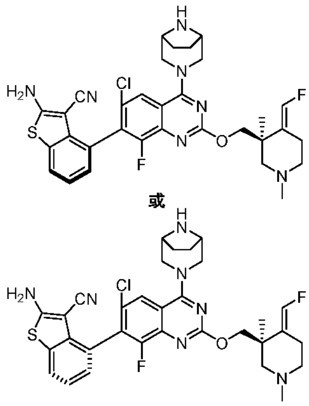 或 | (*S*)-4-(4-((1*R*,5*S*)-3,8-diazabicyclo[3.2.1]octan-3-yl)-6-chloro-8-fluoro-2-(((*S,E*)-4-(fluoromethylene)-1,3-dimethylpiperidin-3-yl methoxy) quinazolin-7-yl)-2-aminobenzo[*b*]thiophene-3-carbonitrile Or (*R*)-4-(4-((1*R*,5*S*)-3,8-diazabicyclo[3.2.1]octan-3-yl)-6-chloro-8-fluoro-2-(((*S,E*)-4-(fluoromethylene)-1,3-dimethylpiperidin-3-yl methoxy) quinazolin-7-yl)-2-aminobenzo[*b*]thiophene-3-carbonitrile | LCMS (m/z): 636.2 (M + H). $^1$H NMR (400 MHz, DMSO-$d_6$) δ 7.80 (dd, *J* = 14.7, 5.6 Hz, 4H), 7.34 - 7.13 (m, 2H), 6.70 (d, *J* = 86.8 Hz, 1H), 4.54 (d, *J* = 10.6 Hz, 1H), 4.42 - 4.14 (m, 3H), 3.62 - 3.46 (m, 5H), 2.71 - 2.61 (m, 2H), 2.27 - 2.09 (m, 4H), 1.95 - 1.79 (m, 2H), 1.70 - 1.52 (m, 4H), 1.10 (s, 3H). $^{19}$F NMR (376 MHz, DMSO-$d_6$) δ-122.70,-138.80. It can be made from Intermediate **313-1-2B** |
| 314 | 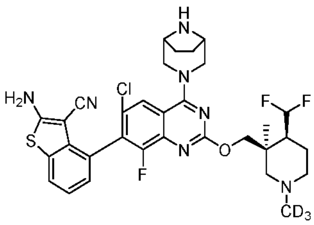 | 4-(4-((1*R*,5*S*)-3,8-diazabicyclo[3.2.1]octan-3-yl)-6-chloro-2-(((3*S*,4*S*)-4-(difluoromethyl)-3-methyl-1-(methyl-$d_3$) piperidin-3-yl)methoxy)-8-fluoroquinazolin-7-yl)-2-aminobenzo[*b*]thiophene-3-carbonitrile | LCMS (m/z): 659.2 (M + H). |
| 314-1 | 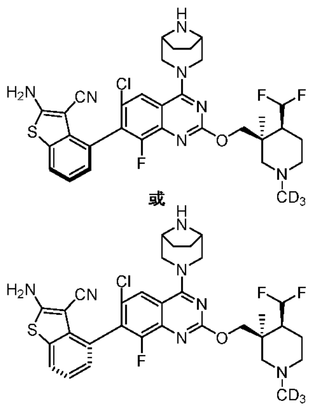 或 | (*S*)-4-(4-((1*R*,5*S*)-3,8-diazabicyclo[3.2.1]octan-3-yl)-6-chloro-2-(((3*S*,4*S*)-4-(difluoromethyl)-3-methyl-1-(methyl-$d_3$) piperidin-3-yl)methoxy)-8-fluoroquinazolin-7-yl)-2-aminobenzo[*b*]thiophene-3-carbonitrile Or (*R*)-4-(4-((1*R*,5*S*)-3,8-diazabicyclo[3.2.1]octan-3-yl)-6-chloro-2-(((3*S*,4*S*)-4-(difluoromethyl)-3-methyl-1-(methyl-$d_3$) piperidin-3-yl)methoxy)-8-fluoroquinazolin-7-yl)-2-aminobenzo[*b*]thiophene-3-carbonitrile | LCMS (m/z): 659.2 (M + H). |

(continued)

| Ex. | Structure | Name | Characterization data |
|---|---|---|---|
| 314-2 | 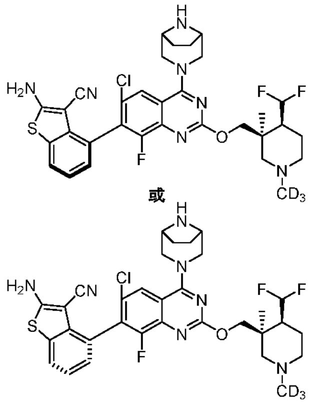 或 | (*S*)-4-(4-((1*R*,5*S*)-3,8-diazabicyclo[3.2.1]octan-3-yl)-6-chloro-2-(((3*S*,4*S*)-4-(difluoromethyl)-3-methyl-1-(methyl-$d_3$) piperidin-3-yl)methoxy)-8-fluoroquinazolin-7-yl)-2-aminobenzo[*b*]thiophene-3-carbonitrile Or (*R*)-4-(4-((1*R*,5*S*)-3,8-diazabicyclo[3.2.1]octan-3-yl)-6-chloro-2-(((3*S*,4*S*)-4-(difluoromethyl)-3-methyl-1-(methyl-$d_3$) piperidin-3-yl)methoxy)-8-fluoroquinazolin-7-yl)-2-aminobenzo[*b*]thiophene-3-carbonitrile | LCMS (m/z): 659.2 (M + H). |
| 315 | 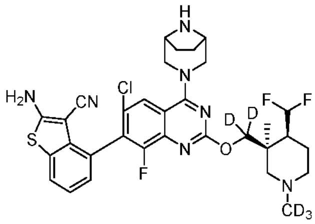 | 4-(4-((1*R*,5*S*)-3,8-diazabicyclo[3.2.1]octan-3-yl)-6-chloro-2-(((3*S*,4*S*)-4-(difluoromethyl)-3-methyl-1-(methyl-$d_3$) piperidin-3-yl)methoxy-$d_2$)-8-fluoroquinazolin-7-yl)-2-aminobenzo[*b*]thiophene-3-carbonitrile | LCMS (m/z): 661.2 (M + H). |
| 315-1 | 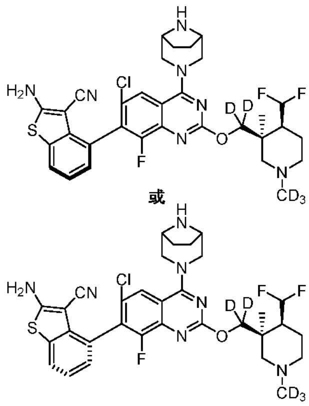 或 | (*S*)-4-(4-((1*R*,5*S*)-3,8-diazabicyclo[3.2.1]octan-3-yl)-6-chloro-2-(((3*S*,4*S*)-4-(difluoromethyl)-3-methyl-1-(methyl-$d_3$) piperidin-3-yl)methoxy-$d_2$)-8-fluoroquinazolin-7-yl)-2-aminobenzo[*b*]thiophene-3-carbonitrile Or (*R*)-4-(4-((1*R*,5*S*)-3,8-diazabicyclo[3.2.1]octan-3-yl)-6-chloro-2-(((3*S*,4*S*)-4-(difluoromethyl)-3-methyl-1-(methyl-$d_3$) piperidin-3-yl)methoxy-$d_2$)-8-fluoroquinazolin-7-yl)-2-aminobenzo[*b*]thiophene-3-carbonitrile | LCMS (m/z): 661.2 (M + H). |

(continued)

| Ex. | Structure | Name | Characterization data |
|---|---|---|---|
| 315-2 | 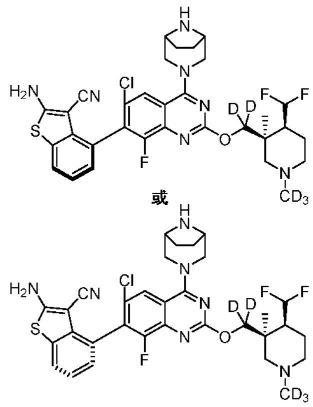 或 | (*S*)-4-(4-((1*R*,5*S*)-3,8-diazabicyclo[3.2.1]octan-3-yl)-6-chloro-2-(((3*S*,4*S*)-4-(difluoromethyl)-3-methyl-1-(methyl-*d*$_3$) piperidin-3-yl)methoxy-*d*$_2$)-8-fluoroquinazolin-7-yl)-2-aminobenzo[*b*]thiophene-3-carbonitrile<br>Or<br>(*R*)-4-(4-((1*R*,5*S*)-3,8-diazabicyclo[3.2.1]octan-3-yl)-6-chloro-2-(((3*S*,4*S*)-4-(difluoromethyl)-3-methyl-1-(methyl-*d*$_3$) piperidin-3-yl)methoxy-*d*$_2$)-8-fluoroquinazolin-7-yl)-2-aminobenzo[*b*]thiophene-3-carbonitrile | LCMS (m/z): 661.2 (M + H). $^1$H NMR (400 MHz, DMSO-*d*$_6$) δ 7.96 -7.62 (m, 4H), 7.29 - 7.13 (m, 2H), 6.48 - 6.11 (m, 1H), 4.42 - 4.17 (m, 2H), 3.65 - 3.41 (m, 4H), 2.92 - 2.74 (m, 2H), 1.92 - 1.50 (m, 9H), 1.11 (s, 3H). $^{19}$F NMR (376 MHz, DMSO-*d*$_6$) δ-114.93 --116.30 (m), -118.20-119.61 (m), -122.65. |
| 316 | 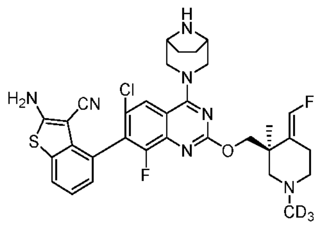 | 4-(4-((1*R*,5*S*)-3,8-diazabicyclo [3.2.1]octan-3-yl)-6-chloro-8-fluoro-2-(((*S,E*)-4-(fluoromethylene)-3-methyl-1-(methyl-*d*$_3$) piperidin-3-yl)methoxy) quinazolin-7-yl)-2-aminobenzo[*b*]thiophene-3-carbonitrile | LCMS (m/z): 639.2 (M + H). |
| 316-1 | 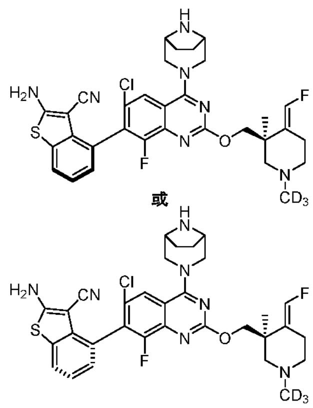 或 | (*S*)-4-(4-((1*R*,5*S*)-3,8-diazabicyclo[3.2.1]octan-3-yl)-6-chloro-8-fluoro-2-(((*S,E*)-4-(fluoromethylene)-3-methyl-1-(methyl-*d*$_3$) piperidin-3-yl)methoxy) quinazolin-7-yl)-2-aminobenzo[*b*]thiophene-3-carbonitrile<br>Or<br>(*R*)-4-(4-((1*R*,5*S*)-3,8-diazabicyclo[3.2.1]octan-3-yl)-6-chloro-8-fluoro-2-(((*S,E*)-4-(fluoromethylene)-3-methyl-1-(methyl-*d*$_3$) piperidin-3-yl)methoxy) quinazolin-7-yl)-2-aminobenzo[*b*]thiophene-3-carbonitrile | LCMS (m/z): 639.2 (M + H). |

(continued)

| Ex. | Structure | Name | Characterization data |
|---|---|---|---|
| 316-2 | 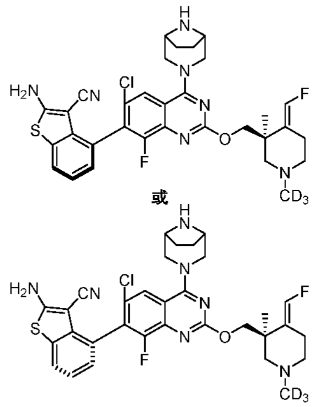 或 | (*S*)-4-(4-((1*R*,5*S*)-3,8-diazabicyclo[3.2.1]octan-3-yl)-6-chloro-8-fluoro-2-(((*S,E*)-4-(fluoromethylene)-3-methyl-1-(methyl-$d_3$) piperidin-3-yl)methoxy) quinazolin-7-yl)-2-aminobenzo[*b*]thiophene-3-carbonitrile<br>Or<br>(*R*)-4-(4-((1*R*,5*S*)-3,8-diazabicyclo[3.2.1]octan-3-yl)-6-chloro-8-fluoro-2-(((S,E)-4-(fluoromethylene)-3-methyl-1-(methyl-$d_3$) piperidin-3-yl)methoxy) quinazolin-7-yl)-2-aminobenzo[*b*]thiophene-3-carbonitrile | LCMS (m/z): 639.2 (M + H). |
| 317 | 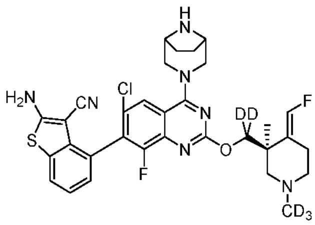 | 4-(4-((1*R*,5*S*)-3,8-diazabicyclo [3.2.1]octan-3-yl)-6-chloro-8-fluoro-2-(((*S,E*)-4-(fluoromethylene)-3-methyl-1-(methyl-$d_3$) piperidin-3-yl)methoxy-$d_2$) quinazolin-7-yl)-2-aminobenzo[*b*] thiophene-3-carbonitrile | LCMS (m/z): 641.2 (M + H). |
| 317-1 | 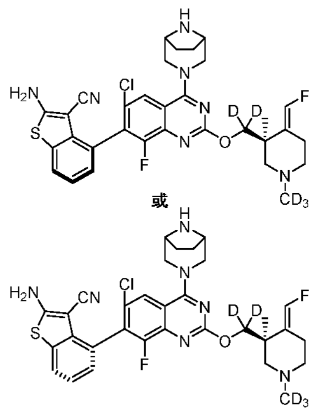 或 | (*S*)-4-(4-((1*R*,5*S*)-3,8-diazabicyclo[3.2.1]octan-3-yl)-6-chloro-8-fluoro-2-(((*S,E*)-4-(fluoromethylene)-3-methyl-1-(methyl-$d_3$) piperidin-3-yl)methoxy-$d_2$) quinazolin-7-yl)-2-aminobenzo[*b*] thiophene-3-carbonitrile<br>Or<br>(*R*)-4-(4-((1*R*,5*S*)-3,8-diazabicyclo[3.2.1]octan-3-yl)-6-chloro-8-fluoro-2-(((*S,E*)-4-(fluoromethylene)-3-methyl-1-(methyl-$d_3$) piperidin-3-yl)methoxy-$d_2$) quinazolin-7-yl)-2-aminobenzo[*b*] thiophene-3-carbonitrile | LCMS (m/z): 641.2 (M + H). |

(continued)

| Ex. | Structure | Name | Characterization data |
|---|---|---|---|
| 317-2 | 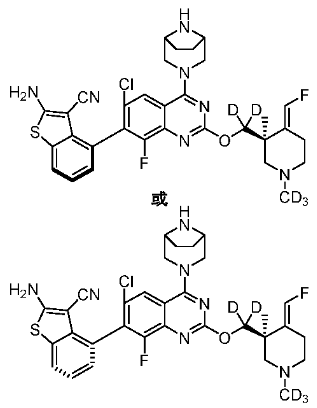 或 | (*S*)-4-(4-((1*R*,5*S*)-3,8-diazabicyclo[3.2.1]octan-3-yl)-6-chloro-8-fluoro-2-(((*S,E*)-4-(fluoromethylene)-3-methyl-1-(methyl-$d_3$) piperidin-3-yl)methoxy-$d_2$) quinazolin-7-yl)-2-aminobenzo[*b*] thiophene-3-carbonitrile Or (*R*)-4-(4-((1*R*,5*S*)-3,8-diazabicyclo[3.2.1]octan-3-yl)-6-chloro-8-fluoro-2-(((*S*, *E*)-4-(fluoromethylene)-3-methyl-1-(methyl-$d_3$) piperidin-3-yl)methoxy-$d_2$) quinazolin-7-yl)-2-aminobenzo[*b*]thiophene-3-carbonitrile | LCMS (m/z): 641.2 (M + H). |

**Example 318**

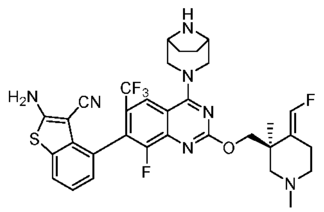

4-(4-((1*R*,5*S*)-3,8-diazabicyclo[3.2.1]octan-3-yl)-8-fluoro-2-(((*S*,*E*)-4-(fluoromethylene)-1,3-dimethylpiperidin-3-yl) methoxy)-6-(trifluoromethyl) quinazolin-7-yl)-2-aminobenzo[*b*]thiophene-3-carbonitrile

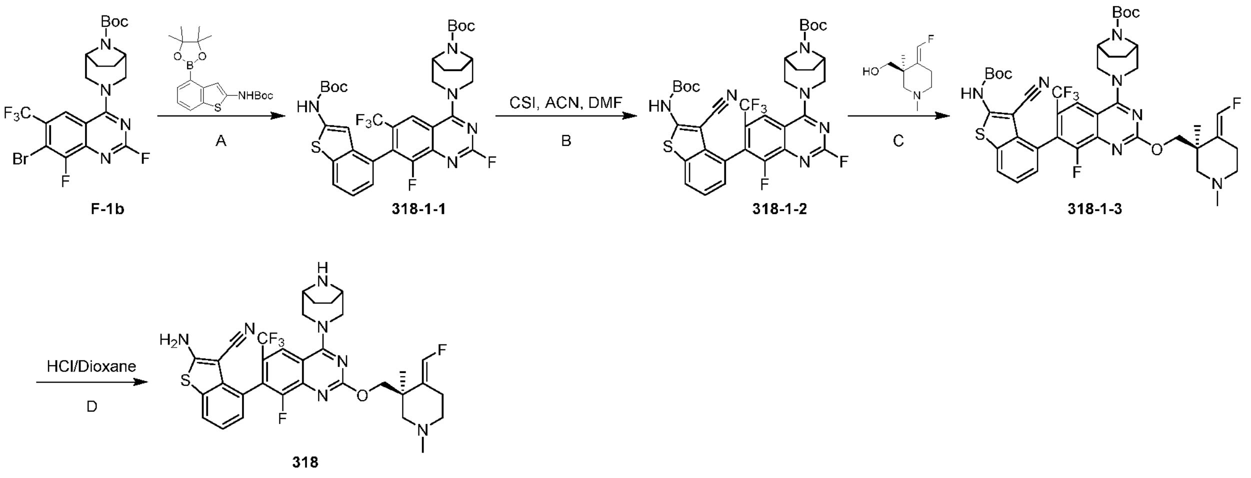

**Step A:** *tert*-butyl (1*R*,5*S*)-3-(7-(2-((*tert*-butoxycarbonyl)amino)benzo[*b*]thiophen-4-yl)-2, 8-difluoro-6-(trifluoromethyl) quinazolin-4-yl)-3,8-diazabicyclo[3.2.1]octane-8-carboxylate

A mixture of *tert*-butyl (1*R*,5*S*)-3-(7-bromo-2,8-difluoro-6-(trifluoromethyl)quinazolin-4-yl)-3,8-diazabicyclo [3.2.1]octane-8-carboxylate (1.3g, 2.5 mmol), tert-butyl (4-(4,4,5,5-tetramethyl-1,3,2-dioxaborolan-2-yl)benzo[*b*]thiophen-2-yl) carbamate (1.4g, 3.75 mmol), Pd-118 (320 mg, 0.5 mmol), potassium phosphate (1.6 g, 7.5 mmol in dioxane/$H_2O$ (V/V = 4:1 , 25 mL) were stirred at 100 °C for 16 h. After the reaction was completed (monitored by LCMS), the reaction was concentrated and purified with FCC ($SiO_2$, EA/PE = 0-30%) to afford *tert*-butyl (1*R*,5*S*)-3-(7-(2-((*tert*-butoxycarbonyl)amino)benzo[*b*]thiophen-4-yl)-2,8-difluoro-6-(trifluoromethyl)quinazolin-4-yl)-3,8-diazabicyclo[3.2.1] octane-8-carboxylate (580 mg, yield 34%) as a yellow solid. LCMS (m/z): 692.6 (M + H).

**Step B:** *tert*-butyl (1*R*,5*S*)-3-(7-(2-((*tert*-butoxycarbonyl)amino)-3-cyanobenzo[*b*]thiophen-4-yl)-2,8-difluoro-6-(trifluoromethyl)quinazolin-4-yl)-3,8-diazabicyclo[3.2.1]octane-8-carboxylate

At 0 °C, CSI (262 mg, 0.16 mL, 1.9 mol) was added to a solution of *tert*-butyl (1*R*,5*S*)-3-(7-(2-((*tert*-butoxycarbonyl)amino)benzo[*b*]thiophen-4-yl)-2,8-difluoro-6-(trifluoromethyl) quinazolin-4-yl)-3,8-diazabicyclo[3.2.1]octane-8-carboxylate (430 mg, 0.62 mmol) in acetonitrile (5 mL). After stirring at 0 °C for 2 h, DMF (2.5 mL) was slowly added to the reaction. The resulting mixture was stirred at 0 °C for 1 h. After the reaction was completed (monitored by LCMS), saturated ammonium chloride solution (10 mL) was added to quench the reaction, and the resulting mixture was extracted with ethyl acetate (20 mL). The organic phase was dried over anhydrous sodium sulfate, filtered, and concentrated. The crude product obtained was purified by FCC ($SiO_2$, EA/PE = 0-50%) to give *tert*-butyl (1*R*,5*S*)-3-(7-(2-((*tert*-butoxycarbonyl)amino)-3-cyanobenzo[*b*]thiophen-4-yl)-2,8-difluoro-6-(trifluoromethyl)quinazolin-4-yl)-3,8-diazabicyclo[3.2.1]octane-8-carboxylate (280 mg, yield 63%) as a yellow solid. LCMS (m/z): 717.3 (M + H).

**Step C:** *tert*-butyl (1*R*,5*S*)-3-(7-(2-((*tert*-butoxycarbonyl)amino)-3-cyanobenzo[*b*]thiophen-4-yl)-8-fluoro-2-(((*S*,*E*)-4-(fluoromethylene)-1,3-dimethylpiperidin-3-yl)methoxy)-6-(trifluoromethyl)quinazolin-4-yl)-3,8-diazabicyclo[3.2.1] octane-8-carboxylate

At 0 °C, NaH (50 mg, 3.5 mmol) was added to a solution of *tert*-butyl (1*R*,5*S*)-3-(7-(2-((*tert*-butoxycarbonyl) amino)-3-cyanobenzo[*b*]thiophen-4-yl)-2,8-difluoro-6-(trifluoromethyl) quinazolin-4-yl)-3,8-diazabicyclo[3.2.1]octane-8-carboxylate (280 mg, 0.39 mmol), (*S,E*)-(4-(fluoromethylene)-1,3-dimethylpiperidin-3-yl) methanol (101 mg, 0.59 mmol) in tetrahydrofuran (3 mL). The reaction mixture was stirred at room temperature for 2 hours. After the reaction was completed (monitored by LCMS), the reaction was quenched with saturated ammonium chloride solution (10 mL) and extracted with ethyl acetate (20 mL). The organic phase was dried over anhydrous sodium sulfate, filtered and concentrated to afford *tert*-butyl (1*R*,5*S*)-3-(7-(2-((*tert*-butoxycarbonyl)amino)-3-cyanobenzo[*b*]thiophen-4-yl)-8-fluoro-2-(((*S,E*)-4-(fluoromethylene)-1,3-dimethylpiperidin-3-yl)methoxy)-6-(trifluoromethyl)quinazolin-4-yl)-3,8-diazabicyclo[3.2.1]octane-8-carboxylate (200 mg, yield 60%) as a yellow solid. LCMS (m/z): 870.3 (M + H).

**Step D:** 4-(4-((1*R*,5*S*)-3,8-diazabicyclo[3.2.1]octan-3-yl)-8-fluoro-2-(((*S*,*E*)-4-(fluoromethylene)-1,3-dimethylpiperidin-3-yl)methoxy)-6-(trifluoromethyl)quinazolin-7-yl)-2-aminobenzo[*b*]thiophene-3-carbonitrile

At room temperature, hydrochloric acid-dioxane solution (4 M, 3 mL) was added to *tert*-butyl (1*R*,5*S*)-3-(7-(2-((*tert*-butoxycarbonyl)amino)-3-cyanobenzo[*b*]thiophen-4-yl)-8-fluoro-2-(((*S,E*)-4-(fluoromethylene)-1,3-dimethylpiperidin-3-yl)methoxy)-6-(trifluoromethyl)quinazolin-4-yl)-3,8-diazabicyclo[3.2.1]octane-8-carboxylate (200 mg, 0.23 mmol). The resulting mixture was stirred at room temperature for 1 h. After the reaction was completed (monitored by LCMS), the reaction was concentrated and subjected to Pre-HPLC preparation to obtain 4-(4-((1*R*,5*S*)-3,8-diazabicyclo[3.2.1]octan-3-yl)-8-fluoro-2-(((*S,E*)-4-(fluoromethylene)-1,3-dimethylpiperidin-3-yl)methoxy)-6-(trifluoromethyl)quinazolin-7-yl)-2-aminobenzo[*b*]thiophene-3-carbonitrile (28 mg, yield 18%) as a white solid. LCMS (m/z): 670.1 (M + H).

**Examples 318-1 and 318-2**

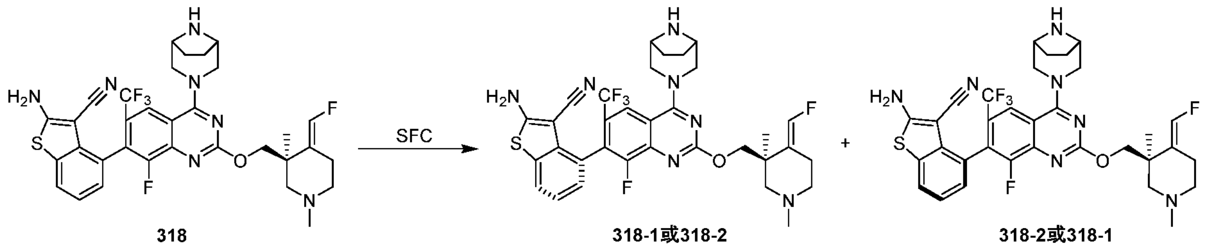


318 318-1或318-2 318-2或318-1

4-(4-((1*R*,5*S*)-3,8-diazabicyclo[3.2.1]octan-3-yl)-8-fluoro-2-(((*S,E*)-4-(fluoromethylene)-1,3-dimethylpiperidin-3-yl)methoxy)-6-(trifluoromethyl)quinazolin-7-yl)-2-aminobenzo[*b*]thiophene-3-carbonitrile (Example 318, 28 mg) was resolved by SFC (Waters SFC 150, Separation column: DAICEL CHIRALCEL ® IC, 250 * 20 mm, 10 μm; Mobile phase: $CO_2$/MeOH (+ 0.1% 7.0 mol/l Ammonia) = 50/50; Flow rate: 120 mL/min). The first eluted white solid isomer 1 was 318-1 (8.2 mg, with a relatively shorter retention time). Chiral analytical method SFC-8, Rt = 1.394 min. LCMS (m/z): 670.3 (M + H). $^1$H NMR (400 MHz, DMSO-$d_6$) δ 8.06 (s, 1H), 7.84 - 7.71 (m, 3H), 7.25 - 7.16 (m, 2H), 6.72 (d, *J*= 86.8 Hz, 1H), 4.57 (d, *J*= 10.6 Hz, 1H), 4.36 - 4.23 (m, 3H), 3.59 (q, *J* = 12.2 Hz, 4H), 2.72 - 2.66 (m, 2H), 2.56 - 2.53 (m, 1H), 2.21 - 2.10 (m, 4H), 1.94 - 1.79 (m, 2H), 1.72 - 1.52 (m, 4H), 1.11 (s, 3H). $^{19}$F NMR (376 MHz, DMSO-$d_6$) δ -57.26, -123.96, -138.72. The subsequently eluted white solid isomer 2 was 318-2 (8.6 mg, with a relatively longer retention time). Chiral analytical method SFC-8, Rt = 2.696 min. LCMS (m/z): 670.2 (M + H). $^1$H NMR (400 MHz, DMSO-$d_6$) δ 8.06 (s, 1H), 7.83 - 7.75 (m, 3H), 7.26 - 7.16 (m, 2H), 6.70 (d, *J* = 86.6 Hz, 1H), 4.59 (d, *J* = 10.7 Hz, 1H), 4.33 - 4.24 (m, 3H), 3.62 - 3.50 (m, 4H), 2.73 - 2.67 (m, 2H), 2.55 - 2.52 (m, 1H), 2.26 - 2.17 (m, 1H), 2.14 (s, 3H), 1.94 - 1.80 (m, 2H), 1.67 - 1.48 (m, 4H), 1.10 (s, 3H). $^{19}$F NMR (376 MHz, DMSO-$d_6$) δ -57.26, -124.03, -138.78.

Intermediates **318-1-2A** and **318-1-2B**

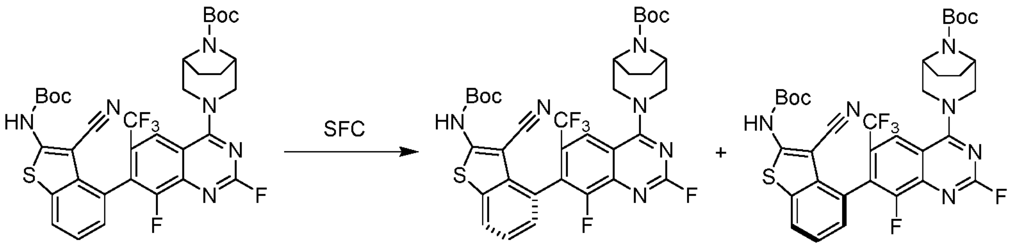


318-1-2 318-1-2A或318-1-2B 318-1-2A或318-1-2B

Intermediate **318-1-2** (1.2 g) was resolved by SFC (Waters SFC 150, separation column: REGIS (S,S) WHELK-O1, 250*25 mm, 10μm; mobile phase: $CO_2$/MeOH (+ 0.1% 7.0 mol/L $NH_3$/MeOH) = 80/20; flow rate: 120 mL/min). The first eluted isomer 1 was intermediate **318-1-2A** (0.48 g, with a relatively shorter retention time). Chiral analytical method SFC-12, Rt = 1.923 min. LCMS (m/z): 717.3 (M + H). The subsequently eluted isomer 2 was intermediate **318-1-2B** (0.49 g, with longer retention time). Chiral analytical method SFC-12, Rt = 2.523 min. LCMS (m/z): 717.3 (M + H).

The following examples can be prepared according to the synthetic methods of **318, 318-1,** or **318-2.** That is, the axial chiral mixture was first prepared and then the final compounds were obtained through chiral resolution. Alternatively, they can be prepared using the axial chiral pure Intermediates **318-1-2A** or **318-1-2B.** It should be noted that for the naming of the following isomers-1 or -2, when the final compounds were subjected to chiral resolution, the one with a shorter retention time was named as -1 and the one with a longer retention time was named as -2. Or, the compound synthesized using **318-1-2A** as the intermediate was named as isomer-1, and the compound synthesized using the intermediate **318-1-2B** was named as isomer-2. When there is a conflict between the two naming methods, the naming based on the synthesis with the atropisomerically pure intermediate prevails.

| Ex. | Structure | Name | Characterization data |
|---|---|---|---|
| 319 | 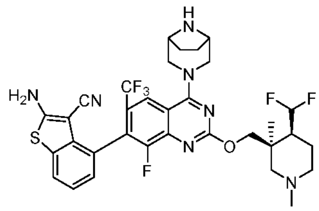 | 4-(4-((1*R*,5*S*)-3,8-diazabicyclo[3.2.1]octan-3-yl)-2-(((3*S*,4*S*)-4-(difluoromethyl)-1,3-dimethylpiperidin-3-yl)methoxy)-8-fluoro-6-(trifluoromethyl)quinazolin-7-yl)-2-aminobenzo[*b*]thiophene-3-carbonitrile | LCMS (m/z): 690.2 (M + H). $^1$H NMR (400 MHz, Methanol-$d_4$) δ 8.10 (s, 1H), 7.67 - 7.63 (m, 1H), 7.25 - 7.16 (m, 2H), 6.38 - 6.05 (m, 1H), 4.62 - 4.57 (m, 2H), 4.51 - 4.43 (m, 2H), 3.75 - 3.68 (m, 1H), 3.67 - 3.57 (m, 4H), 3.10 - 3.02 (m, 1H), 3.00 - 2.90 (m, 1H), 2.23 (s, 3H), 2.02 - 1.94 (m, 1H), 1.85 - 1.77 (m, 7H), 1.21 (s, 3H). $^{19}$F NMR (376 MHz, Methanol-$d_4$) δ -59.93, -117.82 --122.72 (m), - 124.72. |
| 319-1 | 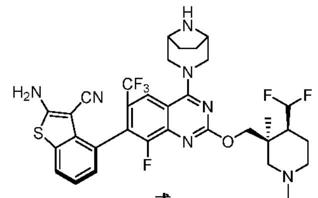 或 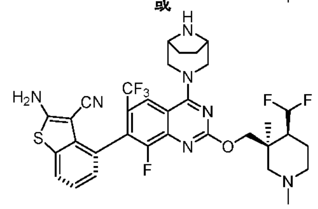 | (*S*)-4-(4-((1*R*,5*S*)-3,8-diazabicyclo[3.2.1]octan-3-yl)-2-((((3*S*,4*S*)-4-(difluoromethyl)-1,3-dimethylpiperidin-3-yl)methoxy)-8-fluoro-6-(trifluoromethyl) quinazolin-7-yl)-2-aminobenzo[*b*]thiophene-3-carbonitrile<br>Or<br>(*R*)-4-(4-((1*R*,5*S*)-3,8-diazabicyclo[3.2.1]octan-3-yl)-2-(((3S,4S)-4-(difluoromethyl)-1,3-dimethylpiperidin-3-yl)methoxy)-8-fluoro-6-(trifluoromethyl) quinazolin-7-yl)-2-aminobenzo[b]thiophene-3-carbonitrile | LCMS (m/z): 690.2 (M + H). |
| 319-2 | 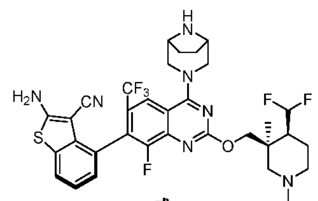 或 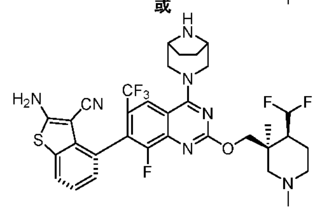 | (*S*)-4-(4-((1*R*,5*S*)-3,8-diazabicyclo[3.2.1]octan-3-yl)-2-(((3*S*,4*S*)-4-(difluoromethyl)-1,3-dimethylpiperidin-3-yl)methoxy)-8-fluoro-6-(trifluoromethyl) quinazolin-7-yl)-2-aminobenzo[*b*]thiophene-3-carbonitrile<br>Or<br>(*R*)-4-(4-((1*R*,5*S*)-3,8-diazabicyclo[3.2.1]octan-3-yl)-2-(((3*S*,4*S*)-4-(difluoromethyl)-1,3-dimethylpiperidin-3-yl)methoxy)-8-fluoro-6-(trifluoromethyl) quinazolin-7-yl)-2-aminobenzo[*b*]thiophene-3-carbonitrile | LCMS (m/z): 690.2 (M + H). |
| 320 | 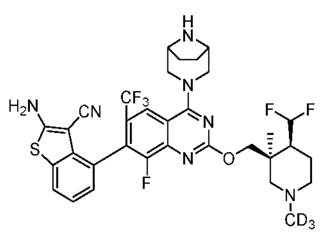 | 4-(4-((1*R*,5*S*)-3,8-diazabicyclo[3.2.1]octan-3-yl)-2-(((3*S*,4*S*)-4-(difluoromethyl)-3-methyl-1-(methyl-$d_3$) piperidin-3-yl)methoxy)-8-fluoro-6-(trifluoromethyl) quinazolin-7-yl)-2-aminobenzo[*b*]thiophene-3-carbonitrile | LCMS (m/z): 693.3 (M + H). |

(continued)

| Ex. | Structure | Name | Characterization data |
|---|---|---|---|
| 320-1 | 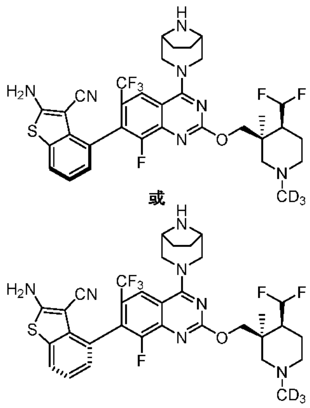 或 | (*S*)-4-(4-((1*R*,5*S*)-3,8-diazabicyclo [3.2.1]octan-3-yl)-2-(((3*S*,4*S*)-4-(difluoromethyl)-3-methyl-1-(methyl-$d_3$) piperidin-3-yl)methoxy)-8-fluoro-6-(trifluoromethyl) quinazolin-7-yl)-2-aminobenzo[*b*]thiophene-3-carbonitrile Or (*R*)-4-(4-((1*R*,5*S*)-3,8-diazabicyclo [3.2.1]octan-3-yl)-2-(((3*S*,4*S*)-4-(difluoromethyl)-3-methyl-1-(methyl-$d_3$) piperidin-3-yl)methoxy)-8-fluoro-6-(trifluoromethyl) quinazolin-7-yl)-2-aminobenzo[*b*]thiophene-3-carbonitrile | LCMS (m/z): 693.3 (M + H). $^1$H NMR (400 MHz, DMSO-$d_6$) δ 8.06 (s, 1H), 7.82 - 7.75 (m, 3H), 7.25 - 7.17 (m, 2H), 6.30 (t, *J* = 55.6 Hz 1H), 4.49 - 4.39 (m, 2H), 4.35 - 4.26 (m, 2H), 3.66 - 3.57 (m, 4H), 2.87 - 2.76 (m, 2H), 1.94 - 1.51 (m, 10H), 1.11 (s, 3H). It can be prepared from Intermediate 318-1-2A |
| 320-2 | 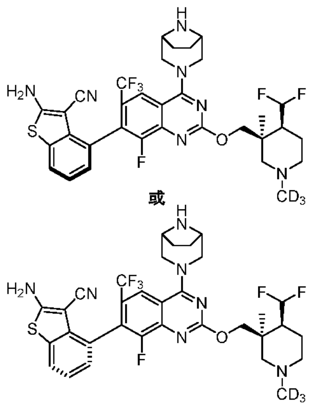 或 | (*S*)-4-(4-((1*R*,5*S*)-3,8-diazabicyclo [3.2.1]octan-3-yl)-2-(((3*S*,4*S*)-4-(difluoromethyl)-3-methyl-1-(methyl-$d_3$) piperidin-3-yl)methoxy)-8-fluoro-6-(trifluoromethyl) quinazolin-7-yl)-2-aminobenzo[*b*]thiophene-3-carbonitrile Or (*R*)-4-(4-((1*R*,5*S*)-3,8-diazabicyclo [3.2.1]octan-3-yl)-2-(((3*S*,4*S*)-4-(difluoromethyl)-3-methyl-1-(methyl-$d_3$) piperidin-3-yl)methoxy)-8-fluoro-6-(trifluoromethyl) quinazolin-7-yl)-2-aminobenzo[*b*]thiophene-3-carbonitrile | LCMS (m/z): 693.3 (M + H). |
| 321 | 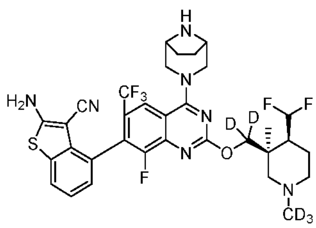 | 4-(4-((1*R*,5*S*)-3,8-diazabicyclo[3.2.1] octan-3-yl)-2-(((3S,4S)-4-(difluoromethyl)-3-methyl-1-(methyl-$d_3$) piperidin-3-yl)methoxy-$d_2$)-8-fluoro-6-(trifluoromethyl) quinazolin-7-yl)-2-aminobenzo[*b*]thiophene-3-carbonitrile | LCMS (m/z): 695.3 (M + H). |

(continued)

| Ex. | Structure | Name | Characterization data |
|---|---|---|---|
| 321-1 | 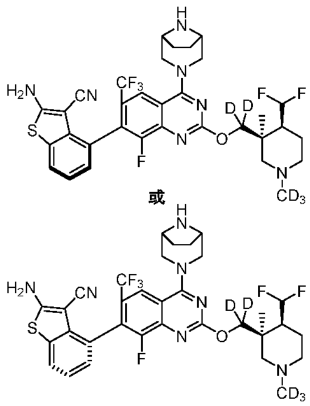 或 | (*S*)-4-(4-((1*R*,5*S*)-3,8-diazabicyclo [3.2.1]octan-3-yl)-2-(((3*S*,4*S*)-4-(difluoromethyl)-3-methyl-1-(methyl-$d_3$) piperidin-3-yl)methoxy-$d_2$)-8-fluoro-6-(trifluoromethyl) quinazolin-7-yl)-2-aminobenzo[*b*]thiophene-3-carbonitrile<br>Or<br>(*R*)-4-(4-((1*R*,5*S*)-3,8-diazabicyclo [3.2.1]octan-3-yl)-2-(((3*S*,4*S*)-4-(difluoromethyl)-3-methyl-1-(methyl-$d_3$) piperidin-3-yl)methoxy-$d_2$)-8-fluoro-6-(trifluoromethyl) quinazolin-7-yl)-2-aminobenzo[*b*]thiophene-3-carbonitrile | LCMS (m/z) 695.3 (M + H). $^1$H NMR (400 MHz, DMSO-$d_6$) δ 8.06 (s, 1H), 7.83 - 7.72 (m, 3H), 7.27 - 7.14 (m, 2H), 6.46 - 6.13 (m, 1H), 4.34 - 4.26 (m, 2H), 3.63 - 3.50 (m, 4H), 2.88 - 2.75 (m, 2H), 1.89 - 1.50 (m, 9H), 1.11 (s, 3H). $^{19}$F NMR (376 MHz, DMSO-$d_6$) δ -57.27 (3F), -115.88, -119.02, -123.88.<br>It can be prepared from Intermediate 318-1-2A |
| 321-2 | 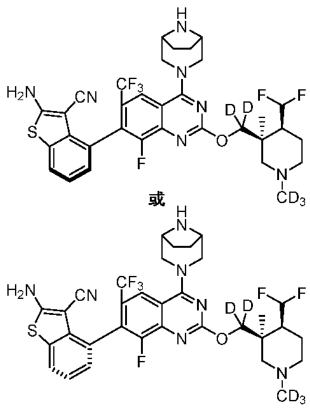 或 | (*S*)-4-(4-((1*R*,5*S*)-3,8-diazabicyclo [3.2.1]octan-3-yl)-2-(((3*S*,4*S*)-4-(difluoromethyl)-3-methyl-1-(methyl-$d_3$) piperidin-3-yl)methoxy-$d_2$)-8-fluoro-6-(trifluoromethyl) quinazolin-7-yl)-2-aminobenzo[*b*]thiophene-3-carbonitrile<br>Or<br>(*R*)-4-(4-((1*R*,5*S*)-3,8-diazabicyclo [3.2.1]octan-3-yl)-2-(((3*S*,4*S*)-4-(difluoromethyl)-3-methyl-1-(methyl-$d_3$) piperidin-3-yl)methoxy-$d_2$)-8-fluoro-6-(trifluoromethyl) quinazolin-7-yl)-2-aminobenzo[*b*]thiophene-3-carbonitrile | LCMS (m/z): 695.3 (M + H). |
| 322 | 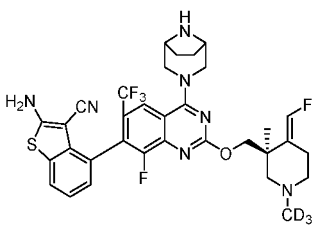 | 4-(4-((1*R*,5*S*)-3,8-diazabicyclo[3.2.1] octan-3-yl)-8-fluoro-2-(((*S*,*E*)-4-(fluoromethylene)-3-methyl-1-(methyl-$d_3$) piperidin-3-yl)methoxy)-6-(trifluoromethyl) quinazolin-7-yl)-2-aminobenzo[*b*]thiophene-3-carbonitrile | LCMS (m/z): 673.3 (M + H). |

(continued)

| Ex. | Structure | Name | Characterization data |
|---|---|---|---|
| 322-1 | 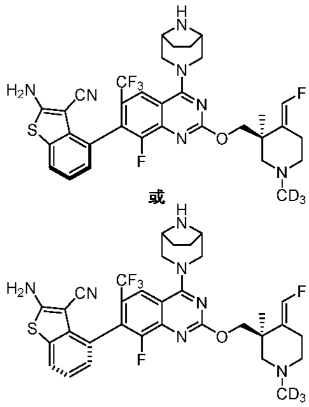 或 | (*S*)-4-(4-((1*R*,5*S*)-3,8-diazabicyclo [3.2.1]octan-3-yl)-8-fluoro-2-(((*S*, *E*)-4-(fluoromethylene)-3-methyl-1-(methyl-$d_3$) piperidin-3-yl) methoxy)-6-(trifluoromethyl) quinazolin-7-yl)-2-aminobenzo[*b*]thiophene-3-carbonitrile<br>Or<br>(*R*)-4-(4-((1*R*,5*S*)-3,8-diazabicyclo [3.2.1]octan-3-yl)-8-fluoro-2-(((*S,E*)-4-(fluoromethylene)-3-methyl-1-(methyl-$d_3$) piperidin-3-yl) methoxy)-6-(trifluoromethyl) quinazolin-7-yl)-2-aminobenzo[*b*]thiophene-3-carbonitrile | LCMS (m/z): 673.3 (M + H). $^1$H NMR (400 MHz, DMSO-$d_6$) δ 8.06 (s, 1H), 7.83 - 7.75 (m, 3H), 7.27 - 7.15 (m, 2H), 6.85 - 6.57 (m, 1H), 4.57 (d, *J* = 10.6 Hz, 1H), 4.37 - 4.24 (m, 3H), 3.64 - 3.53 (m, 4H), 2.71 - 2.64 (m, 2H), 2.58 - 2.52 (m, 1H), 2.22 - 2.10 (m, 1H), 1.94 - 1.78 (m, 2H), 1.70 - 1.51 (m, 4H), 1.11 (s, 3H). $^{19}$H NMR (400 MHz, DMSO-$d_6$) δ-57.26,-123.97,-138.74. It can be prepared from Intermediates **318-1-2A** |
| 322-2 | 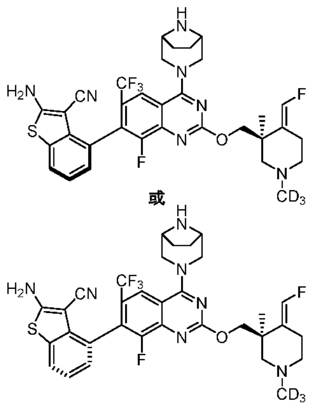 或 | (*S*)-4-(4-((1*R*,5*S*)-3,8-diazabicyclo [3.2.1]octan-3-yl)-8-fluoro-2-(((*S*, *E*)-4-(fluoromethylene)-3-methyl-1-(methyl-$d_3$) piperidin-3-yl) methoxy)-6-(trifluoromethyl) quinazolin-7-yl)-2-aminobenzo[*b*]thiophene-3-carbonitrile<br>Or<br>(*R*)-4-(4-((1*R*,5*S*)-3,8-diazabicyclo [3.2.1]octan-3-yl)-8-fluoro-2-(((*S,E*)-4-(fluoromethylene)-3-methyl-1-(methyl-$d_3$) piperidin-3-yl) methoxy)-6-(trifluoromethyl) quinazolin-7-yl)-2-aminobenzo[*b*]thiophene-3-carbonitrile | LCMS (m/z): 673.3 (M + H). |
| 323 | 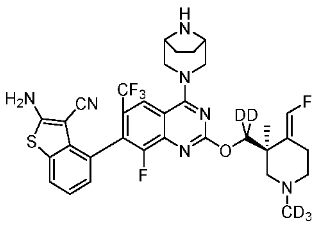 | 4-(4-((1*R*,5*S*)-3,8-diazabicyclo[3.2.1] octan-3-yl)-8-fluoro-2-(((*S,E*)-4-(fluoromethylene)-3-methyl-1-(methyl-$d_3$) piperidin-3-yl)methoxy-$d_2$)-6-(trifluoromethyl) quinazolin-7-yl)-2-aminobenzo[*b*]thiophene-3-carbonitrile | LCMS (m/z): 675.3 (M + H). |

(continued)

| Ex. | Structure | Name | Characterization data |
|---|---|---|---|
| 323-1 | 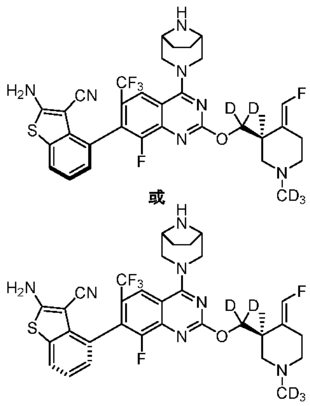 或 | (*S*)-4-(4-((1*R*,5*S*)-3,8-diazabicyclo [3.2.1]octan-3-yl)-8-fluoro-2-(((*S,E*)-4-(fluoromethylene)-3-methyl-1-(methyl-$d_3$) piperidin-3-yl) methoxy-$d_2$)-6-(trifluoromethyl) quinazolin-7-yl)-2-aminobenzo[*b*]thiophene-3-carbonitrile<br><br>Or<br>(*R*)-4-(4-((1*R*,5*S*)-3,8-diazabicyclo [3.2.1]octan-3-yl)-8-fluoro-2-(((*S,E*)-4-(fluoromethylene)-3-methyl-1-(methyl-$d_3$) piperidin-3-yl) methoxy-$d_2$)-6-(trifluoromethyl) quinazolin-7-yl)-2-aminobenzo[*b*]thiophene-3-carbonitrile | LCMS (m/z): 675.3 (M + H). |
| 323-2 | 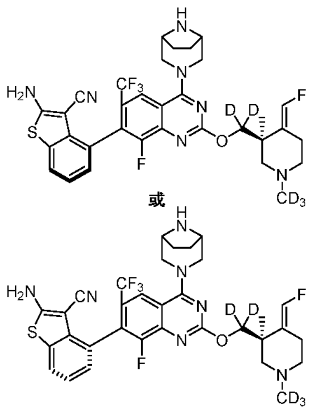 或 | (*S*)-4-(4-((1*R*,5*S*)-3,8-diazabicyclo [3.2.1]octan-3-yl)-8-fluoro-2-(((S,E)-4-(fluoromethylene)-3-methyl-1-(methyl-$d_3$) piperidin-3-yl) methoxy-$d_2$)-6-(trifluoromethyl) quinazolin-7-yl)-2-aminobenzo[b]thiophene-3-carbonitrile<br><br>Or<br>*(R)-4-(4-((1R,5S)-3,8*-diazabicyclo [3.2.1]octan-3-yl)-8-fluoro-2-(((S,E)-4-(fluoromethylene)-3-methyl-1-(methyl-$d_3$) piperidin-3-yl) methoxy-$d_2$)-6-(trifluoromethyl) quinazolin-7-yl)-2-aminobenzo[b]thiophene-3-carbonitrile | **LCMS** (m/z): 675.3 (M + H). |

**Examples 324-1 and 324-2**

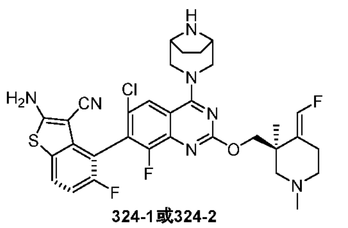

324-1或324-2

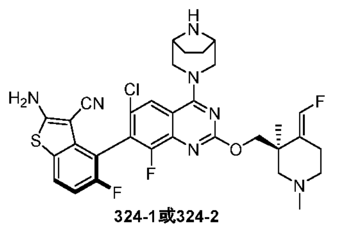

324-1或324-2

(*S*)-4-(4-((1*R*,5*S*)-3,8-diazabicyclo[3.2.1]octan-3-yl)-6-chloro-8-fluoro-2-(((*S,E*)-4-(fluoromethylene)-1,3-dimethylpiperidin-3-yl)methoxy)quinazolin-7-yl)-2-amino-5-fluorobenzo[*b*]thiophene-3-carbonitrile or (*R*)-4-(4-((1R,5S)-3,8-diazabicyclo[3.2.1]octan-3-yl)-6-chloro-8-fluoro-2-(((*S,E*)-4-(fluoromethylene)-1,3-dimethylpiperidin-3-yl)methoxy) quinazolin-7-yl)-2-amino-5-fluorobenzo[*b*]thiophene-3-carbonitrile

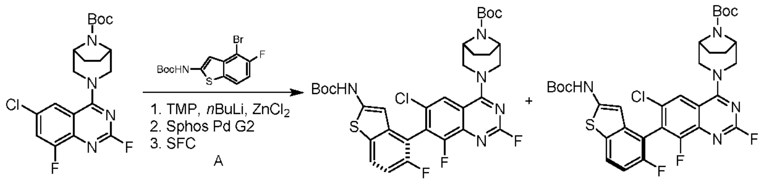


E-2b

314-1-1A或314-1-1B

314-1-1A或314-1-1B

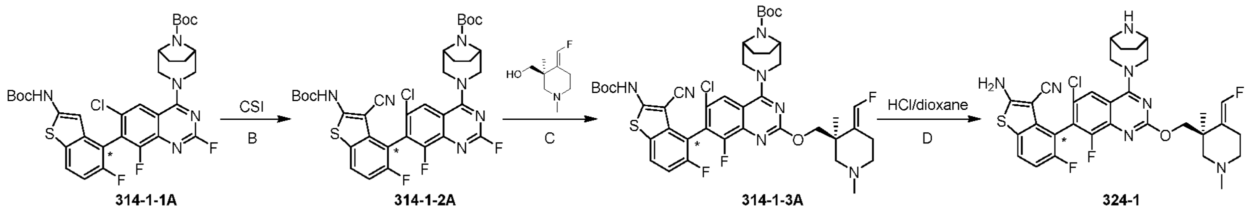


314-1-1A

314-1-2A

314-1-3A

324-1

**Step A:** *tert-butyl* 3-(7-(2-((*tert*-butyl)(oxycarbonylamino))-5-fluoro-1-benzothiophen-4-yl)-6-chloro-2,8-difluoro-4-quinazolinyl)-3,8-diazabicyclo[3.2.1]octane-8-carboxylate

At -70 °C, n-butyllithium (5.6 mL, 8.9 mmol, 1.6N) was added to a solution of 2,2,6,6-tetramethylpiperidine (1.3 g, 8.9 mmol) in tetrahydrofuran (10 mL), and the reaction mixture was stirred at the same temperature for 0.5 h. A solution of tert-butyl-3 (6-chloro-2,8-difluoro-4-quinazolinyl)-3,8-diazabicyclo[3.2.1]octane-8-carboxylate (2.3 g, 5.6 mmol) in tetrahydrofuran (3 mL) was added to the above solution, and the reaction was stirred at -70 °C for 0.5 h. Zinc chloride (8.9 mL, 8.9 mmol, 1.0 N) was added to the above reaction solution, and the reaction was heated to 60 °C and stirred for 1 h. The above-obtained solution was added to a solution of tert-butyl (4-bromo-5-fluoro-1-benzothiophen-2-yl) carbamate (2.3 g, 6.7 mmol) and Sphos Pd G2 (403 mg, 0.56 mmol) in tetrahydrofuran (10 mL), and the resulting mixture was heated to 60 °C and stirred for 12 h. After the reaction was completed (monitored by LCMS), the reaction was filtered through celite and the filtrate was concentrated to dryness. The resulting crude product was purified by FCC ($SiO_2$, PE/DCM = 0 - 20%) to obtain tert-butyl 3-(7-(2-((*tert*-butyl)(oxycarbonylamino))-5-fluoro-1-benzothiophen-4-yl)-6-chloro-2,8-difluoro-4-quinazolinyl)-3,8-diazabicyclo[3.2.1]octane-8-carboxylate (1.0 g, yield 26%) as a yellow solid. The obtained product was resolved by chiral Waters SFC150 (separation column: DAICEL CHIRALPAK® IG, 250 * 25 mm, 10 μm; mobile phase: $CO_2$/MeOH = 70/30; flow rate: 100 g/min. The first eluted isomer 1 was **324-1-1A** (360 mg, with a relatively shorter retention time). Chiral analytical method SFC-13, Rt = 1.692 min. LCMS (m/z): 676.1 (M + H). The subsequently eluted isomer 2 was **324-1-1B** (450 mg, with a relatively longer retention time). Chiral analytical method SFC-13, Rt = 3.087 min. LCMS (m/z): 6 76.1 (M + H).

**Step B:** tert-butyl 3-(7-(2-(tert-butyl)(oxycarbonylamino))-3-cyano-5-fluoro-1-benzothiophen-4-yl)-6-chloro-2,8-difluoro-4-quinazolinyl)-3,8-diazabicyclo[3.2.1]octane-8-carboxylate

Under an ice bath and under nitrogen atmosphere, chlorosulfonyl isocyanate (168 mg, 1.1 mmol) was added to a solution of *tert-butyl* 3-(7-(2-((*tert*-butyl)(oxycarbonylamino))-5-fluoro-1-benzothiophen-4-yl)-6-chloro-2,8-difluoro-4-quinazolinyl)-3,8-diazabicyclo[3.2.1]octane-8-carboxylate **(324-1-1A,** 360 mg, 0.5 mmol) in acetonitrile (5 mL). The reaction was stirred at room temperature for 1 h, then 0.5 mL DMF was added, and the resulting reaction mixture was stirred at room

temperature for 2 h. After the reaction was completed (monitored by LCMS), 20 mL saturated aqueous sodium bicarbonate solution was added to the reaction, and the resulting mixture was extracted with ethyl acetate. The organic phase was combined, washed with brine, dried over anhydrous sodium sulfate filtrated and concentrated. The crude product was purified by FCC ($SiO_2$, EA/PE = 0-25%) to give *tert*-butyl 3-(7-(2-(tert-butyl)(oxycarbonylamino))-3-cyano-5-fluoro-1-benzothiophen-4-yl)-6-chloro-2,8-difluoro-4-quinazolinyl)-3,8-diazabicyclo[3.2.1]octane-8-carboxylate (290 mg, yield 78%) as a white solid. LCMS (m/z): 701.1 (M + H).

**Step C:** *tert*-butyl 3-(2-(((*S*)-(*E*)-4-fluoromethylene-1,3-dimethylpiperidin-3-yl)methoxy)-7-(2-((*tert*-butoxycarbonyl)amino)-3-cyano-5-fluoro-1-benzothiophen-4-yl)-6-chloro-8-fluoro-4-quinazolinyl)-3,8-diazabicyclo[3.2.1]octane-8-carboxylate

Under ice-bath, sodium hydride (20 mg, 0.8 mmol) was added to a solution of ((*S*)-(*E*)-4-fluoromethylene-1,3-dimethylpiperidin-3-yl)methanol (86 mg, 0.5 mmol) in anhydrous tetrahydrofuran (10 mL). After stirring at this temperature for 0.5 h, *tert-butyl* 3-(7-(2-(tert-butyl)(oxycarbonylamino))-3-cyano-5-fluoro-1-benzothiophen-4-yl)-6-chloro-2,8-difluoro-4-quinazolinyl)-3,8-diazabicyclo[3.2.1]octane-8-carboxylate (290 mg, 0.4 mmol) was added to the reaction. The resulting mixture was stirred at room temperature for 0.5 h. After the reaction was completed (monitored by LCMS), the reaction was poured into 20 mL of saturated aqueous ammonium chloride solution and extracted with ethyl acetate. The organic phase was combined, washed with brine, dried over anhydrous sodium sulfate, filtered and concentrated to dryness. The resulting crude product was purified by FCC ($SiO_2$, EA/PE = 0 - 60%) to obtained tert-butyl 3-(2-(((S)-(E)-4-fluoromethylene-1,3-dimethylpiperidin-3-yl)methoxy)-7-(2-((tert-butoxycarbonyl)amino)-3-cyano-5-fluoro-1-benzothiophen-4-yl)-6-chloro-8-fluoro-4-quinazolinyl)-3,8-diazabicyclo[3.2.1]octane-8-carboxylate (100 mg, yield 28%) as a yellow solid. LCMS (m/z): 854.1 (M + H).

**Step D:** 4-(2-(((*S*)-(*E*)-4-fluoromethylene-1,3-dimethylpiperidin-3-yl)methoxy)-6-chloro-4-(3,8-diazabicyclo[3.2.1]octan-3-yl)-8-fluoro-7-quinazolinyl)-2-amino-5-fluoro-1-benzothiophene-3-carbonitrile

At room temperature, a solution of HCl in 1,4-dioxane (1 mL, 4 N) was added to *tert-butyl 3*-(2-(((S)-(E)-4-fluoromethylene-1,3-dimethylpiperidin-3-yl)methoxy)-7-(2-((*tert*-butoxycarbonyl)amino)-3-cyano-5-fluoro-1-benzothiophen-4-yl)-6-chloro-8-fluoro-4-quinazolinyl)-3,8-diazabicyclo[3.2.1]octane-8-carboxylate (100 mg, 0.1 mmol), and the reaction was stirred at same temperature for 0.5 h. After the reaction was completed (monitored by LCMS), the reaction was purified by pre-HPLC (C18, CAN/(10 mmol $NH_4HCO_3/H_2O$) = 30-50%) to obtain 2-(4-((1*R*,5*S*)-3,8-diazabicyclo[3.2.1]octan-3-yl)-8-fluoro-2-(((3S,4S)-4-(fluoromethyl)-1,3-dimethylpiperidin-3-yl)methoxy)pyrido[4,3-d]pyrimidin-7-yl)-6-amino-4-methyl-3-(trifluoromethyl)benzonitrile (19 mg, yield 25%) as a white solid. $^1H$ NMR (400 MHz, DMSO-$d_6$) δ 7.98 (s, 2H), 7.89 - 7.81 (m, 2H), 7.18 - 7.09 (m, 1H), 6.85 - 6.56 (m, 1H), 4.58 - 4.48 (m, 1H), 4.36 - 4.15 (m, 3H), 3.58 - 3.41 (m, 4H), 2.72 - 2.52 (m, 3H), 2.21 - 2.10 (m, 4H), 1.94 - 1.80 (m, 2H), 1.66 - 1.51 (m, 4H), 1.10 (s, 3H). $^{19}F$ NMR (376 MHz, DMSO-$d_6$) δ-118.97,-121.80,-138.84. LCMS (m/z): 654.2 (M + H).

By referring to the above method and using **324-1-1B** instead of **324-1-1A** in Step B, Example **324-2** was obtained. $^1H$ NMR (400 MHz, DMSO-$d_6$) δ 7.98 (s, 2H), 7.91 - 7.79 (m, 2H), 7.19 - 7.07 (m, 1H), 6.87 - 6.51 (m, 1H), 4.60 - 4.50 (m, 1H), 4.33 - 4.19 (m, 3H), 3.57 - 3.43 (m, 4H), 2.75 - 2.56 (m, 3H), 2.27 - 2.06 (m, 4H), 1.95 - 1.78 (m, 2H), 1.67 - 1.50 (m, 4H), 1.10 (s, 3H). $^{19}F$ NMR (376 MHz, DMSO-$d_6$) δ-118.96,-121.84,-138.88. LCMS (m/z): 654.3 (M + H).

The following compounds were prepared by referring to the above synthesis methods or appropriate variants.

It should be noted that for the naming of the following isomers-1 or -2, when the final compounds were subjected to chiral resolution, the one with a shorter retention time was named as -1 and the one with a longer retention time was named as -2. Or, the compound synthesized using **324-1-1A** as the intermediate was named as isomer-1, and the compound synthesized using the intermediate **324-1-1B** was named as isomer-2. When there is a conflict between the two naming methods, the naming based on the synthesis with the atropisomerically pure intermediate prevails.

| No. | Structure | Compound name | Characterization data |
|---|---|---|---|
| 325 | 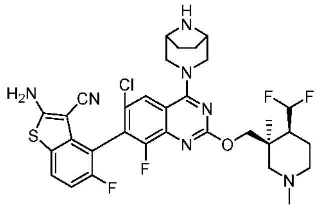 | 4-(4-((1R,5S)-3,8-diazabicyclo [3.2.1]octan-3-yl)-6-chloro-2-(((3S,4S)-4-(difluoromethyl)-1,3-dimethylpiperidin-3-yl) methoxy)-8-fluoroquinazolin-7-yl)-2-amino-5-fluorobenzo[b]thiophene-3-carbonitrile | LCMS (m/z): 674.2 (M + H). $^1H$ NMR (400 MHz, DMSO-$d_6$) δ 7.98 (s, 2H), 7.88 (s, 1H), 7.85 (dd, J = 8.7, 5.0 Hz, 2H), 7.19 - 7.10 (m, 1H), 6.29 (t, *J* = 56.0 Hz, 1H), 4.47 - 4.35 (m, 2H), 4.32 - 4.24 (m, 2H), 3.60 - 3.49 (m, 4H), 2.90 - 2.73 (m, 3H), 2.10 (s, 3H), 1.86 - 1.71 (m, 3H), 1.69 - 1.59 (m, 7H), 1.11 (s, 3H). $^{19}F$ NMR (376 MHz, DMSO-$d_6$) δ -73.41, -115.21 ~-116.02, - 118.52 ~-119.29, - 121.65. |
| 325-1 | 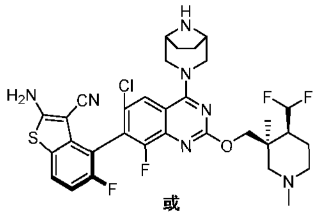 或 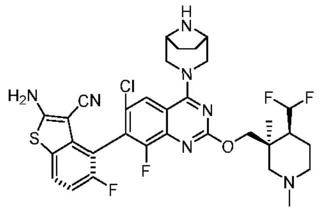 | (*S*)-4-(4-((1*R*,5*S*)-3,8-diazabicyclo [3.2.1]octan-3-yl)-6-chloro-2-(((3S,4S)-4-(difluoromethyl)-1,3-dimethylpiperidin-3-yl) methoxy)-8-fluoroquinazolin-7-yl)-2-amino-5-fluorobenzo[b]thiophene-3-carbonitrile (*R*)-4-(4-((1*R*,5*S*)-3,8-diazabicyclo [3.2.1]octan-3-yl)-6-chloro-2-(((3S,4S)-4-(difluoromethyl)-1,3-dimethylpiperidin-3-yl) methoxy)-8-fluoroquinazolin-7-yl)-2-amino-5-fluorobenzo[b]thiophene-3-carbonitrile | LCMS (m/z): 674.2 (M + H). |
| 325-2 | 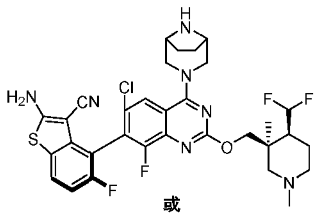 或 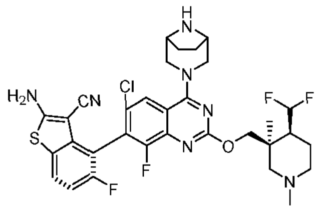 | (*S*)-4-(4-((1*R*,5*S*)-3,8-diazabicyclo [3.2.1]octan-3-yl)-6-chloro-2-(((3S,4S)-4-(difluoromethyl)-1,3-dimethylpiperidin-3-yl) methoxy)-8-fluoroquinazolin-7-yl)-2-amino-5-fluorobenzo[b]thiophene-3-carbonitrile *(R)-4-(4-((1R,5S)-3,8-*diazabicyclo [3.2.1]octan-3-yl)-6-chloro-2-(((3S,4S)-4-(difluoromethyl)-1,3-dimethylpiperidin-3-yl) methoxy)-8-fluoroquinazolin-7-yl)-2-amino-5-fluorobenzo[b]thiophene-3-carbonitrile | LCMS (m/z): 674.2 (M + H). |
| 326 | 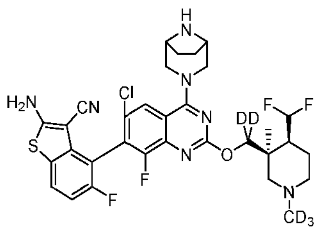 | 4-(4-((1*R*,5*S*)-3,8-diazabicyclo [3.2.1]octan-3-yl)-6-chloro-2-(((3S,4S)-4-(difluoromethyl)-3-methyl-1-(methyl-$d_3$)piperidin-3-yl) methoxy-$d_2$)-8-fluoroquinazolin-7-yl)-2-amino-5-fluorobenzo[*b*]thiophene-3-carbonitrile | LCMS (m/z): 679.2 (M + H). |

(continued)

| No. | Structure | Compound name | Characterization data |
|---|---|---|---|
| 326-1 | 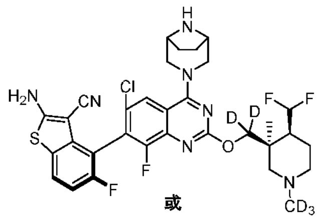 或 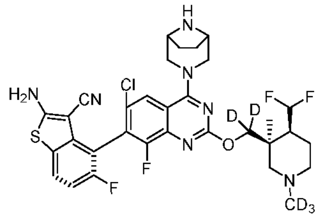 | (*S*)-4-(4-((1*R*,5*S*)-3,8-diazabicyclo [3.2.1]octan-3-yl)-6-chloro-2-(((3S,4S)-4-(difluoromethyl)-3-methyl-1-(methyl-$d_3$)piperidin-3-yl) methoxy-$d_2$)-8-fluoroquinazolin-7-yl)-2-amino-5-fluorobenzo[*b*]thiophene-3-carbonitrile<br>Or<br>*(R)-4-(4-((1R,5S)-3,8*-diazabicyclo [3.2.1]octan-3-yl)-6-chloro-2-(((3S,4S)-4-(difluoromethyl)-3-methyl-1-(methyl-$d_3$)piperidin-3-yl) methoxy-$d_2$)-8-fluoroquinazolin-7-yl)-2-amino-5-fluorobenzo[*b*]thiophene-3-carbonitrile | LCMS (m/z): 679.2 (M + H). |
| 326-2 | 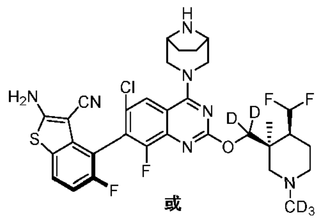 或 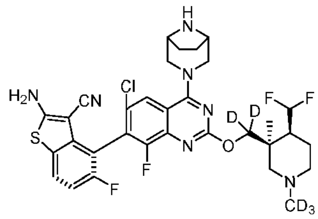 | (*S*)-4-(4-((1*R*,5*S*)-3,8-diazabicyclo [3.2.1]octan-3-yl)-6-chloro-2-(((3S,4S)-4-(difluoromethyl)-3-methyl-1-(methyl-$d_3$)piperidin-3-yl) methoxy-$d_2$)-8-fluoroquinazolin-7-yl)-2-amino-5-fluorobenzo[*b*]thiophene-3-carbonitrile<br>Or<br>*(R)-4-(4-((1R,5S)-3,8*-diazabicyclo [3.2.1]octan-3-yl)-6-chloro-2-(((3S,4S)-4-(difluoromethyl)-3-methyl-1-(methyl-$d_3$)piperidin-3-yl) methoxy-$d_2$)-8-fluoroquinazolin-7-yl)-2-amino-5-fluorobenzo[b]thiophene-3-carbonitrile | LCMS (m/z): 679.2 (M + H). |
| 327 | 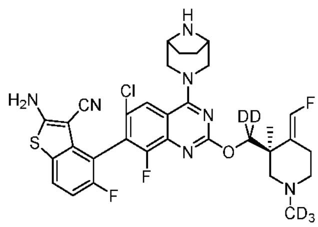 | 4-(4-((1R,5S)-3,8-diazabicyclo [3.2.1]octan-3-yl)-6-chloro-8-fluoro-2-(((S,E)-4-(fluoromethylene)-3-methyl-1-(methyl-$d_3$)piperidin-3-yl) methoxy-$d_2$)quinazolin-7-yl)-2-amino-5-fluorobenzo[b]thiophene-3-carbonitrile | LCMS (m/z): 659.2 (M + H). |
| 327-1 | 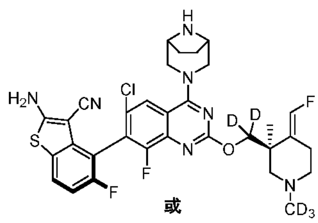 或 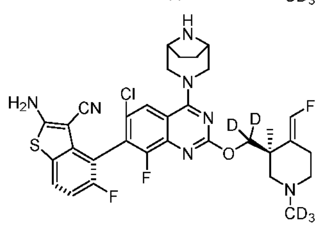 | (*S*)-4-(4-((1*R*,5*S*)-3,8-diazabicyclo [3.2.1]octan-3-yl)-6-chloro-8-fluoro-2-(((S,E)-4-(fluoromethylene)-3-methyl-1-(methyl-$d_3$)piperidin-3-yl) methoxy-$d_2$)quinazolin-7-yl)-2-amino-5-fluorobenzo[b]thiophene-3-carbonitrile<br>Or<br>*(R)-4-(4-((1R,5S)-3,8*-diazabicyclo [3.2.1]octan-3-yl)-6-chloro-8-fluoro-2-(((S,E)-4-(fluoromethylene)-3-methyl-1-(methyl-$d_3$)piperidin-3-yl) methoxy-$d_2$)quinazolin-7-yl)-2-amino-5-fluorobenzo[b]thiophene-3-carbonitrile | LCMS (m/z): 659.2 (M + H). |

(continued)

| No. | Structure | Compound name | Characterization data |
|---|---|---|---|
| 327-2 | 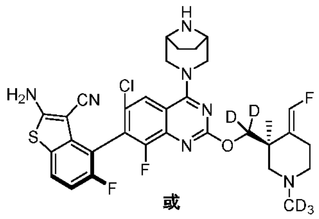 或 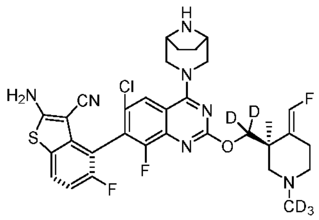 | (*S*)-4-(4-((1*R*,5*S*)-3,8-diazabicyclo[3.2.1]octan-3-yl)-6-chloro-8-fluoro-2-(((S,E)-4-(fluoromethylene)-3-methyl-1-(methyl-*d*$_3$)piperidin-3-yl) methoxy-*d*$_2$)quinazolin-7-yl)-2-amino-5-fluorobenzo[b]thiophene-3-carbonitrile<br>Or<br>*(R)-4-(4-((1R,5S)-3,8*-diazabicyclo[3.2.1]octan-3-yl)-6-chloro-8-fluoro-2-(((S,E)-4-(fluoromethylene)-3-methyl-1-(methyl-*d*$_3$)piperidin-3-yl) methoxy-*d*$_2$)quinazolin-7-yl)-2-amino-5-fluorobenzo[b]thiophene-3-carbonitrile | LCMS (m/z): 659.2 (M + H). |
| 328 | 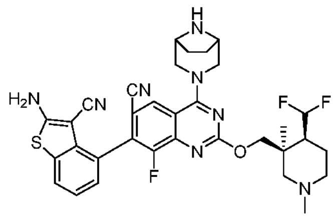 | 7-(2-amino-3-cyanobenzo[*b*]thiophen-4-yl)-4-((1R,5S)-3,8-diazabicyclo[3.2.1]octan-3-yl)-2-(((3*S*,4*S*)-4-(difluoromethyl)-1,3-dimethylpiperidin-3-yl) methoxy)-8-fluoroquinazoline-6-carbonitrile | LCMS (m/z): 647.3 (M + H). $^{1}$H NMR (400 MHz, DMSO-*d*$_6$) δ 8.32 (s, 1H), 7.90 (s, 2H), 7.87 - 7.81 (m, 1H), 7.33 - 7.23 (m, 2H), 6.48 - 6.12 (m, 1H), 4.51 - 4.38 (m, 2H), 4.33 (d, *J* = 12.1 Hz, 2H), 3.67 - 3.55 (m, 2H), 3.55 - 3.46 (m, 2H), 2.88 - 2.74 (m, 2H), 2.10 (s, 3H), 1.88 - 1.49 (m, 9H), 1.11 (s, 3H). $^{19}$F NMR (376 MHz, DMSO-*d*$_6$) δ - 114.78 --119.74 (m), -124.67. |
| 328-1 | 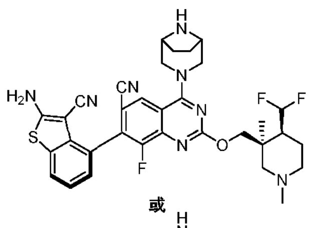 或 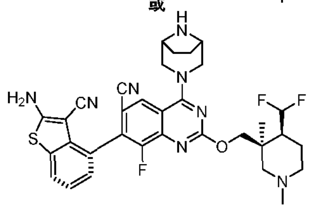 | (S)-7-(2-amino-3-cyanobenzo[*b*] thiophen-4-yl)-4-((1R,5S)-3,8-diazabicyclo[3.2.1]octan-3-yl)-2-(((3*S*,4*S*)-4-(difluoromethyl)-1,3-dimethylpiperidin-3-yl) methoxy)-8-fluoroquinazoline-6-carbonitrile<br>Or<br>(R)-7-(2-amino-3-cyanobenzo[*b*] thiophen-4-yl)-4-((1R,5S)-3,8-diazabicyclo[3.2.1]octan-3-yl)-2-(((3*S*,4*S*)-4-(difluoromethyl)-1,3-dimethylpiperidin-3-yl) methoxy)-8-fluoroquinazoline-6-carbonitrile | LCMS (m/z): 647.3 (M + H). |

(continued)

| No. | Structure | Compound name | Characterization data |
|---|---|---|---|
| 328-2 | 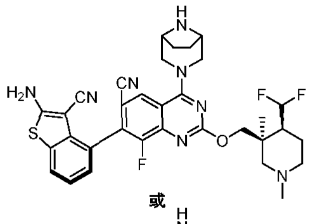 或 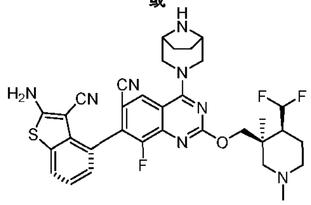 | (S)-7-(2-amino-3-cyanobenzo[*b*]thiophen-4-yl)-4-((1R,5S)-3,8-diazabicyclo[3.2.1]octan-3-yl)-2-(((3*S*,4*S*)-4-(difluoromethyl)-1,3-dimethylpiperidin-3-yl)methoxy)-8-fluoroquinazoline-6-carbonitrile Or (R)-7-(2-amino-3-cyanobenzo[*b*]thiophen-4-yl)-4-((1R,5S)-3,8-diazabicyclo[3.2.1]octan-3-yl)-2-(((3*S*,4*S*)-4-(difluoromethyl)-1,3-dimethylpiperidin-3-yl)methoxy)-8-fluoroquinazoline-6-carbonitrile | LCMS (m/z): 647.3 (M + H). |
| 329 | 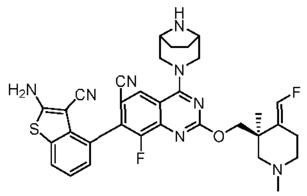 | 7-(2-amino-3-cyanobenzo[*b*]thiophen-4-yl)-4-*((1R,55)-3,8*-diazabicyclo[3.2.1]octan-3-yl)-8-fluoro-2-((S,E)-4-(fluoromethylene)-1,3-dimethylpiperidin-3-yl methoxy)quinazoline-6-carbonitrile | LCMS (m/z): 627.2 (M + H). |
| 329-1 | 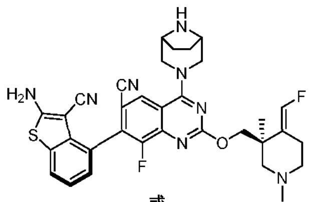 或 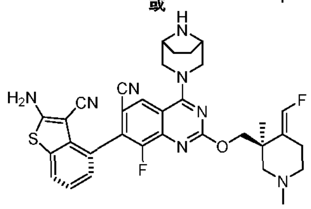 | (S)-7-(2-amino-3-cyanobenzo[*b*]thiophen-4-yl)-4-((1R,5S)-3,8-diazabicyclo[3.2.1]octan-3-yl)-8-fluoro-2-(((S,E)-4-(fluoromethylene)-1,3-dimethylpiperidin-3-yl)methoxy)quinazoline-6-carbonitrile Or (R)-7-(2-amino-3-cyanobenzo[*b*]thiophen-4-yl)-4-((1R,5S)-3,8-diazabicyclo[3.2.1]octan-3-yl)-8-fluoro-2-(((S,E)-4-(fluoromethylene)-1,3-dimethylpiperidin-3-yl)methoxy)quinazoline-6-carbonitrile | LCMS (m/z): 627.2 (M + H). |
| 329-2 | 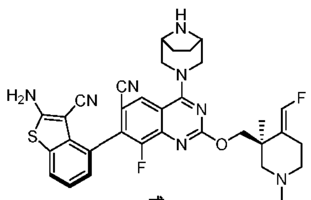 或 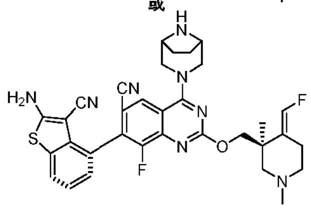 | (S)-7-(2-amino-3-cyanobenzo[*b*]thiophen-4-yl)-4-((1R,5S)-3,8-diazabicyclo[3.2.1]octan-3-yl)-8-fluoro-2-(((S,E)-4-(fluoromethylene)-1,3-dimethylpiperidin-3-yl)methoxy)quinazoline-6-carbonitrile Or (R)-7-(2-amino-3-cyanobenzo[*b*]thiophen-4-yl)-4-((1R,5S)-3,8-diazabicyclo[3.2.1]octan-3-yl)-8-fluoro-2-(((S,E)-4-(fluoromethylene)-1,3-dimethylpiperidin-3-yl)methoxy)quinazoline-6-carbonitrile | LCMS (m/z): 627.2 (M + H). |

(continued)

| No. | Structure | Compound name | Characterization data |
|---|---|---|---|
| 330 | 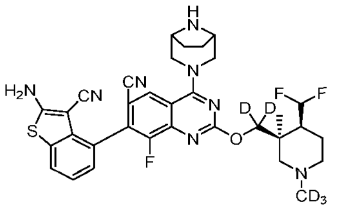 | 7-(2-amino-3-cyanobenzo[*b*]thiophen-4-yl)-4-((1R,5S)-3,8-diazabicyclo[3.2.1]octan-3-yl)-2-(((3*S*,4*S*)-4-(difluoromethyl)-3 - methyl-1-(methyl-$d_3$) piperidin-3-yl)methoxy-$d_2$)-8-fluoroquinazoline-6-carbonitrile | LCMS (m/z): 652.3 (M + H). |
| 330-1 | 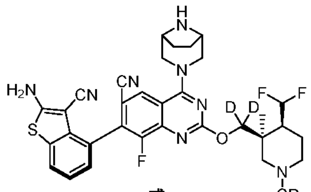 或 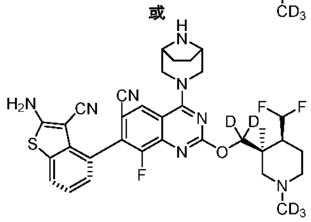 | (S)-7-(2-amino-3-cyanobenzo[*b*] thiophen-4-yl)-4-((1R,5S)-3,8-diazabicyclo[3.2.1]octan-3-yl)-2-(((3*S*,4*S*)-4-(difluoromethyl)-3 - methyl-1-(methyl-$d_3$) piperidin-3-yl)methoxy-$d_2$)-8-fluoroquinazoline-6-carbonitrile<br>Or<br>(R)-7-(2-amino-3-cyanobenzo[*b*] thiophen-4-yl)-4-((1R,5S)-3,8-diazabicyclo[3.2.1]octan-3-yl)-2-(((3*S*,4*S*)-4-(difluoromethyl)-3-methyl-1-(methyl-$d_3$) piperidin-3-yl)methoxy-$d_2$)-8-fluoroquinazoline-6-carbonitrile | LCMS (m/z): 652.3 (M + H). |
| 330-2 | 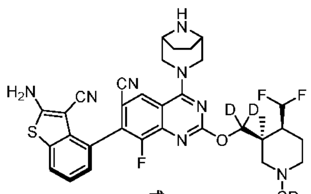 或 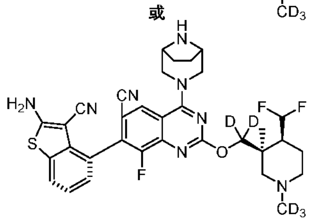 | (S)-7-(2-amino-3-cyanobenzo[*b*] thiophen-4-yl)-4-((1R,5S)-3,8-diazabicyclo[3.2.1]octan-3-yl)-2-(((3*S*,4*S*)-4-(difluoromethyl)-3-methyl-1-(methyl-$d_3$) piperidin-3-yl)methoxy-$d_2$)-8-fluoroquinazoline-6-carbonitrile<br>Or<br>(R)-7-(2-amino-3-cyanobenzo[*b*] thiophen-4-yl)-4-((1R,5S)-3,8-diazabicyclo[3.2.1]octan-3-yl)-2-(((3*S*,4*S*)-4-(difluoromethyl)-3-methyl-1-(methyl-$d_3$) piperidin-3-yl)methoxy-$d_2$)-8-fluoroquinazoline-6-carbonitrile | LCMS (m/z): 652.3 (M + H). |
| 331 | 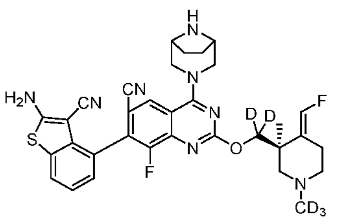 | 7-(2-amino-3-cyanobenzo[*b*]thiophen-4-yl)-4-((1R,5S)-3,8-diazabicyclo[3.2.1]octan-3-yl)-8-fluoro-2-(((S,E)-4-(fluoromethylene)-3-methyl-1-(methyl-$d_3$)piperidin-3-yl) methoxy-$d_2$)quinazoline-6-carbonitrile | LCMS (m/z): 632.3 (M + H). |

(continued)

| No. | Structure | Compound name | Characterization data |
|---|---|---|---|
| 331-1 | 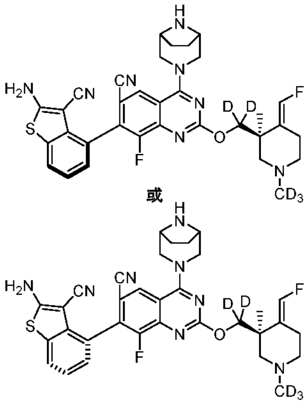 或 | (S)-7-(2-amino-3-cyanobenzo[*b*]thiophen-4-yl)-4-((1R,5S)-3,8-diazabicyclo[3.2.1]octan-3-yl)-8-fluoro-2-(((S,E)-4-(fluoromethylene)-3-methyl-1-(methyl-$d_3$)piperidin-3-yl)methoxy-$d_2$)quinazoline-6-carbonitrile Or (R)-7-(2-amino-3-cyanobenzo[*b*]thiophen-4-yl)-4-((1R,5S)-3,8-diazabicyclo[3.2.1]octan-3-yl)-8-fluoro-2-(((S,E)-4-(fluoromethylene)-3-methyl-1-(methyl-$d_3$)piperidin-3-yl)methoxy-$d_2$)quinazoline-6-carbonitrile | LCMS (m/z): 632.3 (M + H). |
| 331-2 | 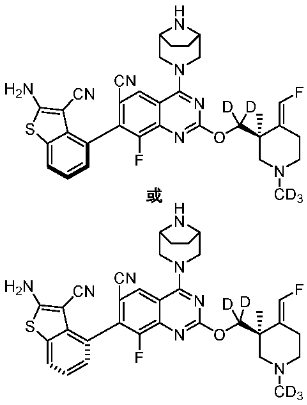 或 | (S)-7-(2-amino-3-cyanobenzo[*b*]thiophen-4-yl)-4-((1R,5S)-3,8-diazabicyclo[3.2.1]octan-3-yl)-8-fluoro-2-(((S,E)-4-(fluoromethylene)-3-methyl-1-(methyl-$d_3$)piperidin-3-yl)methoxy-$d_2$)quinazoline-6-carbonitrile Or (R)-7-(2-amino-3-cyanobenzo[*b*]thiophen-4-yl)-4-((1R,5S)-3,8-diazabicyclo[3.2.1]octan-3-yl)-8-fluoro-2-(((S,E)-4-(fluoromethylene)-3-methyl-1-(methyl-$d_3$)piperidin-3-yl)methoxy-$d_2$)quinazoline-6-carbonitrile | LCMS (m/z): 632.3 (M + H). |

Example 332

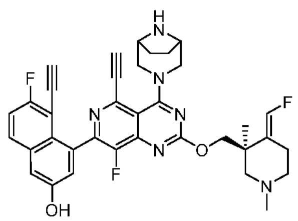

4-(4-(4-((1*R*,5*S*)-3,8-diazabicyclo[3.2.1]octan-3-yl)-5-ethynyl-8-fluoro-2-(((*S,E*)-4-(fluoromethyl)-1,3-dimethylpiperidin-3-yl)methoxy)pyrido[4,3-d]pyrimidin-7-yl)-5-ethynyl-6-fluoronaphthalene-2-ol

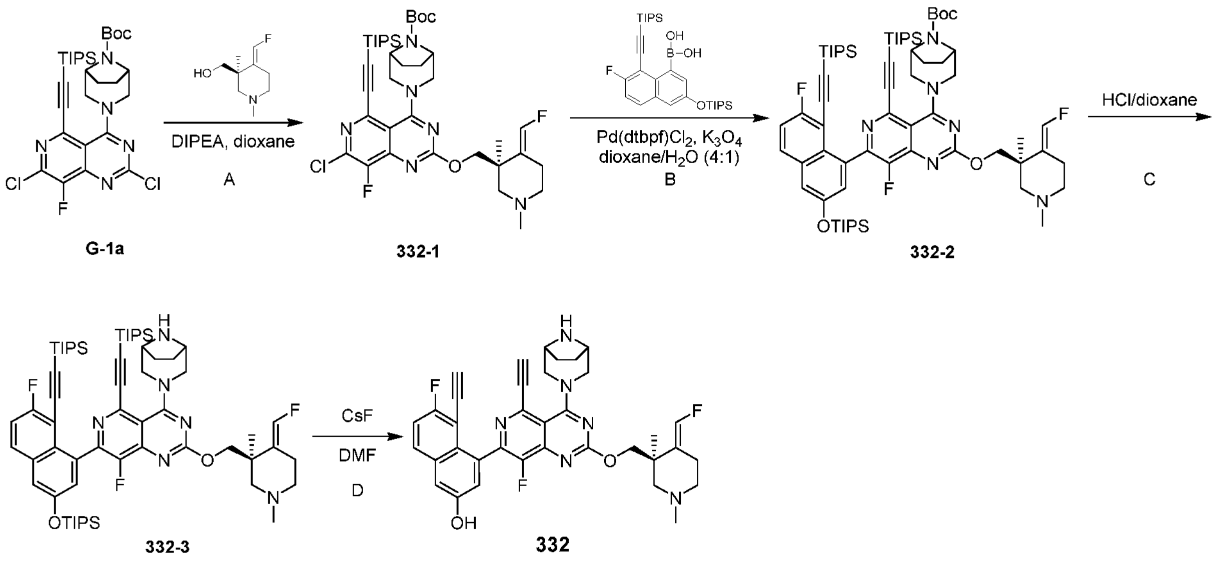


G-1a 332-1 332-2 332-3 332

**Step A:** *tert-butyl* (1R,5S)-3-(7-chloro-8-fluoro-2-(((S,E)-4-(fluoromethylene)-1,3-dimethylpiperidin-3-yl)methoxy)-5-((triisopropylsilyl)ethynyl)pyrido[4,3-*d*]pyrimidin-4-yl)-3, 8-diazabicyclo[3.2.1]octane-8-carboxylate

At room temperature, *tert-butyl* (1R,5S)-3-(2,7-dichloro-8-fluoro-5-((triisopropylsilyl)ethynyl)pyrido[4,3-d]pyrimidin-4-yl)-3,8-diazabicyclo[3.2.1]octane-8-carboxylate (960 mg , 1 .58 mmol) was dissolved in anhydrous dioxane (10 mL). Then *(S,E)-(4-*(fluoromethylene)-1,3-dimethylpiperidin-3-yl) methanol (546 mg, 3.15 mmol) and DIPEA (612 mg, 4.73 mmol) were added. The resulting mixture was heated to 100 °C and stirred overnight. The reaction was quenched by adding 30 mL saturated ammonium chloride solution and extracted with EtOAc (30 mL × 3). The organic phase was combined, washed with brine, dried over anhydrous sodium sulfate, filtered and concentrated. The resulting crude product was purified by FCC ($SiO_2$, EA/PE = 0 - 100%) to obtain tert-butyl (1*R*,5*S*)-3-(7-chloro-8-fluoro-2-(((*S,E*)-4-(fluoromethylene)-1,3-dimethylpiperidin-3-yl)methoxy)-5-((triisopropylsilyl)ethynyl)pyrido[4,3-d]pyrimidin-4-yl)-3,8-diazabicyclo [3.2.1]octane-8-carboxylate (270 mg, yield 23%) as a white solid. LCMS (m/z): 745.4 (M + H).

**Step B:** *tert-butyl* (1*R*,5*S*)-3-(8-fluoro-7-(7-fluoro-8-((triisopropylsilyl)ethynyl)-3-((triisopropylsilyl)oxy)naphthalene-1-yl)-2-(((S,E)-4-(fluoromethylene)-1,3-dimethylpiperidin-3-yl)methoxy)-5-((triisopropylsilyl)ethynyl)pyrido[4,3-d]pyrimidine-4-yl)-3,8-diazabicyclo[3.2.1]octane-8-carboxylate

At room temperature, tert-butyl (1R,5S)-3-(7-chloro-8-fluoro-2-(((S,E)-4-(fluoromethylene)-1,3-dimethylpiperidin-3-yl)methoxy)-5-((triisopropylsilyl)ethynyl) pyrido[4,3-d]pyrimidin-4-yl)-3,8-diazabicyclo[3.2.1]octane-8-carboxylate (120 mg, 0.161 mmol), (7-fluoro-8-((triisopropylsilyl)ethynyl)-3-((triisopropylsilyl)oxy)naphthalen-1-yl) boronic acid (131 mg, 0.241 mmol), potassium phosphate (103 mg, 0.483 mmol), and $Pd(dtbpf)Cl_2$ (15.6 mg, 0.024 mmol) were added to the reaction flask, and the flask was degassed with nitrogen thress times. Dioxane (4 mL) and water (1 mL) were added, and the resulting mixture was heated to 100 °C and stirred overnight. The reaction was cooled to room temperature, quenched by adding 30 mL of saturated ammonium chloride solution, and extracted with EtOAc (30 mL × 3). The combined organic phase was washed with brine, dried over anhydrous sodium sulfate, filtered and concentrated. The resulting crude product was purified by FCC ($SiO_2$, EA/PE = 0 - 100%) to obtain tert-butyl (1*R*,5*S*)-3-(8-fluoro-7-(7-fluoro-8-((triisopropylsilyl)ethynyl)-3-((triisopropylsilyl)oxy)naphthalene-1-yl)-2-(((S,E)-4-(fluoromethylene)-1,3-dimethylpiperidin-3-yl)methoxy)-5-((triisopropylsilyl)ethynyl)pyrido[4,3-d]pyrimidine-4-yl)-3,8-diazabicyclo[3.2.1]octane-8-carboxylate (90 mg, yield 46%) as a white solid. LCMS (m/z): 604.4 (M/2 + H).

**Step C:** 4-((1*R*,5*S*)-3,8-diazabicyclo[3.2.1]octan-3-yl)-8-fluoro-7-(7-fluoro-8-((triisopropylsilyl)ethynyl)-3-((triisopropylsilyl)oxy)naphthalen-1-yl)-2-(((S,E)-4-(fluoromethylene)-1,3-dimethylpiperidin-3-yl)methoxy)-5-((triisopropylsilyl)ethynyl) pyrido[4,3-d]pyrimidine

At room temperature, tert-butyl (1*R*,5*S*)-3-(8-fluoro-7-(7-fluoro-8-((triisopropylsilyl)ethynyl)-3-((triisopropylsilyl)oxy)naphthalene-1-yl)-2-(((S,E)-4-(fluoromethylene)-1,3-dimethylpiperidin-3-yl)methoxy)-5-((triisopropylsilyl)ethynyl) pyrido[4,3-d]pyrimidine-4-yl)-3, 8-diazabicyclo[3.2.1]octane-8-carboxylate (90 mg , 0 .075 mmol) was dissolved in HCl/dioxane (20 mL, 80 mmol, 4 M) and the resulting reaction solution was stirred at room temperature for 1 h. After the reaction was completed (monitored by LCMS), the solution was concentrated to dryness, and the residue was

neutralized by adding saturated sodium bicarbonate solution (20 mL) and then extracted with EtOAc (30 mL × 3). The organic phase was dried over anhydrous sodium sulfate, filtered, and concentrated to obtain crude product 4-((1R,5S)-3,8-diazabicyclo[3.2.1]octan-3-yl)-8-fluoro-7-(7-fluoro-8-((triisopropylsilyl)ethynyl)-3-((triisopropylsilyl)oxy) naphthalen-1-yl)-2-(((S,E)-4-(fluoromethylene)-1,3-dimethylpiperidin-3-yl)methoxy)-5-((triisopropylsilyl)ethynyl) pyrido [4,3-d]pyrimidine (80 mg, yield 97%) as a yellow solid. LCMS (m/z): 554.3 (M/2 + H).

**Step D:** 4-(4-((1*R*,5*S*)-3,8-diazabicyclo[3.2.1]octan-3-yl)-5-ethynyl-8-fluoro-2-(((*S,E*)-4-(fluoromethylene)-1,3-dimethylpiperidin-3-yl)methoxy)pyrido[4,3-d]pyrimidin-7-yl)-5-ethynyl-6-fluoronaphthalene-2-ol

A solution of 4-((1*R*,5*S*-3,8-diazabicyclo[3.2.1]octan-3-yl)-8-fluoro-7-(7-fluoro-8-((triisopropylsilyl)ethynyl)-3-((triisopropylsilyl)oxy)naphthalen-1-yl)-2-(((S,E)-4-(fluoromethylene)-1,3-dimethylpiperidin-3-yl)methoxy)-5-((triisopropylsilyl)ethynyl) pyrido[4,3-d]pyrimidine (80 mg, 0.072 mmol) and cesium fluoride (55 mg , 0.361 mmol) in DMF (3 mL) were heated to 60 °C and stirred for 1 h. After the reaction was completed (monitored by LCMS), insoluble objects were removed by filtration, and the collected filtrate was purified by pre-HPLC (C18, CAN/(0.1% $NH_4HCO_3/H_2O$) = 40-60%) to obtain 4-(4-((1R,5S)-3,8-diazabicyclo[3.2.1]octan-3-yl)-5-ethynyl-8-fluoro-2-(((S,E)-4-(fluoromethylene)-1,3-dimethylpiperidin-3-yl)methoxy)pyrido[4,3-d]pyrimidin-7-yl)-5-ethynyl-6-fluoronaphthalene-2-ol (30 mg, yield 65%) as a white solid. LCMS (m/z): 639.3 (M + H). $^1$H NMR (400 MHz, DMSO-$d_6$) δ 10.19 (s, 1H), 8.00 (dd, *J* = 9.2, 5.9 Hz, 1H), 7.52 - 7.45 (m, 1H), 7.41 (d, *J*= 2.6 Hz, 1H), 7.26 - 7.18 (m, 1H), 6.81 (d, *J= 5.6* Hz, 0.5H), 6.60 (d, *J* = 4.8 Hz, 0.5H), 4.77 (s, 1H), 4.61 (dd, *J* = 13.6, 10.6 Hz, 1H), 4.30 (d, *J* = 10.6 Hz, 1H), 3.92 - 3.70 (m, 2H), 3.60 - 3.48 (m, 2H), 3.43 - 3.33 (m, 2H), 3.29 (s, 1H), 2.74 - 2.63 (m, 2H), 2.56 - 2.52 (m, 1H), 2.28 - 2.17 (m, 1H), 2.14 (s, 3H), 1.94 - 1.78 (m, 2H), 1.69 - 1.28 (m, 4H), 1.10 (d, *J= 2.6* Hz, 3H). $^{19}$F NMR (376 MHz, DMSO-$d_6$) δ -110.44, -138.72, -138.74.

| Ex. | Structure | Name | Characterization data |
|---|---|---|---|
| 333 | 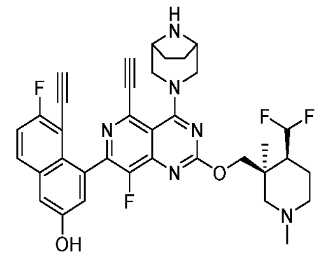 | 4-(4-((1*R*,5*S*)-3,8-diazabicyclo[3.2.1]octan-3-yl)-2-(((3*S*,4*S*)-4-(difluoromethyl)-1,3-dimethylpiperidin-3-yl)methoxy)-5-ethynyl-8-fluoropyrido[4,3-*d*]pyrimidin-7-yl)-5-ethynyl-6-fluoronaphthalene-2-ol | LCMS (m/z): 659.3 (M + H). |
| 334 | 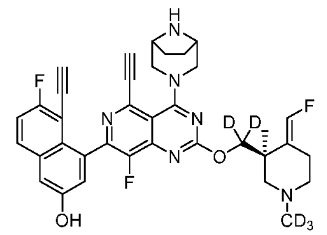 | 4-(4-((1*R*,5*S*)-3,8-diazabicyclo[3.2.1]octan-3-yl)-5-ethynyl-8-fluoro-2-(((*S*,*E*)-4-(fluoromethylene)-3-methyl-1-(methyl-$d_3$) piperidin-3-yl)methoxy-$d_2$)pyrido[4,3-*d*]pyrimidin-7-yl)-5-ethynyl-6-fluoronaphthalene-2-ol | LCMS (m/z): 644.3 (M + H). |
| 335 | 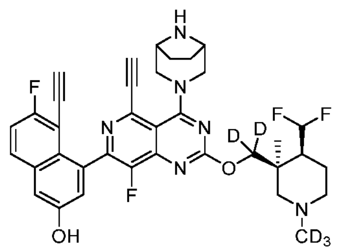 | 4-(4-((1*R*,5*S*)-3,8-diazabicyclo[3.2.1]octan-3-yl)-2-(((3*S*,4*S*)-4-(difluoromethyl)-3-methyl-1-(methyl-$d_3$)piperidin-3-yl)methoxyl-$d_2$)-5-ethynyl-8-fluoropyrido[4,3-*d*]pyrimidin-7-yl)-5-ethynyl-6-fluoronaphthalene-2-ol | LCMS (m/z): 664.3 (M + H). |
| 336 | 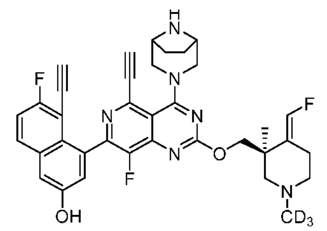 | 4-(4-((1*R*,5*S*)-3,8-diazabicyclo[3.2.1]octan-3-yl)-5-ethynyl-8-fluoro-2-(((*S*,*E*)-4-(fluoromethylene)-3-methyl-1-(methyl-$d_3$)piperidin-3-yl)methoxy)pyrido[4,3-*d*]pyrimidin-7-yl)-5-ethynyl-6-fluoronaphthalene-2-ol | LCMS (m/z): 642.3 (M + H). |

(continued)

| Ex. | Structure | Name | Characterization data |
|---|---|---|---|
| 337 | 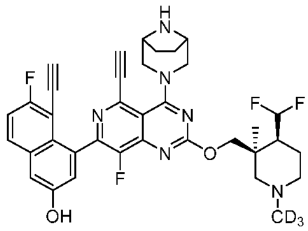 | 4-(4-((1*R*,5*S*)-3,8-diazabicyclo[3.2.1]octan-3-yl)-2-(((3*S*,4*S*)-4-difluoromethyl-3-methyl-1-(methyl-$d_3$)piperidin-3-yl)methoxy)-5-ethynyl-8-fluoropyrido[4,3-*d*]pyrimidin-7-yl)-5-ethynyl-6-fluoronaphthalene-2-ol | LCMS (m/z): 662.3 (M + H). |
| 338 | 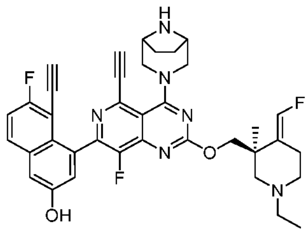 | 4-(4-((1*R*,5*S*)-3,8-diazabicyclo[3.2.1]octan-3-yl)-5-ethynyl-8-fluoro-2-(((*S*,*E*)-4-(fluoromethylene)-3-methyl-1-ethylpiperidin-3-yl)methoxy)pyrido[4,3-*d*] pyrimidin-7-yl)-5-ethynyl-6-fluoronaphthalene-2-ol | LCMS (m/z): 653.3 (M + H). |
| 339 | 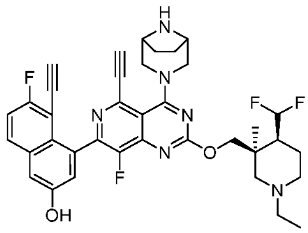 | 4-(4-((1*R*,5*S*)-3,8-diazabicyclo[3.2.1]octan-3-yl)-2-(((3*S*,4*S*)-4-(difluoromethyl)-3-methyl-1-ethylpiperidin-3-yl)methoxy)-5-ethynyl-8-fluoropyrido [4,3-*d*]pyrimidin-7-yl)-5-ethynyl-6-fluoronaphthalene-2-ol | LCMS (m/z): 673.3 (M + H). |
| 340 | 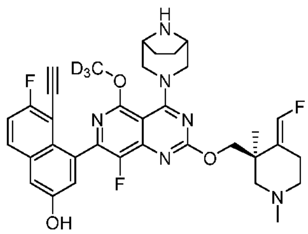 | 4-(4-((1*R*,5*S*)-3,8-diazabicyclo[3.2.1]octan-3-yl)-8-fluoro-2-(((*S*,*E*)-4-(fluoromethylene)-1,3-dimethylpiperidin-3-yl)methoxy)-5-methoxy-$d_3$) pyrido[4,3-*d*] pyrimidin-7-yl)-5-ethynyl-6-fluoronaphthalene-2-ol | LCMS (m/z): 648.3 (M + H). |
| 341 | 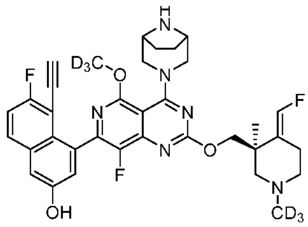 | 4-(4-((1*R*,5*S*)-3,8-diazabicyclo[3.2.1]octan-3-yl)-8-fluoro-2-(((*S*,*E*)-4-(fluoromethylene)-3-methyl-1-(methyl-$d_3$)piperidin-3-yl)methoxy)-5-(methoxy-$d_3$) pyrido[4,3-d]pyrimidin-7-yl)-5-ethynyl-6-fluoronaphthalene-2-ol | LCMS (m/z): 651.3 (M + H). |
| 342 | 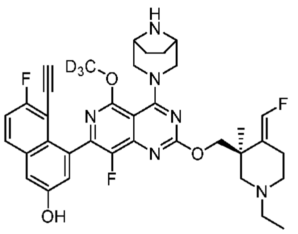 | 4-(4-((1*R*,5*S*)-3,8-diazabicyclo[3.2.1]octan-3-yl-2-(((*S*,*E*)-1-ethyl-4-(fluoromethylene)-3-methylpiperidin-3-ylmethoxy)-8-fluoro-5-(methoxy-$d_3$) pyrido [4,3-*d*]pyrimidin-7-yl)-5-ethynyl-6-fluoronaphthalene-2-ol | LCMS (m/z): 662.3 (M + H). |

(continued)

| Ex. | Structure | Name | Characterization data |
|---|---|---|---|
| 343 | 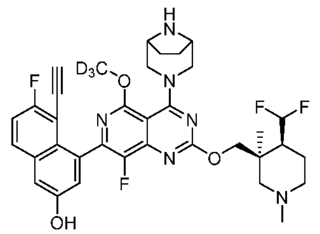 | 4-(4-((1*R*,5*S*)-3,8-diazabicyclo[3.2.1]octan-3-yl)-2-(((3*S*,4*S*)-4-(difluoromethyl)-1,3-dimethylpiperidin-3-yl)methoxy)-8-fluoro-5-(methoxy-*d*$_3$) pyrido [4,3-*d*]pyrimidin-7-yl)-5-ethynyl-6-fluoronaphthalene-2-ol | LCMS (m/z): 668.3 (M + H). |
| 344 | 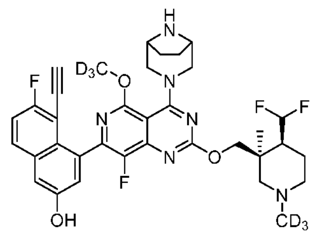 | 4-(4-((1*R*,5*S*)-3,8-diazabicyclo[3.2.1]octan-3-yl)-2-(((3*S*,4*S*)-4-(difluoromethyl)-3-methyl-1-(methyl-*d*$_3$) piperidin-3-yl)methoxy)-8-fluoro-5-(methoxy-*d*$_3$) pyridio[4,3-*d*]pyrimidin-7-yl)-5-ethynyl-6-fluoronaphthalene-2-ol | LCMS (m/z): 671.3 (M + H). |
| 345 | 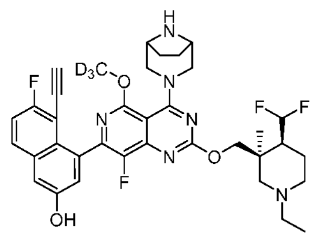 | 4-(4-((1*R*,5*S*)-3,8-diazabicyclo[3.2.1]octan-3-yl-2-(((3*S*,4*S*)-4-(difluoromethyl)-1-ethyl-3-methylpiperidin-3-yl)methoxy)-8-fluoro-5-(methoxy-*d*$_3$) pyrido[4,3-*d*]pyrimidin-7-yl)-5-ethynyl-6-fluoronaphthalene-2-ol | LCMS (m/z): 682.3 (M + H). |
| 346 | 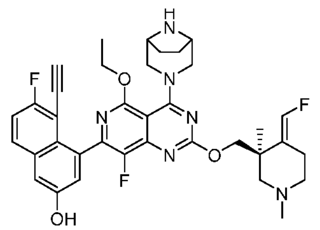 | 4-(4-((1*R*,5*S*)-3,8-diazabicyclo[3.2.1]octan-3-yl)-5-ethoxy-8-fluoro-2-(((*S*,*E*)-4-(fluoromethylene)-1,3-dimethylpiperidin-3-yl)methoxy)pyrido[4,3-*d*]pyrimidin-7-yl)-5-ethynyl-6-fluoronaphthalene-2-ol | LCMS (m/z): 659.3 (M + H). |
| 347 | 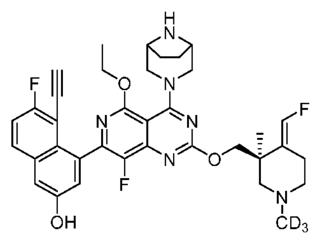 | 4-(4-((1*R*,5*S*)-3,8-diazabicyclo[3.2.1]octan-3-yl)-5-ethoxy-8-fluoro-2-(((*S*,*E*)-4-(fluoromethylene)-3-methyl-1-(methyl-*d*$_3$) piperidin-3-yl)methoxy)pyrido [4,3-*d*]pyrimidin-7-yl)-5-ethynyl-6-fluoronaphthalene-2-ol | LCMS (m/z): 662.3 (M + H). |
| 348 | 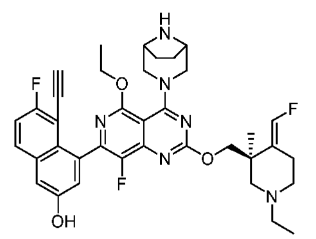 | 4-(4-((1*R*,5*S*)-3,8-diazabicyclo[3.2.1]octan-3-yl)-5-ethoxy-2-(((*S* , *E*)-1-ethyl-4-(fluoromethylene)-3-methylpiperidin-3-yl)methoxy)-8-fluoropyrido[4,3-d] pyrimidin-7-yl)-5-ethynyl-6-fluoronaphthalene-2-ol | LCMS (m/z): 673.3 (M + H). |

(continued)

| Ex. | Structure | Name | Characterization data |
|---|---|---|---|
| 349 | 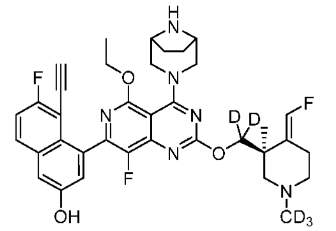 | 4-(4-((1*R*,5*S*)-3,8-diazabicyclo[3.2.1]octan-3-yl)-5-ethoxy-8-fluoro-2-(((*S*,*E*)-4-(fluoromethylene)-3-methyl-1-(methyl-*d*$_3$)piperidin-3-yl)methoxy-*d*$_2$) pyrido[4,3-*d*]pyrimidin-7-yl)-5-ethynyl-6-fluoronaphthalene-2-ol | LCMS (m/z): 664.3 (M + H). |
| 350 | 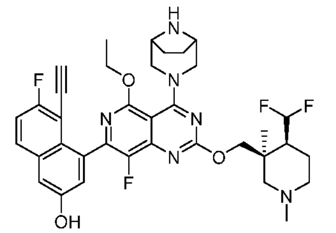 | 4-(4-((1*R*,5*S*)-3,8-diazabicyclo[3.2.1]octan-3-yl)-2-(((3*S*,4*S*)-4-(difluoromethyl)-1,3-dimethylpiperidin-3-yl)methoxy)-5-ethoxy-8-fluoropyrido[4,3-*d*] pyrimidin-7-yl)-5-ethynyl-6-fluoronaphthalene-2-ol | LCMS (m/z): 679.3 (M + H). |
| 351 | 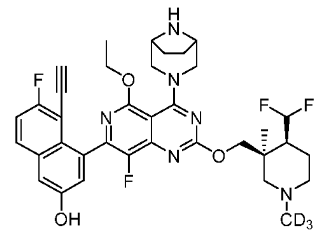 | 4-(4-((1*R*,5*S*)-3,8-diazabicyclo[3.2.1]octan-3-yl)-2-(((3*S*,4*S*)-4-(difluoromethyl)-3-methyl-1-(methyl-*d*$_3$)piperidin-3-yl)methoxy)-5-ethoxy-8-fluoropyrido[4,3-*d*]pyrimidin-7-yl)-5-ethynyl-6-fluoronaphthalene-2-ol | LCMS (m/z): 682.3 (M + H). |
| 352 | 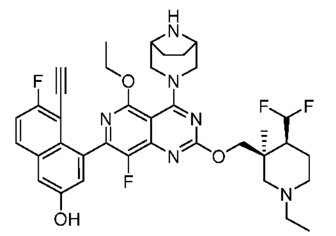 | 4-(4-((1*R*,5*S*)-3,8-diazabicyclo[3.2.1]octan-3-yl)-2-(((3*S*,4*S*)-4-(difluoromethyl)-1-ethyl-3-methylpiperidin-3-yl)methoxy)-5-ethoxy-8-fluoropyrido [4,3-*d*]pyrimidin-7-yl)-5-ethynyl-6-fluoronaphthalene-2-ol | LCMS (m/z): 693.3 (M + H). |
| 353 | 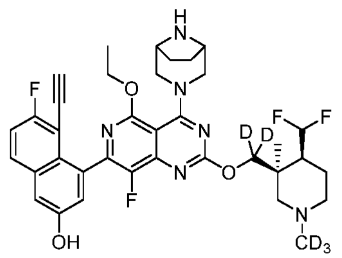 | 4-(4-((1*R*,5*S*)-3,8-diazabicyclo[3.2.1]octan-3-yl)-2-(((3*S*,4*S*)-4-(difluoromethyl)-3-methyl-1-methyl-*d*$_3$)piperidin-3-yl)methoxy-*d*$_2$)-5-ethoxy-8-fluoropyrido[4,3-*d*]pyrimidin-7-yl)-5-ethynyl-6-fluoronaphthalene-2-ol | LCMS (m/z): 684.3 (M + H). |

**Example 354-1 and Example 354-2**

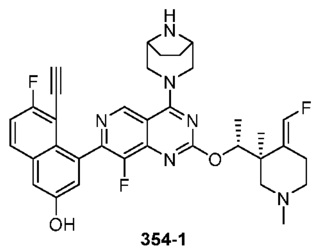
354-1

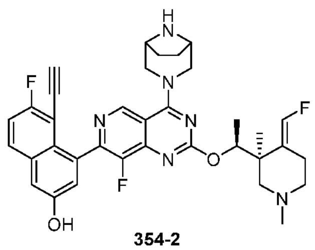
354-2

4-(4-((1*R*,5*S*)-3,8-diazabicyclo[3.2.1]octan-3-yl)-8-fluoro-2-(*R*)-1-((*S*,*E*)-4-(fluoromethylene)-1,3-dimethylpiperidin-3-yl)ethoxy)pyrido[4,3-d]pyrimidin-7-yl)-5-ethynyl-6-fluoronaphthalen-2-ol **(354-1)** and 4-(4-((1*R*,5*S*)-3,8-diazabicyclo[3.2.1]octan-3-yl)-8-fluoro-2-((*S*)-1-((*S*,*E*)-4-(fluoromethylene)-1,3-dimethylpiperidin-3-yl)ethoxy)pyrido[4,3-d]pyrimidin-7-yl)-5-ethynyl-6-fluoronaphthalen-2-ol **(354-2)**

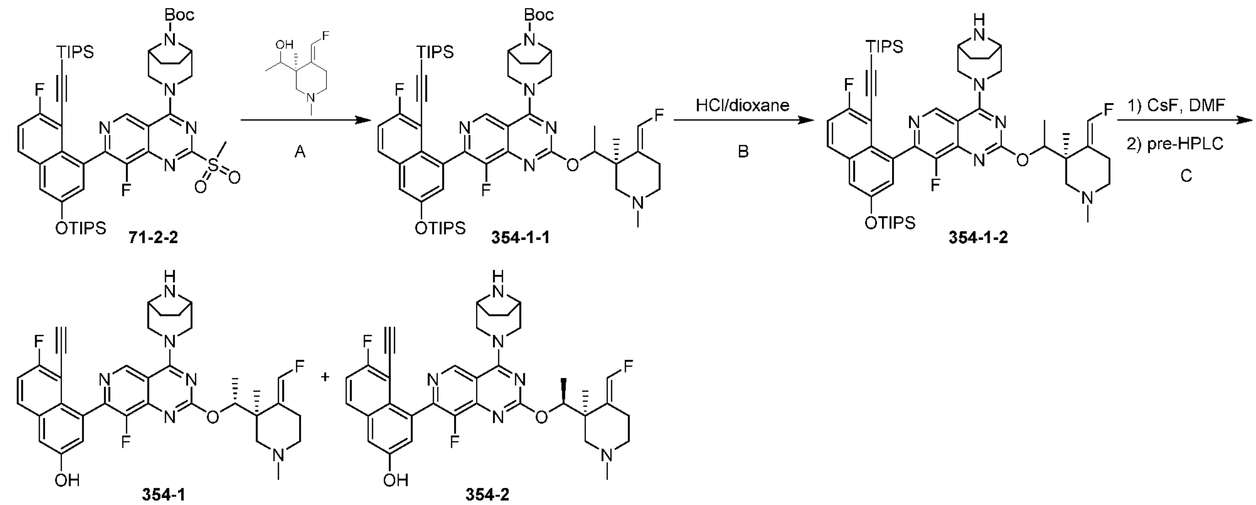


**Step A:** *tert-butyl* (1R,5S)-3-(8-fluoro-7-(7-fluoro-8-((triisopropylsilyl)ethynyl)-3-((triisopropylsilyl)oxy)naphthalen-1-yl)-2-(1-((S,E)-4-(fluoromethylene)-1,3-dimethylpiperidin-3-yl)ethoxy)pyrido[4,3-*d*]pyrimidin-4-yl)-3,8-diazabicyclo[3.2.1]octane-8-carboxylate

Under a dry-ice/ethanol bath, NaOtBu (0.32 mL, 0.64 mmol, 2 M in THF solution) was added dropwise to a solution of 1-((S,E)-4-fluoromethylene-1,3-dimethylpiperidin-3-yl) ethyl-1-ol (60.1 mg, 0.321 mmol), tert-butyl (1R,5S)-3-(8-fluoro-7-(7-fluoro-8-((triisopropylsilyl)ethynyl)-3-((triisopropylsilyl)oxy)naphthalen-1-yl)-2-(methylsulfonyl)pyrido[4,3-d]pyrimidin-4-yl)-3,8-diazabicyclo[3.2.1]octane-8-carboxylate (200 mg, 0.214 mmol) and THF (5 mL) , and the reaction was stirred at this temperature for 0.5 h. After the reaction was completed (monitored by LCMS), the reaction was poured into a saturated $NH_4Cl$ aqueous solution (50 mL) and extracted with EA (30 mL × 3). The organic phase was collected, washed with brine (70 mL), dried over anhydrous $Na_2SO_4$, filtered, concentrated, and purified by FCC ($SiO_2$, THF/PE = 0 - 25%) to afford *tert-butyl (1R,5S)-3-*(8-fluoro-7-(7-fluoro-8-((triisopropylsilyl)ethynyl)-3-((triisopropylsilyl)oxy)naphthalen-1-yl)-2-(1-((S,E)-4-(fluoromethylene)-1,3-dimethylpiperidin-3-yl)ethoxy)pyrido[4,3-d]pyrimidin-4-yl)-3,8-diazabicyclo[3.2.1]octane-8-carboxylate (170 mg, yield 76%) as a yellow oil. LCMS (m/z): 521.3 (1/2M + H).

**Step B:** 4-((1*R*,5*S*)-3,8-diazabicyclo[3.2.1]octan-3-yl)-8-fluoro-7-(7-fluoro-8-((triisopropylsilyl)ethynyl)-3-((triisopropylsilyl)oxy)naphthalen-1-yl)-2-(1-((S,E)-4-(fluoromethylene)-1,3-dimethylpiperidin-3-yl) ethoxy)pyrido[4,3-d]pyrimidine

At room temperature, hydrochloric acid-dioxane (4 M, 5.0 mL) was added to *tert-butyl* (1*R*,5*S*)-3-(8-fluoro-7-(7-fluoro-8-((triisopropylsilyl)ethynyl)-3-((triisopropylsilyl)oxy)naphthalen-1-yl)-2-(1-((S,E)-4-(fluoromethylene)-1,3-dimethylpiperidin-3-yl)ethoxy)pyrido[4,3-d]pyrimidin-4-yl)-3,8-diazabicyclo[3.2.1]octane-8-carboxylate (170mg, 0.163 mmol), and the resulting mixture was stirred at room temperature for 1 h. After the reaction was completed (monitored by TLC, EA/PE = 1: 1), the reaction was concentrated, and saturated aqueous sodium bicarbonate solution (50 mL) was added then. The reaction was extracted with EA (30 mL × 3). The combined organic phase was washed with brine (70 mL),

dried with anhydrous $Na_2SO_4$, concentrated to obtain 4-((1R,5S)-3,8-diazabicyclo[3.2.1]octan-3-yl)-8-fluoro-7-(7-fluoro-8-((triisopropylsilyl)ethynyl)-3-((triisopropylsilyl)oxy)naphthalen-1-yl)-2-(1-((S,E)-4-(fluoromethylene)-1,3-dimethylpiperidin-3-yl)ethoxy)pyrido[4,3-d]pyrimidine (154 mg, yield 100%) as a brown solid.

**Step C:** 4-(4-((1*R*,5*S*)-3,8-diazabicyclo[3.2.1]octan-3-yl)-8-fluoro-2-(*R*)-1-((*S,E*)-4-(fluoromethylene)-1,3-dimethylpiperidin-3-yl)ethoxy)pyrido[4,3-d]pyrimidin-7-yl)-5-ethynyl-6-fluoronaphthalen-2-ol **(354-1)** and 4-(4-((1*R*,5*S*)-3,8-diazabicyclo[3.2.1]octan-3-yl)-8-fluoro-2-((*S*)-1-((S,E)-4-(fluoromethylene)-1,3-dimethylpiperidin-3-yl)ethoxy)pyrido[4,3-d]pyrimidin-7-yl)-5-ethynyl-6-fluoronaphthalen-2-ol **(354-2)**

At room temperature, CsF (124 mg, 0.816 mmol) was added to a solution of 4-((1*R*,5*S*)-3,8-diazabicyclo[3.2.1]octan-3-yl)-8-fluoro-7-(7-fluoro-8-((triisopropylsilyl)ethynyl)-3-((triisopropylsilyl)oxy)naphthalen-1-yl)-2-(1-((S,E)-4-(fluoromethylene)-1,3-dimethylpiperidin-3-yl)ethoxy)pyrido[4,3-d]pyrimidine (154 mg, 0.163 mmol) in DMF (3 mL). The resulting mixture was heated to 50 °C and stirred for 1 h. After the reaction was completed (monitored by LCMS), the reaction solution was filtered and purified by pre-HPLC (C18, CAN/(10 mmol $NH_4HCO_3/H_2O$) = 55-75%). The first eluted isomer 1 (with a relatively shorter retention time) was obtained as Example **354-1** (201 mg, yield 20%), LCMS (m/z): 629.3 (M + H). $^1H$ NMR (400 MHz, DMSO-$d_6$) δ 10.20 (s, 1H), 9.00 (s, 1H), 8.06 - 7.90 (m, 1H), 7.53 - 7.43 (m, 1H), 7.39 (d, *J* = 2.5 Hz, 1H), 7.28 - 7.11 (m, 1H), 6.76 - 6.41 (m, 1H), 6.00 - 5.78 (m, 1H), 4.55 - 4.37 (m, 1H), 4.34 - 4.17 (m, 1H), 3.98 - 3.72 (m, 1H), 3.67 - 3.49 (m, 4H), 2.87 - 2.69 (m, 2H), 2.50 - 2.41 (m, 1H), 2.38 - 2.23 (m, 1H), 2.13 (s, 3H), 1.87 - 1.50 (m, 6H), 1.33 - 1.17 (m, 3H), 1.03 (s, 3H). $^{19}F$ NMR (376 MHz, DMSO-$d_6$) δ - 110.74, -139.03, -140.27. The subsequently eluted isomer 2 (with a relatively longer retention time) was obtained as Example **354-2** (6.6 mg, yield 6%), LCMS (m/z): 629.3 (M + H). $^1H$ NMR (400 MHz, DMSO-$d_6$) δ 10.21 (s, 1H), 9.01 (s, 1H), 8.08 - 7.89 (m, 1H), 7.54 - 7.43 (m, 1H), 7.39 (d, *J* = 2.6 Hz, 1H), 7.28 - 7.13 (m, 1H), 6.95 - 6.61 (m, 1H), 5.92 - 5.68 (m, 1H), 4.56 - 4.43 (m, 1H), 4.40 - 4.28 (m, 1H), 3.97 - 3.86 (m, 1H), 3.69 - 3.55 (m, 4H), 2.93 - 2.74 (m, 2H), 2.67 - 2.55 (m, 1H), 2.09 - 1.89 (m, 4H), 1.86 - 1.48 (m, 6H), 1.22 - 1.11 (m, 3H), 1.05 (s, 3H). $^{19}F$ NMR (376 MHz, DMSO-$d_6$) δ -110.74, -138.51, -140.17.

The following compounds were prepared by referring to the above synthetic method.

| Ex. | Structure | Name | Characterization data |
|---|---|---|---|
| 355 | 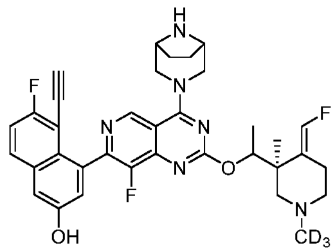 | 4-(4-((1*R*,5*S*)-3,8-diazabicyclo[3.2.1]octan-3-yl)-8-fluoro-2-(1-((*S*,*E*)-4-(fluoromethylene)-3-methyl-1-(methyl-$d_3$)piperidin-3-yl) ethoxy)pyrido [4,3-*d*]pyrimidin-7-yl)-5-ethynyl-6-fluoronaphthalene-2-ol | LCMS (m/z): 632.3 (M + H). |
| 356 | 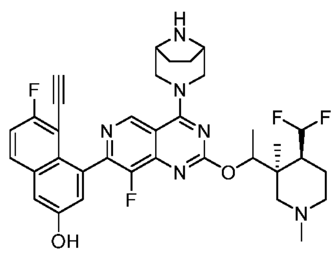 | 4-(4-((1*R*,5*S*)-3,8-diazabicyclo[3.2.1]octan-3-yl)-2-(1-((3*S*,4*S*)-4-(difluoromethyl)-1,3-dimethylpiperidin-3-yl) ethoxy)-8-fluoropyrido[4,3-*d*]pyrimidin-7-yl)-5-ethynyl-6-fluoronaphthalene-2-ol | LCMS (m/z): 649.3 (M + H). |
| 357 | 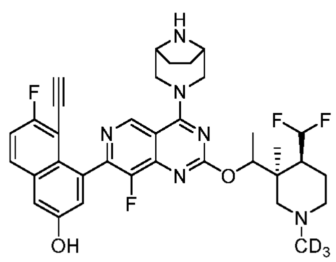 | 4-(4-((1*R*,5*S*)-3,8-diazabicyclo[3.2.1]octan-3-yl)-2-(1-((3*S*,4*S*)-4-(difluoromethyl)-3-methyl-1-(methyl-$d_3$)piperidin-3-yl) ethoxy)-8-fluoropyridine[4,3-*d*]pyrimidin-7-yl)-5-ethynyl-6-fluoronaphthalene-2-ol | LCMS (m/z): 652.3 (M + H). |

(continued)

| Ex. | Structure | Name | Characterization data |
|---|---|---|---|
| 358 | 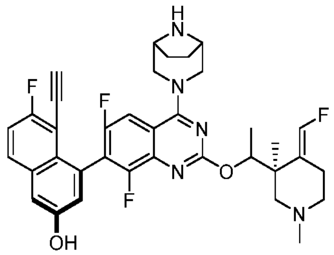 | 4-(4-((1*R*,5*S*)-3,8-diazabicyclo[3.2.1]octan-3-yl)-6,8-difluoro-2-(1-((*S*,*E*)-4-(fluoromethylene)-1,3-dimethylpiperidin-3-yl) ethoxy)quinazolin-7-yl)-5-ethynyl-6-fluoronaphthalene-2-ol | LCMS (m/z): 646.3 (M + H). |
| 359 | 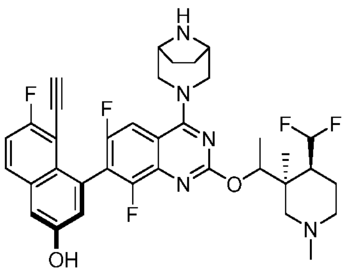 | 4-(4-((1*R*,5*S*)-3,8-diazabicyclo[3.2.1]octan-3-yl)-2-(1-((3*S*,4*S*)-4-(difluoromethyl)-1,3-dimethylpiperidin-3-yl) ethoxy)-6,8-difluoroquinazolin-7-yl)-5-ethynyl-6-fluoronaphthalene-2-ol | LCMS (m/z): 666.3 (M + H). |
| 360 | 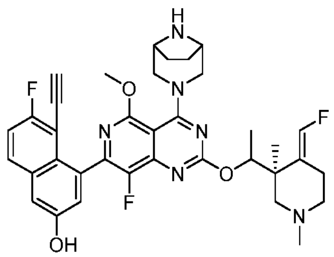 | 4-(4-((1*R*,5*S*)-3,8-diazabicyclo[3.2.1]octan-3-yl)-8-fluoro-2-(1-((*S*,*E*)-4-(fluoromethylene)-1,3-dimethylpiperidin-3-yl) ethoxy)-5-methoxypyridine [4,3-*d*]pyrimidin-7-yl)-5-ethynyl-6-fluoronaphthalene-2-ol | LCMS (m/z): 659.3 (M + H). |
| 361 | 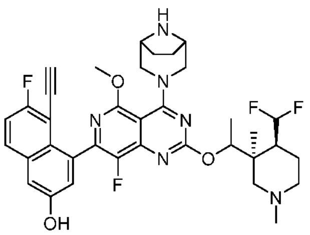 | 4-(4-((1*R*,5*S*)-3,8-diazabicyclo[3.2.1]octan-3-yl)-2-(1-((3*S*,4*S*)-4-(difluoromethyl)-1,3-dimethylpiperidin-3-yl) ethoxy)-8-fluoro-5-methoxypyridine [4,3-*d*]pyrimidin-7-yl)-5-ethynyl-6-fluoronaphthalene-2-ol | LCMS (m/z): 679.3 (M + H). |

**Example 362-1 and 362-2**

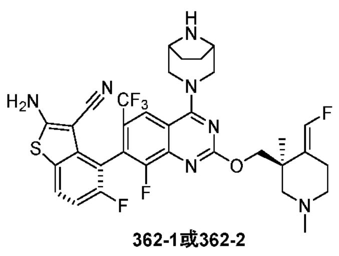

362-1或362-2

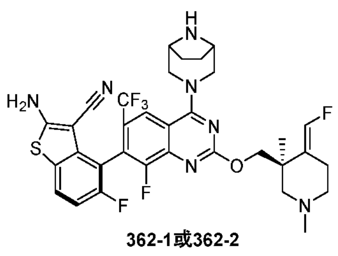

362-1或362-2

(*Sa*)-4-(4-((1*R*,5*S*)-3,8-diazabicyclo[3.2.1]octan-3-yl)-8-fluoro-2-(((*S,E*)-4-(fluoromethylene)-1,3-dimethylpiperidin-3-yl)methoxy)-6-(trifluoromethyl)quinazolin-7-yl)-2-amino-5-fluorobenzo[b]thiophene-3-carbonitrile **(362-1** or **362-2)** and (*Ra*)-4-(4-((1*R*,5*S*)-3,8-diazabicyclo[3.2.1]octan-3-yl)-8-fluoro-2-(((*S,E*)-4-(fluoromethylene)-1,3-dimethylpiperidin-3-yl)methoxy)-6-(trifluoromethyl)quinazolin-7-yl)-2-amino-5-fluorobenzo[b]thiophene-3-carbonitrile (**362-1** or **362-2)**

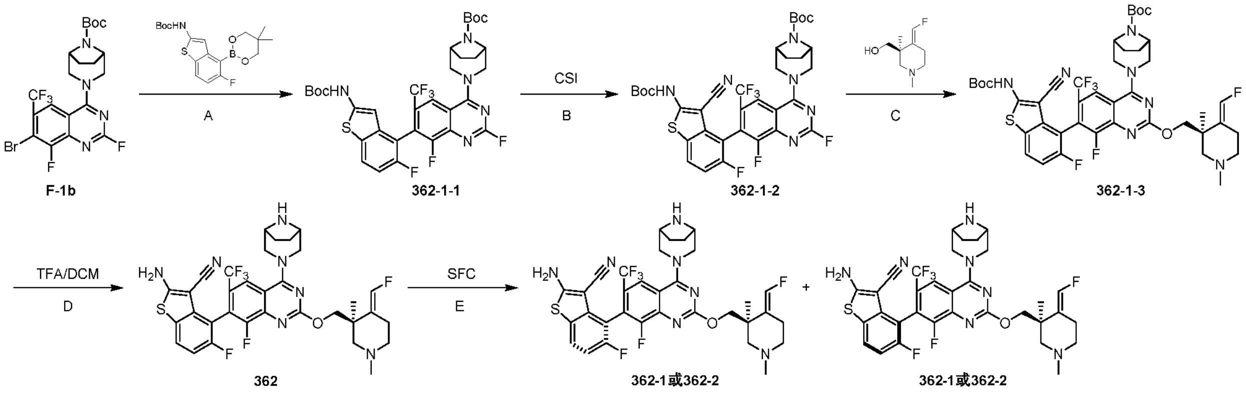


**Step A:** tert-butyl (1R,5S)-3-(7-(2-((tert-butoxycarbonyl)amino)-5-fluorobenzo[b]thiophen-4-yl)-2,8-difluoro-6-(trifluoromethyl)quinazolin-4-yl)-3,8-diazabicyclo[3.2.1]octane-8-carboxylate

Under nitrogen protection at room temperature, tert-butyl 4-(5,5-dimethyl-1,3,2-dioxaborolan-2-yl)-5-fluorobenzo[b]thiophen-2-yl)carbamate (379 mg, 1.0 mmol), potassium phosphate (426 mg, 2.0 mmol), and RuPhos Pd $G_4$ (114 mg, 0.13 mmol) were added to a solution of *tert-butyl (1R,5S)-3-*(7-bromo-2,8-difluoro-6-(trifluoromethyl) quinazolin-4-yl)-3,8-diazabicyclo[3.2.1]octane-8-carboxylate (350 mg, 0.67 mmol) in 1,4-dioxane (10 mL) and water (2 mL). The reaction was heated to 80 °C and stirred for 2 h. After the reaction was completed (monitored by LCMS), the reaction was filtered with celite, and the filtrate was concentrated and dried. The crude product obtained was purified by FCC ($SiO_2$, EA/PE = 0-18%) to afford *tert-butyl* (1*R*,5*S*)-3-(7-(2-((*tert*-butoxycarbonyl)amino)-5-fluorobenzo[*b*]thiophen-4-yl)-2,8-difluoro-6-(trifluoromethyl)quinazolin-4-yl)-3,8-diazabicyclo[3.2.1]octane-8-carboxylate (210 mg, yield 44%) as a yellow solid. LCMS (m/z): 710.2 (M + H).

**Step B:** tert-butyl (1R,5S)-3-(7-(2-((tert-butoxycarbonyl)amino)-3-cyano-5-fluorobenzo[b]thiophen-4-yl)-2,8-difluoro-6-(trifluoromethyl)quinazolin-4-yl)-3,8-diazabicyclo[3.2.1]octane-8-carboxylate

Under nitrogen atmosphere and at -10 °C, chlorosulfonyl isocyanate (113 mg, 0.80 mmol) was added to a solution of *tert-butyl* (1R,5S)-3-(7-(2-((tert-butoxycarbonyl)amino)-5-fluorobenzo[b]thiophen-4-yl)-2,8-difluoro-6-(trifluoromethyl) quinazolin-4-yl)-3,8-diazabicyclo[3.2.1]octane-8-carboxylate (190 mg, 0.27 mmol) in acetonitrile (4 mL). The reaction was stirred at the same temperature for 1 h, and DMF (98 mg, 1 .3 mmol) was added to the reaction then. The resulting mixture was stirred at -10 °C for 1 h. After the reaction was completed (monitored by LCMS), 20 mL of saturated aqueous sodium bicarbonate was added, and the mixture was extracted with ethyl acetate. The combined organic phase was washed with brine, dried over anhydrous sodium sulfate, filtered and concentrated to dryness. The resulting crude product was purified by FCC ($SiO_2$, EA/PE = 0 - 20%) to afford tert-butyl (1*R*,5*S*)-3-(7-(2-((*tert*-butoxycarbonyl)amino)-3-cyano-5-fluorobenzo [b]thiophen-4-yl)-2,8-difluoro-6-(trifluoromethyl)quinazolin-4-yl)-3,8-diazabicyclo[3.2.1]octane-8-carboxylate (193 mg, yield 97%) as a yellow solid. LCMS (m/z): 735.2 (M + H).

Step C: *tert-butyl* (1R,5S)-3-(7-(2-((tert-butoxycarbonyl)amino)-3-cyano-5-fluorobenzo[b]thiophen-4-yl)-8-fluoro-2-(((S,E)-4-(fluoromethylene)-1,3-dimethylpiperidin-3-yl)methoxy)-6-(trifluoromethyl)quinazolin-4-yl)-3,8-diazabicyclo[3.2.1]octane-8-carboxylate

Under nitrogen atmosphere and at -70 °C, potassium tert-butoxide (0.35 mL, 0.69 mmol) was added to a solution of tert-butyl (1*R*,5*S*)-3-(7-(2-((*tert*-butoxycarbonyl)amino)-3-cyano-5-fluorobenzo[b]thiophen-4-yl)-2,8-difluoro-6-(trifluoromethyl)quinazolin-4-yl)-3,8-diazabicyclo[3.2.1]octane-8-carboxylate (120 mg, 0.69 mmol) in anhydrous tetrahydrofuran (1.5 mL). The resulting mixture was stirred at -70 °C for 0.5 h. After the reaction was completed (monitored by LCMS), the reaction was poured into 20 mL of saturated aqueous ammonium chloride solution. The resulting mixture was extracted with ethyl acetate. The combined organic phase was washed with brine, dried over anhydrous sodium sulfate,

filtered and concentrated to dryness. The resulting crude product was purified by FCC ($SiO_2$, EA/PE = 0 - 54%) to obtain *tert-butyl* (1*R*,5*S*)-3-(7-(2-((*tert*-butoxycarbonyl)amino)-3-cyano-5-fluorobenzo[b]thiophen-4-yl)-8-fluoro-2-(((S,E)-4-(fluoromethylene)-1,3-dimethylpiperidin-3-yl)methoxy)-6-(trifluoromethyl)quinazolin-4-yl)-3,8-diazabicyclo[3.2.1]octane-8-carboxylate (93 mg, yield 90%) as a white solid. LCMS (m/z): 888.3 (M + H).

**Step D:** 4-(4-((1*R*,5*S*)-3,8-diazabicyclo[3.2.1]octan-3-yl)-8-fluoro-2-(((*S,E*)-4-(fluoromethylene)-1,3-dimethylpiperidin-3-yl)methoxy)-6-(trifluoromethyl)quinazolin-7-yl)-2-amino-5-fluorobenzo[b]thiophene-3-carbonitrile

At room temperature, TFA (1 mL) was added to a solution of *tert-butyl* (1*R*,5*S*)-3-(7-(2-((*tert*-butoxycarbonyl) amino)-3-cyano-5-fluorobenzo[b]thiophen-4-yl)-8-fluoro-2-(((S,E)-4-(fluoromethylene)-1,3-dimethylpiperidin-3-yl)methoxy)-6-(trifluoromethyl)quinazolin-4-yl)-3,8-diazabicyclo[3.2.1]octane-8-carboxylate (93 mg, 0.10 mmol) in DCM (2 mL). The reaction was stirred at the same temperature for 1 h. After the reaction was completed (monitored by LCMS), the reaction was concentrated, and the crude product obtained was purified by pre-HPLC (C18, CAN/(10 mmol $NH_4HCO_3/H_2O$) = 30-50%) to give 4-(4-((1R,5S)-3,8-diazabicyclo[3.2.1]octan-3-yl)-8-fluoro-2-(((S,E)-4-(fluoromethylene)-1,3-dimethylpiperidin-3-yl)methoxy)-6-(trifluoromethyl)quinazolin-7-yl)-2-amino-5-fluorobenzo[b]thiophene-3-carbonitrile (45 mg, yield 62%) as a white solid. LCMS (m/z): 688.3 (M + H).

**Step E:** (*S*)-4-(4-((1*R*,5*S*)-3,8-diazabicyclo[3.2.1]octan-3-yl)-8-fluoro-2-(((*SE*)-4-(fluoromethylene)-1,3-dimethylpiperidin-3-yl)methoxy)-6-(trifluoromethyl)quinazolin-7-yl)-2-amino-5-fluorobenzo[b]thiophene-3-carbonitrile **(374-1** or **374-2)** and *(R)-4-(4-((1R,5S)-3,8-*diazabicyclo[3.2.1]octan-3-yl)-8-fluoro-2-(((S,E)-4-(fluoromethylene)-1,3-dimethylpiperidin-3-yl)methoxy)-6-(trifluoromethyl)quinazolin-7-yl)-2-amino-5-fluorobenzo[*b*]thiophene-3-carbonitrile **(374-1** or **374-2)**

4-(4-((1*R*,5*S*)-3,8-diazabicyclo[3.2.1]octan-3-yl)-8-fluoro-2-(((*S,E*)-4-(fluoromethylene)-1,3-dimethylpiperidin-3-yl)methoxy)-6-(trifluoromethyl)quinazolin-7-yl)-2-amino-5-fluorobenzo[b]thiophene-3-carbonitrile (45mg) was resolved by chiral Waters SFC150 (separation column: DAICEL CHIRALCEL ® IC, 250 * 25 mm, 10μm; Mobile phase: $CO_2$/IPA (+ 0.2% 7.0 mol/l $NH_3$/MeOH) = 65/35; flow rate: 120 mL/min). The first eluted isomer 1 was example **362-1** (12.6 mg, with a relatively shorter retention time). Chiral analytical method SFC-9, Rt = 2.170 min. $^1$H NMR (400 MHz, DMSO-$d_6$) δ 8.09 (s, 1H), 7.93 (s, 2H), 7.87 - 7.80 (m, 1H), 7.16 - 7.09 (m, 1H), 6.86 - 6.56 (m, 1H), 4.58 (d, *J* = 10.6 Hz, 1H), 4.39 - 4.23 (m, 3H), 3.63 - 3.53 (m, 4H), 2.72 - 2.64 (m, 2H), 2.56 - 2.52 (m, 1H), 2.25 - 2.16 (m, 1H), 2.14 (s, 3H), 1.97 - 1.80 (m, 2H), 1.69 - 1.57 (m, 3H), 1.57 - 1.47 (m, 1H), 1.11 (s, 3H). $^{19}$F NMR (376 MHz, DMSO-$d_6$) δ -59.26, -117.91, -123.52, - 138.73. LCMS (m/z): 688.3 (M + H). The subsequently eluted isomer 2 was example **362-2** (13.6 mg, with a relatively longer retention time). Chiral analytical method SFC-9, Rt = 4.085 min. $^1$H NMR (400 MHz, DMSO-$d_6$) δ 8.09 (s, 1H), 7.93 (s, 2H), 7.87 - 7.80 (m, 1H), 7.17 - 7.08 (m, 1H), 6.86 - 6.54 (m, 1H), 4.59 (d, *J* = 10.6 Hz, 1H), 4.42 - 4.19 (m, 3H), 3.63 - 3.49 (m, 4H), 2.71 - 2.65 (m, 2H), 2.55 - 2.52 (m, 1H), 2.28 - 2.18 (m, 1H), 2.14 (s, 3H), 1.95 - 1.78 (m, 2H), 1.66 - 1.57 (m, 3H), 1.55 - 1.48 (m, 1H), 1.10 (s, 3H). $^{19}$F NMR (400 MHz, DMSO-$d_6$) δ -59.26, -117.91, -123.58, -138.79. LCMS (m/z): 688.3 (M + H).

**Example 363-1 and Example 363-2**

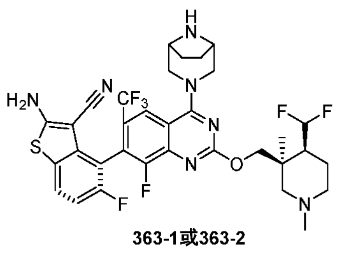

363-1或363-2

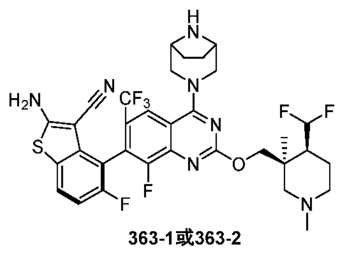

363-1或363-2

(*Sa*)-4-(4-((1*R*,5*S*)-3,8-diazabicyclo[3.2.1]octan-3-yl)-2-(((3*S*,4*S*)-4-(difluoromethyl)-1,3-dimethylpiperidin-3-yl)methoxy)-8-fluoro-6-(trifluoromethyl)quinazolin-7-yl)-2-amino-5-fluorobenzo[b]thiophene-3-carbonitrile **(363-1 or 363-2)** and (*Ra*)-4-(4-((1*R*,5*S*)-3,8-diazabicyclo[3.2.1]octan-3-yl)-2-(((3*S*,4*S*)-4-(difluoromethyl)-1,3-dimethylpiperidin-3-yl) methoxy)-8-fluoro-6-(trifluoromethyl)quinazolin-7-yl)-2-amino-5-fluorobenzo[b]thiophene-3-carbonitrile **(363-1 or 363-2)**

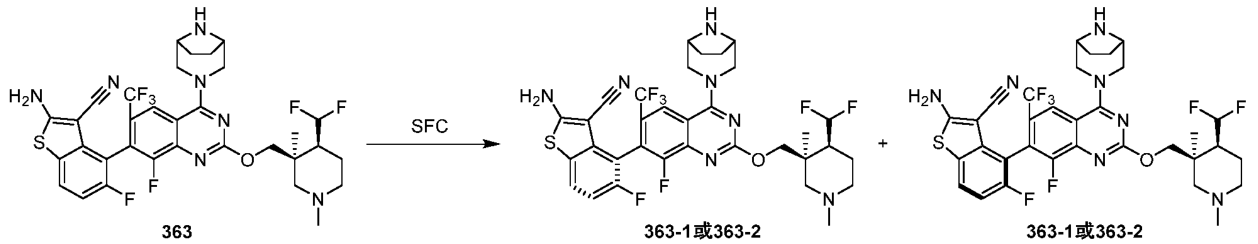


363 363-1或363-2 363-1或363-2

Compound 363 was synthesized according to the method described above for compound 362. Compound 363 was resolved by chiral Waters SFC150 (separation column: DAICEL CHIRALCEL ® IC, 250 * 25 mm, 10 μm; mobile phase: $CO_2$/IPA (+ 0.2% 7.0 mol/l $NH_3$/MeOH) = 70/30; flow rate: 120 mL/min). The first eluted isomer 1 was example **363-1** (with a relatively shorter retention time). Chiral analytical method SFC-14, Rt = 1.660, LCMS (m/z): 708.3 (M + H). The subsequently eluted isomer 2 was example **363-2** (with a relatively longer retention time). Chiral analytical method SFC-14, Rt = 2.657, LCMS (m/z): 708.3 (M + H).

## Intermediates 362-1-2A and 362-1-2B

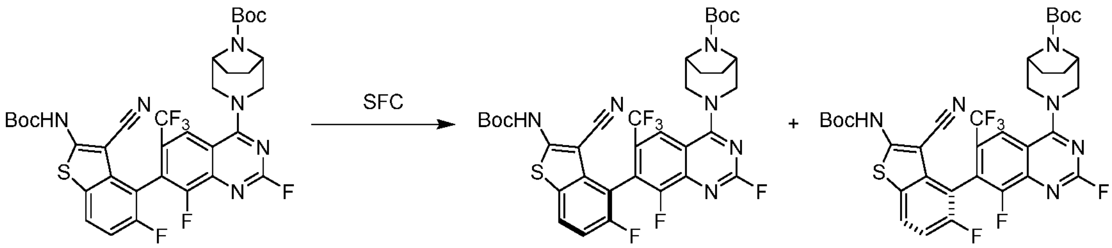


362-1-2 362-1-2A或362-1-2B 362-1-2A或362-1-2B

Compound **362-1-2** (2.0 g) was resolved by chiral Waters SFC150 (separation column: DAICEL CHIRALCEL®IC, 250*30 mm, 10 μm; mobile phase: $CO_2$/EtOH (+0.2% 7.0 mol/l $NH_3$/MeOH) = 80/20; Flow rate: 120 mL/min) to obtain the first eluted isomer 1 as example **362-1-2A** (0.8 g, with a relatively shorter retention time). Chiral analytical method SFC-15, Rt = 2.136. The subsequently eluted isomer 2 as example **362-1-2B** (1.0 g, with a relatively longer retention time). Chiral analytical method SFC-15, Rt = 2.735.

The following compounds were prepared by referring to the above-mentioned methods using Intermediates **362-1-2A** and **362-1-2B.** It should be noted that for the naming of the following isomers-1 or -2, the one with a shorter retention time during the chiral resolution of the final compound was named as -1, and the one with a longer retention time was named as -2. Alternatively, the compound synthesized using **362-1-2A** as the intermediate was named as isomer-1, and the compound synthesized using intermediate **362-1-2B** as the intermediate was named as isomer-2. When there is a conflict between the two naming methods, the naming based on the preparation with the latter chiral-pure intermediate prevails.

| Ex. | Structure | Int. | Name | Characterization data |
|---|---|---|---|---|
| 364-1 | 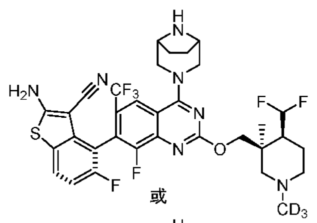 或 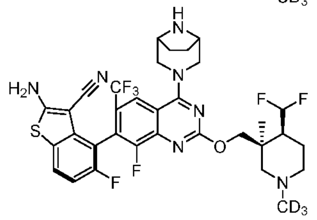 | **362-1-2A** | (*Sa*) or (*Ra*)-4-(4-((1*R*,5*S*)-3,8-diazabicyclo[3.2.1]octan-3-yl)-2-(((3*S*,4*S*)-4-(difluoromethyl)-3-methyl-1-(methyl-$d_3$)piperidin-3-yl) methoxy)-8-fluoro-6-(trifluoromethyl) quinazoli n-7-yl)-2-amino-5-fluorobenzo[*b*]thiophene-3-carbonitrile | LCMS (m/z): 711.2 (M + H). |

(continued)

| Ex. | Structure | Int. | Name | Characterization data |
|---|---|---|---|---|
| 364-2 | 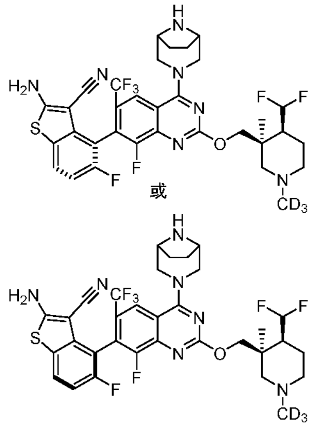 或 | **362-1-2B** | (*Sa*) or (*Ra*)-4-(4-((1*R*,5*S*)-3,8-diazabicyclo[3.2.1]octan-3-yl)-2-(((3*S*,4*S*)-4-(difluoromethyl)-3-methyl-1-(methyl-*d*$_3$)piperidin-3-yl) methoxy)-8-fluoro-6-(trifluoromethyl) quinazoli n-7-yl)-2-amino-5-fluorobenzo[*b*]thiophene-3-carbonitrile | LCMS (m/z): 711.2 (M + H). |
| 365-1 | 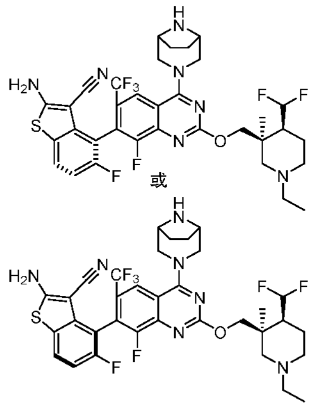 或 | **302-1-2A** | (*Sa*) or (*Ra*)-4-(4-((1*R*,5*S*)-3,8-diazabicyclo[3.2.1]octan-3-yl)-2-(((3*S*,4*S*)-4-(difluoromethyl)-1-ethyl-3-methylpiperidin-3-yl)methoxy)-8-fluoro-6-(trifluoromethyl)quinazoli n-7-yl)-2-amino-5-fluorobenzo [*b*]thiophene-3-carbonitrile | LCMS (m/z): 722.2 (M + H). |
| 365-2 | 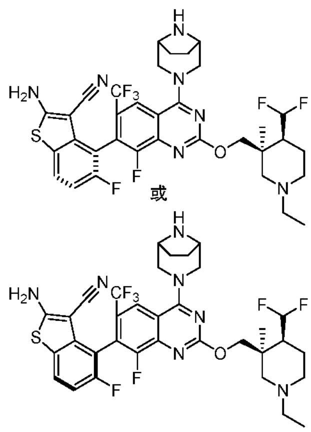 或 | **362-1-2B** | (*Sa*) or (*Ra*)-4-(4-((1*R*,5*S*)-3,8-diazabicyclo[3.2.1]octan-3-yl)-2-(((3*S*,4*S*)-4-(difluoromethyl)-1-ethyl-3-methylpiperidin-3-yl)methoxy)-8-fluoro-6-(trifluoromethyl)quinazoli n-7-yl)-2-amino-5-fluorobenzo [*b*]thiophene-3-carbonitrile | LCMS (m/z): 722.2 (M + H). |

(continued)

| Ex. | Structure | Int. | Name | Characterization data |
|---|---|---|---|---|
| 366-1 | 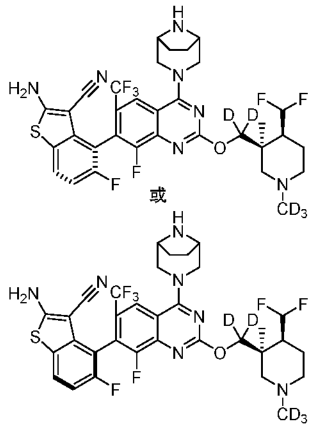 或 | **362-1-2A** | (*Sa*) or (*Ra*)-4-(4-((1*R*,5*S*)-3,8-diazabicyclo[3.2.1]octan-3-yl)-2-(((3*S*,4*S*)-4-(difluoromethyl)-3-methyl-1-methyl-*d*$_3$)piperidin-3-yl) methoxy-*d*$_2$)-8-fluoro-6-(trifluoromethyl)quinazoli n-7-yl)-2-amino-5-fluorobenzo[*b*]thiophene-3-carbonitrile | LCMS (m/z): 713.3 (M + H). |
| 366-2 | 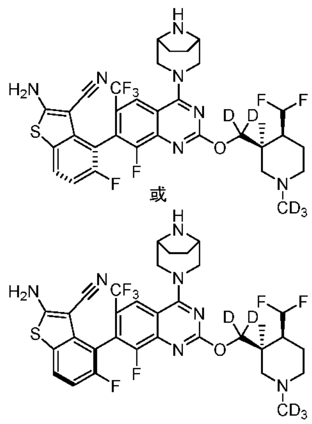 或 | **362-1-2B** | (*Sa*) or (*Ra*)-4-(4-((1*R*,5*S*)-3,8-diazabicyclo[3.2.1]octan-3-yl)-2-(((3*S*,4*S*)-4-(difluoromethyl)-3-methyl-1-methyl-*d*$_3$)piperidine3-yl) methoxy-*d*$_2$)-8-fluoro-6-(trifluoromethyl)quinazoli n-7-yl)-2-amino-5-fluorobenzo[*b*]thiophene-3-carbonitrile | LCMS (m/z): 713.3 (M + H). |
| 367-1 | 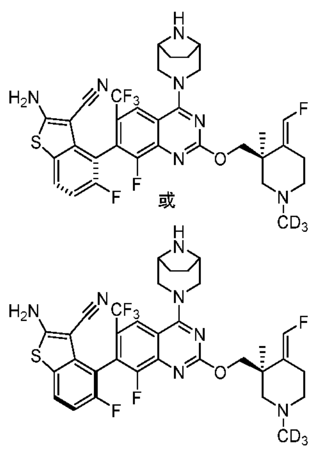 或 | **362-1-2A** | (*Sa*) or (*Ra*)-4-(4-((1*R*,5*S*)-3,8-diazabicyclo[3.2.1]octan-3-yl)-8-fluoro-2-(((*S*,*E*)-4-(fluoromethylene)-3-methyl-1-(methyl-*d*$_3$)piperidin-3-yl)methoxy)-6-(trifluoromethyl) quinazoli n-7-yl)-2-amino-5-fluorobenzo[*b*]thiophene-3-carbonitrile | LCMS (m/z): 691.2 (M + H). |

(continued)

| Ex. | Structure | Int. | Name | Characterization data |
|---|---|---|---|---|
| 367-2 | 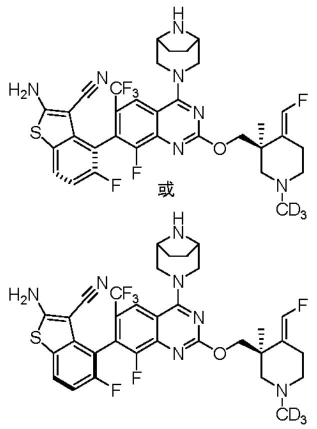 或 | **362-1-2B** | (*Sa*) or (*Ra*)-4-(4-((1*R*,5*S*)-3,8-diazabicyclo[3.2.1]octan-3-yl)-8-fluoro-2-(((*S*,*E*)-4-(fluoromethylene)-3-methyl-1-(methyl-*d*$_3$)piperidin-3-yl)methoxy)-6-(trifluoromethyl) quinazoli n-7-yl)-2-amino-5-fluorobenzo[*b*]thiophene-3-carbonitrile | LCMS (m/z): 691.3 (M + H). $^1$H NMR (400 MHz, DMSO-*d*$_6$) δ 8.10 (s, 1H), 7.95 (s, 2H), 7.87 - 7.80 (m, 1H), 7.17 - 7.08 (m, 1H), 6.88 - 6.56 (m, 1H), 4.58 (d, *J* = 10.6 Hz, 1H), 4.38 - 4.27 (m, 3H), 3.71 - 3.52 (m, 4H), 2.73 - 2.62 (m, 2H), 2.56 - 2.51 (m, 1H), 2.26 - 2.10 (m, 1H), 1.97 - 1.79 (m, 2H), 1.74 - 1.49 (m, 4H), 1.11 (s, 3H). $^{19}$H NMR (400 MHz, DMSO-*d*$_6$) δ - 59.26, -117.89, - 123.49, -138.73. |
| 368-1 | 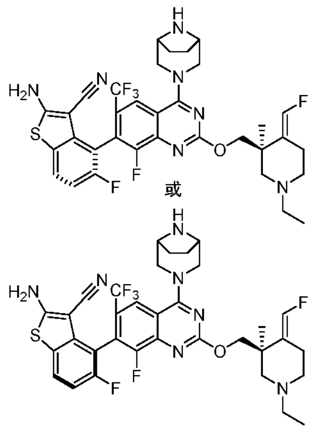 或 | **362-1-2A** | (*Sa*) or (*Ra*)-4-(4-((1*R*,5*S*)-3,8-diazabicyclo[3.2.1]octan-3-yl)-2-(((*S*,*E*)-1-ethyl-4-(fluoromethylene)-3-methylpiperidin-3-yl)methoxy)-8-fluoro-6-(trifluoromethyl)quinazoli n-7-yl)-2-amino-5-fluorobenzo[*b*]thiophene-3-carbonitrile | LCMS (m/z): 702.2 (M + H). |
| 368-2 | 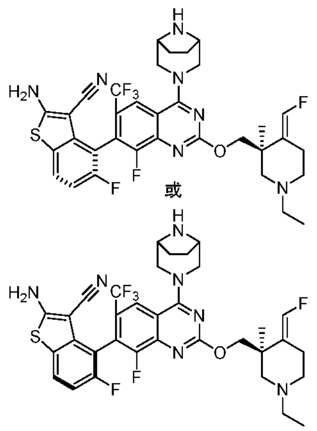 或 | **362-1-2B** | (*Sa*) or (*Ra*)-4-(4-((1*R*,5*S*)-3,8-diazabicyclo[3.2.1]octan-3-yl)-2-(((*S*,*E*)-1-ethyl-4-(fluoromethylene)-3-methylpiperidin-3-yl)methoxy)-8-fluoro-6-(trifluoromethyl)quinazoli n-7-yl)-2-amino-5-fluorobenzo[*b*]thiophene-3-carbonitrile | LCMS (m/z): 702.2 (M + H). |

(continued)

| Ex. | Structure | Int. | Name | Characterization data |
|---|---|---|---|---|
| 369-1 | 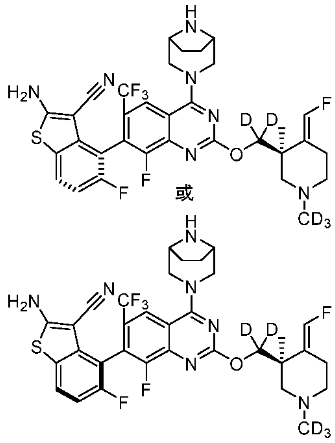 或 | **362-1-2A** | (*Sa*) or (*Ra*)-4-(4-((1*R*,5*S*)-3,8-diazabicyclo[3.2.1]octan-3-yl)-8-fluoro-2-(((*S*,*E*)-4-(fluoromethylene)-3-methyl-1-(methyl-$d_3$)piperidin-3-yl)methoxy-$d_2$)-6-(trifluoromethyl)quinazoli n-7-yl)-2-amino-5-fluorobenzo[*b*]thiophene-3-carbonitrile | LCMS (m/z): 693.2 (M + H). |
| 369-2 | 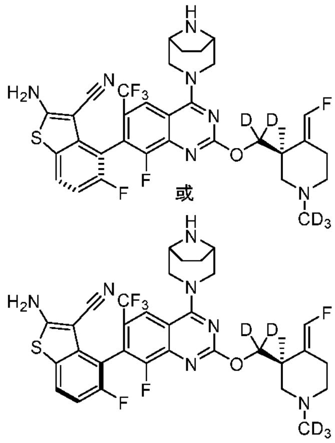 或 | **362-1-2B** | (*Sa*) or (*Ra*)-4-(4-((1*R*,5*S*)-3,8-diazabicyclo[3.2.1]octan-3-yl)-8-fluoro-2-(((*S*,*E*)-4-(fluoromethylene)-3-methyl-1-(methyl-$d_3$)piperidin-3-yl)methoxy-$d_2$)-6-(trifluoromethyl)quinazoli n-7-yl)-2-amino-5-fluorobenzo[*b*]thiophene-3-carbonitrile | LCMS (m/z): 693.2 (M + H). |

The following compounds were prepared by referring to the above example synthesis method.

| Ex. | Structural Formula | Name | Characterization data |
|---|---|---|---|
| 370-1 | 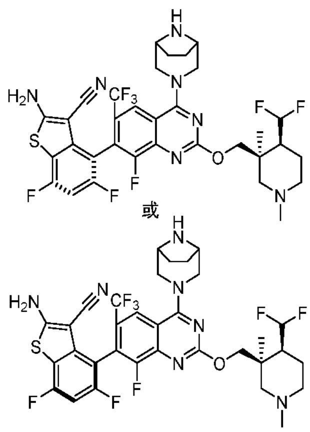 或 | (*Sa*) or (*Ra*)-4-(4-((1*R*,5*S*)-3,8-diazabicyclo[3.2.1]octan-3-yl)-2-(((3*S*,4*S*)-4-(difluoromethyl)-1,3-dimethylpiperidin-3-yl)methoxy)-8-fluoro-6-(trifluoromethyl)quinazolin-7-yl)-2-amino-5,7-difluorobenzo[b]thiophene-3-carbonitrile | LCMS (m/z): 726.2 (M + H). |

(continued)

| Ex. | Structural Formula | Name | Characterization data |
|---|---|---|---|
| 370-2 | 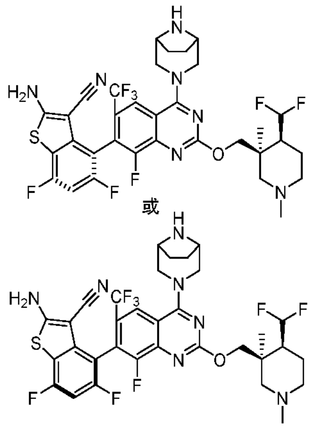 或 | (*Sa*) or (*Ra*)-4-(4-((1*R*,5*S*)-3,8-diazabicyclo[3.2.1]octan-3-yl)-2-(((3*S*,4*S*)-4-(difluoromethyl)-1,3-dimethylpiperidin-3-yl)methoxy)-8-fluoro-6-(trifluoromethyl)quinazolin-7-yl)-2-amino-5,7-difluorobenzo[b]thiophene-3-carbonitrile | LCMS (m/z): 726.2 (M + H). |
| 371-1 | 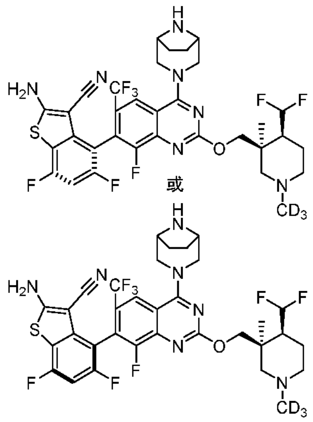 或 | (*Sa*) or (*Ra*)-4-(4-((1*R*,5*S*)-3,8-diazabicyclo[3.2.1]octan-3-yl)-2-(((3*S*,4*S*)-4-(difluoromethyl)-3-methyl-1-(methyl-*d*$_3$)piperidin-3-yl)methoxy)-8-fluoro-6-(trifluoromethyl)quinazolin-7-yl)-2-amino-5,7-difluorobenzo[b]thiophene-3-carbonitrile | LCMS (m/z): 729.2 (M + H). |
| 371-2 | 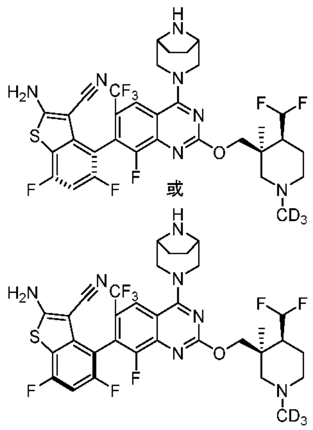 或 | (*Sa*) or (*Ra*)-4-(4-((1*R*,5*S*)-3,8-diazabicyclo[3.2.1]octan-3-yl)-2-(((3*S*,4*S*)-4-(difluoromethyl)-3-methyl-1-(methyl-*d*$_3$)piperidin-3-yl)methoxy)-8-fluoro-6-(trifluoromethyl)quinazolin-7-yl)-2-amino-5,7-difluorobenzo[b]thiophene-3-carbonitrile | LCMS (m/z): 729.2 (M + H). |

(continued)

| Ex. | Structural Formula | Name | Characterization data |
|---|---|---|---|
| 372-1 | 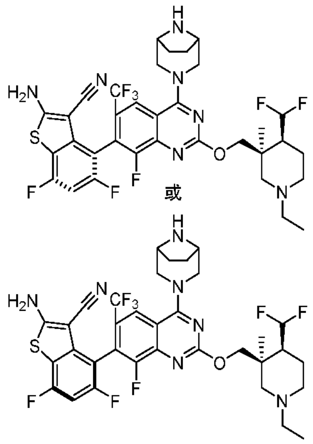 或 | (*Sa*) or (*Ra*)-4-(4-((1*R*,5*S*)-3,8-diazabicyclo[3.2.1]octan-3-yl)-2-(((3*S*,4*S*)-4-(difluoromethyl)-1-ethyl-3-methylpiperidin-3-yl)methoxy)-8-fluoro-6-(trifluoromethyl)quinazolin-7-yl)-2-amino-5,7-difluorobenzo[b]thiophene-3-carbonitrile | LCMS (m/z): 740.2 (M + H). |
| 372-2 | 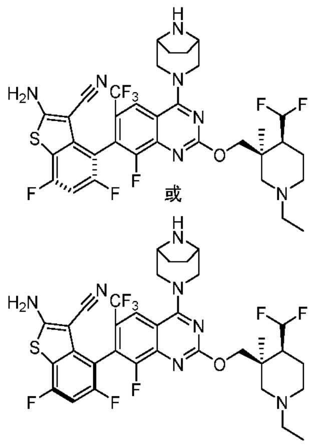 或 | (*Sa*) or (*Ra*)-4-(4-((1*R*,5*S*)-3,8-diazabicyclo[3.2.1]octan-3-yl)-2-(((3*S*,4*S*)-4-(difluoromethyl)-1-ethyl-3-methylpiperidin-3-yl)methoxy)-8-fluoro-6-(trifluoromethyl)quinazolin-7-yl)-2-amino-5,7-difluorobenzo[b]thiophene-3-carbonitrile | LCMS (m/z): 740.2 (M + H). |
| 373-1 | 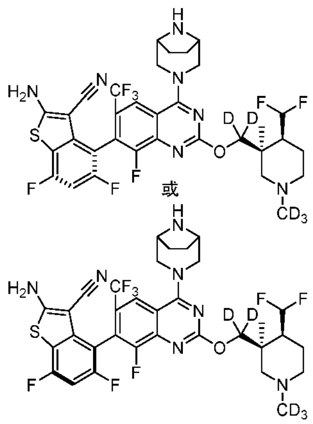 或 | (*Sa*) or (*Ra*)-4-(4-((1*R*,5*S*)-3,8-diazabicyclo[3.2.1]octan-3-yl)-2-(((3*S*,4*S*)-4-(difluoromethyl)-3-methyl-1-(methyl-*d*$_3$)piperidin-3-yl)methoxy-*d*$_2$)-8-fluoro-6-(trifluoromethyl)quinazolin-7-yl)-2-amino-5,7-difluorobenzo[b]thiophene-3-carbonitrile | LCMS (m/z): 731.3 (M + H). |

(continued)

| Ex. | Structural Formula | Name | Characterization data |
|---|---|---|---|
| 373-2 | 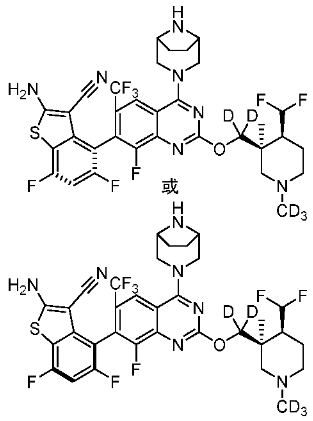 或 | (*Sa*) or (*Ra*)-4-(4-((1*R*,5*S*)-3,8-diazabicyclo[3.2.1] octan-3-yl)-2-(((3*S*,4*S*)-4-(difluoromethyl)-3-methyl-1-(methyl-*d*$_3$)piperidin-3-yl)methoxy-*d*$_2$)-8-fluoro-6-(trifluoromethyl)quinazolin-7-yl)-2-amino-5,7-difluorobenzo[b]thiophene-3-carbonitrile | LCMS (m/z): 731.3 (M + H). |
| 374-1 | 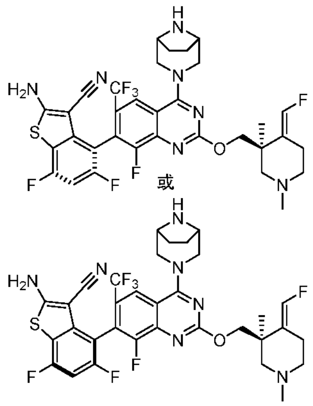 或 | (*Sa*) or (*Ra*)-4-(4-((1*R*,5*S*)-3,8-diazabicyclo[3.2.1] octan-3-yl)-8-fluoro-2-(((*S*,*E*)-4-(fluoromethylene)-1,3-dimethylpiperidin-3-yl)methoxy)-6-(trifluoromethyl)quinazolin-7-yl)-2-amino-5,7-difluorobenzo[b]thiophene-3-carbonitrile | LCMS (m/z): 706.2 (M + H). |
| 374-2 | 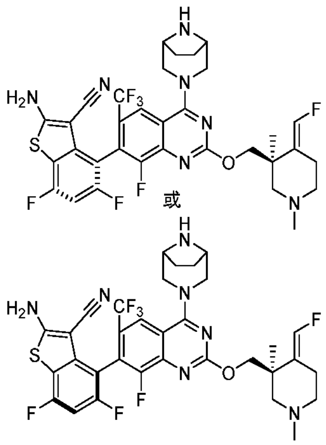 或 | (*Sa*) or *(Ra)-4-(4-((1R,5S)-3,8*-diazabicyclo[3.2.1] octan-3-yl)-8-fluoro-2-(((*S*,*E*)-4-(fluoromethylene)-1,3-dimethylpiperidin-3-yl)methoxy)-6-(trifluoromethyl)quinazolin-7-yl)-2-amino-5,7-difluorobenzo[b]thiophene-3-carbonitrile | LCMS (m/z): 706.2 (M + H). |

(continued)

| Ex. | Structural Formula | Name | Characterization data |
|---|---|---|---|
| 375-1 | 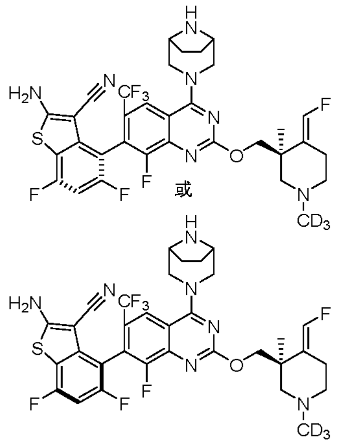 或 | (*Sa*) or (*Ra*)-4-(4-((1*R*,5*S*)-3,8-diazabicyclo[3.2.1]octan-3-yl)-8-fluoro-2-(((*S*,*E*)-4-(fluoromethylene)-3-methyl-1-(methyl-$d_3$)piperidin-3-yl)methoxy)-6-(trifluoromethyl)quinazolin-7-yl)-2-amino-5,7-difluorobenzo[b]thiophene-3-carbonitrile | LCMS (m/z): 709.2 (M + H). |
| 375-2 | 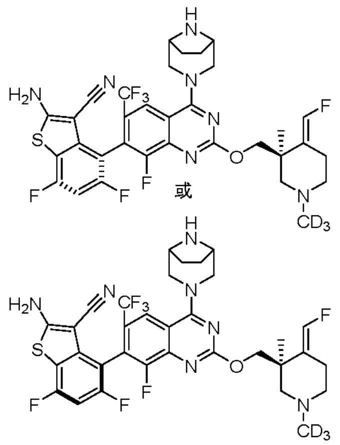 或 | (*Sa*) or (*Ra*)-4-(4-((1*R*,5*S*)-3,8-diazabicyclo[3.2.1]octan-3-yl)-8-fluoro-2-(((S,E)-4-(fluoromethylene)-3-methyl-1-(methyl-$d_3$)piperidin-3-yl)methoxy)-6-(trifluoromethyl)quinazolin-7-yl)-2-amino-5,7-difluorobenzo[b]thiophene-3-carbonitrile | LCMS (m/z): 709.2 (M + H). |
| 376-1 | 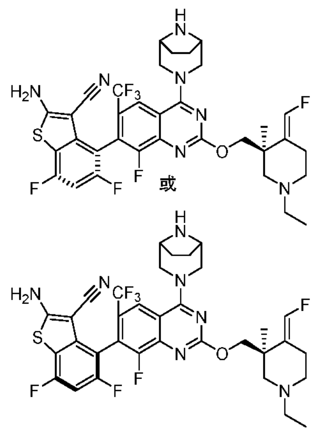 或 | (*Sa*) or (*Ra*)-4-(4-((1*R*,5*S*)-3,8-diazabicyclo[3.2.1]octan-3-yl)-2-(((*S*,*E*)-1-ethyl-4-(fluoromethylene)-3-methylpiperidin-3-yl)methoxy)-8-fluoro-6-(trifluoromethyl)quinazolin-7-yl)-2-amino-5,7-difluorobenzo[b]thiophene-3-carbonitrile | LCMS (m/z): 720.2 (M + H). |

(continued)

| Ex. | Structural Formula | Name | Characterization data |
|---|---|---|---|
| 376-2 | 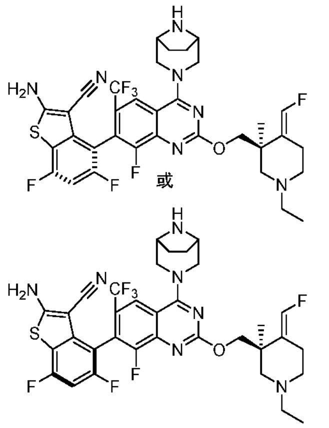 或 | (*Sa*) or (*Ra*)-4-(4-((1*R*,5*S*)-3,8-diazabicyclo[3.2.1] octan-3-yl)-2-(((*S*,*E*)-1-ethyl-4-(fluoromethylene)-3-methylpiperidin-3-yl)methoxy)-8-fluoro-6-(trifluoromethyl)quinazolin-7-yl)-2-amino-5,7-difluorobenzo[b]thiophene-3-carbonitrile | LCMS (m/z): 720.2 (M + H). |
| 377-1 | 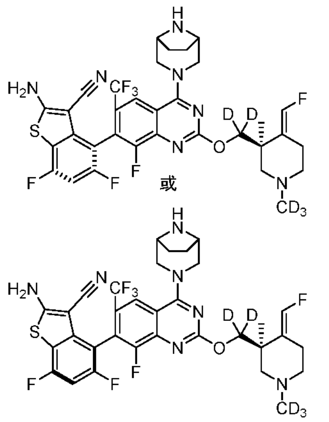 或 | (*Sa*) or (*Ra*)-4-(4-((1*R*,5*S*)-3,8-diazabicyclo[3.2.1] octan-3-yl)-8-fluoro-2-(((*S*,*E*)-4-(fluoromethylene)-3-methyl-1-(methyl-$d_3$)piperidin-3-yl)methoxy-$d_2$)-6-(trifluoromethyl)quinazolin-7-yl)-2-amino-5,7-difluorobenzo[*b*]thiophene-3-carbonitrile | LCMS (m/z): 711.2 (M + H). |
| 377-2 | 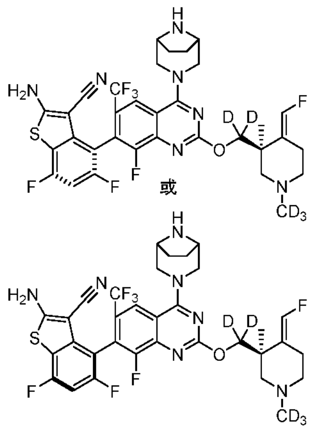 或 | (*Sa*) or (*Ra*)-4-(4-((1*R*,5*S*)-3,8-diazabicyclo[3.2.1] octane-3-yl)-8-fluoro-2-(((*S*,*E*)-4-(fluoromethylene)-3-methyl-1-(methyl-$d_3$)piperidin-3-yl)methoxy-$d_2$)-6-(trifluoromethyl)quinazolin-7-yl)-2-amino-5,7-difluorobenzo[*b*]thiophene-3-carbonitrile | LCMS (m/z): 711.2 (M + H). |

## Intermediates 378-1-2A and 378-1-2B

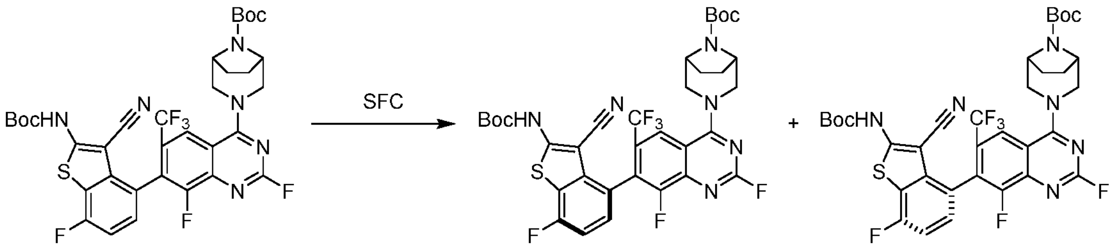


378-1-2 378-1-2A或378-1-2B 378-1-2A或378-1-2B

The synthesis of compound **378-1-2** was carried out by referring to the method for compound **362-1-2.** Compound **378-1-2** (970 mg) was resolved by chiral Waters SFC150 (separation column: (S,S) WHELK-01, 250*30 mm, 10μm; mobile phase: $CO_2$/MeOH = 70/30; flow rate: 120 mL/min). The first eluted isomer 1 was obtained as Example **378-1-2A** (300 mg, with a relatively shorter retention time). Chiral analytical method SFC-16, Rt = 2.120. The subsequently eluted isomer 2 was obtained as Example **378-1-2B** (200 mg, with longer retention time). Chiral analytical method SFC-16, Rt = 2.678.

The following compounds were prepared by referring to the above-mentioned methods using Intermediates **378-1-2A** and **378-1-2B.** It should be noted that for the naming of the following isomers-1 or -2, the one with a shorter retention time during the chiral resolution of the final compound was named as -1, and the one with a longer retention time was named as -2. Alternatively, the compound synthesized using **378-1-2A** as the intermediate was named as isomer-1, and the compound synthesized using intermediate **378-1-2B** as the intermediate was named as isomer-2. When there is a conflict between the two naming methods, the naming based on the preparation with the latter chiral-pure intermediate prevails.

| Ex. | Structural Formula | Name | Characterization data |
|---|---|---|---|
| 378-1 | 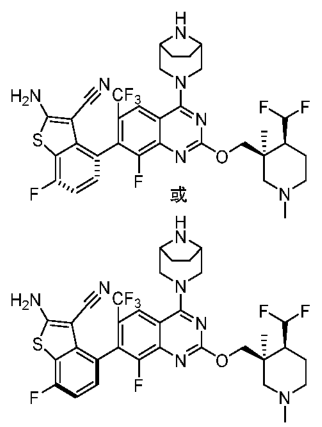 或 | (*Sa*) or (*Ra*)-4-(4-((1*R*,5*S*)-3,8-diazabicyclo[3.2.1]octan-3-yl)-2-(((3*S*,4*S*)-4-(difluoromethyl)-1,3-dimethylpiperidin-3-yl)methoxy)-8-fluoro-6-(trifluoromethyl)quinazolin-7-yl)-2-amino-7-fluorobenzo[*b*]thiophene-3-carbonitrile | LCMS (m/z): 708.2 (M + H). |
| 378-2 | 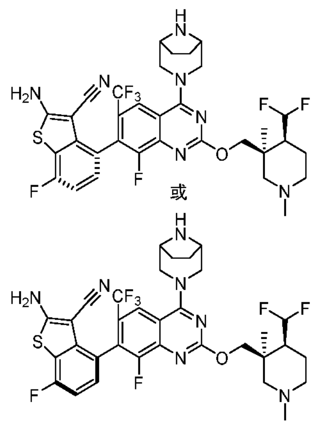 或 | (*Sa*) or (*Ra*)-4-(4-((1*R*,5*S*)-3,8-diazabicyclo[3.2.1]octan-3-yl)-2-(((3*S*,4*S*)-4-(difluoromethyl)-1,3-dimethylpiperidin-3-yl)methoxy)-8-fluoro-6-(trifluoromethyl)quinazolin-7-yl)-2-amino-7-fluorobenzo[*b*]thiophene-3-carbonitrile | LCMS (m/z): 708.2 (M + H). |

(continued)

| Ex. | Structural Formula | Name | Characterization data |
|---|---|---|---|
| 379-1 | 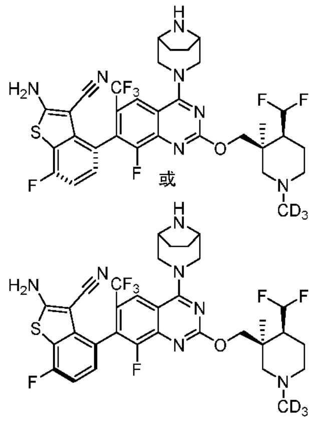 或 | (*Sa*) or (*Ra*)-4-(4-((1*R*,5*S*)-3,8-diazabicyclo[3.2.1] octan-3-yl)-2-(((3*S*,4*S*)-4-(difluoromethyl)-3-methyl-1-(methyl-$d_3$)piperidin-3-yl)methoxy)-8-fluoro-6-(trifluoromethyl)quinazolin-7-yl)-2-amino-7-fluorobenzo[*b*]thiophene-3-carbonitrile | LCMS (m/z): 711.2 (M + H). |
| 379-2 | 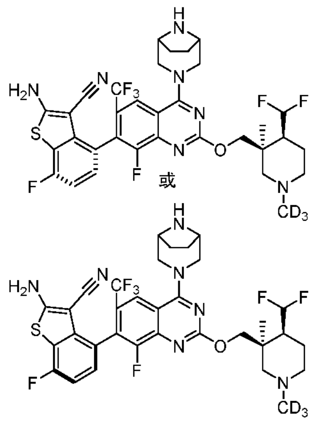 或 | (*Sa*) or (*Ra*)-4-(4-((1*R*,5*S*)-3,8-diazabicyclo[3.2.1] octan-3-yl)-2-(((3*S*,4*S*)-4-(difluoromethyl)-3-methyl-1-(methyl-$d_3$)piperidin-3-yl)methoxy)-8-fluoro-6-(trifluoromethyl)quinazolin-7-yl)-2-amino-7-fluorobenzo[*b*]thiophene-3-carbonitrile | LCMS (m/z): 711.2 (M + H). |
| 380-1 | 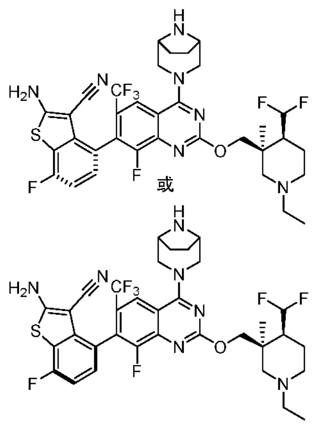 或 | (*Sa*) or (*Ra*)-4-(4-((1*R*,5*S*)-3,8-diazabicyclo[3.2.1] octan-3-yl)-2-(((3*S*,4*S*)-4-(difluoromethyl)-1-ethyl-3-methylpiperidin-3-yl)methoxy)-8-fluoro-6-(trifluoromethyl)quinazolin-7-yl)-2-amino-7-fluorobenzo[*b*] thiophene-3-carbonitrile | LCMS (m/z): 722.2 (M + H). |

(continued)

| Ex. | Structural Formula | Name | Characterization data |
|---|---|---|---|
| 380-2 | 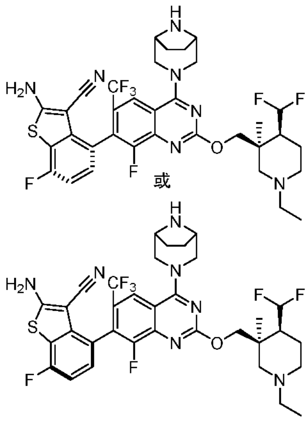 或 | (*Sa*) or (*Ra*)-4-(4-((1*R*,5*S*)-3,8-diazabicyclo[3.2.1] octan-3-yl)-2-(((3*S*,4*S*)-4-(difluoromethyl)-1-ethyl-3-methylpiperidin-3-yl)methoxy)-8-fluoro-6-(trifluoromethyl)quinazolin-7-yl)-2-amino-7-fluorobenzo[*b*] thiophene-3-carbonitrile | LCMS (m/z): 722.2 (M + H). |
| 381-1 | 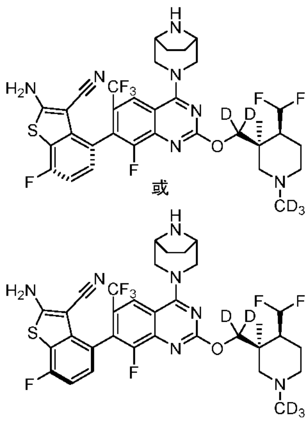 或 | (*Sa*) or (*Ra*)-4-(4-((1*R*,5*S*)-3,8-diazabicyclo[3.2.1] octan-3-yl)-2-(((3*S*,4*S*)-4-(difluoromethyl)-3-methyl-1-(methyl-*d*$_3$)piperidin-3-yl)methoxy-*d*$_2$)-8-fluoro-6-(trifluoromethyl)quinazolin-7-yl)-2-amino-7-fluorobenzo[*b*]thiophene-3-carbonitrile | LCMS (m/z): 713.3 (M + H). |
| 381-2 | 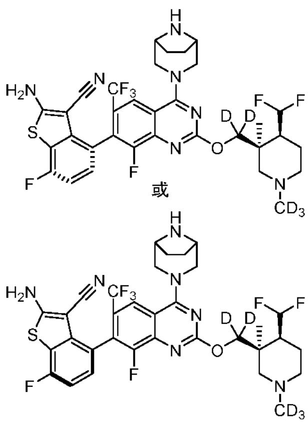 或 | (*Sa*) or (*Ra*)-4-(4-((1*R*,5*S*)-3,8-diazabicyclo[3.2.1] octan-3-yl)-2-(((3*S*,4*S*)-4-(difluoromethyl)-3-methyl-1-(methyl-*d*$_3$)piperidin-3-yl)methoxy-*d*$_2$)-8-fluoro-6-(trifluoromethyl)quinazolin-7-yl)-2-amino-7-fluorobenzo[*b*]thiophene-3-carbonitrile | LCMS (m/z): 713.3 (M + H). |

(continued)

| Ex. | Structural Formula | Name | Characterization data |
|---|---|---|---|
| 382-1 | 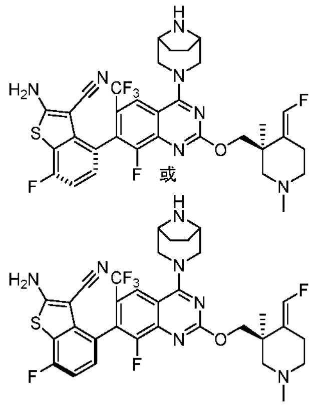 或 | (*Sa*) or (*Ra*)-4-(4-((1*R*,5*S*)-3,8-diazabicyclo[3.2.1] octan-3-yl)-8-fluoro-2-(((*S*,*E*)-4-(fluoromethylene)-1,3-dimethylpiperidin-3-yl)methoxy)-6-(trifluoromethyl)quinazolin-7-yl)-2-amino-7-fluorobenzo[*b*] thiophene-3-carbonitrile | LCMS (m/z): 688.2 (M + H). |
| 382-2 | 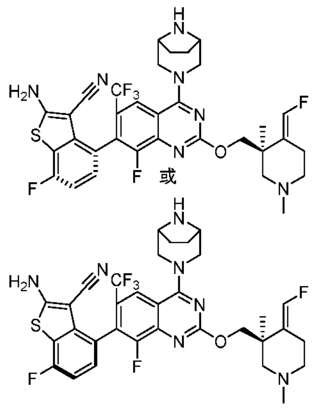 或 | (*Sa*) or (*Ra*)-4-(4-((1*R*,5*S*)-3,8-diazabicyclo[3.2.1] octan-3-yl)-8-fluoro-2-(((*S*,*E*)-4-(fluoromethylene)-1,3-dimethylpiperidin-3-yl)methoxy)-6-(trifluoromethyl)quinazolin-7-yl)-2-amino-7-fluorobenzo[*b*] thiophene-3-carbonitrile | LCMS (m/z): 688.2 (M + H). |
| 383-1 | 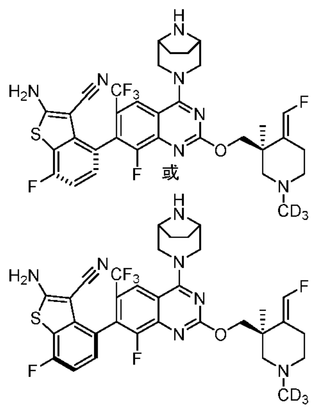 或 | (*Sa*) or (*Ra*)-4-(4-((1*R*,5*S*)-3,8-diazabicyclo[3.2.1] octan-3-yl)-8-fluoro-2-(((*S*,*E*)-4-(fluoromethylene)-3-methyl-1-(methyl-$d_3$)piperidin-3-yl)methoxy)-6-(trifluoromethyl)quinazolin-7-yl)-2-amino-7-fluorobenzo [*b*]thiophene-3-carbonitrile | LCMS (m/z): 691.2 (M + H). |
| 383-2 | 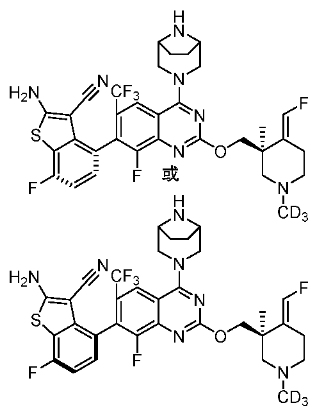 或 | (*Sa*) or (*Ra*)-4-(4-((1*R*,5*S*)-3,8-diazabicyclo[3.2.1] octan-3-yl)-8-fluoro-2-(((*S*,*E*)-4-(fluoromethylene)-3-methyl-1-(methyl-$d_3$)piperidin-3-yl)methoxy)-6-(trifluoromethyl)quinazolin-7-yl)-2-amino-7-fluorobenzo [*b*]thiophene-3-carbonitrile | LCMS (m/z): 691.2 (M + H). |

(continued)

| Ex. | Structural Formula | Name | Characterization data |
|---|---|---|---|
| 384-1 | 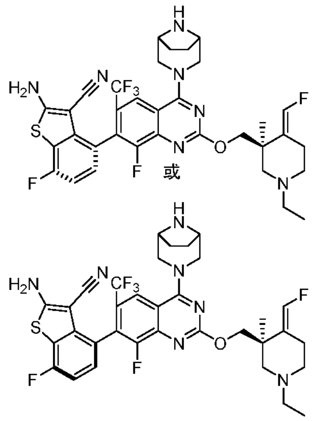 或 | (*Sa*) or (*Ra*)-4-(4-((1*R*,5*S*)-3,8-diazabicyclo[3.2.1] octan-3-yl)-2-(((*S*,*E*)-1-ethyl-4-(fluoromethylene)-3-methylpiperidin-3-yl)methoxy)-8-fluoro-6-(trifluoromethyl)quinazolin-7-yl)-2-amino-7-fluorobenzo[*b*] thiophene-3-carbonitrile | LCMS (m/z): 702.2 (M + H). |
| 384-2 | 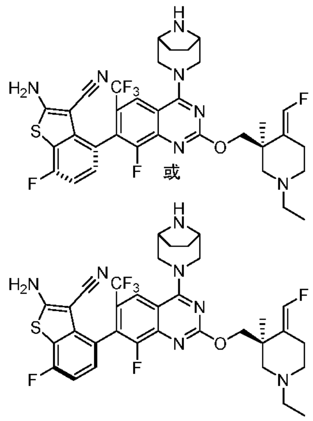 或 | (*Sa*) or (*Ra*)-4-(4-((1*R*,5*S*)-3,8-diazabicyclo[3.2.1] octan-3-yl)-2-(((*S*,*E*)-1-ethyl-4-(fluoromethylene)-3-methylpiperidin-3-yl)methoxy)-8-fluoro-6-(trifluoromethyl)quinazolin-7-yl)-2-amino-7-fluorobenzo[*b*] thiophene-3-carbonitrile | LCMS (m/z): 702.2 (M + H). |
| 385-1 | 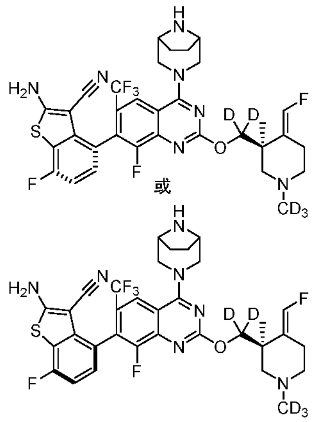 或 | (*Sa*) or (*Ra*)-4-(4-((1*R*,5*S*)-3,8-diazabicyclo[3.2.1] octan-3-yl)-8-fluoro-2-(((*S*,*E*)-4-(fluoromethylene)-3-methyl-1-(methyl-$d_3$)piperidin-3-yl)methoxy-$d_2$)-6-(trifluoromethyl)quinazolin-7-yl)-2-amino-7-fluorobenzo[*b*]thiophene-3-carbonitrile | LCMS (m/z): 693.2 (M + H). |

(continued)

| Ex. | Structural Formula | Name | Characterization data |
|---|---|---|---|
| 385-2 | 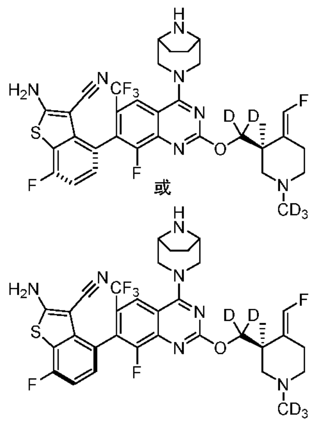 | (*Sa*) or (*Ra*)-4-(4-((1*R*,5*S*)-3,8-diazabicyclo[3.2.1]octane-3-yl)-8-fluoro-2-(((*S,E*)-4-(fluoromethylene)-3-methyl-1-(methyl-$d_3$)piperidin-3-yl)methoxy-$d_2$)-6-(trifluoromethyl)quinazolin-7-yl)-2-amino-7-fluorobenzo[*b*]thiophene-3-carbonitrile | LCMS (m/z): 693.2 (M + H). |

The following compounds were also prepared in a similar manner by referring to the above method.

| | | | |
|---|---|---|---|
| 386-1 | 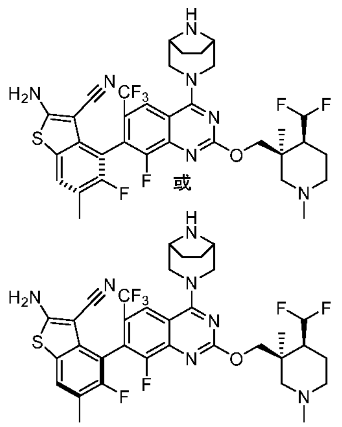 | (*Sa*) or (*Ra*)-4-(4-((1*R*,5*S*)-3,8-diazabicyclo[3.2.1]octan-3-yl)-2-(((3*S*,4*S*)-4-(difluoromethyl)-1,3-dimethylpiperidin-3-yl)methoxy)-8-fluoro-6-(trifluoromethyl)quinazolin-7-yl)-2-amino-5-fluoro-6-methylbenzo[*b*]thiophene-3-carbonitrile | LCMS (m/z): 722.2 (M + H). |
| 386-2 | 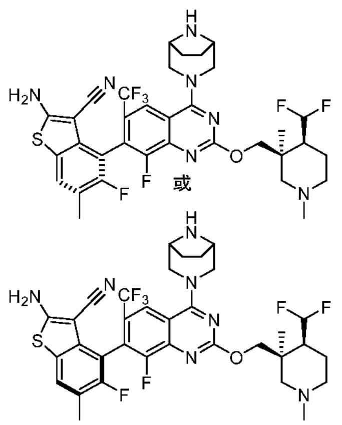 | (*Sa*) or (*Ra*)-4-(4-((1*R*,5*S*)-3,8-diazabicyclo[3.2.1]octan-3-yl)-2-(((3*S*,4*S*)-4-(difluoromethyl)-1,3-dimethylpiperidin-3-yl)methoxy)-8-fluoro-6-(trifluoromethyl)quinazolin-7-yl)-2-amino-5-fluoro-6-methylbenzo[*b*]thiophene-3-carbonitrile | LCMS (m/z): 722.2 (M + H). |

(continued)

| | | | |
|---|---|---|---|
| 387-1 | 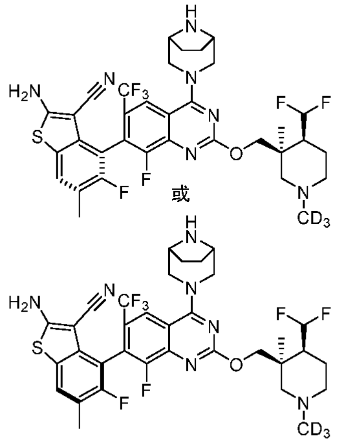 或 | (*Sa*) or (*Ra*)-4-(4-((1*R*,5*S*)-3,8-diazabicyclo[3.2.1]octan-3-yl)-2-(((3*S*,4*S*)-4-(difluoromethyl)-3-methyl-1-(methyl-*d*$_3$)piperidin-3-yl)methoxy)-8-fluoro-6-(trifluoromethyl)quinazolin-7-yl)-2-amino-5-fluoro-6-methylbenzo[*b*]thiophene-3-carbonitrile | LCMS (m/z): 725.3 (M + H). |
| 387-2 | 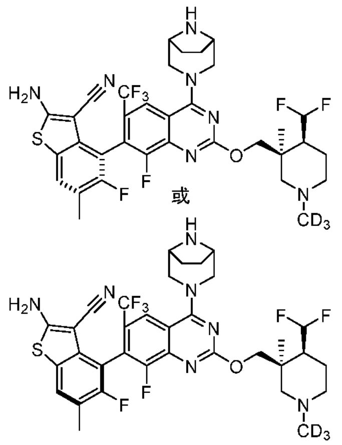 或 | (*Sa*) or (*Ra*)-4-(4-((1*R*,5*S*)-3,8-diazabicyclo[3.2.1]octan-3-yl)-2-(((3*S*,4*S*)-4-(difluoromethyl)-3-methyl-1-(methyl-*d*$_3$)piperidin-3-yl)methoxy)-8-fluoro-6-(trifluoromethyl)quinazolin-7-yl)-2-amino-5-fluoro-6-methylbenzo[*b*]thiophene-3-carbonitrile | LCMS (m/z): 725.3 (M + H). |
| 388-1 | 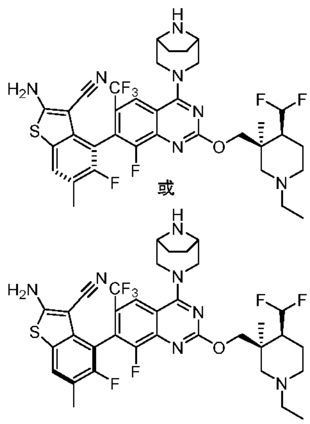 或 | (*Sa*) or (*Ra*)-4-(4-((1*R*,5*S*)-3,8-diazabicyclo[3.2.1]octan-3-yl)-2-(((3*S*,4*S*)-4-(difluoromethyl)-1-ethyl-3-methylpiperidin-3-yl)methoxy)-8-fluoro-6-(trifluoromethyl)quinazolin-7-yl)-2-amino-5-fluoro-6-methylbenzo[*b*]thiophene-3-carbonitrile | LCMS (m/z): 736.3 (M + H). |

(continued)

| | | | |
|---|---|---|---|
| 388-2 | 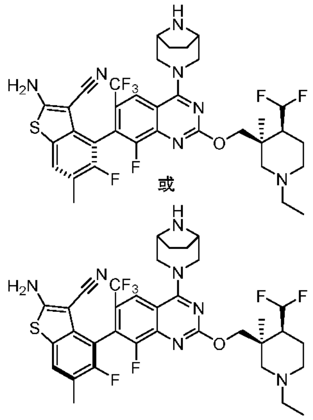 或 | (*Sa*) or (*Ra*)-4-(4-((1*R*,5*S*)-3,8-diazabicyclo[3.2.1]octane-3-yl)-2-(((3*S*,4*S*)-4-(difluoromethyl)-1-ethyl-3-methylpiperidin-3-yl)methoxy)-8-fluoro-6-(trifluoromethyl)quinazoline-7-yl)-2-amino-5-fluoro-6-methylbenzo[*b*]thiophene-3-carbonitrile | LCMS (m/z): 736.3 (M + H). |
| 389-1 | 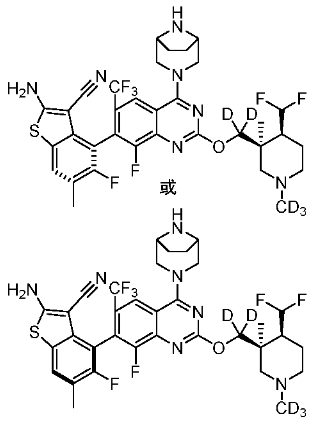 或 | (*Sa*) or (*Ra*)-4-(4-((1*R*,5*S*)-3,8-diazabicyclo[3.2.1]octan-3-yl)-2-(((3*S*,4*S*)-4-(difluoromethyl)-3-methyl-1-(methyl-$d_3$)piperidin-3-yl)methoxy-$d_2$)-8-fluoro-6-(trifluoromethyl)quinazolin-7-yl)-2-amino-5-fluoro-6-methylbenzo[*b*]thiophene-3-carbonitrile | LCMS (m/z): 727.3 (M + H). |
| 389-2 | 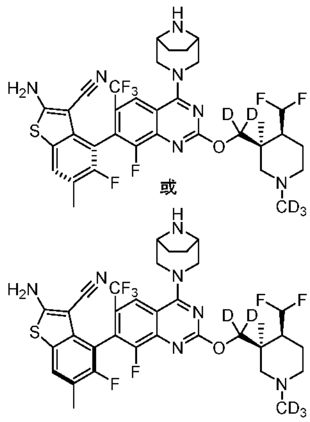 或 | (*Sa*) or (*Ra*)-4-(4-((1*R*,5*S*)-3,8-diazabicyclo[3.2.1]octan-3-yl)-2-((3*S*,4*S*)-4-(difluoromethyl)-3-methyl-1-(methyl-$d_3$)piperidin-3-yl)methoxy-$d_2$)-8-fluoro-6-(trifluoromethyl)quinazolin-7-yl)-2-amino-5-fluoro-6-methylbenzo[*b*]thiophene-3-carbonitrile | LCMS (m/z): 727.3 (M + H). |
| 390-1 | 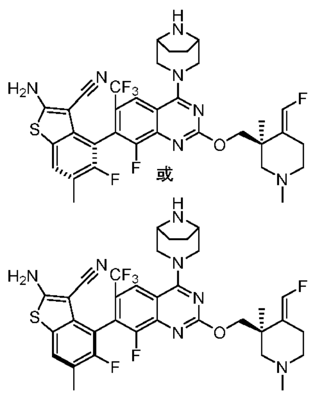 或 | (*Sa*) or (*Ra*)-4-(4-((1*R*,5*S*)-3,8-diazabicyclo[3.2.1]octan-3-yl)-8-fluoro-2-((*S*,*E*)-4-(fluoromethylene)-1,3-dimethylpiperidin-3-yl)methoxy)-6-(trifluoromethyl)quinazolin-7-yl)-2-amino-5-fluoro-6-methylbenzo[*b*]thiophene-3-carbonitrile | LCMS (m/z): 702.2 (M + H). |

(continued)

| | | | |
|---|---|---|---|
| 390-2 | 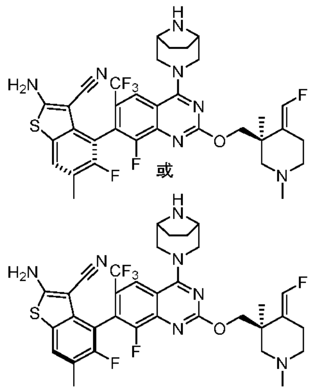 或 | (*Sa*) or (*Ra*)-4-(4-((1*R*,5*S*)-3,8-diazabicyclo[3.2.1]octan-3-yl)-8-fluoro-2-((*S,E*)-4-(fluoromethylene)-1,3-dimethylpiperidien-3-yl)methoxy)-6-(trifluoromethyl)quinazolin-7-yl)-2-amino-5-fluoro-6-methylbenzo[*b*]thiophene-3-carbonitrile | LCMS (m/z): 702.2 (M + H). |
| 391-1 | 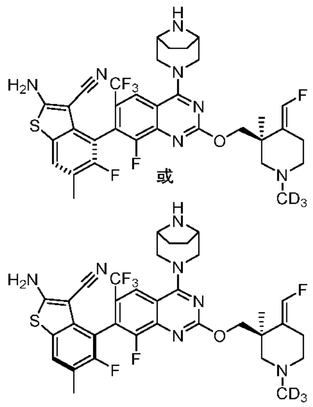 或 | (*Sa*) or (*Ra*)-4-(4-((1*R*,5*S*)-3,8-diazabicyclo[3.2.1]octan-3-yl)-8-fluoro-2-(((*S*,*E*)-4-(fluoromethylene)-3-methyl-1-(methyl-$d_3$)piperidin-3-yl)methoxy)-6-(trifluoromethyl)quinazolin-7-yl)-2-amino-5-fluoro-6-methylbenzo[*b*] thiophene-3-carbonitrile | LCMS (m/z): 705.3 (M + H). |
| 391-2 | 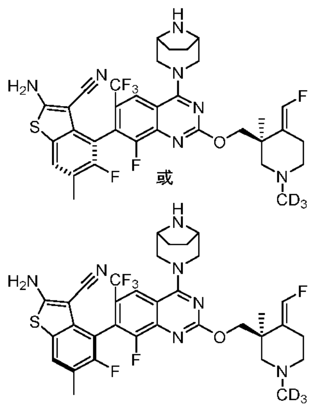 或 | (*Sa*) or (*Ra*)-4-(4-((1*R*,5*S*)-3,8-diazabicyclo[3.2.1]octan-3-yl)-8-fluoro-2-(((*S*,*E*)-4-(fluoromethylene)-3-methyl-1-(methyl-$d_3$)piperidin-3-yl)methoxy)-6-(trifluoromethyl)quinazolin-7-yl)-2-amino-5-fluoro-6-methylbenzo[*b*] thiophene-3-carbonitrile | LCMS (m/z): 705.3 (M + H). |
| 392-1 | 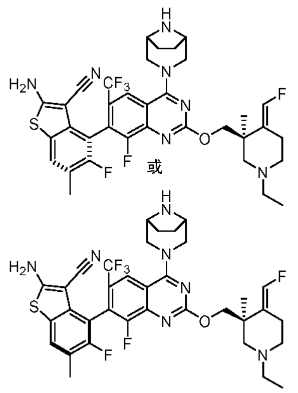 或 | (*Sa*) or (*Ra*)-4-(4-((1*R*,5*S*)-3,8-diazabicyclo[3.2.1]octan-3-yl)-2-(((*S*,*E*)-1-ethyl-4-(fluoromethylene)-3-methylpiperidin-3-yl)methoxy)-8-fluoro-6-(trifluoromethyl)quinazolin-7-yl)-2-amino-5-fluoro-6-methylbenzo[*b*]thiophene-3-carbonitrile | LCMS (m/z): 716.3 (M + H). |

(continued)

| | | | |
|---|---|---|---|
| 392-2 | 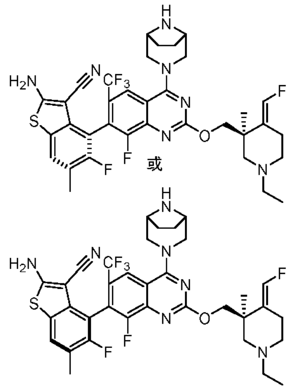 或 | (*Sa*) or (*Ra*)-4-(4-((1*R*,5*S*)-3,8-diazabicyclo[3.2.1]octan-3-yl)-2-(((*S*,*E*)-1-ethyl-4-(fluoromethylene)-3-methylpiperidin-3-yl)methoxy)-8-fluoro-6-(trifluoromethyl)quinazolin-7-yl)-2-amino-5-fluoro-6-methylbenzo[*b*]thiophene-3-carbonitrile | LCMS (m/z): 716.3 (M + H). |
| 393-1 | 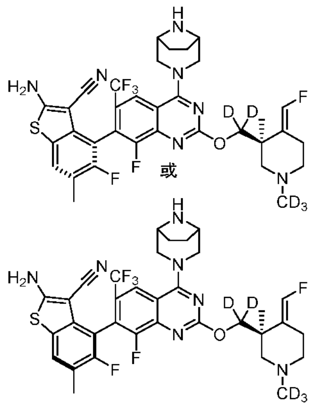 或 | (*Sa*) or (*Ra*)-4-(4-((1*R*,5*S*)-3,8-diazabicyclo[3.2.1]octan-3-yl)-8-fluoro-2-(((*S*,*E*)-4-(fluoromethylene)-3-methyl-1-(methyl-*d*$_3$)piperidin-3-yl)methoxy-*d*$_2$)-6-(trifluoromethyl)quinazolin-7-yl)-2-amino-5-fluoro-6-methylbenzo[*b*]thiophene-3-carbonitrile | LCMS (m/z): 707.3 (M + H). |
| 393-2 | 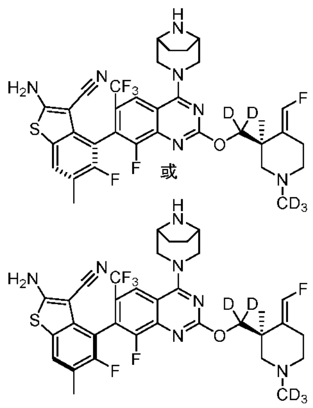 或 | (*Sa*) or (*Ra*)-4-(4-((1*R*,5*S*)-3,8-diazabicyclo[3.2.1]octan-3-yl)-8-fluoro-2-(((*S*,*E*)-4-(fluoromethylene)-3-methyl-1-(methyl-*d*$_3$)piperidin-3-yl)methoxy-*d*$_2$)-6-(trifluoromethyl)quinazolin-7-yl)-2-amino-5-fluoro-6-methylbenzo[*b*]thiophene-3-carbonitrile | LCMS (m/z): 707.3 (M + H). |
| 394-1 | 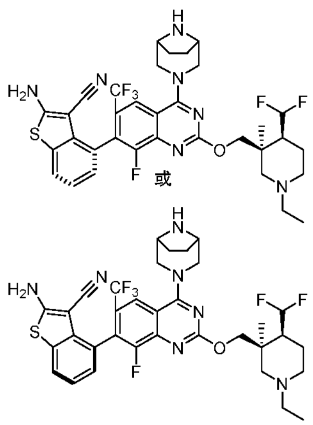 或 | (*Sa*) or (*Ra*)-4-(4-((1*R*,5*S*)-3,8-diazabicyclo[3.2.1]octan-3-yl)-2-(((3*S*,4*S*)-4-(difluoromethyl)-1-ethyl-3-methylpiperidin-3-yl)methoxy)-8-fluoro-6-(trifluoromethyl)quinazolin-7-yl)-2-aminobenzo[*b*]thiophene-3-carbonitrile | LCMS (m/z): 704.3 (M + H). |

(continued)

| | | | |
|---|---|---|---|
| 394-2 | 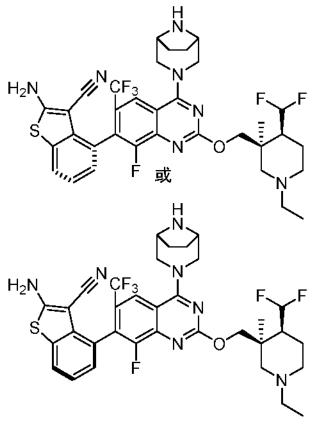 或 | (*Sa*) or (*Ra*)-4-(4-((1*R*,5*S*)-3,8-diazabicyclo[3.2.1]octan-3-yl)-2-(((3*S*,4*S*)-4-(difluoromethyl)-1-ethyl-3-methylpiperidin-3-yl)methoxy)-8-fluoro-6-(trifluoromethyl)quinazolin-7-yl)-2-aminobenzo[*b*]thiophene-3-carbonitrile | LCMS (m/z): 704.3 (M + H). |
| 395-1 | 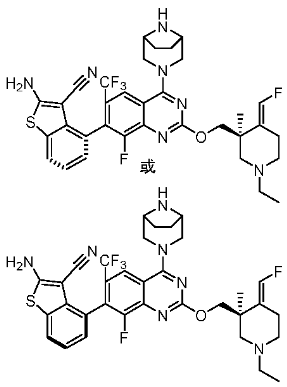 或 | (*Sa*) or (*Ra*)-4-(4-((1*R*,5*S*)-3,8-diazabicyclo[3.2.1]octan-3-yl)-2-(((*S,E*)-1-ethyl-4-(fluoromethylene)-3-methylpiperidin-3-yl)methoxy)-8-fluoro-6-(trifluoromethyl)quinazolin-7-yl)-2-aminobenzo[*b*]thiophene-3-carbonitrile | LCMS (m/z): 684.3 (M + H). |
| 395-2 | 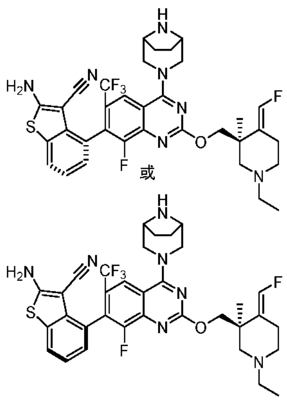 或 | (*Sa*) or (*Ra*)-4-(4-((1*R*,5*S*)-3,8-diazabicyclo[3.2.1]octan-3-yl)-2-(((*S,E*)-1-ethyl-4-(fluoromethylene)-3-methylpiperidin-3-yl)methoxy)-8-fluoro-6-(trifluoromethyl)quinazolie-7-yl)-2-aminobenzo[*b*]thiophene-3-carbonitrile | LCMS (m/z): 684.3 (M + H). |

**Example 296**

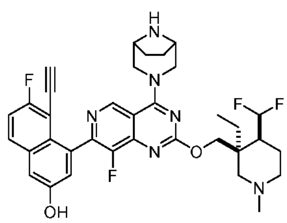

(±)4-(4-((1*R*,5*S*)-3,8-diazabicyclo[3.2.1]octan-3-yl)-2-(((3*S*,4*S*)-4-(difluoromethyl)-3-ethyl-1-methylpiperidin-3-yl)methoxy)-8-fluoropyrido[4,3-*d*]pyrimidin-7-yl)-5-ethynyl-6-fluoronaphthalen-2-ol

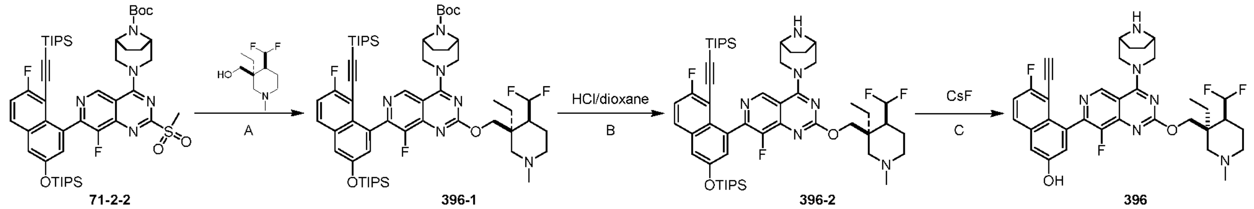

71-2-2 396-1 396-2 396

**Step** A: (±)-*tert*-butyl (1*R*,5*S*)-3-(2-(((3*S*,4*S*)-4-(difluoromethyl)-3-ethyl-1-methylpiperidin-3-yl)methoxy)-8-fluoro-7-(7-fluoro-8-((triisopropylsilyl)ethynyl)-3-((triisopropylsilyl)oxy)naphthalen-1-yl)pyrido[4,3-*d*]pyrimidin-4-yl)-3,8-diazabicyclo [3.2.1]octan-8-carboxylate

At room temperature, tert-butyl (1*R*,5*S*)-3-(8-fluoro-7-(7-fluoro-8-((triisopropylsilyl)ethynyl)-3-((triisopropylsilyl) oxy)naphthalen-1-yl)-2-(methylsulfonyl)pyrido[4,3-d]pyrimidin-4-yl)-3,8-diazabicyclo[3.2.1]octan-8-carboxylate (300 mg, 0.321 mmol) and (±)-(3*S*,4*S*)-4-(difluoromethyl)-3-ethyl-1-methylpiperidin-3-yl)methanol (90 mg, 0.434 mmol) were dissolved in anhydrous toluene (3 mL), and the system was degassed with nitrogen three times. Under the condition of dry ice-ethanol, the resulting reaction solution was cooled to -70 °C, and a 2 M sodium tert-butoxide solution in tetrahydrofuran (0.482 mL, 0.963 mmol) was added dropwise. The resulting mixture was stirred at - 70 °C for 1 h. After the reaction was completed (monitored by LCMS), the reaction was quenched with saturated aqueous ammonium chloride (50 mL) and extracted with ethyl acetate (50 mL × 3). The combined organic layer was washed with brine, dried over anhydrous sodium sulfate, filtered and concentrated to dryness to obtain (±)-*tert*-butyl (1R,5S)-3-(2-(((3S,4S)-4-(difluoromethyl)-3-ethyl-1-methylpiperidin-3-yl)methoxy)-8-fluoro-7-(7-fluoro-8-((triisopropylsilyl)ethynyl)-3-((triisopropylsilyl)oxy)naphthalen-1-yl) pyrido[4,3-*d*]pyrimidin-4-yl)-3,8-diazabicyclo[3.2.1]octan-8-carboxylate (350 mg, crude) as a white solid. LCMS (m/z): 531.3 (M/2 + H).

**Step B:** (±)-4-((1*R*,5*S*)-3,8-diazabicyclo[3.2.1]octan-3-yl)-2-(((3*S*,4*S*)-4-(difluoromethyl)-3-ethyl-1-methylpiperidin-3-yl)methoxy)-8-fluoro-7-(7-fluoro-8-((triisopropylsilyl)ethynyl)-3-((triisopropylsilyl)oxy) naphthalen-1-yl)pyrido[4,3-d]pyrimidine

At room temperature, (±)-*tert*-butyl (1*R*,5*S*)-3-(2-(((3*S*,4*S*)-4-(difluoromethyl)-3-ethyl-1-methylpiperidin-3-yl) methoxy)-8-fluoro-7-(7-fluoro-8-((triisopropylsilyl)ethynyl)-3-((triisopropylsilyl)oxy)naphthalen-1-yl)pyrido[4,3-d]pyrimidin-4-yl)-3,8-diazabicyclo[3.2.1]octan-8-carboxylate (350 mg, crude) was dissolved in 4 M hydrochloric acid-dioxane (10 mL), and the resulting mixture was stirred at room temperature for 1 h. After the reaction was completed (monitored by LCMS), the reaction was concentrated. The crude product was diluted with saturated sodium bicarbonate (30 mL) and extracted by ethyl acetate (30 mL × 3). The combined organic layer was washed with brine, dried over anhydrous sodium sulfate, filtered and concentrated to dryness to obtain (±)-4-((1*R*,5*S*)-3,8-diazabicyclo[3.2.1]octan-3-yl)-2-(((3*S*,4*S*)-4-(difluoromethyl)-3-ethyl-1-methylpiperidin-3-yl)methoxy)-8-fluoro-7-(7-fluoro-8-((triisopropylsilyl)ethynyl)-3-((triisopropylsilyl)oxy)naphthalen-1-yl)pyrido[4,3-d]pyrimidine (300 mg, crude) as a white solid. LCMS (m/z): 961.3 (M + H).

**Step C:** (±)4-(4-((1*R*,5*S*)-3,8-diazabicyclo[3.2.1]octan-3-yl)-2-(((3*S*,4*S*)-4-(difluoromethyl)-3-ethyl-1-methylpiperidin-3-yl)methoxy)-8-fluoropyrido[4,3-d]pyrimidin-7-yl)-5-ethynyl-6-fluoronaphthalen-2-ol

At room temperature, CsF (237 mg, 1.56 mmol) was added to a solution of (±)-4-((1*R*,5*S*)-3,8-diazabicyclo [3.2.1]octan-3-yl)-2-(((3*S*,4*S*)-4-(difluoromethyl)-3-ethyl-1-methylpiperidin-3-yl)methoxy)-8-fluoro-7-(7-fluoro-8-((triisopropylsilyl)ethynyl)-3-((triisopropylsilyl)oxy) naphthalen-1-yl)pyrido[4,3-d]pyrimidine (300 mg, crude) in DMF (5 mL), and the resulting mixture was stirred at 60 °C for 1 h. After the reaction was completed (monitored by LCMS), the reaction was directly filtered, and the filtrate was purified by pre-HPLC (C18, CAN/(10 mmol $NH_4HCO_3/H_2O$) = 35-75%) to obtain (±) 4-(4-((1*R*,5*S*)-3,8-diazabicyclo[3.2.1]octan-3-yl)-2-(((3*S*,4*S*)-4-(difluoromethyl)-3-ethyl-1-methylpiperidin-3-yl)methoxy)-8-fluoropyrido[4,3-d]pyrimidin-7-yl)-5-ethynyl-6-fluoronaphthalen-2-ol (70.0 mg) as a white solid. LCMS (m/z): 649.2 (M + H). $^1$H NMR (400 MHz, DMSO-$d_6$) δ 10.19 (s, 1H), 9.07 (s, 1H), 7.98 (dd, *J* = 9.2, 5.9 Hz, 1H), 7.50 - 7.44 (m, 1H), 7.40 (d, *J* = 2.6 Hz, 1H), 7.19 (d, *J= 2.5* Hz, 1H), 6.45 - 6.10 (m, 1H), 4.62 - 4.49 (m, 2H), 4.45 - 4.32 (m, 2H), 3.95 (dd, *J= 4.2,* 1.0 Hz, 1H), 3.93 - 3.83 (m, 2H), 3.80 - 3.66 (m, 2H), 2.86 - 2.79 (m, 1H), 2.79 - 2.70 (m, 1H), 2.12 (s, 3H), 1.96 - 1.71 (m, 9H),

1.70 - 1.60 (m, 1H), 1.60 - 1.49 (m, 1H), 0.92 - 0.83 (m, 3H). $^{19}F$ NMR (376 MHz, DMSO-$d_6$) δ- 110.77, -115.08, -119.15, -139.70.

The compounds listed in the following table were prepared and characterized by referring to the above synthetic methods or appropriate variations, as well as appropriate chiral resolution:

| Ex. | Structure | Name | Characterization data |
|---|---|---|---|
| 397 | 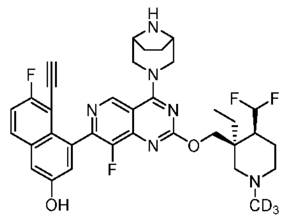 | 4-(4-((1R, 5S)-3,8-diazabicyclo[3.2.1]octan-3-yl)-2-(((3*S*,4*S*)-4-(difluoromethyl)-3-ethyl-1-(methyl-$d_3$)piperidin-3-yl)methoxy)-8-fluoropyrido[4,3-d]pyrimidin-7-yl)-5-ethynyl-6-fluoronaphthalen-2-ol | LCMS (m/z): 652.3 (M + H). |
| 398 | 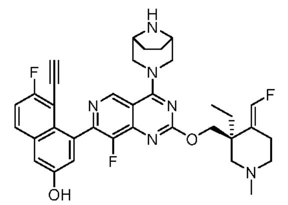 | 4-(4-((1R, 5S)-3,8-diazabicyclo[3.2.1]octan-3-yl)-2-(((*S,E*)-3-ethyl-4-(fluoromethylene)-1-methylpiperidin-3-yl)methoxy)-8-fluoropyrido[4,3-*d*]pyrimidin-7-yl)-5-ethynyl-6-fluoronaphthalen-2-ol | LCMS (m/z): 629.3 (M + H). |
| 399 | 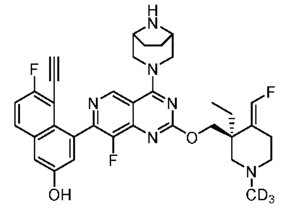 | 4-(4-((1R, 5S)-3,8-diazabicyclo[3.2.1]octan-3-yl)-2-(((*S,E*)-3-ethyl-4-(fluoromethylene)-1-methyl-$d_3$) piperidin-3-yl)methoxy)-8-fluoropyrido[4,3-*d*]pyrimidin-7-yl)-5-ethynyl-6-fluoronaphthalen-2-ol | LCMS (m/z): 632.3 (M + H). |
| 400 | 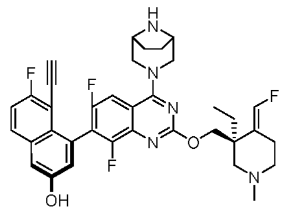 | (*Ra*)-4-(4-((1*R*,5*S*)-3,8-diazabicyclo[3.2.1]octan-3-yl)-2-(((S,E)-3-ethyl-4-(fluoromethylene)-1-methylpiperidin-3-yl)methoxy)-6,8-difluoroquinazolin-7-yl)-5-ethynyl-6-fluoronaphthalen-2-ol | LCMS (m/z): 646.3 (M + H). |
| 401 | 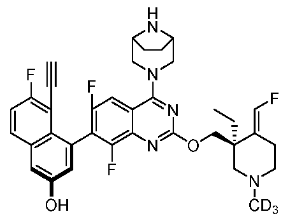 | (*Ra*)-4-(4-((1*R*,5*S*)-3,8-diazabicyclo[3.2.1]octan-3-yl)-2-(((S,E)-3-ethyl-4-fluoromethyl-1-methyl-$d_3$)piperidin-3-yl)methoxy)-6,8-difluoroquinazolin-7-yl)-5-ethynyl-6-fluoronaphthalen-2-ol | LCMS (m/z): 649.3 (M + H). |
| 402 | 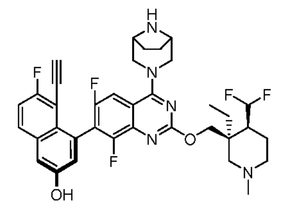 | (*Ra*)-4-(4-((1*R*,5*S*)-3,8-diazabicyclo[3.2.1]octan-3-yl)-2-(((3S, 4S)-4-(difluoromethyl)-3 - ethyl-1-methylpiperidin-3-yl)methoxy)-6,8-difluoroquinazolin-7-yl)-5-ethynyl-6-fluoronaphthalen-2-ol | LCMS (m/z): 666.3 (M + H). |

(continued)

| Ex. | Structure | Name | Characterization data |
|---|---|---|---|
| 403 | 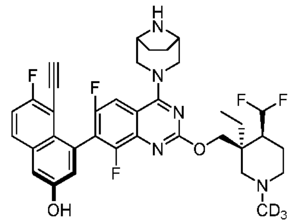 | (*Ra*)-4-(4-((1R, 5S)-3,8-diazabicyclo[3.2.1]octan-3-yl)-2-(((3S, 4S)-4-(difluoromethyl)-3 - ethyl-1-(methyl-$d_3$)piperidin-3-yl)methoxy)-6,8-difluoroquinazolin-7-yl)-5-ethynyl-6-fluoronaphthalen-2-ol | LCMS (m/z): 669.3 (M + H). |
| 404 | 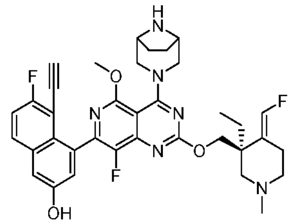 | 4-(4-((1R, 5S)-3,8-diazabicyclo[3.2.1]octan-3-yl)-2-(((*S,E*)-3-ethyl-4-(fluoromethylene)-1-methylpiperidin-3-yl)methoxy)-8-fluoro-5-methoxypyrido[4,3-d]pyrimidin-7-yl)-5-ethynyl-6-fluoronaphthalen-2-ol | LCMS (m/z): 659.3 (M + H). |
| 405 | 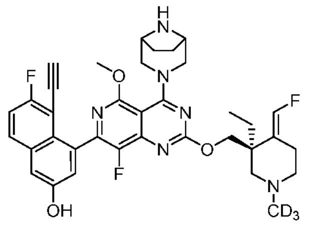 | 4-(4-((1R, 5S)-3,8-diazabicyclo[3.2.1]octan-3-yl)-2-(((*S,E*)-3-ethyl-4-(fluoromethylene)-1-methyl-$d_3$)piperidin-3-yl)methoxy)-8-fluoro-5-methoxypyrido[4,3-d]pyrimidin-7-yl)-5-ethynyl-6-fluoronaphthalen-2-ol | **LCMS** (m/z): 662.3 (M + H). |
| 406 | 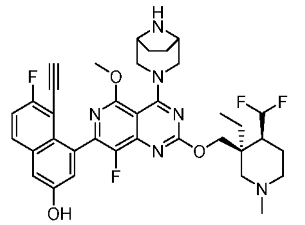 | 4-(4-(1*R*,5*S*)-3,8-diazabicyclo[3.2.1]octan-3-yl)-2-(((3*S*,4*S*)-4-(difluoromethyl)-3-ethyl-1-methylpiperidin-3-yl)methoxy)-8-fluoro-5-methoxypyrido[4,3-d]pyrimidin-7-yl)-5-ethynyl-6-fluoronaphthalen-2-ol | LCMS (m/z): 679.3 (M + H). |
| 407 | 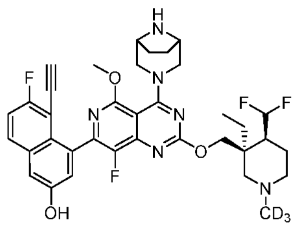 | 4-(4-(((1R, 5S)-3, 8-diazabicyclo[3.2.1]octan-3-yl)-2-(((3S, 4S)-4-(difluoromethyl)-3 - ethyl-1-methyl-$d_3$)piperidin-3-yl)methoxy)-8-fluoro-5-methoxypyrido[4,3-d]pyrimidin-7-yl)-5-ethynyl-6-fluoronaphthalene-2-ol | LCMS (m/z): 682.3 (M + H). |
| 408 | 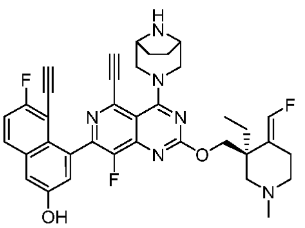 | 4-(4-((1R, 5S)-3,8-diazabicyclo[3.2.1]octan-3-yl)-2-(((S,E)-3-ethyl-4-(fluoromethylene)-1-methylpiperidin-3-yl)methoxy)-5-ethynyl-8-fluoropyrido[4,3-d]pyrimidin-7-yl)-5-ethynyl-6-fluoronaphthalen-2-ol | **LCMS** (m/z): 653.3 (M + H). |

(continued)

| Ex. | Structure | Name | Characterization data |
|---|---|---|---|
| 409 | 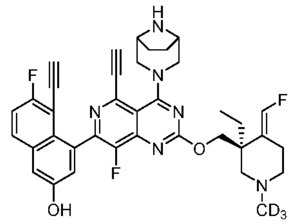 | 4-(4-((1R, 5S)-3,8-diazabicyclo[3.2.1]octan-3-yl)-2-(((S,E)-3-ethyl-4-(fluoromethylene)-1-(methyl-*d*$_3$) piperidin-3-yl)methoxy)-5-ethynyl-8-fluoropyrido[4,3-d] pyrimidin-7-yl)-5-ethynyl-6-fluoronaphthalen-2-ol | LCMS (m/z): 656.3 (M + H). |
| 410 | 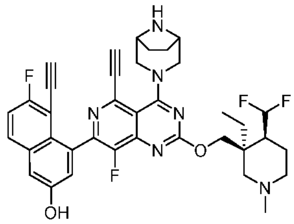 | 4-(4-((1R, 5S)-3,8-diazabicyclo[3.2.1]octan-3-yl)-2-(((3*S*,4*S*)-4-(difluoromethyl)-3-ethyl-1-methylpiperidin-3-yl)methoxy)-5-ethynyl-8-fluoropyrido[4,3-*d*] pyrimidin-7-yl)-5-ethynyl-6-fluoronaphthalen-2-ol | LCMS (m/z): 673.3 (M + H). |
| 411 | 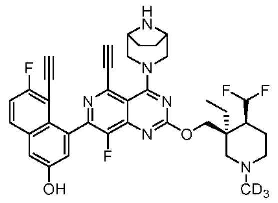 | 4-(4-((1R, 5S)-3,8-diazabicyclo[3.2.1]octan-3-yl)-2-(((3*S*,4*S*)-4-(difluoromethyl)-3-ethyl-1-(methyl-*d*$_3$)piperidin-3-yl)methoxy)-5-ethynyl-8-fluoropyrido[4,3-*d*]pyrimidin-7-yl)-5-ethynyl-6-fluoronaphthalen-2-ol | LCMS (m/z): 676.3 (M + H). |
| 412 | 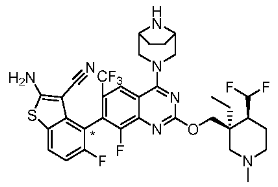 | (*Sa*) or (*Ra*)-4-(4-((1*R*,5*S*)-3,8-diazabicyclo[3.2.1]octan-3-yl)-2-(((3*S*,4*S*)-4-(difluoromethyl)-3-ethyl-1-methylpiperidin-3-yl)methoxy)-8-fluoro-6-(trifluoromethyl)quinazolin-7-yl)-2-amino-5-fluorobenzo[*b*]thiophen-3-carbonitrile | LCMS (m/z): 722.2 (M + H). |
| 413 | 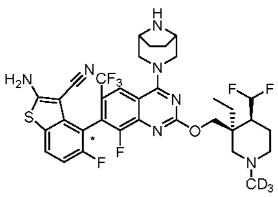 | (*Sa*) or (*Ra*)-4-(4-((1*R*,5*S*)-3,8-diazabicyclo[3.2.1]octan-3-yl)-2-(((3*S*,4*S*)-4-(difluoromethyl)-3 - ethyl-1-methyl-*d*$_3$)piperidin-3-yl)methoxy)-8-fluoro-6-(trifluoromethyl)quinazolin-7-yl)-2-amino-5-fluorobenzo[*b*]thiophen-3-carbonitrile | LCMS (m/z): 725.3 (M + H). |
| 414 | 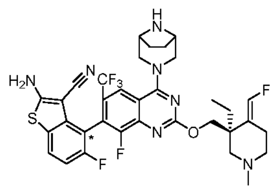 | (*Sa*) or (*Ra*)-4-(4-((1*R*,5*S*)-3,8-diazabicyclo[3.2.1]octan-3-yl)-2-(((S,E)-3-ethyl-4-(fluoromethylene)-1-methyl-*d*$_3$)piperidin-3-yl)methoxy)-8-fluoro-6-(trifluoromethyl)quinazolin-7-yl)-2-amino-5-fluorobenzo[*b*]thiophen-3-carbonitrile | LCMS (m/z): 702.2 (M + H). |
| 415 | 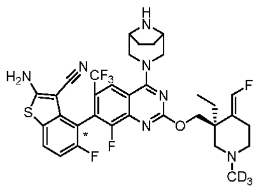 | (*Sa*) or (*Ra*)-4-(4-((1*R*,5*S*)-3,8-diazabicyclo[3.2.1]octan-3-yl)-2-(((S,E)-3-ethyl-4-(fluoromethylene)-1-methyl-*d*$_3$)piperidin-3-yl)methoxy)-8-fluoro-6-(trifluoromethyl)quinazolin-7-yl)-2-amino-5-fluorobenzo[*b*]thiophen-3-carbonitrile | LCMS (m/z): 705.3 (M + H). |

(continued)

| Ex. | Structure | Name | Characterization data |
|---|---|---|---|
| 416 | 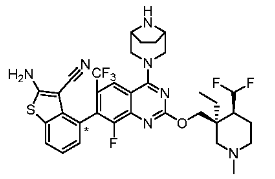 | (*Sa*) or (*Ra*)-4-(4-((1*R*,5*S*)-3,8-diazabicyclo[3.2.1]octan-3-yl)-2-(((3*S*,4*S*)-4-(difluoromethyl)-3-ethyl-1-methylpiperidin-3-yl)methoxy)-8-fluoro-6-(trifluoromethyl)quinazolin-7-yl)-2-aminobenzo[b]thiophen-3-carbonitrile | LCMS (m/z): 704.3 (M + H). |
| 417 | 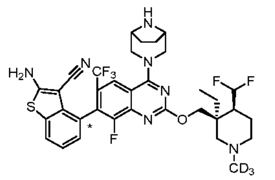 | (*Sa*) or (*Ra*)-4-(4-((1*R*,5*S*)-3,8-diazabicyclo[3.2.1]octan-3-yl)-2-(((3*S*,4*S*)-4-(difluoromethyl)-3-ethyl-1-methyl-$d_3$)piperidin-3-yl)methoxy)-8-fluoro-6-(trifluoromethyl)quinazolin-7-yl)-2-aminobenzo[b]thiophen-3-carbonitrile | LCMS (m/z): 707.3 (M + H). |
| 418 | 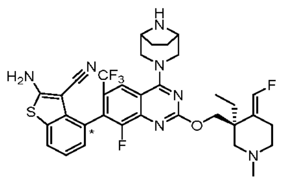 | (*Sa*) or (*Ra*)-4-(4-((1*R*,5*S*)-3,8-diazabicyclo[3.2.1]octan-3-yl)-2-(((S,E)-3-ethyl-4-(fluoromethylene)-1-methylpiperidin-3-yl)methoxy)-8-fluoro-6-(trifluoromethyl)quinazolin-7-yl)-2-aminobenzo[b]thiophen-3-carbonitrile | LCMS (m/z): 684.2 (M + H). |
| 419 | 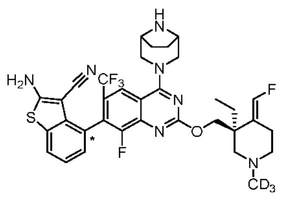 | (*Sa*) or (*Ra*)-4-(4-((1*R*,5*S*)-3,8-diazabicyclo[3.2.1]octan-3-yl)-2-(((*S*,*E*)-3-ethyl-4-(fluoromethylene)-1-(methyl-$d_3$)piperidin-3-yl)methoxy)-8-fluoro-6-(trifluoromethyl)quinazolin-7-yl)-2-aminobenzo[b]thiophen-3-carbonitrile | LCMS (m/z): 687.3 (M + H). |
| 420 | 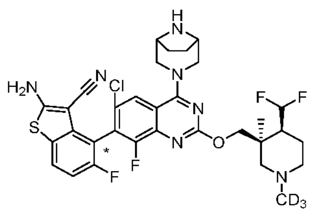 | (*Sa*) or (*Ra*)-4-(4-(1*R*,5*S*)-3,8-diazabicyclo[3.2.1]octan-3-yl)-6-chloro-2-((3S,4S)-4-(difluoromethyl)-3-methyl-1-(methyl-$d_3$)piperidin-3-methoxy)-8-fluoroquinazolin-7-yl)-2-amino-5-fluorobenzo[b]thiophene-3-carbonitrile | LCMS (m/z): 677.2 (M + H). |
| 421 | 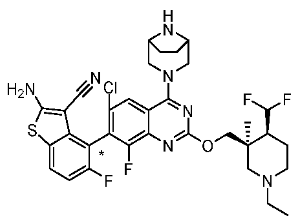 | (*Sa*) or (*Ra*)-4-(4-((1*R*,5*S*)-3,8-diazabicyclo[3.2.1]octan-3-yl)-6-chloro-2-(((3S, 4S)-4-(difluoromethyl)-1-ethyl-3-methylpiperidin-3-yl)methoxy)-8-fluoroquinazoline-7-yl)-2-amino-5-fluorobenzo[b]thiophen-3-carbonitrile | LCMS (m/z): 688.2 (M + H). |
| 422 | 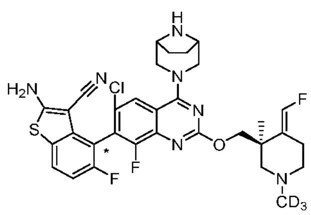 | (*Sa*) or (*Ra*)-4-(4-((1*R*,5*S*)-3,8-diazabicyclo[3.2.1]octan-3-yl)-6-chloro-8-fluoro-2-(((*S*,*E*)-4-(fluoromethylene)-3-methyl-1-(methyl-$d_3$)piperidin-3-yl)methoxy)quinazolin-7-yl)-2-amino-5-fluorobenzo[*b*]thiophen-3-carbonitrile | LCMS (m/z): 657.2 (M + H). |

(continued)

| Ex. | Structure | Name | Characterization data |
|---|---|---|---|
| 423 | 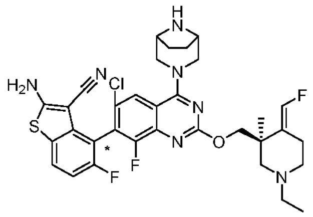 | (*Sa*) or (*Ra*)-4-(4-((1*R*,5*S*)-3,8-diazabicyclo[3.2.1]octan-3-yl)-6-chloro-2-(((*S,E*)-1-ethyl-4-(fluoromethylene)-3-methylpiperidin-3-yl)methoxy)-8-fluoroquinazolin-7-yl)-2-amino-5-fluorobenzo[b]thiophen-3-carbonitrile | LCMS (m/z): 668.2 (M + H). |
| 424 | 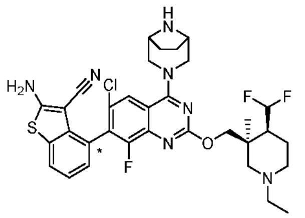 | (*Sa*) or (*Ra*)-4-(4-((1*R*,5*S*)-3,8-diazabicyclo[3.2.1]octan-3-yl)-6-chloro-2-(((3S, 4S)-4-difluoromethyl-1-ethyl-3-methylpiperidin-3-yl)methoxy)-8-fluoroquinazolin-7-yl)-2-aminobenzo[b]thiophen-3-carbonitrile | LCMS (m/z): 670.2 (M + H). |
| 425 | 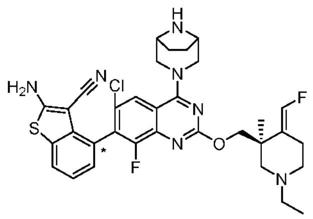 | (*Sa*) or (*Ra*)-4-(4-((1*R*,5*S*)-3,8-diazabicyclo[3.2.1]octan-3-yl)-6-chloro-2-(((*S,E*)-1-ethyl-4-(fluoromethylene)-3-methylpiperidin-3-yl)methoxy)-8-fluoroquinazolin-7-yl)-2-aminobenzo[b]thiophen-3-carbonitrile | LCMS (m/z): 650.2 (M + H). |
| 426 | 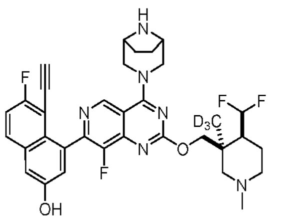 | (*Sa*) or (*Ra*)-4-(4-((1*R*,5*S*)-3,8-diazabicyclo[3.2.1]octan-3-yl)-2-(((3*S*,4*S*)-4-(difluoromethyl)-1-methyl-3-(methyl-$d_3$)piperidin-3-yl)methoxy)-8-fluoropyrido[4,3-d]pyrimidin-7-yl)-5-ethynyl-6-fluoronaphthalen-2-ol | LCMS (m/z): 638.3 (M + H). |

Compounds listed in the following table can be prepared by referring to the above synthesis methods or appropriate variants:

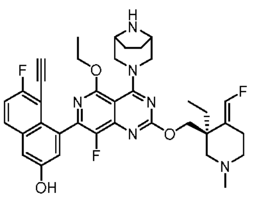 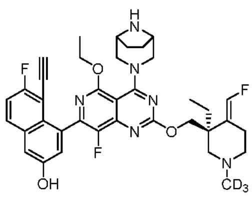 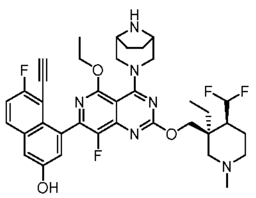 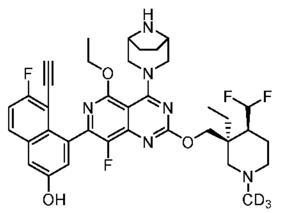

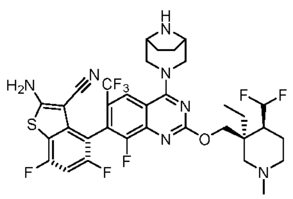 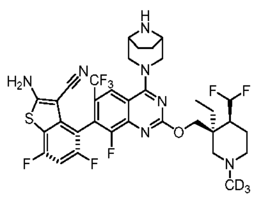 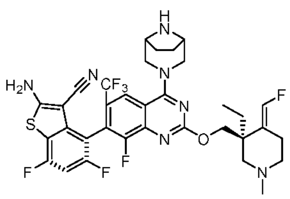 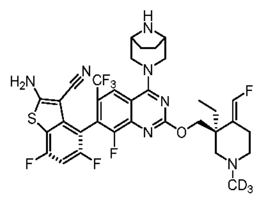

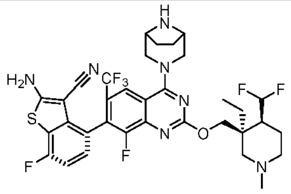 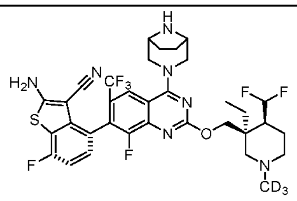 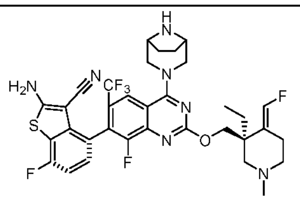 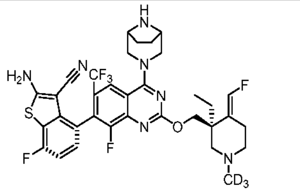

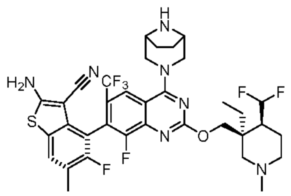 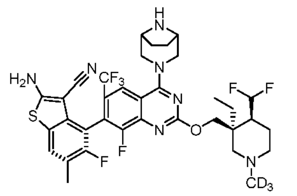 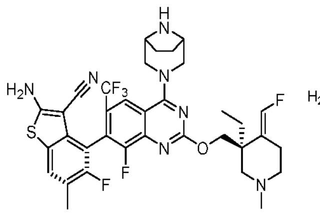 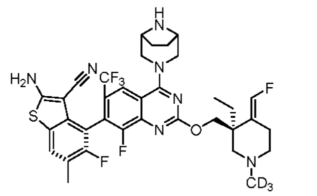

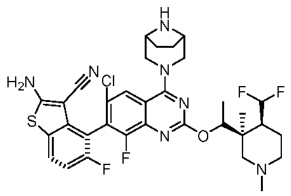 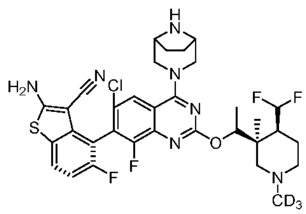 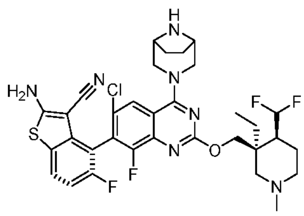 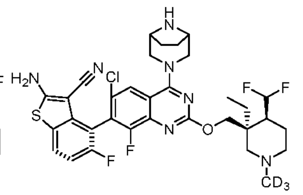

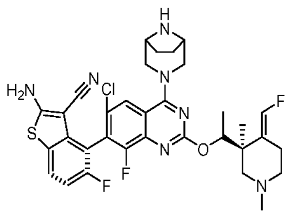 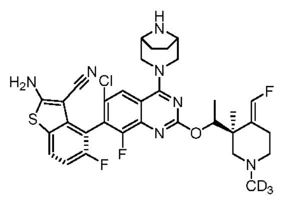 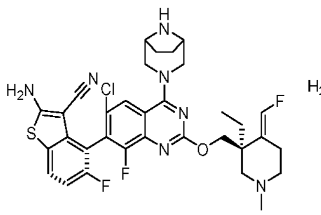 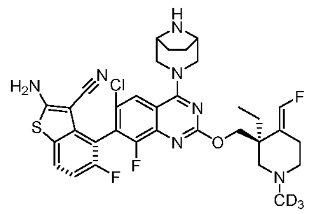

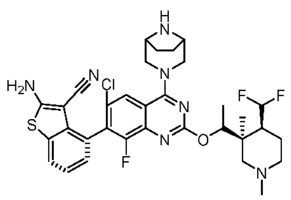 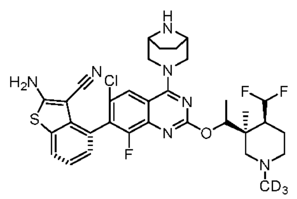 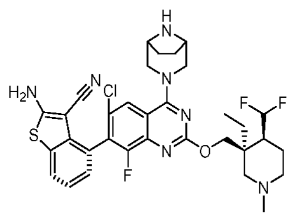 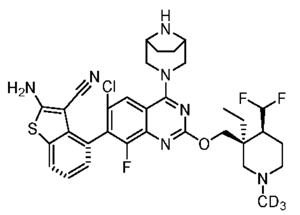

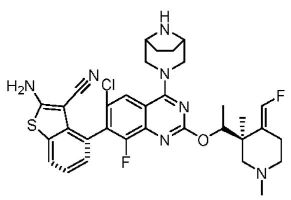 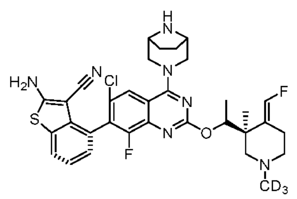 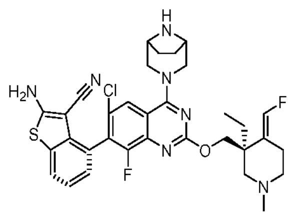 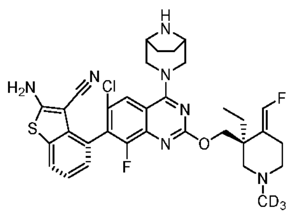

## Activity Examples

### Example 1: proliferation inhibitory effect of the compounds of the present invention on AGS Cells with KRAS G12D mutation

This experiment assessed and validated the proliferation inhibitory activity of some compounds of the present invention against KRAS G12D mutant AGS cells.

| Cell lines | Cell type | Number of cells/well | Culture medium |
|---|---|---|---|
| AGS | Adherent | 3000 | RPMI-1640 + 10% FBS |
| Incubate at 37 °C, 5% $CO_2$, 95% humidity. | | | |

The following materials, instruments, and reagents were used in this experiment: Fetal Bovine Serum FBS (GIBCO, Cat#10099-141); CellTiter-Glo® Luminescent Cell Viability Assay (Promega, Cat#G7572); 96-well clear flat-bottom black-wall plate (Corning®, Cat#3603); SpectraMax multimode microplate reader (MD, 2104-0010A); $CO_2$ Incubator (Thermo Scientific, Model 3100 Series); Biosafety cabinet (Thermo Scientific, Model 1300 Series A2); Inverted microscope (Olympus, CKX41SF); AGS cell line was purchased from Nanjing Kebai Biotechnology Co., Ltd., under the lot number CBP60476 .

The experiment was carried out as follows:

Cell culture and inoculation: The cells in the logarithmic growth phase were harvested, and cell counting was performed using a platelet counter. The cell viability was detected by Trypan Blue exclusion method to ensure that the cell viability was above 90%. The cell concentration was adjusted. Then, 90 μL of the cell suspension was added to each well of a 96-well plate respectively, and the cells in the 96-well plate were cultured at 37 °C and 5% $CO_2$.

Drug dilution and addition: a) For the preparation of drugs for single-point inhibition rate determination: A 10-fold concentrated drug solution was prepared. 10 μL of the drug solution was added to each well of the 96-well plate inoculated with cells to make the working concentration 1 μM, and three replicate wells were set for each drug concentration; b) For the preparation of drugs for $IC_{50}$ determination: A 10-fold concentrated drug solution was prepared. 10 μL of the drug solution was added to each well of the 96-well plate inoculated with cells to make the maximum working concentration 10 μM, with a 3.16 × dilution and a total of 9 concentrations. Three replicate wells were set for each drug concentration. The cells in the 96-well plate with the added drugs were incubated at 37 °C and 5% $CO_2$ for another 3 days, and then the CTG assay was performed.

The CTG reagent was thawed, and the cell plate was equilibrated to room temperature for 30 minutes. An equal volume of CTG solution was added to each well, and the plate was shaken on an orbital shaker for 5 minutes to lyse the cells. The cell plate was placed at room temperature for 20 minutes to stabilize the luminescence signal, and then the luminescence value was read.

The data were analyzed using GraphPad Prism 7.0 software. The data were fitted by nonlinear S-curve regression to obtain the dose-response curve, and the $IC_{50}$ value was calculated accordingly.

$$\text{Cell survival rate (\%)} = (\text{Lum}_{\text{Test drug}} - \text{Lum}_{\text{medium control}})/(\text{Lum}_{\text{cell control}} - \text{Lum}_{\text{medium control}}) \times 100\%.$$

The representative compounds of the present invention showed satisfactory antiproliferative activity against KRAS G12D mutated AGS human gastric adenocarcinoma cell lines. Some of the activity data were shown in Table 1.

| Compound | $IC_{50}$ (μM) | Compound | $IC_{50}$ (μM) |
|---|---|---|---|
| Example 1 | 0.094 | Example 8 | 0.251 |
| Example 2 | 0.050 | Example 9 | 0.347 |
| Example 3 | 0.158 | Example 10-4 | 0.020 |
| Example 4 | 0.088 | Example 10-2 | 0.034 |
| Example 5 | 0.175 | Example 39-1 | 0.127 |
| Example 5-4 | 0.065 | Example 39-2 | 0.022 |
| Example 5-3 | 0.035 | Example 42 | 0.150 |
| Example 6 | 0.055 | Example 43 | 0.037 |
| Example 6-2 | 0.032 | Example 44 | 0.217 |
| Example 7 | 0.781 | | |

In another experiment, using a protocol similar to the one described above, the inhibitory activity of some representative compounds of the present invention against KRAS G12D mutated AGS human gastric adenocarcinoma cells was determined. These compounds exhibited satisfactory antiproliferative activity. Some of the activity data are

shown in the following table.

| Compound | $IC_{50}$ (μM) |
|---|---|
| Example 29-1A | 0.630 |
| Example 40 | 0.009 |
| Example 45-3 | 0.005 |
| Example 46-3 | 0.007 |
| Example 49 | 0.386 |
| Example 50-1 | 0.015 |
| Example 50-3 | 0.007 |
| Example 52-1 | 0.010 |
| Example 63 | 0.011 |
| Example 67-1 | 0.077 |
| Example 67-3 | 0.017 |
| Example 71-2 | 0.008 |
| Example 77 | 0.004 |
| Example 79 | 0.004 |
| Example 85-2 | 0.038 |
| Example 88 | 0.017 |
| Example 95 | 0.008 |
| Example 99-1 | 0.018 |
| Example 103 | 0.004 |
| Example 105 | 0.006 |
| Example 122-1 | 0.034 |
| Example 190 | 0.047 |
| Example 194 | 0.097 |
| Example 258 | 0.008 |
| Example 260 | 0.003 |
| Example 261 | 0.002 |
| Example 262 | 0.015 |
| Example 263 | 0.012 |
| Example 265 | 0.002 |
| Example 304 | 0.002 |
| Example 306 | 0.005 |
| Example 312 | 0.025 |
| Example 312-1 | 0.011 |
| Example 313-2 | 0.017 |
| Example 315-2 | 0.013 |
| Example 318 | 0.028 |
| Example 318-1 | 0.009 |
| Example 324-1 | 0.004 |
| Example 325-1 | 0.002 |

(continued)

| Compound | $IC_{50}$ (μM) |
|---|---|
| Example 328 | 0.045 |
| Example 332 | 0.001 |
| Example 362-1 | 0.005 |
| Example 363-1 | 0.004 |
| Example 396 | 0.011 |
| Example 426 | 0.007 |
| MRTX1133 | 0.028 |

The synthesis of compound MRTX1133 was performed according to the method described in J. Med. Chem. 2022, 65, 4,3123 - 3133.

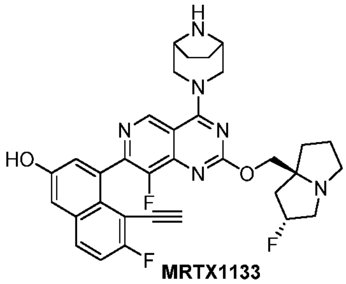

**MRTX1133**

**Example 2-1: Pharmacokinetic properties of the compounds of the present invention in mouse cassette dosing (cassette)**

The pharmacokinetic characteristics of some compounds of the present invention were evaluated through a mouse Cassette pharmacokinetic experiment (Nagilla R. et al., J. Pharm. Sci. 2011, 100, 3862-3874.).

Male CD-1 mice used in this study, 6-8 weeks old and weighed 18-22 g, were purchased from JOINN Laboratories (Suzhou). The following reagents were used: Tolbutamide (Aladdin, Cat# H1401054); MC (Methylcellulose, Aladdin, Cat#M112866); DMSO (Sigma-Aldrich, Cat# 186403); Acetonitrile (Sigma-Aldrich, Cat#WXBD547V; Methanol (Sigma-Aldrich, Cat# F22M67201).

The compound combination was formulated into a solvent of 5% DMSO + 95% (0.5% MC), and the final concentration of each compound was 1 mg/mL. The drug preparation was administered to CD-1 mice by gavage at a volume of 10 mL/kg. Blood samples were collected from the saphenous vein of the hind limb at 15 min, 30 min, 1 h, 2 h, 4 h, 6 h, 8 h, and 24 h, respectively. The samples were centrifuged at a low temperature for 20 minutes, and the plasma was collected and stored at - 20 °C for testing.

LC-MS/MS analytical methods for the compounds were established as follows:

Preparation of standard curve: 20 μL of the 1 mg/mL DMSO stock solution of each compound was pipetted and transferred to 900 μL of 50% methanol working solution and then diluted step by step to obtain a standard curve working solution with concentrations of 20000, 10000, 5000, 1000, 500, 100, 50, 20, and 10 ng/mL. Then, 2 μL of the standard curve working solution was pipetted and mixed with 18 μL of mouse blank plasma to obtain a standard curve with concentrations of 2000, 1000, 500, 100, 50, 10, 5, 2, and 1 ng/mL, which was used for quantifying unknown samples.

Sample pretreatment: For 20 μL of the unknown plasma sample and the standard curve sample, 250 μL of acetonitrile containing tolbutamide as an internal standard was added as a precipitating agent to precipitate plasma proteins and extract the compounds to be tested in the plasma. After centrifugation at a low temperature for 20 minutes, the supernatant was taken. The supernatant was mixed with an aqueous solution of 0.1% formic acid at a ratio of 1:1, and 10 μL of the mixture was injected for analyzing the drug blood concentration by LC-MS.

The standard curve was plotted using the mass spectrometry analysis software Analyst 1.6.1 to quantify the unknown samples. The pharmacokinetic parameters were calculated using Winnonlin 8.2 according to the drug concentrations at different time points of the unknown samples.

The experimental results showed that in the pharmacokinetic evaluation of cassette dosing, the compounds of the present invention exhibited good pharmacokinetic properties.

**Example 2-2: Pharmacokinetic properties of the compounds of the present invention in mice**

The pharmacokinetic properties of the compounds of the present invention were evaluated through a mouse pharmacokinetic experiment.

Male CD-1 mice, aged 6-8 weeks old, were purchased from Zhejiang Vital River Laboratory Animal Co., Ltd.

IV administration group: The compound was formulated into a sodium acetate buffer solution (10 mM sodium acetate solution, adjusted to pH 4.0 with acetic acid) containing 20% Captisol (sulfobutyl β-cyclodextrin) so that the final concentration of each compound was 0.6 mg/mL. The drug formulation was injected into the tail vein of CD-1 mice at an injection volume of 5 mL/kg. After administration, blood samples were collected from the submandibular vein or by other appropriate methods at 5 min, 15 min, 30 min, 1 h, 2 h, 4 h, 6 h, 8 h, and 24 h. The blood samples were centrifuged at a low temperature for 6 minutes, and the plasma was collected and stored at - 80 °C for testing. The PO administration group formulated the compound into a solvent of 0.5% Tween 80 + 99.5% (0.5% MC (400 cp)) or 5% DMSO + 95% (0.5% MC (400 cp)) so that the final concentration of each compound was 1 mg/mL. The drug formulation was administered to CD-1 mice by gavage at a volume of 10 mL/kg. After administration, blood samples were collected from the submandibular vein or by other appropriate methods at 15 min, 30 min, 1 h, 2 h, 4 h, 6 h, 8 h, and 24 h. The blood samples were centrifuged at a low temperature for 6 minutes, and the plasma was collected and stored at -80 °C for testing.

10 μL of the plasma sample was taken, and 200 μL of methanol containing an internal standard was added as a precipitating agent to precipitate plasma proteins and extract the compounds to be tested in the plasma. After centrifugation at a low temperature, 10 μL of the supernatant was injected, and the drug blood concentration was analyzed by LC-MS/MS. The pharmacokinetic parameters were calculated using Winnonlin based on the drug concentration data at different time points.

The experimental results showed that in the pharmacokinetic evaluation in mice, the compounds of the present invention, such as the compounds in the examples, exhibited good pharmacokinetic properties.

| Example | Route of administration | $T_{1/2}$ (hr) | $AUC_{0-t}$ (ng.hr/mL) | $AUC_{0-\infty}$ (ng.hr/mL) | Formulation |
|---|---|---|---|---|---|
| 46-3 | PO | 10.75 | 384.10 | 413.82 | 5% DMSO + 95% (0.5% MC (400cp)) |
| 103 | PO | 8.15 | 652.58 | 703.82 | 5% DMSO + 95% (0.5% MC (400cp)) |
| 304 | PO | 8.74 | 1423.56 | 1498.90 | 0.5% Tween 80 + 99.5% (0.5% MC (400cp)) |
| 306 | PO | 7.17 | 758.85 | 801.16 | 5% DMSO + 95% (0.5% MC (400cp)) |

**Example 3: The inhibition assay of the compounds of the present invention on cytochrome P450 enzyme system**

This experiment evaluated the inhibitory effect of the compounds of the present invention on the cytochrome P450 enzyme system.

The following reagents were used in this experiment: human liver microsomes (HLMs) (Corning, Cat#452161); Reduced nicotinamide adenine dinucleotide phosphate (NADPH), (MCE, Cat#HY-F0003/CS-4998); Phenacetin, Diclofenac, α-Naphthoflavone, Omeprazole, and Ketoconazole were purchased from TCI; S-Mephenytoin and Testosterone were purchased from CAYMAN; Midazolam was purchased from Bioreclamation IVT; Quinidine was purchased from Damas-beta; Sulfaphenazole was purchased from MCE; Bufuralol was purchased from TRC.

The experiment was carried out as follows:

A 100 mM potassium phosphate buffer (K-buffer) was prepared using potassium dihydrogen phosphate and dipotassium hydrogen phosphate, and the pH was adjusted to 7.4 to obtain a 0.1 M potassium phosphate buffer (K-buffer). 8 μL of the 10 mM test compound stock solution was dissolved in 12 μL of acetonitrile to prepare a 400× test compound. 12 μL of 1 mM α-Naphthoflavone, 10 μL of 40 mM Sulfaphenazole, 10 μL of 10 mM Quinidine and 8 μL of DMSO solution were mixed to prepare a mixed solution of CYP1A2, CYP2C9 and CYP2D6 inhibitors. 8 μL of the 2.5 mM ketoconazole DMSO solution and 100 mM omeprazole DMSO solution were respectively dissolved in 12 μL of acetonitrile to prepare inhibitor solutions for CYP3A4 and CYP2C19.

66.7 mg of NADPH was added to 10 mL of 0.1 M K-buffer, pH 7.4 to prepare a 4× NADPH potassium phosphate solution. Different substrates were prepared into solutions required for 4-fold concentration determination with 10 mL of 0.1 M K-buffer according to the concentration requirements to obtain a 4× substrate potassium phosphate solution. 10 μL of 20 mg/mL human liver microsomes was added to 990 μL of K-buffer to prepare a 0.2 mg/mL human liver microsomes (HLM) solution, which was stored on ice for later use.

600 μL of the 0.2 mg/mL HLM was added to a 96-well plate, and 3 μL of the 400-fold test compound solution was

added; 200 μL of the 0.2 mg/mL HLM was added to a 96-well plate, and 1 μL of the diluted positive control inhibitor solution was added. 30 μL of the mixed solution of the compound and human liver microsomes was dispensed into a 96-well plate, and then 15 μL of the substrate solution was added. The above-obtained solution and the prepared NADPH solution were preheated at 37 °C for 5 min. 15 μL of the preheated NADPH solution was added to the reaction plate, mixed, and the reaction was started. The reaction plate was incubated at 37 °C. The 3A4 reaction lasted for 5 minutes; the 1A2, 2C9 and 2D6 reactions lasted for 10 minutes; the 2C19 reaction lasted for 45 minutes. At the end of the reaction, 120 μL of acetonitrile containing an internal standard was added to terminate the reaction. The sample was vortexed for 10 min and centrifuged at 5594 g for 15 minutes, and the prepared sample was sent for LC-MS/MS analysis.

The experimental results showed that at the tested concentrations, the compounds of the present invention had no significant inhibitory effect on the key CYP subtypes related to drug metabolism, indicating better safety in drug-drug interactions.

### Example 4: In vivo pharmacodynamic study of the compounds of the present invention on a subcutaneous xenograft tumor model of human pancreatic cancer AsPC-1 cells in BALB/c nude mice

The following materials were used in this experiment: BALB/c nude mice, 6-8 weeks old, female, Shanghai Lingchang Biotechnology Co., Ltd.; AsPC-1 cells (carrying KRas G12D mutant protein, ATCC, Cat#CRL-1682); Captisol (Wuhan Fengyao Tonghui Chemical Products Co., Ltd., Cas#182410-00-0); PBS (Hyclone, Cat#SH30256.01)

The experiment was carried out as follows: Female BALB/c nude mice aged 6-8 weeks were subcutaneously inoculated with $5\times10^6$ human pancreatic cancer AsPC-1 cells on the right shoulder, with an inoculation volume of 0.1 mL. When the tumors grew to an average volume of 150 $mm^3$, the mice were randomly grouped for administration according to the tumor size and body weight. The day of administration was regarded as day 0. The administration was carried out once a day. The test compound was administered by tail vein injection or gavage, with 5 mice in each group at a dose of 10 mg/kg. The control group was administered with an equal volume of the solvent control by tail vein injection or gavage.

During the experiment, the tumor volume and body weight were measured twice a week. The formula for calculating the tumor volume was: Tumor volume ($mm^3$) = 0.5 × (tumor major axis × tumor minor axis$^2$). The average tumor inhibition rate TGI% =[(average tumor volume of the control group - average initial tumor volume of the control group) - (average tumor volume of the administration group - average initial tumor volume of the administration group)]/ (average tumor volume of the control group - average initial tumor volume of the control group).

The experimental results showed that the representative compounds of the present invention exhibited excellent target-related tumor inhibitory activity in the human pancreatic cancer AsPC-1 model. At the same time, there was no significant change in the body weight of the mice in each group.

### Example 5: Proliferation inhibitory effect of the compound of the present invention against KRAS G12D mutated AGS (3D) cells

The inhibitory effect of representative compounds on the 3D proliferation of the AGS cell line was evaluated by non-adherent, spherical growth in a methylcellulose 3D culture medium.

The following materials, instruments and reagents were used in this experiment: Culture medium RPMI1640 (HyClone, Cat#SH30809.01); Fetal bovine serum FBS (GBICO, Cat#10099-141); CellTiter-Glo® Luminescent Cell Viability Assay (Novozymes Nanjing, Cat#DD1101-04); 96-well Polystyrene microplates (black-wall) (Greiner Bio one, Cat.# 655096); Methylcellulose (SIGMA, CAS: 9004-67-5); AGS cells (KC-0405, Kangyuan Bochuang Biotechnology (Beijing) Co., Ltd.); Multifunctional microplate reader (BMG LABTECH, CLARIOstar® Plus); $CO_2$ incubator (Thermo Scientific, Model 3100 Series).

Cell Culture and Inoculation: The cells in the logarithmic growth phase were harvested and cell counting was performed using a platelet counter. The cell viability was detected by Trypan Blue exclusion method to ensure that the cell viability was above 90%. The cell concentration was adjusted. After there were no visible air bubbles in the cell suspension, 180 μL of the cell suspension was added to each well of a 96-well plate respectively, so that the final concentration of methylcellulose was 0.65% and the number of cells was 2400 per well. The cells in the 96-well plate were cultured overnight at 37 °C, 5% $CO_2$ and 95% humidity.

The drug dilution and administration were carried out as follows: A 10-fold concentrated drug solution was prepared with the culture medium. 20 μL of the drug solution was added to each well of the 96-well plate inoculated with cells to make the maximum working concentration 10 μM, with a 3.16 × dilution, a total of 9 concentrations. Three replicate wells were set for each drug concentration and the DMSO content was 0.1%. The cells in the 96-well plate with the added drugs were continuously cultured at 37 °C, 5% $CO_2$ and 95% humidity for 120 h, and then a CTG analysis was performed.

The CTG reagent was thawed and the cell plate was equilibrated to room temperature for 30 minutes. An equal volume of CTG solution was added to each well, and the plate was shaken on an orbital shaker for 5 minutes to lyse the cells. The cell plate was placed at room temperature for 20 minutes to stabilize the luminescence signal, and then the

luminescence value was read.

The data were analyzed using GraphPad Prism 7.0 software. The data were fitted by nonlinear S-curve regression to obtain the dose-response curve, and the $IC_{50}$ value was calculated accordingly.

$$\text{Cell survival rate (\%)} = (\text{Lum}_{\text{Test drug}} - \text{Lum}_{\text{Medium control}})/(\text{Lum}_{\text{Cell control}} - \text{Lum}_{\text{Medium control}}) \times 100\%.$$

The compounds of the present invention exhibited satisfactory proliferation inhibitory activity against KRas G12D mutant cells in the 3D-cultured AGS cell line, with $IC_{50}$ values of 0.01 ~ 0.5 μM, such as 0.01 ~ 0.1 μM, 0.01 ~ 0.05 μM. Specific data for representative compounds are presented in the table below.

| Compound | $IC_{50}$ (μM) |
|---|---|
| Example 5-3 | 0.016 |
| Example 6-2 | 0.017 |
| Example 10-4 | 0.006 |
| Example 10-2 | 0.017 |
| Example 39-1 | 0.088 |
| Example 39-2 | 0.023 |
| Example 42 | 0.047 |
| Example 43 | 0.011 |
| Example 44 | 0.118 |

**Example 7: In vivo pharmacodynamic study of the compounds of the present invention on a subcutaneous xenograft tumor NOD/SCID mouse model of human pancreatic cancer cells HPAC**

The following materials were used in this experiment: NOD/SCID mice, 6-8 weeks old, female, Jiangsu Ji Cai Yao Kang Biotechnology Co., Ltd.; HPAC cells (carrying KRas G12D mutant protein, ATCC); Captisol (Wuhan Fengyao Tonghui Chemical Products Co., Ltd., Cas#182410-00-0); Sodium acetate (aladdin); MC (sigma, 9004-67-5).

The experiment was carried out as follows: Female NOD/SCID mice aged 6-8 weeks were subcutaneously inoculated with $1\times10^7$ HPAC cells on the right posterior side of the back, with an inoculation volume of 0.1 mL. When the tumors grew to an average volume of 150 $mm^3$, the mice were randomly grouped for administration according to the tumor size and body weight. The day of administration was regarded as day 0. The administration was carried out once a day. The test compound was administered by tail vein injection or gavage, with 5 mice in each group at a dose of 10 mg/kg. The control group was administered with an equal volume of the solvent control by tail vein injection or gavage.

During the experiment, the tumor volume and body weight were measured twice a week. The formula for calculating the tumor volume was: Tumor volume ($mm^3$) = 0.5 × (tumor major axis × tumor minor axis$^2$). The average tumor inhibition rate TGI% = [(average tumor volume of the control group - average initial tumor volume of the control group) - (average tumor volume of the administration group - average initial tumor volume of the administration group)] / (average tumor volume of the control group - average initial tumor volume of the control group).

The experimental results showed that the representative compounds of the present invention exhibited excellent target-related tumor inhibitory activity in the human pancreatic cancer HPAC model. At the same time, there was no significant change in the body weight of the mice in each group.

| Group | Dose | Route of administration and frequency | Tumor volume (mm3) (Day 20) | TGI (%) |
|---|---|---|---|---|
| Solvent control * | / | PO, BID × 21 | 771 | / |
| Example 10-4 | 50mpk | PO, BID × 21 | 310 | 74 |
| Example 46-3 | 50mpk | PO, BID × 21 | 135 | 102 |
| Example 77 | 50mpk | PO, BID × 21 | 255 | 83 |
| Example 103 | 50mpk | PO, BID × 21 | 77 | 112 |
| Example 105 | 50mpk | PO, BID × 21 | 122 | 104 |

(continued)

| Group | Dose | Route of administration and frequency | Tumor volume (mm3) (Day 20) | TGI (%) |
|---|---|---|---|---|
| Example 261 | 50mpk | PO, BID × 21 | 117 | 105 |
| Example 265 | 50mpk | PO, BID × 21 | 113 | 106 |
| * The vehicle for gavage administration was 5% DMSO + 95% (0.5% MC (400 cp)). | | | | |

**Example 8: Antitumor effect of the compounds of the present invention on a subcutaneous xenograft BALB/c nude mouse animal model of KRAS-G12D mutated human colon carcinoma cells GP2d**

GP2d cells were purchased from Nanjing Kebai Biotechnology Co., Ltd. BALB/c nude mice, female, were purchased from Jiangsu GemPharmatech LLC. GP2d cells were cultured in culture medium containing DMEM and 10% FBS. The cells in the logarithmic growth phase were collected and resuspended in PBS to an appropriate concentration for subcutaneous tumor inoculation in mice. The experimental mice were subcutaneously inoculated on the right posterior side of the back. The inoculation dose of GP2d cells was $1 \times 10^7$ cells per mouse, and the cells were resuspended in a 1:1 mixture of PBS and Matrigel (the inoculation volume was 0.1 mL). When the average tumor volume grew to ~ 150 $mm^3$, the mice were randomly grouped for administration according to the tumor size and body weight. Administration was started immediately after grouping, and the day of administration was regarded as day 0. The administration was carried out twice a day by gavage, at a dose of 10 mg/kg or a solvent control (0.5% Tween 80 + 99.5% of (0.5% MC (400 cp))). During the experiment, the body weight of the mice and the size of the tumors were measured twice a week.

The formula for calculating the tumor size: Tumor volume ($mm^3$) = 0.5 × (long diameter of tumor × (short diameter of tumor)$^2$).

Relative tumor inhibition rate TGI (%): TGI% = [1-(average tumor volume at the end of administration of the treatment group - average tumor volume at the beginning of administration of the treatment group)/(average tumor volume at the end of administration of the vehicle control group - average tumor volume at the beginning of administration of the vehicle control group)] × 100%.

| Group | Tumor volume (mm3) (Day 21) | TGI (%) |
|---|---|---|
| Solvent control * | 1026 | / |
| Example 313-1 | 256 | 88 |
| Example 315-1 | 325 | 80 |
| Example 321-1 | 70 | 109 |
| Example 325-1 | 228 | 91 |
| Example 332 | 419 | 70 |

The experimental results showed that the representative compounds of the present invention exhibited excellent target-related tumor inhibitory activity in the human colorectal cancer cell GP2d model. Meanwhile, there was no significant change in the body weight of the mice in each group.

**Example 9: Proliferation inhibitory effect of the compounds of the present invention against five strains of KRAS G12D mutated tumor cells**

This experiment evaluated and verified the proliferation inhibitory activity of the representative compounds of the present invention against the following five strains of KRAS-related tumor cells.

| KRAS | Cell lines | Cell type | Complete medium |
|---|---|---|---|
| G12D | GP2D | Adherent | DMEM + 10% FBS |
| G12D | HPAC | Adherent | HPAC complete medium |

(continued)

| KRAS | Cell lines | Cell type | Complete medium |
|---|---|---|---|
| G12D | AsPC-1 | Adherent | RPMI-1640 + 10% FBS |
| G12 D | KP-4 | Adherent | RPMI-1640 + 10% FBS |
| G12 D | Panc 04.03 | Adherent | RPMI-1640 + 15% FBS + 10 ug/ml Insulin |

The following materials, reagents and instruments were used in this experiment: Five strains of cells were all from Nanjing Kebai Biotechnology Co., Ltd. (GP2D, CBP60010; HPAC, CBP60539; AsPC-1, CBP60546; KP-4, CBP60541; Panc 04.03, CBP60683); DMEM (Gibco, 11965092); RPMI-1640 (Gibco, SH30809.01); HPAC complete medium (Cobioer, CBP60539M); Fetal bovine serum FBS (Gibco, 10091-148); Insulin (Gibco, 41400045); Methylcellulose (methylcellulose, Sigma, 9004-67-5); Phosphate-buffered saline DPBS (Corning, 21-031-CVC); CellTiter-Glo® Luminescent Cell Viability Assay (Promega, Cat#G7573); 96-well clear flat-bottom black-wall plate (Corning, 3603); EnVision multilabel plate reader (PerkinElmer#2105); $CO_2$ incubator (Thermo Scientific, Model 3 71 Series).

Experimental methods:

A sterilized 1% methylcellulose 3D culture medium was prepared in advance. The cells in the logarithmic growth phase were harvested, and a Countstar automatic cell counter based on the principle of the classic trypan blue staining method was used for cell counting and detecting cell viability to ensure that the cell viability was above 90%. The cell concentrations of GP2D, HPAC, AsPC-1, KP-f4, and Panc 04.03 were adjusted so that the final concentration of methylcellulose was 0.65%, and the mixture was allowed to stand after being well-mixed. After there were no visible air bubbles in the cell suspension, 80 μL of the cell suspension was added to each well of a 96-well plate, with a total of 1000-2000 cells. The cells in the 96-well plate were cultured overnight at 37 °C, 5% $CO_2$, and 95% humidity.

Drug Preparation for $IC_{50}$ Determination: First, a 10 mM DMSO stock solution of each compound was prepared. For the first dilution, using DMSO as the solvent, each compound was first diluted to 5 mM and then serially diluted 3-fold to obtain 9 concentrations, preparing DMSO solutions of each compound at different concentrations. For the second dilution, a 5-fold diluted solution of each compound was prepared by a 1: 100 dilution, using the complete medium as the solvent. Finally, 20 μL of the diluted solution of each compound was added to each well of the 96-well plate inoculated with cells. The final maximum drug concentration was 10 μM, with 9 concentrations and a 3-fold dilution, and two replicate wells were set for each concentration.

The cells in the 96-well plate with the added drugs were further cultured at 37 °C and 5% $CO_2$ for 120 hours, and then a CTG analysis was performed. The CTG reagent was thawed, and the cell plate was equilibrated to room temperature for 30 minutes. 75 μL of the CTG solution was added to each well. The plate was shaken on an orbital shaker for 5 minutes to lyse the cells. The cell plate was placed at room temperature for 20 minutes to stabilize the luminescence signal. The luminescence value was read, and the data were collected.

The data were analyzed using GraphPad Prism 9.0 software. Non-linear S-curve regression was used to fit the data to obtain the dose-response curve, and the $IC_{50}$ value was calculated accordingly.

$$\text{Cell survival rate (\%)} = (\text{Lum}_{\text{Test drug}} - \text{Lum}_{\text{Medium control}})/(\text{Lum}_{\text{vehicle control}} - \text{Lum}_{\text{Medium control}}) \times 100\%$$

The representative compounds of the present invention exhibited satisfactory antiproliferative activity against the five types of KRAS G12D mutant cells in this example.

| Example | GP2D | HPAC | AsPC-1 | KP-4 | Panc 04.03 |
|---|---|---|---|---|---|
| 103 | 0.42 | 1.44 | 2.09 | 6.92 | 2.12 |
| 321-1 | 0.43 | 5.38 | 1.38 | 4.74 | 1.36 |
| 324-1 | 0.39 | 4.10 | N.D. | 4.74 | 1.34 |
| MRTX1133 | 3.00 | 8.19 | 18.3 | 42.58 | 20.59 |

The specific embodiments provided herein are only used for exemplifying the present invention and do not constitute a limitation to the scope defined by the claims. Based on the content disclosed in this application, those skilled in the art can obviously understand the equivalent variants of the technical solutions of this application, and these variants are

also covered within the scope of this application.

## Claims

1. A compound of formula (I), a stereoisomer, tautomer, stable isotopic variant, pharmaceutically acceptable salt or solvate thereof,

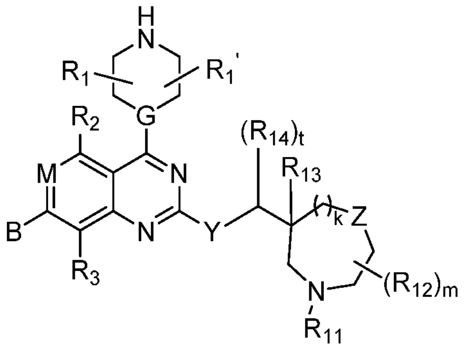

(I)

wherein,

$R_1$ and $R_1'$ together form an introcyclic bridging $-(CH_2)_t-$ or $CH_2=CH_2-$;
$R_2$ and $R_3$ are each independently selected from H, halogen, optionally halogen-substituted $-C_{1-6}$ alkyl, optionally halogen-substituted $-C_{2-6}$ alkynyl, and/or optionally halogen-substituted $-O-C_{1-6}$ alkyl;
G is selected from CH and N;
Y is selected from O, S and Se;
M is selected from N and $C-R_4$;
Z is selected from N, C, O, S and Se;
B is selected from

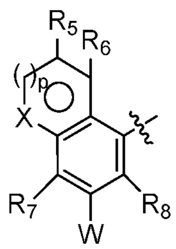

and

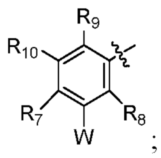

;

X is selected from C and S, p is selected from 0 and 1, with a proviso that p is 0 when of X is S, p is 1 when X is C;
W is selected from H, halogen, $-C_{1-6}$ alkyl, OH or $NH_2$;
$R_4$ is selected from H, halogen, CN, $-C_{1-6}$ alkyl or $-(CH_2)_n-C_{3-6}$ cycloalkyl, wherein the $-C_{1-6}$ alkyl and the-$C_{3-6}$ cycloalkyl are each optionally independently substituted by halogen or CN;
$R_5$ is selected from H, halogen and $NH_2$;
$R_6$ is selected from H, halogen, CN, $-C_{1-6}$ alkyl, $-O-C_{1-6}$ alkyl, $-S-C_{1-6}$ alkyl, $-Se-C_{1-6}$ alkyl or - $C_{2-6}$ alkynyl, wherein the $-C_{1-6}$ alkyl and the $-C_{2-6}$ alkynyl are each independently and optionally substituted by halogen;
$R_7$ and $R_8$ are each independently selected from H, halogen, $-NO_2$, CN, $-C_{1-6}$ alkyl, $-N(R^a)_2$, - $C(O)N(R^a)_2$ or $-C(O)OR^{a,}$, wherein the $-C_{1-6}$ alkyl is optionally substituted by halogen or $-N(R^a)_2$;
$R_9$ is selected from $-Si(R^b)_3$, CN, $NO_2$, $-C_{1-6}$ alkyl, $-O-C_{1-6}$ alkyl, $-S-C_{1-6}$ alkyl, $-C_{2-6}$ alkenyl, - $C_{2-6}$ alkynyl, $-(CH_2)_n-C_{3-6}$ cycloalkyl, $-(CH_2)_n$-5-6 membered heteroaryl or $-(CH_2)_n$-phenyl, wherein the $-C_{1-6}$ alkyl, the $-C_{2-6}$ alkenyl, the $-C_{3-6}$ cycloalkyl, the 5-6 membered heteroaryl and phenyl are each independently and optionally substituted by a group selected from halogen, $-Se-C_{1-6}$ alkyl and optionally halogen-substituted $-C_{1-6}$ alkyl;
$R^a$ is selected from H and optionally halogen-substituted $-C_{1-6}$ alkyl;
$R^b$ is selected from $-C_{1-6}$ alkyl and $-C_{2-6}$ alkenyl, each optionally substituted by halogen;
$R_{10}$ is selected from H, halogen, CN, $-C_{1-6}$ alkyl and $-(CH_2)_n-C_{3-6}$ cycloalkyl, wherein the $-C_{1-6}$ alkyl and the $-C_{3-6}$ cycloalkyl are each independently and optionally substituted by halogen or CN;

$R_{11}$ is selected from H, -$C_{1-6}$ alkyl, -$C_{2-6}$ alkenyl, -$C_{2-6}$ alkynyl and -$(CH_2)_n$-$C_{3-6}$ cycloalkyl, wherein the -$C_{1-6}$ alkyl, the-$C_{2-6}$ alkenyl, the-$C_{2-6}$ alkynyl or the $C_{3-6}$ cycloalkyl is each independently and optionally substituted by halogen, -CN, -O-$C_{1-6}$ alkyl or -O-CON$(R^a)_2$;

$R_{12}$ is selected from H, halogen, -CN, -OH, -N$(R^a)_2$, -O-$C_{1-6}$ alkyl, -O-$C_{3-6}$ cycloalkyl, -$C_{1-6}$ alkyl, -$C_{2-6}$ alkenyl, -$C_{2-6}$ alkynyl and -$(CH_2)_n$-$C_{3-6}$ cycloalkyl, wherein the-$C_{1-6}$ alkyl, -$C_{2-6}$ alkenyl, -$C_{2-6}$ alkynyl or $C_{3-6}$ cycloalkyl at each occurrence is each independently and optionally substituted by halogen, -CN, or -O-$C_{1-6}$ alkyl,

or two $R_{12}$ attached to the same carbon atom together form =C$(R^c)_2$, spiro $C_{3-6}$ cycloalkyl or spiro 4-7 membered-heterocycloalkyl, wherein $R^c$ is each independently selected from H, halogen or optionally halogen-substituted -$C_{1-6}$ alkyl, and the spiro $C_{3-6}$ cycloalkyl or spiro 4-7 membered-heterocycloalkyl is optionally substituted by halogen or optionally halogen-substituted -$C_{1-6}$ alkyl,

or two $R_{12}$ attached to the adjacent ring carbon atoms together with the carbon atoms to which they are attached form a $C_{3-4}$ cycloalkyl,

or two $R_{12}$ attached to non-adjacent ring carbon atoms together with the carbon atoms to which they are attached form a bridging methylene or ethylene;$R_{13}$ is selected from H, -$C_{1-6}$ alkyl, and - $(CH_2)_n$-$C_{3-6}$ cycloalkyl, wherein the -$C_{1-6}$ alkyl or -$C_{3-6}$ cycloalkyl is each independently and optionally substituted by halogen or -O-$C_{1-6}$ alkyl;

or when $R_{12}$ and $R_{13}$ are attached to adjacent ring carbon atoms, they together with the caron atoms to which they are attached form a -$C_{3-4}$ cycloalkyl;

$R_{14}$ is selected from H, -$C_{1-6}$ alkyl, and -$(CH_2)_n$-$C_{3-6}$ cycloalkyl, wherein the -$C_{1-6}$ alkyl or the-$C_{3-6}$ cycloalkyl is each independently and optionally substituted by halogen or -O-$C_{1-6}$ alkyl, or two $R_{14}$ attached to the same carbon atom together with the atom to which they are attached form a-$C_{3-4}$ cycloalkyl;

k is selected from 0 or 1;

m and n are each independently selected from an integer from 0 to 2; and

t is selected from an integer from 1 to 2.

**2.** The compound of formula (I), a stereoisomer, tautomer, stable isotopic variant, pharmaceutically acceptable salt or solvate thereof according to claim 1, wherein B is

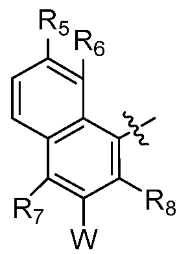

,

wherein $R_5$ is halogen, $R_6$ is selected from halogen, -$C_{1-6}$ alkyl or -$C_{2-6}$ alkynyl, $R_7$ and $R_8$ are each independently selected from H, and W is -OH.

**3.** The compound of formula (I), a stereoisomer, tautomer, stable isotopic variant, pharmaceutically acceptable salt or solvate thereof according to claim 1, wherein B is

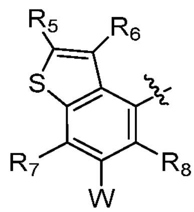

,

wherein $R_5$ is H or halogen, $R_6$ is selected from halogen, -$C_{2-6}$ alkynyl and -$C_{1-6}$ alkyl, $R_7$ and $R_8$ are each independently selected from H, halogen, CN and $NO_2$, and W is-OH; or $R_5$ is $NH_2$, $R_6$ is-CN, $R_7$ and $R_8$ are each independently selected from H or halogen, and W is H.

**4.** The compound of formula (I), a stereoisomer, tautomer, stable isotopic variant, pharmaceutically acceptable salt or solvate thereof according to claim 1, wherein B is

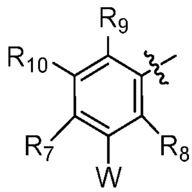

wherein $R_7$ is H; $R_8$ is selected from H, CN, halogen, $NO_2$, $-C_{1-6}$ alkyl, $-N(R^a)_2$, $-C(O)N(R^a)_2$ or $-C(O)OR^a$, wherein the $-C_{1-6}$ alkyl is optionally substituted by halogen or $-N(R^a)_2$, wherein $R^a$ is selected from H and optionally halogen-substituted $-C_{1-6}$ alkyl; $R_9$ is selected from $-Si(R^b)_3$, $NO_2$, CN, $-C_{1-6}$ alkyl, $-(CH_2)_n-C_{3-6}$ cycloalkyl, wherein the $-C_{1-6}$ alkyl and $-C_{3-6}$ cycloalkyl are each independently and optionally substituted by halogen, $-Se-C_{1-6}$ alkyl and optionally halogen-substituted $-C_{1-6}$ alkyl; $R_{10}$ is selected from halogen and $-C_{1-6}$ alkyl, and W is -OH or $-NH_2$.

**5.** The compound of formula (I), a stereoisomer, tautomer, stable isotopic variant, pharmaceutically acceptable salt or solvate thereof according to any one of claims 1-4, wherein G is N, and $R_1$ and $R_1'$ together form $-CH_2-$, $-CH_2CH_2-$, or $-CH_2=CH_2-$, preferably $-CH_2CH_2-$.

**6.** The compound of formula (I), a stereoisomer, tautomer, stable isotopic variant, pharmaceutically acceptable salt or solvate thereof according to any one of claims 1-5, wherein $R_2$ is H, and $R_3$ is halogen, preferably F.

**7.** The compound of formula (I), a stereoisomer, tautomer, stable isotopic variant, pharmaceutically acceptable salt or solvate thereof according to any one of claims 1-6, wherein M is N or C-$R_4$, and $R_4$ is selected from halogen, CN or halogen substituted-$C_{1-6}$ alkyl, preferably M is selected from N, C-F, C-Cl, C-CN and C-$CF_3$.

**8.** The compound of formula (I), a stereoisomer, tautomer, stable isotopic variant, pharmaceutically acceptable salt or solvate thereof according to any one of claims 1-7, wherein Y is O.

**9.** The compound of formula (I), a stereoisomer, tautomer, stable isotopic variant, pharmaceutically acceptable salt or solvate thereof according to any one of claims 1-8, wherein $R_{14}$ is each independently H or D.

**10.** The compound of formula (I), a stereoisomer, tautomer, stable isotopic variant, pharmaceutically acceptable salt or solvate thereof according to any one of claims 1-9, wherein the fragment

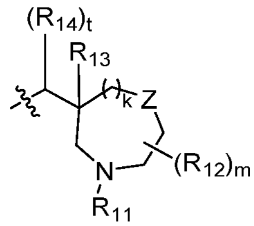

is

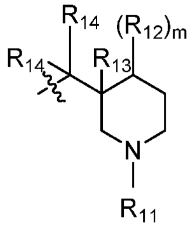

preferably

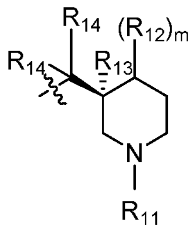

,

wherein $R_{11}$ is $-C_{1-6}$ alkyl optionally substituted by $-O-C_{1-6}$ alkyl or halogen or D, preferably $-C_{1-3}$ alkyl optionally substituted by one or more D; and/or $R_{12}$ is selected from halogen, CN, optionally halogen-substituted $-C_{1-6}$ alkyl, optionally halogen-substituted $-C_{2-6}$ alkynyl and optionally halogen-substituted $-O-C_{1-6}$ alkyl, or two $R_{12}$ attached to the same carbon atom form $=C(R^c)_2$ or spiro-$C_{3-6}$ cycloalkyl, wherein $R^c$ is each independently selected from H, halogen and optionally halogen-substituted $-C_{1-6}$ alkyl, and m is selected from 1 or 2; and/or $R_{14}$ is each independently selected from H or D; and/or $R_{13}$ is $-C_{1-6}$ alkyl, preferably $-C_{1-3}$ alkyl.

**11.** The compound of formula (I), a stereoisomer, tautomer, stable isotopic variant, pharmaceutically acceptable salt or solvate thereof according to claim 1, having the sub-formula below:

| | |
|---|---|
| 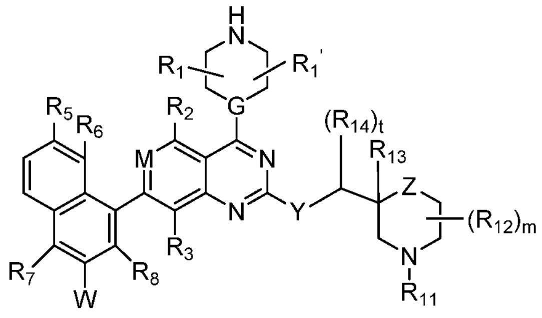 (I-A-1) | 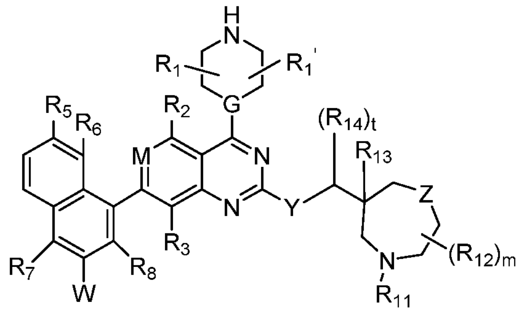 (I-A-2) |
| 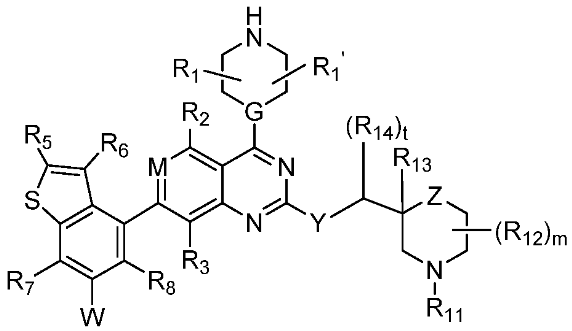 (I-A-3) | 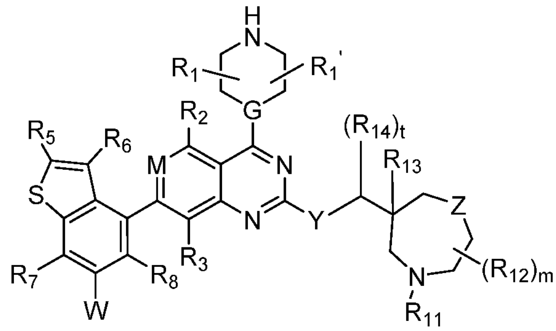 (I-A-4) |

wherein:

Yis O;
G is CH or N;
M is $C-R_4$;
Z is selected from C, Se, and O;
W is -OH or $-NH_2$;
$R_1$ and $R_1'$ together form $-(CH_2)_t-$;
$R_2$ is H;
$R_3$ is halogen;
$R_4$ is halogen;
$R_5$ is selected from H and halogen;
$R_5$ is selected from halogen, $-C_{1-6}$ alkyl, and-$C_{2-6}$ alkynyl;
$R_7$ and $R_8$ are each independently selected from H, halogen, CN and $NO_2$;

$R_{11}$ is-$C_{1-6}$ alkyl optionally substituted by -O-$C_{1-6}$ alkyl or halogen;

$R_{12}$ is selected from H, halogen, CN, optionally halogen-substituted -$C_{1-6}$ alkyl, optionally halogen-substituted -$C_{2-6}$ alkynyl, optionally halogen-substituted -O-$C_{1-6}$ alkyl, or two $R_{12}$ attached to the same carbon atom form =$C(R^c)_2$, wherein $R^c$ is each independently selected from H, halogen and optionally halogen-substituted -$C_{1-6}$ alkyl;

$R_{13}$ is selected from H, halogen, and optionally halogen-substituted -$C_{1-6}$ alkyl;

$R_{14}$ is H;

m and n are each independently selected from an integer from 0 to 2; and

t is selected from 1 or 2.

**12.** The compound of formula (I), a stereoisomer, tautomer, stable isotopic variant, pharmaceutically acceptable salt or solvate thereof according to claim 11, wherein $R_{11}$ is -$C_{1-6}$ alkyl substituted by one or more hydrogen isotopes, preferably -$CD_3$, and/or $R_{14}$ is D.

**13.** The compound of formula (I), a stereoisomer, tautomer, stable isotopic variant, pharmaceutically acceptable salt or solvate thereof according to claim 1, which is:

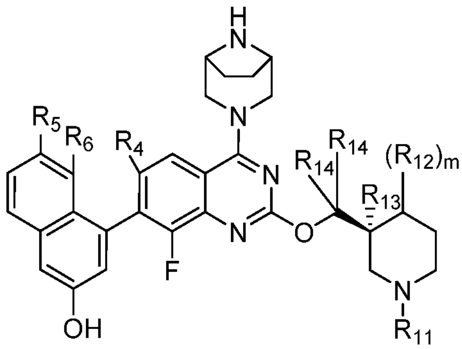
(I-A-1'') or
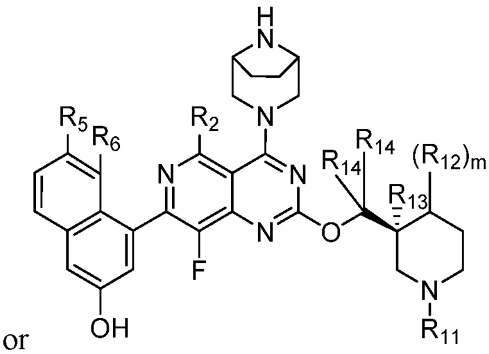
(I-A-1'''')

wherein $R_2$ is selected from H, -$C_{2-6}$ alkynyl, optionally halogen or D-substituted -$C_{1-6}$ alkyl, or optionally halogen or D-substituted -O-$C_{1-6}$ alkyl; $R_5$ is halogen, preferably F; and/or $R_6$ is-$C_{2-6}$ alkynyl, preferably ethynyl; and/or $R_4$ is halogen, preferably F; and/or $R_{14}$ is each independently selected from H and D; and/or $R_{13}$ is -$C_{1-6}$ alkyl, preferably -$C_{1-3}$ alkyl; and/or $R_{11}$ is -$C_{1-6}$ alkyl optionally substituted by -O-$C_{1-6}$ alkyl or halogen or D, preferably -$C_{1-3}$ alkyl optionally substituted by one or more D; and/or $R_{12}$ is selected from halogen, CN, optionally halogen-substituted -$C_{1-6}$ alkyl, optionally halogen-substituted -$C_{2-6}$ alkynyl, and optionally halogen-substituted -O-$C_{1-6}$ alkyl, or two $R_{12}$ attached to the same carbon atom together form =$C(R^c)_2$ or spiro $C_{3-6}$ cycloalkyl, wherein $R^c$ is each independently selected from H, halogen and optionally halogen-substituted -$C_{1-6}$ alkyl, and m is selected from 1 or 2.

**14.** The compound of formula (I), a stereoisomer, tautomer, stable isotopic variant, pharmaceutically acceptable salt or solvate thereof according to claim 1, which is:

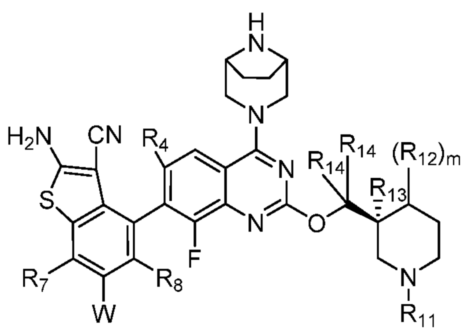
(I-A-3'') or
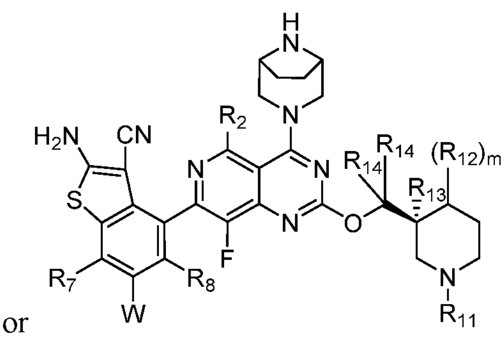
(I-A-3'''')

wherein $R_2$ is selected from H, -$C_{2-6}$ alkynyl, optionally halogen or D-substituted -$C_{1-6}$ alkyl, or optionally halogen or D-substituted -O-$C_{1-6}$ alkyl; and/or $R_4$ is selected from halogen (preferably F or Cl), CN or optionally halogen (preferably F) substituted -$C_{1-6}$ alkyl; and/or $R_7$ and $R_8$ both are H, or both are halogen (preferably F), or one of them is an H and the other is halogen (preferably F), or one of them is an H and the other is halogen (preferably F) substituted $C_{1-6}$ alkyl;

and/or $R_{11}$ is $-C_{1-6}$ alkyl optionally substituted by $-O-C_{1-6}$ alkyl or halogen or D, preferably $-C_{1-3}$ alkyl optionally substituted by one or more D; and/or $R_{12}$ is selected from halogen, CN, optionally halogen-substituted $-C_{1-6}$ alkyl, optionally halogen-substituted $-C_{2-6}$ alkynyl or optionally halogen-substituted $-O-C_{1-6}$ alkyl, or two $R_{12}$ attached to the same carbon atom together form $=C(R^c)_2$ or spiro $C_{3-6}$ cycloalkyl, wherein $R^c$ is each independently selected from H, halogen and optionally halogen-substituted $-C_{1-6}$ alkyl, and/or $R_{13}$ is $-C_{1-6}$ alkyl, preferably $-C_{1-3}$ alkyl; and/or $R_{14}$ is each independently selected from H or D; and/or W is H; and m is selected from 1 or 2.

**15.** The compound of formula (I), a stereoisomer, tautomer, stable isotopic variant, pharmaceutically acceptable salt or solvate thereof according to claim 1, having the sub-formula below:

| | |
|---|---|
| 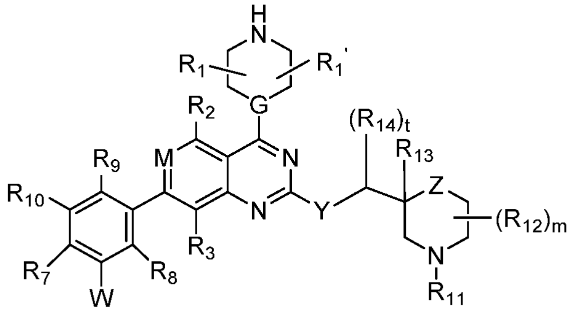 (I-B-1) | 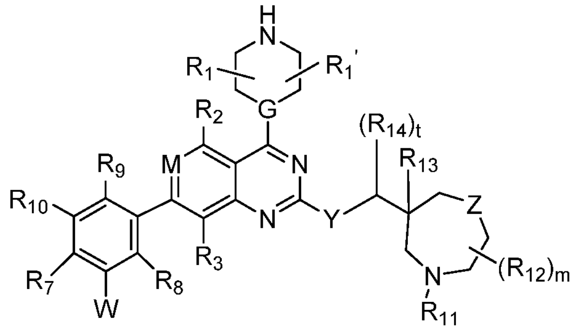 (I-B-2) |

Wherein:

Y is O;
G is CH or N;
M is $C-R_4$;
Z is selected from C, Se, or O;
W is-OH or $-NH_2$;
$R_1$ and $R_1'$ together form $-(CH_2)_t-$;
$R_2$ is H;
$R_3$ is halogen;
$R_4$ is halogen;
$R_7$ is H;
$R_8$ is selected from H, CN, halogen, $NO_2$, $-C_{1-6}$ alkyl, $-N(R^a)_2$, $-C(O)N(R^a)_2$ and $-C(O)OR^a$, wherein the $-C_{1-6}$ alkyl is optionally substituted by halogen or $-N(R^a)_2$, wherein $R^a$ is selected from H and optionally halogen-substituted $-C_{1-6}$ alkyl;
$R_9$ is selected from $-Si(R^b)_3$, $-C_{1-6}$ alkyl or $-(CH_2)_n-C_{3-6}$ cycloalkyl, wherein the $-C_{1-6}$ alkyl and the - $C_{3-6}$ cycloalkyl are each independently and optionally substituted by halogen, $-Se-C_{1-6}$ alkyl or optionally halogen-substituted $-C_{1-6}$ alkyl, and $R_b$ is selected from $-C_{1-6}$ alkyl or $-C_{2-6}$ alkenyl, each optionally substituted by halogen;
$R_{10}$ is selected from halogen and $-C_{1-6}$ alkyl;
$R_{11}$ is selected from $-C_{1-6}$ alkyl optionally substituted by $-O-C_{1-6}$ alkyl and halogen;
$R_{12}$ is selected from H, halogen, CN, optionally halogen-substituted $-C_{1-6}$ alkyl, optionally halogen-substituted $-C_{2-6}$ alkynyl, and optionally halogen-substituted $-O-C_{1-6}$ alkyl, or two $R_{12}$ attached to the same carbon atom together form $=C(R^c)_2$, wherein $R^c$ is independently selected from H, halogen, and optionally halogen-substituted $-C_{1-6}$ alkyl;
$R_{13}$ is selected from H, halogen and optionally halogen-substituted $-C_{1-6}$ alkyl;
$R_{14}$ is H;
k is selected from 0 or 1;
m and n are each independently selected from an integer from 0 to 2; and
t is selected from 1 or 2.

**16.** A compound selected from the example compounds, a stereoisomer, tautomer, stable isotopic variant, pharmaceutically acceptable salt or solvate thereof.

**17.** A pharmaceutical composition comprising a compound according to any one of claims 1-16, a stereoisomer, tautomer, stable isotopic variant, pharmaceutically acceptable salt or solvate thereof, and a pharmaceutically acceptable

excipient.

**18.** The compound according to any one of claims 1-16, or a pharmaceutically acceptable salt or solvate thereof, or the pharmaceutical composition according to claim 17, for use as medicament, for the treatment and/or prevention of a disease mediated by a KRas mutation, preferably KRas G12V and/or KRas G12D mutation.

**19.** Use of the compound according to any one of claims 1-16, or a pharmaceutically acceptable salt or solvate thereof, or the pharmaceutical composition according to claim 17 in the preparation of a medicament for preventing or treating a disease mediated by a KRas mutation, preferably KRas G12V and/or KRas G12D mutation.

**20.** The use according to claim 19, wherein the disease mediated by a KRas mutation, preferably KRas G12V and/or KRas G12D mutation, is selected from pancreatic cancer, lung cancer, lung adenocarcinoma, bone cancer, skin cancer, head and neck cancer, skin or intraocular melanoma, uterine cancer, ovarian cancer, rectal cancer, anal region cancer, stomach cancer, colon cancer, breast cancer, fallopian tube cancer, endometrial cancer, cervical cancer, vaginal cancer, vulvar cancer, Hodgkin's disease, esophageal cancer, small bowel cancer, endocrine system cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or ureteral cancer, renal cell carcinoma, renal pelvic cancer, central nervous system tumors (CNS), primary CNS lymphoma, spinal tumors, brainstem gliomas, or pituitary adenomas.

**21.** The use according to claim 20, wherein the disease mediated by a KRas mutation, preferably KRas G12V and/or KRas G12D mutation, is selected from pancreatic cancer, colon cancer, rectal cancer, lung adenocarcinoma, lung cancer, bile duct cancer, endometrial cancer, ovarian cancer, leukemia; most preferably selected from pancreatic cancer, colon cancer, rectal cancer, lung adenocarcinoma, and bile duct cancer.

**22.** A method for treating and/or preventing a disease mediated by a Ras mutation, especially KRas mutation, preferably KRas G12Vand/or KRas G12D mutation, comprising administering a therapeutically effective amount of the compound according to any one of claims 1-16, or a pharmaceutically acceptable salt or solvate thereof or the pharmaceutical composition according to claim 17, to a subject in need thereof.

**23.** The method according to claim 22, wherein the disease mediated by a KRas mutation, preferably KRas G12V and/or KRas G12D mutation, is selected from pancreatic cancer, colon cancer, rectal cancer, lung adenocarcinoma, lung cancer, bile duct cancer, endometrial cancer, ovarian cancer, leukemia; most preferably selected from pancreatic cancer, colon cancer, rectal cancer, lung adenocarcinoma, and bile duct cancer.

**INTERNATIONAL SEARCH REPORT**

International application No.
**PCT/CN2023/122129**

**A. CLASSIFICATION OF SUBJECT MATTER**

C07D487/08(2006.01)i; C07D519/00(2006.01)i; A61P35/00(2006.01)i; A61K31/517(2006.01)i; A61K31/4995(2006.01)i

According to International Patent Classification (IPC) or to both national classification and IPC

**B. FIELDS SEARCHED**

Minimum documentation searched (classification system followed by classification symbols)

IPC C07D A61K A61P

Documentation searched other than minimum documentation to the extent that such documents are included in the fields searched

Electronic data base consulted during the international search (name of data base and, where practicable, search terms used)

CNTXT, WPABS, ENTXTC, STN (REG, CAPLUS, MARPAT), CNKI, 百度, Baidu, Google学术, Google Scholar, 喹唑啉, 苯并嘧啶, 哌啶, 哌嗪, 肿瘤, 癌症, 突变, KRAS, quinazolin?, pyrimidin?, tumor, cancer, 结构检索, structure search

**C. DOCUMENTS CONSIDERED TO BE RELEVANT**

| Category* | Citation of document, with indication, where appropriate, of the relevant passages | Relevant to claim No. |
|---|---|---|
| PX | WO 2023125989 A1 (SHANGHAI PHARMACEUTICALS HOLDING CO., LTD.) 06 July 2023 (2023-07-06)<br>see description, pages 75-76, and claims 1 and 10-12 | 1-23 |
| PX | WO 2022228568 A1 (GENFLEET THERAPEUTICS (SHANGHAI) INC. et al.) 03 November 2022 (2022-11-03)<br>see description, pages 147-148, and claims 1 and 13-15 | 1-23 |
| PX | WO 2023072188 A1 (BETTA PHARMACEUTICALS CO., LTD.) 04 May 2023 (2023-05-04)<br>see claims 1-29 | 1, 3, 5-12, 14, 16-23 |
| PX | WO 2023098832 A1 (3D MEDICINES (SHANGHAI) CO., LTD.) 08 June 2023 (2023-06-08)<br>see claims 1-15 | 1-23 |
| X | WO 2022192794 A1 (BRISTOL-MYERS SQUIBB COMPANY) 15 September 2022 (2022-09-15)<br>see description, pages 53-54, 122, 184-187 and 238, and claims 1 and 36-39 | 1-23 |
| X | WO 2022061251 A1 (PLEXXIKON INC.) 24 March 2022 (2022-03-24)<br>see description, page 211, and claims 1 and 26-30 | 1-23 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **05 December 2023** | **11 December 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/122129**

**C. DOCUMENTS CONSIDERED TO BE RELEVANT**

| Category* | Citation of document, with indication, where appropriate, of the relevant passages | Relevant to claim No. |
|---|---|---|
| X | WO 2022002102 A1 (INVENTISBIO CO., LTD.) 06 January 2022 (2022-01-06) see description, pages 48-49, and claims 1 and 51-54 | 1-23 |
| X | WO 2022148422 A1 (BEIGENE LTD. et al.) 14 July 2022 (2022-07-14) see description, page 78, and claims 1 and 39-40 | 1, 3, 5-12, 16-23 |
| X | WO 2022199586 A1 (SUZHOU ZELGEN BIOPHARMACEUTICALS CO., LTD. et al.) 29 September 2022 (2022-09-29) see claims 1-15 | 1-23 |
| A | WO 2022068921 A1 (SHANGHAI PHARMACEUTICALS HOLDING CO., LTD.) 07 April 2022 (2022-04-07) see claims 1-22 | 1-23 |

Form PCT/ISA/210 (second sheet) (July 2022)

**INTERNATIONAL SEARCH REPORT**

International application No.
**PCT/CN2023/122129**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **22-23**
because they relate to subject matter not required to be searched by this Authority, namely:

Claims 22-23 relate to a method for diagnosing and treating a disease. The present search report is provided on the basis of the pharmaceutical use of the compound or composition.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/122129**

| Patent document cited in search report | Publication date (day/month/year) | Patent family member(s) | Publication date (day/month/year) |
|---|---|---|---|
| WO 2023125989 A1 | 06 July 2023 | None | |
| WO 2022228568 A1 | 03 November 2022 | None | |
| WO 2023072188 A1 | 04 May 2023 | None | |
| WO 2023098832 A1 | 08 June 2023 | None | |
| WO 2022192794 A1 | 15 September 2022 | IL 305568 A | 01 October 2023 |
| | | CA 3210053 A1 | 15 September 2022 |
| WO 2022061251 A1 | 24 March 2022 | None | |
| WO 2022002102 A1 | 06 January 2022 | None | |
| WO 2022148422 A1 | 14 July 2022 | None | |
| WO 2022199586 A1 | 29 September 2022 | None | |
| WO 2022068921 A1 | 07 April 2022 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202211208795
- CN 202211583282
- CN 2023100802872
- CN 2023102587885
- CN 2023107213489
- CN 2023112477769
- WO 2021041671 A1
- WO 2021118877 A1


### Non-patent literature cited in the description


- **ALAMGEER et al.** *Current Opin Pharmacol.*, 2013, vol. 13, 394-401
- **ZHI TAN et al.** *Mini-Reviews in Medicinal Chemistry*, 2016, vol. 16, 345-357
- **CHANG, E.H. et al.** *Proceedings of the National Academy of Sciences of the United States of America*, 1982, vol. 79 (16), 4848-4852
- **CCORMICK, F. et al.** *Clinical Cancer Research*, 2015, vol. 21 (8), 1797-1801
- Dictionary of Chemical Chemistry Terms. McGraw-Hill Book Company, 1984
- **ELIEL, E.** ; **WILEN, S.** Stereotics of Organic Compounds. John Wiley & Sons, Inc, 1994
- **REMINGTON**. Pharmaceutical Sciences
- **ANSEL, HOWARD C et al.** Ansel's Pharmaceutical Dosage Forms and Drug Delivery Systems. Lippincott, Williams & Wilkins, 2004
- **SMITH M.B**. March's Advanced Organic Chemistry: Reactions, Mechanisms, and Structure. Wiley, 2013
- **E. L. ELIEL** ; **S. H. WILEN** ; **L. N. MANDER**. Stereochemistry of Organic Compounds. Wiley-Interscience, 1994
- **T. GREENE** ; **P. WUTS**. Protective Groups in Organic Synthesis. John Wiley & Sons, 1999
- *J. Med. Chem.*, 2022, vol. 65 (4), 3123-3133
- **NAGILLA R. et al.** *J. Pharm. Sci*, 2011, vol. 100, 3862-3874
- *CHEMICAL ABSTRACTS*, 9004-67-5